# EUROPEAN PATENT APPLICATION

(11) **EP 4 477 653 A1**
(43) Date of publication of application: **18.12.2024**
(21) Application number: 23305945.0
(22) Date of filing: 14.06.2023
(51) Int. Cl.: C07D 487/04, A61K 31/5025, A61P 27/16, A61P 35/00, A61P 13/12

(54) **[1,2,4]-TRIAZOLO[4,3-B]PYRIDAZINE DERIVATIVES USEFUL AS A MEDICAMENT**

(71) Applicant: Perha Pharmaceuticals, 29680 Roscoff (FR)
(72) Inventor: The designation of the inventor has not yet been filed
(74) Representative: Cabinet Nony

(57) **Abstract**

The present invention relates to a compound of formula (I) or any of its pharmaceutically acceptable salt

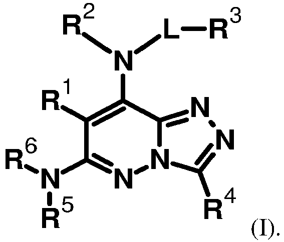

The present invention further relates to a synthesis process for manufacturing compound of formula (I) or any of its pharmaceutically acceptable salts, comprising at least a step of reacting a compound of formula (II) 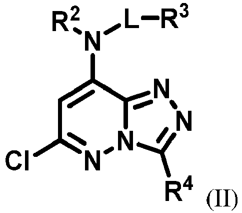 with an amine of formula (IV)
NHR⁵R⁶ (IV)
for example in a molar ratio ranging from 1 to 20 eq, with respect to the compound of formula (II), in an aprotic solvent or without solvent or else in a protic solvent.

## Description

### FIELD OF THE INVENTION

The present invention relates to [1,2,4]-triazolo[4,3-b]pyridazine derivatives useful in treating pathologies mediated by adenosine A3 receptor (A₃AR). It further relates to their manufacturing process and to pharmaceutical compositions containing them.

### BACKGROUND

Adenosine interacts with four types of G-protein-coupled receptors (GPCRs), the adenosine receptors (AR): A₁AR, A_{2A}AR, A_{2B}AR, A₃AR as described in IJzerman, A. P. et al., International Union of Basic and Clinical Pharmacology, CXII: Adenosine Receptors: A Further Update, Pharmacol Rev 2022, 74 (2), 340-372. A_{2A}AR has been most extensively studied while A₃AR is the least studied AR.

A large number of sub-type specific AR agonists and antagonists have been developed in the last few decades and evaluated in clinical trials for numerous indications as described in IJzerman, A. P. et al., International Union of Basic and Clinical Pharmacology, CXII: Adenosine Receptors: A Further Update, Pharmacol Rev 2022, 74, 340-372; and described in Jacobson, K. A. et al. Medicinal Chemistry of P2 and Adenosine Receptors: Common Scaffolds Adapted for Multiple Targets, Biochem Pharmacol 2021, 187, 114311. The approved AR drugs are the A₁AR agonist adenosine (paroxysmal supraventricular tachycardia), the A_{2A}AR agonist Regadenoson (used for myocardial perfusion imaging), the A₁AR antagonist Bamifylline (asthma) and the A_{2A}AR antagonist Istradefylline (Parkinson's disease).

A₃AR (P0DMS8) (gene located on human chromosome 1 (1p13.2); 318 amino acids in human) was discovered in the early nineties as described in Borea, P. A. et al., The A3 Adenosine Receptor: History and Perspectives, Pharmacol Rev 2015, 67, 74-102. A₃AR is coupled to Gi proteins, leading to inhibition of adenylate cyclase, and to Go proteins, leading to activation of phospholipase C and Ca⁺⁺ release. A₃AR is also coupled to PI3K/Akt, MAPKs and NF-κB signaling pathways. The biodistribution of A₃AR is rather species-dependent. In man, A₃AR is expressed in lung, liver, kidney, pancreas, brain, testis and cells of the immune system. A₃AR knock-out mice have been generated to investigate the functions of A₃AR. A₃AR^{-/-} mice are viable, fertile and apparently morphologically indistinguishable from wild-type mice, suggesting that inhibition of this receptor is unlikely to lead to gross toxicity.

Although A₁AR, A_{2A}AR and A_{2B}AR are well conserved throughout evolution, there are rather important differences in the A₃AR sequences of rodents and man (72% and 73% sequence identity between human A₃AR and rat or mouse A₃AR, respectively). Many human A₃AR antagonists are very poorly active or even inactive on rat and mouse orthologs. This complicates translational investigations on the action of A₃AR agonists and antagonists in rodent models of human diseases. A₃AR-humanized (A₃AR^{h/h}) and human/mouse chimeric A₃AR (A₃AR^{c/c}) mice have been constructed which are sensitive to the agonists developed against human A₃AR.

In cerebral ischemia A₃AR may play both a protective role, by inhibiting excitatory synaptic transmission in synergy with A_{1A}R, and a deleterious effect, by favoring excitotoxicity. A₃AR agonists may regulate pain signaling, in particular in the case of anticancer drugs-induced neuropathic pain. A₃AR is overexpressed in several tumors and has anti-proliferative and pro-proliferative effects. A₃AR agonists and antagonists display antitumor activity. A₃AR appears to play a key role in the modulation of inflammation by adenosine. A₃AR agonists have therefore been evaluated in clinical trials to treat various inflammatory diseases such as osteoarthritis of the knee, asthma, lung fibrosis. A₃AR agonists have been investigated for induction of hypothermia as well as for potential cardioprotective activity, yet the deletion of A₃AR appears to confer some resistance to myocardial ischemic injury.

Several potent and selective A₃AR agonists have been described, Piclidenoson (IB-MECA, N⁶-iodobenzyl-substituted methylcarboxamidoadenosine, CF101, Can-Fite BioPharma) and Namodenoson (2-Cl-IB-MECA, CF102) being the most advanced drug candidates. Piclidenoson is in clinical trials against in rheumatoid arthritis and psoriasis, while Namodenoson is in clinical trials against hepatocellular carcinoma and non-alcoholic steatohepatitis (NASH).

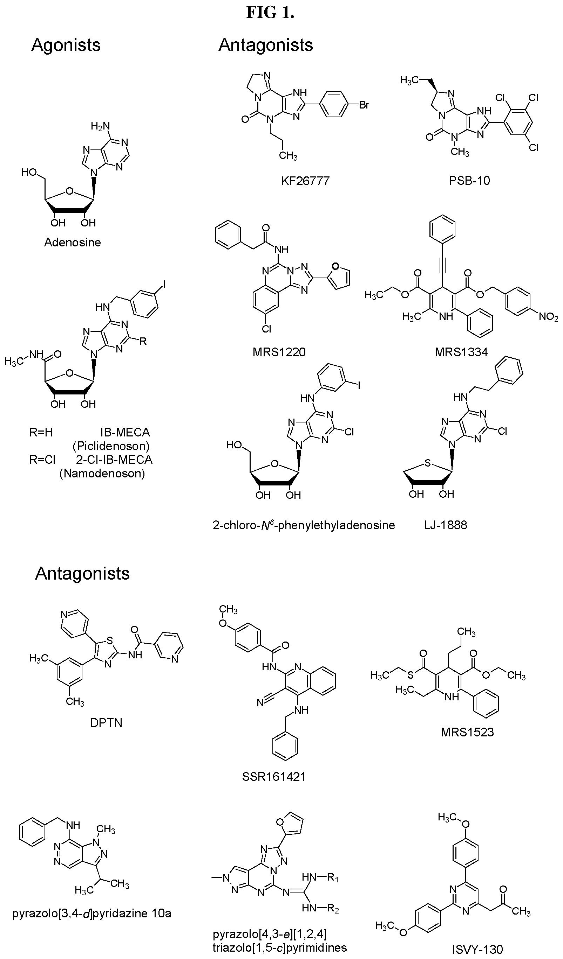

Several A₃AR antagonists have also been described, such as KF2677, PSB-10, DPTN (N-[4-(3,5-dimethylphenyl)-5-(4-pyridyl)-1,3-thiazol-2-yl]nicotinamide), MRS1523, SSR161421, LJ-1888, pyrazolo-triazolo-pyrimidines, ISVY-130 analogs, 2-chloro-N6-phenylethyladenosine as described in Borea, P. A. *et al.* previously mentioned, 7-Amino-pyrazolo[3,4-d]pyridazine 10a (dual A1/A3AR antagonist), as described in Gao, Z.-G. et al. Species Dependence of A3 Adenosine Receptor Pharmacology and Function, Purinergic Signal 2022, 1-28, as illustrated in **figure 1****.**

In prior art, only the compounds PBF-677 and PBF-1650, (undisclosed structures) have entered clinical trials, for ulcerative colitis (phase 2) and atopic dermatitis (phase 1), respectively (https://www.palobiofarma.com/pipeline-2/). A₃AR antagonists are evaluated for the treatment of glaucoma as they lower intraocular pressure (IOP) in animal models of glaucoma. A₃AR antagonists have been evaluated as treatment of asthma, ulcerative colitis, liver fibrosis, kidney diseases in particular kidney fibrosis, nephropathy, nephrotoxicity, atherosclerosis and hypercholestemia. Stimulation of A₃AR by adenosine, under hypoxia, contributes to the migration, invasiveness and chemoresistance of glioblastoma stem-like cells and probably in other cancer types under hypoxic conditions. A₃AR antagonists inhibit the proliferation of glioblastoma cell lines.

Hearing loss (HL) originates from exposure to ototoxic agents (such as platinum-based anticancer drugs or aminoglycoside antibiotics - there are over 600 ototoxic agents described, among which over 200 marketed drugs), from noise, from acoustic trauma (high intensity noise) or from aging. These aggressions trigger irreversible death of ear hair cells located in the organ of Corti (cochlea). The function of these cells is to capture sound vibrations and transduce them as neuronal signals sent to the brain auditory structures. There is considerable interest in the development of otoprotective compounds.

Cisplatin is a highly effective anticancer drug widely used for the treatment of cancers. Cisplatin's side effects include nephrotoxicity and ototoxicity. Cisplatin-induced ototoxicity occurs in 23-50% of adults and up to 60% in children. Elevated hearing thresholds may occur in up to 100% of cisplatin-treated cancer patients. Otoprotection would allow the use of these highly effective drugs for life threatening diseases at reduced risk of HL, thereby addressing a significant unmet clinical need. Similarly addressing cisplatin-induced nephrotoxicity would be beneficial. The same applies for the potent but HL-inducing antibiotics, in particular ototoxicity is observed in up to 47% of patients treated with gentamycin, and other products such as neomycin, tobramycin, kanamycin. Noise-induced HL occurs when the ear is exposed to unsafe levels of sounds. The industries where workers often experience noise exposure include agriculture, mining, construction, manufacturing and utilities, transportation, and the military. Globally, some 1.1 billion teenagers and young adults are at risk of HL due to the unsafe use of personal audio devices and exposure to damaging levels of sound at noisy entertainment venues. In all these situations, external protective ear coverings are either insufficient or inappropriate. Moreover, A₃R is in particular present in the rat cochlea and is predominantly expressed in the organ of Corti (inner and outer hair cells, Deiter's cells, Claudius cells, pillar cells). Furthermore, adenosine plays a protective role in nephrotoxicity induced by various drug agents. In particular, there is data available in the litterature suggesting that A₂AR and A₃AR antagonism may have some protective effects in kidneys exposed to nephrotoxic agents as described in Dewaeles et al. Istradefylline protects from cisplatin-induced nephrotoxicity and peripheral neuropathy while preserving cisplatin antitumor effects, J. Clin. Invest. 2022, 132, e152924; Lee et al. A3 adenosine receptor knockout mice are protected against ischemia- and myoglobinuria-induced renal failure, Am. J. Physiol. Renal Physiol. 2003, 284, 267-273; and Min et al. Renopprotective effects of a highly selective A3 adenosine receptor antagonist in a mouse model of adriamycin-induced nephropathy, J. Korean Med. Sci. 2016, 31, 1403-1412.

There is a thus a need to find therapeutic agents to target specific inhibition of A₃R.

### SUMMARY OF THE INVENTION

It has now been found that the compounds as defined in formula (I) hereinafter are useful in the treatment and/or prevention of diseases mediated by A₃R.

### FIGURES

**Figure 1** depicts two agonists and a few reported antagonists of A3AR.
**Figure 2** depicts the results of an evaluation of the potential agonist or antagonist activity of compound (175) on 168 GPCRs and being illustrated in example 10.2.
**Figure 3** depicts the results of a study evaluating the selectivity of compound (175) towards various ARs being illustrated in example 10.3.
**Figure 4** depicts the results of an A₃AR functional assay being illustrated in exemple 10.3.
**Figure 5** depicts the effects of compound (175) on ciliated cells in *ex vivo* cultured mice organs of Corti exposed to either cisplatin (5A), gentamycin (5B) or neomycin (5C), and being illustrated in example 10.4.

### DEFINITIONS

As used herein, the term *"patient"* refers to either an animal, such as a valuable animal for breeding, company or preservation purposes, or preferably a human or a human child, which is afflicted with, or has the potential to be afflicted with one or more diseases and conditions described herein.

In particular, as used in the present application, the term *"patient"* refers to a mammal such as a rodent, cat, dog, primate or human, preferably said subject is a human.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term *"treating"* or *"treatment",* as used herein, means preventing, reversing, alleviating, inhibiting the progress of, or preventing the diseases as described herein after in the paragraph "PATHOLOGIES".

Therefore, the term *"treating"* or *"treatment"* encompasses within the framework of the present invention the improvement of medical conditions of patients suffering from the diseases as described herein after in the paragraph "PATHOLOGIES", mediated by the A₃AR receptor.

As used herein, an "A₃AR *inhibitor"* or "A₃AR *antagonist"* is a compound able to inhibit A₃AR in the assays displayed in example 10.1 in biological activity part described hereafter. In one embodiment, the IC₅₀ value from the compounds of formula (I) according to the invention may be less than or equal to 1000 nM, in particular less than or equal to 100 nM, and more particularly less than or equal to 10 nM, and even more particularly less than or equal to 1 nM, said IC₅₀ values all attesting the inhibitory activity of the compounds.

As used herein, an *"effective amount"* refers to an amount of a compound of the present invention which is effective in preventing, reducing, eliminating, treating or controlling the symptoms of the herein-described diseases and conditions. An *"effective amount"* also refers to an amount of a compound of the present invention which is effective in inhibiting A₃AR.

The term *"controlling"* is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term *"effective amount"* includes *"prophylaxis-effective amount"* as well as "treatment-effective amount".

The term *"preventing",* as used herein, means reducing the risk of onset or slowing the occurrence of a given phenomenon, namely in the present invention, a disease mediated by ADORA3.

As used herein, *"preventing"* also encompasses *"reducing the likelihood of occurrence"* or *"reducing the likelihood of reoccurrence".*

The term *"prophylaxis-effective amount"* refers to a concentration of compound of this invention that is effective in inhibiting, preventing, decreasing the likelihood of anyone of the hereabove described diseases.

Likewise, the term *"treatment-effective amount"* refers to a concentration of compound that is effective in treating the hereabove described diseases.

As used herein, the term *"pharmaceutically acceptable"* refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### DETAILED DESCRIPTION OF THE INVENTION

The inventors have surprisingly found that the compounds of formula (I) as disclosed herein after, present inhibition activity of A₃AR.

This assertion is based on data as illustrated in the following examples and more detailed herein after.

According to a first aspect, a subject-matter of the present invention relates to a compound of formula (I) or any of its pharmaceutically acceptable salt 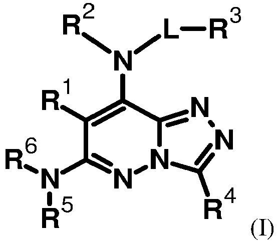 wherein
**R¹** represents a hydrogen atom or a (C₁-C₆)alkyl group;
**R²** represents a hydrogen atom;
**R³** represents
   - a linear or branched (C₁-C₆)alkyl group, optionally substituted by
      - one phenyl group, optionally substituted by at least one substituent selected from a -OR⁷ group, a -NH₂ group, a halogen atom, a phenyl group and a (C₅-C₆)heteroaryl group,
      - one (C₆-C₁₀)heteroaryl group, or
      - one -OR⁷ group,
   - a phenyl group or a phenyl group fused with a (C₄-C₆)cycloalkyl group; or
   - a (C₅-C₆)heteroaryl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom, in particular a fluorine atom or a chlorine atom;
**L** represents a bond, a -CO- group or a -C(O)O- group;
or alternately **L** is a bond and **R²** and **R³** form together with the nitrogen atom bearing them a (C₅-C₆)heterocycloalkyl group containing at least a nitrogen atom fused with a phenyl group;
**R⁴** represents a hydrogen atom, a linear or branched (C₁-C₆)alkyl group, a (C₃-C₆)cycloalkyl group, a (C₁-C₄)alkoxy group, a (C₅-C₆)heterocyclocalkyl group, a phenyl group, a (C₅-C₆)heteroaryl group or a -CF₃ group;
**R⁵** represents a hydrogen atom or a (C₁-C₄)alkyl group;
**R⁶** represents
   - a linear or branched (C₁-C₆)alkyl group, optionally interrupted by one or two oxygen atoms and optionally substituted by
      - one phenyl group, optionally substituted by one or two substituents selected from a (C₁-C₄)alkyl group and a (C₁-C₄)alkoxy group,
      - one (C₃-C₈)cycloalkyl group, optionally substituted by one or two substituents selected from a halogen atom, a (C₁-C₄)alkyl group and a -OR⁷ group,
      - one (C₅-C₆)heterocycloalkyl,
      - one (C₅-C₆)heteroaryl group, optionally substituted by one or two (C₁-C₆)alkyl group,
      - one or two -OR⁷ groups,
      - one -SR⁷ group,
      - one -C(O)NR⁸R⁹ group,
      - one -C(O)OR¹⁰ group, or
      - one -NHR¹¹ group,
   - a (C₃-C₇)cycloalkyl group, optionally substituted by one or two substituents selected from a halogen atom, a (C₁-C₄)alkyl group and a -OR⁷ group,
   - one bridged (C₆-C₁₀)cycloalkyl group,
   - one spiro(C₅-C₁₁)bicyclic ring,
   - a (C₅-C₆)heteroaryl group,
   - a (C₅-C₆)heterocycloalkyl group, or
   - a phenyl group fused with a (C₄-C₆)cycloalkyl group;
or **R⁵** and **R⁶** form together with the nitrogen atom bearing them a (C₅-C₆)heterocycloalkyl group;
**R⁷** represents a hydrogen atom, a (C₁-C₄)alkyl group or a deuterated (C₁-C₄)alkyl group;
**R⁸** and **R⁹** independently represent a hydrogen atom or a (C₁-C₆)alkyl group;
**R¹⁰** represents a (C₁-C₄)alkyl group; and
**R¹¹** represents a hydrogen atom or a -CO-(C₁-C₆)alkyl group;
provided that when NR²LR³ represents a benzylamino group, then R⁴ does not represent a hydrogen atom.

The inventors have surprisingly discovered that compounds of formula (I) present an inhibitory activity specific to the Adenosine receptor A3 (A₃AR) and that some of them even present a powerful inhibitory activity.

According to another particular embodiment, herein is further provided a compound of formula (I) as defined herein above, wherein **R¹** represents a hydrogen atom or a methyl group.

According to another particular embodiment, herein is further provided a compound of formula (I) as defined herein above, wherein
**R²** represents a hydrogen atom;
**R³** represents
   - a linear or branched (C₁-C₆)alkyl group, optionally substituted by
      - one phenyl group, optionally substituted by one substituent selected from a hydroxy group, a methoxy group, an amino group, a halogen atom, a phenyl group and a pyridyl group,
      - one pyridyl, one pyrazinyl, one pyrimidinyl or one pyridazinyl group, in particular one pyridyl, pyridazinyl or pyrimidinyl group, or one indolyl or isoindolyl group, in particular an indolyl group,
      - one hydroxy group,
      - one methoxy group, one deuterated methoxy group, or
      - a halogen atom, in particular a fluorine atom or a chlorine atom,
   - a phenyl group or an indanyl group; or
   - a pyridyl, a pyrazinyl, a pyrimidinyl or a pyridazinyl group, optionally substituted by one methoxy group or deuterated methoxy group, in particular a pyridyl group;
**L** represents a bond, a -CO- group or a -C(O)O- group;
or alternately **L** is a bond and **R²** and **R³** form together with the nitrogen atom bearing them an indolinyl, an isoindolinyl, a tetrahydroquinolinyl or a tetrahydroisoquinolinyl group, in particular an isoindolinyl or a tetrahydroisoquinolinyl group.

According to another particular embodiment, herein is further provided a compound of formula (I) as defined herein above, wherein **R⁴** represents a hydrogen atom, a linear or branched (C₁-C₅)alkyl group, a (C₃-C₆)cycloalkyl group, a phenyl group, a pyridyl group or a -CF₃ group.

According to another particular embodiment, herein is further provided a compound of formula (I) as defined herein above, wherein
**R⁵** represents a hydrogen atom or a (C₁-C₄) alkyl group, in particular a methyl group;
**R⁶** represents
   - a linear or branched (C₁-C₆)alkyl group, optionally interrupted by one or two oxygen atoms and optionally substituted by
      - one phenyl group, optionally substituted by one or two substituents selected from a methyl group and a methoxy group,
      - one (C₃-C₆)cycloalkyl group, optionally substituted by a methyl group,
      - one tetrahydropyranyl or one tetrahydrofuranyl group,
      - one pyrazolyl, imidazolyl, pyrimidinyl, pyrazinyl, pyridyl, furanyl or thienyl group, optionally substituted by one or two methyl group,
      - one or two hydroxy group or methoxy group,
      - one methylthio group,
      - one -CONH₂ group, one -CONHCH₃ group or one -CON(CH₃)₂ group,
      - one -COOCH₃ or one -COOCH₂CH₃ group, or
      - one -NH₂ group or one -NCOCH₃ group,
   - a (C₃-C₇)cycloalkyl group, optionally substituted by one or two substituents selected from a halogen atom, a hydroxy group or a methoxy group,
   - an adamantyl group,
   - a spiro[3.3]heptanyl group,
   - an imidazolyl group, optionally substituted by one or two methyl group,
   - a morpholinyl group, a tetrahydropyranyl or a tetrahydrofuranyl group, or
   - an indanyl group;
or **R⁵** and **R⁶** form together with the nitrogen atom bearing them a morpholinyl group or a piperidinyl group.

Herein is further provided a pharmaceutical composition comprising a compound of formula (I) as defined herein after, or at least any of compounds **(1)** to **(220)** as defined hereinafter or any of their pharmaceutically acceptable salts.

As illustrated in example 10.1, the compounds of formula (I) have been tested as A₃AR antagonists. It has been observed that the compounds of formula (I) exert an inhibitory activity on A₃AR and thus may be useful in the treatment of pathologies mediated by A₃AR.

As illustrated in example 10.2, a compound of formula (I) has been tested with respect to 168 GPCR receptors. It has been surprisingly observed that this compound of formula (I) is only active on one of them.

As illustrated in example 10.3, a compound of formula (I) has been additionnaly tested with respect to other adenosine receptors A₁AR, A_{2A}AR and A_{2B}AR for selectivity study. It has been surprisingly observed that this compound of formula (I) is only active on A₃AR. Moreover, a compound of formula (I) has been additionnaly tested in A₃AR functional assay. It has been observed that this compound of formula (I) is an antagonist inhibitor of A₃AR.

The unusal and unexpected specificity and functionality highligted in examples 10.2 and 10.3 calls for the development of potent and selective A₃AR antagonists with clinical applications.

In other terms, as illustrated in examples 10.1, 10.2 and 10.3 the compounds of formula (I) are potent and selective A₃AR antagonists.

Additionaly, the inventors have suprisingly found that compounds of formula (I) display protective activity towards inner ear hair cells of the organ of Corti exposed to ototoxic agents, in particular platin-based anticancer drugs such as cisplatin, or antibiotics aminoglycosides such as gentamicin or neomycin. Typically, as illustrated in example 10.4, the inventors have shown that the compounds of the present invention display otoprotective activity on *ex vivo* models of ototoxicity (cultured organs of Corti) induced by ototoxic agents, in particular platin-based anticancer drugs such as cisplatin, or antibiotics aminoglycosides such as gentamicin or neomycin.

In the context of the present invention, the term:
- "halogen" is understood to mean chlorine, fluorine, bromine, or iodine, and in particular denotes chlorine, fluorine or bromine,
- "(C₁-Cₓ)alkyl", as used herein, respectively refers to a C₁-Cₓ normal, secondary or tertiary monovalent saturated hydrocarbon radical, for example (C₁-C₆)alkyl. Examples are, but are not limited to, methyl, ethyl, propyl, *n*-propyl, isopropyl, butyl, isobutyl, *sec-*butyl, *tert*-butyl, pentyl, isopentyl, hexyl and isohexyl groups, and the like.
- "(C₁-Cₓ)alkoxy", as used herein, refers to a -O-(C₁-Cₓ)alkyl or -O-(C₃-Cₓ)cycloalkyl moiety, wherein alkyl and cycloalkyl are as defined above, for example (C₁-C₆)alkoxy. Examples are, but are not limited to, methoxy, ethoxy, 1-propoxy, 2-propoxy, cyclopropoxy, butoxy, *tert*-butoxy and pentoxy.
- a (C₁-C₄)alkylthio group also named a (C₁-C₄)alkylsulfanyl group: a -S-alkyl group where the alkyl group is as previously defined. By way of examples, mention may be made of, but not limited to: methylthio, ethylthio, propylthio, isopropylthio, linear, secondary or tertiary butylthio, isobutylthio, and the like;
- "(C₃-C₈)cycloalkyl", as used herein, refers to a monocyclic saturated hydrocarbon, from 3 to 8 carbon atoms, saturated or partially unsaturated and unsubstituted or substituted. Examples of monocyclic rings are, but are not limited to, cyclopropyl, cyclobutyl, cyclopentyl, cyclohexyl, cycloheptyl and cyclooctyl.
- a "bridged (C₆-C₁₀)cycloalkyl" group, as used herein, refers to a bi- or tricyclic compound where the cycles are cycloalkyls, the rings share three or more atoms and the bridge contains at least one atom, for example 1, 2 or 3 atoms. Such bridged cycloalkyl groups may be substituted by one or more C₁-C₃ alkyl. Examples are, but not limited to adamantyl, 2,6,6-trimethylbicyclo[3.1.1]heptyl, 6,6-dimethylbicyclo[3.1.1]heptyl, bicyclo[3.1.1]heptyl, 1,6,6-trimethylbicyclo[3.1.1]heptyl.
- "spiro(C₅-C₁₁)bicyclic ring" refers to two rings connected through a defining single common atom. Such spiro bicyclic alkyl generally comprises 5 to 11 carbon atoms referring to a "spiro(C₅-C₁₁)bicyclic alkyl group". Such spirobicyclic ring may be unsubstituted or substituted, in particular by at least one (C₁-C₃)alkyl group such as methyl. Examples are, but are not limited to spiro[3.3]heptanyl, spiro[2.5]octanyl, in particular a spiro[3.3]heptanyl.
- "(C₅-C₆)heterocycloalkyl group", as used herein, refers to a (C₅-C₆)cycloalkyl group wherein one or two of the carbon atoms are replaced with a heteroatom such as oxygen, nitrogen or sulphur, and more particularly with at least one nitrogen atom. Such heterocycloalkyl group may be saturated or partially saturated and unsubstituted or substituted. Examples are, but are not limited to piperazinyl, piperidinyl, pyrrolidinyl, aziridinyl, oxanyl, oxetanyl, tetrahydropyranyl, morpholinyl, tetrahydrofuranyl, oxazolidinyl, oxepanyl, diazepanyl, dioxanyl and tetrahydrothiopyranyl, and more particularly pyrrolidinyl, piperidinyl, morpholinyl, tetrahydropyranyl or one tetrahydrofuranyl group.
- a (C₅-C₁₀)heteroaryl group, as used herein, refers to a monocyclic aromatic or a bicyclic group wherein one to three carbon atom is replaced by a heteroatom, such as nitrogen, oxygen or sulphur. By way of examples of monocyclic aromatic ring of heteroaryl groups, more particulary as a (C₅-C₆)heteroaryl group, mention may be made of, but not limited to: oxazolyl, isoxazolyl, pyridyl, pyrimidinyl, pyridazinyl, triazinyl, pyrazinyl, oxadiazolyl, furanyl, pyrazolyl, thiazolyl, isothiazolyl, thiadiazolyl, imidazolyl, triazolyl and the like. In the framework of the present invention, the heteroaryl is advantageously pyridyl, imidazolyl, pyrazinyl, furanyl, thiazolyl, pyrazolyl, thiadiazolyl, pyridazinyl and pyrimidinyl, and in particular pyridyl, pyrazinyl, pyrimidinyl or pyridazinyl. By way of examples of bicyclic aromatic ring of heteroaryl groups, mention may be made of indolyl, isoindolyl, indolinyl, isoindolinyl, quinolinyl or isoquinolinyl.
- an aromatic ring means, according to Hückel's rule, that a molecule has 4n + 2 π-electrons.
- "deuterated" refers to a group wherein at least one hydrogen atom is replaced by a deuterium atom, such as perdeuterated group wherein all hydrogen atoms are replaced by deuterium atoms. In other terms, a deuterated group may be partially or totally deuterated. By way of examples of deuterated group, more particularly as deuterated (C₁-C₄)alkyl group, mention may be made of, but not limited to: -CH₂D group, -CHD₂ group or -CD₃ group. By way of other examples of deuterated group, more particularly as deuterated (C₁-C₄)alkoxy group, mention may be made of, but not limited to: deuterated methoxy group such as - -OCH₂D group, -OCHD₂ group or -OCD₃ group.

In the context of the present invention, the terms "aromatic ring", and "heteroaryl" include all the positional isomers.

The nomenclature of the following compounds (1) to (220) was generated according to the principles of the International Union of Pure and Applied Chemistry, using ChemDraw^{®} Professional v22.0.022. To avoid any confusion, the "(±)" symbol added to designate a racemic mixture; *"cis"* and *"trans"* prefixes were also used to assign the relative stereochemistry of two adjacent chiral centers.

Specific compounds of formula (I) of the present invention are listed herein after:
**(1)** (2*R*)-2-[[8-(benzylamino)-3-isopropyl-[1,2,4]triazolo[4,3-*b*]pyridazin-6-yl]amino]butan-1-ol,
**(2)** (2*S*)-2-[[8-(benzylamino)-3-isopropyl-[1,2,4]triazolo[4,3-*b*]pyridazin-6-yl]amino]butan-1-ol,
**(3)** 2-[[8-(benzylamino)-3-isopropyl-[1,2,4]triazolo[4,3-*b*]pyridazin-6-yl]amino]propane-1,3-diol,
**(4)** (2*R*,3*R*)-2-[[8-(benzylamino)-3-isopropyl-[[1,2,4]triazolo[4,3-*b*]pyridazin-6-yl]amino]butane-1,3-diol,
**(5)** (2*S*,3*S*)-2-[[8-(benzylamino)-3-isopropyl-[1,2,4]triazolo[4,3-*b*]pyridazin-6-yl]amino]butane-1,3-diol,
**(6)** *N*-benzyl-3-isopropyl-6-morpholino-[1,2,4]triazolo[4,3-*b*]pyridazin-8-amine
**(7)** (2*S*)-3-[[8-(benzylamino)-3-isopropyl-[1,2,4]triazolo[4,3-*b*]pyridazin-6-yl]amino]propane-1,2-diol,
**(8)** *N*6-[2-[2-(2-aminoethoxy)ethoxy]ethyl]-*N8*-benzyl-3-isopropyl-[1,2,4]triazolo[4,3-*b*]pyridazine-6,8-diamine,
**(9)** 3-[[8-(benzylamino)-3-isopropyl-[1,2,4]triazolo[4,3-*b*]pyridazin-6-yl]amino]propan- 1-ol,
**(10)** *N8*-benzyl-3-isopropyl-*N6*-(3-methoxypropyl)-[1,2,4]triazolo[4,3-*b*]pyridazine-6,8-diamine,
**(11)** 2-[[8-(benzylamino)-3-isopropyl-[1,2,4]triazolo[4,3-*b*]pyridazin-6-yl] amino] ethanol,
**(12)** *N8*-benzyl-3-isopropyl*-N6*-(2-methoxyethyl)-[1,2,4]triazolo[4,3-*b*]pyridazine-6,8-diamine,
**(13)** 4-[[8-(benzylamino)-3-isopropyl-[1,2,4]triazolo[4,3-*b*]pyridazin-6-yl]amino]butan-1-ol,
**(14)** *N8*-benzyl-3-isopropyl-*N6*-(4-methoxybutyl)-[1,2,4]triazolo[4,3-*b*]pyridazine-6,8-diamine,
**(15)** 5-[[8-(benzylamino)-3-isopropyl-[1,2,4]triazolo[4,3-*b*]pyridazin-6-yl]amino]pentan-1-ol,
**(16)** *N8*-benzyl-3-isopropyl-*N6*-(5-methoxypentyl)-[1,2,4]triazolo[4,3-*b*]pyridazine-6,8-diamine,
**(17)** *N8*-benzyl-3-isopropyl-*N6*-(3-methylsulfanylpropyl)-[1,2,4]triazolo[4,3-*b*]pyridazine-6,8-diamine,
**(18)** *N8*-benzyl-*N6*-(1-ethylpropyl)-3-isopropyl-[1,2,4]triazolo[4,3-*b*]pyridazine-6,8-diamine,
**(19)** *N8*-benzyl-3-isopropyl-*N6*-[(3*R*)-tetrahydrofuran-3-yl]-[1,2,4]triazolo[4,3-*b*]pyridazine-6,8-diamine,
**(20)** *N8*-benzyl-3-cyclopentyl-*N6*-[(3*R*)-tetrahydrofuran-3-yl]-[1,2,4]triazolo[4,3-*b*]pyridazine-6,8-diamine,
**(21)** *N8*-benzyl-3-isopropyl-*N6*-[(3*R*)-tetrahydropyran-3-yl]-[1,2,4]triazolo[4,3-*b*]pyridazine-6,8-diamine,
**(22)** *N8*-benzyl-3-isopropyl-*N6*-[(3*S*)-tetrahydropyran-3-yl]-[1,2,4]triazolo[4,3-*b*]pyridazine-6,8-diamine,
**(23)** *N8*-benzyl-3-isopropyl-*N6*-tetrahydropyran-4-yl-[1,2,4]triazolo[4,3-*b*]pyridazine-6,8-diamine,
**(24)** *N*-[3-[[8-(benzylamino)-3-isopropyl-[1,2,4]triazolo[4,3-*b*]pyridazin-6-yl] amino]propyl] acetamide,
**(25)** 3-[[8-(benzylamino)-3-isopropyl-[1,2,4]triazolo[4,3-*b*]pyridazin-6-yl]amino]propanamide,
**(26)** 3-[[8-(benzylamino)-3-isopropyl-[1,2,4]triazolo[4,3-*b*]pyridazin-6-yl]amino]-*N-*methyl-propanamide,
**(27)** 3-[[8-(benzylamino)-3-isopropyl-[1,2,4]triazolo[4,3-*b*]pyridazin-6-yl]amino]-*N*,*N*-dimethyl-propanamide,
**(28)** Methyl 3-[[8-(benzylamino)-3-isopropyl-[1,2,4]triazolo[4,3-*b*]pyridazin-6-yl]amino]propanoate,
**(29)** ethyl 3-[[8-(benzylamino)-3-isopropyl-[1,2,4]triazolo[4,3-*b*]pyridazin-6-yl]amino]propanoate,
**(30)** (2*R*)-2-[[8-(benzylamino)-3-cyclopropyl-[1,2,4]triazolo[4,3-*b*]pyridazin-6-yl]amino]butan-1-ol,
**(31)** *N8*-benzyl-3-cyclopropyl-*N6*-(3-methoxypropyl)-[1,2,4]triazolo[4,3-*b*]pyridazine-6,8-diamine,
**(32)** *N8*-benzyl-*N6*-(3-methoxypropyl)-3-methyl-[1,2,4]triazolo[4,3-*b*]pyridazine-6,8-diamine,
**(33)** (2*R*)-2-[[8-(benzylamino)-3-methyl-[1,2,4]triazolo[4,3-*b*]pyridazin-6-yl]amino]butan-1-ol,
**(34)** *N8*-benzyl-*N6*-(3-methoxypropyl)-3-phenyl-[1,2,4]triazolo[4,3-*b*]pyridazine-6,8-diamine,
**(35)** (2*R*)-2-[[8-(benzylamino)-3-phenyl-[1,2,4]triazolo[4,3-*b*]pyridazin-6-yl]amino]butan-1-ol,
**(36)** *N8*-benzyl-*N6*-(3-methoxypropyl)-3-(trifluoromethyl)-[1,2,4]triazolo[4,3-*b*]pyridazine-6,8-diamine,
**(37)** (2*R*)-2-[[8-(benzylamino)-3-(trifluoromethyl)-[1,2,4]triazolo[4,3-*b*]pyridazin-6-yl]amino]butan-1-ol,
**(38)** 2-[[[6-[[1*R*)-1-(hydroxymethyl)propyl]amino]-3-isopropyl-[1,2,4]triazolo[4,3-*b*]pyridazin-8-yl]amino]methyl]phenol,
**(39)** 2-[[[3-isopropyl-6-(3-methoxypropylamino)-[1,2,4]triazolo[4,3-*b*]pyridazin-8-yl] amino] methyl]phenol,
**(40)** 2-[[[6-(1-ethylpropylamino)-3-isopropyl-[1,2,4]triazolo[4,3-*b*]pyridazin-8-yl] amino] methyl]phenol,
**(41)** 3-[[[6-(1-ethylpropylamino)-3-isopropyl-[1,2,4]triazolo[4,3-*b*]pyridazin-8-yl] amino] methyl]phenol,
**(42)** 4-[[[6-(1-ethylpropylamino)-3-isopropyl-[1,2,4]triazolo[4,3-*b*]pyridazin-8-yl] amino] methyl]phenol,
**(43)** 2-[[[3-isopropyl-6-[[(3*R*)-tetrahydropyran-3-yl]amino]-[1,2,4]triazolo[4,3-*b*]pyridazin-8-yl] amino methyl]phenol,
**(44)** 2-[[[3-isopropyl-6-(tetrahydropyran-4-ylamino)-[1,2,4]triazolo[4,3-*b*]pyridazin-8-yl]amino]methyl]phenol,
**(45)** *N6*-(1-ethylpropyl)-3-isopropyl-*N8*-phenyl-[1,2,4]triazolo[4,3-*b*]pyridazine-6,8-diamine,
**(46)** *N6*-(1-ethylpropyl)-3-isopropyl-*N8*-(2-pyridyl)-[1,2,4]triazolo[4,3-*b*]pyridazine-6,8-diamine,
**(47)** *N6*-(1-ethylpropyl)-3-isopropyl-*N8*-(3-pyridyl)-[1,2,4]triazolo[4,3-*b*]pyridazine-6,8-diamine,
**(48)** *N6*-(1-ethylpropyl)-3-isopropyl-*N8*-(4-pyridyl)-[1,2,4]triazolo[4,3-*b*]pyridazine-6,8-diamine,
**(49)** *N6*-(1-ethylpropyl)-3-isopropyl-*N8*-(4-pyridylmethyl)-[1,2,4]triazolo[4,3-*b*]pyridazine-6,8-diamine,
**(50)** *N6*-(1-ethylpropyl)-3-isopropyl-*N*8-(3-pyridylmethyl)-[1,2,4]triazolo[4,3-*b*]pyridazine-6,8-diamine,
**(51)** *N6*-(1-ethylpropyl)-3-isopropyl-*N8*-(2-pyridylmethyl)-[1,2,4]triazolo[4,3-*b*]pyridazine-6,8-diamine,
**(52)** *N6*-tert-butyl-3-isopropyl-*N8*-(2-pyridylmethyl)-[1,2,4]triazolo[4,3-*b*]pyridazine-6,8-diamine,
**(53)** 3-isopropyl-*N8*-(2-pyridylmethyl)-*N6*-spiro[3.3]heptan-2-yl-[1,2,4]triazolo[4,3-*b*]pyridazine-6,8-diamine,
**(54)** *N6*-(3,3-difluorocyclobutyl)-3-isopropyl-*N8*-(2-pyridylmethyl)-[1,2,4]triazolo[4,3-*b*]pyridazine-6,8-diamine,
**(55)** *N6*-(4,4-difluorocyclohexyl)-3-isopropyl-*N8*-(2-pyridylmethyl)-[1,2,4]triazolo[4,3-*b*]pyridazine-6,8-diamine,
**(56)** *N6*-benzyl-3-isopropyl-*N8*-(2-pyridylmethyl)-[1,2,4]triazolo[4,3-*b*]pyridazine-6,8-diamine,
**(57)** *N6*-(1-ethylpropyl)-*N8*-(2-pyridylmethyl)-[1,2,4]triazolo[4,3-*b*]pyridazine-6,8-diamine,
**(58)** *N6-*(1-ethylpropyl)-3-methyl-N8-(2-pyridylmethyl)-[1,2,4]triazolo[4,3-*b*]pyridazine-6,8-diamine,
**(59)** 3-ethyl-*N6*-(1-ethylpropyl)-*N8*-(2-pyridylmethyl)-[1,2,4]triazolo[4,3-*b*]pyridazine-6,8-diamine,
**(60)** *N6*-(1-ethylpropyl)-3-propyl-*N8*-(2-pyridylmethyl)-[1,2,4]triazolo[4,3-*b*]pyridazine-6,8-diamine,
**(61)** 3-tert-butyl-*N6*-(1-ethylpropyl)-*N8*-(2-pyridylmethyl)-[1,2,4]triazolo[4,3-*b*]pyridazine-6,8-diamine,
**(62)** 3-cyclopropyl-*N6*-(1-ethylpropyl)-*N8*-(2-pyridylmethyl)-[1,2,4]triazolo[4,3-*b*]pyridazine-6,8-diamine,
**(63)** 3-cyclobutyl-*N6*-(1-ethylpropyl)-*N8*-(2-pyridylmethyl)-[1,2,4]triazolo[4,3-*b*]pyridazine-6,8-diamine,
**(64)** 3-cyclopentyl-*N6*-(1-ethylpropyl)-*N8*-(2-pyridylmethyl)-[1,2,4]triazolo[4,3-*b*]pyridazine-6,8-diamine,
**(65)** 3-cyclohexyl-*N6*-(1-ethylpropyl)-*N8*-(2-pyridylmethyl)-[1,2,4]triazolo[4,3-*b*]pyridazine-6,8-diamine,
**(66)** *N6*-(1-ethylpropyl)-3-phenyl-*N8*-(2-pyridylmethyl)-[1,2,4]triazolo[4,3-*b*]pyridazine-6,8-diamine,
**(67)** *N6*-(1-ethylpropyl)-*N8*-(2-pyridylmethyl)-3-sec-butyl-[1,2,4]triazolo[4,3-*b*]pyridazine-6,8-diamine,
**(68)** *N6*,3-bis(1-ethylpropyl)-*N8*-(2-pyridylmethyl)-[1,2,4]triazolo[4,3-*b*]pyridazine-6,8-diamine,
**(69)** *N6*-(1-ethylpropyl)-3-isobutyl-*N8*-(2-pyridylmethyl)-[1,2,4]triazolo[4,3-*b*]pyridazine-6,8-diamine,
**(70)** *N6*-(1-ethylpropyl)-3-(2-pyridyl)-*N8*-(2-pyridylmethyl)-[1,2,4]triazolo[4,3-*b*]pyridazine-6, 8-diamine,
**(71)** *N6*-(1-ethylpropyl)-3-(3-pyridyl)-*N8*-(2-pyridylmethyl)-[1,2,4]triazolo[4,3-*b*]pyridazine-6,8-diamine,
**(72)** *N6*-(1-ethylpropyl)-3-(4-pyridyl)-*N8*-(2-pyridylmethyl)-[1,2,4]triazolo[4,3-*b*]pyridazine-6,8-diamine,
**(73)** *N6*-(1-ethylpropyl)-3-isopropyl-*N8*-[(2-methoxyphenyl)methyl]-[1,2,4]triazolo[4,3-*b*]pyridazine-6,8-diamine,
**(74)** (*2R*)-2-[[8-[(4-aminophenyl)methylamino]-3-isopropyl-[1,2,4]triazolo[4,3-*b*]pyridazin-6-yl]amino]butan-1-ol,
**(75)** *N8*-[(4-aminophenyl)methyl]-*N6*-(1-ethylpropyl)-3-isopropyl-[1,2,4]triazolo[4,3-*b*]pyridazine-6,8-diamine,
**(76)** *N8*-[(3-aminophenyl)methyl]-*N6*-(1-ethylpropyl)-3-isopropyl-[1,2,4]triazolo[4,3-*b*]pyridazine-6,8-diamine,
**(77)** *N8*-[(2-aminophenyl)methyl]-*N6*-(1-ethylpropyl)-3-isopropyl-[1,2,4]triazolo[4,3-*b*]pyridazine-6,8-diamine,
**(78)** *N6*-(1-ethylpropyl)-*N8*-(*1H*-indol-2-ylmethyl)-3-isopropyl-[1,2,4]triazolo[4,3-*b*]pyridazine-6,8-diamine,
**(79)** *N*-(1-ethylpropyl)-8-isoindolin-2-yl-3-isopropyl-[1,2,4]triazolo[4,3-*b*]pyridazin-6-amine,
**(80)** 8-(3,4-dihydro-1*H*-isoquinolin-2-yl)-*N*-(1-ethylpropyl)-3-isopropyl-[1,2,4]triazolo[4,3-*b*]pyridazin-6-amine,
**(81)** *N6*-(1-ethylpropyl)-*N8*-indan-1-yl-3-isopropyl-[1,2,4]triazolo[4,3-*b*]pyridazine-6,8-diamine,
**(82)** *N6*-(1-ethylpropyl)-3-isopropyl-*N8*-(2-phenylethyl)-[1,2,4]triazolo[4,3-*b*]pyridazine-6,8-diamine,
**(83)** *N6*-(1-ethylpropyl)-3-isopropyl-*N8*-[2-(2-pyridyl)ethyl]-[1,2,4]triazolo[4,3-*b*]pyridazine-6,8-diamine,
**(84)** *N6*-(1-ethylpropyl)-3-isopropyl-*N8*-[2-(3-pyridyl)ethyl]-[1,2,4]triazolo[4,3-*b*]pyridazine-6,8-diamine,
**(85)** *N6*-(1-ethylpropyl)-3-isopropyl-*N8*-[2-(4-pyridyl)ethyl]-[1,2,4]triazolo[4,3-*b*]pyridazine-6,8-diamine,
**(86)** 3-cyclopropyl-*N6*-(1-ethylpropyl)-*N8*-[2-(2-pyridyl)ethyl]-[1,2,4]triazolo[4,3-*b*]pyridazine-6,8-diamine,
**(87)** 3-cyclopropyl-*N6*-(1-ethylpropyl)-*N8*-[2-(3-pyridyl)ethyl]-[1,2,4]triazolo[4,3-*b*]pyridazine-6,8-diamine,
**(88)** 3-cyclopropyl-*N6*-(1-ethylpropyl)-*N8*-[2-(4-pyridyl)ethyl]-[1,2,4]triazolo[4,3-*b*]pyridazine-6,8-diamine,
**(89)** *N6*-(1-ethylpropyl)-3-isopropyl-*N8*-[3-(2-pyridyl)propyl]-[1,2,4]triazolo[4,3-*b*]pyridazine-6,8-diamine,
**(90)** *N6*-(1-ethylpropyl)-3-isopropyl-*N8*-[3-(3-pyridyl)propyl]-[1,2,4]triazolo[4,3-*b*]pyridazine-6,8-diamine,
**(91)** *N6*-(1-ethylpropyl)-3-isopropyl-*N8-*[3-(4-pyridyl)propyl]-[1,2,4]triazolo[4,3-*b*]pyridazine-6,8-diamine
**(92)** *N6*-(1-ethylpropyl)-3-isopropyl-*N8*-(pyrimidin-2-ylmethyl)-[1,2,4]triazolo[4,3-*b*]pyridazine-6,8-diamine,
**(93)** *N6*-(1-ethylpropyl)-3-isopropyl-*N8*-(pyrimidin-5-ylmethyl)-[1,2,4]triazolo[4,3-*b*]pyridazine-6,8-diamine,
**(94)** *N6*-(1-ethylpropyl)-3-isopropyl-*N8*-(pyrazin-2-ylmethyl)-[1,2,4]triazolo [4,3-*b*]pyridazine-6,8-diamine,
**(95)** *N6*-(1-ethylpropyl)-3-isopropyl-*N8*-(pyridazin-3-ylmethyl)-[1,2,4]triazolo[4,3-*b*]pyridazine-6,8-diamine,
**(96)** *N*-[6-(1-ethylpropylamino)-3-isopropyl-[1,2,4]triazolo[4,3-*b*]pyridazin-8-yl]benzamide,
**(97)** *N*-[6-(1-ethylpropylamino)-3-isopropyl-[1,2,4]triazolo[4,3-*b*]pyridazin-8-yl]pyridine-2-carboxamide,
**(98)** *N*-[6-(1-ethylpropylamino)-3-isopropyl-[[1,2,4]triazolo[4,3-*b*]pyridazin-8-yl]-2-phenyl-acetamide,
**(99)** ethyl *N*-[6-(1-ethylpropylamino)-3-isopropyl-[1,2,4]triazolo[4,3-*b*]pyridazin-8-yl]carbamate,
**(100)** phenyl *N*-[6-(1-ethylpropylamino)-3-isopropyl-[1,2,4]triazolo[4,3-*b*]pyridazin-8-yl]carbamate,
**(101)** (2*S*)-2-[[8-[(4-bromophenyl)methylamino]-3-isopropyl-[1,2,4]triazolo[4,3-*b*]pyridazin-6-yl]amino]butan-1-ol,
**(102)** (2*S*)-2-[[3-isopropyl-8-[(4-phenylphenyl)methylamino]-[1,2,4]triazolo[4,3-*b*]pyridazin-6-yl] amino]butan-1-ol,
**(103)** (2*S*)-2-[[3-isopropyl-8-[[4-(4-pyridyl)phenyl]methylamino]-[1,2,4]triazolo[4,3-*b*]pyridazin-6-yl] amino]butan-1-ol,
**(104)** (2*S*)-2-[[3-isopropyl-8-[[4-(3-pyridyl)phenyl]methylamino]-[1,2,4]triazolo[4,3-*b*]pyridazin-6-yl] amino]butan-1-ol,
**(105)** (2*S*)-2-[[3-isopropyl-8-[[4-(2-pyridyl)phenyl]methylamino]-[1,2,4]triazolo[4,3-*b*]pyridazin-6-yl] amino]butan-1-ol,
**(106)** (2*R*,3*R*)-2-[[8-[(4-bromophenyl)methylamino]-3-isopropyl-[1,2,4]triazolo[4,3-*b*]pyridazin-6-yl]amino]butane-1,3-diol,
**(107)** (2*R*,3*R*)-2-[[3-isopropyl-8-[[4-(2-pyridyl)phenyl]methylamino]-[1,2,4]triazolo[4,3-*b*]pyridazin-6-yl]amino]butane-1,3-diol,
(108) (2*R*,3*R*)-2-[[3-isopropyl-8-[[4-(4-pyridyl)phenyl]methylamino]-[1,2,4]triazolo[4,3-*b*]pyridazin-6-yl]amino]butane-1,3-diol,
(109) *N6*,*N8*-bis(3-methoxypropyl)-3-phenyl-[1,2,4]triazolo[4,3-*b*]pyridazine-6,8-diamine,
(110) 3-cyclopropyl-*N6*,*N8*-bis(3-methoxypropyl)-[1,2,4]triazolo[4,3-*b*]pyridazine-6,8-diamine,
**(111)** *N6*,*N8*-bis(3-methoxypropyl)-3-(trifluoromethyl)-[1,2,4]triazolo[4,3-*b*]pyridazine-6,8-diamine,
**(112)** 3-isopropyl-*N6*,*N8*-bis(2-methoxyethyl)-[1,2,4]triazolo[4,3-*b*]pyridazine-6,8-diamine,
**(113)** (2*R*)-2-[[6-[[(1*R*)-1-(hydroxymethyl)propyl]amino]-3-isopropyl-[1,2,4]triazolo[4,3-*b*]pyridazin-8-yl]amino]butan-1-ol,
**(114)** *N6,N8*-bis(1-ethylpropyl)-3-isopropyl-[1,2,4]triazolo[4,3-*b*]pyridazine-6,8-diamine,
**(115)** (2*R*)-2-[[8-(benzylamino)-3-isopropyl-7-methyl-[1,2,4]triazolo[4,3-*b*]pyridazin-6-yl]amino-ol,
**(116)** *N8*-[(4-bromophenyl)methyl]-3-isopropyl-*N6*-methyl-[1,2,4]triazolo[4,3-*b*]pyridazine-6,8-diamine,
**(117)** *N8*-benzyl-*N6*-cyclobutyl-3-isopropyl-[1,2,4]triazolo[4,3-*b*]pyridazine-6,8-diamine,
**(118)** *N8*-benzyl-*N6*-cyclopentyl-3-isopropyl-[1,2,4]triazolo[4,3-*b*]pyridazine-6,8-diamine,
**(119)** *N8*-benzyl-*N6*-cyclohexyl-3-isopropyl-[1,2,4]triazolo[4,3-*b*]pyridazine-6,8-diamine,
**(120)** *N8*-benzyl-3-isopropyl-*N6*-(4-methoxycyclohexyl)-[1,2,4]triazolo[4,3-*b*]pyridazine-6,8-diamine,
**(121)** *N8*-benzyl-*N6*-cycloheptyl-3-isopropyl-[1,2,4]triazolo[4,3-*b*]pyridazine-6,8-diamine,
**(122)** *N8*-benzyl-*N6*-(cyclopropylmethyl)-3-isopropyl-[1,2,4]triazolo[4,3-*b*]pyridazine-6,8-diamine,
**(123)** *N8*-benzyl-*N6*-(cyclobutylmethyl)-3-isopropyl-[1,2,4]triazolo[4,3-*b*]pyridazine-6,8-diamine,
**(124)** *N8*-benzyl-*N6*-(cyclohexylmethyl)-3-isopropyl-[1,2,4]triazolo[4,3-*b*]pyridazine-6,8-diamine,
**(125)** *N8*-benzyl-3-isopropyl-*N6*-(tetrahydropyran-4-ylmethyl)-[1,2,4]triazolo[4,3-*b*]pyridazine-6,8-diamine,
**(126)** *N8*-benzyl-*N6*-(2-ethylbutyl)-3-isopropyl-[1,2,4]triazolo[4,3-*b*]pyridazine-6,8-diamine,
**(127)** *N8*-benzyl-3-isopropyl-*N6*-(2-phenylethyl)-[1,2,4]triazolo[4,3-*b*]pyridazine-6,8-diamine,
**(128)** *N8*-benzyl-3-isopropyl-*N6*-(2-phenoxyethyl)-[1,2,4]triazolo[4,3-*b*]pyridazine-6,8-diamine,
**(129)** *N8*-benzyl-3-isopropyl-*N6-*[(1-methylimidazol-2-yl)methyl]-[1,2,4]triazolo[4,3-*b*]pyridazine-6,8-diamine,
**(130)** *N6*,*N8*-dibenzyl-3-isopropyl-[1,2,4]triazolo[4,3-*b*]pyridazine-6,8-diamine,
**(131)** *N8*-benzyl-3-isopropyl-*N6*-(2-pyridylmethyl)-[1,2,4]triazolo[4,3-*b*]pyridazine-6,8-diamine,
**(132)** *N8*-benzyl-3-isopropyl-*N6*-(3-pyridylmethyl)-[1,2,4]triazolo[4,3-*b*]pyridazine-6,8-diamine,
**(133)** *N8*-benzyl-3-isopropyl-*N6*-(4-pyridylmethyl)-[1,2,4]triazolo[4,3-*b*]pyridazine-6,8-diamine,
**(134)** racemic trans-2-[[8-(benzylamino)-3-isopropyl-[1,2,4]triazolo[4,3-*b*]pyridazin-6-yl]amino]cyclohexanol,
**(135)** racemic trans-4-[[8-(benzylamino)-3-isopropyl-[1,2,4]triazolo[4,3-*b*]pyridazin-6-yl]amino]cyclohexanol,
**(136)** *N8-*benzyl-3-isopropyl-*N6*-[(1-methylcyclohexyl)methyl]-[1,2,4]triazolo[4,3-*b*]pyridazine-6,8-diamine,
**(137)** *N6*-(1-adamantyl)-*N8*-benzyl-3-isopropyl-[1,2,4]triazolo[4,3-*b*]pyridazine-6,8-diamine,
**(138)** *N8*-benzyl-*N6*-indan-2-yl-3-isopropyl-[1,2,4]triazolo[4,3-*b*]pyridazine-6,8-diamine,
**(139)** *N8*-benzyl-3-isopropyl-*N6*-[(5-methylpyrazin-2-yl)methyl]-[1,2,4]triazolo[4,3-*b*]pyridazine-6,8-diamine,
**(140)** *N8*-benzyl-3-isopropyl-*N6-*[(1-methylpyrazol-4-yl)methyl]-[1,2,4]triazolo[4,3-*b*]pyridazine-6,8-diamine,
**(141)** *N8*-benzyl-*N6*-[(3,5-dimethylphenyl)methyl]-3-isopropyl-[1,2,4]triazolo[4,3-*b*]pyridazine-6,8-diamine,
**(142)** *N8*-benzyl-3-isopropyl-*N6*-(tetrahydrofuran-2-ylmethyl)-[1,2,4]triazolo[4,3-*b*]pyridazine-6,8-diamine,
**(143)** *N8*-benzyl-3-isopropyl-*N6*-(2-methylsulfanylethyl)-[1,2,4]triazolo[4,3-*b*]pyridazine-6,8-diamine,
**(144)** *N8*-benzyl-3-isopropyl-*N6-*(1-methylimidazol-2-yl)-[1,2,4]triazolo[4,3-*b*]pyridazine-6,8-diamine,
**(145)** *N8*-benzyl-*N6*-[(4,6-dimethylpyrimidin-2-yl)methyl]-3-isopropyl-[1,2,4]triazolo[4,3-*b*]pyridazine-6,8-diamine,
**(146)** *N8*-benzyl-*N6*-indan-1-yl-3-isopropyl-[1,2,4]triazolo[4,3-*b*]pyridazine-6,8-diamine,
**(147)** *N6*-(2-ethylbutyl)-3-isopropyl-*N8*-(pyridazin-3-yl)-[1,2,4]triazolo[4,3-*b*]pyridazine-6,8-diamine,
**(148)** 3-isopropyl-*N6*-(pentan-3-yl)-*N8*-(pyridazin-3-yl)-[1,2,4]triazolo[4,3-*b*]pyridazine-6,8-diamine,
**(149)** 3-isopropyl-*N6*-(pentan-3-yl)-*N8*-(pyridazin-4-yl)-[1,2,4]triazolo[4,3-*b*]pyridazine-6,8-diamine,
**(150)** 3-isopropyl-*N6*-(pentan-3-yl)-*N8-*(pyrimidin-4-yl)-[1,2,4]triazolo[4,3-*b*]pyridazine-6,8-diamine,
**(151)** 3-isopropyl-*N6*-(pentan-3-yl)-*N8*-(pyrimidin-5-yl)-[1,2,4]triazolo[4,3-*b*]pyridazine-6,8-diamine,
**(152)** 3-isopropyl-*N6*-(pentan-3-yl)-*N8*-(pyrimidin-2-yl)-[1,2,4]triazolo[4,3-*b*]pyridazine-6,8-diamine,
**(153)** 3-isopropyl-*N6*-(pentan-3-yl)-*N8*-(pyrazin-2-yl)-[1,2,4]triazolo[4,3-*b*]pyridazine-6,8-diamine,
**(154)** 3-isopropyl-*N8*-(6-methoxypyridin-2-yl)-*N6*-(pentan-3-yl)-[1,2,4]triazolo[4,3-*b*]pyridazine-6,8-diamine,
**(155)** 3-isopropyl-*N8*-(5-methoxypyridin-2-yl)-*N6*-(pentan-3-yl)-[1,2,4]triazolo[4,3-*b*]pyridazine-6,8-diamine,
**(156)** 3-isopropyl-*N8*-(4-methoxypyridin-2-yl)-*N6*-(pentan-3-yl)-[1,2,4]triazolo[4,3-*b*]pyridazine-6,8-diamine,
**(157)** 3-isopropyl-*N8*-(3-methoxypyridin-2-yl)-*N6*-(pentan-3-yl)-[1,2,4]triazolo[4,3-*b*]pyridazine-6,8-diamine,
**(158)** 3-isopropyl-*N8*-(2-methoxypyridin-3-yl)-*N6*-(pentan-3-yl)-[1,2,4]triazolo[4,3-*b*]pyridazine-6,8-diamine,
**(159)** 3-isopropyl-*N8*-(6-methoxypyridin-3-yl)-*N6*-(pentan-3-yl)-[1,2,4]triazolo[4,3-*b*]pyridazine-6,8-diamine,
**(160)** isopropyl-*N8*-(5-methoxypyridin-3-yl)-*N6-*(pentan-3-yl)-[1,2,4]triazolo[4,3-*b*]pyridazine-6,8-diamine,
**(161)** 3-isopropyl-*N8*-(4-methoxypyridin-3-yl)-*N6*-(pentan-3-yl)-[1,2,4]triazolo[4,3-*b*]pyridazine-6,8-diamine,
**(162)** 3-isopropyl-*N8*-(5-methoxypyridazin-3-yl)-*N6*-(pentan-3-yl)-[1,2,4]triazolo[4,3-*b*]pyridazine-6,8-diamine,
**(163)** (2*R*)-2-[[3-isopropyl-8-(2-pyridylamino)-[1,2,4]triazolo[4,3-*b*]pyridazin-6-yl]amino]butan-1-ol,
**(164)** *N6*-(2-ethylbutyl)-3-isopropyl-*N8*-[2-(2-pyridyl)ethyl]-[1,2,4]triazolo[4,3-*b*]pyridazine-6,8-diamine,
**(165)** *N6*-cyclopentyl-3-isopropyl-*N8*-[2-(2-pyridyl)ethyl]-[1,2,4]triazolo[4,3-*b*]pyridazine-6,8-diamine,
**(166)** *N6*-(2-ethylbutyl)-3-isopropyl-*N8-*[2-(3-pyridyl)ethyl]-[1,2,4]triazolo[4,3-*b*]pyridazine-6,8-diamine,
**(167)** *N6*,*N8*-bis(2-ethylbutyl)-3-isopropyl-[1,2,4]triazolo[4,3-*b*]pyridazine-6,8-diamine
**(168)** *N*-[6-(1-ethylpropylamino)-3-isopropyl-[1,2,4]triazolo[4,3-*b*]pyridazin-8-yl]-2-(3-pyridyl)acetamide,
**(169)** (2*R*)-2-[[3-isopropyl-8-(2-pyridylmethylamino)imidazo[1,2-*b*]pyridazin-6-yl]amino]butan-1-ol,
**(170)** *N6*-(1-ethylpropyl)-3-isopropyl-*N8*-(3-phenylpropyl)-[1,2,4]triazolo[4,3-*b*]pyridazine-6,8-diamine,
**(171)** 3-cyclopropyl-*N6*-(1-ethylpropyl)-*N8*-phenyl-[1,2,4]triazolo[4,3-b]pyridazine-6,8-diamine,
**(172)** 3-cyclopropyl-*N6*-(1-ethylpropyl)-*N8*-(2-pyridyl)-[1,2,4]triazolo[4,3-*b*]pyridazine-6,8-diamine,
**(173)** 3-cyclopropyl-*N6*-(1-ethylpropyl)-*N8*-(3-pyridyl)-[1,2,4]triazolo[4,3-*b*]pyridazine-6,8-diamine,
**(174)** 3-cyclopropyl-*N6*-(1-ethylpropyl)-*N8*-(4-pyridyl)-[1,2,4]triazolo[4,3-*b*]pyridazine-6,8-diamine,
**(175)** 3-cyclopropyl-*N6*-(pentan-3-yl)-*N8*-(pyridazin-3-yl)-[1,2,4]triazolo[4,3-*b*]pyridazine-6,8-diamine,
**(176)** 3-cyclopropyl-*N6*-(pentan-3-yl)-*N8*-(pyridazin-4-yl)-[1,2,4]triazolo[4,3-*b*]pyridazine-6,8-diamine,
**(177)** 3-cyclopropyl-*N6*-(pentan-3-yl)-*N8*-(pyrimidin-4-yl)-[1,2,4]triazolo[4,3-*b*]pyridazine-6,8-diamine,
**(178)** 3-cyclopropyl-*N6*-(1-ethylpropyl)-*N8*-pyrimidin-5-yl-[1,2,4]triazolo[4,3-*b*]pyridazine-6,8-diamine,
**(179)** 3-cyclopropyl-*N6*-(pentan-3-yl)-*N8*-(pyrimidin-2-yl)-[1,2,4]triazolo[4,3-*b*]pyridazine-6,8-diamine,
**(180)** 3-cyclopropyl-*N6*-(pentan-3-yl)-*N8*-(pyrazin-2-yl)-[1,2,4]triazolo[4,3-*b*]pyridazine-6,8-diamine,
**(181)** (*R*)-2-((3-cyclopropyl-8-(pyridazin-3-ylamino)-[1,2,4]triazolo[4,3-*b*]pyridazin-6-yl)amino)butan-1-ol,
**(182)** (*S*)-2-((3-cyclopropyl-8-(pyridazin-3-ylamino)-[1,2,4]triazolo[4,3-*b*]pyridazin-6-yl)amino)butan-1-ol,
**(183)** 3-cyclopropyl-*N8*-(6-methoxypyridin-2-yl)-*N6*-(pentan-3-yl)-[1,2,4]triazolo[4,3-*b*]pyridazine-6,8-diamine,
**(184)** 3-cyclopropyl-*N8*-(2-methoxypyridin-3-yl)-*N6*-(pentan-3-yl)-[1,2,4]triazolo[4,3-*b*]pyridazine-6,8-diamine,
**(185)** (2*R*)-2-[[3-cyclopropyl-8-[2-(2-pyridyl)ethylamino]-[1,2,4]triazolo[4,3-*b*]pyridazin-6-yl]amino]butan-1-ol,
**(186)** *N6*-benzyl-3-cyclopropyl-*N8*-(pyridin-2-ylmethyl)-[1,2,4]triazolo[4,3-*b*]pyridazine-6,8-diamine,
**(187)** 3-cyclopropyl-*N6*-(4-methoxybenzyl)-*N8*-(pyridin-2-ylmethyl)-[1,2,4]triazolo [4,3 -*b*]pyridazine-6,8-diamine,
**(188)** 3-cyclopropyl-*N6*-(4-methoxybenzyl)-*N6-*methyl-*N8*-(pyridin-2-ylmethyl)-[1,2,4]triazolo[4,3-*b*]pyridazine-6,8-diamine,
**(189)** 3-cyclopropyl-*N6*)-ethyl-*N8*-(2-pyridylmethyl)-[1,2,4]triazolo[4,3-*b*]pyridazine-6,8-diamine,
**(190)** 3-cyclopropyl-*N6*-propyl-*N8*-(pyridin-2-ylmethyl)-[1,2,4]triazolo[4,3-*b*]pyridazine-6,8-diamine,
**(191)** *N6*-butyl-3-cyclopropyl-*N8*-(pyridin-2-ylmethyl)-[1,2,4]triazolo[4,3-*b*]pyridazine-6,8-diamine,
**(192)** 3-cyclopropyl-*N6*-isopropyl-*N8-*(pyridin-2-ylmethyl)-[1,2,4]triazolo[4,3-*b*]pyridazine-6,8-diamine,
**(193)** *N6*-(sec-butyl)-3-cyclopropyl-*N8*-(pyridin-2-ylmethyl)-[1,2,4]triazolo[4,3-*b*]pyridazine-6,8-diamine,
**(194)** 3-cyclopropyl-*N6*-isobutyl-*N8*-(pyridin-2-ylmethyl)-[1,2,4]triazolo[4,3-*b*]pyridazine-6,8-diamine,
**(195)** *N6*-(tert-butyl)-3-cyclopropyl-*N8*-(pyridin-2-ylmethyl)-[1,2,4]triazolo[4,3-*b*]pyridazine-6,8-diamine,
**(196)** 3-cyclopropyl-*N6*-(2-ethylbutyl)-*N8*-(pyridin-2-ylmethyl)-[1,2,4]triazolo[4,3-*b*]pyridazine-6,8-diamine,
**(197)** *N6*,3-dicyclopropyl-*N8*-(pyridin-2-ylmethyl)-[1,2,4]triazolo[4,3-*b*]pyridazine-6,8-diamine,
**(198)** *N6*-cyclobutyl-3-cyclopropyl-*N8*-(pyridin-2-ylmethyl)-[1,2,4]triazolo[4,3-*b*]pyridazine-6,8-diamine,
**(199)** *N6*-cyclopentyl-3-cyclopropyl-*N8*-(pyridin-2-ylmethyl)-[1,2,4]triazolo[4,3-*b*]pyridazine-6,8-diamine,
**(200)** *N6*-cyclohexyl-3-cyclopropyl-*N8*-(pyridin-2-ylmethyl)-[1,2,4]triazolo[4,3-*b*]pyridazine-6,8-diamine,
**(201)** *N6*-cycloheptyl-3-cyclopropyl-*N8*-(pyridin-2-ylmethyl)-[1,2,4]triazolo[4,3-*b*]pyridazine-6,8-diamine,
**(202)** *N6*-((3s,5s,7s)-adamantan-1-yl)-3-cyclopropyl-*N8*-(pyridin-2-ylmethyl)-[1,2,4]triazolo[4,3-*b*]pyridazine-6,8-diamine,
**(203)** 3-cyclopropyl-*N6*-(cyclopropylmethyl)-*N8*-(pyridin-2-ylmethyl)-[1,2,4]triazolo[4,3-*b*]pyridazine-6,8-diamine,
**(204)** *N6*-(cyclobutylmethyl)-3-cyclopropyl-*N8*-(pyridin-2-ylmethyl)-[1,2,4]triazolo[4,3-*b*]pyridazine-6,8-diamine,
**(205)** 3-cyclopropyl-*N8*-(pyridin-2-ylmethyl)-*N6*-(spiro[3.3]heptan-2-yl)-[1,2,4]triazolo[4,3-*b*]pyridazine-6,8-diamine,
**(206)** 3-cyclopropyl-*N6*-((1-methylcyclobutyl)methyl)-*N8*-(pyridin-2-ylmethyl)-[1,2,4]triazolo[4,3-*b*]pyridazine-6,8-diamine,
**(207)** 3-cyclopropyl-6-(1-piperidyl)-*N*-(2-pyridylmethyl)-[1,2,4]triazolo[4,3-*b*]pyridazin-8-amine,
**(208)** 3-cyclopropyl-*N6*-(furan-2-ylmethyl)-*N8*-(pyridin-2-ylmethyl)-[1,2,4]triazolo[4,3-*b*]pyridazine-6,8-diamine,
**(209)** 3-cyclopropyl-*N6*-(furan-3-ylmethyl)-*N8*-(pyridin-2-ylmethyl)-[1,2,4]triazolo[4,3-*b*]pyridazine-6,8-diamine,
**(210)** 3-cyclopropyl-*N8-*(pyridin-2-ylmethyl)-*N6*-(thiophen-2-ylmethyl)-[1,2,4]triazolo[4,3-*b*]pyridazine-6,8-diamine,
**(211)** (*R*)-2-((3-cyclopropyl-8-((pyridin-2-ylmethyl)amino)-[1,2,4]triazolo[4,3-*b*]pyridazin-6-yl)amino)butan-1-ol,
**(212)** (*S*)-2-((3-cyclopropyl-8-((pyridin-2-ylmethyl)amino)-[1,2,4]triazolo[4,3-*b*]pyridazin-6-yl)amino)butan-1-ol,
**(213)** (*R*)-3-cyclopropyl-*N8*-(pyridin-2-ylmethyl)-*N6-*(tetrahydrofuran -3-yl)-[1,2,4]triazolo[4,3-*b*]pyridazine-6,8-diamine,
**(214)** (*R*)-3-cyclopropyl-*N8*-(pyridin-2-ylmethyl)-*N6*-(tetrahydro-2*H*-pyran-3-yl)-[1,2,4]triazolo[4,3-*b*]pyridazine-6,8-diamine,
**(215)** 3-cyclopropyl-*N8*-(6-methoxypyridazin-3-yl)-*N6*-(pentan-3-yl)-[1,2,4]triazolo[4,3-*b*]pyridazine-6,8-diamine,
**(216)** 3-cyclopropyl-*N6*-(pentan-3-yl)-N8-(pyridazin-3-ylmethyl)-[1,2,4]triazolo[4,3-*b*]pyridazine-6,8-diamine,
**(217)** 3-cyclopropyl-*N6*-(pentan-3-yl)-*N8*-phenethyl-[1,2,4]triazolo[4,3-*b*]pyridazine-6,8-diamine,
**(218)** 3-cyclopropyl-*N8*-(pyridin-2-ylmethyl)-*N6*-(thiophen-3-ylmethyl)-[1,2,4]triazolo[4,3-*b*]pyridazine-6,8-diamine,
**(219)** 3-cyclopropyl-*N6*-methyl-*N8*-(pyridin-2-ylmethyl)-[1,2,4]triazolo[4,3-*b*]pyridazine-6,8-diamine,
**(220)** 3-cyclopropyl-*N*-(pyridin-2-ylmethyl)-6-(pyrrolidin-1-yl)-[1,2,4]triazolo[4,3-*b*]pyridazin-8-amine,
and their pharmaceutically acceptable salts.

Thus, herein is provided anyone of the compounds (1) to (220) as defined above or any of their pharmaceutically acceptable salts.

According to an even more preferred embodiment of the present invention, the compound of formula (I) is chosen from the group consisting of compounds (1), (2), (10), (13), (14), (17) to (19), (21) to (23), (26), (28), (29), (31), (35) to (56), (58) to (64), (66) to (69), (71) to (78), (80) to (105), (107), (110), (111), (114), (117) to (119), (121) to (124), (126), (127), (130) to (132), (134), (136), (138), (140), (143), (146) to (160), (162) to (187), (189) to (206), (208) to (212) and (215) to (218), and their pharmaceutically acceptable salts.

According to an even more preferred embodiment of the present invention, the compound of formula (I) is chosen from the group consisting of compounds(18), (38), (40) to (42), (45) to (53), (56), (59), (62), (63), (67), (68), (73), (75), (76), (80), (82) to (88), (90), (91), (93) to (95), (98) to (100), (114), (118), (126), (130), (147) to (151), (153) to (156), (158) to (160), (162), (164) to (168), (170) to (178), (180) to (183), (186), (187), (191) to (193), (195), (196), (198) to (202), (204) to (206), (208) to (210) and (215) to (218), and their pharmaceutically acceptable salts.

According to an even more particularly preferred embodiment of the present invention, the compound of formula (I) is chosen from the group consisting of compounds (46), (47), (83), (86), (87), (98), (147), (148), (150), (151), (153) to (155), (158), (159), (162), (164), (168), (171) to (175), (177), (178), (180) to (183) and (215), and their pharmaceutically acceptable salts.

According to another aspect, a subject-matter of the present invention relates to a compound of formula (I) as defined above or any of its pharmaceutically acceptable salts, or at least any of compounds (1) to (220) or any of their pharmaceutically acceptable salts, for use as a medicament.

"Pharmaceutically acceptable salt thereof' refers to salts which are formed from acid addition salts formed with inorganic acids (*e.g*. hydrochloric acid, hydrobromic acid,), as well as salts formed with organic acids such as acetic acid, tartaric acid, succinic acid.

Suitable physiologically acceptable acid addition salts of compounds of formula (I) include hydrobromide, tartrate, hydrochloride, mesylate, succinate and acetate.

The compounds of formula (I), and any of compounds (1) to (220) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, *i.e.* combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

**List of abbreviations:**

| **Abbreviation/acronym** | **Name** |
|---|---|
| **A₁AR** | Adenosine receptor A₁ |
| **A_{2A}AR** | Adenosine receptor A_{2A} |
| **A_{2B}AR** | Adenosine receptor A_{2B} |
| **A₃AR** | Adenosine receptor A₃ |
| **ACN** | Acetonitrile |
| **AcOH** | Acetic acid |
| **Alk** | Alkyl |
| **ATP** | Adenosine triphosphate |
| **br s** | Broad singlet |
| **nBuOH** | Butan-1-ol |
| **C** | Molar concentration |
| **cHex** | Cyclohexane |
| **Cpd N°** | Compound number |
| **d** | Doublet |
| **DCM** | Methylene chloride |
| **dd** | Doublet of doublets |
| **DIPEA** | *N*,*N*-Diisopropylethylamine |
| **DMF** | Dimethylformamide |
| **DMSO** | Dimethylsulfoxide |
| **eq** | Equivalent |
| **ESI** | Electrospray ionization |
| **Et** | Ethyl |
| **EtOAc** | Ethyl Acetate |
| **GP** | General protocol |
| **Hal** | Halogen |
| **HPLC** | High pressure liquid chromatography |
| **IC₅₀** | Half maximal inhibitory concentration |
| **iPr** | isopropyl |
| **iPrOH** | Isopropanol |
| **KHMDS** | Potassium bis(trimethylsilyl)amide |
| **m** | Multiplet |
| **M** | Molarity |
| **Me** | Methyl |
| **2MePrOH** | 2-Methylpropan-1-ol |
| **MS** | Mass spectroscopy |
| **MW** | Molecular weight |
| **NBS** | N-Bromosuccinimide |
| **NMP** | N-Methyl-2-pyrrolidone |
| **NMR** | Nuclear magnetic resonance |
| **N/A** | Not applicable |
| **n.t.** | Not tested |
| **Phe** | phenyl |
| **PIDA** | phenyliodine(III) diacetate |
| **nPrOH** | Propan-1-ol |
| **PTLC** | Preparative thin layer chromatography |
| **PTSA.H₂O or APTS.H₂O** | p-Toluenesulfonic acid monohydrate |
| **Rac** | Racemic |
| **r.t.** | Room temperature |
| **s** | Singlet |
| **t** | Triplet |
| **TEA** | Triethylamine |
| **TFA** | Trifluoroacetic acid |
| **THF** | Tetrahydrofuran |
| **TLC** | Thin layer chromatography |
| **Ts** | Tosyl |
| **T °C** | Temperature in degrees Celsius |
| **UV** | Ultraviolet |
| **v/v** | Volume per volume |
| **w/v** | Weight per volume |
| **Δ** | chemical shift |
| **µw** | Microwave irradiation |

The compounds of general formula (I) can be prepared according to **Route 1** depicted in scheme 1 below.

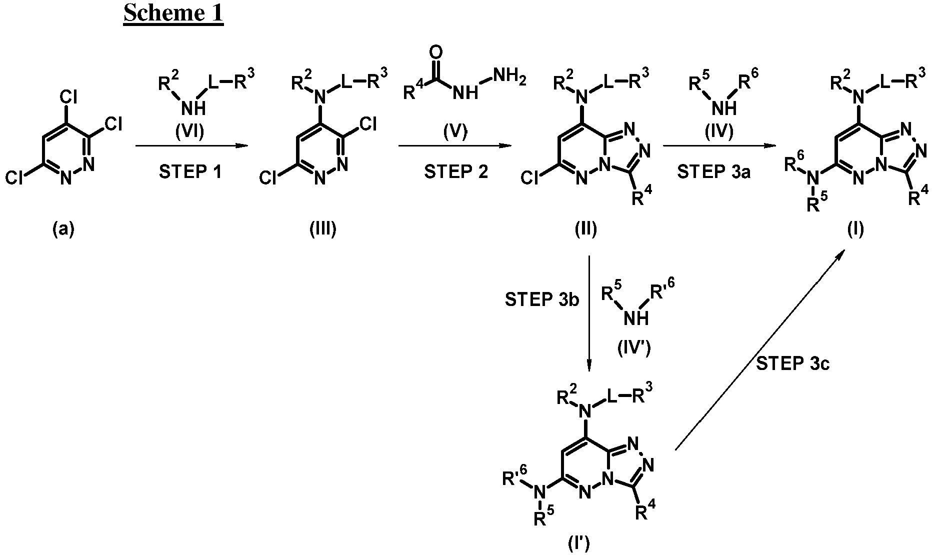

According to Route 1, the synthesis of compounds according to the invention is based on a functionalization of a compound of formula (II) wherein L, R², R³ and R⁴ are as defined above by an amine of formula (IV) wherein R⁵ and R⁶ are as defined above. The compound of formula (II) may be placed in STEP 3a in an aprotic solvent such as Et₃N, THF, 2-MeTHF, dioxane, cineol or NMP or a mixture of both or without solvent or else in a protic solvent such as ethylene glycol. The amine of formula (IV) may be added, for example in a molar ratio ranging from 1 to 20 eq, in particular of 10 eq, with respect to the compound of formula (II). The reaction mixture may be heated at a temperature between 150 to 180°C. Upon completion of the reaction, the mixture may be brought back to room temperature.

The compound of formula (II) may be placed in STEP 3b in an aprotic solvent such as Et₃N, THF, 2-MeTHF, dioxane, cineol or NMP or a mixture of both or without solvent or else in a protic solvent such as ethylene glycol. The amine of formula (IV'), wherein R'⁶ comprises a -COOH group, may be added, for example in a molar ratio ranging from 1 to 20 eq, in particular of 10 eq, with respect to the compound of formula (II). The reaction mixture may be heated at a temperature between 150 to 180°C. Upon completion of the reaction, the mixture may be brought back to room temperature.

STEP 3c may be implemented according to known procedures for obtaining the corresponding amide or ester compound of formula (I).

A compound of formula (II) as defined above may be obtained in STEP 2 by reaction of a compound of formula (III) wherein L, R² and R³ are as defined above, in an aprotic solvent such as dioxane. The hydrazide of formula (V) may be added, for example in a molar ratio ranging from 1 to 3 eq, in particular of 1.2 eq, with respect to the compound of formula (III). The resulting mixture may then be stirred, for example from 1 to 36 hours, in particular for 16 hours at a temperature between 100 °C to 115 °C by refluxing or in a sealed tube.

A compound of formula (III) as defined above may be obtained in STEP 1 by reaction of a compound of formula (a) with a compound of formula (VI) wherein L, R² and R³ are as defined above, in an aprotic solvent such as DCM, THF, 2-MeTHF or dioxane, or a mixture thereof. The reaction mixture may be stirred at room temperature or reflux temperature or in sealed tube with DCM at 50 °C for a duration ranging from 1 to 24 hours, for example for 16 hours.

The compounds of general formula (I) can alternatively be prepared according to **Route 2,** depicted in scheme 2 below.

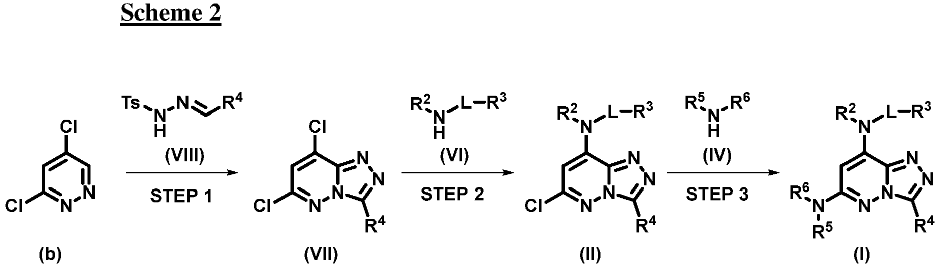

According to Route 2, the synthesis of compounds according to the invention is based on a functionalization of a compound of formula (II) wherein L, R², R³, R⁴ and R⁵ are as defined above by an amine of formula (IV) wherein R⁵ and R⁶ are as defined above. The compound of formula (II) may be placed in STEP 3 in an aprotic solvent such as Et₃N, THF, 2-MeTHF, dioxane, cineol or NMP or a mixture of both or without solvent or else in a protic solvent such as ethylene glycol. The amine of formula (IV) may be added, for example in a molar ratio ranging from 1 to 20 eq, in particular of 10 eq, with respect to the compound of formula (II). The reaction mixture may be heated at a temperature between 150 to 180°C. Upon completion of the reaction, the mixture may be brought back to room temperature.A compound of formula (II) as defined above may be obtained in STEP 2 by reaction of a compound of formula (VII) wherein R⁴ is as defined above by an amine of formula (VI) wherein L, R² and R³ are as defined above, in an aprotic solvent such as DCM, THF or 2MeTHF. The amine of formula (VI) may be added, for example in a molar ratio ranging from 1 to 5 eq, in particular of 1.2 eq, with respect to the compound of formula (VII). The resulting mixture may then be stirred, for example from 1 to 24 hours, in particular for 3 hours at a temperature between room temperature to solvent refluxing.

A compound of formula (VII) as defined above may be obtained in STEP 1 by reaction of a compound of formula (b) with a compound of formula (VIII) wherein R⁴ is as defined above, in an aprotic solvent such as THF, 2-MeTHF or dioxane. The reaction mixture may be stirred at room temperature for a duration ranging from 1 to 24 hours, for example for 16 hours.

The compounds of general formula (I) can alternatively be prepared according to **Routes 3,** depicted in scheme 3 below.

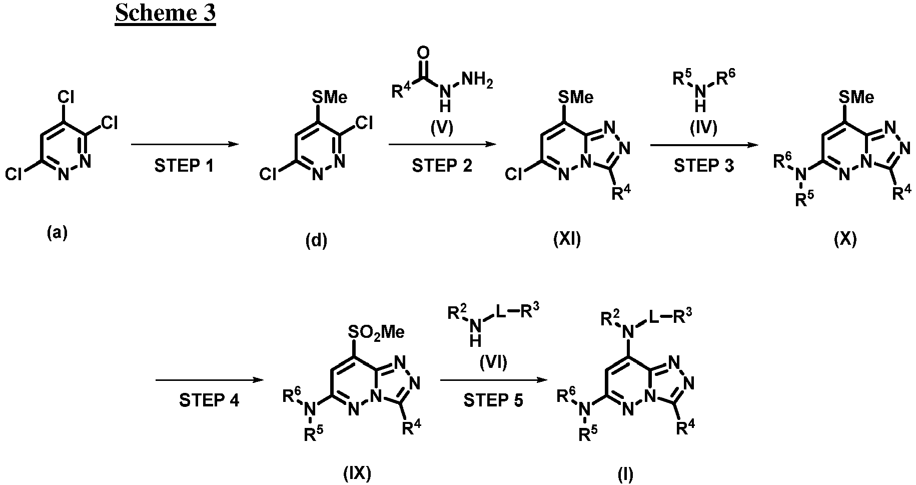

According to Route 3, the synthesis of compounds according to the invention is based on a functionalization of a compound of formula (IX) wherein R⁴, R⁵ and R⁶ are as defined above by an amine of formula (VI) wherein L, R² and R³ are as defined above. The compound of formula (IX) may be placed in STEP 5 in an aprotic solvent such as dioxane. The amine of formula (VI) may be added, for example in a molar ratio ranging from 1 to 10 eq, in particular of 6 eq, with respect to the compound of formula (IX). The reaction mixture may be heated at a temperature between 100 to 130°C, in particular 110°C in a sealed tube. Upon completion of the reaction, the mixture may be brought back to room temperature.

A compound of formula (IX) as defined above may be obtained in STEP 4 by oxydation of a compound of formula (X) wherein R⁴, R⁵ and R⁶ are as defined above, in a mixture of THF and water. The oxidazing reagent may be added, for example in a molar ratio ranging from 2 to 5 eq, in particular of 3.3 eq, with respect to the compound of formula (IX). The resulting mixture may then be stirred, for example from 1 to 24 hours, in particular for 18 hours at room temperature.

A compound of formula (X) as defined above may be obtained in STEP 3 by an amine of formula (V) wherein R¹ and R² are as defined above. The compound of formula (X) may be placed in STEP 3 in an aprotic solvent such as Et₃N, DIPEA, THF, 2-MeTHF, dioxane, cineol or NMP or a mixture of both or without solvent. The amine reagent may be added, for example in a molar ratio ranging from 2 to 20 eq, in particular of 15 eq, with respect to the compound of formula (IX). The resulting mixture may then be stirred, for example from 0.5 to 24 hours, in particular for 18 hours at a temperature between 100 to 150°C, in particular 120°C in a sealed tube.

A compound of formula (XI) as defined above may be obtained in STEP 2 by reaction of a compound of formula (d) with a compound of formula (V) wherein R⁴ is as defined above, in an aprotic solvent such as THF, 2-MeTHF or dioxane. The reaction mixture may be stirred for a duration ranging from 1 to 24 hours, for example for 16 hours at a temperature ranging between 100 to 150°C, in particular 110°C in a sealed tube.

A compound of formula (d) as defined above may be obtained in STEP 2 by reaction of a compound of formula (a) with methylsulfanylsodium in an aprotic solvent such as THF or 2-MeTH. The reaction mixture may be stirred for a duration ranging from 1 to 6 hours, for example for 3 hours at room temperature or solvent refluxing.

The compounds of general formula (I) can alternatively be prepared according to **Routes 4,** depicted in scheme 4 below.

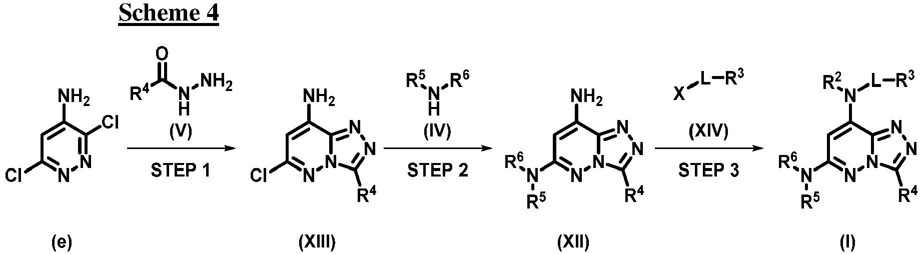

According to Route 4, the synthesis of compounds according to the invention is based on a functionalization of a compound of formula (XII) wherein R⁴, R⁵ and R⁶ are as defined above by a reagent of formula (XIV) wherein Land R³ are as defined above and X represents a hydroxy group, a chlorine atom, a bromine atom, an (C₁-C₃)alkyl sulfonate group or a phenyl sulfonate group . The compound of formula (XII) may be placed in STEP 3 in an aprotic solvent such as THF, 2-MeTHF, dioxane, DMSO or DMF. The reagent of formula (XIV) may be added, for example in a molar ratio ranging from 1 to 10 eq, in particular of 2 eq, with respect to the compound of formula (XII). The reaction mixture may be stirred for a duration ranging from 1 to 24 hours, for example for 3 hours at room temperature or solvent refluxing.

A compound of formula (XII) as defined above may be obtained in STEP 2 by reacting a compound of formula (XIII) wherein R⁴ is as defined above with an amine of formula (IV) wherein R⁵ and R⁶ are as defined above. The compound of formula (XIII) may be placed in STEP 2 in an aprotic solvent such as Et₃N, DIPEA, THF, 2-MeTHF, dioxane, cineol or NMP or a mixture of both or without solvent. The amine reagent may be added, for example in a molar ratio ranging from 2 to 20 eq, in particular of 10 eq, with respect to the compound of formula (XIII). The resulting mixture may then be stirred, for example from 0.5 to 72 hours, in particular for 24 hours at a temperature between 150 to 220 °C, in particular 180°C in a sealed tube or under microwave irradiation system.

A compound of formula (XIII) as defined above may be obtained in STEP 1 by reaction of a compound (e) with a compound of formula (V) wherein R⁴ is as defined above, in an aprotic solvent such as THF, 2-MeTHF or dioxane or an protic solvent such as iPrOH, nPrOH, 2-MePrOH or nBuOH. The reaction mixture may be stirred for a duration ranging from 1 to 24 hours, for example for 18 hours at a temperature ranging between 100 to 150°C, in particular 110°C in a sealed tube or solvent refluxing.

Accordingly, herein is further provided a synthesis process for manufacturing a compound of formula (I) as defined above or any of its pharmaceutically acceptable salts, or at least any of compounds (1) to (220) as defined above or any of their pharmaceutically acceptable salts, comprising at least a step of reacting a compound of formula (II) 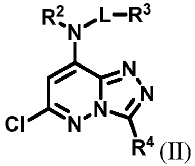
wherein L, R², R³ and R⁴ are as defined above, with an amine of formula (IV)

   NHR⁵R⁶ (IV)
wherein R⁵ and R⁶ are as defined above, for example in a molar ratio ranging from 1 to 20 eq, with respect to the compound of formula (II), in an aprotic solvent or without solvent or else in a protic solvent.

Herein is further provided a synthesis process for manufacturing a compound of formula (I) as defined above or any of its pharmaceutically acceptable salts, or at least any of compounds (1) to (220) as defined above or any of their pharmaceutically acceptable salts, comprising at least a step of reacting a compound of formula (IX) 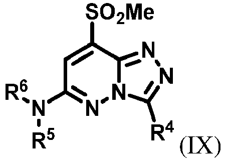 wherein R⁴, R⁵ and R⁶ are as defined above, with a compound of formula (VI) 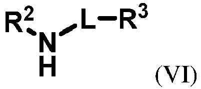 wherein R² and R³ are as defined above, in an aprotic solvent, for example in a molar ratio ranging from 1 to 10 eq, with respect to the compound of formula (IX).

Herein is further provided a synthesis process for manufacturing a compound of formula (I) as defined above or any of its pharmaceutically acceptable salts, or at least any of compounds (1) to (220) as defined above or any of their pharmaceutically acceptable salts, comprising at least a step of reacting a compound of formula (XII) 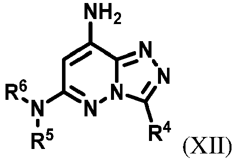 wherein R⁵ and R⁶ are as defined above, with a compound of formula (XIV) 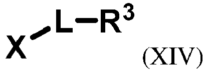 wherein L and R³ are as defined above and X represents a hydroxy group, a chlorine atom, a bromine atom, an (C₁-C₃)alkyl sulfonate group or a phenyl sulfonate group, in an aprotic solvent for example in a molar ratio ranging from 1 to 10 eq, with respect to the compound of formula (XII).

Herein is further provided a compound of formula (II), (X), (XI) or (VII), in particular as an intermediate compound 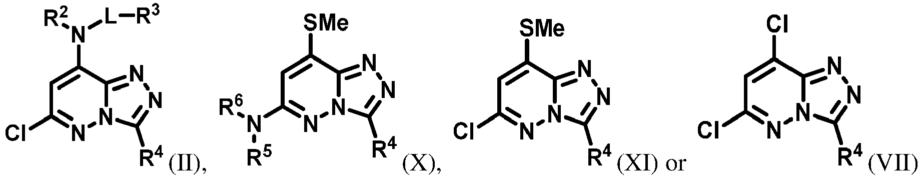 wherein R⁴, R⁵ and R⁶ are as defined above, provided that R⁴ is not a methyl group in formula (XI).

The intermediate products (a), (b) and (e) may be obtained commercially or according to methods known to the man skilled in the art.

The chemical structures, the analytical and spectroscopic data of some compounds of formula (I) of the invention are illustrated respectively in the following **Table 1** and **Table 2.**

Reactions were performed using glassware or oven-dried glassware under inert atmosphere of argon or not. Unless otherwise noted, all reagent-grade chemicals and solvents were obtained from commercial suppliers and were used as received. Reactions were monitored by thin-layer chromatography with silica gel 60 F254 pre-coated aluminum plates (0.25 mm). Visualization was performed under UV light and 254 or 365 nm, or with appropriate TLC stains including, but not limited to: phosphomolybdic acid, KMnO₄, ninhydrin, CAM, vanillin, *p*-anisaldehyde.

Chromatographic purifications of compounds were achieved on an automated Interchim Puriflash XS420 equipped with 30 µm spherical silica-filled prepacked columns as stationary phase (normal phase) or with C18 silica prepacked columns as stationary phase (reversed phase). Nevertheless, a second purification could be achieved with preparative thin layer chromatography with standard silica. Purifications work with various solvent as pur or mixed such as ethyl acetate, cyclohexane, methanol, methanol with ammonia 7N, dichloromethane, triethylamine and tetrahydrofuran on a normal phase then ACN, MeOH, H₂O and NH₄OH on a reversed phase.

Mixtures possibilities: cHex/EtOAc, cHex/DCM, cHex/DCM/EtOAc, DCM/EtOAc, DCM/MeOH, DCM/MeOH NH₃ 7N, DCM/MeOH/Et₃N, DCM/MeOH/THF, EtOAc/MeOH, EtOAc/MeOH NH₃ 7N, EtOAc/THF, ACN/H₂O, ACN/NH₄OH, MeOH/H₂O, MeOH/NH₄OH.

Some compounds of the invention are described with their structure in the below **Table 1,** which is merely illustrative and does not limit the scope of the present invention.

**Table 1: Structure of compounds (1) to (220). Formulas and molecular weights were generated using Perkin Elmer's ChemDraw^{®} Professional v22.0.022.**

| 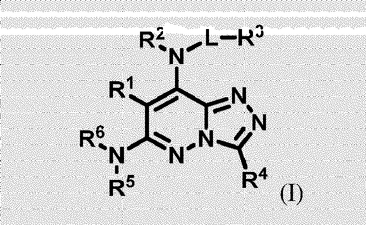 | | | | | | | |
|---|---|---|---|---|---|---|---|
| **Cpd N°** | **NR²LR³** | **R4** | **NR⁵R⁶** | **R¹** | **Formula** | **MW** | **Stereo-chemistry** |
| **(1)** | 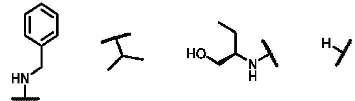 | | | | C₁₉H₂₆N₆O | 354.46 | (R) |
| **(2)** | 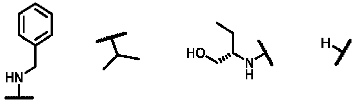 | | | | C₁₉H₂₆N₆O | 354.46 | (S) |
| **(3)** | 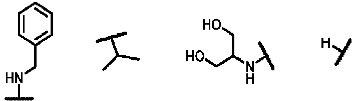 | | | | C₁₈H₂₄N₆O₂ | 356.43 | - |
| **(4)** | 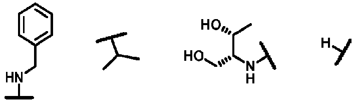 | | | | C₁₉H₂₆N₆O₂ | 370.46 | (2R,3R) |
| **(5)** | 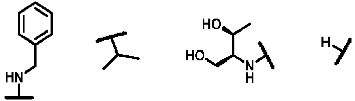 | | | | C₁₉H₂₆N₆O₂ | 370.46 | (2S,3S) |
| **(6)** | 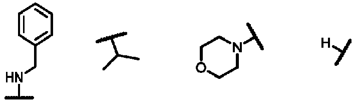 | | | | C₁₉H₂₄N₆O | 352.44 | - |
| **(7)** | 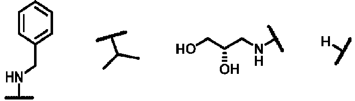 | | | | C₁₈H₂₄N₆O₂ | 356.43 | (S) |
| **(8)** | 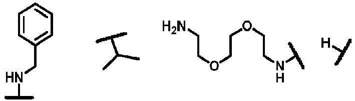 | | | | C₂₁H₃₁N₇O₂ | 413.53 | - |
| **(9)** | 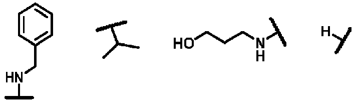 | | | | C₁₈H₂₄N₆O | 340.43 | - |
| **(10)** | 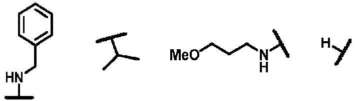 | | | | C₁₉H₂₆N₆O | 354.46 | - |
| **(11)** | 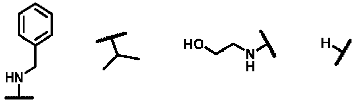 | | | | C₁₇H₂₂N₆O | 326.40 | - |
| **(12)** | 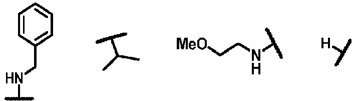 | | | | C₁₈H₂₄N₆O | 340.43 | - |
| **(13)** | 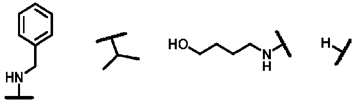 | | | | C₁₉H₂₆N₆O | 354.46 | - |
| **(14)** | 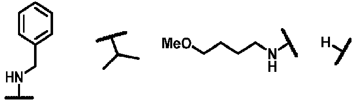 | | | | C₂₀H₂₈N₆O | 368.49 | - |
| **(15)** | 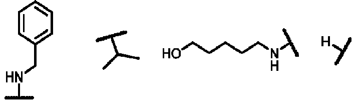 | | | | C₂₀H₂₈N₆O | 368.49 | - |
| **(16)** | 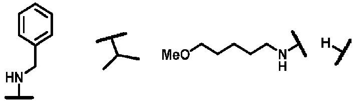 | | | | C₂₁H₃₀N₆O | 382.51 | - |
| **(17)** | 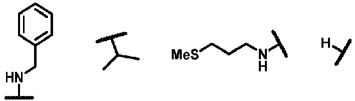 | | | | C₁₉H₂₆N₆S | 370.52 | - |
| **(18)** | 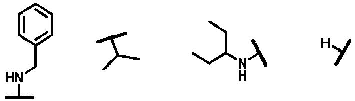 | | | | C₂₀H₂₈N₆ | 352.49 | - |
| **(19)** | 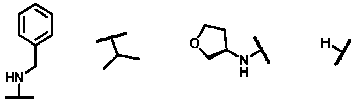 | | | | C₁₉H₂₄N₆O | 352.44 | (R) |
| **(20)** | 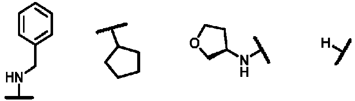 | | | | C₂₁H₂₆N₆O | 378.48 | (R) |
| **(21)** | 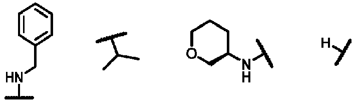 | | | | C₂₀H₂₆N₆O | 366.47 | (R) |
| **(22)** | 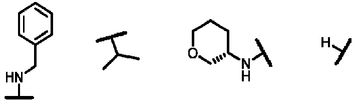 | | | | C₂₀H₂₆N₆O | 366.47 | (S) |
| **(23)** | 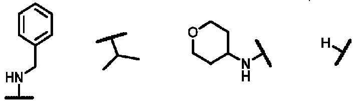 | | | | C₂₀H₂₆N₆O | 366.47 | - |
| **(24)** | 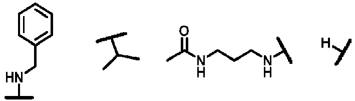 | | | | C₂₀H₂₇N₇O | 381.48 | - |
| **(25)** | 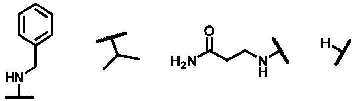 | | | | C₁₈H₂₃N₇O | 353.43 | - |
| **(26)** | 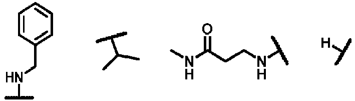 | | | | C₁₉H₂₅N₇O | 367.46 | - |
| **(27)** | 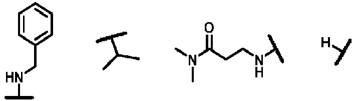 | | | | C₂₀H₂₇N₇O | 381.48 | - |
| **(28)** | 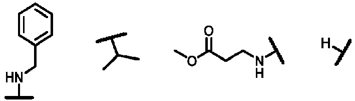 | | | | C₁₉H₂₄N₆O₂ | 368.44 | - |
| **(29)** | 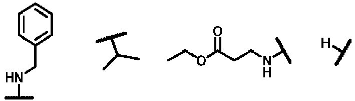 | | | | C₂₀H₂₆N₆O₂ | 382.47 | - |
| **(30)** | 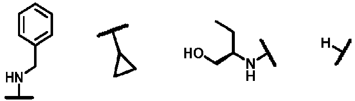 | | | | C₁₉H₂₄N₆O | 352.44 | (R) |
| **(31)** | 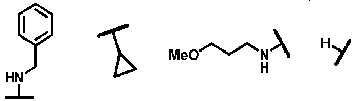 | | | | C₁₉H₂₄N₆O | 352.44 | - |
| **(32)** | 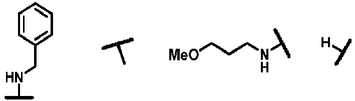 | | | | C₁₇H₂₂N₆O | 326.40 | - |
| **(33)** | 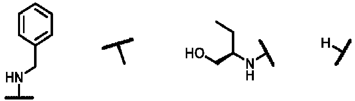 | | | | C₁₇H₂₂N₆O | 326.40 | (R) |
| **(34)** | 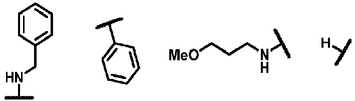 | | | | C₂₂H₂₄N₆O | 388.48 | - |
| **(35)** | 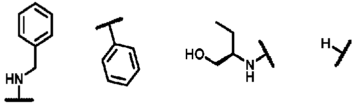 | | | | C₂₂H₂₄N₆O | 388.48 | (R) |
| **(36)** | 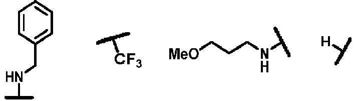 | | | | C₁₇H₁₉F₃N₆O | 380.38 | - |
| **(37)** | 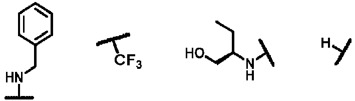 | | | | C₁₇H₁₉F₃N₆O | 380.38 | (R) |
| **(38)** | 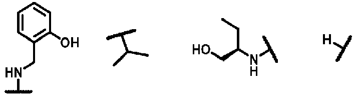 | | | | C₁₉H₂₆N₆O₂ | 370.46 | (R) |
| **(39)** | 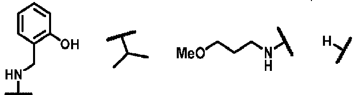 | | | | C₁₉H₂₆N₆O₂ | 370.46 | - |
| **(40)** | 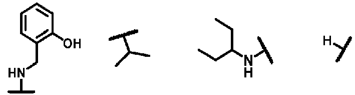 | | | | C₂₀H₂₈N₆O | 368.49 | - |
| **(41)** | 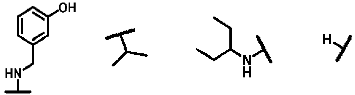 | | | | C₂₀H₂₈N₆O | 368.49 | - |
| **(42)** | 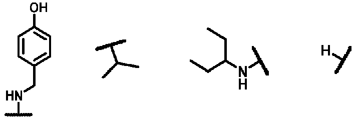 | | | | C₂₀H₂₈N₆O | 368.49 | - |
| **(43)** | 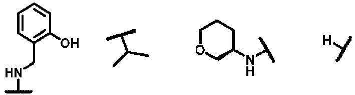 | | | | C₂₀H₂₆N₆O₂ | 382.47 | (R) |
| **(44)** | 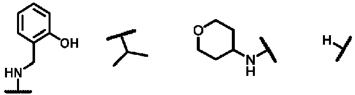 | | | | C₂₀H₂₆N₆O₂ | 382.47 | - |
| **(45)** | 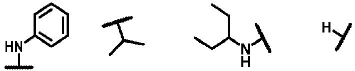 | | | | C₁₉H₂₆N₆ | 338.46 | - |
| **(46)** | 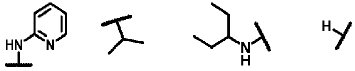 | | | | C₁₈H₂₅N₇ | 339.45 | - |
| **(47)** | 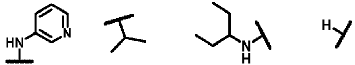 | | | | C₁₈H₂₅N₇ | 339.45 | - |
| **(48)** | 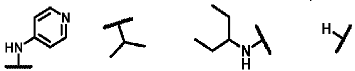 | | | | C₁₈H₂₅N₇ | 339.45 | - |
| **(49)** | 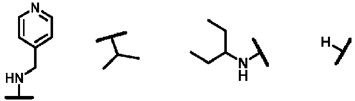 | | | | C₁₉H₂₇N₇ | 353.47 | - |
| **(50)** | 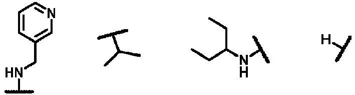 | | | | C₁₉H₂₇N₇ | 353.47 | - |
| **(51)** | 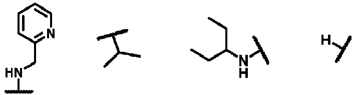 | | | | C₁₉H₂₇N₇ | 353.47 | - |
| **(52)** | 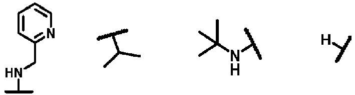 | | | | C₁₉H₂₇N₇ | 339.45 | - |
| **(53)** | 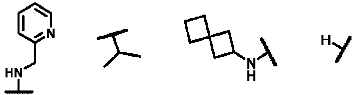 | | | | C₂₁H₂₇N₇ | 377.50 | - |
| **(54)** | 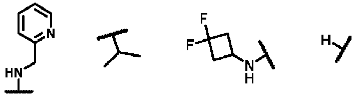 | | | | C₁₈H₂₁F₂N₇ | 373.41 | - |
| **(55)** | 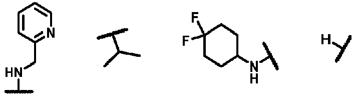 | | | | C₂₀H₂₅F₂N₇ | 401.47 | - |
| **(56)** | 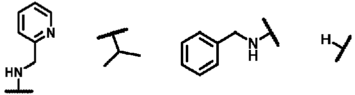 | | | | C₂₁H₂₃N₇ | 373.46 | - |
| **(57)** | 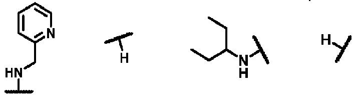 | | | | C₁₆H₂₁N₇ | 311.39 | - |
| **(58)** | 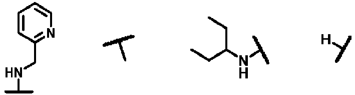 | | | | C₁₇H₂₃N₇ | 325.42 | - |
| **(59)** | 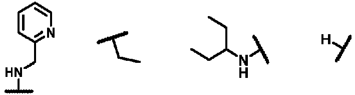 | | | | C₁₈H₂₅N₇ | 339.45 | - |
| (**60**) | 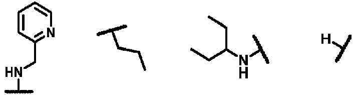 | | | | C₁₉H₂₇N₇ | 353.47 | - |
| **(61)** | 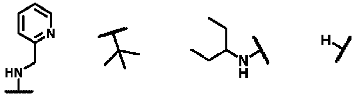 | | | | C₂₀H₂₉N₇ | 367.50 | - |
| **(62)** | 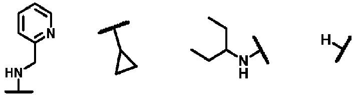 | | | | C₁₉H₂₅N₇ | 351.46 | - |
| **(63)** | 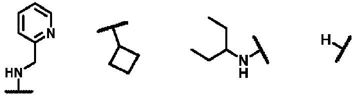 | | | | C₂₀H₂₇N₇ | 365.49 | - |
| **(64)** | 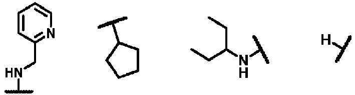 | | | | C₂₁H₂₉N₇ | 379.51 | - |
| **(65)** | 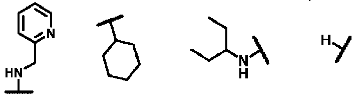 | | | | C₂₂H₃₁N₇ | 393.54 | - |
| **(66)** | 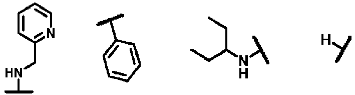 | | | | C₂₂H₂₅N₇ | 387.49 | - |
| **(67)** | 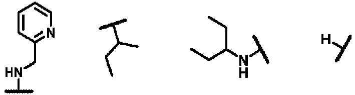 | | | | C₂₀H₂₉N₇ | 367.50 | - |
| **(68)** | 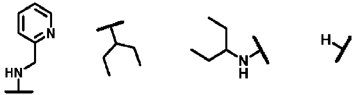 | | | | C₂₁H₃₁N₇ | 381.53 | - |
| **(69)** | 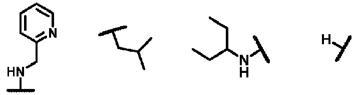 | | | | C₂₀H₂₉N₇ | 367.50 | - |
| **(70)** | 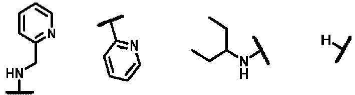 | | | | C₂₁H₂₄N₈ | 388.48 | - |
| **(71)** | 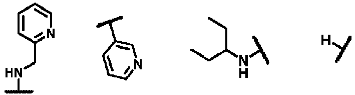 | | | | C₂₁H₂₄N₈ | 388.48 | - |
| **(72)** | 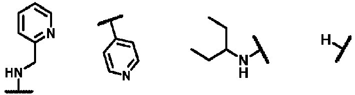 | | | | C₂₁H₂₄N₈ | 388.48 | - |
| **(73)** | 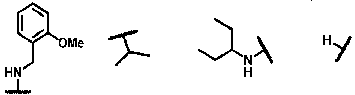 | | | | C₂₁H₃₀N₆O | 382.51 | - |
| **(74)** | 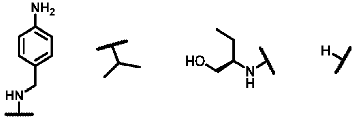 | | | | C₁₉H₂₇N₇O | 369.47 | (R) |
| **(75)** | 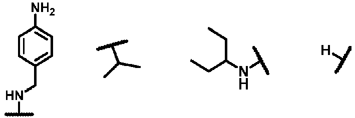 | | | | C₂₀H₂₉N₇ | 367.50 | - |
| **(76)** | 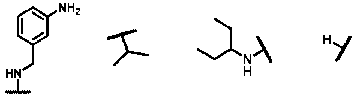 | | | | C₂₀H₂₉N₇ | 367.50 | - |
| **(77)** | 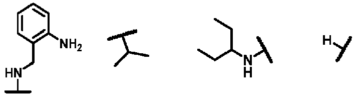 | | | | C₂₀H₂₉N₇ | 367.50 | - |
| **(78)** | 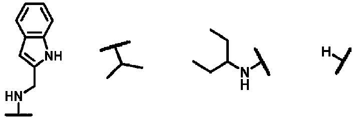 | | | | C₂₂H₂₉N₇ | 391.52 | - |
| **(79)** | 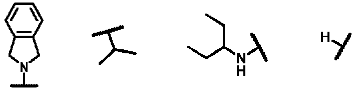 | | | | C₂₁H₂₈N₆ | 360.50 | - |
| **(80)** | 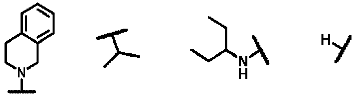 | | | | C₂₂H₃₀N₆ | 378.52 | - |

| **Cpd N°** | **NR²LR³** | **R⁴** | **NR⁵R⁶** | **R¹** | **Formula** | **MW** | **Stereo-chemistry** |
|---|---|---|---|---|---|---|---|
| **(81)** | 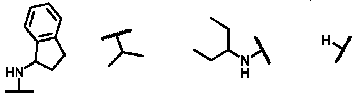 | | | | C₂₂H₃₀N₆ | 378.52 | Rac |
| **(82)** | 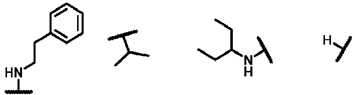 | | | | C₂₁H₃₀N₆ | 366.51 | - |
| **(83)** | 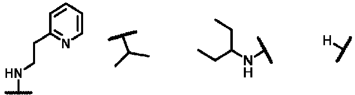 | | | | C₂₀H₂₉N₇ | 367.50 | - |
| **(84)** | 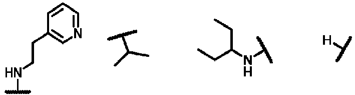 | | | | C₂₀H₂₉N₇ | 367.50 | - |
| **(85)** | 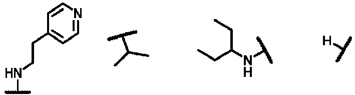 | | | | C₂₀H₂₉N₇ | 367.50 | - |
| **(86)** | 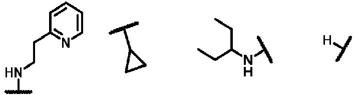 | | | | C₂₀H₂₇N₇ | 365.49 | - |
| **(87)** | 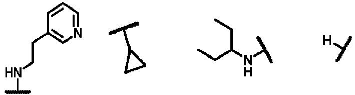 | | | | C₂₀H₂₇N₇ | 365.49 | - |
| **(88)** | 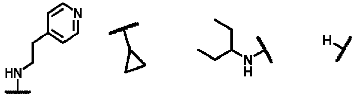 | | | | C₂₀H₂₇N₇ | 365.49 | - |
| **(89)** | 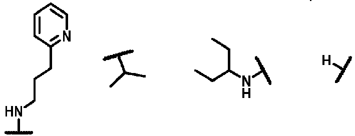 | | | | C₂₁H₃₁N₇ | 381.53 | - |
| **(90)** | 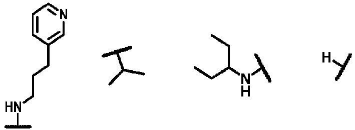 | | | | C₂₁H₃₁N₇ | 381.53 | - |
| **(91)** | 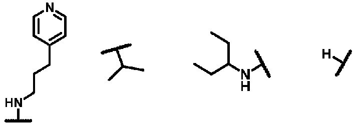 | | | | C₂₁H₃₁N₇ | 381.53 | - |
| **(92)** | 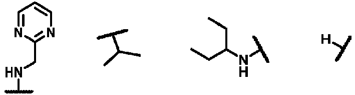 | | | | C₁₈H₂₆N₈ | 354.46 | - |
| **(93)** | 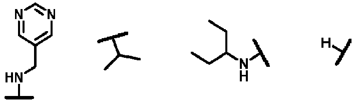 | | | | C₁₈H₂₆N₈ | 354.46 | - |
| **(94)** | 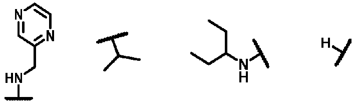 | | | | C₁₈H₂₆N₈ | 354.46 | - |
| **(95)** | 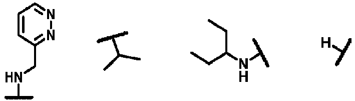 | | | | C₁₈H₂₆N₈ | 354.46 | - |
| **(96)** | 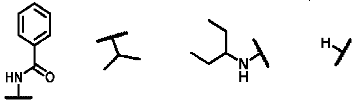 | | | | C₂₀H₂₆N₆O | 366.47 | - |
| **(97)** | 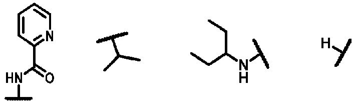 | | | | C₁₉H₂₅N₇O | 367.46 | - |
| **(98)** | 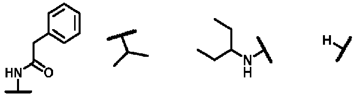 | | | | C₂₁H₂₈N₆O | 380.50 | - |
| **(99)** | 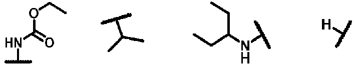 | | | | C₁₆H₂₆N₆O₂ | 334.42 | - |
| **(100)** | 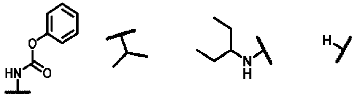 | | | | C₂₀H₂₆N₆O₂ | 382.47 | - |
| **(101)** | 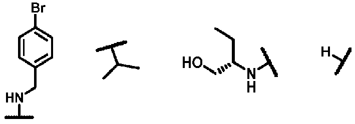 | | | | C₁₉H₂₅BrN₆O | 433.35 | (S) |
| **(102)** | 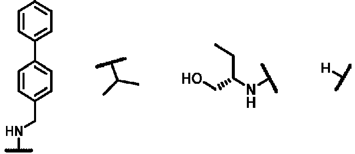 | | | | C₂₅H₃₀N₆O | 430.56 | (S) |
| **(103)** | 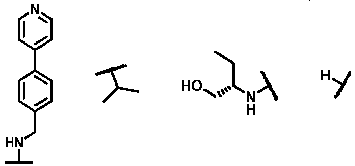 | | | | C₂₄H₂₉N₇O | 431.54 | (S) |
| **(104)** | 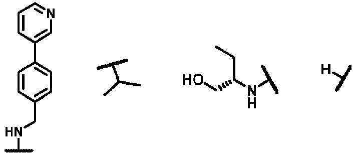 | | | | C₂₄H₂₉N₇O | 431.54 | (S) |
| **(105)** | 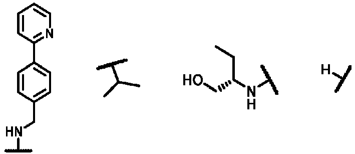 | | | | C₂₄H₂₉N₇O | 431.54 | (S) |
| **(106)** | 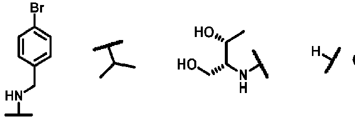 | | | | C₁₉H₂₅BrN₆O₂ | 449.35 | (S) |
| **(107)** | 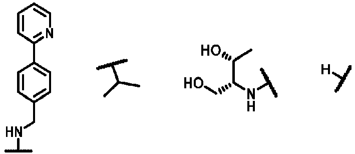 | | | | C₂₄H₂₉N₇O₂ | 447.54 | (2R,3R) |
| (**108**) | 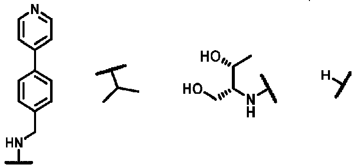 | | | | C₂₄H₂₉N₇O₂ | 447.54 | (2R,3R) |
| (**109**) | 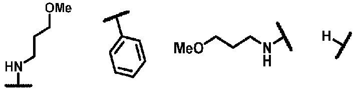 | | | | C₁₉H₂₆N₆O₂ | 370.46 | - |
| (**110**) | 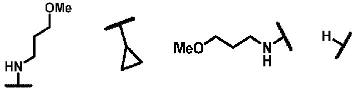 | | | | C₁₆H₂₆N₆O₂ | 334.42 | - |
| (**111**) | 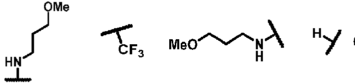 | | | | C₁₄H₂₁F₃N₆O₂ | 362.36 | - |
| (**112**) | 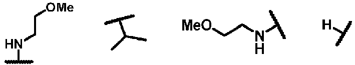 | | | | C₁₄H₂₄N₆O₂ | 308.39 | - |
| (**113**) | 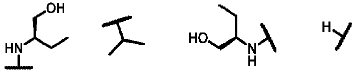 | | | | C₁₆H₂₈N₆O₂ | 336.44 | (R), (R) |
| (**114**) | 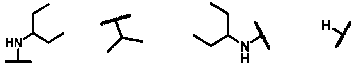 | | | | C₁₈H₃₂N₆ | 332.50 | - |
| (**115**) | 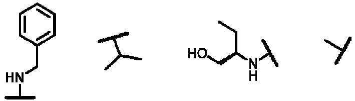 | | | | C₂₀H₂₈N₆O | 368.49 | (R) |
| (**116**) | 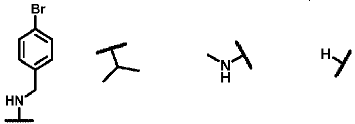 | | | | C₁₆H₁₉BrN₆ | 375.27 | - |
| (**117**) | 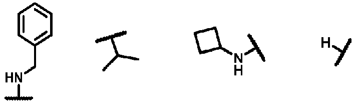 | | | | C₁₉H₂₄N₆ | 336.44 | - |
| (**118**) | 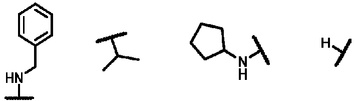 | | | | C₂₀H₂₆N₆ | 350.47 | - |
| (**119**) | 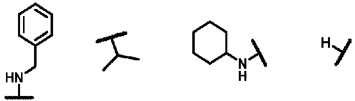 | | | | C₂₁H₂₈N₆ | 364.50 | - |
| (**120**) | 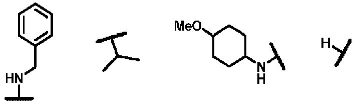 | | | | C₂₂H₃₀N₆O | 394.52 | Z/E, rac. |
| (**121**) | 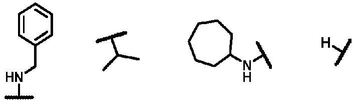 | | | | C₂₂H₃₀N₆ | 378.52 | - |
| (**122**) | 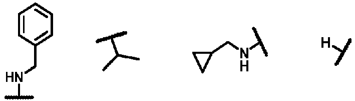 | | | | C₁₉H₂₄N₆ | 336.44 | - |
| (**123**) | 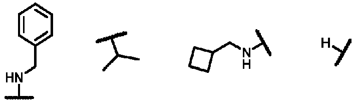 | | | | C₂₀H₂₆N₆ | 350.47 | - |
| (**124**) | 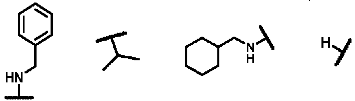 | | | | C₂₂H₃₀N₆ | 378.52 | - |
| (**125**) | 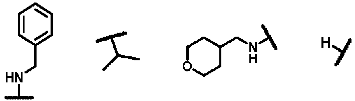 | | | | C₂₁H₂₈N₆O | 380.50 | - |
| (**126**) | 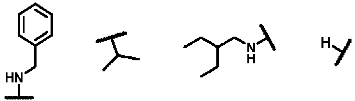 | | | | C₂₁H₃₀N₆ | 366.51 | - |
| (**127**) | 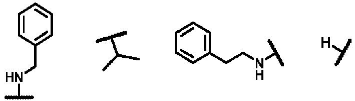 | | | | C₂₃H₂₆N₆ | 386.50 | - |
| (**128**) | 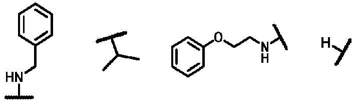 | | | | C₂₃H₂₆N₆O | 402.50 | - |
| (**129**) | 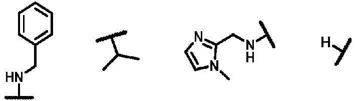 | | | | C₂₀H₂₄N₈ | 376.47 | - |
| (**130**) | 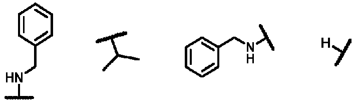 | | | | C₂₂H₂₄N₆ | 372.48 | - |
| (**131**) | 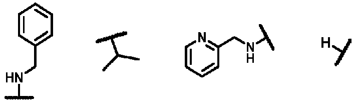 | | | | C₂₁H₂₃N₇ | 373.46 | - |
| (**132**) | 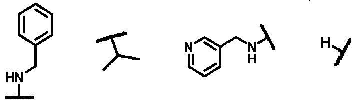 | | | | C₂₁H₂₃N₇ | 373.46 | - |
| (**133**) | 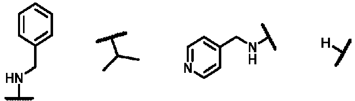 | | | | C₂₁H₂₃N₇ | 372.48 | - |
| (**134**) | 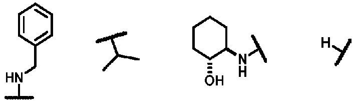 | | | | C₂₁H₂₈N₆O | 380.50 | Z, rac. |
| (**135**) | 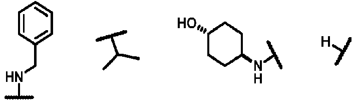 | | | | C₂₁H₂₈N₆O | 380.50 | Z, rac. |
| (**136**) | 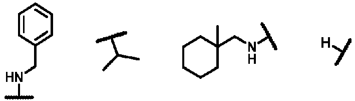 | | | | C₂₃H₃₂N₆ | 379.51 | - |
| (**137**) | 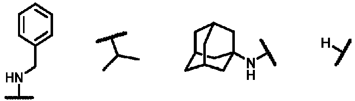 | | | | C₂₅H₃₂N₆ | 380.50 | - |
| (**138**) | 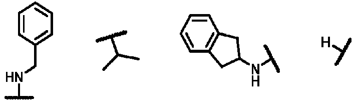 | | | | C₂₄H₂₆N₆ | 380.50 | - |
| (**139**) | 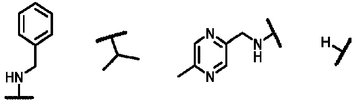 | | | | C₂₁H₂₄N₈ | 392.35 | - |
| (**140**) | 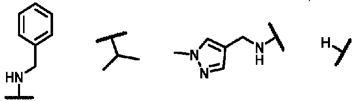 | | | | C₂₀H₂₄N₈ | 393.54 | - |
| (**141**) | 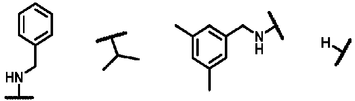 | | | | C₂₄H₂₈N₆ | 416.57 | - |
| (**142**) | 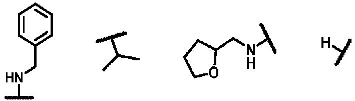 | | | | C₂₀H₂₆N₆O | 366.47 | rac. |
| (**143**) | 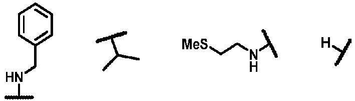 | | | | C₁₈H₂₄N₆S | 356.49 | - |
| (**144**) | 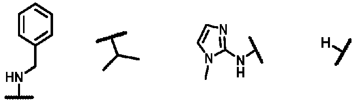 | | | | C₁₉H₂₂N₈ | 362.44 | - |
| (**145**) | 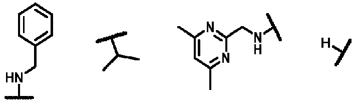 | | | | C₂₂H₂₆N₈ | 402.51 | - |
| (**146**) | 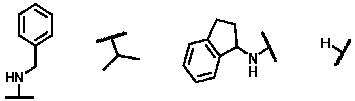 | | | | C₂₄H₂₆N₆ | 398.51 | rac. |
| (**147**) | 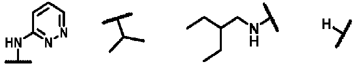 | | | | C₁₈H₂₆N₈ | 354.46 | |
| (**148**) | 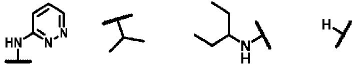 | | | | C₁₇H₂₄N₈ | 340.44 | - |
| (**149**) | 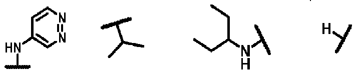 | | | | C₁₇H₂₄N₈ | 340.44 | - |
| (**150**) | 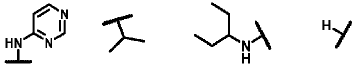 | | | | C₁₇H₂₄N₈ | 340.44 | - |
| (**151**) | 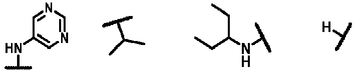 | | | | C₁₇H₂₄N₈ | 340.44 | - |
| (**152**) | 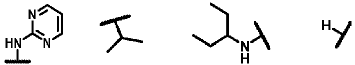 | | | | C₁₇H₂₄N₈ | 340.44 | - |
| (**153**) | 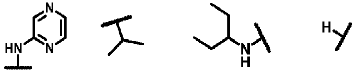 | | | | C₁₇H₂₄N₈ | 340.44 | - |
| (**154**) | 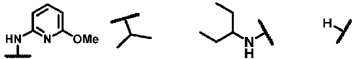 | | | | C₁₉H₂₇N₇O | 369.47 | - |
| (**155**) | 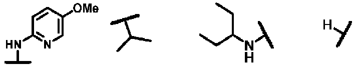 | | | | C₁₉H₂₇N₇O | 369.47 | - |
| (**156**) | 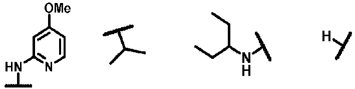 | | | | C₁₉H₂₇N₇O | 369.47 | - |
| (**157**) | 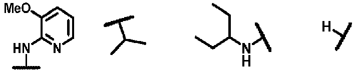 | | | | C₁₉H₂₇N₇O | 369.47 | - |
| (**158**) | 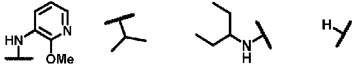 | | | | C₁₉H₂₇N₇O | 369.47 | - |
| (**159**) | 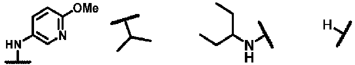 | | | | C₁₉H₂₇N₇O | 369.47 | - |
| (**160**) | 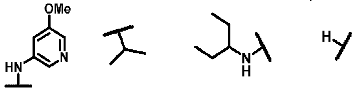 | | | | C₁₉H₂₇N₇O | 369.47 | - |
| (**161**) | 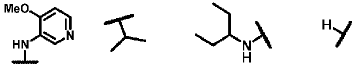 | | | | C₁₉H₂₇N₇O | 369.47 | - |
| (**162**) | 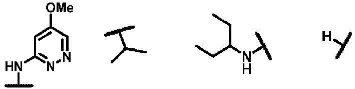 | | | | C₁₈H₂₆N₈O | 370.46 | - |
| (**163**) | 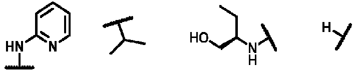 | | | | C₁₇H₂₃N₇O | 341.42 | (R) |
| (**164**) | 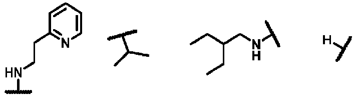 | | | | C₂₁H₃₁N₇ | 381.53 | - |
| (**165**) | 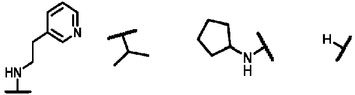 | | | | C₂₀H₂₇N₇ | 365.49 | - |
| (**166**) | 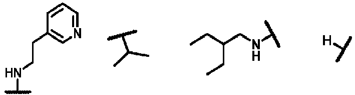 | | | | C₂₁H₃₁N₇ | 381.53 | - |
| (**167**) | 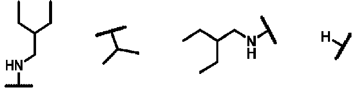 | | | | C₂₀H₃₆N₆ | 360.55 | - |
| (**168**) | 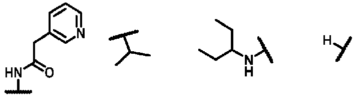 | | | | C₂₀H₂₇N₇O | 381.48 | - |
| (**169**) | 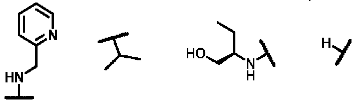 | | | | C₁₈H₂₅N₇O | 355.45 | (R) |
| (**170**) | 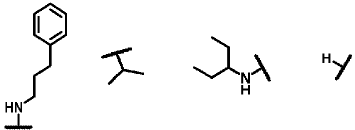 | | | | C₂₂H₃₂N₆ | 380.54 | - |
| (**171**) | 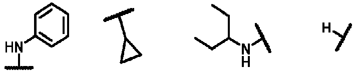 | | | | C₁₉H₂₄N₆ | 336.44 | - |
| (**172**) | 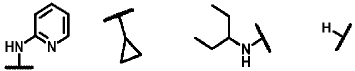 | | | | C₁₈H₂₃N₇ | 337.43 | - |
| (**173**) | 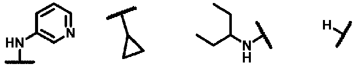 | | | | C₁₈H₂₃N₇ | 337.43 | - |
| (**174**) | 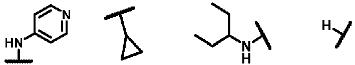 | | | | C₁₈H₂₃N₇ | 337.43 | - |
| (**175**) | 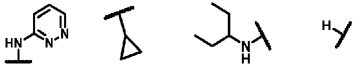 | | | | C₁₇H₂₂N₈ | 338.42 | - |
| (**176**) | 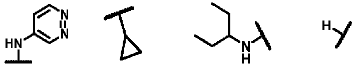 | | | | C₁₇H₂₂N₈ | 338.42 | - |
| (**177**) | 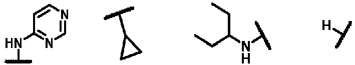 | | | | C₁₇H₂₂N₈ | 338.42 | - |
| (**178**) | 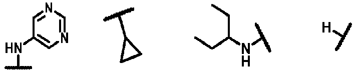 | | | | C₁₇H₂₂N₈ | 338.42 | - |
| (**179**) | 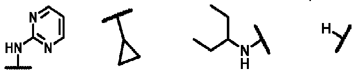 | | | | C₁₇H₂₂N₈ | 338.42 | - |
| (**180**) | 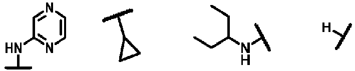 | | | | C₁₇H₂₂N₈ | 338.42 | - |
| (**181**) | 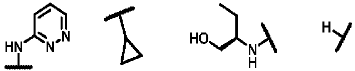 | | | | C₁₆H₂₀N₈O | 340.39 | (R) |
| (**182**) | 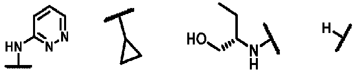 | | | | C₁₆H₂₀N₈O | 340.39 | (S) |
| (**183**) | 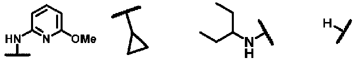 | | | | C₁₉H₂₅N₇O | 367.46 | - |
| (**184**) | 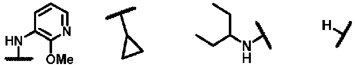 | | | | C₁₉H₂₅N₇O | 367.46 | - |
| (**185**) | 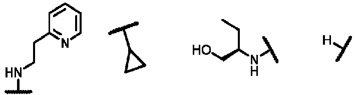 | | | | C₁₉H₂₅N₇O | 367.46 | (R) |
| (**186**) | 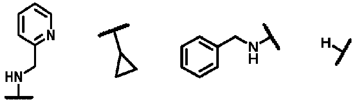 | | | | C₂₁H₂₁N₇ | 371.45 | - |
| (**187**) | 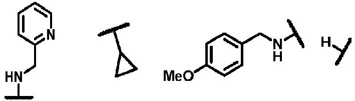 | | | | C₂₂H₂₃N₇O | 401.47 | - |
| (**188**) | 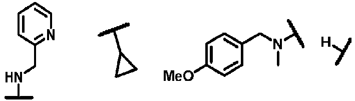 | | | | C₂₃H₂₅N₇O | 415.50 | - |
| (**189**) | 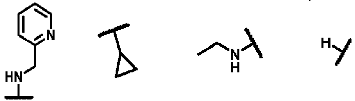 | | | | C₁₆H₁₉N₇ | 309.38 | - |
| (**190**) | 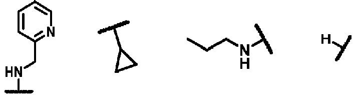 | | | | C₁₇H₂₁N₇ | 323.40 | - |
| (**191**) | 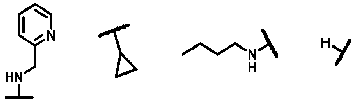 | | | | C₁₈H₂₃N₇ | 337.43 | - |
| (**192**) | 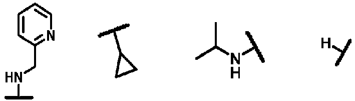 | | | | C₁₇H₂₁N₇ | 323.40 | - |
| (**193**) | 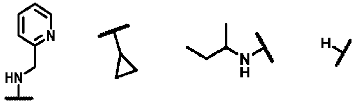 | | | | C₁₈H₂₃N₇ | 337.43 | Rac. |
| (**194**) | 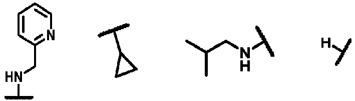 | | | | C₁₇H₂₁N₇ | 337.43 | - |
| (**195**) | 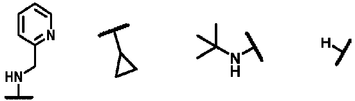 | | | | C₁₈H₂₃N₇ | 337.43 | - |
| (**196**) | 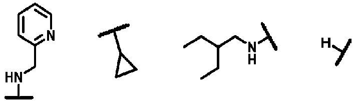 | | | | C₂₀H₂₇N₇ | 365.49 | - |
| (**197**) | 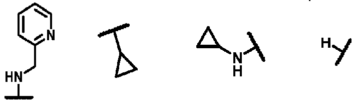 | | | | C₁₇H₁₉N₇ | 321.39 | - |
| (**198**) | 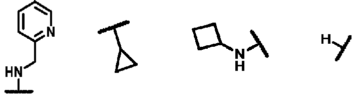 | | | | C₁₈H₂₁N₇ | 335.42 | - |
| (**199**) | 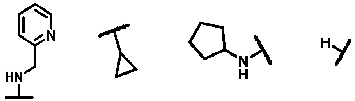 | | | | C₁₉H₂₃N₇ | 349.44 | - |
| (**200**) | 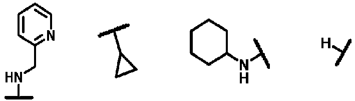 | | | | C₂₀H₂₅N₇ | 363.47 | - |
| (**201**) | 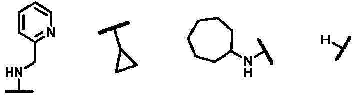 | | | | C₂₁H₂₇N₇ | 377.50 | - |
| (**202**) | 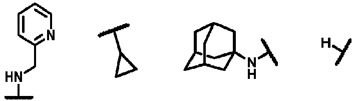 | | | | C₂₄H₂₉N₇ | 415.55 | - |
| (**203**) | 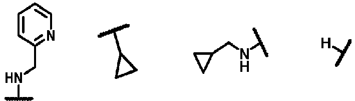 | | | | C₁₈H₂₁N₇ | 335.42 | - |
| (**204**) | 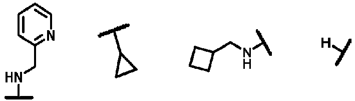 | | | | C₁₆H₂₃N₇ | 349.44 | - |
| (**205**) | 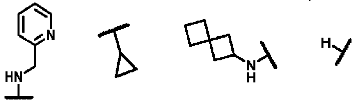 | | | | C₂₁H₂₅N₇ | 375.45 | - |
| (**206**) | 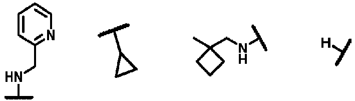 | | | | C₂₀H₂₅N₇ | 363.47 | - |
| (**207**) | 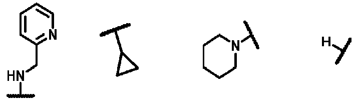 | | | | C₁₉H₂₃N₇ | 349.44 | - |
| (**208**) | 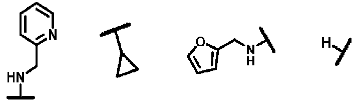 | | | | C₁₉H₁₉N₇O | 361.41 | - |
| (**209**) | 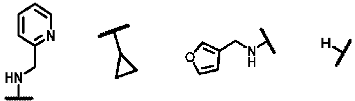 | | | | C₁₉H₁₉N₇O | 361.41 | - |
| (**210**) | 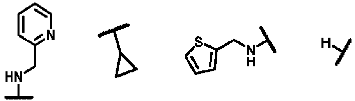 | | | | C₁₉H₁₉N₇S | 377.47 | - |
| (**211**) | 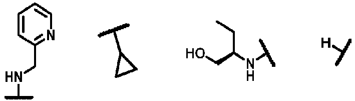 | | | | C₁₈H₂₃N₇O | 353.43 | - |
| (**212**) | 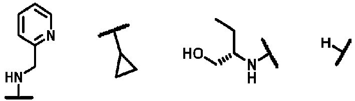 | | | | C₁₈H₂₃N₇O | 353.43 | - |
| (**213**) | 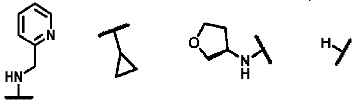 | | | | C₁₈H₂₁N₇O | 351.41 | - |
| (**214**) | 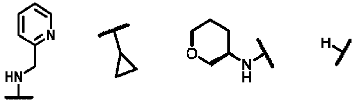 | | | | C₁₉H₂₃N₇O | 365.44 | - |
| (**215**) | 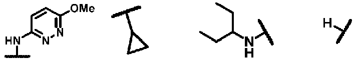 | | | | C₁₈H₂₄N₈O | 368.45 | - |
| (**216**) | 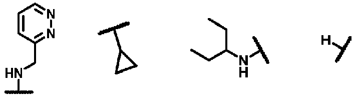 | | | | C₁₈H₂₄N₈ | 352.45 | - |
| (**217**) | 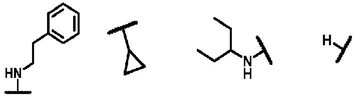 | | | | C₂₁H₂₈N₆ | 364.50 | - |
| (**218**) | 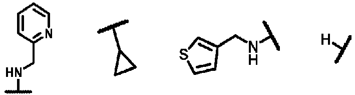 | | | | C₁₉H₁₉N₇S | 377.47 | - |
| (**219**) | 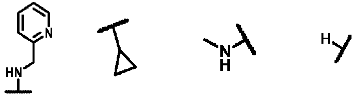 | | | | C₁₅H₁₇N₇ | 295.35 | - |
| (**220**) | 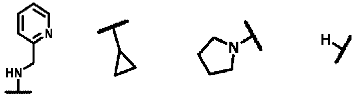 | | | | C₁₈H₂₁N₇ | 335.42 | - |

The below **Table 2** describes the analytical and spectroscopic data of the compounds introduced in **Table 1.**

¹H NMR analyses (400 or 500 MHz) and ¹³C NMR spectra (101 MHz) were recorded with a Bruker ULTRASHIELD 500 or 400 spectrometer. Processing and analyses of the spectra were performed with MestReNova. Data appear in the following order: chemical shifts in ppm which were referenced to the internal solvent signal, multiplicity, number of protons, and coupling constant *J* in Hertz.

Synthetic intermediates: reversed-phase UPLC/MS analyses were carried out with a UPLC Acquity (Waters) with an UV-DAD detector and a mass detector (SQD2). Compounds (0.2 to 0.6 mg) were solubilized in a mixture of DMSO/H₂O (1/1) and filtered on syringe filter 0.2 µm.
- Acidic conditions:
   Acquity BEH C18 column, 2.1 × 50 mm, 1.7 µm. Flow rate: 0.65 mL/min. Gradient: (H₂O + 0.1% HCOOH v/v)/(CAN + 0.1% HCOOH v/v) from 95/5 to 5/95 in 4.0 min.

Final compounds: Reversed-phase HPLC/MS analyses were carried out with a HPLC Ultimate 3000 (ThermoScientific) with an UV-DAD detector. Mass detection processing with direct infusion in mass detector (SQD2) from UPLC Acquity (Waters). Compounds (0.2 to 0.6 mg) were solubilized in a mixture of DMSO/H₂O (1/1) and filtered on syringe filter 0.2 µm.
- Acidic conditions:
   Thermo Scientific Syncronis C18 column, 150 x 4.6 mm, 5 µm. Flow rate: 1 mL/min. Gradient: (H₂O + 0.1% HCOOH v/v)/(ACN + 0.1% HCOOH v/v) from 95/5 to 5/95 in 19.0 min.
- Alkaline conditions:
   Thermo Scientific Syncronis C18 column, 150 × 4.6 mm, 5 µm or XTERRA RP18, 150×4.6mm, 3.5µm. Flow rate: 1 mL/min. Gradient: (HCOONH₄ 10mM + NH₄OH 25% aq. to ajust pH = 10)/ACN from 95/5 to 5/95 in 19.0 min.

**Table 2: Spectroscopic and analytical characterization of compounds (1) to (220)**

| **Cpd N°** | **Structure** | **HPLC** | **Description** |
|---|---|---|---|
| (1) | 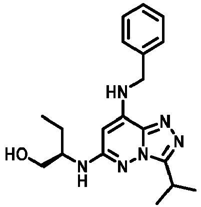 | > 98% ^{a} | Pale yellow solid. |
| | | | **¹H NMR (400 MHz, DMSO-*d₆*) δ :** 0.85 (t, *J* = 7.4 Hz, 3H, CH₃), 1.35 (d, *J* = 6.9 Hz, 6H, 2×CH₃), 1.37 - 1.66 (m, 2H, CH₂), 3.25 - 3.31 (m, 1H, CH_{Alk}), 3.41 (dq, *J* = 18.6, 5.3 Hz, 2H, CH₂), 3.62 (h, *J* = 5.9 Hz, 1H, CH_{Alk}), 4.37 - 4.48 (m, 2H, CH₂), 4.57 (t, *J* = 5.4 Hz, 1H, OH), 5.47 (s, 1H, H_{Ar}), 6.29 (d, *J* = 7.7 Hz, 1H, NH_{Alk}), 7.24 (td, *J* = 6.2, 2.6 Hz, 1H, H_{Ar}), 7.31 - 7.38 (m, 4H, 4×H_{Ar}), 7.86 (t, *J* = 6.4 Hz, 1H, NH_{Bn}). |
| | | | **¹³C NMR (101 MHz, DMSO-*d₆*)** δ : 10.5 (CH₃), 19.6 (CH₃), 23.6 (CH₂), 24.7 (CH), 45.2 (CH₂), 53.8 (CH), 61.9 (CH₂), 84.4 (CH), 126.8 (CH_{Ar}), 126.9 (CH_{Ar}), 128.4 (CH_{Ar}), 138.4 (C_{q}), 139.7 (C_{q}), 139.8 (C_{q}), 152.7 (C_{q}), 155.8 (C_{q}). |
| | | | **MS (ESI+) : m/z calcd for C₁₉H₂₆N₆O :** 355.2 [M+H]+, **found** : 355.3. |
| (2) | 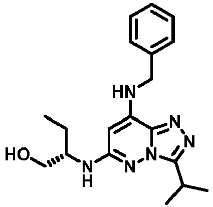 | > 98% ^{a} | White Solid. |
| | | | **¹H NMR (400 MHz, DMSO-*d₆*) δ :** 0.85 (t, *J* = 7.4 Hz, 3H, CH_{3 Alk}), 1.35 (d, *J* = 6.9 Hz, 6H, 2×CH_{3 iPr}), 1.39 - 1.67 (m, 2H, CH_{2-CH3}), 3.27 - 3.34 (m, 1H, H_{Alk}), 3.41 (dq, *J* = 18.5, 5.4 Hz, 2H, CH_{2-OH}), 3.62 (h, *J* = 6.0 Hz, 1H, H_{Alk iPr}), 4.34 - 4.52 (m, 2H, CH_{2 Bn}), 4.57 (t, *J* = 5.4 Hz, 1H, OH), 5.47 (s, 1H, H_{Ar}), 6.29 (d, *J* = 7.8 Hz, 1H, NH), 7.17 - 7.45 (m, 5H, 5xH_{Ar}), 7.86 (t, *J* = 6.3 Hz, 1H, NH_{Bn}). |
| | | | **¹³C NMR (101 MHz, DMSO-*d₆*) δ :** 10.5 (CH_{3 Alk}), 19.6 (2×CH_{3 iPr}), 23.6 (CH_{2-CH3}), 24.7 (CH_{Alk}), 45.2 (CH_{2 Bn}), 53.8 (CH_{Alk iPr}), 61.9 (CH_{2-OH}), 84.4 (CH_{Ar}), 126.8 (2xCH_{Ar}), 126.9 (CH_{Ar}), 128.4 (2xCH_{Ar}), 138.4 (C_{q}), 139.7 (C_{q}), 139.8 (C_{q}), 152.7 (C_{q}), 155.8 (C_{q}). |
| | | | **MS (ESI+) : m/z calcd for C₁₉H₂₆N₆O** : 355.2 [M+H]+, **found** : 355.3. |
| (3) | 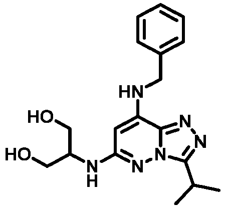 | > 96 % ^{a} | White Solid. |
| | | | **¹H NMR (400 MHz, DMSO-*d₆*) δ :** 1.36 (d, *J* = 7.0 Hz, 6H, 2×CH₃), 3.27 - 3.38 (m, 1H, H_{Alk}), 3.50 (t, *J* = 5.4 Hz, 4H, 2×CH_{2 Alk}), 3.74 (h, *J* = 5.4 Hz, 1H, H_{Alk iPr}), 4.42 (d, *J* = 6.3 Hz, 2H, CH_{2 Bn}), 4.56 (t, *J* = 5.4 Hz, 2H, 2×OH), 5.53 (s, 1H, H_{Ar}), 6.28 (d, *J* = 7.5 Hz, 1H, NH), 7.21 - 7.44 (m, 5H, 5xH_{Ar}), 7.90 (t, *J* = 6.4 Hz, 1H, NH_{Bn}). |
| | | | **¹³C NMR (101 MHz, DMSO-*d₆*) δ :** 19.7 (2×CH₃), 24.7 (CH_{Alk}), 45.2 (CH_{2 Bn}), 54.3 (CH_{Alk iPr}), 59.7 (2×CH₂), 84.5 (CH_{Ar}), 126.9 (2xCH_{Ar}), 126.9 (CH_{Ar}), 128.4 (2×CH_{Ar}), 138.5 (C_{q}), 139.7 (C_{q}), 139.8 (C_{q}), 152.8 (C_{q}), 155.7 (C_{q}). |
| | | | **MS (ESI+)** : **m/z calcd for C₁₉H₂₆N₆O** : 357.2 [M+H]+, **found** : 327.35. |
| (4) | 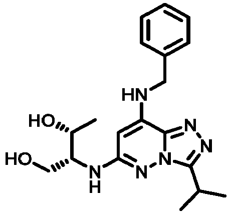 | > 96 % ^{a} | White Solid. |
| | | | **¹H NMR (400 MHz, DMSO-*d₆*) δ :** 1.04 (d, *J* = 6.4 Hz, 3H, CH_{3 Alk}), 1.35 (dd, *J* = 7.0, 4.5 Hz, 6H, 2×CH_{3 iPr}), 3.27 - 3.36 (m, 1H, H_{iPr}), 3.48 (ddt, *J* = 36.1, 10.5, 5.7 Hz, 2H, CH_{2-OH}), 3.73 (q, *J* = 7.6, 7.1 Hz, 1H, H_{Alk-NH}), 3.93 - 4.04 (m, 1H, H_{Alk-OH}), 4.42 (d, *J* = 6.4 Hz, 2H, CH_{2 Bn}), 4.54 (t, *J* = 5.6 Hz, 2H, 2×OH), 5.65 (s, 1H, H_{Ar}), 6.12 (d, *J* = 8.3 Hz, 1H, NH), 7.20 - 7.41 (m, 5H, 5xH_{Ar}), 7.86 (t, *J* = 6.4 Hz, 1H, NH_{Bn}). |
| | | | **¹³C NMR (101 MHz, DMSO-*d₆*) δ :** 19.7 (CH_{3 iPr}), 19.7 (CH_{3 iPr}), 20.3 (CH_{3 Alk}), 24.6 (CH_{iPr}), 45.2 (CH_{2 Bn}), 56.9 (CH_{Alk}), 60.1 (CH_{Alk}), 64.5 (CH_{2-OH}), 84.5 (CH_{Ar}), 126.9 (CH_{Ar}), 127.0 (2xCH_{Ar}), 128.4 (2xCH_{Ar}), 138.5 (C_{q}), 139.7 (C_{q}), 139.7 (C_{q}), 152.7 (C_{q}), 156.3 (C_{q}). |
| | | | **MS (ESI+)** : **m/z calcd for C₁₉H₂₆N₆O₂** : 371.22 [M+H]+, **found** : 371.36. |
| (5) | 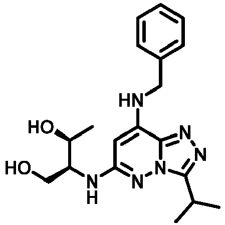 | > 96 % ^{a} | White solid. |
| | | | **¹H NMR (400 MHz, DMSO-*d₆*) δ :** 1.03 (d, *J* = 6.4 Hz, 3H, CH_{3 Alk}), 1.35 (dd, *J* = 7.0, 4.4 Hz, 6H, 2×CH_{3 iPr}), 3.24 - 3.38 (m, 1H, H_{iPr}), 3.47 (ddt, *J* = 36.1, 10.5, 5.6 Hz, 2H, CH_{2-OH}), 3.73 (qt, *J* = 6.2, 2.8 Hz, 1H, H_{Alk-NH}), 3.97 (dq, *J* = 9.6, 6.2 Hz, 1H, H_{Alk-OH}), 4.41 (d, *J* = 6.4 Hz, 2H, CH_{2 Bn}), 4.53 (t, *J* = 5.6 Hz, 2H, 2×OH), 5.64 (s, 1H, H_{Ar}), 6.11 (d, *J* = 8.3 Hz, 1H, NH), 7.20 - 7.41 (m, 5H, 5×H_{Ar}), 7.86 (t, *J* = 6.4 Hz, 1H, NH_{Bn}). |
| | | | **¹³C NMR (101 MHz, DMSO-*d₆*) δ :** 19.7 (CH_{3 iPr}), 19.7 (CH_{3 iPr}), 20.3 (CH_{3 Aik}), 24.6 (CH_{iPr}), 45.2 (CH_{2 Bn}), 56.9 (CH_{Alk}), 60.1 (CH_{Alk}), 64.5 (CH_{2-OH}), 84.5 (CH_{Ar}), 126.9 (CH_{Ar}), 127.0 (2×CH_{Ar}), 128.4 (2×CH_{Ar}), 138.5 (C_{q}), 139.7 (C_{q}), 139.7 (C_{q}), 152.7 (C_{q}), 156.3 (C_{q}). |
| | | | **MS (ESI+) : m/z calcd for C₁₉H₂₆N₆O₂ :** 371.22 [M+H]+, **found** : 371.36. |
| (6) | 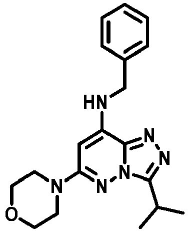 | > 96% ^{a} | White solid. |
| | | | **¹H NMR (400 MHz, DMSO-*d₆*) δ :** 1.36 (d, *J* = 6.9 Hz, 6H, 2×CH₃), 3.28 - 3.41 (m, 5H, 2×CH_{2-N} & CH_{iPr}), 3.62 - 3.73 (m, 4H, 2xCH_{2-O}), 4.56 (d, *J* = 6.4 Hz, 2H, CH_{2 Bn}), 5.83 (s, 1H, H_{Ar}), 7.23 (t, *J* = 7.3 Hz, 1H, H_{Ar}), 7.32 (t, *J* = 7.5 Hz, 2H, 2×H_{Ar}), 7.40 (d, *J* = 7.5 Hz, 2H, 2×H_{Ar}), 8.15 (t, *J* = 6.4 Hz, 1H, NH). |
| | | | **¹³C NMR (101 MHz, DMSO-*d₆*) δ :** 19.7 (2×CH_{3 iPr}), 24.7 (CH_{iPr}), 44.9 (CH_{2 Bn}), 46.1 (2×CH_{2-N}), 65.7 (2×CH_{2-O}), 82.4 (CH_{Ar}), 127.0 (CH_{Ar}), 127.3 (2×CH_{Ar}), 128.4 (2×CH_{Ar}), 138.5 (C_{q}), 139.4 (C_{q}), 140.9 (C_{q}), 153.1 (C_{q}), 157.6 (C_{q}). |
| | | | **MS (ESI+) : m/z calcd for C₁₉H₂₆N₆O** : 353.21 [M+H]+, **found :** 353.26. |
| (7) | 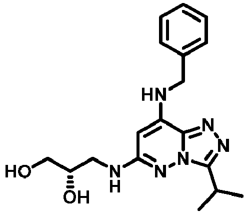 | > 97 % ^{a} | White Solid. |
| | | | **¹H NMR (400 MHz, DMSO-*d₆*) δ :** 1.35 (d, *J* = 6.9 Hz, 6H, 2×CH_{3 iPr}), 2.99 - 3.11 (m, 1H, H_{Alk(CH2-NH)}), 3.25 - 3.36 (m, 4H, H_{Alk iPr} & CH_{2-OH} & H_{Alk(CH2-NH)}), 3.64 (h, *J* = 5.4 Hz, 1H, H_{Alk-OH}), 4.42 (d, *J* = 6.3 Hz, 2H, CH_{2 Bn}), 4.53 (t, *J* = 5.6 Hz, 1H, OH_{-CH2}), 4.71 (d, *J* = 5.0 Hz, 1H, OH_{-HAlk}), 5.51 (s, 1H, H_{Ar}), 6.47 (t, *J* = 5.4 Hz, 1H, NH_{Alk}), 7.21 - 7.41 (m, 5H, 5×H_{Ar}), 7.90 (t, *J* = 6.5 Hz, 1H, NH_{Bn}). |
| | | | **¹³C NMR (101 MHz, DMSO-*d₆*) δ :** 19.7 (2×CH_{3 iPr}), 24.6 (CH_{iPr}), 44.3 (CH_{2-NH}), 45.2 (CH_{2 Bn}), 64.0 (CH_{2-OH}), 70.0 (CH_{Alk}), 84.3 (CH_{Ar}), 126.9 (CH_{Ar}), 126.9 (2×CH_{Ar}), 128.4 (2×CH_{Ar}), 138.4 (C_{q}), 139.7 (C_{q}), 139.8 (C_{q}), 152.9 (C_{q}), 156.2 (C_{q}). |
| | | | **MS (ESI+) : m/z calcd for C₁₈H₂₄N₆O₂ :** 357.20 [M+H]+, **found :.** 357.35. |
| (8) | 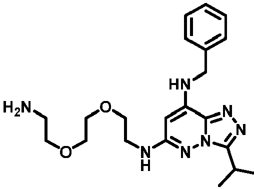 | > 99% ^{b} | White crystalline solid. |
| | | | **¹H NMR (400 MHz, DMSO-*d₆*) δ :** 1.36 (d, *J* = 6.9 Hz, 6H, 2×CH_{3 iPr}), 2.63 (t, *J* = 5.7 Hz, 2H, CH₂), 3.29 - 3.36 (m, 5H, 2×CH₂ & H_{iPr}), 3.46 - 3.58 (m, 6H, 3xCH₂), 4.43 (d, *J* = 6.3 Hz, 2H, CH_{2 Bn}), 5.43 (s, 1H, H_{Ar}), 6.62 (t, *J* = 5.5 Hz, 1H, NH_{Bn}), 7.20 - 7.38 (m, 5H, 5×H_{Ar}), 7.93 (t, *J* = 6.4 Hz, 1H, NH_{Bn}). |
| | | | **¹³C NMR (101 MHz, DMSO-*d₆*) δ :** 19.7 (2xCH₃), 24.7 (CH_{iPr}), 40.6 (CH₂), 41.1 (CH₂), 45.2 (CH_{2 Bn}), 68.6 (CH₂), 69.5 (CH₂), 69.6 (CH₂), 72.6 (CH₂), 84.1 (CH_{Ar}), 126.9 (2xCH_{Ar}), 126.9 (CH_{Ar}), 128.4 (2×CH_{Ar}), 138.3 (C_{q}), 139.7 (C_{q}), 139.9 (C_{q}), 152.9 (C_{q}), 155.8 (C_{q}). |
| | | | **MS (ESI+) : m/z calcd for C₂₁H₃₁N₇O₂** : 414.26 [M+H]+, **found** : 414.40. |
| (9) | 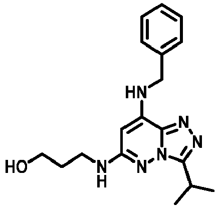 | > 99 % ^{a} | White solid. |
| | | | **¹H NMR (400 MHz, DMSO-*d₆*) δ :** 1.36 (d, *J* = 6.9 Hz, 6H, 2×CH_{3 iPr}), 1.66 (p, *J* = 6.6 Hz, 2H, CH_{2-CH2-NH}), 3.17 (q, *J* = 6.6 Hz, 2H, CH_{2-NH Aik}), 3.29 - 3.37 (m, 1H, H_{iPr}), 3.45 (q, *J* = 6.0 Hz, 2H, CH_{2-OH}), 4.43 (dd, *J* = 6.2, 3.4 Hz, 3H, CH_{2 Bn} & OH), 5.38 (s, 1H, H_{Ar}), 6.50 (t, *J* = 5.3 Hz, 1H, NH_{Alk}), 7.20 - 7.40 (m, 5H, 5xH_{Ar}), 7.90 (t, *J* = 6.4 Hz, 1H, NH_{Bn}). |
| | | | **¹³C NMR (101 MHz, DMSO-*d₆*) δ :** 19.7 (2xCH_{3 iPr}), 24.7 (CH_{iPr}), 31.8 (CH_{2-CH2-NH}), 38.0 (CH_{2-NH Alk}), 45.2 (CH_{2 Bn}), 58.6 (CH_{2-OH}), 84.2 (CH_{Ar}), 126.8 (2xCH_{Ar}), 126.9 (CH_{Ar}), 128.4 (2xCH_{Ar}), 138.4 (C_{q}), 139.7 (C_{q}), 139.8 (C_{q}), 152.9 (C_{q}), 156.0 (C_{q}). |
| | | | **MS (ESI+) : m/z calcd for C₁₈H₂₄N₆O** : 341.21 [M+H]+, **found** : 341.36 |
| (10) | 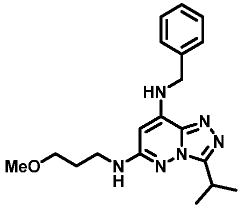 | > 98 % ^{a} | White solid. |
| | | | **¹H NMR (400 MHz, DMSO-*d₆*) δ :** 1.36 (d, *J* = 6.9 Hz, 6H, 2×CH_{3 iPr}), 1.75 (p, *J* = 6.6 Hz, 2H, CH_{2-CH2}), 3.17 (q, *J* = 6.5 Hz, 2H, CH_{2-NH Alk}), 3.22 (s, 3H, OCH₃), 3.29 - 3.39 (m, 3H, CH_{iPr} & CH_{2-OCH3}), 4.44 (d, *J* = 6.3 Hz, 2H, CH_{2 Bn}), 5.37 (s, 1H, H_{Ar}), 6.54 (t, *J* = 5.4 Hz, 1H, NH_{Alk}), 7.19 - 7.41 (m, 5H, 5xH_{Ar}), 7.91 (t, *J* = 6.4 Hz, 1H, NH_{Bn}). |
| | | | **¹³C NMR (101 MHz, DMSO-*d₆*) δ :** 19.7 (2×CH_{3 iPr}), 24.7 (CH_{iPr}), 28.5 (CH_{2-CH2}), 38.0 (CH_{2-NH Alk}), 45.2 (CH_{2 Bn}), 57.8 (OCH₃), 69.8 (CH_{2-OCH3}), 84.1 (CH_{Ar}), 126.8 (2xCH_{Ar}), 126.9 (CH_{Ar}), 128.4 (2×CH_{Ar}), 138.3 (C_{q}), 139.7 (C_{q}), 139.8 (C_{q}), 152.9 (C_{q}), 155.9 (C_{q}). |
| | | | **MS (ESI+) : m/z calcd for C₁₉H₂₆N₆O** : 355.2 [M+H]+, **found** : 355.3. |
| (11) | 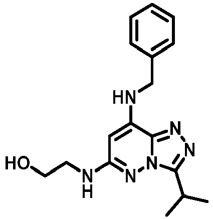 | > 99 % ^{a} | White solid. |
| | | | **¹H NMR (400 MHz, DMSO-*d₆*) δ :** 1.35 (d, *J* = 6.9 Hz, 6H, 2xCH₃), 3.21 (q, *J* = 5.7 Hz, 2H, CH_{2-NH Alk}), 3.28 - 3.36 (m, 1H, H_{iPr}), 3.52 (q, *J* = 5.7 Hz, 2H, CH_{2-OH}), 4.42 (d, *J* = 6.3 Hz, 2H, CH_{2 Bn}), 4.63 (t, *J* = 5.3 Hz, 1H, OH), 5.44 (s, 1H, H_{Ar}), 6.55 (t, *J* = 5.5 Hz, 1H, NH_{Alk}), 7.19 - 7.39 (m, 5H, 5xH_{Ar}), 7.91 (t, *J* = 6.4 Hz, 1H, NH_{Bn}). |
| | | | **¹³C NMR (101 MHz, DMSO-*d₆*) δ :** 19.7 (2xCH₃), 24.6 (CH_{iPr}), 43.6 (CH_{2-NH}), 45.2 (CH_{2 Bn}), 59.3 (CH_{2-OH}), 84.3 (CH_{Ar}), 126.9 (2xCH_{Ar}), 126.9 (CH_{Ar}), 128.4 (2xCH_{Ar}), 138.4 (C_{q}), 139.7 (C_{q}), 139.8 (C_{q}), 152.8 (C_{q}), 156.0 (C_{q}). |
| | | | **MS (ESI+) : m/z calcd for C₁₇H₂₂N₆O** : 327.19 [M+H]+, **found** : 327.35. |
| (12) | 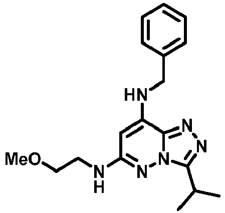 | > 95 % ^{a a} | White crystalline solid. |
| | | | **¹H NMR (400 MHz, DMSO-*d₆*) δ** : 1.35 (d, *J* = 6.9 Hz, 6H, 2×CH_{3 iPr}), 3.24 (s, 3H, OCH₃), 3.27 - 3.37 (m, 3H, H_{iPr} & CH_{2-NH Alk}), 3.45 (t, *J* = 5.6 Hz, 2H, CH_{2-OCH3}), 4.42 (d, *J* = 6.3 Hz, 2H, CH_{2 Bn}), 5.44 (s, 1H, H_{Ar}), 6.62 (t, *J* = 5.5 Hz, 1H, NH_{Alk}), 7.19 - 7.41 (m, 5H, 5xH_{Ar}), 7.93 (t, *J* = 6.4 Hz, 1H, NH_{Bn}). |
| | | | **¹³C NMR (101 MHz, DMSO-*d₆*) δ :** 19.6 (2xCH_{3 iPr}), 24.7 (CH_{iPr}), 40.4 (CH_{2-NH Alk}), 45.2 (CH_{2-OCH3}), 57.9 (OCH₃), 70.1 (CH_{2 Bn}), 84.1 (CH_{Ar}), 126.9 (2xCH_{Ar}), 126.9 (CH_{Ar}), 128.4 (2xCH_{Ar}), 138.3 (C_{q}), 139.7 (C_{q}), 139.9 (C_{q}), 152.8 (C_{q}), 155.8 (C_{q}). |
| | | | **MS (ESI+) : m/z calcd for C₁₈H₂₄N₆O** : 341.21 [M+H]+, **found :** 341.33. |
| (13) | 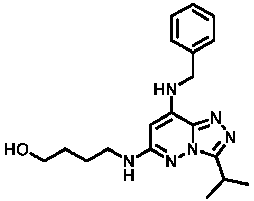 | > 97 % ^{a} | White solid. |
| | | | **¹H NMR (400 MHz, DMSO-*d₆*) δ :** 1.37 (d, *J* = 6.9 Hz, 6H, 2×CH_{3 iPr}), 1.41 - 1.49 (m, 2H, CH_{2-CH2-OH}), 1.49 - 1.59 (m, 2H, CH_{2-CH2-NH}), 3.13 (q, *J* = 6.3 Hz, 2H, CH_{2-NH}), 3.27 - 3.37 (m, 1H, H_{iPr}), 3.39 (q, *J* = 6.1 Hz, 2H, CH_{2-OH}), 4.37 (t, *J* = 5.1 Hz, 1H, OH), 4.43 (d, *J* = 6.4 Hz, 2H, CH_{2 Bn}), 5.38 (s, 1H, H_{Ar}), 6.52 (t, *J* = 5.4 Hz, 1H, NH_{Alk}), 7.18 - 7.41 (m, 5H, 5xH_{Ar}), 7.89 (t, *J* = 6.4 Hz, 1H, NH_{Bn}). |
| | | | **¹³C NMR (101 MHz, DMSO-*d₆*) δ :** 19.6 (2× CH_{3 iPr}), 24.7 (CH_{iPr}), 25.1 (CH_{2-CH2-NH}), 30.2 (CH_{2-CH2-OH}), 40.6 (CH_{2-NH}), 45.2 (CH_{2 Bn}), 60.5 (CH_{2-OH}), 84.1 (CH_{Ar}), 126.8 (2×CH_{Ar}), 126.9 (CH_{Ar}), 128.4 (2xCH_{Ar}), 138.4 (C_{q}), 139.7 (C_{q}), 139.8 (C_{q}), 152.8 (C_{q}), 155.9 (C_{q}). |
| | | | **MS (ESI+) : m/z calcd for C₁₉H₂₆N₆O** : 355.22 [M+H]+, **found** : 355.34. |
| (14) | 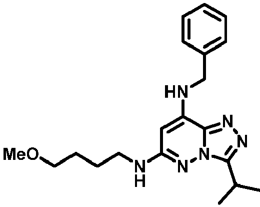 | > 96 % ^{a} | White crystalline solid. |
| | | | **¹H NMR (400 MHz, DMSO-*d₆*) δ :** 1.36 (d, *J* = 6.9 Hz, 6H, 2×CH_{3 iPr}), 1.53 (p, *J* = 3.5 Hz, 4H, 2×CH₂), 3.09 - 3.17 (m, 2H, CH₂), 3.20 (s, 3H, CH_{3-O}), 3.26 - 3.38 (m, 3H, CH₂ & H_{iPr}), 4.43 (d, *J* = 6.3 Hz, 2H, CH_{2 Bn}), 5.37 (s, 1H, H_{Ar}), 6.53 (t, *J* = 5.4 Hz, 1H, NH), 7.19 - 7.40 (m, 5H, 5xH_{Ar}), 7.88 (t, *J* = 6.4 Hz, 1H, NH_{Bn}). |
| | | | **¹³C NMR (101 MHz, DMSO-*d₆*) δ :** 19.6 (2×CH_{3 iPr}), 24.7 (CH_{iPr}), 25.2 (CH₂), 26.7 (CH₂), 40.5 (CH₂), 45.2 (CH_{2 Bn}), 57.8 (CH_{3-O}), 71.6 (CH₂), 84.1 (CH_{Ar}), 126.8 (2xCH_{Ar}), 126.9 (CH_{Ar}), 128.4 (2xCH_{Ar}), 138.3 (C_{q}), 139.7 (C_{q}), 139.8 (C_{q}), 152.8 (C_{q}), 155.9 (C_{q}). |
| | | | **MS (ESI+) : m/z calcd for C₂₀H₂₈N₆O :** 369.24 [M+H]+, **found** : 369.45. |
| (15) | 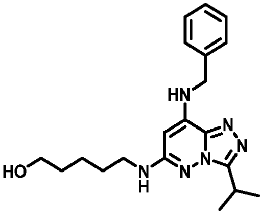 | > 96 % ^{a} | White solid. |
| | | | **¹H NMR (400 MHz, DMSO-*d₆*) δ :** 1.26 - 1.34 (m, 2H, CH_{2-CH2-CH2}), 1.36 (d, *J* = 7.0 Hz, 6H, 2×CH_{3 iPr}), 1.42 (p, *J* = 6.6 Hz, 2H, CH_{2-CH2-OH}), 1.51 (p, *J* = 7.3 Hz, 2H, CH_{2-CH2-NH}), 3.11 (q, *J* = 6.5 Hz, 2H, CH_{2-NH Alk}), 3.32 (s, 1H, H_{iPr}), 3.37 (q, *J* = 6.1 Hz, 2H, CH_{2-OH}), 4.33 (t, *J* = 5.1 Hz, 1H, OH), 4.43 (d, *J* = 6.3 Hz, 2H, CH_{2 Bn}), 5.37 (s, 1H, H_{Ar}), 6.51 (t, *J* = 5.4 Hz, 1H, NH_{Alk}), 7.19 - 7.40 (m, 5H, 5xH_{Ar}), 7.88 (t, *J* = 6.4 Hz, 1H, NH_{Bn}). |
| | | | **¹³C NMR (101 MHz, DMSO-*d₆*) δ :** 19.6 (2×CH_{3 iPr}), 23.2 (CH_{2-CH2-CH2}), 24.7 (CH_{iPr}), 28.4 (CH_{2-CH2-NH}), 32.3 (CH_{2-CH2-OH}), 40.8 (CH_{2-NH Alk}), 45.2 (CH_{2 Bn}), 60.7 (CH_{2-OH}), 84.1 (CH_{Ar}), 126.8 (2xCH_{Ar}), 126.9 (CH_{Ar}), 128.4 (2xCH_{Ar}), 138.4 (C_{q}), 139.7 (C_{q}), 139.8 (C_{q}), 152.8 (C_{q}), 155.9 (C_{q}). |
| | | | **MS (ESI+) : m/z calcd for C₂₀H₂₈N₆O :** 369.24 [M+H]+, **found** : 369.38. |
| (16) | 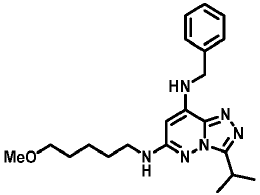 | > 95 % ^{a} | Light yellow solid. |
| | | | **¹H NMR (400 MHz, DMSO-*d₆*) δ :** 1.30 (dt, *J* = 10.2, 7.0 Hz, 2H, CH₂), 1.36 (d, *J* = 6.9 Hz, 6H, 2xCH_{3 iPr}), 1.45 - 1.55 (m, 4H, 2×CH₂), 3.11 (q, *J* = 6.7 Hz, 2H, CH₂), 3.19 (s, 3H, CH_{3-O}), 3.28 (t, *J* = 6.5 Hz, 2H, CH₂), 3.31 - 3.39 (m, 1H, H_{iPr}), 4.43 (d, *J* = 6.3 Hz, 2H, CH_{2 Bn}), 5.37 (s, 1H, H_{Ar}), 6.52 (t, *J* = 5.4 Hz, 1H, NH), 7.19 - 7.38 (m, 5H, 5xH_{Ar}), 7.89 (t, *J* = 6.3 Hz, 1H, NH_{Bn}). |
| | | | **¹³C NMR (101 MHz, DMSO-*d₆*) δ** : 19.6 (2×CH_{3 iPr}), 23.4 (CH₂), 24.7 (CH_{iPr}), 28.3 (CH₂), 28.8 (CH₂), 40.7 (CH₂), 45.2 (CH_{2 Bn}), 57.8 (CH_{3-O}), 71.8 (CH₂), 84.2 (CH_{Ar}), 126.8 (2×CH_{Ar}), 126.9 (CH_{Ar}), 128.4 (2xCH_{Ar}), 138.4 (C_{q}), 139.7 (C_{q}), 139.8 (C_{q}), 152.8 (C_{q}), 155.9 (C_{q}). |
| | | | **MS (ESI+) : m/z calcd for C₂₁H₃₀N₆O** : 383.26 [M+H]+, **found** : 383.45. |
| (17) | 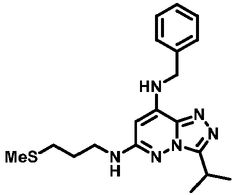 | Unstable ^{a} | Brown solid. |
| | | | **¹H NMR (400 MHz, DMSO-*d₆*) δ :** 1.36 (d, *J* = 6.9 Hz, 6H, 2×CH_{3 iPr}), 1.79 (h, *J* = 7.0 Hz, 2H, CH₂), 2.02 (s, 3H, CH_{3-S}), 3.21 (q, *J* = 6.4 Hz, 2H, CH₂), 3.34 (d, *J* = 6.9 Hz, 1H, H_{iPr}), 4.43 (d, *J* = 6.3 Hz, 2H, CH_{2 Bn}), 5.36 (s, 1H, H_{Ar}), 6.58 (q, *J* = 5.5, 4.5 Hz, 1H, NH), 7.18 - 7.42 (m, 5H, 5xH_{Ar}), 7.91 (t, *J* = 6.4 Hz, 1H, NH_{Bn}). |
| | | | **¹³C NMR (101 MHz, DMSO-*d₆*) δ :** N.D. |
| | | | **MS (ESI+) : m/z calcd for C₁₉H₂₆N₆S** : 371.20 [M+H]+, **found** : 371.27. |
| (18) | 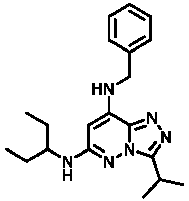 | > 97 % ^{a} | White crystalline solid. |
| | | | **¹H NMR (400 MHz, DMSO-*d₆*) δ :** 0.82 (t, *J* = 7.4 Hz, 6H, 2xCH_{3-CH2}), 1.35 (d, *J* = 6.9 Hz, 6H, 2xCH_{3 iPr}), 1.47 (dtt, *J* = 32.1, 14.4, 7.4 Hz, 4H, 2xCH_{2-CH3}), 3.30 (h, *J* = 6.8 Hz, 1H, H_{iPr}), 3.54 (hept, *J* = 6.0, 5.4 Hz, 1H, H_{Alk-NH}), 4.43 (d, *J* = 6.3 Hz, 2H, CH_{2 Bn}), 5.43 (s, 1H, H_{Ar}), 6.30 (d, *J* = 7.9 Hz, 1H, NH_{Alk}), 7.19 - 7.41 (m, 5H, 5xH_{Ar}), 7.84 (t, *J* = 6.4 Hz, 1H, NH_{Bn}). |
| | | | **¹³C NMR (101 MHz, DMSO-*d₆*) δ :** 10.4 (2xCH_{3-CH2}), 19.5 (2xCH_{3 iPr}), 24.8 (CH_{iPr}), 26.1 (2xCH_{2-CH3}), 45.2 (CH_{2 Bn}), 53.0 (CH_{Alk-NH}), 84.2 (CH_{Ar}), 126.8 (2xCH_{Ar}), 126.9 (CH_{Ar}), 128.4 (2xCH_{Ar}), 138.4 (C_{q}), 139.7 (C_{q}), 139.8 (C_{q}), 152.7 (C_{q}), 155.9 (C_{q}). |
| | | | **MS (ESI+) : m/z calcd for C₂₀H₂₈N₆ :** 353.24 [M+H]+, **found** : 353.29. |
| (19) | 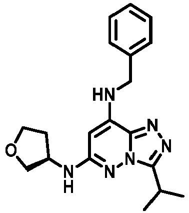 | > 99 % ^{a} | White solid. |
| | | | **¹H NMR (400 MHz, DMSO-*d₆*) δ :** 1.36 (dd, *J* = 7.1, 2.8 Hz, 6H, 2xCH_{3 iPr}), 1.78 (dq, *J* = 12.3, 6.2 Hz, 1H, H_{(CH2)-CH2-O}), 2.13 (dq, *J* = 14.5, 7.4 Hz, 1H, H_{(CH2)-CH2-O}), 3.26 - 3.37 (m, 1H, H_{iPr}), 3.51 (dd, *J* = 8.9, 3.9 Hz, 1H, _{CH-}H_{(CH2)-O}), 3.67 (td, *J* = 8.1, 5.5 Hz, 1H, _{CH2-}H(_{CH2})_{-O}), 3.77 (q, *J* = 7.6 Hz, 1H, _{CH2-}H(_{CH2})_{-O}), 3.85 (dd, *J* = 8.9, 5.8 Hz, 1H, _{CH-}H_{(CH2)-O}), 4.16 (q, *J* = 6.6, 6.0 Hz, 1H, H_{THF}), 4.43 (d, *J* = 6.3 Hz, 2H, CH_{2Bn}), 5.37 (s, 1H, H_{Ar}), 6.81 (d, *J* = 5.5 Hz, 1H, NH_{Alk}), 7.20 - 7.42 (m, 5H, 5xH_{Ar}), 7.98 (t, *J* = 6.4 Hz, 1H, NH_{Bn}). |
| | | | **¹³C NMR (101 MHz, DMSO-*d₆*) δ :** 19.5 (CH_{3 iPr}), 19.6 (CH_{3 iPr}), 24.8 (CH_{iPr}), 32.1 (CH_{2-CH2-O}), 45.2 (CH_{2 Bn}), 51.5 (CH_{THF}), 66.3 (_{CH2-}CH_{2-O}), 72.7 (_{CH-}CH_{2-O}), 84.0 (CH_{Ar}), 126.8 (2xCH_{Ar}), 126.9 (CH_{Ar}), 128.5 (2xCH_{Ar}), 138.3 (C_{q}), 139.6 (C_{q}), 140.0 (C_{q}), 152.9 (C_{q}), 155.5 (C_{q}). |
| | | | **MS (ESI+) : m/z calcd for C₁₉H₂₄N₆O :** 353.21 [M+H]+, **found :** 353.26. |
| (20) | 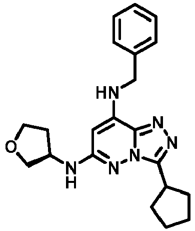 | > 96 % ^{a} | White solid. |
| | | | **¹H NMR (400 MHz, DMSO-*d₆*) δ :** 1.59 - 1.84 (m, 5H, H_{(CH2) THF} 2xCH_{2 cPent}), 1.99 (dddd, *J* = 27.3, 12.3, 7.7, 4.3 Hz, 4H, CH_{2 THF} & CH_{2 cPent}), 2.13 (dq, *J* = 15.0, 7.6 Hz, 1H, H(_{CH2}) _{THF}), 3.44 (p, *J* = 8.2 Hz, 1H, H_{cPent}), 3.51 (dd, *J* = 8.9, 3.8 Hz, 1H, H(_{CH2}) _{THF}), 3.67 (td, *J* = 8.1, 5.6 Hz, 1H, H(_{CH2}) _{THF}), 3.76 (q, *J* = 7.6 Hz, 1H, H(_{CH2}) _{THF}), 3.83 (dd, *J* = 8.9, 5.8 Hz, 1H, H(_{CH2}) _{THF}), 4.14 (dq, *J* = 9.9, 4.9 Hz, 1H, H_{THF}), 4.43 (d, *J* = 6.3 Hz, 2H, CH_{2 Bn}), 5.37 (s, 1H, H_{Ar}), 6.80 (d, *J* = 5.5 Hz, 1H, NH_{THF}), 7.17 - 7.45 (m, 5H, 5xH_{Ar}), 7.96 (t, *J* = 6.3 Hz, 1H, NH_{Bn}). |
| | | | **¹³C NMR (101 MHz, DMSO-*d₆*) δ :** 25.3 (2xCH_{2 cPent}), 29.6 (CH_{2 ePent}), 29.7 (CH_{2 cPent}), 32.0 (CH_{2 THF}), 34.9 (CH_{2 cPent}), 45.3 (CH2 _{Bn}), 51.5 (CHTHF), 66.3 (CH_{2 THF}), 72.7 (CH_{2 THF}), 84.0 (CH_{Ar}), 126.7 (2xCH_{Ar}), 126.9 (CH_{Ar}), 128.4 (2xCH_{Ar}), 138.3 (C_{q}), 139.7 (C_{q}), 140.0 (C_{q}), 151.8 (C_{q}), 155.4 (C_{q}). |
| | | | **MS (ESI+) : m/z calcd for C₂₁H₂₆N₆O :** 379.22 [M+H]+, **found :** 379.29. |
| (21) | 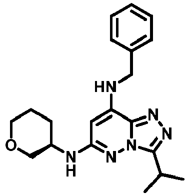 | > 96 % ^{a} | White solid. |
| | | | **¹H NMR (400 MHz, DMSO-*d₆*) δ :** 1.35 (dd, *J* = 7.2, 4.2 Hz, 6H, 2xCH_{3 iPr}), 1.42 - 1.58 (m, 2H, H_{(CH2)-CH2-O} & H_{(CH2)-CH THP}), 1.61 - 1.75 (m, 1H, H_{(CH2)-CH THP}), 1.85 - 2.00 (m, 1H, H(CH2)-CH2-o), 3.08 (dd, *J* = 10.6, 8.2 Hz, 1H, _{O-}H_{(CH2)-CH THP}), 3.33 (m, 2H, H_{iPr} & _{CH2-}H(_{CH2})_{-O}), 3.62 - 3.72 (m, 2H, H_{THP} & _{CH2-}H(_{CH2})_{-O}), 3.91 (dd, *J* = 11.0, 3.8 Hz, 1H, _{O-}H_{(CH2)-CH THP}), 4.42 (d, *J* = 6.3 Hz, 2H, CH_{2 Bn}), 5.42 (s, 1H, H_{Ar}), 6.50 (d, *J* = 7.1 Hz, 1H, NH_{Alk}), 7.16 - 7.38 (m, 5H, 5xH_{Ar}), 7.94 (t, *J* = 6.4 Hz, 1H, NH_{Bn}). |
| | | | **¹³C NMR (101 MHz, DMSO-*d₆*) δ :** 19.6 (CH_{3 iPr}), 19.7 (CH_{3 iPr}), 24.2 (CH_{2-CH THP}), 24.7 (CH_{iPr}), 28.7 (CH_{2-CH2-O}), 45.2 (CH_{2 Bn}), 46.6 (CH_{THP}), 67.1 (_{CH2-}CH_{2-O}), 70.3 (_{CH-}CH_{2-O}), 84.1 (CH_{Ar}), 126.8 (2xCH_{Ar}), 126.9 (CH_{Ar}), 128.5 (2xCH_{Ar}), 138.3 (C_{q}), 139.7 (C_{q}), 140.0 (C_{q}), 152.8 (C_{q}), 155.2 (C_{q}). |
| | | | **MS (ESI+) : m/z calcd for C₂₀H₂₆N₆O :** 367.22 [M+H]+, **found :** 367.30. |
| (22) | 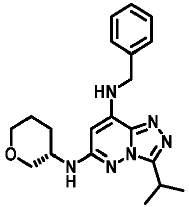 | > 96 % ^{a} | White solid. |
| | | | **¹H NMR (400 MHz, DMSO-*d₆*) δ :** 1.35 (dd, *J* = 7.0, 4.4 Hz, 6H, 2xCH_{3 iPr}), 1.43 - 1.59 (m, 2H, H_{(CH2)-CH2-O} & H_{(CH2)-CH THP}), 1.64 - 1.74 (m, 1H, H_{(CH2)-CH THP}), 1.84 - 1.96 (m, 1H, H_{(CH2)-CH2-O}), 3.08 (dd, *J* = 10.7, 8.1 Hz, 1H, _{O-}H_{(CH2)-CH THP}), 3.26 - 3.38 (m, 2H, H_{iPr} & _{CH2-}H(_{CH2})_{-O}), 3.61 - 3.76 (m, 2H, H_{THP} & _{CH2-}H(_{CH2})_{-O}), 3.91 (dd, *J* = 11.2, 3.8 Hz, 1H, _{O-}H_{(CH2)-CH THP}), 4.42 (d, *J* = 6.4 Hz, 2H, CH_{2 Bn}), 5.42 (s, 1H, H_{Ar}), 6.50 (d, *J* = 7.1 Hz, 1H, NH_{Alk}), 7.21 - 7.36 (m, 5H, 5xH_{Ar}), 7.94 (t, *J* = 6.3 Hz, 1H, NH_{Bn}). |
| | | | **¹³C NMR (101 MHz, DMSO-*d₆*) δ :** 19.6 (CH_{3 iPr}), 19.7 (CH_{3 iPr}), 24.2 (CH_{2-CH THP}), 24.7 (CH_{iPr}), 28.6 (CH_{2-CH2-O}), 45.2 (CH_{2 Bn}), 46.6 (CH_{THP}), 67.1 (CH_{2-CH2-O}), 70.3 (_{CH-}CH_{2-O}), 84.1 (CH_{Ar}), 126.8 (2xCH_{Ar}), 126.9 (CH_{Ar}), 128.4 (2xCH_{Ar}), 138.3 (C_{q}), 139.6 (C_{q}), 140.0 (C_{q}), 152.8 (C_{q}), 155.2 (C_{q}). |
| | | | **MS (ESI+) : m/z calcd for C₂₀H₂₆N₆O :** 367.22 [M+H]+, **found :** 367.30. |
| (23) | 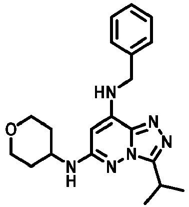 | > 96 % ^{a} | White solid. |
| | | | **¹H NMR (400 MHz, DMSO-*d₆*) δ :** 1.35 (d, *J* = 7.0 Hz, 8H, 2xCH3 iPr & CH_{2-CH THP}), 1.90 (d, *J* = 12.5 Hz, 2H, CH_{2-CH THP}), 3.32 (s, 1H, H_{iPr}), 3.37 (td, *J* = 10.2, 9.0, 2.7 Hz, 2H, CH_{2-O}), 3.64 - 3.79 (m, 1H, H_{THP}), 3.83 (dt, *J* = 11.7, 3.7 Hz, 2H,CH_{2-O}), 4.43 (d, *J* = 6.3 Hz, 2H, CH_{2Bn}), 5.37 (s, 1H, H_{Ar}), 6.52 (d, *J* = 6.9 Hz, 1H, NH_{THP}), 7.19 - 7.39 (m, 5H, 5xH_{Ar}), 7.93 (t, *J* = 6.4 Hz, 1H, NH_{Bn}). |
| | | | **¹³C NMR (101 MHz, DMSO-*d₆*) δ :** 19.6 (2xCH_{3 iPr}), 24.7 (CH_{iPr}), 32.3 (2xCH_{2-CH THP}), 45.2 (CH_{2 Bn}), 46.8 (CH_{THP}), 65.9 (2xCH_{2-O}), 84.1 (CH_{Ar}), 126.7 (2xCH_{Ar}), 126.9 (CH_{Ar}), 128.5 (2xCH_{Ar}), 138.3 (C_{q}), 139.6 (C_{q}), 140.0 (C_{q}), 152.8 (C_{q}), 155.0 (C_{q}). |
| | | | **MS (ESI+) : m/z calcd for C₂₀H₂₆N₆O :** 367.22 [M+H]+, **found :** 367.27. |
| (24) | 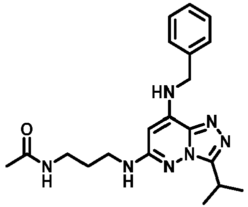 | > 96 % ^{a} | White solid |
| | | | **¹H NMR (400 MHz, DMSO-*d₆*) δ :** 1.37 (d, *J* = 6.9 Hz, 6H, 2xCH_{3 iPr}), 1.65 (p, *J* = 6.9 Hz, 2H, CH_{2-CH2-NH}), 1.78 (s, 3H, CH_{3-CO}), 3.08 (q, *J* = 6.6 Hz, 2H, CH_{2-NH}), 3.15 (q, *J* = 6.5 Hz, 2H, CH_{2-NH}), 3.33 (q, *J* = 7.0 Hz, 1H, H_{iPr}), 4.46 (d, *J* = 6.3 Hz, 2H, CH_{2 Bn}), 5.39 (s, 1H, H_{Ar}), 6.55 (t, *J* = 5.4 Hz, 1H, NH_{Alk}), 7.19 - 7.41 (m, 5H, 5xH_{Ar}), 7.83 (t, *J* = 5.6 Hz, 1H, NH_co), 7.95 (t, *J* = 6.3 Hz, 1H, NH_{Bn}). |
| | | | **¹³C NMR (101 MHz, DMSO-*d₆*) δ :** 19.7 (2xCH_{3 iPr}), 22.6 (CH_{3-CO}), 24.7 (CH_{iPr}), 28.6 (CH_{2-CH2-NH}), 36.7 (CH_{2-NH}), 38.58 (CH_{2-NH}), 45.2 (CH_{2 Bn}), 84.2 (CH_{Ar}), 126.8 (2xCH_{Ar}), 126.9 (CH_{Ar}), 128.4 (2xCH_{Ar}), 138.3 (C_{q}), 139.7 (C_{q}), 139.9 (C_{q}), 152.9 (C_{q}), 155.9 (C_{q}), 169.0 (C_{q}). |
| | | | **MS (ESI+) : m/z calcd for C₂₀H₂₇N₇O :** 382.23 [M+H]+, **found :** 382.32. |
| (25) | 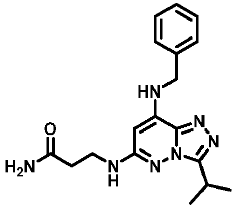 | > 99 % ^{a} | White solid. |
| | | | **¹H NMR (400 MHz, DMSO-*d₆*) δ :** 1.36 (d, *J* = 6.9 Hz, 6H, 2xCH_{3 iPr}), 2.33 (t, *J* = 6.9 Hz, 2H, CH_{2-CO}), 3.27 - 3.42 (m, 3H, H_{iPr} & CH_{2-NH}), 4.42 (d, *J* = 6.3 Hz, 2H, CH_{2 Bn}), 5.41 (s, 1H, H_{Ar}), 6.55 (t, *J* = 5.5 Hz, 1H, NH), 6.78 (s, 1H, H_{amide}), 7.23 (tt, *J* = 5.7, 2.6 Hz, 1H, H_{Ar}), 7.27 (s, 1H, H_{amide}), 7.30 - 7.37 (m, 4H, 4xH_{Ar}), 7.90 (t, *J* = 6.4 Hz, 1H, NH_{Bn}). |
| | | | **¹³C NMR (101 MHz, DMSO-*d₆*) δ :** 19.7 (2xCH_{3 iPr}), 24.6 (CH_{iPr}), 34.3 (CH_{2-NH}), 37.2 (CH_{2-CO}), 45.2 (CH_{2 Bn}), 84.2 (CH_{Ar}), 126.8 (2xCH_{Ar}), 126.9 (CH_{Ar}), 128.4 (2xCH_{Ar}), 138.3 (C_{q}), 139.7 (C_{q}), 139.8 (C_{q}), 152.9 (C_{q}), 155.8 (C_{q}), 172.8 (C_{q}). |
| | | | **MS (ESI+) : m/z calcd for C₁₈H₂₃N₇O :** 354.20 [M+H]+, **found :** 354.30. |
| (26) | 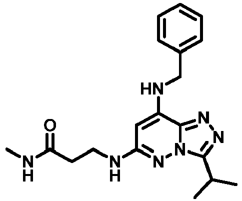 | > 99 % ^{a} | White solid. |
| | | | **¹H NMR (400 MHz, DMSO-*d₆*) δ :** 1.36 (d, *J* = 6.9 Hz, 6H, 2xCH_{3 iPr}), 2.34 (t, *J* = 6.8 Hz, 2H, CH_{2-CO}), 2.54 (d, *J* = 4.6 Hz, 3H, CH_{3-NH}), 3.28 - 3.39 (m, 3H, H_{iPr} & CH_{2-NH}), 4.42 (d, *J* = 6.3 Hz, 2H, CH_{2 Bn}), 5.40 (s, 1H, H_{Ar}), 6.56 (t, *J* = 5.6 Hz, 1H, NH), 7.19 - 7.39 (m, 5H, 5xH_{Ar}), 7.73 (d, *J* = 4.9 Hz, 1H, NH_co), 7.90 (t, *J* = 6.3 Hz, 1H, NH_{Bn}). |
| | | | **¹³C NMR (101 MHz, DMSO-*d₆*) δ :** 19.7 (2xCH_{3 iPr}), 24.6 (CH_{iPr}), 25.4 (CH_{3-NH}), 34.6 (CH_{2-CO}), 37.3 (CH_{2-NH}), 45.2 (CH_{2 Bn}), 84.2 (CH_{Ar}), 126.8 (2xCH_{Ar}), 126.9 (CH_{Ar}), 128.4 (2xCH_{Ar}), 138.3 (C_{q}), 139.7 (C_{q}), 139.9 (C_{q}), 152.9 (C_{q}), 155.7 (C_{q}), 171.0 (C_{q}). |
| | | | **MS (ESI+) : m/z calcd for C₁₉H₂₅N₇O :** 368.46 [M+H]+, **found :** 368.31. |
| (27) | 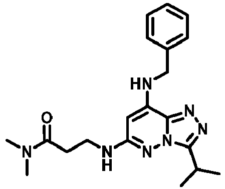 | > 99 % ^{a} | White solid. |
| | | | **¹H NMR (400 MHz, DMSO-*d₆*) δ :** 1.36 (d, *J* = 6.9 Hz, 6H, 2xCH_{3 iPr}), 2.56 (t, *J* = 7.0 Hz, 2H, CH_{2-CO}), 2.80 (s, 3H, CH_{3-N}), 2.92 (s, 3H, CH_{3-N}), 3.29 - 3.38 (m, 3H, H_{iPr} & CH_{2-NH}), 4.42 (d, *J* = 6.3 Hz, 2H, CH_{2 Bn}), 5.39 (s, 1H, H_{Ar}), 6.57 (t, *J* = 5.6 Hz, 1H, NH), 7.18 - 7.39 (m, 5H, 5xH_{Ar}), 7.92 (t, *J* = 6.4 Hz, 1H, NH_{Bn}). |
| | | | **¹³C NMR (101 MHz, DMSO-de) δ :** 19.7 (2xCH_{3 iPr}), 24.6 (CH_{iPr}), 32.0 (CH_{2-CO}), 34.6 (CH_{3-N}), 36.6 (CH_{3-N}), 37.1 (CH_{2-NH}), 45.2 (CH_{2 Bn}), 84.2 (CH_{Ar}), 126.8 (2xCH_{Ar}), 126.9 (CH_{Ar}), 128.4 (CH_{Ar}), 138.3 (C_{q}), 139.7 (C_{q}), 139.9 (C_{q}), 152.9 (C_{q}), 155.8 (C_{q}), 170.6 (C_{q}). |
| | | | **MS (ESI+) : m/z calcd for C₂₀H₂₇N₇O :** 382.23 [M+H]+, **found :** 382.32. |
| (28) | 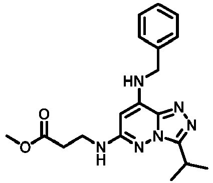 | > 98 % ^{a} | White solid. |
| | | | **¹H NMR (400 MHz, DMSO-*d₆*) δ :** 1.37 (d, *J* = 6.9 Hz, 6H, 2xCH_{3 iPr}), 2.60 (t, *J* = 6.6 Hz, 2H, CH_{2-CO}), 3.35 (m, 3H, H_{iPr} & CH_{2-NH}), 3.58 (s, 3H, OCH₃), 4.43 (d, *J* = 6.2 Hz, 2H, CH_{2Bn}), 5.39 (s, 1H, H_{Ar}), 6.72 (d, *J* = 5.6 Hz, 1H, NH), 7.19 - 7.38 (m, 5H, 5xH_{Ar}), 7.98 (t, *J* = 6.3 Hz, 1H, NH_{Bn}). |
| | | | **¹³C NMR (101 MHz, DMSO-*d₆*) δ :** 19.5 (2xCH_{3 iPr}), 24.7 (CH_{iPr}), 33.0 (CH_{2-CO}), 36.8 (CH_{2-NH}), 45.2 (CH_{2 Bn}), 51.3 (CH_{3-O}), 84.3 (CH_{Ar}), 126.8 (2xCH_{Ar}), 126.9 (CH_{Ar}), 128.4 (2xCH_{Ar}), 138.2 (C_{q}), 139.6 (C_{q}), 139.9 (C_{q}), 152.8 (C_{q}), 155.8 (C_{q}), 172.0 (C_{q}). |
| | | | **MS (ESI+) : m/z calcd for C₁₉H₂₄N₆O₂ :** 369.20 [M+H]+, **found :** 369.38. |
| (29) | 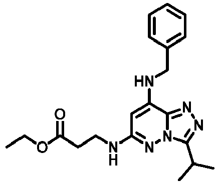 | > 98 % ^{a} | White solid. |
| | | | **¹H NMR (400 MHz, DMSO-*d₆*) δ :** 1.14 (t, *J* = 7.1 Hz, 3H, CH_{3-CH2}), 1.36 (d, *J* = 6.9 Hz, 6H, 2xCH_{3 iPr}), 2_{.}57 (t, *J* = 6.7 Hz, 2H, CH_{2-CO}), 3.29 - 3.41 (m, 3H, H_{iPr} & CH_{2-NH}), 4.03 (q, *J* = 7.1 Hz, 2H, CH_{2-CH3}), 4.43 (d, *J* = 6.3 Hz, 2H, CH_{2 Bn}), 5.37 (s, 1H, H_{Ar}), 6.67 (t, *J* = 5.5 Hz, 1H, NH), 7.19 - 7.38 (m, 5H, 5xH_{Ar}), 7.96 (t, *J* = 6.4 Hz, 1H, NH_{Bn}). |
| | | | **¹³C NMR (101 MHz, DMSO-*d₆*) δ :** 14.1 (CH_{3-CH2}), 19.6 (2xCH_{3 iPr}), 24.7 (CH_{iPr}), 33.2 (CH_{2-CO}), 36.8 (CH_{2-NH}), 45.2 (CH_{2 Bn}), 59.8 (CH_{2-CH3}), 84.0 (CH_{Ar}), 126.8 (2xCH_{Ar}), 126.9 (CH_{Ar}), 128.4 (2xCH_{Ar}), 138.3 (C_{q}), 139.7 (C_{q}), 140.0 (C_{q}), 152.9 (C_{q}), 155.7 (C_{q}), 171.6 (C_{q}). |
| | | | **MS (ESI+) : m/z calcd for C₂₀H₂₆N₆O₂ :** 383.22 [M+H]+, **found :** 383.30 |
| (30) | 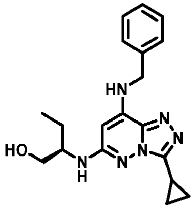 | > 97 % ^{a} | Beige solid. |
| | | | **¹H NMR (400 MHz, DMSO-*d₆*) δ :** 0.85 (t, *J* = 7.4 Hz, 3H, CH_{3 Alk}), 1.03 (dt, *J* = 8.7, 3.1 Hz, 2H, CH_{2 cPr}), 1.11 (dq, *J* = 5.2, 3.2, 2.6 Hz, 2H, CH_{2 cPr}), 1.51 (ddq, *J* = 79.0, 14.1, 7.3, 6.7 Hz, 2H, CH_{2 Alk}), 2.24 (tt, *J* = 8.3, 5.1 Hz, 1H, H_{cPr}), 3.40 (ddt, *J* = 22.1, 11.0, 5.6 Hz, 2H, CH_{2 Alk}), 3.64 (ddt, *J* = 13.0, 7.5, 5.4 Hz, 1H, H_{Alk}), 4.42 (dd, *J* = 6.3, 2.2 Hz, 2H, CH_{2 Bn}), 4.58 (t, *J* = 5.4 Hz, 1H, OH), 5.46 (s, 1H, H_{Ar}), 6.28 (d, *J* = 7.9 Hz, 1H, NH_{Alk}), 7.24 (td, *J* = 5.9, 2.7 Hz, 1H, H_{Ar}), 7.29 - 7.39 (m, 4H, 4xH_{Ar}), 7.83 (t, *J* = 6.4 Hz, 1H, NH_{Bn}). |
| | | | **¹³C NMR (101 MHz, DMSO-*d₆*) δ :** 5.3 (CH_{cPr}), 6.5 (CH_{2 cPr}), 6.5 (CH_{2 cPr}), 10.5 (CH_{3 Alk}), 23.7 (CH_{2 Alk}), 45.2 (CH_{2 Bn}), 53.6 (CH_{Alk}), 62.1 (CH_{2 Alk}), 84.4 (CH_{Ar}), 126.8 (2xCH_{Ar}), 126.9 (CH_{Ar}), 128.4 (2xCH_{Ar}), 138.4 (C_{q}), 139.6 (C_{q}), 139.7 (C_{q}), 149.9 (C_{q}), 156.1 (C_{q}). |
| | | | **MS (ESI+) : m/z calcd for C₁₉H₂₄N₆O :** 353.21 [M+H]+, **found :** 353.36. |
| (31) | 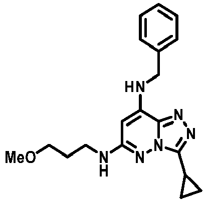 | > 97 % ^{a} | White solid. |
| | | | **¹H NMR (400 MHz, DMSO-*d₆*) δ :** 1.00 - 1.12 (m, 4H, 2xCH_{2 iPr}), 1.74 (p, *J* = 6.7 Hz, 2H, CH_{2-CH2-NH}), 2.25 (tt, *J* = 9.1, 5.3 Hz, 1H, H_{cPr}), 3.17 (q, *J* = 6.5 Hz, 2H, CH_{2-NH}), 3.21 (s, 3H, OCH₃), 3.36 (t, *J* = 6.4 Hz, 2H, CH_{2-OCH3}), 4.42 (d, *J* = 6.3 Hz, 2H, CH_{2Bn}), 5.36 (s, 1H, H_{Ar}), 6.52 (t, *J* = 5.4 Hz, 1H, NH_{Alk}), 7.24 (q, *J* = 4.5 Hz, 1H, H_{Ar}), 7.33 (d, *J* = 4.4 Hz, 4H, 4xH_{Ar}), 7.87 (t, *J* = 6.4 Hz, 1H, NH_{Bn}). |
| | | | **¹³C NMR (101 MHz, DMSO-*d₆*) δ :** 5.2 (CH_{cPr}), 6.6 (2cCH_{2 cPr}), 28.6 (CH_{2-CH2-NH}), 38.0 (CH_{2-NH}), 45.2 (CH_{2 Bn}), 57.8 (OCH₃), 69.8 (CH_{2-OCH3}), 84.1 (CH_{Ar}), 126.8 (2xCH_{Ar}), 126.9 (CH_{Ar}), 128.4 (2xCH_{Ar}), 138.3 (C_{q}), 139.6 (C_{q}), 139.8 (C_{q}), 150.1 (C_{q}), 156.2 (C_{q}). |
| | | | **MS (ESI+) : m/z calcd for C₁₉H₂₄N₆O :** 353.21 [M+H]+, **found :** 353.32. |
| (32) | 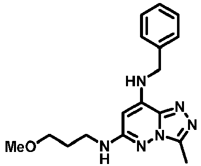 | > 98 % ^{a} | White solid. |
| | | | **¹H NMR (400 MHz, DMSO-*d₆*) δ :** 1.74 (p, *J* = 6.6 Hz, 2H, CH_{2-CH2}), 2.46 (s, 3H, CH₃), 3.17 (q, *J* = 6.5 Hz, 2H, CH_{2-NH}), 3.21 (s, 3H, OCH₃), 3.36 (t, *J* = 6.3 Hz, 2H, CH_{2-OCH3}), 4.44 (d, *J* = 6.3 Hz, 2H, CH_{2Bn}), 5.38 (s, 1H, H_{Ar}), 6.53 (t, *J* = 5.3 Hz, 1H, NH_{Alk}), 7.21 - 7.39 (m, 5H, 5xH_{Ar}), 7.88 (t, *J* = 6.3 Hz, 1H, NH_{Bn}). |
| | | | **¹³C NMR (101 MHz, DMSO-*d₆*) δ :** 9.4 (CH₃), 28.6 (CH_{2-CH2}), 38.0 (CH_{2-NH}), 45.2 (CH_{2 Bn}), 57.8 (OCH₃), 69.8 (CH_{2-OCH3}), 84.1 (CH_{Ar}), 126.8 (2xCH_{Ar}), 126.9 (CH_{Ar}), 128.4 (2xCH_{Ar}), 138.3 (C_{q}), 139.4 (C_{q}), 139.8 (C_{q}), 145.6 (C_{q}), 156.3 (C_{q}). |
| | | | **MS (ESI+) : m/z calcd for C₁₇H₂₂N₆O :** 327.19 [M+H]+, **found :** 327.32. |
| (33) | 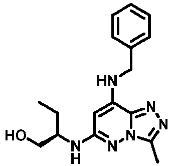 | > 97 % ^{a} | White solid. |
| | | | **¹H NMR (400 MHz, DMSO-*d₆*) δ :** 0.85 (t, *J* = 7.4 Hz, 3H, CH_{3 Alk}), 1.50 (dtt, *J* = 91.5, 14.5, 7.2 Hz, 2H, CH_{2-CH3}), 2.45 (s, 3H, CH₃), 3.35 - 3.47 (m, 2H, CH_{2-OH}), 3.66 (h, *J* = 6.0 Hz, 1H, H_{Alk-NH}), 4.36 - 4.50 (m, 2H, CH_{2 Bn}), 4.58 (t, *J* = 5.4 Hz, 1H, OH), 5.48 (s, 1H, H_{Ar}), 6.29 (d, *J* = 7.9 Hz, 1H, NH_{Alk}), 7.19 - 7.41 (m, 5H, 5xH_{Ar}), 7.84 (t, *J* = 6.4 Hz, 1H, NH_{Bn}). |
| | | | **¹³C NMR (101 MHz, DMSO-*d₆*) δ :** 9.4 (CH₃), 10.6 (CH_{3 Alk}), 23.7 (CH₂), 45.3 (CH_{2 Bn}), 53.6 (CH_{Alk}), 62.1 (CH₂), 84.4 (H_{Ar}), 126.8 (2xCH_{Ar}), 126.9 (CH_{Ar}), 128.4 (2xCH_{Ar}), 138.4 (C_{q}), 139.4 (C_{q}), 139.8 (C_{q}), 145.5 (C_{q}), 156.2 (C_{q}). |
| | | | **MS (ESI+) : m/z calcd for C₁₇H₂₂N₆O :** 327.19 [M+H]+, **found :** 327.32. |
| (34) | 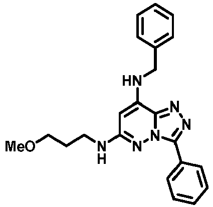 | > 99 % ^{a} | Pale yellow solid. |
| | | | **¹H NMR (400 MHz, DMSO-*d₆*) δ :** 1.79 (p, *J* = 6.6 Hz, 2H, CH_{2-CH2}), 3.20 - 3.29 (m, 5H, CH_{2-NH} & CH₃), 3.39 (t, *J* = 6.3 Hz, 2H, CH_{2-OCH3}), 4.48 (d, *J* = 6.3 Hz, 2H, CH_{2 Bn}), 5.48 (s, 1H, H_{Ar}), 6.75 (t, *J* = 5.4 Hz, 1H, NH_{Alk}), 7.22 - 7.28 (m, 1H, H_{Ar}), 7.32 - 7.40 (m, 4H, 4xH_{Ar}), 7.44 - 7.50 (m, 1H, H_{Ar}), 7.53 (dd, *J* = 8.3, 6.6 Hz, 2H, 2xH_{Ar}), 8.07 (t, *J* = 6.3 Hz, 1H, NH_{Bn}), 8.43 - 8.56 (m, 2H, 2xH_{Ar}). |
| | | | **¹³C NMR (101 MHz, DMSO-*d₆*) δ :** 28.5 (CH_{2-CH2}), 38.1 (CH_{2-NH}), 45.3 (CH_{2 Bn}), 57.9 (OCH₃), 69.8 (CH_{2-OCH3}), 84.3 (CH_{Ar}), 126.5 (2xCH_{Ar}), 126.8 (2xCH_{Ar}), 126.9 (CH_{Ar}), 127.5 (C_{q}), 128.5 (2xCH_{Ar}), 128.5 (2xCH_{Ar}), 129.1 (CH_{Ar}), 138.2 (C_{q}), 139.9 (C_{q}), 140.6 (C_{q}), 146.3 (C_{q}), 156.5 (C_{q}). |
| | | | **MS (ESI+) : m/z calcd for C₂₂H₂₄N₆O :** 389.48 [M+H]+, **found :** 389.34. |
| (35) | 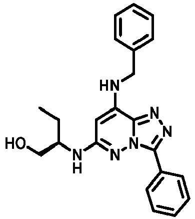 | > 99 % ^{a} | White solid. |
| | | | **¹H NMR (400 MHz, DMSO-*d₆*) δ :** 0.90 (t, *J* = 7.4 Hz, 3H, CH₃), 1.56 (ddq, *J* = 50.3, 14.0, 7.0 Hz, 2H, CH_{2-CH3}), 3.47 (dq, *J* = 14.3, 5.4 Hz, 2H, CH_{2-OH}), 3.67 (h, *J* = 5.7 Hz, 1H, H_{Alk}), 4.48 (d, *J* = 6.4 Hz, 2H, CH_{2 Bn}), 4.64 (t, *J* = 5.4 Hz, 1H, OH), 5.60 (s, 1H, H_{Ar}), 6.52 (d, *J* = 7.6 Hz, 1H, NH_{Alk}), 7.26 (t, *J* = 7.0 Hz, 1H, H_{Ar}), 7.33 - 7.43 (m, 4H, 4xH_{Ar}), 7.47 (t, *J* = 7.3 Hz, 1H, H_{Ar}), 7.54 (dd, *J* = 8.4, 6.7 Hz, 2H, 2xH_{Ar}), 8.04 (t, *J* = 6.4 Hz, 1H, NH_{Bn}), 8.41 - 8.54 (m, 2H, 2xH_{Ar}). |
| | | | **¹³C NMR (101 MHz, DMSO-*d₆*) δ :** 10.7 (CH₃), 23.6 (CH₂), 45.3 (CH_{2 Bn}), 54.3 (CH_{Alk}), 61.9 (CH₂), 84.6 (CH_{Ar}), 126.5 (2xCH_{Ar}), 126.9 (2xCH_{Ar}), 127.0 (CH_{Ar}), 127.5 (C_{q}), 128.5 (2xCH_{Ar}), 128.5 (2xCH_{Ar}), 129.1 (CH_{Ar}), 138.3 (C_{q}), 139.8 (C_{q}), 140.6 (C_{q}), 146.3 (C_{q}), 156.4 (C_{q}). |
| | | | **MS (ESI+) : m/z calcd for C₂₂H₂₄N₆O :** 389.48 [M+H]+, [M+H]+, **found :** 389.34. |
| (36) | 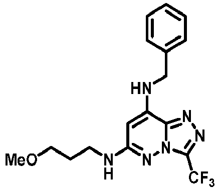 | > 97 % ^{a} | White solid. |
| | | | **¹H NMR (400 MHz, DMSO-*d₆*) δ :** 1.73 (p, *J* = 6.6 Hz, 2H, CH_{2-CH2}), 3.15 (q, *J* = 6.6 Hz, 2H, CH_{2-NH}), 3.20 (s, 3H, OCH₃), 3.35 (t, *J* = 6.3 Hz, 2H, CH_{2-OCH3}), 4.47 (d, *J* = 6.3 Hz, 2H, CH_{2 Bn}), 5.55 (s, 1H, H_{Ar}), 6.94 (t, *J* = 5.3 Hz, 1H, NH_{Alk}), 7.22 - 7.40 (m, 5H, 5xH_{Ar}), 8.31 (t, *J* = 6.3 Hz, 1H, NH_{Bn}). |
| | | | **¹³C NMR (101 MHz, DMSO-*d₆*) δ :** 28.3 (CH_{2-CH2}), 37.9 (CH_{2-NH}), 45.3 (CH_{2 Bn}), 57.8 (OCH₃), 69.6 (CH_{2-OCH3}), 85.5 (CH_{Ar}), 118.7 (q, *J* = 269.1 Hz, CF₃), 126.8 (2xCH_{Ar}), 127.0 (CH_{Ar}), 128.5 (2xCH_{Ar}), 137.8 (C_{q}), 137.8 (q, *J=* 39.3, 38.3 Hz, C_{q-CF3}), 139.3 (C_{q}), 141.7 (C_{q}), 157.3 (C_{q}). |
| | | | **MS (ESI+) : m/z calcd for C₁₇H₁₉F₃N₆O :** 381.16 [M+H]+, **found :** 381.31. |
| (37) | 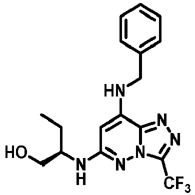 | > 97 % ^{a} | White solid. |
| | | | **¹H NMR (400 MHz, DMSO-*d₆*) δ :** 0.84 (t, *J* = 7.4 Hz, 3H, CH₃), 1.50 (ddq, *J* = 66.5, 14.1, 7.0 Hz, 2H, CH_{2-CH3}), 3.42 (t, *J* = 5.3 Hz, 2H, CH_{2-OH}), 3.60 (h, *J* = 6.3, 5.9 Hz, 1H, H_{Alk}), 4.40 - 4.55 (m, 2H, CH_{2 Bn}), 4.62 (t, *J* = 5.3 Hz, 1H, OH), 5.67 (s, 1H, H_{Ar}), 6.74 (d, *J* = 7.8 Hz, 1H, NH_{Alk}), 7.23 - 7.43 (m, 5H, 5xH_{Ar}), 8.28 (t, *J* = 6.4 Hz, 1H, NH_{Bn}). |
| | | | **¹³C NMR (101 MHz, DMSO-*d₆*) δ :** 10.4 (CH₃), 23.5 (CH_{2-CH3}), 45.3 (CH_{2 Bn}), 53.9 (CH_{Alk}), 61.7 (CH_{2-OH}), 85.8 (CH_{Ar}), 118.8 (d, *J* = 269.1 Hz, CF₃), 126.9 (2xCH_{Ar}), 127.0 (CH_{Ar}), 128.5 (2xCH_{Ar}), 137.7 (d, *J* = 39.5 Hz, C_{q-CF3}), 137.9 (C_{q}), 139.2 (C_{q}), 141.7 (C_{q}), 157.3 (C_{q}). |
| | | | **MS (ESI+) : m/z calcd for C₁₇H₁₉F₃N₆O :** 381.16 [M+H]+, **found :** 381.31. |
| (38) | 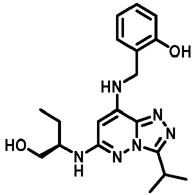 | > 96 % ^{a} | Beige solid. |
| | | | **¹H NMR (400 MHz, DMSO-*d₆*) δ :** 0.85 (t, *J* = 7.4 Hz, 3H, CH_{3-CH2}), 1.36 (d, *J* = 6.9 Hz, 6H, 2xCH_{3 iPr}), 1.39 - 1.68 (m, 2H, CH_{2-CH3}), 3.24 - 3.48 (m, 3H, H_{iPr} & CH_{2-OH}), 3.63 (dp, *J* = 9.4, 5.7 Hz, 1H, H_{Alk}), 4.36 (d, *J* = 6.2 Hz, 2H, CH_{2Bn}), 4.55 (d, *J* = 5.6 Hz, 1H, OH_{-CH2}), 5.44 (s, 1H, H_{Ar}), 6.29 (d, *J* = 7.7 Hz, 1H, NH_{Alk}), 6.73 (t, *J* = 7.4 Hz, 1H, H_{Ar}), 6.84 (d, *J* = 7.8 Hz, 1H, H_{Ar}), 7.00 - 7.17 (m, 2H, 2xH_{Ar}), 7.48 (t, *J* = 6.3 Hz, 1H, NH_{Bn}), 9.65 (s, 1H, OH). |
| | | | **¹³C NMR (101 MHz, DMSO-*d₆*) δ :** 10.5 (CH_{3-CH2}), 19.6 (2xCH_{3 iPr}), 23.6 (CH_{2-CH3}), 24.8 (CH_{Alk}), 40.8 (CH_{2 Bn}), 53.8 (CH_{iPr}), 62.0 (CH_{2-OH}), 84.2 (CH_{Ar}), 114.9 (CH_{Ar}), 118.8 (CH_{Ar}), 123.8 (C_{q}), 127.0 (CH_{Ar}), 127.7 (CH_{Ar}), 139.7 (C_{q}), 140.0 (C_{q}), 152.8 (C_{q}), 154.8 (C_{q}), 155.9 (C_{q}). |
| | | | **MS (ESI+) : m/z calcd for C₁₉H₂₆N₆O₂ :** 371.46 [M+H]+, **found :** 371.36. |
| (39) | 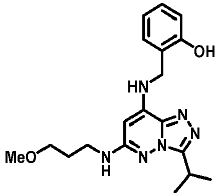 | 92 % ^{a} | Pale yellow solid. |
| | | | **¹H NMR (400 MHz, DMSO-*d₆*) δ :** 1.37 (d, *J* = 6.9 Hz, 6H, 2xCH_{3 iPr}), 1.75 (p, *J* = 6.6 Hz, 2H, CH_{2-CH2-NH-Alk}), 3.17 (q, *J* = 6.6 Hz, 2H, CH_{2-NH Alk}), 3.22 (s, 3H, OCH₃), 3.30 - 3.39 (m, 3H, H_{iPr &} CH_{2-OCH3}), 4.37 (d, *J* = 6.3 Hz, 2H, CH_{2Bn}), 5.35 (s, 1H, H_{Ar}), 6.55 (t, *J* = 5.3 Hz, 1H, NH_{Alk}), 6.73 (t, *J* = 7.4 Hz, 1H, H_{Ar}), 6.84 (d, *J* = 7.8 Hz, 1H, H_{Ar}), 7.06 (t, *J* = 7.5 Hz, 2H, 2xH_{Ar}), 7.55 (t, *J* = 6.3 Hz, 1H, NH_{Bn}), 9.66 (s, 1H, OH). |
| | | | **¹³C NMR (101 MHz, DMSO-*d₆*) δ :** 19.7 (2xCH_{3 iPr}), 24.7 (CH_{iPr}), 28.5 (CH_{2-CH2-NH Alk}), 38.0 (CH_{2-NH Alk}), 40.7 (CH_{2 Bn}), 57.8 (OCH₃), 69.8 (CH_{2-OCH3}), 83.9 (CH_{Ar}), 114.9 (CH_{Ar}), 118.8 (CH_{Ar}), 123.7 (C_{q}), 127.0 (CH_{Ar}), 127.7 (CH_{Ar}), 139.7 (C_{q}), 140.1 (C_{q}), 152.9 (C_{q}), 154.8 (C_{q}), 156.0 (C_{q}). |
| | | | **MS (ESI+) : m/z calcd for C₁₉H₂₆N₆O₂ :** 371.46 [M+H]+, **found :** 371.26. |
| (40) | 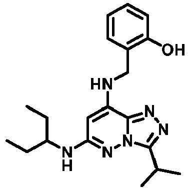 | 88 % ^{a} | White crystalline solid. |
| | | | **¹H NMR (400 MHz, DMSO-*d₆*) δ :** 0.84 (t, *J* = 7.4 Hz, 6H, 2xCH_{3-CH2}), 1.36 - 1.38 (m, 6H, 2xCH_{3 iPr}), 1.39 - 1.57 (m, 4H, 2xCH_{2-CH3}), 3.29 (d, *J* = 7.1 Hz, 1H, H_{iPr}), 3.56 (q, *J* = 6.7 Hz, 1H, HiPent), 4.37 (d, *J* = 6.2 Hz, 2H, CH_{2Bn}), 5.42 (s, 1H, H_{Ar}), 6.32 (d, *J* = 8.0 Hz, 1H, NH_{-CH}), 6.69 - 6.80 (m, 1H, H_{Ar}), 6.85 (d, *J* = 7.9 Hz, 1H, H_{Ar}), 7.08 (t, *J* = 8.2 Hz, 2H, 2xH_{Ar}), 7.47 (t, *J* = 6.2 Hz, 1H, NH_{Bn}), 9.64 (s, 1H, OH). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.1 (2×CH_{2-CH3}), 40.8 (CH_{2Bn}), 53.0 (CH_{-NH}), 84.0 (CH_{Ar}), 114.9 (CH_{Ar}), 118.8 (CH_{Ar}), 123.8 (C_{q}), 127.0 (CH_{Ar}), 127.7 (CH_{Ar}), 139.7 (C_{q}), 140.0 (C_{q}), 152.7 (C_{q}), 154.8 (C_{q}), 156.0 (C_{q}). |
| | | | **MS (ESI+) : m/z calcd for C₂₀H₂₈N₆O** : 369.24 [M+H]+, **found** : 369.35. |
| (41) | 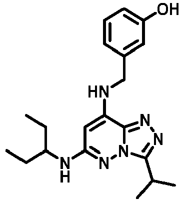 | > 96 % ^{a} | White solid. |
| | | | **¹H NMR (400 MHz, DMSO-*d₆*) δ** : 0.84 (t, *J* = 7.4 Hz, 6H, 2×CH_{3 iPent}), 1.37 (d, *J* = 7.0 Hz, 6H, 2×CH_{3 iPr}), 1.39 - 1.58 (m, 4H, 2xCH_{2-CH3}), 3.31 (q, *J* = 6.9 Hz, 1H, H_{iPr}), 3.56 (h, *J* = 6.5 Hz, 1H, H_{iPent}), 4.36 (d, *J* = 6.3 Hz, 2H, CH_{2 Bn}), 5.41 (s, 1H, H_{Ar}), 6.32 (d, *J* = 8.0 Hz, 1H, NH_{iPent}), 6.63 (ddd, *J* = 8.1, 2.5, 1.0 Hz, 1H, H_{Ar}), 6.74 (t, *J* = 2.0 Hz, 1H, H_{Ar}), 6.77 (dt, *J* = 7.6, 1.2 Hz, 1H, H_{Ar}), 7.12 (t, *J* = 7.8 Hz, 1H, H_{Ar}), 7.79 (t, *J* = 6.3 Hz, 1H, NH_{Bn}), 9.33 (s, 1H, OH). |
| | | | **¹³C NMR (101 MHz, DMSO-*d₆*) δ :** 10.9 (2xCH_{3 iPent}), 20.0 (2xCH_{3 iPr}), 25.3 (CH_{iPr}), 26.6 (2×CH_{2-CH3}) , 45.6 (CH_{2 Bn}), 53.4 (H_{iPent}), 84.7 (CH_{Ar}), 113.7 (CH_{Ar}), 114.3 (CH_{Ar}), 117.7 (CH_{Ar}), 129.8 (CH_{Ar}), 140.2 (C_{q}), 140.3 (C_{q}), 140.3 (C_{q}), 153.2 (C_{q}), 156.4 (C_{q}), 158.0 (C_{q}). |
| | | | **MS (ESI+) : m/z calcd for C₂₀H₂₈N₆O :** 369.24 [M+H]+, **found** : 369.38. |
| (42) | 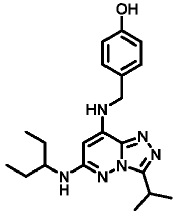 | > 97 % ^{a} | White solid. |
| | | | **¹H NMR (400 MHz, DMSO-*d₆*) δ :** 0.84 (t, *J* = 7.4 Hz, 6H, 2xCH_{3-CH2}), 1.35 (d, *J* = 7.0 Hz, 6H, 2xCH_{3 iPr}), 1.38 - 1.57 (m, 4H, 2xCH_{2-CH3}), 3.25 - 3.31 (m, 1H, H_{iPr}), 3.55 (h, *J* = 6.8 Hz, 1H, H_{iPent}), 4.29 (d, *J* = 6.2 Hz, 2H, CH_{2 Bn}), 5.45 (s, 1H, H_{Ar}), 6.29 (d, *J* = 8.0 Hz, 1H, NH), 6.65 - 6.79 (m, 2H, 2xH_{Ar}), 7.10 - 7.23 (m, 2H, 2xH_{Ar}), 7.68 (t, *J* = 6.3 Hz, 1H, NH_{Bn}), 9.28 (s, 1H, OH). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 44.8 (CH_{2Bn}), 53.0 (CH_{iPr}), 84.1 (CH_{Ar}), 115.1 (2xCH_{Ar}), 128.2 (2×CH_{Ar}), 128.4 (C_{q}), 139.7 (C_{q}), 139.7 (C_{q}), 152.6 (C_{q}), 156.0 (C_{q}), 156.3 (C_{q}). |
| | | | **MS (ESI+) : m/z calcd for C₂₀H₂₈N₆O :** 369.24 [M+H]+, **found** : 369.30. |
| (43) | 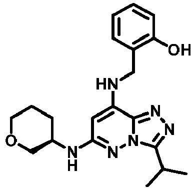 | 93 % ^{a} | White solid. |
| | | | **¹H NMR (400 MHz, DMSO-*d₆*) δ : 1.36** (dd, *J* = 6.9, 4.4 Hz, 6H, 2xCH_{3 iPr}), 1.43 - 1.58 (m, 2H, H_{(CH2)-CH2-O} & H(_{CH2)-CH THP}), 1.64 - 1.72 (m, 1H, H(_{CH2)-CH THP}), 1.92 (dt, *J* = 8.1, 4.0 Hz, 1H, H(_{CH2)-CH2-O}), 3.07 (dd, *J* = 10.5, 8.4 Hz, 1H, _{O-}H_{(CH2)-CH THP}), 3.32 (m, 2H, H_{iPr} & _{CH2-}H_{(CH2)-O}), 3.68 (dt, *J* = 11.3, 3.8 Hz, 2H, H_{THP} & _{CH2-}H_{(CH2)-O}), 3.92 (dd, *J* = 10.7, 3.1 Hz, 1H, _{O-}H(_{CH2)-CH THP}), 4.36 (d, *J* = 6.2 Hz, 2H, CH_{2 Bn}), 5.39 (s, 1H, H_{Ar}), 6.50 (d, *J* = 7.1 Hz, 1H, NH_{THP}), 6.73 (t, *J* = 7.4 Hz, 1H, H_{Ar}), 6.83 (d, *J* = 8.1 Hz, 1H, H_{Ar}), 7.06 (m, 2H, 2xH_{Ar}), 7.58 (t, *J* = 6.2 Hz, 1H, NH_{Bn}), 9.63 (s, 1H, OH). |
| | | | **¹³C NMR (101 MHz, DMSO-*d₆*) δ :** 19.6 (CH_{3 iPr}), 19.7 (CH_{3 iPr}), 24.2 (CH_{2 THP}), 24.7 (CH_{2 THP}), 28.7 (CH_{iPr}), 40.7 (CH_{2 Bn}), 46.6 (CH_{THP}), 67.1 (CH_{2 THP}), 70.3 (CH_{2 THP}), 83.9 (CH_{Ar}), 114.8 (CH_{Ar}), 118.8 (CH_{Ar}), 123.6 (C_{q}), 126.9 (CH_{Ar}), 127.7 (CH_{Ar}), 139.7 (C_{q}), 140.3 (C_{q}), 152.8 (C_{q}), 154.8 (C_{q}), 155.3 (C_{q}). |
| | | | **MS (ESI+) : m/z calcd for C₂₀H₂₆N₆O₂ :** 383.22 [M+H]+, **found** : 383.26. |
| (44) | 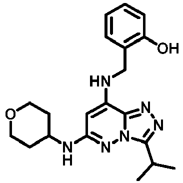 | 94 % ^{a} | White solid. |
| | | | **¹H NMR (400 MHz, DMSO-*d₆*) δ :** 1.32 - 1.45 (m, 8H, 2xCH_{3 iPr} & CH_{2-CH THP}), 1.90 (dd, *J* = 13.0, 3.6 Hz, 2H, CH_{2-CH THP}), 3.36 (dp, *J* = 14.7, 7.0, 4.6 Hz, 3H, , CH_{2-O} & HiPr), 3.71 (qd, *J* = 10.4, 9.8, 4.7 Hz, 1H, H_{THP}), 3.83 (dt, *J* = 11.6, 3.7 Hz, 2H, CH_{2-O}), 4.37 (d, *J* = 6.2 Hz, 2H, CH_{2 Bn}), 5.36 (s, 1H, H_{Ar}), 6.54 (d, *J =* 6.9 Hz, 1H, NH_{THP}), 6.73 (td, *J* = 7.5, 1.2 Hz, 1H, H_{Ar}), 6.84 (dd, *J* = 8.5, 1.2 Hz, 1H, H_{Ar}), 7.06 (ddd, *J* = 7.6, 6.3, 1.8 Hz, 2H, 2xH_{Ar}), 7.59 (t, *J=* 6.3 Hz, 1H, NH_{Bn}), 9.64 (s, 1H, OH). |
| | | | **¹³C NMR (101 MHz, DMSO-*d₆*) δ :** 19.6 (2xCH_{3 iPr}), 24.8 (CH_{iPr}), 32.3 (2×CH_{2-CH THP}), 40.7 (CH_{iPr}), 46.8 (CH_{THP}), 65.8 (2xCH_{2-O}), 83.9 (CH_{Ar}), 114.9 (CH_{Ar}), 118.9 (CH_{Ar}), 123.7 (C_{q}), 126.9 (CH_{Ar}), 127.7 (CH_{Ar}), 139.7 (C_{q}), 140.2 (C_{q}), 152.8 (C_{q}), 154.8 (C_{q}), 155.1 (C_{q}). |
| | | | **MS (ESI+) : m/z calcd for C₂₀H₂₆N₆O₂** : 383.22 [M+H]+, **found** : 383.29. |
| (45) | 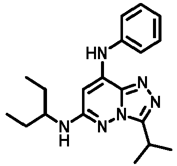 | > 96 % ^{a} | White solid. |
| | | | **¹H NMR (400 MHz, DMSO-*d₆*) δ :** 0.86 (t, *J* = 7.4 Hz, 6H, 2xCH_{3-CH2}), 1.39 (d, *J* = 7.0 Hz, 6H, 2xCH_{3 iPr}), 1.41 - 1.61 (m, 4H, 2xCH_{2-CH3}), 3.36 (q, *J* = 6.6 Hz, 1H, H_{iPr}), 3.61 (q, *J* = 6.6 Hz, 1H, H_{iPent}), 6.20 (s, 1H, H_{Ar}), 6.49 (d, *J* = 7.9 Hz, 1H, NH_{iPent}), 7.14 (tt, *J* = 5.8, 2.4 Hz, 1H, H_{Ar}), 7.32 - 7.52 (m, 4H, 4×H_{Ar}), 9.23 (s, 1H, NH_{-Ph}). |
| | | | **¹³C NMR (101 MHz, DMSO-*d₆*) δ :** 10.3 (2xCH_{3-CH2}), 19.5 (2xCH_{3 iPr}), 24.8 (CH_{iPr}), 26.1 (2×CH_{2-CH3}), 53.0 (CH_{iPent}), 86.7 (CH_{Ar}), 122.3 (2xCH_{Ar}), 123.7 (CH_{Ar}), 129.2 (2xCH_{Ar}), 137.1 (C_{q}), 139.5 (C_{q}), 139.7 (C_{q}), 153.0 (C_{q}), 155.9 (C_{q}). |
| | | | **MS (ESI+) : m/z calcd for C₁₉H₂₆N₅ :** 339.23 [M+H]+, **found :** 339.32. |
| (46) | 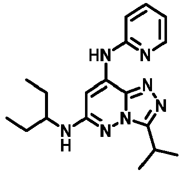 | > 96 % ^{a} | Beige solid. |
| | | | **¹H NMR (400 MHz, DMSO-*d₆*) δ :** 0.89 (t, *J* = 7.4 Hz, 6H, 2×CH_{3-CH2}), 1.39 (d, *J* = 7.0 Hz, 6H, 2xCH_{3 iPr}), 1.45 - 1.67 (m, 4H, 2×CH_{2-CH3}), 3.36 (p, *J* = 7.1 Hz, 1H, H_{iPr}), 3.66 (dtd, *J* = 12.8, 7.5, 5.4 Hz, 1H, H_{iPent}), 6.81 (d, *J* = 8.0 Hz, 1H, NH_{iPent}), 6.99 (ddd, *J* = 7.4, 5.0, 1.0 Hz, 1H, H_{Ar}), 7.48 (d, *J* = 8.4 Hz, 1H, H_{Ar}), 7.72 (ddd, *J* = 8.8, 7.2, 2.0 Hz, 1H, H_{Ar}), 7.82 (s, 1H, H_{Ar}), 8.33 (dd, *J* = 5.2, 1.9 Hz, 1H, H_{Ar}), 9.85 (s, 1H, NH_{Ph}). |
| | | | **¹³C NMR (101 MHz, DMSO-*d₆*) δ :** 10.5 (2xCH_{3-CH2}), 19.5 (2xCH_{3 iPr}), 24.8 (CH_{iPr}), 26.1 (2×CH_{2-CH3}), 53.2 (CH_{iPent}), 94.0 (CH_{Ar}), 113.7 (CH_{Ar}), 117.0 (CH_{Ar}), 133.6 (C_{q}), 137.6 (C_{q}), 139.4 (C_{q}), 146.9 (CH_{Ar}), 153.0 (C_{q}), 154.9 (C_{q}), 155.9 (C_{q}). |
| | | | **MS (ESI+) : m/z calcd for C₁₈H₂₅N₇ :** 340.22 [M+H]+, found : 340.32. |
| (47) | 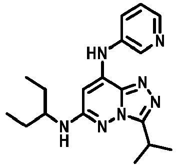 | > 98 % ^{b} | Beige solid. |
| | | | **¹H NMR (400 MHz, DMSO-*d₆*) δ :** 0.87 (t, *J* = 7.4 Hz, 6H, 2xCH_{3-CH2}), 1.39 (d, *J* = 7.0 Hz, 6H, 2xCH_{3 iPr}), 1.41 - 1.62 (m, 4H, 2xCH_{2-CH3}), 3.37 (q, *J* = 7.0 Hz, 1H, CH_{iPr}), 3.61 (qd, *J* = 7.3, 3.6 Hz, 1H, CH_{iPent}), 6.24 (s, 1H, CH_{Ar}), 6.53 (d, *J* = 7.9 Hz, 1H, NH_{iPent}), 7.44 (dd, *J* = 8.3, 4.7 Hz, 1H, CH_{Ar}), 7.80 (ddd, *J* = 8.3, 2.7, 1.5 Hz, 1H, CH_{Ar}), 8.34 (dd, *J* = 4.7, 1.5 Hz, 1H, CH_{Ar}), 8.67 (d, *J* = 2.6 Hz, 1H, CH_{Ar}), 9.44 (s, 1H, NH_{Pyr}). |
| | | | **¹³C NMR (101 MHz, DMSO-d*₆*) δ :** 10.3 (2xCH_{3-CH2}), 19.5 (2xCH_{3 iPr}), 24.8 (CH_{iPr}), 26.0 (2xCH_{2-CH3}), 53.0 (CH_{iPent}), 87.5 (CH_{Ar}), 123.9 (CH_{Ar}), 129.2 (CH_{Ar}), 136.4 (C_{q}), 136.8 (C_{q}), 139.5 (C_{q}), 143.9 (CH_{Ar}), 144.5 (CH_{Ar}), 153.0 (C_{q}), 155.8 (C_{q}). |
| | | | **MS (ESI+) : m/z calcd for C₁₈H₂₅N₇ :** 340.22 [M+H]+, **found :** 340.32. |
| (48) | 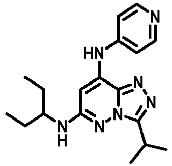 | > 98 % ^{a} | White solid. |
| | | | **¹H NMR (400 MHz, DMSO-*d₆*) δ :** 0.89 (t, *J* = 7.4 Hz, 6H, 2xCH_{3-CH2}), 1.39 (d, *J* = 7.0 Hz, 6H, 2×CH_{3 iPr}), 1.42 - 1.66 (m, 4H, 2xCH_{2-CH3}), 3.36 (p, *J* = 6.8 Hz, 1H, H_{iPr}), 3.64 (h, *J* = 6.9 Hz, 1H, H_{iPent}), 6.61 (s, 1H, H_{Ar}), 6.68 (d, *J* = 7.9 Hz, 1H, NH_{iPent}), 7.26 - 7.51 (m, 2H, 2xH_{Ar}), 8.37 - 8.58 (m, 2H, 2×H_{Ar}), 9.75 (s, 1H, NH_{Pyr}). |
| | | | **¹³C NMR (101 MHz, DMSO-*d₆*) δ :** 10.3 (2xCH_{3-CH2}), 19.4 (2xCH_{3 iPr}), 24.8 (CH_{iPr}), 26.0 (2xCH_{2-CH3}), 53.1 (CH_{iPent}), 91.6 (CH_{Ar}), 113.8 (CH_{Ar}), 134.7 (C_{q}), 139.4 (C_{q}), 147.2 (C_{q}), 150.4 (CH_{Ar}), 153.2 (C_{q}), 155.4 (C_{q}). |
| | | | **MS (ESI+) : m/z calcd for C₁₈H₂₅N₇ :** 340.22 [M+H]+, **found :** 340.32. |
| (49) | 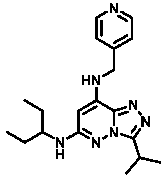 | > 98 % ^{a} | White solid. |
| | | | **¹H NMR (400 MHz, DMSO-*d₆*) δ :** 0.82 (t, *J* = 7.4 Hz, 6H, 2xCH_{3-CH2}), 1.36 (d, *J* = 6.9 Hz, 6H, 2xCH_{3 iPr}), 1.38 - 1.58 (m, 4H, 2xCH_{2-CH3}), 3.24 - 3.33 (m, 1H, H_{iPr}), 3.54 (h, *J* = 6.6 Hz, 1H, H_{iPent}), 4.47 (d, *J* = 6.3 Hz, 2H, CH_{2 Bn}), 5.34 (s, 1H, H_{Ar}), 6.30 (d, *J* = 7.9 Hz, 1H, NH_{iPent}), 7.24 - 7.39 (m, 2H, 2xH_{Ar}), 7.91 (t, *J* = 6.4 Hz, 1H, NH_{Bn}), 8.44 - 8.61 (m, 2H, 2xH_{Ar}). |
| | | | **¹³C NMR (101 MHz, DMSO-*d₆*) δ :** 10.4 (2×CH_{3-CH2}), 19.5 (2xCH_{3 iPr}), 24.8 (CH_{iPr}), 26.1 (2xCH_{2-CH3}), 44.4 (CH_{2Bn}), 53.0 (CH_{iPent}), 84.5 (CH_{Ar}), 121.8 (2xCH_{Ar}), 139.5 (C_{q}), 139.7 (C_{q}), 147.5 (C_{q}), 149.7 (2xCH_{Ar}), 152.7 (C_{q}), 155.8 (C_{q}). |
| | | | **MS (ESI+) : m/z calcd for C₁₉H₂₇N₇ :** 354.24 [M+H]+, **found :** 354.30. |
| (50) | 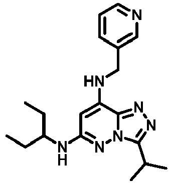 | > 97 % ^{a} | Light pink solid. |
| | | | **¹H NMR (400 MHz, DMSO-*d₆*) δ :** 0.83 (t, *J* = 7.4 Hz, 6H, 2xCH_{3-CH2}), 1.35 (d, *J* = 7.0 Hz, 6H, 2xCH_{3 iPr}), 1.38 - 1.58 (m, 4H, 2×CH_{2-CH3}), 3.24 - 3.31 (m, 1H, H_{iPr}), 3.55 (h, *J* = 6.2 Hz, 1H, H_{iPent}), 4.45 (d, *J* = 6.3 Hz, 2H, CH_{2 Bn}), 5.47 (s, 1H, H_{Ar}), 6.31 (d, *J* = 8.0 Hz, 1H, NH-_{CH}), 7.37 (dd, *J* = 7.9, 4.8 Hz, 1H, H_{Ar}), 7.75 (dt, *J* = 7.9, 2.0 Hz, 1H, H_{Ar}), 7.90 (t, *J* = 6.4 Hz, 1H, NH_{Bn}), 8.41 - 8.51 (m, 1H, H_{Ar}), 8.60 (s, 1H, H_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.1 (2×CH_{2-CH3}), 43.0 (CH_{2Bn}), 53.0 (CH_{iPent}), 84.4 (CH_{Ar}), 123.6 (CH_{Ar}), 133.9 (C_{q}), 134.8 (CH_{Ar}), 139.5 (C_{q}), 139.6 (C_{q}), 148.3 (CH_{Ar}), 148.6 (CH_{Ar}), 152.7 (C_{q}), 155.8 (C_{q}). |
| | | | **MS (ESI+) : m/z calcd for C₁₉H₂₇N₇ :** 354.24 [M+H]+, **found :** 354.33. |
| (51) | 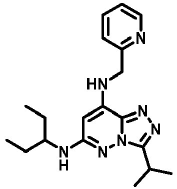 | > 98 % ^{a} | White solid. |
| | | | **¹H NMR (400 MHz, DMSO-*d₆*) δ :** 0.82 (t, *J* = 7.4 Hz, 6H, 2xCH_{3-CH2}), 1.36 (d, *J* = 7.0 Hz, 6H, 2xCH_{3 iPr}), 1.38 - 1.55 (m, 4H, 2xCH_{2-CH3}), 3.25 - 3.32 (m, 1H, H_{iPr}), 3.55 (h, *J* = 6.4 Hz, 1H, H_{iPent}), 4.52 (d, *J* = 6.2 Hz, 2H, CH_{2 Bn}), 5.42 (s, 1H, H_{Ar}), 6.35 (d, *J* = 7.9 Hz, 1H, NH_{iPent}), 7.28 (dd, *J* = 7.5, 4.9 Hz, 1H, H_{Ar}), 7.34 (d, *J* = 7.9 Hz, 1H, H_{Ar}), 7.71 - 7.86 (m, 2H, H_{Ar} & NH_{Bn}), 8.49 - 8.62 (m, 1H, H_{Ar}). |
| | | | **¹³C NMR (101 MHz, DMSO-*d₆*) δ :** 10.4 (2xCH_{3-CH2}), 19.5 (2xCH_{3 iPr}), 24.8 (CH_{iPr}), 26.1 (2xCH_{2-CH3}), 47.2, 53.0 (CH_{iPent}), 84.50 (CH_{Ar}), 120.7 (CH_{Ar}), 122.3 (CH_{Ar}), 137.0 (CH_{Ar}), 139.7 (C_{q}), 139.8 (C_{q}), 149.0 (CH_{Ar}), 152.7 (C_{q}), 155.9 (C_{q}), 157.7 (C_{q}). |
| | | | **MS (ESI+) : m/z calcd for C₁₉H₂₇N₇ :** 354.24 [M+H]+, **found :** 354.30. |
| (52) | 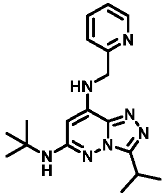 | 93 % ^{a} | White solid. |
| | | | **¹H NMR (400 MHz, DMSO-*d₆*) δ :** 1.35 (s, 9H, 3×CH_{3 tBu}), 1.38 (d, *J* = 6.9 Hz, 6H, 2×CH_{iPr}), 3.36 (dd, *J* = 13.5, 6.8 Hz, 1H, H_{iPr}), 4.49 (d, *J* = 6.2 Hz, 2H, CH_{2 Bn}), 5.42 (s, 1H, H_{Ar}), 6.25 (s, 1H, NH), 7.28 (ddd, *J* = 6.3, 5.0, 1.2 Hz, 1H, H_{Ar}), 7.31 (d, *J* = 7.9 Hz, 1H, H_{Ar}), 7.76 (ddd, *J* = 9.5, 5.3, 1.8 Hz, 2H, NH_{Bn} & H_{Ar}), 8.50-8.61 (m, 1H, H_{Ar}). |
| | | | **¹³C NMR (101 MHz, DMSO-*d₆*) δ :** 19.6 (2xCH_{3 iPr}), 24.9 (CH_{iPr}), 28.5 (3×CH_{3 tBu}), 47.1 (CH_{2Bn}), 50.6 (C_{q}), 85.5 (CH_{Ar}), 120.5 (CH_{Ar}), 122.3 (CH_{Ar}), 136.9 (CH_{Ar}), 139.2 (C_{q}), 139.6 (C_{q}), 149.0 (CH_{Ar}), 152.8 (C_{q}), 155.2 (C_{q}), 157.7 (C_{q}). |
| | | | **MS (ESI+) : m/z calcd for C₁₉H₂₇N₇ :** 340.22 [M+H]+, **found :** 340.39. |
| (53) | 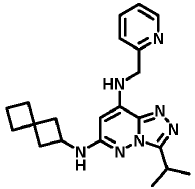 | 93 % ^{a} | Grey solid. |
| | | | **¹H NMR (400 MHz, DMSO-*d₆*) δ : 1.36** (d, *J* = 6.9 Hz, 6H, 2×CH_{3 iPr}), 1.79 (dtd, *J* = 12.5, 8.3, 7.4, 2.7 Hz, 4H, 2xCH_{2 spiro}), 1.87 (dd, *J* = 8.4, 5.7 Hz, 2H, CH₂ spiro), 2.00 (t, *J* = 7.2 Hz, 2H, CH₂ spiro), 2.34 (ddd, *J* = 11.6, 6.1, 2.4 Hz, 2H, CH₂ spiro), 3.23 - 3.38 (m, 1H, H_{iPr}), 3.90 (h, *J* = 7.9 Hz, 1H, Hₛₚᵢᵣₒ), 4.51 (d, *J* = 6.1 Hz, 2H, CH_{2 Bn}), 5.29 (s, 1H, H_{Ar}), 6.78 (d, *J* = 6.2 Hz, 1H, NHₛₚᵢᵣₒ), 7.28 (dd, *J* = 7.5, 4.9 Hz, 1H, H_{Ar}), 7.32 (d, *J* = 7.9 Hz, 1H, H_{Ar}), 7.76 (td, *J* = 7.7, 1.8 Hz, 1H, H_{Ar}), 7.87 (t, *J* = 6.2 Hz, 1H, NH_{Bn}), 8.54 (dd, *J* = 4.8, 1.7 Hz, 1H, H_{Ar}). |
| | | | **¹³C NMR (101 MHz, DMSO-*d₆*) δ :** 16.4 (CH_{2 spiro}), 19.6 (2xCH_{3 iPr}), 24.7 (CH_{iPr}), 34.1 (CH₂ spiro), 34.8 (CH_{2 spiro}), 37.2 (C_{q}), 41.6 (CHₛₚᵢᵣₒ), 42.5 (2×CH_{2 spiro}), 47.2 (CH_{2Bn}), 84.2 (CH_{Ar}), 120.7 (CH_{Ar}), 122.3 (CH_{Ar}), 136.9 (CH_{Ar}), 139.6 (C_{q}), 140.0 (C_{q}), 149.0 (CH_{Ar}), 152.8 (C_{q}), 155.0 (C_{q}), 157.6 (C_{q}). |
| | | | **MS (ESI+) : m/z calcd for C₂₁H₂₇N₇ :** 378.24 [M+H]+, **found :** 378.29. |
| (54) | 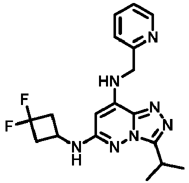 | > 98 % ^{a} | Beige solid. |
| | | | **¹H NMR (400 MHz, DMSO-*d₆*) δ : 1.36** (d, *J* = 6.9 Hz, 6H, 2×CH_{3 iPr}), 2.54 (dd, *J* = 15.3, 6.1 Hz, 2H, CH_{2 cBut}), 2.92 (tt, *J* = 14.1, 7.6 Hz, 2H, CH_{2 cBut}), 3.39 (d, *J* = 9.3 Hz, 1H, H_{iPr}), 3.95 (dq, *J* = 14.0, 7.2 Hz, 1H, H_{cBut}), 4.54 (d, *J* = 6.2 Hz, 2H, CH_{2 Bn}), 5.32 (s, 1H, H_{Ar}), 7.14 (d, *J* = 5.4 Hz, 1H, NH_{cBut}), 7.28 (dd, *J* = 7.5, 4.9 Hz, 1H, H_{Ar}), 7.33 (d, *J* = 7.9 Hz, 1H, H_{Ar}), 7.76 (td, *J* = 7.7, 1.8 Hz, 1H, H_{Ar}), 8.01 (t, *J* = 6.2 Hz, 1H, NH_{Bn}), 8.49 - 8.59 (m, 1H, H_{Ar}). |
| | | | **¹³C NMR (101 MHz, DMSO-*d₆*) δ :** 19.6 (2xCH_{3 iPr}), 24.7 (CH_{iPr}), 35.8 (dd, *J* = 17.9, 6.5 Hz, CH_{cBut}), 42.0 (d, *J* = 21.4 Hz, CH_{2 cBut}), 42.2 (d, *J* = 21.0 Hz, CH_{2 cBut}), 47.3 (CH_{2Bn}), 83.9 (CH_{Ar}), 120.1 (t, *J* = 277.4 Hz, CF₂), 120.8 (CH_{Ar}), 122.4 (CH_{Ar}), 137.0 (CH_{Ar}), 139.6 (C_{q}), 140.4 (C_{q}), 149.0 (CH_{Ar}), 153.0 (C_{q}), 155.0 (C_{q}), 157.6 (C_{q}). |
| | | | **MS (ESI+) : m/z calcd for C₁₈H₂₁F₂N₇ :** 374.19 [M+H]+, **found :** 374.35. |
| (55) | 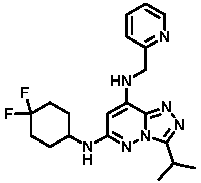 | > 99 % ^{a} | White solid. |
| | | | **¹H NMR (400 MHz, DMSO-*d₆*) δ :** 1.38 (d, *J* = 6.9 Hz, 6H, 2xCH_{3 iPr}), 1.56 (dd, *J* = 12.2, 8.5 Hz, 2H, CH_{2 cHex}), 1.82 - 2.10 (m, 6H, 3×CH_{2 cHex}), 3.36 (d, *J* = 7.5 Hz, 1H, H_{iPr}), 3.75 (d, *J* = 4.9 Hz, 1H, H_{cHex}), 4.53 (d, *J* = 6.2 Hz, 2H, CH_{2 Bn}), 5.40 (s, 1H, H_{Ar}), 6.61 (d, *J* = 6.9 Hz, 1H, NH), 7.29 (dd, *J* = 7.5, 4.8 Hz, 1H, H_{Ar}), 7.33 (d, *J* = 7.9 Hz, 1H, H_{Ar}), 7.77 (td, *J* = 7.7, 1.8 Hz, 1H, H_{Ar}), 7.91 (t, *J* = 6.2 Hz, 1H, NH_{Bn}), 8.48 - 8.63 (m, 1H, H_{Ar}). |
| | | | ¹³C **NMR (101 MHz, DMSO-*d₆*) δ** : 19.6 (2×CH_{3 iPr}), 24.7 (CH_{iPr}), 27.4 (t, *J* = 4.7 Hz, 2xCH_{2 cHex}), 31.0 (t, *J* = 23.8 Hz, 2×CH_{2 cHex}), 46.5 (CH_{cHex}), 47.2 (CH_{2Bn}), 84.3 (CH_{Ar}), 120.6 (CH_{Ar}), 122.3 (CH_{Ar}), 123.8 (t, *J* = 240.4 Hz, CF₂), 137.0 (CH_{Ar}), 139.6 (C_{q}), 140.0 (C_{q}), 149.0 (CH_{Ar}), 152.9 (C_{q}), 155.2 (C_{q}), 157.6 (C_{q}). |
| | | | **MS (ESI+) : m/z calcd for C₂₀H₂₅F₂N**₇ **:** 402.22 [M+H]+, **found :** 402.40 |
| (56) | 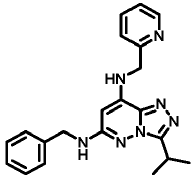 | > 98 % ^{a} | White solid. |
| | | | **¹H NMR (400 MHz, DMSO-*d₆*) δ :** 1.30 (d, *J* = 6.9 Hz, 6H, 2xCH_{3 iPr}), 3.25 - 3.31 (m, 1H, H_{iPr}), 4.32 (d, *J* = 5.8 Hz, 2H, CH_{2 Bn}), 4.53 (d, *J* = 6.1 Hz, 2H, CH_{2 Bn}), 5.45 (s, 1H, H_{Ar}), 7.16 (t, *J* = 5.9 Hz, 1H, NH), 7.17 - 7.22 (m, 1H, H_{Ar}), 7.25 - 7.37 (m, 6H, 6×H_{Ar}), 7.75 (td, *J* = 7.7, 1.8 Hz, 1H, H_{Ar}), 7.89 (t, *J* = 6.2 Hz, 1H, NH), 8.54 (dt, *J* = 4.8, 1.5 Hz, 1H, H_{Ar}). |
| | | | **¹³C NMR (101 MHz, DMSO-*d₆*) δ :** 19.6 (2xCH_{3 iPr}), 24.7 (CH_{iPr}), 44.5 (CH_{2 Bn}), 47.2 (CH_{2Bn}), 84.3 (CH_{Ar}), 120.8 (CH_{Ar}), 122.3 (CH_{Ar}), 126.6 (CH_{Ar}), 127.6 (2×CH_{Ar}), 128.1 (2xCH_{Ar}), 136.9 (CH_{Ar}), 139.6 (C_{q}), 139.8 (C_{q}), 140.0 (C_{q}), 149.0 (CH_{Ar}), 152.9 (C_{q}), 155.6 (C_{q}), 157.5 (C_{q}). |
| | | | **MS (ESI+) : m/z calcd for C₂₁H₂₃F₂N₇ :** 374.21 [M+H]+, **found :** 374.35. |
| (57) | 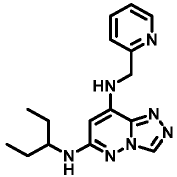 | > 96 % ^{a} | White solid. |
| | | | **¹H NMR (400 MHz, DMSO-*d₆*) δ** : 0.81 (t, *J* = 7.4 Hz, 6H, 2xCH_{3 iPent}), 1.30 - 1.58 (m, 4H, 2×CH_{2 iPent}), 3.56 (h, *J* = 6.4 Hz, 1H, H_{iPent}), 4.54 (d, *J* = 6.1 Hz, 2H, CH_{2 Bn}), 5.47 (s, 1H, H_{Ar}), 6.39 (d, *J* = 8.3 Hz, 1H, NH_{iPent}), 7.29 (dd, *J* = 7.5, 4.9 Hz, 1H, H_{Ar}), 7.35 (d, *J* = 7.9 Hz, 1H, H_{Ar}), 7.77 (td, *J* = 7.7, 1.8 Hz, 1H, H_{Ar}), 7.85 (t, *J* = 6.2 Hz, 1H, NH_{Bn}), 8.56 (d, *J* = 4.7 Hz, 1H, H_{Ar}), 8.94 (s, 1H, H_{Ar}). |
| | | | **¹³C NMR (101 MHz, DMSO-*d₆*) δ :** 10.2 (2xCH_{3 iPent}), 25.9 (2xCH_{2 iPent}), 47.2 (CH_{2 Bn}), 52.5 (CH_{iPent}), 85.2 (CH_{Ar}), 120.7 (CH_{Ar}), 122.3 (CH_{Ar}), 136.9 (CH_{Ar}), 138.7 (CH_{Ar}), 139.2 (C_{q}), 139.5 (C_{q}), 149.0 (CH_{Ar}), 156.7 (C_{q}), 157.5 (C_{q}). |
| | | | **MS (ESI+) : m/z calcd for C₁₆H₂₁N₇ :** 312.19 [M+H]+, **found :** 312.27. |
| (58) | 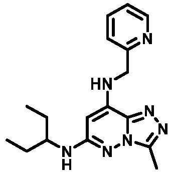 | > 96 % ^{a} | White solid. |
| | | | **¹H NMR (400 MHz, DMSO-*d₆*) δ :** 0.82 (t, *J* = 7.4 Hz, 6H, 2xCH_{3 iPent}), 1.44 (ddp, *J* = 35.5, 14.4, 7.0 Hz, 4H, 2×CH_{2 iPent}), 2.45 (s, 3H, CH₃), 3.58 (h, *J* = 6.6 Hz, 1H, H_{iPent}), 4.53 (d, *J* = 6.1 Hz, 2H, CH_{2 Bn}), 5.44 (s, 1H, H_{Ar}), 6.35 (d, *J* = 8.1 Hz, 1H, NH_{Bn}), 7.29 (dd, *J* = 7.5, 4.9 Hz, 1H, H_{Ar}), 7.33 (d, *J* = 7.9 Hz, 1H, H_{Ar}), 7.68 - 7.83 (m, 2H, H_{Ar} & NH_{iPent}), 8.55 (d, *J* = 4.9 Hz, 1H, H_{Ar}). |
| | | | **¹³C NMR (101 MHz, DMSO-*d₆*) δ :** 9.3 (CH₃), 10.3 (2×CH_{3 iPent}), 26.1 (2xCH_{2 iPent}), 47.2 (CH_{2Bn}), 52.7 (CH_{iPent}), 84.5 (CH_{Ar}), 120.7 (CH_{Ar}), 122.3 (CH_{Ar}), 136.9 (CH_{Ar}), 139.3 (C_{q}), 139.8 (C_{q}), 145.4 (C_{q}), 149.0 (CH_{Ar}), 156.3 (C_{q}), 157.6 (C_{q}). |
| | | | **MS (ESI+) : m/z calcd for C₁₇H₂₃N₇ :** 326.21 [M+H]+, **found :** 326.35. |
| (59) | 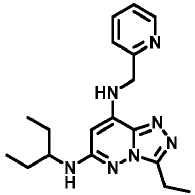 | 81 % ^{a} | White solid. |
| | | | **¹H NMR (400 MHz, DMSO-*d₆*) δ :** 0.82 (t, *J* = 7.4 Hz, 6H, _{2×CH3 iPent}), 1.30 (t, *J* = 7.5 Hz, 3H, CH_{3-CH2}), 1.35 - 1.62 (m, 4H, 2×CH_{2 iPent}), 2.88 (q, *J* = 7.5 Hz, 2H, CH_{2-CH3}), 3.57 (p, *J* = 6.8 Hz, 1H, H_{iPent}), 4.53 (d, *J* = 6.2 Hz, 2H, CH_{2 Bn}), 5.44 (s, 1H, H_{Ar}), 6.35 (d, *J* = 7.9 Hz, 1H, NH_{iPent}), 7.29 (dd, *J* = 7.5, 4.8 Hz, 1H, H_{Ar}), 7.34 (d, *J* = 7.9 Hz, 1H, H_{Ar}), 7.77 (ddt, *J* = 7.7, 5.5, 2.1 Hz, 2H, H_{Ar} & NH_{Bn}), 8.55 (d, *J* = 4.9 Hz, 1H, H_{Ar}). |
| | | | **¹³C NMR (101 MHz, DMSO-*d₆*)** δ : 10.3 (CH_{3 iPent}), 10.9 (CH_{3-CH2}), 17.5 (CH_{2-CH3}), 26.1 (CH_{2 iPent}), 47.2 (CH_{2Bn}), 52.8 (CH_{iPent}), 84.5 (CH_{Ar}), 120.7 (CH_{Ar}), 122.3 (CH_{Ar}), 136.9 (CH_{Ar}), 139.5 (C_{q}), 139.8 (C_{q}), 149.0 (C_{q}), 149.7 (C_{q}), 156.1 (C_{q}), 157.6 (C_{q}). |
| | | | **MS (ESI+) : m/z calcd for C₁₈H₂₅N₇ :** 340.22 [M+H]+, **found :** 340.41. |
| (60) | 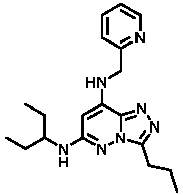 | > 98 % ^{a} | Beige solid. |
| | | | **¹H NMR (400 MHz, DMSO-*d₆*) δ :** 0.83 (t, *J* = 7.4 Hz, 6H, 2xCH_{3-CH2}), 0.93 (t, *J* = 7.4 Hz, 3H, CH₃), 1.46 (ddq, *J* = 28.4, 13.9, 7.1, 6.5 Hz, 4H, 2xCH_{2-CH3}), 1.78 (h, *J* = 7.5 Hz, 2H, CH₂), 2.86 (t, *J* = 7.4 Hz, 2H, CH₂), 3.55 (td, *J* = 12.8, 6.3 Hz, 2H, H_{iPent}), 4.53 (d, *J* = 6.1 Hz, 2H, CH_{2 Bn}), 5.44 (s, 1H, H_{Ar}), 6.36 (d, *J* = 7.9 Hz, 1H, NH), 7.26 - 7.32 (m, 1H, H_{Ar}), 7.35 (d, *J* = 7.9 Hz, 1H), 7.78 (td, *J* = 7.4, 1.8 Hz, 2H), 8.56 (d, *J* = 4.9 Hz, 1H). |
| | | | **¹³C NMR (101 MHz, DMSO-*d₆*) δ :** 10.4 (2xCH_{3-CH2}), 13.7 (CH₃), 19.5 (CH₂), 25.7 (CH₂), 26.1 (2xCH_{2-CH3}), 47.2 (CH_{2Bn}), 52.9 (CH_{iPent}), 84.5 (CH_{Ar}), 120.7 (CH_{Ar}), 122.3 (CH_{Ar}), 137.0 (CH_{Ar}), 139.4 (C_{q}), 139.8 (C_{q}), 148.7 (C_{q}), 149.0 (CH_{Ar}), 156.1 (C_{q}), 157.6 (C_{q}). |
| | | | **MS (ESI+) : m/z calcd for C₁₉H₂₇N₇** : 354.24 [M+H]+, **found :** 354.40. |
| (61) | 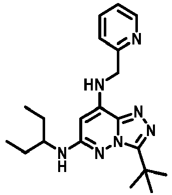 | 91 % ^{a} | White solid. |
| | | | **¹H NMR (400 MHz, DMSO-*d₆*) δ :** 0.83 (t, *J* = 7.4 Hz, 6H, 2xCH_{3-CH2}), 1.37 - 1.58 (m, 13H, 2×CH_{2-CH3} & 3xCH_{3 tBu}), 3.52 (h, *J* = 6.5 Hz, 1H, H_{iPent}), 4.51 (d, *J* = 6.1 Hz, 2H, CH_{2 Bn}), 5.42 (s, 1H, H_{Ar}), 6.35 (d, *J* = 7.8 Hz, 1H, NH_{iPent}), 7.28 (dd, *J* = 7.5, 4.9 Hz, 1H, H_{Ar}), 7.34 (d, *J* = 7.8 Hz, 1H, H_{Ar}), 7.70 - 7.84 (m, 2H, NH_{Bn} & H_{Ar}), 8.47 - 8.60 (m, 1H, H_{Ar}). |
| | | | **¹³C NMR (101 MHz, DMSO-*d₆*) δ :** 10.4 (2xCH_{3-CH2}), 26.0 (2×CH_{2-CH3}), 26.9 (3×CH_{3 tBu}), 32.1 (C_{q}), 47.2 (CH_{2 Bn}), 53.3 (CH_{iPent}), 84.2 (CH_{Ar}), 120.7 (CH_{Ar}), 122.3 (CH_{Ar}), 137.0 (CH_{Ar}), 139.8 (C_{q}), 140.3 (C_{q}), 149.0 (CH_{Ar}), 154.1 (C_{q}), 155.3 (C_{q}), 157.7 (C_{q}). |
| | | | **MS (ESI+) : m/z calcd for C₂₀H₂₉N₇ :** 368.26 [M+H]+, **found :** 368.37. |
| (62) | 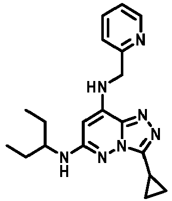 | > 96 % ^{a} | White solid. |
| | | | **¹H NMR (400 MHz, DMSO-*d₆*) δ :** 0.83 (t, *J* = 7.4 Hz, 6H, 2xCH_{3 iPent}), 1.08 (ddq, *J* = 43.6, 7.1, 4.2 Hz, 4H, 2×CH_{2 cPr}), 1.45 (ddt, *J* = 40.6, 13.9, 6.8 Hz, 4H, 2xCH_{2 iPent}), 2.24 (tt, *J* = 8.8, 5.1 Hz, 1H, H_{cPr}), 3.55 (h, *J* = 6.4 Hz, 1H, H_{iPent}), 4.52 (d, *J* = 6.1 Hz, 2H, CH_{2 Bn}), 5.42 (s, 1H, H_{Ar}), 6.35 (d, *J* = 8.0 Hz, 1H, NH_{iPent}), 7.28 (dd, *J* = 7.5, 4.9 Hz, 1H, H_{Ar}), 7.33 (d, *J* = 7.8 Hz, 1H, H_{Ar}), 7.76 (q, *J* = 5.4, 3.7 Hz, 2H, H_{Ar} & NH_{Bn}), 8.55 (d, *J* = 4.8 Hz, 1H, H_{Ar}). |
| | | | ¹³C **NMR (101 MHz, DMSO-*d₆*) δ :** 5.4 (CH_{cPr}), 6.3 (2xCH_{2 cPr}), 10.3 (2xCH_{3 iPent}), 26.1 (2xCH_{2 iPent}), 47.2 (CH_{2 Bn}), 52.8 (CH_{iPent}), 84.5 (CH_{Ar}), 120.7 (CH_{Ar}), 122.3 (CH_{Ar}), 136.9 (CH_{Ar}), 139.5 (C_{q}), 139.7 (C_{q}), 149.0 (CH_{Ar}), 149.9 (C_{q}), 156.1 (C_{q}), 157.6 (C_{q}). |
| | | | **MS (ESI+) : m/z calcd for C₁₉H₂₅N₇ :** 352.22 [M+H]+, **found :** 352.32. |
| (63) | 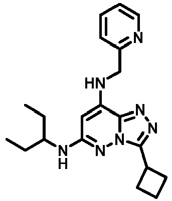 | > 95 % a | White solid. |
| | | | **¹H NMR (400 MHz, DMSO-*d₆*) δ :** 0.82 (t, *J* = 7.4 Hz, 6H, 2xCH_{3-CH2}), 1.45 (ddq, *J* = 28.0, 13.9, 7.0 Hz, 4H, 2xCH_{2-CH3}), 1.87 - 2.16 (m, 2H, CH_{2-CH2-CH}), 2.33 (dtd, *J* = 12.1, 8.6, 3.1 Hz, 2H, _{CH2-}CH2_{-CH}), 2.52 - 2.59 (m, 2H, _{CH2-}CH2_{-CH}), 3.54 (h, *J* = 6.6, 6.2 Hz, 1H, H_{iPent}), 3.86 (p, *J=* 8.7 Hz, 1H, H_{cBut}), 4.52 (d, *J=* 6.1 Hz, 2H, CH_{2Bn}), 5.43 (s, 1H, H_{Ar}), 6.34 (d, *J=* 7.8 Hz, 1H, NH_{iPent}), 7.28 (dd, *J* = 7.5, 4.9 Hz, 1H, H_{Ar}), 7.33 (d, *J* = 7.8 Hz, 1H, H_{Ar}), 7.72 - 7.86 (m, 2H, H_{Ar} & NH_{Bn}), 8.55 (d, *J* = 4.8 Hz, 1H, H_{Ar}). |
| | | | ¹³C NMR (101 MHz, **DMSO-*d₆*) δ :** 10.4 (2xCH_{3-CH2}), 18.6 (CH_{2-CH2-CH}), 25.9 (2x_{CH2}-CH_{2-CH}), 26.0 (2xCH_{2-CH3}), 29.7 (CH_{cBut}), 47.2 (CH_{2 Bn}), 53.0 (CH_{iPent}), 84.6 (CH_{Ar}), 120.7 (CH_{Ar}), 122.3 (CH_{Ar}), 136.9 (CH_{Ar}), 139.6 (C_{q}), 139.8 (C_{q}), 149.0 (CH_{Ar}), 150.9 (C_{q}), 156.0 (C_{q}), 157.6 (C_{q}). |
| | | | **MS (ESI+) : m/z calcd for C₂₀H₂₇N₇** : 366.24 [M+H]+, **found :** 366.33. |
| (64) | 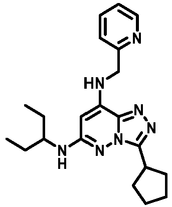 | > 97 % ^{a} | White solid. |
| | | | **¹H NMR (400 MHz, DMSO-*d₆*) δ :** 0.82 (t, *J* = 7.3 Hz, 6H, 2xCH_{3-CH2}), 1.44 (ddp, *J* = 28.3, 14.4, 7.4, 6.9 Hz, 4H, 2xCH_{2- CH3}), 1.60 - 1.84 (m, 4H, 2xCH_{2-CH2-CH}), 1.90 - 2.10 (m, 4H, 2x_{CH2-}CH_{2-CH}), 3.42 (p, *J* = 8.2 Hz, 1H, H_{cPent}), 3.54 (h, *J* = 6.4, 5.9 Hz, 1H, H_{iPent}), 4.52 (d, *J* = 6.1 Hz, 2H, CH_{2Bn}), 5.43 (s, 1H, H_{Ar}), 6.34 (d, *J* = 7.9 Hz, 1H, NH_{iPent}), 7.28 (dd, *J* = 7.4, 4.9 Hz, 1H, H_{Ar}), 7.34 (d, *J* = 7.9 Hz, 1H, H_{Ar}), 7.77 (t, *J* = 7.2 Hz, 2H, H_{Ar} & NH_{Bn}), 8.55 (d, *J* = 4.8 Hz, 1H, H_{Ar}). |
| | | | **¹³C NMR (101 MHz,** DMSO-*d₆*) δ **:** 10.4 (2xCH_{3-CH2}), 25.3 (2xCH_{2-CH3}), 26.0 (2xCH_{2-CH2-CH}), 29.6 (2_{XCH2-}CH_{2-CH}), 35.0 (CH_{cPent}), 47.2 (CH_{2 Bn}), 53.0 (CH_{iPent}), 84.5 (CH_{Ar}), 120.7 (CH_{Ar}), 122.3 (CH_{Ar}), 137.0 (CH_{Ar}), 139.8 (C_{q}), 139.8 (C_{q}), 149.0 (CH_{Ar}), 151.6 (C_{q}), 155.9 (C_{q}), 157.7 (C_{q}). |
| | | | **MS (ESI+)** : **m/z calcd** for **C₂₁H₂₉N₇** : 380.26 [M+H]+, **found** : 380.30. |
| (65) | 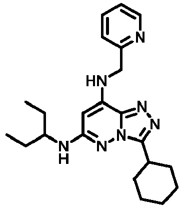 | 87 % ^{a} | White solid. |
| | | | ¹H **NMR (400 MHz,** DMSO-*d₆*) **δ :** 0.83 (t, *J* = 7.4 Hz, 6H, 2xCH_{3-CH2}), 1.22 - 1.55 (m, 7H, 2xCH_{2-CH3} & CH_{2 cHex} & H_{(CH2) cHex}), 1.70 (qd, *J* = 12.4, 12.0, 3.3 Hz, 3H, CH₂ chex & H_{(CH2) chex}), 1.77 - 1.86 (m, 2H, CH₂ chex), 1.93 - 2.06 (m, 2H, CH₂ chex), 3.04 (tt, *J=* 11.4, 3.6 Hz, 1H, HcHex), 3.48 (hept, *J* = 5.7, 5.2 Hz, 1H, H_{iPent}), 4.52 (d, *J=* 6.1 Hz, 2H, CH_{2Bn}), 5.42 (s, 1H, H_{Ar}), 6.35 (d, *J=* 7.7 Hz, 1H, NH_{iPent}), 7.28 (ddd, *J* = 7.6, 4.9, 1.2 Hz, 1H, H_{Ar}), 7.34 (d, *J=* 7.9 Hz, 1H, H_{Ar}), 7.68 - 7.88 (m, 2H, H_{Ar} & NH_{Bn}), 8.55 (ddd, *J* = 4.9, 1.8, 0.9 Hz, 1H, H_{Ar}). |
| | | | **¹³C NMR (101 MHz,** DMSO-*d₆*) **δ :** 10.5 (2xCH_{3-CH2}), 25.6 (2xCH_{2 cHex}), 25.7 (CH₂ cHex), 26.1 (2xCH_{2-CH3}), 29.2 (2xCH_{2 cHex}), 34.0 (CH_{cHex}), 47.2 (CH2Bn), 53.3 (CH_{iPent}), 84.5 (CH_{Ar}), 120.7 (CH_{Ar}), 122.3 (CH_{Ar}), 136.9 (CH_{Ar}), 139.4 (C_{q}), 139.8 (C_{q}), 149.0 (CH_{Ar}), 152.0 (C_{q}), 155.8 (C_{q}), 157.6 (C_{q}). |
| | | | **MS (ESI+)** : **m/z calcd for C₂₂H₃₁N₇** : 394.27 [M+H]+, **found** : 394.41. |
| (66) | 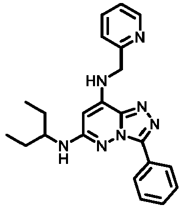 | > 96 % ^{a} | White solid. |
| | | | **¹H NMR (400 MHz,** DMSO-*d₆*) **δ :** 0.87 (t, *J* = 7.4 Hz, 6H, 2xCH_{3-CH2}), 1.43 - 1.63 (m, 4H, 2xCH_{2-CH3}), 3.55 (h, *J=* 6.4 Hz, 1H, H_{iPent}), 4.57 (d, *J* = 6.1 Hz, 2H, CH_{2 Bn}), 5.55 (s, 1H, H_{Ar}), 6.57 (d, *J* = 7.7 Hz, 1H, NH_{iPent}), 7.30 (dd, *J* = 7.5, 4.9 Hz, 1H, H_{Ar}), 7.38 (d, *J=* 7.9 Hz, 1H, H_{Ar}), 7.43 - 7.50 (m, 1H, H_{Ar}), 7.53 (dd, *J* = 8.3, 6.6 Hz, 2H, 2xH_{Ar}), 7.78 (td, *J* = 7.7, 1.8 Hz, 1H, H_{Ar}), 7.96 (t, *J=* 6.2 Hz, 1H, NH_{Bn}), 8.44 - 8.51 (m, 2H, 2xH_{Ar}), 8.57 (dd, *J* = 5.1, 1.7 Hz, 1H, H_{Ar}). |
| | | | **¹³C NMR (101 MHz, DMSO-*d₆*) δ :** 10.5 (2xCH_{3-CH2}), 26.0 (2xCH_{2-CH3}), 47.2 (CH₂), 53.6 (CH_{iPent}), 84.7 (CH_{Ar}), 120.8 (CH_{Ar}), 122.4 (CH_{Ar}), 126.5 (2xCH_{Ar}), 127.5 (C_{q}), 128.5 (2xCH_{Ar}), 129.1 (CH_{Ar}), 137.0 (CH_{Ar}), 139.9 (C_{q}), 140.5 (C_{q}), 146.2 (C_{q}), 149.0 (CH_{Ar}), 156.5 (C_{q}), 157.5 (C_{q}). |
| | | | **MS (ESI+) : m/z calcd for C₂₂H₂₅N₇ :** 388.22 [M+H]+, **found :** 388.33. |
| (67) | 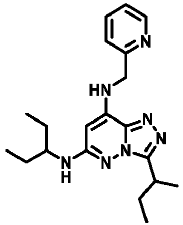 | > 95 % ^{a} | White solid. |
| | | | **¹H NMR (400 MHz, DMSO-*d₆*) δ :** 0.82 (t, *J* = 7.4 Hz, 9H, 3xCH_{3-CH2}), 1.34 (d, *J* = 7.0 Hz, 3H, CH_{3-CH}), 1.37 - 1.54 (m, 4H, 2xCH_{2-CH3 iPent}), 1.81 (ddt, *J* = 94.8, 13.7, 7.1 Hz, 2H, CH_{2-CH3} sec-but), 3.16 (h, *J =* 7.0 Hz, 1H, H_{C-CH3}), 3.53 (h, *J=* 6.0 Hz, 1H, H_{iPent}), 4.52 (d, *J* = 6.1 Hz, 2H, CH_{2Bn}), 5.43 (s, 1H, H_{Ar}), 6.34 (d, *J* = 7.9 Hz, 1H, NH_{iPent}), 7.29 (dd, *J* = 7.4, 4.9 Hz, 1H, H_{Ar}), 7.35 (d, *J=* 7.8 Hz, 1H, H_{Ar}), 7.71 - 7.83 (m, 2H, H_{Ar} & NH_{Bn}), 8.55 (d, *J=* 4.9 Hz, 1H, H_{Ar}). |
| | | | **¹³C NMR (101 MHz, DMSO-*d₆*) δ :** 10.8 (CH_{3-CH2 iPent}), 10.9 (CH_{3-CH2 iPent}), 12.2 (CH_{3-CH2 sec-but}), 17.7 (CH_{3-CH}), 26.5 (CH₂₋ CH3 iPent), 26.6 (CH_{2-CH3 iPent}), 27.0 (CH_{2-CH3 sec-but}), 31.9 (CH_{sec-but}), 47.7 (CH_{2 Bn}), 53.5 (CH_{iPent}), 85.0 (CH_{Ar}), 121.2 (CH_{Ar}), 122.8 (CH_{Ar}), 137.4 (CH_{Ar}), 140.0 (C_{q}), 140.3 (C_{q}), 149.5 (CH_{Ar}), 152.4 (C_{q}), 156.3 (C_{q}), 158.1 (C_{q}). |
| | | | **MS (ESI+) : m/z calcd for C₂₀H₂₉N₇ :** 368.26 [M+H]+, **found :** 368.37. |
| (68) | 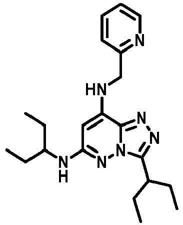 | > 96 % ^{a} | White solid. |
| | | | **¹H NMR (400 MHz, DMSO-*d₆*) δ :** 0.76 (t, *J* = 7.4 Hz, 6H, 2xCH_{3-CH2}), 0.82 (t, *J* = 7.4 Hz, 6H, 2xCH_{3-CH2}), 1.33 - 1.57 (m, 4H, 2xCH_{2-CH3}), 1.68 - 2.01 (m, 4H, 2xCH_{2-CH3}), 3.04 (tt, *J* = 8.8, 5.5 Hz, 1H, H_{iPent}), 3.52 (h, *J=* 6.6 Hz, 1H, H_{iPent}), 4.52 (d, *J* = 6.1 Hz, 2H, CH_{2Bn}), 5.43 (s, 1H, H_{Ar}), 6.33 (d, *J=* 7.8 Hz, 1H, NH_{iPent}), 7.29 (dd, *J* = 7.5, 4.8 Hz, 1H, H_{Ar}), 7.36 (d, *J=* 7.9 Hz, 1H, H_{Ar}), 7.71 - 7.86 (m, 2H, NH_{Bn} & H_{Ar}), 8.51 - 8.64 (m, 1H, H_{Ar}). |
| | | | **¹³C NMR (101 MHz, DMSO-*d₆*) δ :** 10.4 (2xCH_{3-CH2}), 11.8 (2xCH_{3-CH2}), 24.7 (2xCH_{2-CH3}), 26.0 (2xCH_{2-CH3}), 38.7 (CH_{iPent}), 47.2 (CH_{2 Bn}), 53.0 (CH_{iPent}), 84.5 (CH_{Ar}), 120.8 (CH_{Ar}), 122.3 (CH_{Ar}), 136.9 (CH_{Ar}), 139.4 (C_{q}), 139.8 (C_{q}), 149.0 (CH_{Ar}), 150.8 (C_{q}), 155.8 (C_{q}), 157.7 (C_{q}). |
| | | | **MS (ESI+) : m/z calcd for C₂₁H₃₁N₇ :** 382.27 [M+H]+, **found** : 382.35. |
| (69) | 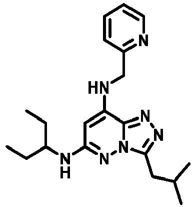 | 82 % ^{a} | Beige solid. |
| | | | **¹H NMR (400 MHz, DMSO-*d₆*) δ :** 0.82 (t, *J* = 7.4 Hz, 6H, 2xCH_{3-CH2}), 0.92 (d, *J* = 6.7 Hz, 6H, 2xCH_{3 iPr}), 1.34 - 1.57 (m, 4H, 2xCH_{2-CH3}), 2.19 (hept, *J* = 6.8 Hz, 1H, H_{iPr}), 2.77 (d, *7 =* 7.0 Hz, 2H, CH_{2- CH}), 3.53 (h, *J* = 6.5 Hz, 1H, H_{iPent}), 4.53 (d, *J* = 6.1 Hz, 2H, CH_{2Bn}), 5.44 (s, 1H, H_{Ar}), 6.35 (d, *J* = 7.9 Hz, 1H, NH_{-CH}), 7.26 - 7.31 (m, 1H, H_{Ar}), 7.35 (d, *J* = 7.9 Hz, 1H, H_{Ar}), 7.72 - 7.81 (m, 2H, NH_{Bn} & H_{Ar}), 8.46 - 8.61 (m, 1H, H_{Ar}). |
| | | | **¹³C NMR (101 MHz, DMSO-*d₆*) δ :** 10.4 (2xCH_{3-CH2}), 22.3 (2xCH_{iPr}), 26.1 (2xCH_{2- CH3}), 26.4 (CH_{iPr}), 32.4 (CH_{2-CH}), 47.2 (CH_{2 Bn}), 53.1 (CH_{iPent}), 84.5 (CH_{Ar}), 120.8 (CH_{Ar}), 122.3 (CH_{Ar}), 136.9 (CH_{Ar}), 139.2 (C_{q}), 139.8 (C_{q}), 148.1 (C_{q}), 149.0 (CH_{Ar}), 156.0 (C_{q}), 157.6 (C_{q}). |
| | | | **MS (ESI+)** : **m/z calcd for C₂₀H₂₉N₇** : 368.26 [M+H]+, **found** : 368.34. |
| (70) | 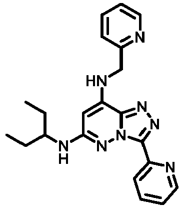 | > 97 % ^{a} | White solid. |
| | | | **¹H NMR (400 MHz, DMSO-*d₆*) δ :** 0.85 (t, *J* = 7.3 Hz, 6H, 2xCH_{3-CH2}), 1.48 (dhept, *J* = 14.0, 6.8 Hz, 4H, 2xCH_{2-CH3}), 3.52 (h, *J* = 6.5 Hz, 1H, H_{iPent}), 4.58 (d, *J* = 6.1 Hz, 2H, CH_{2Bn}), 5.58 (s, 1H, H_{Ar}), 6.58 (d, *J* = 7.7 Hz, 1H, NH_{iPent}), 7.30 (dd, *J* = 7.4, 4.9 Hz, 1H, H_{Ar}), 7.39 (d, *J* = 7.9 Hz, 1H, H_{Ar}), 7.48 (dd, *J* = 7.6, 4.8 Hz, 1H, H_{Ar}), 7.79 (td, *J* = 7.7, 1.8 Hz, 1H, H_{Ar}), 7.94 - 8.06 (m, 2H, H_{Ar} & NH_{Bn}), 8.51 (d, *J* = 8.0 Hz, 1H, H_{Ar}), 8.57 (d, *J* = 4.8 Hz, 1H, H_{Ar}), 8.75 (d, *J* = 4.7 Hz, 1H, H_{Ar}). |
| | | | *¹³C NMR (101 MHz,* **DMSO-*d₆*) δ :** 11.0 (2xCH_{3-CH2}), 26.5 (2xCH_{2-CH3}), 47.7 (CH_{2 Bn}), 54.0 (CH_{iPent}), 85.4 (CH_{Ar}), 121.3 (CH_{Ar}), 122.9 (CH_{Ar}), 123.3 (CH_{Ar}), 124.4 (CH_{Ar}), 137.0 (CH_{Ar}), 137.5 (CH_{Ar}), 140.3 (C_{q}), 140.8 (C_{q}), 146.7 (C_{q}), 147.0 (C_{q}), 149.5 (CH_{Ar}), 150.3 (CH_{Ar}), 157.1 (C_{q}), 158.0 (C_{q}). |
| | | | *MS (ESI+)* : *m*/*z calcd* **for** *C₂₁H₂₄N₈* : 389.22 [M+H]+, *found* : 389.30. |
| (71) | 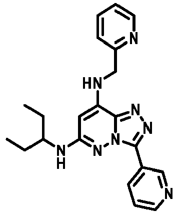 | > 97 % ^{a} | Grey solid. |
| | | | **¹H NMR (400 MHz, DMSO-*d₆*) δ :** 0.86 (t, *J* = 7.3 Hz, 6H, 2xCH_{3-CH2}), 1.50 (ddp, *J* = 21.0, 14.2, 6.9 Hz, 4H, 2xCH_{2-CH3}), 3.56 (h, *J* = 6.4 Hz, 1H, H_{iPent}), 4.58 (d, *J* = 6.1 Hz, 2H, CH_{2Bn}), 5.58 (s, 1H, H_{Ar}), 6.65 (d, *J* = 7.8 Hz, 1H, NH_{iPent}), 7.30 (dd, *J* = 7.4, 4.9 Hz, 1H, H_{Ar}), 7.38 (d, *J* = 7.9 Hz, 1H, H_{Ar}), 7.58 (dd, *J* = 8.1, 4.8 Hz, 1H, H_{Ar}), 7.75 - 7.86 (m, 1H, H_{Ar}), 8.03 (t, *J* = 6.2 Hz, 1H, NH_{Bn}), 8.57 (d, *J* = 4.8 Hz, 1H, H_{Ar}), 8.66 (d, *J* = 4.8 Hz, 1H, H_{Ar}), 8.76 (dd, *J* = 8.1, 2.5 Hz, 1H, H_{Ar}), 9.62 (d, *J* = 2.1 Hz, 1H, H_{Ar}). |
| | | | ¹³C **NMR (101 MHz, DMSO-*d₆*) δ :** 10.5 (2xCH_{3-CH2}), 26.0 (2xCH_{2-CH3}), 47.2 (CH_{2 Bn}), 53.6 (CH_{iPent}), 85.0 (CH_{Ar}), 120.8 (CH_{Ar}), 122.4 (CH_{Ar}), 123.6 (CH_{Ar}), 123.7 (C_{q}), 133.5 (CH_{Ar}), 137.0 (CH_{Ar}), 139.9 (C_{q}), 140.7 (C_{q}), 144.3 (C_{q}), 147.2 (CH_{Ar}), 149.1 (CH_{Ar}), 149.8 (CH_{Ar}), 156.7 (C_{q}), 157.5 (C_{q}). |
| | | | **MS (ESI+) : m/z calcd for C₂₁H₂₄N₈** : 389.22 [M+H]+, **found** : 389.30. |
| (72) | 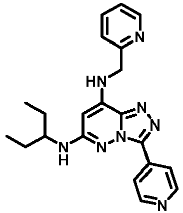 | > 97 % ^{a} | Beige solid. |
| | | | **¹H NMR (400 MHz, DMSO-*d₆*) δ :** 0.88 (t, *J* = 7.4 Hz, 6H, 2xCH_{3-CH2}), 1.52 (dq, *J* = 18.3, 7.0 Hz, 4H, 2xCH_{2-CH3}), 3.60 (h, *J* = 6.4 Hz, 1H, H_{iPent}), 4.58 (d, *J* = 6.1 Hz, 2H, CH_{2Bn}), 5.60 (s, 1H, H_{Ar}), 6.70 (d, *J* = 7.7 Hz, 1H, NH_{iPent}), 7.30 (dd, *J* = 7.5, 4.9 Hz, 1H, H_{Ar}), 7.38 (d, *J* = 7.9 Hz, 1H, H_{Ar}), 7.78 (td, *J* = 7.7, 1.8 Hz, 1H, H_{Ar}), 8.07 (t, *J* = 6.2 Hz, 1H, NH_{Bn}), 8.42 (d, *J* = 5.6 Hz, 2H, 2xH_{Ar}), 8.57 (d, *J* = 4.8 Hz, 1H, H_{Ar}), 8.74 (d, *J* = 5.3 Hz, 2H, 2xH_{Ar}). |
| | | | **¹³C NMR (101 MHz, DMSO-*d₆*) δ :** 10.5 (2xCH_{3-CH2}), 26.0 (2xCH_{2-CH3}), 47.2 (CH_{2 Bn}), 53.7 (CH_{iPent}), 85.1 (CH_{Ar}), 119.9 (CH_{Ar}), 120.8 (CH_{Ar}), 122.4 (CH_{Ar}), 134.3 (C_{q}), 137.0 (CH_{Ar}), 139.8 (C_{q}), 141.2 (C_{q}), 144.2 (C_{q}), 149.1 (CH_{Ar}), 150.1 (CH_{Ar}), 156.9 (C_{q}), 157.4 (C_{q}). |
| | | | **MS (ESI+) : m/z calcd for** C₂₁H₂₄N₈ **:** 389.22 [M+H]+, **found :** 389.30. |
| (73) | 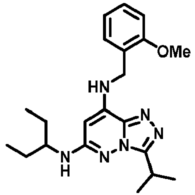 | 91 % ^{a} | White solid. |
| | | | **¹H NMR (400 MHz, DMSO-*d₆*) δ :** 0.82 (t, *J* = 7.4 Hz, 6H, 2xCH_{3 iPent}), 1.36 (d, *J* = 7.0 Hz, 6H, 2xCH_{3 iPr}), 1.38 - 1.56 (m, 4H, 2xCH_{2 iPent}), 3.30 (d, *J* = 6.8 Hz, 1H, H_{iPr}), 3.55 (h, *J* = 6.8 Hz, 1H, H_{iPent}), 3.85 (s, 3H, OCH₃), 4.39 (d, *J* = 6.2 Hz, 2H, CH_{2Bn}), 5.36 (s, 1H, H_{Ar}), 6.29 (d, *J* = 8.0 Hz, 1H, NH_{iPent}), 6.89 (td, *J* = 7.5, 1.1 Hz, 1H, H_{Ar}), 7.02 (dd, *J* = 8.3, 1.1 Hz, 1H, H_{Ar}), 7.13 (dd, *J* = 7.5, 1.7 Hz, 1H, H_{Ar}), 7.24 (td, *J* = 7.9, 1.7 Hz, 1H, H_{Ar}), 7.55 (t, *J* = 6.2 Hz, 1H, NH_{Bn}). |
| | | | **^{*1*3}C NMR (101 MHz, DMSO-*d₆*) δ :** 10.4 (2xCH_{3 iPent}), 19.5 (2xCH_{3 iPr}), 24.8 (CH_{iPr}), 26.1 (2xCH_{2 iPent}), 40.6 (CH_{2 Bn}), 52.9 (CH_{iPent}), 55.3 (OCH₃), 84.1 (CH_{Ar}), 110.5 (CH_{Ar}), 120.2 (CH_{Ar}), 125.3 (C_{q}), 126.5 (CH_{Ar}), 128.1 (CH_{Ar}), 139.7 (C_{q}), 140.0 (C_{q}), 152.7 (C_{q}), 156.0 (C_{q}), 156.6 (C_{q}). |
| | | | **MS (ESI+) : m/z calcd for C₂₁H₃₀N₅ :** 383.26 [M+H]+, **found** : 383.31. |
| (74) | 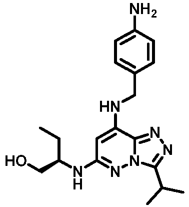 | > 95 % ^{a} | White solid. |
| | | | **¹H NMR (400 MHz, DMSO-*d₆*) δ :** 0.86 (t, *J* = 7.4 Hz, 3H, CH_{3-CH2}), 1.35 (d, *J* = 6.9 Hz, 6H, 2xCH_{3 iPr}), 1.39 - 1.71 (m, 2H, CH_{2-CH3}), 3.25 - 3.32 (m, 1H, H_{iPr}), 3.44 (ddt, *J* = 20.8, 10.6, 5.1 Hz, 2H, CH_{2-OH}), 3.63 (q, *J* = 6.8 Hz, 1H, H_{Alk}), 4.12 - 4.30 (m, 2H, CH_{2 Bn}), 4.58 (t, *J* = 5.4 Hz, 1H, OH), 4.95 (s, 2H, NH₂), 5.50 (s, 1H, H_{Ar}), 6.29 (d, *J* = 7.8 Hz, 1H, NH_{Alk}), 6.47 - 6.58 (m, 2H, 2xH_{Ar}), 7.02 (d, *J* = 8.1 Hz, 2H, 2xH_{Ar}), 7.61 (t, *J* = 6.2 Hz, 1H, NH_{Bn}). |
| | | | ¹³C NMR (101 MHz, **DMSO-*d₆*) δ :** 10.6 (CH_{3-CH2}), 19.6 (2xCH_{3 iPr}), 23.6 (CH_{2-CH3}), 24.7 (CH_{iPr}), 45.0 (CH_{2 Bn}), 53.8 (CH_{-NH}), 62.0 (CH_{3-CH2}), 84.2 (CH_{Ar}), 113.8 (2xCH_{Ar}), 125.1 (C_{q}), 127.9 (2xCH_{Ar}), 139.7 (C_{q}), 139.7 (C_{q}), 147.6 (C_{q}), 152.6 (C_{q}), 155.9 (C_{q}). |
| | | | **MS (ESI+) : m/z calcd for C₁₉H₂₇N₇O :** 370.23 [M+H]+, found : 370.36. |
| (75) | 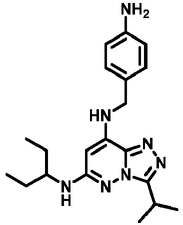 | > 96 % ^{a} | Yellow solid. |
| | | | **¹H NMR (400 MHz, DMSO-*d₆*) δ :** 0.84 (t, *J* = 7.4 Hz, 6H, 2xCH_{3-CH2}), 1.35 (d, *J* = 7.0 Hz, 6H, 2xCH_{3 iPr}), 1.47 (ddt, *J* = 28.5, 13.9, 7.0 Hz, 4H, 2xCH_{2-CH3}), 3.25 - 3.30 (m, 1H, H_{iPr}), 3.56 (dd, *J* = 13.6, 6.7 Hz, 1H, H_{iPent}), 4.22 (d, *J* = 6.2 Hz, 2H, CH_{2Bn}), 4.96 (s, 2H, NH₂), 5.46 (s, 1H, H_{Ar}), 6.29 (d, *J* = 8.0 Hz, 1H, NH), 6.51 (d, *J* = 8.1 Hz, 2H, 2xH_{Ar}), 7.02 (d, *J* = 8.1 Hz, 2H, 2xH_{Ar}), 7.56 (t, *J* = 6.2 Hz, 1H, NH_{Bn}). |
| | | | **¹³C NMR (101 MHz, DMSO-*d₆*) δ :** 10.4 (2xCH_{3 iPr}), 19.5 (2xCH_{3 iPr}), 24.8 (CH_{iPr}), 26.2 (2xCH_{2-CH3}), 45.0 (CH_{2 Bn}), 53.0 (CH_{iPent}), 84.0 (CH_{Ar}), 113.8 (2xCH_{Ar}), 125.0 (C_{q}), 127.9 (2xCH_{Ar}), 139.7 (C_{q}), 139.7 (C_{q}), 147.6 (C_{q}), 152.6 (C_{q}), 156.0 (C_{q}). |
| | | | **MS (ESI+)** : **m/z calcd for C₂₀H₂₉N₇** : 368.26 [M+H]+, **found** : 368.41. |
| (76) | 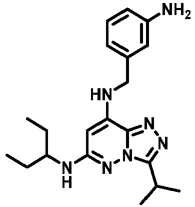 | > 96 % ^{a} | Light brown solid. |
| | | | **¹H NMR (400 MHz, DMSO-*d₆*) δ :** 0.83 (t, *J* = 7.4 Hz, 6H, 2xCH_{3-CH2}), 1.36 (d, *J* = 7.0 Hz, 6H, 2xCH_{3 iPr}), 1.40 - 1.57 (m, 4H, 2xCH_{2-CH3}), 3.28 - 3.31 (m, 1H, H_{iPr}), 3.55 (q, *J* = 6.6 Hz, 1H, H_{iPent}), 4.29 (d, *J* = 6.2 Hz, 2H, CH_{2Bn}), 5.02 (s, 2H, NH₂), 5.39 (s, 1H, H_{Ar}), 6.31 (d, *J* = 7.9 Hz, 1H, NH), 6.38 - 6.44 (m, 1H, H_{Ar}), 6.47 (d, *J* = 7.5 Hz, 1H, H_{Ar}), 6.51 (t, *J* = 2.0 Hz, 1H, H_{Ar}), 6.96 (t, *J* = 7.7 Hz, 1H, H_{Ar}), 7.67 (t, *J* = 6.4 Hz, 1H, NH_{Bn}). |
| | | | **¹³C NMR (101 MHz, DMSO-*d₆*) δ :** N.D. |
| | | | **MS (ESI+) : m/z calcd for C₂₀H₂₉N₇ :** 368.26 [M+H]+, **found :** 368.47. |
| (77) | 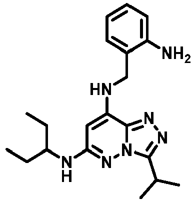 | > 93 % ^{a} | Beige solid. |
| | | | **¹H NMR (400 MHz, DMSO-*d₆*) δ :** 0.83 (t, *J* = 7.4 Hz, 6H, 2xCH_{3-CH2}), 1.36 (d, *J* = 6.9 Hz, 6H, 2xCH_{3 iPr}), 1.38 - 1.57 (m, 4H, 2xCH_{2-CH3}), 3.35 (s, 1H, H_{iPr}), 3.56 (h, *J* = 6.8, 6.3 Hz, 1H, H_{iPent}), 4.23 (d, *J* = 6.2 Hz, 2H, CH_{2Bn}), 5.13 (s, 2H, NH₂), 5.45 (s, 1H, H_{Ar}), 6.35 (d, *J* = 8.0 Hz, 1H, NH_{iPent}), 6.49 (td, *J* = 7.4, 1.2 Hz, 1H, H_{Ar}), 6.63 (dd, *J* = 8.0, 1.2 Hz, 1H, H_{Ar}), 6.95 (ddd, *J* = 8.5, 7.4, 1.6 Hz, 1H, H_{Ar}), 7.02 (dd, *J* = 7.5, 1.6 Hz, 1H, H_{Ar}), 7.63 (t, *J* = 6.1 Hz, 1H, NH_{Bn}). |
| | | | **¹³C NMR (101 MHz, DMSO-*d₆*) δ :** 10.4 (2xCH_{3-CH2}), 19.5 (2xCH_{3 iPr}), 24.8 (CH_{iPr}), 26.2 (2xCH_{2-CH3}), 42.6 (CH_{2 Bn}), 53.0 (CH_{iPent}), 84.1 (CH_{Ar}), 114.7 (CH_{Ar}), 115.6 (CH_{Ar}), 120.2 (C_{q}), 127.5 (CH_{Ar}), 127.6 (CH_{Ar}), 139.7 (C_{q}), 139.9 (C_{q}), 146.2 (C_{q}), 152.7 (C_{q}), 156.0 (C_{q}). |
| | | | **MS (ESI+) : m/z calcd for C₂₀H₂₉N₇ :** 368.26 [M+H]+, **found** : 368.42. |
| (78) | 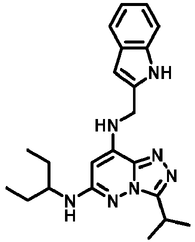 | > 95 % ^{a} | Beige solid. |
| | | | **¹H NMR (400 MHz, DMSO-*d₆*) δ :** 0.82 (t, *J* = 7.4 Hz, 6H, 2xCH_{3-CH2}), 1.36 (d, *J* = 7.0 Hz, 6H, 2xCH_{3 iPr}), 1.39 - 1.57 (m, 4H, 2xCH_{2-CH3}), 3.22 - 3.46 (m, 1H, H_{iPr}), 3.56 (q, *J* = 6.5 Hz, 1H, H_{iPent}), 4.59 (d, *J* = 6.1 Hz, 2H, CH_{2Bn}), 5.58 (s, 1H, H_{Ar}), 6.33 (d, *J* = 9.1 Hz, 2H, NH_{Alk} & H_{Indole}), 6.94 (t, *J* = 7.4 Hz, 1H, H_{Indole}), 7.02 (t, *J* = 7.8 Hz, 1H, H_{Indole}), 7.32 (d, *J* = 8.1 Hz, 1H, H_{Indole}), 7.43 (d, *J* = 7.8 Hz, 1H, H_{Indole}), 7.56 (t, *J* = 6.2 Hz, 1H, NH_{Bn}), 10.96 (s, 1H, NH_{Indole}). |
| | | | **¹³C NMR (101 MHz, DMSO-*d₆*) δ :** 10.4 (2xCH_{3-CH2}), 19.5 (2xCH_{3 iPr}), 24.8 (CH_{iPr}), 26.2 (2xCH_{2-CH3}), 39.7 (CH_{2 Bn}), 53.0 (CH_{Ar}), 84.7 (CH_{Indole}), 99.2 (CH_{Indole}), 111.1 (CH_{Indole}), 118.9 (CH_{Indole}), 119.6 (CH_{Indole}), 120.7 (CH_{Indole}), 127.9 (C_{q}), 136.1 (C_{q}), 136.2 (C_{q}), 139.7 (C_{q}), 139.9 (C_{q}), 152.7 (C_{q}), 155.9 (C_{q}). |
| | | | **MS (ESI+) : m/z calcd for C₂₂H₂₉N₇** : 392.26 [M+H]+, **found :** 392.33. |
| (79) | 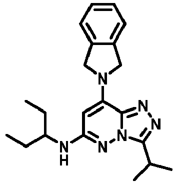 | > 97 % ^{a} | Solid beige. |
| | | | **¹H NMR (400 MHz, DMSO-*d₆*) δ :** 0.89 (t, *J =* 7.4 Hz, 6H, 2xCH_{3-CH2}), 1.38 (d, *J =* 6.9 Hz, 6H, 2xCH_{3 iPr}), 1.44 - 1.63 (m, 4H, 2xCH_{2-CH3}), 3.28 - 3.35 (m, 1H, H_{iPr}), 3.58 - 3.70 (m, 1H, H_{iPent}), 5.07 (s, 4H, 2xCH_{2- N}), 5.50 (s, 1H, H_{Ar}), 6.39 (d, *J* = 8.0 Hz, 1H, NH), 7.34 (dd, *J* = 5.6, 3.1 Hz, 2H, 2xH_{Ar}), 7.45 (dt, *J* = 7.5, 3.6 Hz, 2H, 2xH_{Ar}). |
| | | | **¹³C NMR (101 MHz, DMSO-*d₆*) δ :** 10.4 (2xCH_{3-CH2}), 19.4 (2xCH_{3 iPr}), 24.8 (CH_{iPr}), 26.3 (2xCH_{2-CH3}), 53.0 (CH_{iPent}), 55.4 (2xCH_{2-N}), 86.3 (CH_{Ar}), 122.7 (2xCH_{Ar}), 127.4 (2xCH_{Ar}), 136.6 (2xC_{q}), 139.5 (C_{q}), 140.4 (C_{q}), 152.5 (C_{q}), 155.9 (C_{q}). |
| | | | **MS (ESI+) : m/z calcd for C₂₁H₂₈N₆ :** 365.24 [M+H]+, **found** : 365.42. |
| (80) | 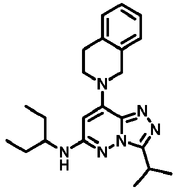 | 92 % ^{a} | Light brown oil. |
| | | | **¹H NMR (400 MHz, DMSO-*d₆*) δ :** 0.88 (t, *J =* 7.4 Hz, 6H, 2xCH_{3-CH2}), 1.36 (d, *J* = 6.9 Hz, 6H, 2xCH_{3 iPr}), 1.41 - 1.62 (m, 4H, 2xCH_{2-CH3}), 2.97 (t, *J=* 5.9 Hz, 2H, CH_{2-CH2}), 3.28 - 3.36 (m, 1H, H_{iPr}), 3.61 (h, *J =* 6.5 Hz, 1H, H_{iPent}), 4.22 (t, *J =* 5.9 Hz, 2H, CH_{2-CH2}), 4.89 (s, 2H, CH_{2 THQ}), 5.83 (s, 1H, H_{Ar}), 6.43 (d, *J=* 8.0 Hz, 1H, NH), 7.21 (s, 4H, 4xH_{Ar}). |
| | | | **¹³C NMR (101 MHz, DMSO-*d₆*) δ :** 10.3 (2xCH_{3-CH2}), 19.4 (2xCH_{3 iPr}), 24.8 (CH_{iPr}), 26.2 (2xCH_{2-CH3}), 27.7 (CH_{2-CH2}), 45.3 (CH_{2-CH2}), 49.1 (CH_{2 THQ}), 53.0 (CH_{iPent}), 88.9 (CH_{Ar}), 126.1 (CH_{Ar}), 126.3 (CH_{Ar}), 126.5 (CH_{Ar}), 128.5 (CH_{Ar}), 133.4 (C_{q}), 134.4 (C_{q}), 140.6 (C_{q}), 141.4 (C_{q}), 152.4 (C_{q}), 155.7 (C_{q}). |
| | | | **MS (ESI+)** : **m/z calcd for C₂₂H₃₀N₆** : 379.26 [M+H]+, **found** : 379.46. |
| (81) | 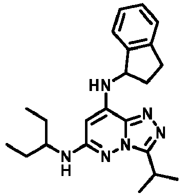 | > 97 % ^{a} | Beige solid. |
| | | | **¹H NMR (400 MHz, DMSO-*d₆*) δ :** 0.88 (td, *J* = 7.4, 4.4 Hz, 6H, 2xCH_{3-CH2}), 1.37 (d, *J* = 6.9 Hz, 6H, 2xCH_{3 iPr}), 1.40 - 1.65 (m, 4H, 2xCH_{2-CH3}), 2.14 (dq, *J* = 12.5, 8.4 Hz, 1H, H_{(CH2)indane}), 2.46 (dt, *J* = 7.7, 3.9 Hz, 1H, H_{(CH2)indane}), 2.85 (dt, *J* = 16.0, 8.2 Hz, 1H, H_{(CH2)indane}), 3.02 (ddd, *J* = 15.9, 8.8, 3.4 Hz, 1H, H_{(CH2)indane}), 3.26 - 3.42 (m, 1H, H_{iPr}), 3.53 - 3.69 (m, 1H, H_{iPent}), 5.22 (s, 1H, H_{indane}), 5.80 (s, 1H, H_{Ar}), 6.33 (d, *J* = 8.0 Hz, 1H, NH_{iPent}), 7.12 - 7.38 (m, 5H, NH_{indane} & 4xH_{Ar}). |
| | | | **¹³C NMR (101 MHz, DMSO-***d₆***) δ :** 10.4 (CH_{3-CH2}), 10.4 (CH_{3-CH2}), 19.5 (2xCH_{3 iPr}), 24.8 (CH_{iPr}), 26.2 (2xCH_{2-CH3}), 29.7 (CH_{2 indane}), 32.1 (CH_{2 indane}), 53.0 (CH_{iPent}), 57.3 (CH_{indane}), 84.6 (CH_{Ar}), 123.9 (CH_{Ar}), 124.7 (CH_{Ar}), 126.4 (CH_{Ar}), 127.6 (CH_{Ar}), 139.7 (C_{q}), 139.8 (C_{q}), 143.0 (C_{q}), 143.6 (C_{q}), 152.7 (C_{q}), 156.1 (C_{q}). |
| | | | **MS (ESI+) : m/z calcd for C₂₂H₃₀N₆ :** 379.26 [M+H]+, found : 379.33. |
| (82) | 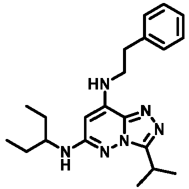 | > 97 % ^{a} | White amorphous solid. |
| | | | **¹H NMR (400 MHz, DMSO-*d₆*) δ :** 0.88 (t, *J* = 7.4 Hz, 6H, 2xCH_{3-CH2}), 1.36 (d, *J* = 7.0 Hz, 6H, 2xCH_{3 iPr}), 1.42 - 1.64 (m, 4H, 2xCH_{2-CH3}), 2.92 (dd, *J* = 8.8, 6.5 Hz, 2H, CH_{2-CH2-NH}), 3.25 - 2.27 (m, 1H, H_{iPr}), 3.40 (q, *J* = 6.8 Hz, 2H, CH_{2-NH}), 3.61 (h, *J* = 6.5 Hz, 1H, H_{iPent}), 5.63 (s, 1H, H_{Ar}), 6.31 (d, *J* = 8.0 Hz, 1H, NH_{iPent}), 7.19 - 7.38 (m, 6H, 5xH_{Ar} & NH_{-CH2}). |
| | | | **¹³C NMR (101 MHz, DMSO-*d₆*) δ :** 10.4 (2xCH_{3-CH2}), 19.5 (2xCH_{3-CH2}), 24.8 (CH_{iPr}), 26.3 (2xCH_{2-CH3}), 33.8 (CH_{2-CH2- NH}), 43.6 (CH_{2-NH}), 52.9 (CH_{iPent}), 83.6 (CH_{Ar}), 126.2 (CH_{Ar}), 128.4 (2xCH_{Ar}), 128.7 (2xCH_{Ar}), 139.2 (C_{q}), 139.7 (C_{q}), 139.7 (C_{q}), 152.6 (C_{q}), 156.1 (C_{q}). |
| | | | **MS (ESI+) : m/z calcd for C₂₁H₃₀N₆ :** 367.26 [M+H]+, **found** : 367.33. |
| (83) | 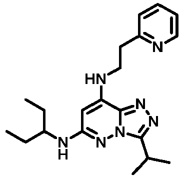 | > 95 % ^{a} | White solid. |
| | | | **¹H NMR (400 MHz, DMSO-*d₆*) δ :** 0.87 (t, *J* = 7.4 Hz, 6H, 2xCH_{3-CH2}), 1.35 (d, *J* = 6.9 Hz, 6H, 2xCH_{3 iPr}), 1.39 - 1.64 (m, 4H, 2xCH_{2-CH3}), 3.09 (t, *J* = 7.3 Hz, 2H, CH₂), 3.27 - 3.32 (m, 1H, H_{iPr}), 3.51 - 3.66 (m, 3H, CH₂ & H_{iPent}), 5.61 (s, 1H, H_{Ar}), 6.34 (d, *J* = 8.0 Hz, 1H, NH_{-CH}), 7.17 - 7.28 (m, 2H, NH_{Bn} & H_{Ar}), 7.34 (dt, *J* = 7.7, 1.1 Hz, 1H, H_{Ar}), 7.72 (td, *J* = 7.6, 1.9 Hz, 1H, H_{Ar}), 8.45 - 8.56 (m, 1H, H_{Ar}). |
| | | | **¹³C NMR (101 MHz, DMSO-*d₆*) δ : 10.4** (2xCH_{3-CH2}), 19.5 (2xCH_{3 iPr}), 24.8 (CH_{iPr}), 26.3 (2xCH_{2-CH3}), 35.9 (CH₂), 41.7 (CH₂), 52.9 (CH_{iPent}), 83.7 (CH_{Ar}), 121.6 (CH_{Ar}), 123.3 (CH_{Ar}), 136.6 (CH_{Ar}), 139.7 (C_{q}), 139.8 (C_{q}), 149.1 (CH_{Ar}), 152.7 (C_{q}), 156.1 (C_{q}), 159.0 (C_{q}). |
| | | | **MS (ESI+) : m/z calcd for C₂₀H₂₉N₇** : 368.26 [M+H]+, **found** : 368.34. |
| (84) | 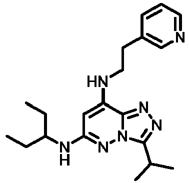 | > 96 % ^{a} | White solid. |
| | | | **¹H NMR (400 MHz, DMSO-*d₆*) δ :** 0.89 (t, *J* = 7.4 Hz, 6H, 2xCH_{3-CH2}), 1.36 (d, *J* = 6.9 Hz, 6H, 2xCH_{3 iPr}), 1.41 - 1.64 (m, 4H, 2xCH_{2-CH3}), 2.95 (t, *J* = 7.3 Hz, 2H, CH2-CH2-NH), 3.33 (s, 1H, H_{iPr}), 3.44 (q, *J* = 6.8 Hz, 2H, CH_{2-NH}), 3.57 - 3.68 (m, 1H, H_{iPent}), 5.62 (s, 1H, H_{Ar}), 6.31 (d, *J* = 8.0 Hz, 1H, NH_{-CH}), 7.30 (t, *J* = 5.9 Hz, 1H, NH_{-CH2-CH2}), 7.34 (dd, *J* = 7.7, 4.7 Hz, 1H, H_{Ar}), 7.72 (dt, *J* = 7.9, 2.0 Hz, 1H, H_{Ar}), 8.44 (dd, *J* = 4.8, 1.6 Hz, 1H, H_{Ar}), 8.53 (d, *J* = 2.2 Hz, 1H, H_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.3 (2xCH_{2-CH3}), 30.8 (CH_{2-CH2- NH}), 43.2 (CH_{2-NH}), 52.9 (CH_{iPent}), 83.8 (CH_{Ar}), 123.4 (CH_{Ar}), 134.7 (C_{q}), 136.2 (CH_{Ar}), 139.6 (C_{q}), 139.7 (C_{q}), 147.5 (CH_{Ar}), 149.9 (CH_{Ar}), 152.6 (C_{q}), 156.1 (C_{q}). |
| | | | **MS (ESI+) : m/z calcd for C₂₀H₂₉N₇ :** 368.26 [M+H]+, **found :** 368.34. |
| (85) | 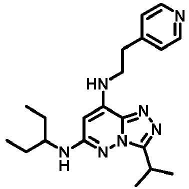 | > 97 % a | White solid. |
| | | | **¹H NMR (400 MHz, DMSO-*d₆*) δ :** 0.88 (t, *J* = 7.4 Hz, 6H, 2xCH_{3-CH2}), 1.35 (d, *J* = 6.9 Hz, 6H, 2×CH_{3 iPr}), 1.41 - 1.64 (m, 4H, 2xCH_{2-CH3}), 2.95 (t, *J=* 7.3 Hz, 2H, CH_{2-CH2-NH}), 3.23 - 3.37 (m, 1H, H_{iPr}), 3.45 (q, *J* = 6.8 Hz, 2H, CH_{2-NH}), 3.61 (h, *J* = 6.6 Hz, 1H, H_{iPent}), 5.62 (s, 1H, H_{Ar}), 6.30 (d, *J* = 8.0 Hz, 1H, NH_{iPent}), 7.28 (t, *J* = 5.8 Hz, 1H, NH_cH2), 7.30 - 7.34 (m, 2H, 2xH_{Ar}), 8.39 - 8.57 (m, 2H, 2xH_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 32.9 (CH_{2-CH2-NH}), 42.4 (CH_{2-NH}), 52.9 (CH_{iPent}), 83.8 (CH_{Ar}), 124.2 (2xCH_{Ar}), 139.6 (C_{q}), 139.7 (C_{q}), 148.2 (C_{q}), 149.5 (2xCH_{Ar}), 152.6 (C_{q}), 156.0 (C_{q}). |
| | | | **MS (ESI+) : m/z calcd for C₂₀H₂₉N₇ :** 368.26 [M+H]+, **found :** 368.34. |
| (86) | 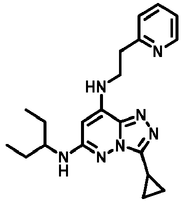 | > 97 % a | Yellow solid. |
| | | | **¹H NMR (400 MHz, DMSO-*d₆*) δ :** 0.87 (t, *J* = 7.4 Hz, 6H, 2xCH_{3-CH2}), 1.02 (dt, *J* = 8.6, 3.1 Hz, 2H, CH_{2 cPr}), 1.11 (dt, *J* = 5.6, 2.9 Hz, 2H, CH_{2 cPr}), 1.50 (dtq, *J* = 42.5, 14.4, 7.3, 6.5 Hz, 4H, 2xCH_{2-CH3}), 2.24 (tt, *J* = 8.5, 5.1 Hz, 1H, H_{cPr}), 3.08 (t, *J* = 7.3 Hz, 2H, CH₂), 3.50 - 3.66 (m, 3H, H_{iPent} & CH₂), 5.60 (s, 1H, H_{Ar}), 6.33 (d, *J* = 8.1 Hz, 1H, NH_{iPent}), 7.18 - 7.26 (m, 2H, NH & H_{Ar}), 7.32 (d, *J* = 7.8 Hz, 1H, H_{Ar}), 7.71 (td, *J* = 7.6, 1.9 Hz, 1H, H_{Ar}), 8.51 (dd, *J* = 4.9, 1.8 Hz, 1H, H_{Ar}). |
| | | | **¹³C NMR (101 MHz, DMSO-*d₆*) δ :** 5.4 (CH_{cPr}), 6.3 (2×CH_{2 cPr}), 10.3 (2xCH_{3-CH2}), 26.2 (2xCH_{2-CH3}), 35.9 (CH₂), 41.7 (CH₂), 52.8 (CH_{iPent}), 83.7 (CH_{Ar}), 121.6 (CH_{Ar}), 123.3 (CH_{Ar}), 136.5 (CH_{Ar}), 139.5 (C_{q}), 139.7 (C_{q}), 149.0 (CH_{Ar}), 149.8 (C_{q}), 156.3 (C_{q}), 158.9 (C_{q}). |
| | | | **MS (ESI+) : m/z calcd for C₂₀H₂₇N₇ :** 366.24 [M+H]+, **found :** 366.42. |
| (87) | 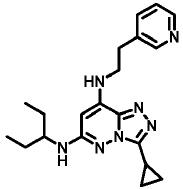 | > 98 % ^{a} | Beige solid. |
| | | | **¹H NMR (400 MHz, DMSO-*d₆*) δ :** 0.88 (t, *J* = 7.4 Hz, 6H, 2xCH_{3-CH2}), 0.98 - 1.16 (m, 4H, 2×CH_{2 cPr}), 1.40 - 1.63 (m, 4H, 2xCH_{2-CH3}), 2.24 (tt, *J* = 8.5, 5.2 Hz, 1H, H_{cPr}), 2.94 (t, *J* = 7.4 Hz, 2H, CH₂), 3.38 - 3.48 (m, 2H, CH₂), 3.56 - 3.68 (m, 1H, H_{iPent}), 5.60 (s, 1H, H_{Ar}), 6.30 (d, *J* = 8.1 Hz, 1H, NH_{iPent}), 7.28 (t, *J* = 5.8 Hz, 1H, NH), 7.32 (dd, *J* = 7.8, 4.7 Hz, 1H, H_{Ar}), 7.70 (dt, *J* = 7.9, 2.0 Hz, 1H, H_{Ar}), 8.42 (d, *J* = 4.0 Hz, 1H, H_{Ar}), 8.47 - 8.56 (m, 1H, H_{Ar}). |
| | | | **¹³C NMR (101 MHz, DMSO-*d₆*) δ :** 5.4 (CH_{cPr}), 6.4 (CH_{2 cPr}), 10.3 (2xCH_{3-CH2}), 26.2 (2xCH_{2-CH3}), 30.8 (CH₂), 43.2 (CH₂), 52.8 (CH_{iPent}), 83.8 (CH_{Ar}), 123.4 (CH_{Ar}), 134.7 (C_{q}), 136.3 (CH_{Ar}), 139.5 (C_{q}), 139.6 (C_{q}), 147.5 (CH_{Ar}), 149.8 (C_{q}), 149.9 (CH_{Ar}), 156.3 (C_{q}). |
| | | | **MS (ESI+) : m/z calcd for C₂₀H₂₇N₇ :** 366.24 [M+H]+, **found :** 366.39. |
| (88) | 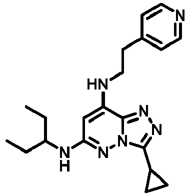 | > 97 % ^{a} | White solid. |
| | | | **¹H NMR (400 MHz, DMSO-*d₆*) δ :** 0.88 (t, *J* = 7.4 Hz, 6H, 2xCH_{3-CH2}), 0.99 - 1.15 (m, 4H, 2×CH_{2 cPr}), 1.40 - 1.63 (m, 4H, 2xCH_{2-CH3}), 2.24 (tt, *J* = 8.5, 5.1 Hz, 1H, H_{cPr}), 2.95 (t, *J* = 7.3 Hz, 2H, CH₂), 3.45 (q, *J* = 6.8 Hz, 2H, CH₂), 3.61 (q, *J* = 6.2 Hz, 1H, H_{iPent}), 5.60 (s, 1H, H_{Ar}), 6.30 (d, *J* = 8.1 Hz, 1H, NH_{iPent}), 7.25 (t, *J* = 5.8 Hz, 1H, NH), 7.28 - 7.36 (m, 2H, 2xH_{Ar}), 8.42 - 8.54 (m, 2H, 2xH_{Ar}). |
| | | | **¹³C NMR (101 MHz, DMSO-*d₆*) δ :** 5.4 (CH_{cPr}), 6.4 (2×CH_{2 cPr}), 10.3 (2xCH_{3-CH2}), 26.2 (2xCH_{2-CH3}), 32.9 (CH₂), 42.4 (CH₂), 52.8 (CH_{Ar}), 83.8 (C_{q}), 124.2 (2xCH_{Ar}), 139.5 (C_{q}), 139.6 (C_{q}), 148.2 (C_{q}), 149.5 (2xCH_{Ar}), 149.8 (C_{q}), 156.3 (C_{q}). |
| | | | **MS (ESI+) : m/z calcd for C₂₀H₂₇N₇ :** 366.24 [M+H]+, **found :** 366.39. |
| (89) | 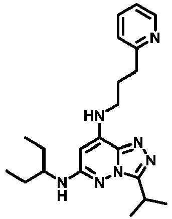 | > 95 % ^{a} | Yellow solid. |
| | | | **¹H NMR (400 MHz, DMSO-*d₆*) δ :** 0.87 (t, *J* = 7.4 Hz, 6H, 2xCH_{3-CH2}), 1.35 (d, *J* = 7.0 Hz, 6H, 2×CH_{3 iPr}), 1.40 - 1.62 (m, 4H, 2xCH_{2-CH3}), 2.03 (p, *J* = 7.3 Hz, 2H, CH₂), 2.82 (t, *J* = 7.7 Hz, 2H, CH₂), 3.23 (q, *J* = 6.6 Hz, 2H, CH₂), 3.30 (q, *J =* 6.9 Hz, 1H, H_{iPr}), 3.61 (qd, *J* = 7.5, 3.7 Hz, 1H, H_{iPent}), 5.52 (s, 1H, H_{Ar}), 6.28 (d, *J =* 8.0 Hz, 1H, NH), 7.19 (ddd, *J* = 7.5, 4.9, 1.2 Hz, 1H, H_{Ar}), 7.28 (d, *J* = 7.8 Hz, 1H, H_{Ar}), 7.33 (t, *J* = 5.7 Hz, 1H, NH), 7.68 (td, *J* = 7.7, 1.9 Hz, 1H, H_{Ar}), 8.48 (dd, *J=* 5.1, 1.7 Hz, 1H, H_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 27.5 (CH₂), 35.0 (CH₂), 41.8 (CH₂), 52.9 (CH_{iPent}), 83.4 (CH_{Ar}), 121.2 (CH_{Ar}), 122.7 (CH_{Ar}), 136.4 (CH_{Ar}), 139.7 (C_{q}), 140.0 (C_{q}), 148.9 (CH_{Ar}), 152.6 (C_{q}), 156.1 (C_{q}), 161.0 (C_{q}). |
| | | | **MS (ESI+) : m/z calcd for C₂₁H₃₁N₇ :** 382.27 [M+H]+, **found** : 382.32. |
| (90) | 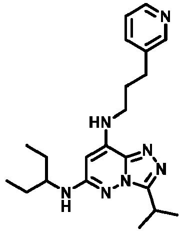 | > 95 % ^{a} | Yellow solid. |
| | | | **¹H NMR (400 MHz, DMSO-*d₆*) δ :** 0.80 - 0.98 (m, 6H, 2xCH_{3-CH2}), 1.36 (dd, *J* = 7.0, 1.3 Hz, 6H, 2×CH_{3 iPr}), 1.51 (ddt, *J* = 33.1, 13.9, 6.9 Hz, 4H, 2xCH_{2-CH3}), 1.95 (p, *J* = 7.3 Hz, 2H, CH₂), 2.70 (t, *J* = 7.9 Hz, 2H, CH₂), 3.19 (q, *J* = 6.7 Hz, 2H, CH₂), 3.29 (s, 1H, H_{iPr}), 3.60 (td, *J* = 12.9, 6.3 Hz, 1H, H_{iPent}), 5.51 (s, 1H, H_{Ar}), 6.28 (d, *J* = 8.0 Hz, 1H, NH_{iPent}), 7.27 (t, *J* = 5.7 Hz, 1H, NH), 7.31 (dd, *J* = 7.8, 4.8 Hz, 1H, H_{Ar}), 7.56 - 7.78 (m, 1H, H_{Ar}), 8.40 (d, *J* = 4.7 Hz, 1H, H_{Ar}), 8.47 (s, 1H, H_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 29.0 (CH₂), 29.7 (CH₂), 41.6 (CH₂), 52.9 (CH_{iPent}), 83.5 (CH_{Ar}), 123.4 (CH_{Ar}), 135.8 (CH_{Ar}), 137.0 (C_{q}), 139.7 (C_{q}), 140.0 (C_{q}), 147.2 (CH_{Ar}), 149.6 (CH_{Ar}), 152.6 (C_{q}), 156.1 (C_{q}). |
| | | | **MS (ESI+) : m/z calcd for C₂₁H₃₁N₇ :** 382.27 [M+H]+, **found :** 382.32. |
| (91) | 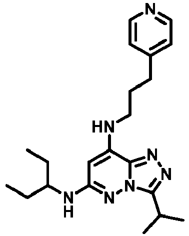 | > 97 % ^{a} | White solid. |
| | | | **¹H NMR (400 MHz, DMSO-*d₆*) δ :** 0.87 (t, *J* = 7.4 Hz, 6H, 2xCH_{3-CH2}), 1.36 (d, *J* = 7.0 Hz, 6H, 2×CH_{3 iPr}), 1.54 (tdd, *J* = 12.3, 7.3, 5.4 Hz, 4H, 2xCH_{2-CH3}), 1.87 - 2.06 (m, 2H, CH_{2-CH2-NH}), 2.65 - 2.77 (m, 2H, CH_{2-CH2-CH2-NH}), 3.19 (q, *J* = 6.5 Hz, 2H, CH_{2-NH}), 3.26 - 3.32 (m, 1H, H_{iPr}), 3.60 (hept, *J* = 5.9, 5.4 Hz, 1H, H_{iPent}), 5.51 (s, 1H, H_{Ar}), 6.28 (d, *J* = 8.0 Hz, 1H, NH_{iPent}), 7.22 - 7.35 (m, 3H, NH_{Bn} & 2xH_{Ar}), 8.45 (d, *J* = 5.0 Hz, 2H, 2xH_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 28.2 (CH_{2-CH2-}CH_{2-NH}), 31.8 (CH_{2-CH2-NH}), 41.5 (CH_{2-NH}), 52.9 (CH_{iPent}), 83.5 (CH_{Ar}), 123.9 (CH_{Ar}), 139.7 (C_{q}), 140.0 (C_{q}), 149.5 (CH_{Ar}), 150.5 (C_{q}), 152.6 (C_{q}), 156.1 (C_{q}). |
| | | | **MS (ESI+) : m/z calcd for C₂₁H₃₁N₇ :** 382.27 [M+H]+, **found :** 382.35. |
| (92) | 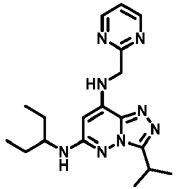 | > 95 % ^{a} | White solid. |
| | | | **¹H NMR (400 MHz, DMSO-*d₆*) δ :** 0.83 (t, *J* = 7.4 Hz, 6H, 2xCH_{3-CH2}), 1.36 (d, *J* = 7.0 Hz, 6H, 2×CH_{3 iPr}), 1.39 - 1.57 (m, 4H, 2xCH_{2-CH3}), 3.26 - 3.33 (m, 1H, H_{iPr}), 3.50 - 3.64 (m, 1H, H_{iPent}), 4.64 (d, *J* = 5.9 Hz, 2H, CH_{2Bn}), 5.46 (s, 1H, H_{Ar}), 6.33 (d, *J* = 8.0 Hz, 1H, NH_{iPent}), 7.44 (t, *J* = 4.9 Hz, 1H, H_{Ar}), 7.49 (t, *J* = 6.0 Hz, 1H, NH_{Bn}), 8.81 (d, *J* = 4.9 Hz, 2H, 2xH_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.2 (2xCH_{2-CH3}), 47.6 (CH_{2Bn}), 53.0 (CH_{iPent}), 84.7 (CH_{Ar}), 120.1 (CH_{Ar}), 139.7 (C_{q}), 139.9 (C_{q}), 152.7 (C_{q}), 156.0 (C_{q}), 157.5 (2xCH_{Ar}), 166.0 (C_{q}). |
| | | | **MS (ESI+) : m/z calcd for C₁₈H₂₆N₈** : 355.24 [M+H]+, **found :** 355.34. |
| (93) | 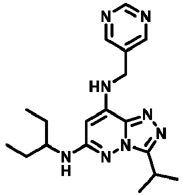 | > 96 % ^{a} | Pale yellow solid. |
| | | | **¹H NMR (400 MHz, DMSO-*d₆*) δ :** 0.84 (t, *J* = 7.4 Hz, 6H, 2xCH₃), 1.35 (d, *J* = 7.0 Hz, 6H, 2xCH₃), 1.38 - 1.57 (m, 4H, 2xCH₂), 3.27 (d, *J* = 6.9 Hz, 1H, H_{iPr}), 3.55 (p, *J* = 6.6 Hz, 1H, H_{iPent}), 4.48 (d, *J* = 6.2 Hz, 2H, CH₂), 5.51 (s, 1H, H_{Ar}), 6.31 (d, *J* = 7.9 Hz, 1H, NH), 7.90 (t, *J* = 6.2 Hz, 1H, NH), 8.82 (s, 2H, 2xH_{Ar}), 9.10 (s, 1H, H_{Ar}). |
| | | | **¹³C NMR (101 MHz, DMSO-*d₆*) δ :** N.D. **MS (ESI+) : m/z calcd for C₁₈H₂₆N₈ :** 355.24 [M+H]+, **found :** 355.34. |
| (94) | 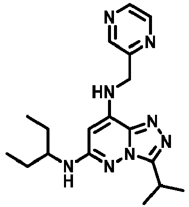 | > 96 % ^{a} | White solid. |
| | | | **¹H NMR (400 MHz, DMSO-*d₆*) δ :** 0.83 (t, *J* = 7.3 Hz, 6H, 2xCH_{3iPr}), 1.36 (d, *J* = 6.9 Hz, 6H, 2×CH_{3 iPr}), 1.47 (ddt, *J* = 32.1, 14.0, 6.8 Hz, 4H, 2xCH_{2-CH3}), 3.26 - 3.35 (m, 1H, H_{iPr}), 3.56 (q, *J* = 6.6 Hz, 1H, H_{iPent}), 4.60 (d, *J* = 6.1 Hz, 2H, CH_{2Bn}), 5.47 (s, 1H, H_{Ar}), 6.35 (d, *J* = 7.9 Hz, 1H, NH_{iPent}), 7.82 (t, *J* = 6.2 Hz, 1H, NH_{Bn}), 8.56 (d, *J* = 2.5 Hz, 1H, H_{Ar}), 8.63 (d, *J* = 6.4 Hz, 2H, 2xH_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.1 (2xCH_{2-CH3}), 45.4 (CH_{2Bn}), 53.0 (CH_{iPent}), 84.7 (CH_{Ar}), 139.6 (C_{q}), 139.7 (C_{q}), 143.3 (CH_{Ar}), 143.4 (CH_{Ar}), 144.1 (CH_{Ar}), 152.7 (C_{q}), 153.4 (C_{q}), 155.8 (C_{q}). |
| | | | **MS (ESI+) : m/z calcd for C₁₈H₂₆N₈ :** 355.24 [M+H]+, **found :** 355.31. |
| (95) | 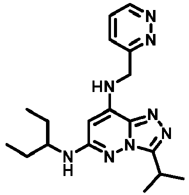 | > 96 % ^{a} | White solid. |
| | | | **¹H NMR (400 MHz, DMSO-*d₆*) δ :** 0.82 (t, *J* = 7.4 Hz, 6H, 2xCH_{3-CH2}), 1.35 (d, *J* = 7.0 Hz, 6H; 2×CH_{3 iPr}), 1.46 (ddt, *J* = 31.0, 14.0, 7.3 Hz, 4H, 2xCH_{2-CH3}), 3.25 - 3.33 (m, 1H, H_{iPr}), 3.54 (q, *J* = 6.7 Hz, 1H, H_{iPent}), 4.73 (d, *J* = 6.3 Hz, 2H, CH_{2Bn}), 5.47 (s, 1H, H_{Ar}), 6.40 (d, *J* = 7.9 Hz, 1H, NH_{iPent}), 7.58 - 7.75 (m, 2H, 2xH_{Ar}), 7.94 (t, *J* = 6.4 Hz, 1H, NH_{Bn}), 9.16 (dd, *J* = 4.7, 1.9 Hz, 1H, H_{Ar}). |
| | | | **¹³C NMR (101 MHz, DMSO-*d₆*) δ :** 10.4 (2xCH_{3-CH2}), 19.5 (2×CH_{3 iPr}), 24.8 (CH_{iPr}), 26.1 (2xCH_{2-CH3}), 45.7 (CH_{2Bn}), 53.0 (CH_{iPent}), 84.8 (CH_{Ar}), 125.0 (CH_{Ar}), 127.5 (CH_{Ar}), 139.6 (C_{q}), 139.6 (C_{q}), 150.8 (CH_{Ar}), 152.7 (C_{q}), 155.8 (C_{q}), 160.3 (C_{q}). |
| | | | **MS (ESI+) : m/z calcd for C₁₈H₂₆N₈ :** 355.24 [M+H]+, **found :** 355.31. |
| (96) | 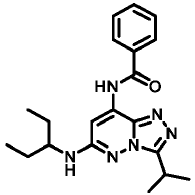 | > 96 % ^{a} | White solid. |
| | | | **¹H NMR (400 MHz, DMSO-*d₆*) δ :** 0.90 (t, *J* = 7.4 Hz, 6H, 2xCH_{3-CH2}), 1.40 (d, *J* = 6.9 Hz, 6H, 2×CH_{3 iPr}), 1.46 - 1.68 (m, 4H, 2xCH_{2-CH3}), 3.38 (p, *J* = 7.0 Hz, 1H, H_{iPr}), 3.67 (h, *J=* 6.7 Hz, 1H, H_{iPent}), 7.12 (d, *J* = 7.9 Hz, 1H, NH_{Alk}), 7.57 (dd, *J* = 8.3, 6.9 Hz, 2H, 2xH_{Ar}), 7.65 (d, *J* = 7.9 Hz, 2H, 2xH_{Ar}), 7.99 (dd, *J* = 7.2, 1.8 Hz, 2H, 2xH_{Ar}), 10.46 (s, 1H, NH_{-CO}). |
| | | | **¹³C NMR (101 MHz, DMSO-*d₆*) δ :** 10.4 (2xCH_{3-CH2}), 19.4 (2×CH_{3 iPr}), 24.8 (CH _{iPr}), 25.9 (2xCH_{2-CH3}), 53.4 (CH_{iPent}), 100.9 (CH_{Ar}), 128.1 (2xCH_{Ar}), 128.5 (2xCH_{Ar}), 131.9 (C_{q}), 132.5 (CH_{Ar}), 133.4 (C_{q}), 138.9 (C_{q}), 153.3 (C_{q}), 155.1 (C_{q}), 167.0 (C_{q}). |
| | | | **MS (ESI+) : m/z calcd for C₂₀H₂₆N₆O :** 367.22 [M+H]+, **found :** 367.30. |
| (97) | 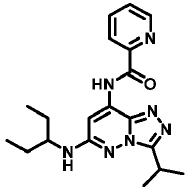 | > 96 % ^{a} | White solid. |
| | | | **¹H NMR (400 MHz, DMSO-*d₆*) δ :** 0.90 (t, *J* = 7.3 Hz, 6H, 2×CH_{3 iPent}), 1.40 (d, *J* = 6.9 Hz, 6H, 2×CH_{3 iPr}), 1.46 - 1.66 (m, 4H, 2×CH_{3 iPent}), 3.34 - 3.43 (m, 1H, H_{iPr}), 3.67 (h, *J=* 6.4 Hz, 1H, H_{iPent}), 7.20 (d, *J* = 7.9 Hz, 1H, NH_{iPent}), 7.73 (s, 1H, H_{Ar}), 7.80 (dd, *J* = 7.6, 4.8 Hz, 1H, H_{Ar}), 8.16 (t, *J* = 7.8 Hz, 1H, H_{Ar}), 8.25 (d, *J* = 7.8 Hz, 1H, H_{Ar}), 8.84 (d, *J=* 4.7 Hz, 1H, H_{Ar}), 10.85 (s, 1H, NH_co). |
| | | | **¹³C NMR (101 MHz, DMSO-*d₆*) δ :** 10.4 (2×CH_{3 iPent}), 19.4 (2×CH_{3 iPr}), 24.8 (CH_{iPr}), 25.9 (2×CH_{2 iPent}), 53.4 (CH_{iPent}), 98.8 (CH_{Ar}), 122.5 (CH_{Ar}), 128.2 (CH_{Ar}), 130.5 (C_{q}), 138.5 (C_{q}), 138.8 (CH_{Ar}), 147.6 (C_{q}), 149.0 (CH_{Ar}), 153.5 (C_{q}), 155.2 (C_{q}), 163.0 (C_{q}). |
| | | | **MS (ESI+) : m/z calcd for C₁₉H₂₅N₇O :** 368.22 [M+H]+, **found :** 368.31. |
| (98) | 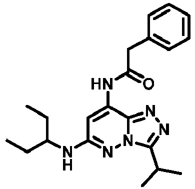 | > 97 % ^{a} | Pale yellow solid. |
| | | | **¹H NMR (400 MHz, DMSO-*d₆*) δ :** 0.86 (t, *J* = 7.4 Hz, 6H, 2xCH_{3-CH2}), 1.37 (d, *J* = 7.0 Hz, 6H, 2×CH_{3 iPr}), 1.52 (dtt, *J* = 38.9, 14.0, 6.5 Hz, 4H, 2xCH_{2-CH3}), 3.33 - 3.39 (m, 1H, H_{iPr}), 3.61 (hept, *J* = 5.9, 5.4 Hz, 1H, H_{iPent}), 3.92 (s, 2H, CH_{2-CO}), 6.98 (d, *J* = 7.9 Hz, 1H, NH_{iPent}), 7.21 - 7.29 (m, 1H, H_{Ar}), 7.29 - 7.41 (m, 4H, 4xH_{Ar}), 7.59 (s, 1H, H_{Ar}), 10.89 (s, 1H, NH_{-CO}). |
| | | | **¹³C NMR (101 MHz, DMSO-*d₆*) δ :** 10.3 (2xCH_{3-CH2}), 19.4 (2×CH_{3 iPr}), 24.8 (CH_{iPr}), 25.9 (2xCH_{2-CH3}), 42.7 (CH_{2-CO}), 53.3 (CH_{iPent}), 99.4 (CH_{Ar}), 126.7 (CH_{Ar}), 128.3 (2xCH_{Ar}), 129.1 (2xCH_{Ar}), 131.8 (C_{q}), 135.5 (C_{q}), 138.5 (C_{q}), 153.2 (C_{q}), 155.2 (C_{q}), 171.9 (C_{q}). |
| | | | **MS (ESI+) : m/z calcd for C₂₁H₂₈N₆O :** 381.24 [M+H]+, **found :** 381.41. |
| (99) | 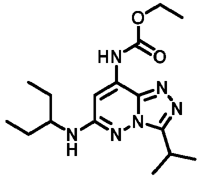 | > 96 % ^{a} | White solid. |
| | | | **¹H NMR (400 MHz, DMSO-*d₆*) δ :** 0.88 (t, *J* = 7.4 Hz, 6H, 2×CH_{3 iPent}), 1.26 (t, *J* = 7.1 Hz, 3H, CH_{3-CH2-O}), 1.37 (d, *J* = 6.9 Hz, 6H, 2×CH_{3 iPr}), 1.43 - 1.66 (m, 4H, 2×CH_{2 iPent}), 3.34 - 3.41 (m, 1H, H_{iPr}), 3.63 (h, *J* = 6.6, 6.1 Hz, 1H, H_{iPent}), 4.20 (q, *J* = 7.1 Hz, 2H, CH_{2-O}), 6.99 (d, *J* = 7.9 Hz, 1H, NH_{iPent}), 7.26 (s, 1H, H_{Ar}), 10.07 (s, 1H, NH_co). |
| | | | **¹³C NMR (101 MHz, DMSO-*d₆*) δ :** 10.3 (2×CH₃ iPent), 14.3 (CH_{3-CH2-O}), 19.4 (2×CH_{3 iPr}), 24.8 (CH_{iPr}), 25.9 (2xCH_{2 iPent}), 53.3 (CH_{iPent}), 61.3 (CH_{2-O}), 97.9 (CH_{Ar}), 132.5 (C_{q}), 138.7 (C_{q}), 153.1 (C_{q}), 153.5 (C_{q}), 155.2 (C_{q}). |
| | | | **MS (ESI+) : m/z calcd for C₁₆H₂₆N₆O₂ :** 335.22 [M+H]+, **found :** 335.32. |
| (100) | 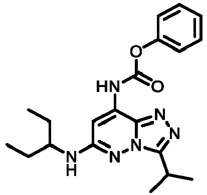 | Unstable ^{b} | White solid. |
| | | | **¹H NMR (400 MHz, DMSO-*d₆*) δ :** 0.88 (t, *J* = 7.4 Hz, 6H, 2×CH_{3 iPent}), 1.39 (d, *J* = 7.0 Hz, 6H, 2×CH_{3 iPr}), 1.42 - 1.65 (m, 4H, 2xCH_{2 iPent}), 3.37 (q, *J* = 7.0 Hz, 1H, H_{iPr}), 3.63 (hept, *J* = 5.9, 5.2 Hz, 1H, H_{iPent}), 7.03 (d, *J* = 7.9 Hz, 1H, NH_{iPent}), 7.22 - 7.34 (m, 4H, 4xH_{Ar}), 7.42 - 7.50 (m, 2H, 2xH_{Ar}), 10.87 (s, 1H, NH_{-CO}). |
| | | | **¹³C NMR (101 MHz, DMSO-*d₆*) δ :** 10.4 (2×CH_{3 iPent}), 19.4 (2×CH_{3 iPr}), 24.8 (CH_{iPr}), 25.9 (2×CH_{2 iPent}), 53.3 (CH_{iPent}), 98.9 (CH_{Ar}), 121.8 (2xCH_{Ar}), 125.9 (CH_{Ar}), 129.5 (2xCH_{Ar}), 132.4 (C_{q}), 138.7 (C_{q}), 150.3 (C_{q}), 151.9 (C_{q}), 153.2 (C_{q}), 155.1 (C_{q}). |
| | | | **MS (ESI+) : m/z calcd for C₂₀H₂₆N₆O₂ :** 383.22 [M+H]+, **found :** 383.32. |
| (101) | 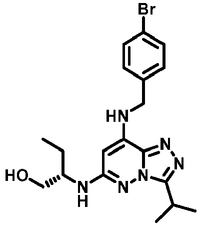 | > 98 % ^{a} | White crystalline solid. |
| | | | **¹H NMR (400 MHz, DMSO-*d₆*) δ :** ¹H NMR (400 MHz, DMSO-*d₆*) δ 0.85 (t, *J* = 7.4 Hz, 3H), 1.35 (d, *J* = 6.9 Hz, 6H), 1.37 - 1.69 (m, 2H), 3.30 (q, *J* = 6.8 Hz, 1H), 3.42 (dq, *J* = 18.4, 5.4 Hz, 2H), 3.57 - 3.69 (m, 1H), 4.39 (d, *J* = 6.3 Hz, 2H), 4.58 (t, *J* = 5.4 Hz, 1H), 5.43 (s, 1H), 6.29 (d, *J* = 7.7 Hz, 1H), 7.31 (d, *J* = 8.2 Hz, 2H), 7.53 (d, *J* = 8.3 Hz, 2H), 7.91 (t, *J* = 6.4 Hz, 1H). |
| | | | **¹³C NMR (101 MHz, DMSO-*d₆*) δ :** 10.5 (CH₃), 19.6 (2xCH₃), 23.6 (CH₂), 24.7 (CH_{iPr}), 44.7 (CH₂), 53.8 (CH_{Alk}), 61.9 (CH2), 84.6 (CH_{Ar}), 119.9 (C_{q}), 129.0 (CH_{Ar}), 131.3 (CH_{Ar}), 137.9 (C_{q}), 139.6 (C_{q}), 139.6 (C_{q}), 152.7 (C_{q}), 155.8 (C_{q}). |
| | | | **MS (ESI+) : m/z calcd for C₁₉H₂₅BrN₆O** : 433.13 [M+H]+, **found :** 433.28. |
| (102) | 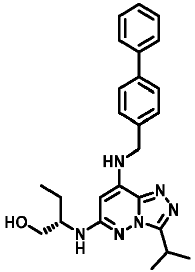 | > 96 % ^{a} | White solid. |
| | | | **¹H NMR (400 MHz, DMSO-*d₆*) δ :** 0.85 (t, *J* = 7.4 Hz, 3H, CH_{3-CH2}), 1.33 - 1.39 (m, 6H, 2×CH_{3 iPr}), 1.39 - 1.68 (m, 2H, CH_{2-CH3}), 3.28 - 3.32 (m, 1H, H_{iPr}), 3.37 - 3.48 (m, 2H, CH_{2-OH}), 3.62 (dt, *J* = 12.6, 6.5 Hz, 1H, H_{C-NH}), 4.41 - 4.55 (m, 2H, CH_{2Bn}), 4.58 (t, *J* = 5.4 Hz, 1H, OH), 5.51 (s, 1H, H_{Ar}), 6.31 (d, *J* = 7.7 Hz, 1H, NH_{Bn}), 7.31 - 7.38 (m, 1H, H_{Ar}), 7.40 - 7.50 (m, 4H, 4xH_{Ar}), 7.57 - 7.69 (m, 4H, 4xH_{Ar}), 7.92 (t, *J* = 6.4 Hz, 1H, NH_{Bn}). |
| | | | **¹³C NMR (101 MHz, DMSO-*d₆*) δ : 10.6** (CH_{3-CH2}), 19.6 (2×CH_{3 iPr}), 23.6 (CH_{2- CH3}), 24.8 (CH_{iPr}), 44.9 (CH_{2Bn}), 53.8 (CH_{- NH}), 61.9 (CH_{2-OH}), 84.5 (CH_{Ar}), 126.6 (2xCH_{Ar}), 126.8 (2xCH_{Ar}), 127.3 (CH_{Ar}), 127.4 (2xCH_{Ar}), 128.9 (2xCH_{Ar}), 137.7 (C_{q}), 138.9 (C_{q}), 139.7 (C_{q}), 139.8 (C_{q}), 139.9 (C_{q}), 152.8 (C_{q}), 155.9 (C_{q}). |
| | | | **MS (ESI+) : m/z calcd for C₂₅H₃₀N₆O :** 431.26 [M+H]+, **found :** 431.43. |
| (103) | 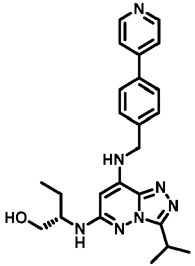 | > 96 % ^{a} | Yellow solid. |
| | | | **¹H NMR (400 MHz, DMSO-*d₆*) δ :** 0.84 (t, *J* = 7.4 Hz, 3H, CH_{3-CH2}), 1.36 (d, *J* = 6.8 Hz, 6H, 2×CH_{3 iPr}), 1.39 - 1.68 (m, 2H, CH_{2-CH3}), 3.27 - 3.31 (m, 1H, H_{iPr}), 3.41 (dq, *J* = 18.5, 5.4 Hz, 2H, CH_{2-OH}), 3.62 (tt, *J* = 7.2, 3.5 Hz, 1H, H_{C-NH}), 4.42 - 4.55 (m, 2H, CH_{2Bn}), 4.57 (t, *J* = 5.4 Hz, 1H, OH), 5.49 (s, 1H, H_{Ar}), 6.30 (d, *J* = 7.8 Hz, 1H, NH_{-CH}), 7.50 (d, *J* = 8.2 Hz, 2H, 2xH_{Ar}), 7.64 - 7.73 (m, 2H, 2xH_{Ar}), 7.79 (d, *J* = 8.3 Hz, 2H, 2xH_{Ar}), 7.95 (t, *J* = 6.4 Hz, 1H, NH_{Bn}), 8.54 - 8.69 (m, 2H, 2xH_{Ar}). |
| | | | **¹³C NMR (101 MHz, DMSO-*d₆*) δ :** 10.5 (CH_{3-CH2}), 19.6 (2xCH_{iPr}), 23.6 (CH_{2-CH3}), 24.7 (CH_{iPr}), 44.9 (CH_{2 Bn}), 53.8 (CH_{-NH}), 61.9 (CH_{2-OH}), 84.5 (CH_{Ar}), 121.1 (2xCH_{Ar}), 126.9 (2xCH_{Ar}), 127.6 (2xCH_{Ar}), 135.8 (C_{q}), 139.7 (C_{q}), 139.8 (C_{q}), 139.8 (C_{q}), 146.7 (C_{q}), 150.2 (2xCH_{Ar}), 152.7 (C_{q}), 155.8 (C_{q}). |
| | | | **MS (ESI+) : m/z calcd for C₂₄H₂₉N₇O :** 432.25 [M+H]+, **found :** 432.40. |
| (104) | 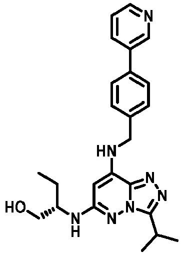 | > 95 % ^{a} | Yellow solid. |
| | | | **¹H NMR (400 MHz, DMSO-*d₆*) δ :** 0.85 (t, *J* = 7.4 Hz, 3H, CH_{3-CH2}), 1.36 (d, *J* = 6.9 Hz, 6H, 2×CH_{3 iPr}), 1.39 - 1.68 (m, 2H, CH_{2-CH3}), 3.30 (s, 1H, H_{iPr}), 3.37 - 3.47 (m, 2H, CH_{2-OH}), 3.63 (h, *J* = 5.9 Hz, ¹H, H_{C-NH}), 4.49 (d, *J* = 6.5 Hz, 2H, CH_{2 Bn}), 4.57 (d, *J* = 5.5 Hz, 1H, OH), 5.51 (s, 1H, H_{Ar}), 6.31 (d, *J* = 7.7 Hz, 1H, NH_{-CH}), 7.44 - 7.51 (m, 3H, 3xH_{Ar}), 7.71 (d, *J* = 8.2 Hz, 2H, 2xH_{Ar}), 7.94 (t, *J* = 6.4 Hz, 1H, NH_{Bn}), 8.05 (dt, *J* = 8.1, 2.0 Hz, 1H), 8.55 (dd, *J* = 4.8, 1.6 Hz, 1H), 8.88 (d, *J* = 2.4 Hz, 1H, H_{Ar}). |
| | | | **¹³C NMR (101 MHz, DMSO-*d₆*) δ :** 10.5 (CH_{3-CH2}), 19.6 (2×CH_{3 iPr}), 23.6 (CH_{2- CH3}), 24.7 (CH_{iPr}), 44.9 (CH_{2Bn}), 53.8 (CH_{- NH}), 61.9 (CH_{2-OH}), 84.5 (CH_{Ar}), 123.9 (CH_{Ar}), 127.0 (2xCH_{Ar}), 127.6 (2xCH_{Ar}), 134.0 (CH_{Ar}), 135.3 (C_{q}), 135.7 (C_{q}), 138.5 (C_{q}), 139.7 (C_{q}), 139.8 (C_{q}), 147.5 (CH_{Ar}), 148.4 (CH_{Ar}), 152.7 (C_{q}), 155.8 (C_{q}). |
| | | | M**S (ESI+) : m/z calcd for C₂₄H₂₉N₇O :** 432.25 [M+H]+, **found :** 432.40. |
| (105) | 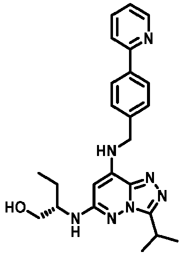 | > 97 % ^{a} | White solid. |
| | | | **¹H NMR (400 MHz, DMSO-*d₆*) δ :** 0.84 (t, *J* = 7.4 Hz, 3H, CH_{3-CH2}), 1.36 (d, *J* = 6.9 Hz, 6H, 2×CH_{3 iPr}), 1.39 - 1.67 (m, 2H, CH_{2-CH3}), 3.32 (s, 1H, H_{iPr}), 3.42 (ddd, *J* = 19.6, 10.7, 5.2 Hz, 2H, CH_{2-OH}), 3.56 - 3.70 (m, 1H, H_{C-NH}), 4.49 (d, *J* = 6.4 Hz, 2H, NH_{Bn}), 4.56 (t, *J* = 5.4 Hz, 1H, OH), 5.49 (s, 1H, H_{Ar}), 6.30 (d, *J =* 7.7 Hz, 1H, NH_{-CH}), 7.33 (dd, *J* = 7.4, 4.8 Hz, 1H, H_{Ar}), 7.47 (d, *J* = 8.0 Hz, 2H, 2xH_{Ar}), 7.86 (td, *J* = 7.7, 1.8 Hz, 1H, H_{Ar}), 7.90 - 7.96 (m, 2H, NH_{Bn} & H_{Ar}), 8.05 (d, *J* = 8.0 Hz, 2H, 2xH_{Ar}), 8.65 (d, *J* = 4.4 Hz, 1H, H_{Ar}). |
| | | | **¹³C NMR (101 MHz, DMSO-*d₆*) δ :** 10.5 (CH_{3-CH2}), 19.6 (2×CH_{3 iPr}), 23.6 (CH_{2- CH3}), 24.7 (CH_{iPr}), 45.0 (CH_{2Bn}), 53.8 (CH_{- NH}), 61.9 (CH_{2-OH}), 84.5 (CH_{Ar}), 120.1 (CH_{Ar}), 122.5 (CH_{Ar}), 126.6 (CH_{Ar}), 127.2 (CH_{Ar}), 137.2 (CH_{Ar}), 137.5 (C_{q}), 139.4 (C_{q}), 139.7 (C_{q}), 139.8 (C_{q}), 148.3 (C_{q}), 149.5 (CH_{Ar}), 152.7 (C_{q}), 155.8 (C_{q}). |
| | | | **MS (ESI+) : m/z calcd for C₂₄H₂₉N₇O :** 432.25 [M+H]+, **found :** 432.40. |
| (106) | 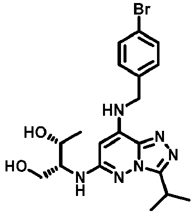 | > 96 % ^{a} | White solid. |
| | | | **¹H NMR (400 MHz, DMSO-*d₆*) δ :** 1.03 (d, *J* = 6.4 Hz, 3H, _{CH3}), 1.35 (dd, *J* = 7.0, 4.4 Hz, 6H, 2×CH_{3 iPr}), 3.23 - 3.36 (m, 1H, H_{iPr}), 3.37 - 3.57 (m, 2H, CH₂), 3.72 (dtd, *J* = 8.5, 5.9, 2.8 Hz, 1H, H_{Alk}), 3.97 (ddd, *J* = 6.5, 4.9, 3.1 Hz, 1H, H_{Alk}), 4.38 (d, *J* = 6.3 Hz, 2H, CH_{2Bn}), 4.54 (dd, *J* = 5.0, 3.5 Hz, 2H, 2xOH), 5.61 (s, 1H, H_{Ar}), 6.11 (d, *J* = 8.4 Hz, 1H, NH), 7.29 - 7.36 (m, 2H, 2xH_{Ar}), 7.46 - 7.58 (m, 2H, 2xH_{Ar}), 7.89 (t, *J* = 6.4 Hz, 1H, NH_{Bn}). |
| | | | **¹³C NMR (101 MHz, DMSO-*d₆*) δ :** 19.6 (CH_{3 iPr}), 19.7 (CH_{3 iPr}), 20.3 (CH₃), 24.6 (CH_{iPr}), 44.6 (CH_{2 Bn}), 56.9 (CH_{Alk}), 60.1 (CH₂), 64.5 (CH_{Alk}), 84.7 (CH_{Ar}), 119.9 (C_{q}), 129.2 (2xCH_{Ar}), 131.2 (2xCH_{Ar}), 138.0 (C_{q}), 139.6 (C_{q}), 139.6 (C_{q}), 152.7 (C_{q}), 156.2 (C_{q}). |
| | | | **MS (ESI+) : m/z calcd for C₁₉H₂₅N₆O₂ :** 449.13 [M+H]+, **found :** 449.19. |
| (107) | 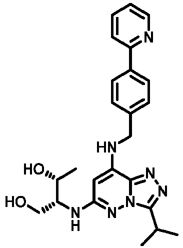 | > 98 % ^{a} | White solid. |
| | | | **¹H NMR (400 MHz, DMSO-*d₆*) δ :** 1.03 (d, *J* = 6.3 Hz, 3H, CH_{3-CH}), 1.35 (dd, *J* = 6.9, 4.4 Hz, 6H, 2xCH_{3 iPr}), 3.27 - 3.37 (m, 1H, H_{iPr}), 3.46 (ddt, *J* = 35.1, 10.6, 5.6 Hz, 2H, CH_{2-OH}), 3.73 (q, *J* = 7.9 Hz, 1H, CH_{-NH}), 3.97 (d, *J* = 7.4 Hz, 1H, CH_{-OH}), 4.44 - 4.58 (m, 4H, CH_{2 Bn} & 2xOH), 5.68 (s, 1H, H_{Ar}), 6.13 (d, *J* = 8.3 Hz, 1H, NH_{-CH}), 7.33 (dd, *J* = 7.3, 4.8 Hz, 1H, H_{Ar}), 7.49 (d, *J* = 8.0 Hz, 2H, 2xH_{Ar}), 7.86 (td, *J* = 7.7, 1.8 Hz, 1H, H_{Ar}), 7.89 - 7.96 (m, 2H, H_{Ar} & NH_{Bn}), 8.05 (d, *J* = 8.0 Hz, 2H, 2xH_{Ar}), 8.64 (d, *J* = 4.7 Hz, 1H, H_{Ar}). |
| | | | **¹³C NMR (101 MHz, DMSO-*d₆*) δ :** 19.6 (CH_{3 iPr}), 19.7 (CH_{3 iPr}), 20.3 (CH_{3-CH}), 24.6 (CH_{iPr}), 45.0 (CH_{2Bn}), 56.9 (CH_{-NH}), 60.1 (CH_{-OH}), 64.6 (CH_{-OH}), 84.6 (CH_{Ar}), 120.1 (CH_{Ar}), 122.5 (CH_{Ar}), 126.6 (2xCH_{Ar}), 127.3 (2xCH_{Ar}), 137.2 (CH_{Ar}), 137.4 (C_{q}), 139.5 (C_{q}), 139.7 (C_{q}), 139.7 (C_{q}), 149.5 (CH_{Ar}), 152.7 (C_{q}), 155.8 (C_{q}), 156.3 (C_{q}). |
| | | | **MS (ESI+) : m/z calcd for C₂₄H₂₉N₇O₂ :** 448.25 [M+H]+, f**ound :** 448.36. |
| (108) | 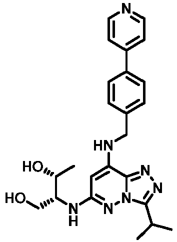 | > 97 % ^{a} | White solid. |
| | | | **¹H NMR (400 MHz, DMSO-*d₆)* δ :** 1.03 (d, *J* = 6.3 Hz, 3H, CH₃), 1.35 (dd, *J* = 6.9, 4.3 Hz, 6H, 2xCH_{3 iPr}), 3.32 (m, 1H, H_{iPr}), 3.47 (ddt, *J* = 35.1, 10.5, 5.7 Hz, 2H, CH_{2-OH}), 3.68 - 3.78 (m, 1H, H_{Alk}), 3.97 (dq, *J* = 9.2, 6.1 Hz, 1H, H_{Alk}), 4.48 (d, *J* = 6.4 Hz, 2H, CH_{2 Bn}), 4.51 - 4.57 (m, 2xOH), 5.67 (s, 1H, H_{Ar}), 6.13 (d, *J* = 8.3 Hz, 1H, NH), 7.52 (d, *J* = 8.0 Hz, 2H, 2×H_{Ar}), 7.65 - 7.73 (m, 2H, 2xH_{Ar}), 7.78 (d, *J* = 8.1 Hz, 2H, 2xH_{Ar}), 7.94 (t, *J* = 6.4 Hz, 1H, NH_{Bn}), 8.55 - 8.68 (m, 2H, 2xH_{Ar}). |
| | | | **¹³C NMR (101 MHz, DMSO-*d₆*) δ :** 19.6 (CH_{3 iPr}), 19.7 (CH_{3 iPr}), 20.3 (CH₃), 24.6 (CH_{iPr}), 44.9 (CH_{2Bn}), 56.9 (CH), 60.1 (CH₂), 64.5 (CH), 84.7 (CH_{Ar}), 121.1 (2xCH_{Ar}), 126.9 (2×CH_{Ar}), 127.8 (2xCH_{Ar}), 135.8 (C_{q}), 139.7 (C_{q}), 139.7 (C_{q}), 139.9 (C_{q}), 146.7 (C_{q}), 150.2 (2xCH_{Ar}), 152.7 (C_{q}), 156.3 (C_{q}). |
| | | | **MS (ESI+) : m/z calcd for C₂₄H₂₉N₇O₂ :** 448.25 [M+H]+, found : 448.33. |
| (109) | 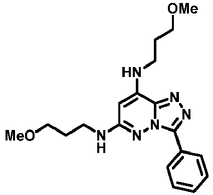 | > 98 % ^{a} | White solid. |
| | | | **¹H NMR (400 MHz, DMSO-*d₆)* δ** : 1.86 (dp, *J* = 13.4, 6.6 Hz, 4H, 2×CH_{2-CH2}), 3.24 - 3.32 (m, 10H, 2×OCH₃ & 2×CH_{2-NH}), 3.43 (td, *J* = 6.2, 2.9 Hz, 4H, 2×CH_{2-OCH3}), 5.58 (s, 1H, H_{Ar}), 6.77 (t, *J* = 5.5 Hz, 1H, NH_{Alk}), 7.37 (t, *J* = 5.7 Hz, 1H, NH_{Alk}), 7.44 - 7.49 (m, 1H, H_{Ar}), 7.53 (dd, *J* = 8.3, 6.6 Hz, 2H, 2xH_{Ar}), 8.49 (d, *J* = 7.1 Hz, 2H, 2×H_{Ar}). |
| | | | **¹³C NMR (101 MHz, DMSO-*d₆)* δ :** 27.9 (CH_{2-CH2}), 28.6 (CH_{2-CH2}), 38.1 (CH_{2-NH}), 39.4 (CH_{2-NH}), 58.0 (OCH₃), 58.0 (OCH₃), 69.7 (CH_{2-OCH3}), 69.8 (CH_{2-OCH3}), 83.4 (CH_{Ar}), 126.5 (2xCH_{Ar}), 127.5 (C_{q}), 128.5 (2xCH_{Ar}), 129.1 (CH_{Ar}), 140.1 (C_{q}), 140.6 (C_{q}), 146.3 (C_{q}), 156.7 (C_{q}). |
| | | | MS (ESI+) : m/z calcd for C₁₉H₂₆N₆O₂ : 371.22 [M+H]+, found : 371.36. |
| (110) | 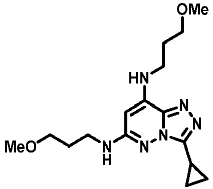 | > 97 % ^{a} | White solid. |
| | | | **¹H NMR (400 MHz, DMSO-*d₆)* δ :** 0.99-1.14 (m, 4H, 2xCH_{2cPr}), 1.81 (dp, *J* = 20.1, 6.5 Hz, 4H, 2×CH_{2-CH2}), 2.22 - 2.31 (m, 1H, H_{cPr}), 3.18 - 3.26 (m, 10H, 2×OCH₃ & 2×CH_{2-NH}), 3.40 (td, *J* = 6.2, 4.2 Hz, 4H, 2xCH₂₋oc_{H3}), 5.46 (s, 1H, H_{Ar}), 6.54 (t, *J* = 5.4 Hz, 1H, NH), 7.17 (t, *J* = 5.7 Hz, 1H, NH). |
| | | | **¹³C NMR (101 MHz, DMSO-*d₆)* δ :** 5.3 (CH_{cPr}), 6.6 (2×CH_{2 cPr}), 28.0 (CH_{2-CH2-NH}), 28.7 (CH_{2-CH2-NH}), 38.0 (CH_{2-NH}), 39.3 (CH_{2-NH}), 57.9 (OCH₃), 58.0 (OCH₃), 69.7 (CH_{2-OCH3}), 69.8 (CH_{2-OCH3}), 83.2 (CH_{Ar}), 139.6 (C_{q}), 140.0 (C_{q}), 150.0 (C_{q}), 156.4 (C_{q}). |
| | | | **MS (ESI+) : m/z calcd for** C₁₆H₂₆N₆O₂ : 335.43 [M+H]+, found : 335.35. |
| (111) | 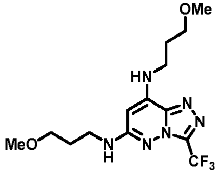 | > 98 % ^{a} | White solid. |
| | | | **¹H NMR (400 MHz, DMSO-*d₆)* δ :** 1.78 (p, *J* = 6.7 Hz, 2H, CH_{2-CH2-O}), 1.86 (p, *J* = 6.5 Hz, 2H, CH₂._{CH2O}), 3.17 - 3.29 (m, 10H, , 2×CH_{2-NH}& 2×CH_{3-NH}), 3.40 (dt, *J* = 6.3 Hz, 4H, 2×CH_{2-O}), 5.66 (s, 1H, H_{Ar}), 6.95 (t, *J* = 5.3 Hz, 1H, NH), 7.62 (t, *J* = 5.5 Hz, 1H, NH). |
| | | | **¹³C NMR (101 MHz, DMSO-*d₆)* δ :** N.D. |
| | | | **MS (ESI+) : m/z calcd for C₁₄H₂₁N₆O₂ :** 363.18 [M+H]+, found : 363.30. |
| (112) | 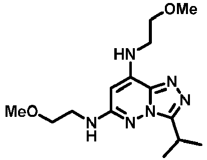 | > 96 % ^{a} | Beige solid. |
| | | | **¹H NMR (400 MHz, DMSO-*d₆)* δ :** 1.36 (d, *J* = 6.9 Hz, 6H, 2×CH_{3 iPr}), 3.28 (s, 6H, 2xOCH₃), 3.31-3.39 (m, 5H, 2×CH_{2-NH} & H_{iPr}), 3.52 (dt, *J* = 13.4, 5.7 Hz, 4H, 2×CH_{2-OCH3}), 5.60 (s, 1H, H_{Ar}), 6.62 (t, *J* = 5.5 Hz, 1H, NH), 7.08 (t, *J* = 5.8 Hz, 1H, NH). |
| | | | **¹³C NMR (101 MHz, DMSO-*d₆)* δ : 19.6** (2xCH_{3 iPr}), 24.7 (CH_{iPr}), 40.4 (CH_{2-NH}), 41.6 (CH_{2-NH}), 57.9 (OCH₃), 58.1 (OCH₃), 69.6 (CH_{2-OCH3}), 70.2 (CH_{2-OCH3}), 83.5 (CH_{Ar}), 139.7 (C_{q}), 140.2 (C_{q}), 152.8 (C_{q}), 156.0 (C_{q}). |
| | | | **MS (ESI+) : m/z calcd for C₁₄H₂₄N₆O₂** : 309.20 [M+H]+, **found** : 309.25. |
| (113) | 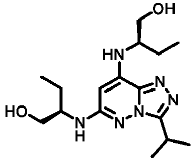 | > 96 % ^{a} | White solid. |
| | | | **¹H NMR (400 MHz, DMSO-*d₆)* δ :** 0.91 (td, *J* = 7.4, 4.4 Hz, 6H, 2×CH_{3-CH2}), 1.36 (d, *J* = 7.0 Hz, 6H, 2×CH_{3 iPr}), 1.40 - 1.62 (m, 2H, CH_{2-CH3}), 1.67 (dddd, *J* = 13.3, 9.6, 7.5, 5.7 Hz, 2H, CH_{2-CH3}), 3.34 (s, 1H, H_{iPr}), 3.39 - 3.58 (m, 5H, 2×CH_{2-OH} & H_{Alk}), 3.62 - 3.74 (m, 1H, H_{Alk}), 4.62 (t, *J* = 5.4 Hz, 1H, OH), 4.83 (t, *J* = 5.5 Hz, 1H, OH), 5.64 (s, 1H, H_{Ar}), 6.27 (d, *J* = 7.8 Hz, 1H, NH), 6.57 (d, *J* = 8.6 Hz, 1H, NH). |
| | | | **¹³C NMR (101 MHz, DMSO-*d₆)* δ :** 10.6 (CH_{3-CH2}), 10.6 (CH_{3-CH2}), 19.6 (2xCH_{3 iPr}), 23.3 (CH_{2-CH3}), 23.7 (CH_{2-CH3}), 24.8 (CH_{iPr}), 53.8 (CH_{Alk}), 55.7 (CH_{Alk}), 62.1 (CH_{2-OH}), 62.2 (CH_{2-OH}), 83.8 (CH_{Ar}), 139.8 (C_{q}), 140.0 (C_{q}), 152.7 (C_{q}), 156.1 (C_{q}). |
| | | | **MS (ESI+) : m/z calcd for C₁₆H₂₈N₆O₂ :** 337.23 [M+H]+, found : 337.30. |
| (114) | 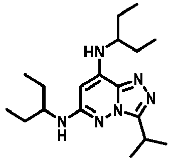 | > 95 % ^{a} | White crystalline solid. |
| | | | **¹H NMR (400 MHz, DMSO-*d₆)* δ :** 0.88 (td, *J* = 7.4, 4.8 Hz, 12H, 4×CH_{3 iPent}), 1.36 (d, *J* = 7.0 Hz, 6H, 2xCH_{3 iPr}), 1.43 - 1.63 (m, 8H, 4×CH_{2 iPent}), 3.23 - 3.38 (m, 2H, HiPr & HiPent), 3.54 - 3.66 (m, 1H, HiPent), 5.54 (s, 1H, H_{Ar}), 6.23 (d, *J* = 8.0 Hz, 1H, NHiPent), 6.75 (d, *J* = 8.7 Hz, 1H, NHiPent). |
| | | | **¹³C NMR (101 MHz, DMSO-*d₆)* δ :** 10.4 (2xCH_{3 iPent}), 10.6 (2xCH_{3 iPent}), 19.5 (2xCH_{3 iPr}), 24.8 (CH_{iPr}), 26.1 (2xCH_{2 iPent}), 26.2 (2xCH_{2 iPent}), 52.9 (CHiPent), 55.2 (CH_{iPent}), 83.2 (CH_{Ar}), 139.7 (C_{q}), 140.1 (C_{q}), 152.7 (C_{q}), 156.2 (C_{q}). |
| | | | **MS (ESI+) : m/z calcd for C₁₈H₃₂N₆ :** 333.28 [M+H]+, found : 333.37. |
| (115) | 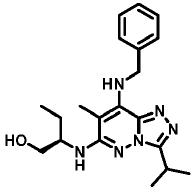 | 91 % ^{a} | White solid. |
| | | | **¹H NMR (400 MHz, DMSO-*d₆*) δ :** 0.89 (t, *J* = 7.4 Hz, 3H, CH_{3-CH2}), 1.34 (dd, *J* = 7.0, 2.9 Hz, 6H, 2xCH_{3 iPr}), 1.50 - 1.74 (m, 2H, CH_{2-CH3}), 2.02 (s, 3H, CH₃), 3.26 - 3.37 (m, 1H, H_{iPr}), 3.51 (dq, *J* = 18.5, 5.3 Hz, 2H, CH_{2-OH}), 3.78 (h, *J* = 6.2 Hz, 1H, H_{Alk}), 4.61 (t, *J* = 5.6 Hz, 1H, OH), 5.26 - 5.39 (m, 2H, CH_{2 Bn}), 5.40 (d, *J* = 7.7 Hz, 1H, NH_{Alk}), 6.81 (t, *J* = 6.9 Hz, 1H, NH_{Bn}), 7.14 - 7.35 (m, 5H, 5×H_{Ar}). |
| | | | **¹³C NMR (101 MHz, DMSO-*d₆)* δ :** 10.1 (CH₃), 10.8 (CH_{3-CH2}), 19.4 (CH_{3 iPr}), 19.4 (CH_{3 iPr}), 23.5 (CH_{2-CH3}), 24.7 (CH_{iPr}), 47.4 (CH_{2 Bn}), 54.5 (CH_{Alk}), 62.1 (CH_{2-OH}), 94.1 (C_{q}), 126.4 (CH_{Ar}), 126.8 (2xCH_{Ar}), 128.2 (2xCH_{Ar}), 138.2 (C_{q}), 139.9 (C_{q}), 141.7 (C_{q}), 151.4 (C_{q}), 154.2 (C_{q}). |
| | | | **MS (ESI+) : m/z calcd for C₂₀H₂₈N₆O :** 369.24 [M+H]+, **found** : 369.31. |
| (116) | 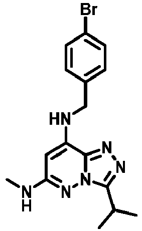 | 94 % ^{a} | Beige solid. |
| | | | **¹H NMR (400 MHz, DMSO-*d₆)* δ :** 1.36 (d, *J* = 6.9 Hz, 6H, 2xCH_{3 iPr}), 2.68 (d, *J* = 4.8 Hz, 3H, CH_{3-NH}), 3.35 (d, *J* = 6.9 Hz, 1H, H_{iPr}), 4.41 (d, *J* = 6.3 Hz, 2H, CH_{2Bn}), 5.29 (s, 1H, H_{Ar}), 6.52 (q, *J* = 4.8 Hz, 1H, NH_{-CH3}), 7.29 (d, *J* = 8.2 Hz, 2H, 2xH_{Ar}), 7.48 - 7.60 (m, 2H, 2xH_{Ar}), 7.93 (t, *J* = 6.3 Hz, 1H, NH_{Bn}). |
| | | | **¹³C NMR (101 MHz, DMSO-*d₆)* δ :** N.D. |
| | | | **MS (ESI+) : m/z calcd for C₁₆H₁₉BrN₆ :** 375.09 [M+H]+, **found** : 375.15. |
| (117) | 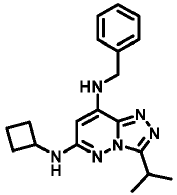 | > 95 %^{b} | Beige solid. |
| | | | **¹H NMR (400 MHz, DMSO-*d₆)* δ :** 1.36 (d, *J* = 7.0 Hz, 6H, 2xCH_{3 iPr}), 1.62-1.71 (m, 2H, CH_{2 cBut}), 1.76 - 1.88 (m, 2H, CH_{2 cBut}), 2.18 - 2.29 (m, 2H, CH_{2 cBut}), 3.32 - 3.36 (m, 1H, H_{iPr}), 4.04 - 4.13 (m, 1H, H_{cBut}), 4.43 (d, *J* = 6.3 Hz, 2H, CH_{2Bn}), 5.31 (s, 1H, H_{Ar}), 6.79 (d, *J* = 6.7 Hz, 1H, NH), 7.24 (dt, *J* = 7.7, 4.0 Hz, 1H, H_{Ar}), 7.34 (d, *J* = 4.4 Hz, 4H, 4xH_{Ar}), 7.90 (t, *J* = 6.8 Hz, 1H, NH_{Bn}). |
| | | | **¹³C NMR (101 MHz, DMSO-*d*₆) δ** : N.D. |
| | | | **MS (ESI+) : m/z calcd for C₁₉H₂₄N₆ :** 337.2 [M+H]+, **found** : 337.2. LogD (HPLC): 3.47 |
| (118) | 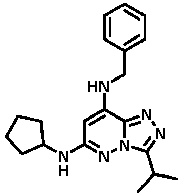 | > 95 %^{b} | Beige solid. |
| | | | **¹H NMR (400 MHz, DMSO-*d₆)* δ : 1.37** (d, *J* = 6.9 Hz, 6H, 2×CH_{3 iPr}), 1.39 - 1.54 (m, 4H, CH₂), 1.61 (d, *J* = 7.1 Hz, 2H, CH₂), 1.89 (dt, *J* = 12.6, 6.0 Hz, 2H, CH₂), 3.32 - 3.37 (m, 1H, H_{iPr}), 3.93 (q, *J* = 6.5 Hz, 1H, H_{cPent}), 4.42 (d, *J* = 6.4 Hz, 2H, CH_{2 Bn}), 5.37 (s, 1H, H_{Ar}), 6.52 (d, *J* = 6.1 Hz, 1H, NH), 7.24 (dt, *J* = 8.6, 4.4 Hz, 1H, H_{Ar}), 7.30 - 7.36 (m, 4H, 4xH_{Ar}), 7.85 (t, *J* = 6.4 Hz, 1H, NH_{Bn}). |
| | | | **¹³C NMR (101 MHz, DMSO-*d₆)* δ :** 19.6 (2xCH_{3 iPr}), 23.6 (2xCH₂), 24.8 (CH_{iPr}), 32.3 (2×CH2), 45.2 (CH_{2 Bn}), 52.3 (CH_{cPent}), 84.3 (CH_{Ar}), 126.7 (2xCH_{Ar}), 126.9 (CH_{Ar}), 128.4 (CH_{Ar}), 138.4 (C_{q}), 139.6 (C_{q}), 139.7 (C_{q}), 152.8 (C_{q}), 155.5 (C_{q}). |
| | | | **MS (ESI+) : m/z calcd for C₂₀H₂₆N₆ :** 351.2 [M+H]+, **found** : 351.1. LogD (HPLC): 3.84 |
| (119) | 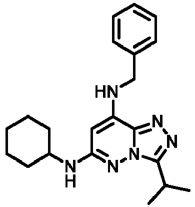 | > 95 %^{b} | Beige solid. |
| | | | **¹H NMR (400 MHz, DMSO-*d₆)* δ :** 1.15 (q, *J* = 10.9, 10.2 Hz, 3H, CH₂ & H_{(CH2)}), 1.22 - 1.30 (m, 2H, CH₂), 1.36 (d, *J* = 7.0 Hz, 6H, 2xCH_{3 iPr}), 1.56 (d, *J* = 12.3 Hz, 1H, H_{(CH2)}), 1.64 - 1.72 (m, 2H, CH₂), 1.92 (d, *J* = 12.1 Hz, 2H, CH₂), 3.26 - 3.35 (m, 1H, H_{iPr}), 3.51 (dt, *J* = 6.9, 3.5 Hz, 1H, H_{cHex}), 4.42 (d, *J* = 6.3 Hz, 2H, CH_{2 Bn}), 5.39 (s, 1H, H_{Ar}), 6.37 (d, *J* = 7.2 Hz, 1H, NH), 7.20 - 7.29 (m, 1H, H_{Ar}), 7.29 - 7.37 (m, 4H, 4xH_{Ar}), 7.85 (t, *J* = 6.4 Hz, 1H, NH_{Bn}). |
| | | | **¹³C NMR (101 MHz, DMSO-*d₆)* δ :** 19.5 (2×CH_{3 iPr}), 24.5 (2xCH₂), 24.8 (CH_{iPr}), 25.5 (CH₂), 32.1 (2xCH₂), 45.2 (CH_{2 Bn}), 49.3 (CH_{cHex}), 84.3 (CH_{Ar}), 126.8 (2xCH_{Ar}), 126.9 (CH_{Ar}), 128.4 (2xCH_{Ar}), 138.4 (C_{q}), 139.6 (C_{q}), 139.8 (C_{q}), 152.7 (C_{q}), 155.0 (C_{q}). |
| | | | **MS (ESI+) : m/z calcd for C₂₁H₂₈N₆ :** 365.2 [M+H]⁺, found : 365.1. LogD (HPLC): 4.17 |
| (120) | 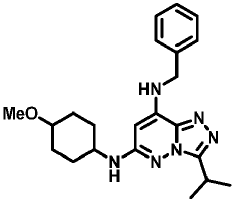 | > 95 %^{b} | Beige solid. |
| | | | **¹H NMR (400 MHz, DMSO-*d₆*) δ :** 1.12-1.22 (m, 4H, 2×CH₂), 1.36 (d, *J* = 7.0 Hz, 6H, 2xCH_{3 iPr}), 1.93 - 2.04 (m, 4H, 2×CH₂), 3.10 (d, *J* = 4.0 Hz, 1H, CH_{-O}), 3.22 (s, 3H, CH_{3-O}), 3.27 - 3.35 (m, 1H, H_{iPr}), 3.48 (s, 1H, CH_{-NH}), 4.42 (d, *J* = 6.3 Hz, 2H, CH_{2 Bn}), 5.36 (s, 1H, H_{Ar}), 6.40 (d, *J* = 7.0 Hz, 1H, NH), 7.24 (td, *J* = 4.9, 2.7 Hz, 1H, H_{Ar}), 7.29 - 7.37 (m, 4H, 4xH_{Ar}), 7.88 (t, *J* = 6.3 Hz, 1H, NH_{Bn}). |
| | | | **¹³C NMR (101 MHz, DMSO-*d₆)* δ :** 19.5 (2xCH_{3 iPr}), 24.8 (CH_{iPr}), 29.4 (2xCH₂), 29.8 (2xCH₂), 45.2 (CH_{2Bn}), 49.0 (CH_{cHex}), 55.0 (CH₂), 77.7 (CH_{3-O}), 84.2 (CH_{Ar}), 126.7 (2xCH_{Ar}), 126.9 (CH_{Ar}), 128.4 (2xCH_{Ar}), 138.4 (C_{q}), 139.6 (C_{q}), 139.9 (C_{q}), 152.7 (C_{q}), 155.2 (C_{q}). |
| | | | **MS (ESI+) : m/z calcd for C₂₂H₃₀N₆O :** 395.3 [M+H]+, **found** : 395.1. LogD (HPLC): 3.09 |
| (121) | 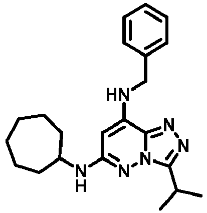 | > 95 %^{b} | White solid. |
| | | | **¹H NMR (400 MHz, DMSO-*d₆)* δ :** 1.36 (d, *J* = 7.0 Hz, 6H, 2xCH_{3 iPr}), 1.39 - 1.67 (m, 10H, 5×CH₂), 1.81 - 1.94 (m, 2H, CH₂), 3.26 - 3.38 (m, 1H, H_{iPr}), 3.70 (d, *J* = 8.5 Hz, 1H, H_{CHept}), 4.42 (d, *J* = 6.3 Hz, 2H, CH_{2 Bn}), 5.39 (s, 1H, H_{Ar}), 6.42 (d, *J* = 7.1 Hz, 1H, NH), 7.24 (td, *J* = 5.3, 3.0 Hz, 1H, H_{Ar}), 7.29 - 7.37 (m, 4H, 2xH_{Ar}), 7.84 (t, *J* = 6.4 Hz, 1H, NH_{Bn}). |
| | | | **¹³C NMR (101 MHz, DMSO-*d₆)* δ :** 19.5 (2xCH_{3 iPr}), 24.1 (2xCH₂), 24.9 (CH_{iPr}), 28.0 (2xCH₂), 33.6 (2×CH₂), 45.2 (CH_{2Bn}), 51.3 (CH_{cHept}), 84.3 (CH_{Ar}), 126.7 (2xCH_{Ar}), 126.9 (CH_{Ar}), 128.4 (2xCH_{Ar}), 138.4 (C_{q}), 139.6 (C_{q}), 139.8 (C_{q}), 152.7 (C_{q}), 154.8 (C_{q}). |
| | | | **MS (ESI+) : m/z calcd for C₂₂H₃₀N₆ :** 379.3 [M+H]+, found : 379.2. LogD (HPLC): 4.57 |
| (122) | 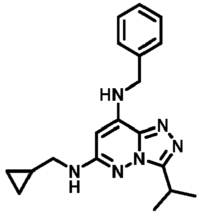 | 91%^{b} | Beige solid. |
| | | | **¹H NMR (400 MHz, DMSO-*d₆)* δ :** 0.14 - 0.21 (m, 2H, CH₂), 0.37 - 0.45 (m, 2H, CH2), 0.98 - 1.07 (m, 1H, H_{cPr}), 1.36 (d, *J* = 7.0 Hz, 6H, 2xCH_{3 iPr}), 3.00 (dd, *J* = 6.9, 5.2 Hz, 2H, CH_{2-NH}), 3.33 (p, *J* = 7.0 Hz, 1H, H_{iPr}), 4.43 (d, *J* = 6.3 Hz, 2H, CH_{2 Bn}), 5.43 (s, 1H, H_{Ar}), 6.66 (t, *J* = 5.3 Hz, 1H, NH), 7.24 (dq, *J* = 6.0, 2.8 Hz, 1H, H_{Ar}), 7.30 - 7.41 (m, 4H, 4xH_{Ar}), 7.89 (t, *J* = 6.4 Hz, 1H, NH_{Bn}). |
| | | | **¹³C NMR (101 MHz, DMSO-*d₆)* δ :** 3.5 (2×CH_{2 cPr}), 10.5 (CH_{cPr}), 19.6 (2xCH_{3 iPr}), 24.6 (CH_{iPr}), 45.2 (CH_{2 Bn}), 45.4 (CH₂), 84.3 (CH_{Ar}), 126.8 (2xCH_{Ar}), 126.9 (CH_{Ar}), 128.4 (2×CH_{Ar}), 138.3 (C_{q}), 139.8 (C_{q}), 141.2 (C_{q}), 152.8 (C_{q}), 155.9 (C_{q}). |
| | | | **MS (ESI+) : m/z calcd for C₁₉H₂₄N₆ :** 337.2 [M+H]+, **found** : 337.2. LogD (HPLC): 3.33 |
| (123) | 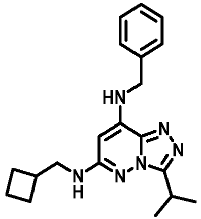 | 87%^{b} | Beige solid. |
| | | | **¹H NMR (400 MHz, DMSO-*d₆)* δ :** 1.36 (d, *J* = 7.0 Hz, 6H, 2xCH_{3 iPr}), 1.67 (tt, *J* = 10.2, 7.8 Hz, 2H, CH₂), 1.78 - 1.86 (m, 2H, CH₂), 1.93 - 2.07 (m, 2H, CH₂), 2.51 - 2.55 (m, 1H, H_{cBut}), 3.16 (dd, *J* = 7.1, 5.3 Hz, 2H, CH₂), 3.32 - 3.38 (m, 1H, H_{iPr}), 4.43 (d, *J* = 6.4 Hz, 2H, CH_{2 Bn}), 5.39 (s, 1H, H_{Ar}), 6.48 (t, *J* = 5.5 Hz, 1H, NH), 7.24 (td, *J* = 5.2, 2.7 Hz, 1H, H_{Ar}), 7.30 - 7.36 (m, 4H, 4xH_{Ar}), 7.86 (t, *J* = 6.4 Hz, 1H, NH_{Bn}). |
| | | | **¹³C NMR (101 MHz, DMSO-*d₆)* δ :** 17.9 (CH_{cBut}), 19.6 (2×CH_{3 iPr}), 24.6 (CH_{iPr}), 25.6 (2xCH₂), 34.3 (CH₂), 45.2 (CH_{2 Bn}), 46.2 (CH₂), 84.1 (CH_{Ar}), 126.8 (2xCH_{Ar}), 126.9 (CH_{Ar}), 128.4 (2xCH_{Ar}), 138.3 (C_{q}), 139.6 (C_{q}), 139.8 (C_{q}), 152.8 (C_{q}), 156.0 (C_{q}). |
| | | | **MS (ESI+) : m/z calcd for C₂₀H₂₆N₆ :** 351.2 [M+H]+, **found** : 351.1. LogD (HPLC): 3.86 |
| (124) | 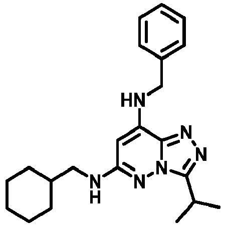 | > 96 %^{b} | White solid. |
| | | | **¹H NMR (400 MHz, DMSO-*d₆)* δ :** 0.90 (q, *J* = 11.1, 10.0 Hz, 2H, CH₂), 1.15 (t, *J* = 11.1 Hz, 3H, H_{(CH2)} & CH₂), 1.36 (d, *J* = 7.0 Hz, 6H, 2xCH_{3 iPr}), 1.47 - 1.56 (m, 1H, H_{cHex}), 1.59 - 1.73 (m, 5H, 2×CH₂ &H_{(CH2)}), 2.97 (t, *J* = 6.1 Hz, 2H, CH₂), 3.27 - 3.36 (m, 1H, H_{iPr}), 4.43 (d, *J* = 6.4 Hz, 2H, CH_{2 Bn}), 5.41 (s, 1H, H_{Ar}), 6.52 (t, *J* = 5.5 Hz, 1H, NH), 7.21 - 7.28 (m, 1H, H_{Ar}), 7.29 - 7.39 (m, 4H, 4xH_{Ar}), 7.85 (t, *J* = 6.4 Hz, 1H, NH_{Bn}). |
| | | | **¹³C NMR (101 MHz, DMSO-*d₆)* δ :** 19.5 (2xCH_{3 iPr}), 24.7 (CH_{iPr}), 25.5 (2xCH₂), 26.0 (CH_{cHex}), 30.7 (2×CH₂), 36.9 (CH₂), 45.2 (CH_{2 Bn}), 47.3 (CH₂), 84.1 (CH_{Ar}), 126.8 (2xCH_{Ar}), 126.9 (CH_{Ar}), 128.4 (2xCH_{Ar}), 138.3 (C_{q}), 139.7 (C_{q}), 139.8 (C_{q}), 139.8 (C_{q}), 156.1 (C_{q}). |
| | | | **MS (ESI⁺) : m/z calcd for C₂₂H₃₀N₆ :** 379.3 [M+H]+, found : 379.2. LogD (HPLC): 4.60 |
| (125) | 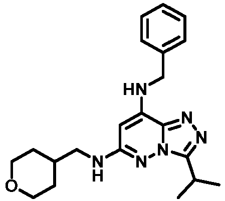 | > 98 %^{b} | White solid. |
| | | | **¹H NMR (400 MHz, DMSO-*d₆***) **δ :** 1.18 (qd, *J* = 12.1, 4.6 Hz, 2H, CH₂), 1.36 (d, *J* = 7.0 Hz, 6H, 2xCH_{3 iPr}), 1.58 (d, *J* = 13.1 Hz, 2H, CH₂), 1.78 (dtq, *J* = 15.6, 7.5, 4.6, 3.8 Hz, 1H, H_{THP}), 3.03 (t, *J =* 6.1 Hz, 2H, CH₂), 3.23 (td, *J =* 11.7, 2.1 Hz, 2H, CH₂), 3.28 - 3.38 (m, 1H, H_{iPr}), 3.82 (dt, *J* = 9.5, 2.7 Hz, 2H, CH₂), 4.43 (d, *J* = 6.3 Hz, 2H, CH2 _{Bn}), 5.40 (s, 1H, H_{Ar}), 6.58 (t, *J =* 5.6 Hz, 1H, NH), 7.25 (dt, *J =* 5.6, 2.9 Hz, 1H, H_{Ar}), 7.29 - 7.38 (m, 4H, 4×H_{Ar}), 7.88 (t, *J* = 6.4 Hz, 1H, NH_{Bn}). |
| | | | **¹³C NMR (101 MHz, DMSO-*d₆)* δ :** 19.6 (2xCH_{3 iPr}) , 24.7 (CH_{iPr}), 30.7 (2xCH₂), 34.3 (CH_{THP}), 45.2 (CH_{2 Bn}), 46.7 (CH₂), 66.8 (2×CH₂), 84.0 (CH_{Ar}), 126.8 (2xCH_{Ar}), 126.9 (CH_{Ar}), 128.4 (2xCH_{Ar}), 138.3 (C_{q}), 139.6 (C_{q}), 139.8 (C_{q}), 152.8 (C_{q}), 156.0 (C_{q}). |
| | | | **MS (ESI+) : m/z calcd for C₂₁H₂₈N₆O :** 381.2 [M+H]+, found : 381.1. LogD (HPLC): 2.74 |
| (126) | 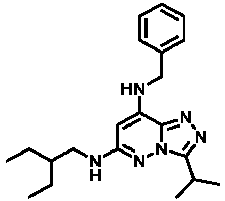 | > 95 %^{b} | Beige solid. |
| | | | **¹H NMR (400 MHz, DMSO-*d₆)* δ :** 0.85 (t, *J* = 7.4 Hz, 6H, 2×CH_{3-CH2}), 1.22 - 1.33 (m, 4H, 2×CH_{2-CH3}), 1.36 (d, *J* = 7.0 Hz, 6H, 2xCH_{3 iPr}), 1.42 - 1.52 (m, 1H, H_{Alk}), 3.07 (t, *J* = 6.0 Hz, 2H, CH₂), 3.31 - 3.36 (m, 1H, H_{iPr}), 4.43 (d, *J* = 6.4 Hz, 2H, CH_{2 Bn}), 5.42 (s, 1H, H_{Ar}), 6.50 (t, *J* = 5.7 Hz, 1H, NH), 7.25 (dt, *J* = 5.8, 2.9 Hz, 1H, H_{Ar}), 7.29 - 7.37 (m, 4H, 4×H_{Ar}), 7.84 (t, *J* = 6.4 Hz, 1H, NH_{Bn}). |
| | | | **¹³C NMR (101 MHz, DMSO-*d₆)* δ :** 10.9 (2×CH_{3-CH2}), 19.6 (2×CH_{3 iPr}), 23.5 (2×CH_{2-CH3}), 24.7 (CH_{iPr}), 39.8 (CH_{Alk}), 43.3 (CH2), 45.2 (CH_{2 Bn}), 84.2 (CH_{Ar}), 126.8 (2xCH_{Ar}), 126.9 (CH_{Ar}), 128.4 (2xCH_{Ar}), 138.4 (C_{q}), 139.7 (C_{q}), 139.8 (C_{q}), 152.8 (C_{q}), 156.1 (C_{q}). |
| | | | **MS (ESI⁺) : m/z calcd for C₂₁H₃₀N₆ :** 367.3 [M+H]+, found : 367.1. LogD (HPLC): 4.42 |
| (127) | 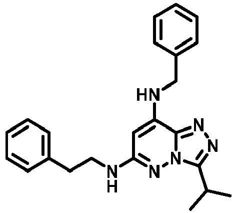 | 94%^{b} | White solid. |
| | | | **¹H NMR (400 MHz, DMSO-*d₆)* δ :** 1.38 (d, *J* = 7.0 Hz, 6H, 2xCH_{3 iPr}), 2.78 - 2.88 (m, 2H, CH₂), 3.36 (td, *J* = 8.5, 7.7, 5.1 Hz, 3H, H_{iPr} & CH₂), 4.44 (d, *J* = 6.4 Hz, 2H, CH_{2 Bn}), 5.38 (s, 1H, H_{Ar}), 6.68 (t, *J* = 5.5 Hz, 1H, NH), 7.16 - 7.36 (m, 10H, 10xH_{Ar}), 7.91 (t, *J* = 6.4 Hz, 1H, NH_{Bn}). |
| | | | **¹³C NMR (101 MHz, DMSO-*d₆)* δ :** N.D. |
| | | | **MS (ESI+) : m/z calcd for C₂₃H₂₆N₆ :** 387.2 [M+H]+, found : 387.1. LogD (HPLC): 3.78 |
| (128) | 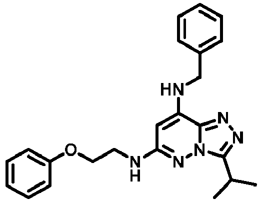 | 88%^{b} | White solid. |
| | | | **¹H NMR (400 MHz, DMSO-*d₆)* δ :** 1.36 (d, *J* = 7.0 Hz, 6H, 2×CH_{3 iPr}), 3.32 - 3.40 (m, 1H, H_{iPr}), 3.52 (q, *J* = 5.7 Hz, 2H, CH₂), 4.11 (t, *J* = 5.8 Hz, 2H, CH₂), 4.44 (d, *J* = 6.4 Hz, 2H, CH_{2Bn}), 5.45 (s, 1H, H_{Ar}), 6.81 (t, *J* = 5.6 Hz, 1H, NH), 6.90 - 6.97 (m, 3H, 3xH_{Ar}), 7.21-7.38 (m, 7H, 7×H_{Ar}), 7.95 (t, *J* = 6.4 Hz, 1H, NH_{Bn}). |
| | | | **¹³C NMR (101 MHz, DMSO-*d₆)* δ :** N.D. |
| | | | **MS (ESI+) : m/z calcd for C₂₃H₂₆N₆O :** 403.2 [M+H]+, found : 403.1. LogD (HPLC): 3.68 |
| (129) | 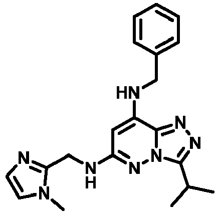 | > 99 %^{b} | White solid. |
| | | | **¹H NMR (400 MHz, DMSO-*d₆)* δ :** 1.34 (d, *J* = 7.0 Hz, 6H, 2×CH_{3 iPr}), 3.31 - 3.39 (m, 1H, H_{iPr}), 3.63 (s, 3H, CH_{3-N}), 4.35 (d, *J* = 5.3 Hz, 2H, CH₂), 4.42 (d, *J* = 6.4 Hz, 2H, CH_{2Bn}), 5.53 (s, 1H, H_{Ar}), 6.76 (d, *J* = 1.2 Hz, 1H, H_{Ar}), 7.00 (t, *J* = 5.4 Hz, 1H, NH), 7.04 (d, *J* = 1.2 Hz, 1H, H_{Ar}), 7.21 - 7.26 (m, 1H, H_{Ar}), 7.29 - 7.38 (m, 4H, 4×H_{Ar}), 7.96 (t, *J* = 6.3 Hz, 1H, NH_{Bn}). |
| | | | **¹³C NMR (101 MHz, DMSO-*d₆)* δ :** 19.7 (2×CH_{3 iPr}), 24.5 (CH_{iPr}), 32.3 (CH_{3-N}), 36.8 (CH2), 45.2 (CH_{2 Bn}), 84.0 (CH_{Ar}), 121.5 (CH_{Ar}), 126.3 (CH_{Ar}), 126.9 (CH_{Ar}), 126.9 (2xCH_{Ar}), 128.4 (2xCH_{Ar}), 138.3 (C_{q}), 139.6 (C_{q}), 140.0 (C_{q}), 144.9 (C_{q}), 152.9 (C_{q}), 155.5 (C_{q}). |
| | | | **MS (ESI+) : m/z calcd for C₂₀H₂₄N₈ :** 377.2 [M+H]+, found : 377.1. LogD (HPLC): 1.95 |
| (130) | 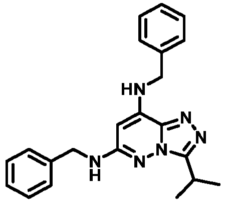 | > 96 %^{b} | White solid. |
| | | | **¹H NMR (400 MHz, DMSO-*d₆)* δ :** 1.30 (d, *J* = 7.0 Hz, 6H, 2×CH_{3 iPr}), 3.25 - 3.33 (m, 1H, H_{iPr}), 4.32 (d, *J* = 5.7 Hz, 2H, CH_{2 Bn}), 4.43 (d, *J* = 6.4 Hz, 2H, CH_{2 Bn}), 5.46 (s, 1H, H_{Ar}), 7.09 (t, *J* = 5.8 Hz, 1H, NH_{Bn}), 7.18 - 7.36 (m, 10H, 10xH_{Ar}), 7.93 (t, *J* = 6.3 Hz, 1H, NH_{Bn}). |
| | | | **¹³C NMR (101 MHz, DMSO-*d₆)* δ :** 19.6 (2xCH_{3 iPr}), 24.6 (CH_{iPr}), 44.5 (CH_{2 Bn}), 45.2 (CH_{2 Bn}), 84.0 (CH_{Ar}), 126.6 (CH_{Ar}), 126.8 (2xCH_{Ar}), 126.9 (CH_{Ar}), 127.7 (2xCH_{Ar}), 128.1 (2xCH_{Ar}), 128.4 (2xCH_{Ar}), 138.3 (C_{q}), 139.6 (C_{q}), 139.8 (C_{q}), 140.0 (C_{q}), 152.9 (C_{q}), 155.6 (C_{q}). |
| | | | **MS (ESI+) : m/z calcd for C₂₂H₂₄N₆ :** 373.2 [M+H]+, found : 373.1. LogD (HPLC): 3.50 |
| (131) | 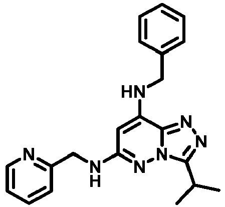 | > 97 %^{b} | Brown solid. |
| | | | **¹H NMR (400 MHz, DMSO-*d₆)* δ :** 1.22 (d, *J* = 7.0 Hz, 6H, 2xCH_{3 iPr}), 3.22 (q, *J* = 7.1 Hz, 1H, H_{iPr}), 4.38 - 4.50 (m, 4H, 2×CH_{2 Bn}), 5.54 (s, 1H, H_{Ar}), 7.19 - 7.27 (m, 3H, 2×H_{Ar} & NH), 7.30 - 7.38 (m, 5H, 5×H_{Ar}), 7.69 (td, *J* = 7.6, 1.8 Hz, 1H, H_{Ar}), 7.95 (t, *J* = 6.4 Hz, 1H, NH_{Bn}), 8.47 (d, *J* = 4.5 Hz, 1H, H_{Ar}). |
| | | | **¹³C NMR (101 MHz, DMSO-*d₆)*** δ : 19.4 (2×CH_{3 iPr}), 24.6 (CH_{iPr}), 45.2 (CH_{2 Bn}), 46.4 (CH₂), 84.0 (CH_{Ar}), 121.2 (CH_{Ar}), 121.8 (CH_{Ar}), 126.9 (2xCH_{Ar}), 127.0 (CH_{Ar}), 128.4 (2xCH_{Ar}), 136.4 (CH_{Ar}), 138.3 (C_{q}), 139.6 (C_{q}), 140.1 (C_{q}), 148.7 (C_{q}), 152.8 (C_{q}), 155.6 (C_{q}), 159.0 (C_{q}). |
| | | | **MS (ESI+) : m/z calcd for C21H23N7** : 374.2 [M+H]+, **found** : 374.1. LogD (HPLC): 2.51 |
| (132) | 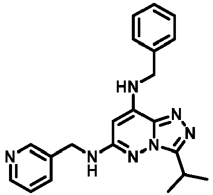 | > 97 %^{b} | White solid. |
| | | | **¹H NMR (400 MHz**, **DMSO-*d₆)* δ :** 1.28 (d, *J* = 6.9 Hz, 6H, 2xCH_{3 iPr}), 3.22 - 3.30 (m, 1H, H_{iPr}), 4.34 (d, *J* = 5.7 Hz, 2H, CH₂), 4.44 (d, *J* = 6.3 Hz, 2H, CH_{2 Bn}), 5.43 (s, 1H, H_{Ar}), 7.18 (t, *J* = 5.8 Hz, 1H, NH), 7.24 (dq, *J* = 5.7, 2.8 Hz, 1H, H_{Ar}), 7.27 - 7.37 (m, 5H, 5×H_{Ar}), 7.70 (dt, *J* = 7.8, 2.0 Hz, 1H, H_{Ar}), 7.98 (t, *J* = 6.4 Hz, 1H, NH_{Bn}), 8.41 (dd, *J* = 4.8, 1.7 Hz, 1H, H_{Ar}), 8.54 (d, *J* = 2.3 Hz, 1H, H_{Ar}). |
| | | | **¹³C NMR (101 MHz, DMSO-*d₆)* δ :** 19.5 (2xCH_{3 iPr}), 24.6 (CH_{iPr}), 42.2 (CH₂), 45.2 (CH_{2 Bn}), 83.8 (CH_{Ar}), 123.3 (CH_{Ar}), 126.8 (2×CH_{Ar}), 126.9 (CH_{Ar}), 128.4 (2xCH_{Ar}), 135.2 (C_{q}), 135.3 (CH_{Ar}), 138.2 (C_{q}), 139.6 (C_{q}), 140.1 (C_{q}), 147.9 (CH_{Ar}), 149.1 (CH_{Ar}), 152.9 (C_{q}), 155.5 (C_{q}). |
| | | | **MS (ESI+) : m/z calcd for C₂₁H₂₃N₇** : 374.2 [M+H]+, **found** : 374.1. LogD (HPLC): 2.36 |
| (133) | 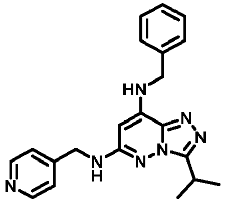 | > 99 %^{b} | White solid. |
| | | | **¹H NMR (400 MHz, DMSO-*d₆)* δ :** 1.21 (d, *J* = 7.0 Hz, 6H, 2xCH_{3 iPr}), 3.20 (p, *J* = 7.0 Hz, 1H, H_{iPr}), 4.35 (d, *J* = 5.8 Hz, 2H, CH₂), 4.46 (d, *J* = 6.3 Hz, 2H, CH_{2Bn}), 5.47 (s, 1H, H_{Ar}), 7.21 - 7.29 (m, 4H, NH & 3×H_{Ar}), 7.29 - 7.38 (m, 4H, 4×H_{Ar}), 7.99 (t, *J* = 6.4 Hz, 1H, NH_{Bn}), 8.42 - 8.47 (m, 2H, 2xH_{Ar}). |
| | | | **¹³C NMR (101 MHz, DMSO-*d₆)* δ :** N.D. **MS (ESI+) : m/z calcd for C₂₁H₂₃N₇ :** 374.2 [M+H]+, found : 374.1. LogD (HPLC): 2.36 |
| (134) | 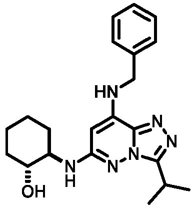 | > 98 % ^{b} | Brown solid. |
| | | | ¹**H NMR (400 MHz, DMSO-*d*₆) δ** : 1.03 (q, *J* = 10.2 Hz, 1H, H_{(CH2)}), 1.20 (dq, *J* = 19.6, 10.7, 9.2 Hz, 3H, 3xH_{(CH2)}), 1.36 (dd, *J* = 7.0, 5.9 Hz, 6H, 2xCH_{3 iPr}), 1.52 - 1.67 (m, 2H, 2xH_{(CH2)}), 1.86 (d, *J* = 10.9 Hz, 1H, H_{(CH2)}), 2.10 (d, *J* = 12.7 Hz, 1H, H_{(CH2)}), 3.25 - 3.44 (s, 3H, H_{iPr} & H_{C-O} & H_{C-NH}), 4.43 (d, *J* = 6.3 Hz, 2H, CH_{2 Bn}), 4.53 (d, *J* = 4.8 Hz, 1H, OH), 5.46 (s, 1H, H_{Ar}), 6.34 (d, *J* = 6.2 Hz, 1H, NH), 7.24 (ddt, *J* = 8.6, 5.7, 2.3 Hz, 1H, H_{Ar}), 7.29 - 7.41 (m, 4H, 4xH_{Ar}), 7.86 (t, *J* = 6.4 Hz, 1H, NH_{Bn}). |
| | | | **¹³C NMR (101 MHz, DMSO-*d*₆) δ** : 19.5 (CH_{3 iPr}), 19.6 (CH_{3 iPr}), 23.7 (CH₂), 23.9 (CH₂), 24.8 (CH_{iPr}), 30.1 (CH₂), 34.0 (CH₂), 45.2 (CH_{2 Bn}), 56.2 (CH_{-NH}), 71.0 (CH_{-OH}), 84.5 (CH_{Ar}), 126.8 (2xCH_{Ar}), 126.9 (CH_{Ar}), 128.4 (2xCH_{Ar}), 138.4 (C_{q}), 139.6 (C_{q}), 139.8 (C_{q}), 152.7 (C_{q}), 155.9 (C_{q}). |
| | | | **MS (ESI+) : m/z calcd for C₂₁H₂₈N₆O :** 381.2 [M+H]+, **found :** 381.2. LogD (HPLC): 2.73 |
| (135) | 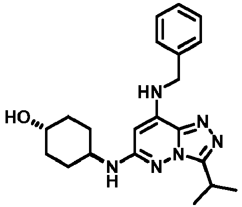 | > 98 %^{b} | Light brown solid. |
| | | | **¹H NMR (400 MHz, DMSO-*d*₆) δ** : 1.06 - 1.30 (m, 4H, 2xCH₂), 1.36 (d, *J* = 7.0 Hz, 6H, 2xCH_{3 iPr}), 1.73 - 2.03 (m, 4H, 2xCH₂), 3.26 - 3.36 (m, 1H, H_{iPr}), 3.41 (ddt, *J* = 18.5, 9.3, 4.3 Hz, 2H, Hc-o & H_{C-NH}), 4.42 (d, *J* = 6.3 Hz, 2H, CH_{2 Bn}), 4.48 (d, *J* = 4.5 Hz, 1H, OH), 5.36 (s, 1H, H_{Ar}), 6.36 (d, *J* = 7.1 Hz, 1H, NH), 7.23 (tt, *J* = 4.9, 3.1 Hz, 1H, H_{Ar}), 7.29 - 7.39 (m, 4H, 4xH_{Ar}), 7.87 (t, *J* = 6.4 Hz, 1H, NH_{Bn}). |
| | | | **¹³C NMR (101 MHz, DMSO-*d*₆) δ :** 19.5 (2xCH_{3 iPr}), 24.8 (CH_{iPr}), 29.9 (2xCH₂), 34.0 (2xCH₂), 45.2 (CH_{2 Bn}), 49.2 (CH_{-NH}), 68.3 (CH_{-OH}), 84.2 (CH_{Ar}), 126.8 (2xCH_{Ar}), 126.9 (CH_{Ar}), 128.4 (2xCH_{Ar}), 138.4 (C_{q}), 139.6 (C_{q}), 139.8 (C_{q}), 152.7 (C_{q}), 155.2 (C_{q}). |
| | | | **MS (ESI+) : m/z calcd for C₂₁H₂₈N₆O** : 381.2 [M+H]+, **found** : 381.2. LogD (HPLC): 2.22 |
| (136) | 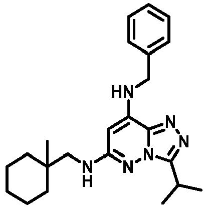 | > 95 %^{b} | Beige solid. |
| | | | **¹H NMR (400 MHz, DMSO-*d*₆) δ** : 0.86 (s, 3H, CH_{3-C}), 1.16 - 1.34 (m, 6H, 3xCH₂), 1.36 (d, *J* = 7.0 Hz, 6H, 2xCH_{3 iPr}), 1.38 - 1.51 (m, 4H, 2xCH₂), 3.08 (d, *J* = 6.0 Hz, 2H, CH_{2-C}), 3.25 - 3.37 (m, 1H, H_{iPr}), 4.43 (d, *J* = 6.4 Hz, 2H, CH_{2 Bn}), 5.55 (s, 1H, H_{Ar}), 6.35 (t, *J* = 6.0 Hz, 1H, NH), 7.24 (ddd, *J* = 8.5, 5.5, 2.4 Hz, 1H, H_{Ar}), 7.29 - 7.40 (m, 4H, 4xH_{Ar}), 7.82 (t, *J* = 6.4 Hz, 1H, NH_{Bn}). |
| | | | **¹³C NMR (101 MHz, DMSO-*d*₆) δ** : N.D. |
| | | | **MS (ESI+) : m/z calcd for C₂₃H₃₂N₆** : 393.3 [M+H]+, **found** : 393.3. LogD (HPLC): 4.88 |
| (137) | 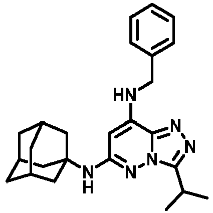 | > 96 %^{b} | Brown solid. |
| | | | **¹H NMR (400 MHz, DMSO-*d*₆) δ** : 1.39 (d, *J* = 7.0 Hz, 6H, 2xCH_{3 iPr}), 1.64 (s, 6H, 3xCH_{2 ada}), 2.05 (s, 9H, 3xCH_{2 ada} & 3xH_{ada}), 3.32 - 3.35 (m, 1H, H_{iPr}), 4.40 (d, *J* = 6.4 Hz, 2H, CH_{2 Bn}), 5.44 (s, 1H, H_{Ar}), 6.09 (s, 1H, NH), 7.20 - 7.27 (m, 1H, H_{Ar}), 7.30 - 7.36 (m, 4H, 4xH_{Ar}), 7.79 (t, *J* = 6.6 Hz, 1H, NH_{Bn}). |
| | | | **¹³C NMR (101 MHz, DMSO-*d*₆) δ :** N.D. |
| | | | **MS (ESI+) : m/z calcd for C₂₅H₃₂N₆** : 417.3 [M+H]+, **found** : 417.3. LogD (HPLC): 5.02 |
| (138) | 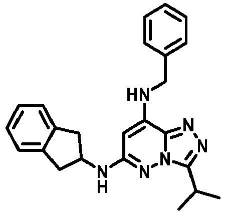 | > 97 %^{b} | Light brown solid. |
| | | | **¹H NMR (400 MHz, DMSO-*d*₆) δ** : 1.39 (d, *J =* 6.9 Hz, 6H, 2xCH_{3 iPr}), 2.81 (dd, *J =* 16.0, 5.6 Hz, 2H, CH₂), 3.25 (dd, *J* = 16.1, 7.3 Hz, 2H, CH₂), 3.36 (p, *J =* 7.0 Hz, 1H, H_{iPr}), 4.42 (t, *J* = 6.0 Hz, 3H, H_{indane} & CH_{2 Bn}), 5.38 (s, 1H, H_{Ar}), 6.86 (d, *J =* 6.1 Hz, 1H, NH), 7.13 (dd, *J* = 5.5, 3.2 Hz, 2H, 2xH_{Ar}), 7.17 - 7.27 (m, 3H, 3xH_{Ar}), 7.27 - 7.35 (m, 4H, 4xH_{Ar}), 7.92 (t, *J* = 6.4 Hz, 1H, NH_{Bn}). |
| | | | **¹³C NMR (101 MHz, DMSO-*d*₆) δ** : N.D. |
| | | | **MS (ESI+) : m/z calcd for C₂₄H₂₆N₆ :** 399.2 [M+H]+, **found** : 399.2. LogD (HPLC): 4.08 |
| (139) | 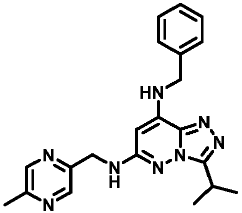 | > 98 %^{b} | Beige solid. |
| | | | **¹H NMR (400 MHz, DMSO-*d*₆) δ :** 1.23 (d, *J* = 6.9 Hz, 6H, 2xCH_{3 iPr}), 2.43 (s, 3H, CH3), 3.22 (p, *J* = 7.0 Hz, 1H, H_{iPr}), 4.42 (d, *J* = 5.8 Hz, 2H, CH_{2 Bn}), 4.45 (d, *J* = 6.3 Hz, 2H, CH_{2 Bn}), 5.49 (s, 1H, H_{Ar}), 7.11 - 7.48 (m, 6H, 5×H_{Ar} & NH), 7.99 (t, *J* = 6.4 Hz, 1H, NH_{Bn}), 8.43 (d, *J* = 1.4 Hz, 1H, H_{Ar}), 8.47 (d, *J* = 1.4 Hz, 1H, H_{Ar}). |
| | | | **¹³C NMR (100 MHz, DMSO-*d*₆) δ :** 19.4 (2xCH_{3 iPr}), 20.6 (CH₃), 24.6 (CH_{iPr}), 44.1 (CH_{2 Bn}), 45.2 (CH_{2 Bn}), 83.8 (CH_{Ar}), 126.8 (2xCH_{Ar}), 126.9 (CH_{Ar}), 128.4 (2xCH_{Ar}), 138.2 (C_{q}), 139.6 (C_{q}), 140.1 (C_{q}), 142.4 (CH_{Ar}), 143.2 (CH_{Ar}), 151.3 (C_{q}), 151.4 (C_{q}), 152.8 (C_{q}), 155.4 (C_{q}). |
| | | | **MS (ESI+) : m/z calcd for C₂₁H₂₄N₈** : 389.2 [M+H]+, **found** : 389.2. LogD (HPLC): 2.30 |
| (140) | 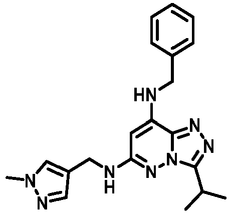 | > 99 %^{b} | Pale yellow solid. |
| | | | **¹H NMR (400 MHz, DMSO-*d*₆) δ** : 1.37 (d, *J* = 6.9 Hz, 6H, 2xCH_{3 iPr}), 3.37 (p, *J =* 6.9 Hz, 1H, H_{iPr}), 3.76 (s, 3H, CH_{3-N}), 4.14 (d, *J* = 5.3 Hz, 2H, CH₂), 4.41 (d, *J* = 6.3 Hz, 2H, CH_{2 Bn}), 5.42 (s, 1H, H_{Ar}), 6.75 (t, *J* = 5.3 Hz, 1H, NH), 7.23 (ddd, *J* = 8.6, 5.5, 2.5 Hz, 1H, H_{Ar}), 7.28 - 7.36 (m, 5H, 5xH_{Ar}), 7.57 (s, 1H, H_{Ar}), 7.91 (t, *J* = 6.4 Hz, 1H, NH_{Bn}). |
| | | | **¹³C NMR (101 MHz, DMSO-*d*₆) δ :** 19.7 (2xCH_{3 iPr}), 24.6 (CH_{iPr}), 35.1 (CH₂), 38.3 (CH_{3-N}), 45.2 (CH_{2 Bn}), 84.1 (CH_{Ar}), 118.7 (C_{q}), 126.8 (2xCH_{Ar}), 126.9 (CH_{Ar}), 128.4 (2xCH_{Ar}), 129.6 (CH_{Ar}), 138.2 (CH_{Ar}), 138.3 (C_{q}), 139.7 (C_{q}), 139.9 (C_{q}), 152.9 (C_{q}), 155.6 (C_{q}). |
| | | | **MS (ESI+) : m/z calcd for C₂₀H₂₄N₈** : 377.2 [M+H]+, **found** : 377.2. LogD (HPLC): 2.30 |
| (141) | 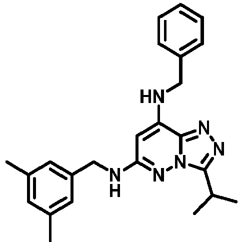 | > 97 %^{b} | White solid. |
| | | | **¹H NMR (400 MHz, DMSO-*d*₆) δ :** 1.33 (d, *J* = 7.0 Hz, 6H, 2xCH_{3 iPr}), 2.21 (s, 6H, 2xCH₃), 3.32 - 3.36 (m, 1H, H_{iPr}), 4.23 (d, *J* = 5.7 Hz, 2H, CH₂), 4.42 (d, *J* = 6.4 Hz, 2H, CH_{2 Bn}), 5.44 (s, 1H, H_{Ar}), 6.84 (s, 1H, H_{Ar}), 6.93 (s, 2H, 2xH_{Ar}), 7.02 (t, *J* = 5.7 Hz, 1H, NH), 7.20 - 7.27 (m, 1H, H_{Ar}), 7.29 - 7.37 (m, 4H, 4xH_{Ar}), 7.91 (t, *J* = 6.3 Hz, 1H, NH_{Bn}). |
| | | | **¹³C NMR (101 MHz, DMSO-*d*₆) δ :** N.D. |
| | | | **MS (ESI+) : m/z calcd for C₂₄H₂₈N₆** : 401.2 [M+H]+, **found** : 401.2. LogD (HPLC): 4.20 |
| (142) | 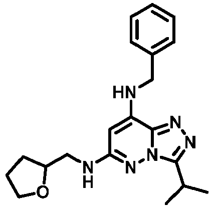 | > 97%^{b} | Colorless oil. |
| | | | **¹H NMR (400 MHz, DMSO-*d*₆) δ** : 1.35 (dd, *J* = 7.0, 1.2 Hz, 6H, 2xCH_{3 iPr}), 1.54 (dt, *J* = 11.2, 7.3 Hz, 1H, H_{(CH2)}), 1.84 (dq, *J* = 32.3, 7.3, 6.5 Hz, 3H, CH₂ & H_{(CH2)}), 3.15 - 3.23 (m, 2H, CH₂), 3.28 - 3.37 (m, 1H, H_{iPr}), 3.60 (q, *J* = 7.4 Hz, 1H, H_{(CH2)}), 3.71 - 3.80 (m, 1H, H_{(CH2)}), 3.97 (p, *J=* 6.5 Hz, 1H, H_{C-O}), 4.42 (d, *J* = 6.4 Hz, 2H, CH_{2 Bn}), 5.48 (s, 1H, H_{Ar}), 6.61 (t, *J* = 5.6 Hz, 1H, NH), 7.24 (t, *J* = 6.5 Hz, 1H, H_{Ar}), 7.29 - 7.38 (m, 4H, 4xH_{Ar}), 7.90 (t, *J* = 6.4 Hz, 1H, NH_{Bn}). |
| | | | **¹³C NMR (101 MHz, DMSO-*d*₆) δ :** 19.6 (2xCH_{3 iPr}), 24.6 (CH_{iPr}), 25.1 (CH₂), 28.8 (CH₂), 44.9 (CH₂), 45.2 (CH_{Bn}), 67.0 (CH₂), 76.7 (CH_{-O}), 84.1 (CH_{Ar}), 126.9 (3xCH_{Ar}), 128.4 (CH_{Ar}), 138.3 (C_{q}), 139.7 (C_{q}), 139.8 (C_{q}), 152.8 (C_{q}), 156.0 (C_{q}). |
| | | | **MS (ESI+): m/z calcd for C₂₀H₂₆N₆O** : 367.2 [M+H]+, **found** : 367.2. LogD (HPLC): 2.87 |
| (143) | 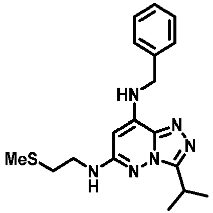 | > 96%^{b} | Colorless oil. |
| | | | **¹H NMR (400 MHz, DMSO-*d*₆) δ :** 1.36 (d, *J* = 6.9 Hz, 6H, 2xCH_{3 iPr}), 2.08 (s, 3H, CH_{3-S}), 2.64 (dd, *J* = 7.9, 6.3 Hz, 2H, CH₂), 3.28 - 3.38 (m, 3H, CH₂ & H_{iPr}), 4.44 (d, *J* = 6.3 Hz, 2H, CH_{2 Bn}), 5.38 (s, 1H, H_{Ar}), 6.73 (t, *J* = 5.7 Hz, 1H, NH), 7.24 (td, *J =* 5.9, 2.7 Hz, 1H, H_{Ar}), 7.28 - 7.37 (m, 4H, 4xH_{Ar}), 7.94 (t, *J=* 6.4 Hz, 1H, NH_{Bn}). |
| | | | **¹³C NMR (101 MHz, DMSO-*d*₆) δ :** 14.6 (CH_{3-S}), 19.6 (2xCH_{3 iPr}), 24.7 (CH_{iPr}), 32.1 (CH₂), 40.3 (CH₂), 45.2 (CH_{2 Bn}), 84.0 (CH_{Ar}), 126.8 (2xCH_{Ar}), 126.9 (CH_{Ar}), 128.4 (2xCH_{Ar}), 138.3 (C_{q}), 139.6 (C_{q}), 140.0 (C_{q}), 152.8 (C_{q}), 155.6 (C_{q}). |
| | | | **MS (ESI+): m/z calcd for C₁₈H₂₆N₆S** : 357.2 [M+H]+, **found** : 357.2. LogD (HPLC): 3.07 |
| (144) | 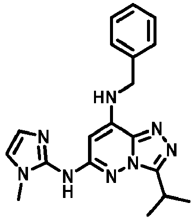 | > 95%^{b} | White solid. |
| | | | **¹H NMR (400 MHz, DMSO-*d*₆) δ :** 1.40 (d, *J* = 7.0 Hz, 6H, 2xCH_{3 iPr}), 2.86 (d, *J =* 4.9 Hz, 3H, CH_{3-N}), 3.55 (p, *J=* 7.0 Hz, 1H, H_{iPr}), 4.67 (s, 2H, CH_{2 Bn}), 6.21 (s, 1H, H_{Ar}), 6.55 (q, *J* = 5.2, 4.8 Hz, 1H, NH), 6.63 (d, *J* = 1.8 Hz, 1H, H_{Ar}), 7.20 (s, 1H, H_{Ar}), 7.24 (t, *J* = 7.2 Hz, 1H, H_{Ar}), 7.33 (t, *J=* 7.5 Hz, 2H, 2xH_{Ar}), 7.42 (d, *J=* 7.5 Hz, 2H, 2xH_{Ar}), 8.96 (s, 1H, NH_{Bn}). |
| | | | **¹³C NMR (101 MHz, DMSO-*d*₆) δ :** N.D. |
| | | | **MS (ESI+): m/z calcd for C₁₉H₂₂N₈** : 363.2 [M+H]+, **found** : 363.2. LogD (HPLC): 2.27 |
| (145) | 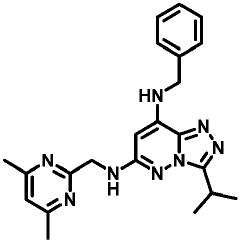 | > 95%^{b} | White solid. |
| | | | **¹H NMR (400 MHz, DMSO-*d*₆) δ :** 1.23 (d, *J=* 7.0 Hz, 6H), 2.36 (s, 6H), 3.21 (h, *J* = 7.0 Hz, 1H), 4.44 (m, 4H), 5.62 (s, 1H), 7.08 (m, 2H), 7.25 (m, 1H), 7.31 - 7.40 (m, 4H), 7.92 (t, *J =* 6.4 Hz, 1H). |
| | | | **¹³C NMR (101 MHz, DMSO-*d*₆) δ :** N.D. |
| | | | **MS (ESI+): m/z calcd for C₂₂H₂₆N₈** : 403.2 [M+H]+, **found** : 403.2. LogD (HPLC): 2.52 |
| (146) | 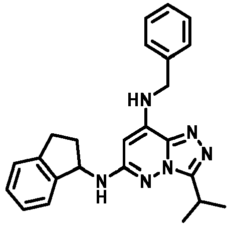 | > 99%^{b} | Colorless oil. |
| | | | **¹H NMR (400 MHz, DMSO-*d*₆) δ :** 1.36 (d, *J =* 7.0 Hz, 6H, 2xCH3 _{iPr}), 1.83 (dq, *J* = 12.6, 8.1 Hz, 1H, H_{(CH2)}), 2.42 - 2.49 (m, 1H, H_{(CH2)}), 2.81 (dt, *J* = 15.9, 8.0 Hz, 1H, H_{(CH2)}), 2.94 (ddd, *J* = 15.8, 8.7, 4.0 Hz, 1H, H_{(CH2)}), 3.36 (q, *J* = 6.9 Hz, 1H, H_{iPr}), 4.43 (d, *J* = 6.3 Hz, 2H, CH_{2 Bn}), 5.27 (q, *J* = 7.4 Hz, 1H, H_{indane}), 5.47 (s, 1H, H_{Ar}), 6.93 (d, *J* = 7.6 Hz, 1H, NH), 7.12 (t, *J* = 7.1 Hz, 1H, H_{Ar}), 7.16 - 7.21 (m, 1H, H_{Ar}), 7.21 - 7.26 (m, 2H, 2xH_{Ar}), 7.33 (h, *J* = 6.6 Hz, 5H, 5xH_{Ar}), 7.95 (t, *J* = 6.4 Hz, 1H, NH_{Bn}). |
| | | | **¹³C NMR (101 MHz, DMSO-*d*₆) δ** : N.D. |
| | | | **MS (ESI+): m/z calcd for C₂₄H₂₆N₆ :** 399.2 [M+H]+, **found** : 399.2. LogD (HPLC): 4.10 |
| (147) | 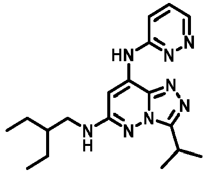 | - | Beige solid. |
| | | | **Rf** (DCM/MeOH, 94/6) : 0.31 |
| | | | **¹H NMR (400 MHz, DMSO-*d*₆) δ :** 0.90 (t, *J* = 7.4 Hz, 6H, 2xCH_{3-CH2}), 1.29 - 1.39 (m, 4H, 2xCH_{2-CH3}), 1.41 (d, *J* = 7.0 Hz, 6H, 2xCH_{3 iPr}), 1.59 (hept, *J* = 6.1 Hz, 1H, H_{c-CH2-NH}), 3.18 (t, *J* = 6.0 Hz, 2H, CH_{2-NH}), 3.40 (h, *J* = 7.0 Hz, 1H, H_{iPr}), 7.29 (t, *J* = 5.6 Hz, 1H, NH_{-CH2}), 7.62 (dd, *J* = 9.0, 4.5 Hz, 1H, H_{Ar}), 7.80 (dd, *J* = 9.0, 1.5 Hz, 1H, H_{Ar}), 7.97 (s, 1H, H_{Ar}), 8.89 (dd, *J* = 4.5, 1.4 Hz, 1H, H_{Ar}), 10.15 (s, 1H, NH). |
| | | | **¹³C NMR (101 MHz, DMSO-*d*₆) δ :** 10.9 (2xCH_{3-CH2}), 19.6 (2xCH_{3 iPr}), 23.6 (2xCH_{2- CH3}), 24.8 (CH_{iPr}), 39.60 (CH_{-CH2-NH}), 43.6 (CH_{2-NH}), 95.9 (CH_{Ar}), 118.7 (CH_{Ar}), 128.1 (CH_{Ar}), 132.9 (C_{q}), 139.3 (C_{q}), 146.8 (CH_{Ar}), 153.3 (C_{q}), 156.1 (C_{q}), 157.6 (C_{q}). |
| (148) | 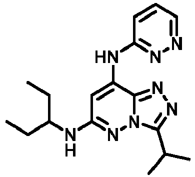 | > 97 %^{b} | White solide. |
| | | | **Rf** (DCM/MeOH, 95/5) : 0.52 |
| | | | **¹H NMR (400 MHz, DMSO-*d*₆) δ** : 0.90 (t, *J* = 7.3 Hz, 6H), 1.40 (d, *J* = 7.0 Hz, 6H), 1.44 - 1.66 (m, 4H), 3.39 (h, *J* = 7.1 Hz, 1H), 3.67 (h, *J* = 6.7 Hz, 1H), 7.07 (d, *J* = 7.9 Hz, 1H), 7.62 (dd, *J* = 9.0, 4.5 Hz, 1H), 7.79 (dd, *J* = 9.0, 1.4 Hz, 1H), 7.97 (s, 1H), 8.89 (dd, *J* = 4.5, 1.3 Hz, 1H), 10.14 (s, 1H). |
| | | | **¹³C NMR (101 MHz, DMSO-*d*₆) δ** : 10.44 (2xCH_{3 iPent}), 19.48 (2xCH_{3 iPr}), 24.86 (CH_{iPr}), 26.02 (2xCH_{2iPent}), 53.26 (CH_{iPent}), 96.06 (CH_{Ar}), 118.65 (CH_{Ar}), 128.07 (CH_{Ar}), 132.89 (C_{q}), 139.30 (C_{q}), 146.77 (CH_{Ar}), 153.15 (C_{q}), 155.89 (C_{q}), 157.61 (C_{q}). |
| | | | **MS (ESI+) : m/z calcd for C₁₇H₂₄N₈ :** 341.22 [M+H]+, **found** : 341.40. |
| (149) | 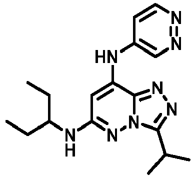 | > 97 %^{b} | Pale pink solid. |
| | | | **¹H NMR (400 MHz, DMSO-*d*₆) δ** : 0.90 (t, *J* = 7.3 Hz, 6H, 2xCH_{3 iPent}), 1.39 (d, *J* = 7.0 Hz, 6H, 2xCH_{3 iPr}), 1.55 (ddt, *J* = 45.4, 13.9, 6.9 Hz, 4H, 2xCH_{2 iPent}), 3.36 - 3.42 (m, 1H, H_{iPr}), 3.59 - 3.70 (m, 1H, H_{iPent}), 6.69 (s, 1H, H_{Ar}), 6.72 (d, *J* = 7.6 Hz, 1H, NH_{iPent}), 7.55 (dd, *J* = 6.1, 3.0 Hz, 1H, H_{Ar}), 9.01 (d, *J* = 6.0 Hz, 1H, H_{Ar}), 9.34 (d, *J* = 2.8 Hz, 1H, H_{Ar}), 10.07 (s, 1H, NH). |
| | | | **¹³C NMR (101 MHz, DMSO-*d*₆) δ :** 10.3 (2xCH_{3-CH2}), 19.4 (2xCH_{3 iPr}), 24.9 (CH_{iPr}), 26.0 (2xCH_{2-CH3}), 53.2 (CH_{iPent}), 93.7 (CH_{Ar}), 112.8 (CH_{Ar}), 134.0 (C_{q}), 139.3 (C_{q}), 139.8 (C_{q}), 144.3 (CH_{Ar}), 150.8 (CH_{Ar}), 153.3 (C_{q}), 155.2 (C_{q}). |
| | | | **MS (ESI+) : m/z calcd for C₁₇H₂₄N₈ :** 341.22 [M+H]+, **found** : 341.36. |
| (150) | 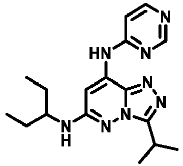 | > 95 %^{b} | Yellow solid. |
| | | | **Rf** (DCM/MeOH, 95/5) : 0.42 |
| | | | **¹H NMR (400 MHz, DMSO-*d*₆) δ :** 0.90 (t, *J* = 7.4 Hz, 6H, 2xCH_{3 iPent}), 1.40 (d, *J =* 7.0 Hz, 6H, 2xCH_{3 iPr}), 1.44 - 1.67 (m, 4H, 2xCH_{2 iPent}), 3.33 - 3.42 (m, 1H, H_{iPr}), 3.63 - 3.71 (m, 1H, H_{iPent}), 7.00 (d, *J* = 8.0 Hz, 1H, NH), 7.49 (d, *J* = 5.9, 1.3 Hz, 1H, H_{Ar}), 7.89 (s, 1H, H_{Ar}), 8.50 (d, *J* = 5.8 Hz, 1H, H_{Ar}), 8.86 (s, 1H, H_{Ar}), 10.41 (s, 1H, NH). |
| | | | **¹³C NMR (101 MHz, DMSO-*d*₆) δ** : 10.45 (2xCH_{3 iPent}), 19.46 (2xCH_{3 iPr}), 24.83 (CHiPr), 26.02 (2xCH_{2iPent}), 53.32 (CH_{iPent}), 97.64 (CH_{Ar}), 110.11 (CH_{Ar}), 132.53 (C_{q}), 139.13 (C_{q}), 153.18 (C_{q}), 155.51 (C_{q}), 156.15 (CH_{Ar}), 157.53 (CH_{Ar}), 160.17 (C_{q}). |
| (151) | 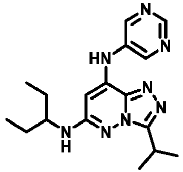 | > 97 %^{b} | White solid. |
| | | | **Rf** (DCM/MeOH, 95/5) : 0.32 |
| | | | **¹H NMR (400 MHz, DMSO-*d*₆) δ** : 0.88 (t, *J* = 7.4 Hz, 6H, 2xCH_{3 iPent}), 1.39 (d, *J* = 7.0 Hz, 6H, 2xCH_{3 iPr}), 1.42 - 1.49 (m, 2H, CH_{2 iPent}), 1.51 - 1.63 (m, 2H, CH_{2 iPent}), 3.33 - 3.41 (m, 1H, H_{iPr}), 3.62 (h, *J* = 6.7 Hz, 1H, H_{iPent}), 6.35 (s, 1H, H_{Ar}), 6.56 (d, *J* = 7.8 Hz, 1H, NH_{iPent}), 8.90 (s, 2H, 2xH_{Ar}), 8.94 (s, 1H, H_{Ar}), 9.62 (s, 1H, NH). |
| | | | **¹³C NMR (101 MHz, DMSO-*d*₆) δ** : 10.26 (2xCH_{3 iPent}), 19.46 (2xCH_{3 iPr}), 24.86 (CH_{iPr}), 26.01 (2xCH_{2iPent}), 53.04 (CH_{iPent}), 88.78 (CH_{Ar}), 135.45 (C_{q}), 135.93 (C_{q}), 139.36 (C_{q}), 149.74 (2xCH_{Ar}), 152.89 (CH_{Ar}), 153.13 (C_{q}), 155.60 (C_{q}). |
| | | | **MS (ESI+) : m/z calcd for C₁₇H₂₄N₈** : 341.22 [M+H]+, **found** : 341.36. |
| (152) | 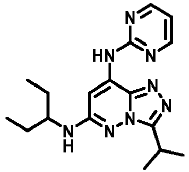 | > 98 %^{b} | Beige solid. |
| | | | **Rf** (DCM/MeOH, 95/5) : 0.35 |
| | | | **¹H NMR (400 MHz, DMSO-*d*₆) δ :** 0.90 (t, *J* = 7.4 Hz, 6H, 2xCH_{3 iPent}), 1.39 (d, *J* = 7.0 Hz, 6H, 2xCH_{3 iPr}), 1.45 - 1.66 (m, 4H, 2xCH_{2 iPent}), 3.32 - 3.42 (m, 1H, H_{iPr}), 3.60 - 3.73 (m, 1H, H_{iPent}), 7.00 (d, *J* = 8.0 Hz, 1H, NH_{iPent}), 7.16 (t, *J* = 4.8 Hz, 1H, H_{Ar}), 7.65 (s, 1H, H_{Ar}), 8.70 (d, *J* = 4.9 Hz, 2H, 2xH_{Ar}), 8.92 (s, 1H, NH). |
| | | | **¹³C NMR (101 MHz, DMSO-*d*₆) δ** : 10.44 (2xCH_{3 iPent}), 19.45 (2xCH_{3 iPr}), 24.84 (CH_{iPr}), 26.02 (2xCH_{2iPent}), 53.30 (CH_{iPent}), 96.05 (CH_{Ar}), 115.25 (CH_{Ar}), 132.40 (C_{q}), 139.05 (C_{q}), 153.26 (C_{q}), 155.58 (C_{q}), 158.47 (2xCH_{Ar}), 158.91 (C_{q}). |
| | | | **MS (ESI+) : m/z calcd for C₁₂H₂₄N₈ :** 341.22 [M+H]+, **found** : 341.40. |
| (153) | 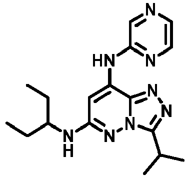 | > 98 %^{b} | White solid. |
| | | | **Rf** (DCM/MeOH, 95/5) : 0.27 |
| | | | **¹H NMR (400 MHz, DMSO-*d*₆) δ** : 0.89 (t, *J* = 7.3 Hz, 6H, 2xCH_{3 iPent}), 1.40 (d, *J* = 6.9 Hz, 6H, 2xCH_{3 iPr}), 1.45 - 1.67 (m, 4H, 2xCH_{2 iPent}), 3.33 - 3.43 (m, 1H, H_{iPr}), 3.66 (q, *J* = 6.3 Hz, 1H, H_{iPent}), 6.91 (d, *J* = 8.0 Hz, 1H, NH_{iPent}), 7.75 (s, 1H), 8.19 (d, *J =* 2.8 Hz, 1H, H_{Ar}), 8.32 (t, *J* = 2.1 Hz, 1H, H_{Ar}), 8.84 (d, *J* = 1.5 Hz, 1H, H_{Ar}), 10.37 (s, 1H, NH). |
| | | | **¹³C NMR (101 MHz, DMSO-*d*₆) δ :** 10.45 (2xCH_{3 iPent}), 19.47 (2xCH_{3 iPr}), 24.84 (CH_{iPr}), 26.06 (2xCH_{2iPent}), 53.27 (CH_{iPent}), 95.07 (CH_{Ar}), 133.08 (C_{q}), 136.31 (CH_{Ar}), 137.25 (CH_{Ar}), 139.24 (C_{q}), 140.77 (CH_{Ar}), 151.55 (C_{q}), 153.14 (C_{q}), 155.69 (C_{q}). |
| | | | **MS (ESI+) : m/z calcd for C₁₇H₂₄N₈** : 341.22 [M+H]+, **found** : 341.36. |
| (154) | 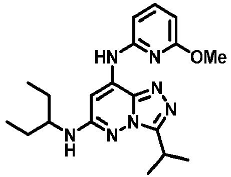 | > 96 %^{b} | White solid. |
| | | | Rf (DCM/MeOH, 96/4) : 0.28 |
| | | | **¹H NMR (400 MHz, DMSO-*d*₆) δ** : 0.89 (t, *J* = 7.4 Hz, 6H, 2xCH_{3-CH2}), 1.39 (d, *J* = 6.9 Hz, 6H, 2xCH_{3 iPr}), 1.45 - 1.66 (m, 4H, 2xCH_{2-CH3}), 3.36 - 3.41 (m, 1H, H_{iPr}), 3.66 (q, *J =* 6.3 Hz, 1H, H_{iPent}), 3.94 (s, 3H, CH_{3- O}), 6.37 - 6.48 (m, 1H, H_{Ar}), 6.64 (d, *J =* 8.1 Hz, 1H, NH_{iPent}), 6.92 - 7.09 (m, 1H, H_{Ar}), 7.45 (s, 1H, H_{Ar}), 7.63 (t, *J* = 7.9 Hz, 1H, H_{Ar}), 9.79 (s, 1H, NH). |
| | | | **¹³C NMR (101 MHz, DMSO-*d*₆) δ** : 10.6 (2xCH_{3-CH2}), 19.5 (2xCH_{3 iPr}), 24.9 (CH_{iPr}), 26.2 (2xCH_{2-CH3}), 53.5 (CH_{iPent}), 54.0 (CH_{3-O}), 93.6 (CH_{Ar}), 101.8 (CH_{Ar}), 105.3 (CH_{Ar}), 133.7 (C_{q}), 139.5 (C_{q}), 140.5 (CH_{Ar}), 152.9 (C_{q}), 153.1 (C_{q}), 155.9 (C_{q}), 162.6 (C_{q}). |
| | | | **MS (ESI+)** : **m/z calcd for C₁₉H₂₇N₇O** : 370.24 [M+H]+, **found** : 370.33. |
| (155) | 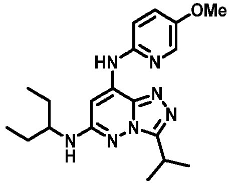 | > 96 %^{b} | White solid. |
| | | | **Rf** (DCM/MeOH, 96/4) : 0.47 |
| | | | **¹H NMR (400 MHz, DMSO-*d*₆) δ :** 0.89 (t, *J* = 7.4 Hz, 6H, 2xCH_{3-CH2}), 1.39 (d, *J* = 6.9 Hz, 6H, 2xCH_{3 iPr}), 1.44 - 1.65 (m, 4H, 2xCH_{2-CH3}), 3.28 - 3.41 (m, 1H, H_{iPr}), 3.65 (q, *J =* 6.5 Hz, 1H, HiPent), 3.81 (s, 3H, CH₃₋ o), 6.73 (d, *J* = 8.0 Hz, 1H, NH_{iPent}), 7.44 (s, 1H, H_{Ar}), 7.45 (s, 1H, H_{Ar}), 7.64 (s, 1H, H_{Ar}), 8.02 (t, *J* = 1.9 Hz, 1H, H_{Ar}), 9.72 (s, 1H, NH). |
| | | | **¹³C NMR (101 MHz, DMSO-*d*₆) δ** : 10.5 (2xCH_{3-CH2}), 19.5 (2xCH_{3 iPr}), 24.9 (CH_{iPr}), 26.2 (2xCH_{2-CH3}), 53.2 (CH_{iPent}), 55.8 (CH_{3-O}), 92.2 (CH_{Ar}), 114.7 (CH_{Ar}), 124.7 (CH_{Ar}), 132.2 (CH_{Ar}), 133.9 (C_{q}), 139.5 (C_{q}), 148.6 (C_{q}), 150.8 (C_{q}), 153.0 (C_{q}), 156.1 (C_{q}). |
| | | | **MS (ESI+) : m/z calcd for C₁₉H₂₇N₇O** : 370.24 [M+H]+, **found** : 370.33. |
| (156) | 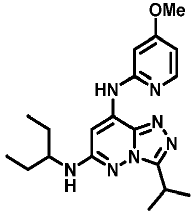 | > 97 %^{b} | White solid. |
| | | | **Rf** (DCM/MeOH, 96/4) : 0.47 |
| | | | **¹H NMR (400 MHz, DMSO-*d*₆) δ** : 0.89 (t, *J* = 7.3 Hz, 6H, 2xCH_{3-CH2}), 1.39 (d, *J* = 7.0 Hz, 6H, 2xCH_{3 iPr}), 1.54 (ddq, *J* = 28.3, 13.9, 7.0 Hz, 4H, 2xCH_{2-CH3}), 3.28 - 3.40 (m, 1H, H_{iPr}), 3.65 (hept, *J* = 5.9, 5.2 Hz, 1H, H_{iPent}), 3.81 (s, 3H, CH_{3-O}), 6.63 (dd, *J* = 6.1, 2.2 Hz, 1H, H_{Ar}), 6.80 (d, *J =* 8.0 Hz, 1H, NH_{iPent}), 7.13 (d, *J* = 2.3 Hz, 1H, H_{Ar}), 7.79 (s, 1H, H_{Ar}), 8.14 (d, *J* = 5.9 Hz, 1H, H_{Ar}), 9.68 (s, 1H, NH). |
| | | | **¹³C NMR (101 MHz, DMSO-*d*₆) δ :** 10.5 (2xCH_{3-CH2}), 19.5 (2xCH_{3 iPr}), 24.9 (CH_{iPr}), 26.1 (2xCH_{2-CH3}), 53.2 (CH_{iPent}), 55.2 (CH_{3-O}), 94.2 (CH_{Ar}), 97.7 (CH_{Ar}), 105.6 (CH_{Ar}), 133.7 (C_{q}), 139.5 (C_{q}), 148.1 (CH_{Ar}), 153.0 (C_{q}), 156.0 (C_{q}), 156.6 (C_{q}), 166.3 (C_{q}). |
| | | | **MS (ESI+) : m/z calcd for C₁₉H₂₇N₇O** : 370.24 [M+H]+, **found** : 370.33. |
| (157) | 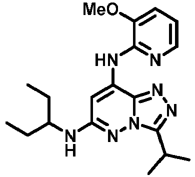 | - | White solid. |
| | | | **Rf** (DCM/MeOH, 96/4) : 0.45 |
| | | | **¹H NMR (400 MHz, DMSO-*d*₆) δ** : 0.89 (t, *J* = 7.4 Hz, 6H, 2xCH_{3-CH2}), 1.39 (d, *J* = 7.0 Hz, 6H, 2xCH_{3 iPr}), 1.45 - 1.67 (m, 4H, 2xCH_{2-CH3}), 3.35 - 3.40 (m, 1H, H_{iPr}), 3.66 (h, *J =* 6.4 Hz, 1H, H_{iPent}), 3.99 (s, 3H, CH_{3- O}), 6.97 (d, *J* = 8.0 Hz, 1H, NH_{iPent}), 7.06 (dd, *J* = 8.0, 5.0 Hz, 1H, H_{Ar}), 7.45 (dd, *J* = 8.1, 1.4 Hz, 1H, H_{Ar}), 7.77 (s, 1H, H_{Ar}), 7.94 (dd, *J* = 5.0, 1.4 Hz, 1H, H_{Ar}), 8.27 (s, 1H, NH). |
| | | | **¹³C NMR (101 MHz, DMSO-*d*₆) δ** : 10.5 (2xCH_{3-CH2}), 19.4 (2xCH_{3 iPr}), 24.9 (CH_{iPr}), 26.0 (2xCH_{2-CH3}), 53.3 (CH_{iPent}), 56.2 (CH_{3-O}), 94.0 (CH_{Ar}), 117.0 (CH_{Ar}), 117.6 (CH_{Ar}), 132.1 (Cq), 137.9 (CH_{Ar}), 139.2 (C_{q}), 143.2 (C_{q}), 144.0 (C_{q}), 153.3 (C_{q}), 155.8 (C_{q}). |
| (158) | 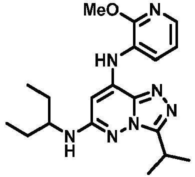 | - | Pale grey solid. |
| | | | **¹H NMR (400 MHz, DMSO) δ :** 8.52 (s, 1H), 8.06 (d, *J* = 4.7 Hz, 1H), 7.71 (d, *J* = 7.1 Hz, 1H), 7.09 (dd, *J* = 7.6, 5.0 Hz, 1H), 6.46 (d, *J* = 7.9 Hz, 1H), 5.71 (s, 1H), 3.93 (d, *J* = 0.8 Hz, 3H), 3.66 - 3.53 (m, 1H), 3.41 - 3.34 (m, 1H), 1.61 - 1.49 (m, 2H), 1.49 - 1.41 (m, 2H), 1.38 (d, *J* = 7.0 Hz, 6H), 0.85 (t, *J* = 7.3 Hz, 6H). |
| | | | **¹³C NMR (101 MHz, DMSO) δ** : 157.1, 155.8, 153.0, 142.6, 139.5, 136.8, 132.6, 122.6, 117.3, 88.1, 53.4, 53.0, 26.0, 24.9, 19.5, 10.3. |
| (159) | 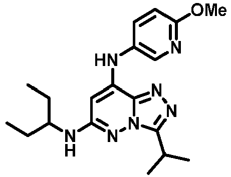 | - | Brown viscous oil. |
| | | | **¹H NMR (400 MHz, DMSO) δ** : 9.12 (s, 1H), 8.20 (d, *J* = 2.7 Hz, 1H), 7.73 (dd, *J* = 8.8, 2.7 Hz, 1H), 6.91 (d, *J* = 8.8 Hz, 1H), 6.43 (d, *J* = 7.9 Hz, 1H), 5.90 (s, 1H), 3.87 (s, 3H), 3.59 (p, *J* = 6.5 Hz, 1H), 3.39 - 3.34 (m, 1H), 1.60 - 1.48 (m, 2H), 1.48 - 1.40 (m, 2H), 1.38 (d, *J* = 7.0 Hz, 6H), 0.85 (t, *J* = 7.4 Hz, 6H). |
| | | | **¹³C NMR (101 MHz, DMSO**) **δ** : 160.6, 155.9, 152.9, 142.1, 139.6, 138.3, 135.8, 130.0, 110.8, 86.2, 53.3, 52.3, 26.1, 24.9, 19.5, 10.3. |
| (160) | 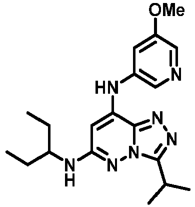 | - | Pale beige solid. |
| | | | **¹H NMR (400 MHz, DMSO) δ** : 9.41 (s, 1H), 8.30 (s, 1H), 8.08 (s, 1H), 7.41 (s, 1H), 6.56 (d, *J* = 7.8 Hz, 1H), 6.32 (s, 1H), 3.85 (s, 3H), 3.66 - 3.56 (m, 1H), 3.42 - 3.35 (m, 1H), 1.64 - 1.51 (m, 2H), 1.50 - 1.41 (m, 2H), 1.39 (d, *J* = 6.9 Hz, 6H), 0.87 (t, *J* = 7.4 Hz, 6H). |
| | | | **¹³C NMR (101 MHz, DMSO) δ** : 156.2, 156.1, 153.6, 140.0, 137.6, 137.0, 136.1, 132.3, 114.4, 88.6, 56.1, 53.5, 26.5, 25.3, 20.0, 10.8. |
| (161) | 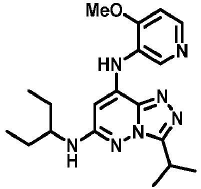 | - | Brown solid. |
| | | | **¹H NMR (400 MHz, DMSO) δ** : 0.84 (t, *J* = 7.4 Hz, 6H, 2xCH_{3-CH2}), 1.38 (d, *J* = 7.0 Hz, 6H, 2xCH_{3 iPr}), 1.40 - 1.58 (m, 4H, 2xCH_{2-CH3}), 3.36 - 3.40 (m, 1H, H_{iPr}), 3.57 (dt, *J* = 12.6, 6.8 Hz, 1H, H_{iPent}), 3.89 (s, 3H, CH_{3-O}), 5.53 (s, 1H, H_{Ar}), 6.41 (d, *J* = 8.0 Hz, 1H, NH_{iPent}), 7.23 (d, *J* = 5.7 Hz, 1H, H_{Ar}), 8.40 (d, *J* = 5.4 Hz, 1H, H_{Ar}), 8.41 (s, 1H, H_{Ar}), 8.67 (s, 1H, NH). |
| | | | **¹³C NMR (101 MHz, DMSO) δ** : N.D. |
| (162) | 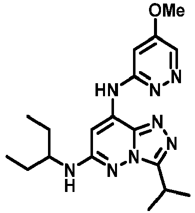 | > 97 %^{b} | Light yellow solid. |
| | | | **Rf** (DCM/MeOH, 94/6) : 0.30 |
| | | | ¹**H NMR (400 MHz, DMSO-*d*₆) δ :** 0.89 (t, *J =* 7.4 Hz, 6H, 2xCH_{3-CH2}), 1.40 (d, *J =* 7.0 Hz, 6H, 2xCH_{3 iPr}), 1.45 - 1.66 (m, 4H, 2xCH_{2-CH3}), 3.37 (p, *J* = 7.0 Hz, 1H, H_{iPr}), 3.67 (qt, *J* = 7.5, 5.3 Hz, 1H, H_{iPent}), 3.87 (s, 3H, CH_{3-O}), 7.06 (d, *J* = 7.9 Hz, 1H, NH_{iPent}), 7.43 (d, *J* = 2.6 Hz, 1H, H_{Ar}), 7.95 (s, 1H, H_{Ar}), 8.67 (d, *J* = 2.6 Hz, 1H, H_{Ar}), 9.94 (s, 1H, NH). |
| | | | **¹³C NMR (101 MHz, DMSO-*d*₆) δ** : 10.4 (2xCH_{3-CH2}), 19.5 (2xCH_{3 iPr}), 24.9 (CH_{iPr}), 26.0 (2xCH_{2-CH3}), 53.2 (CH_{iPent}), 55.4 (CH_{3-O}), 96.1 (CH_{Ar}), 99.1 (CH_{Ar}), 133.0 (C_{q}), 139.3 (C_{q}), 139.8 (CH_{Ar}), 153.1 (C_{q}), 155.9 (C_{q}), 158.1 (C_{q}), 158.3 (C_{q}). |
| | | | **MS (ESI+) : m/z calcd for C₁₈H₂₆N₈O :** 371.23 [M+H]+, **found** : 317.33. |
| (163) | 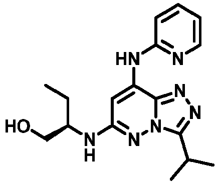 | > 98 %^{b} | White solid. |
| | | | **Rf** (DCM/MeOH, 94/6) : 0.23 |
| | | | **¹H NMR (400 MHz, DMSO-*d*₆) δ :** 0.91 (t, *J* = 7.4 Hz, 3H, CH_{3-CH2}), 1.40 (dd, *J* = 6.9, 1.5 Hz, 6H, 2xCH_{3 iPr}), 1.44 - 1.77 (m, 2H, CH_{2-CH3}), 3.37 (p, *J* = 7.2 Hz, 1H, H_{iPr}), 3.50 (dp, *J* = 19.4, 5.2 Hz, 2H, CH_{2-OH}), 3.73 (tq, *J* = 10.2, 5.6 Hz, 1H, H_{C-NH}), 4.62 (t, *J* = 5.5 Hz, 1H, OH), 6.79 (d, *J* = 7.8 Hz, 1H, NH_{-CH}), 6.99 (dd, *J* = 7.2, 5.0 Hz, 1H, H_{Ar}), 7.48 (d, *J* = 8.4 Hz, 1H, H_{Ar}), 7.72 (td, *J* = 7.8, 7.3, 2.0 Hz, 1H, H_{Ar}), 7.84 (s, 1H, H_{Ar}), 8.32 (dd, *J* = 5.0, 1.9 Hz, 1H, H_{Ar}), 9.87 (s, 1H, NH). |
| | | | **¹³C NMR (101 MHz, DMSO-*d*₆) δ** : 10.6 (CH_{3-CH2}), 19.6 (2xCH_{3 iPr}), 23.5 (CH_{2-CH3}), 24.8 (CH_{iPr}), 54.1 (CH_{-NH}), 62.0 (CH_{2-OH}), 94.2 (CH_{Ar}), 113.7 (CH_{Ar}), 117.0 (CH_{Ar}), 133.6 (C_{q}), 137.7 (CH_{Ar}), 139.4 (C_{q}), 146.9 (CH_{Ar}), 153.1 (C_{q}), 154.9 (C_{q}), 155.9 (C_{q}). |
| | | | **MS (ESI+) : m/z calcd for C₁₇H₂₃N₇O** : 342.20 [M+H]+, **found** : 342.42. |
| (164) | 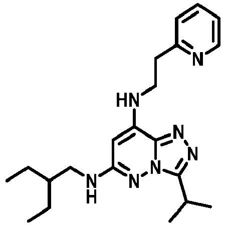 | 94 %^{b} | Light brown solid. |
| | | | **Rf** (DCM/MeOH, 94/6) : 0.19 |
| | | | **¹H NMR (400 MHz, DMSO-*d*₆) δ :** 0.88 (t, *J* = 7.4 Hz, 6H, 2xCH_{3-CH2}), 1.29 - 1.39 (m, 10H, 2xCH_{3 iPr} & 2xCH_{2-CH3}), 1.52 (p, *J* = 6.2 Hz, 1H, H_{C-CH2-NH}), 3.09 (t, *J* = 7.3 Hz, 2H, CH₂), 3.13 (t, *J* = 5.9 Hz, 2H, CH₂), 3.27 - 3.37 (m, 1H, H_{iPr}), 3.55 (q, *J* = 6.8 Hz, 2H, CH₂), 5.60 (s, 1H, H_{Ar}), 6.55 (t, *J* = 5.6 Hz, 1H, NH), 7.18 - 7.29 (m, 2H, NH & H_{Ar}), 7.33 (d, *J* = 7.8 Hz, 1H, H_{Ar}), 7.72 (td, *J* = 7.7, 1.9 Hz, 1H, H_{Ar}), 8.52 (dd, *J* = 5.0, 1.8 Hz, 1H, H_{Ar}). |
| | | | **¹³C NMR (101 MHz, DMSO-*d*₆) δ** : 10.9 (2xCH_{3-CH2}), 19.6 (2xCH_{3 iPr}), 23.5 (2xCH_{2- CH3}), 24.7 (CH_{iPr}), 35.9 (CH₂), 39.9 (CH_{- CH2-NH}), 41.7 (CH₂), 43.3 (CH₂), 83.5 (CH_{Ar}), 121.6 (CH_{Ar}), 123.3 (CH_{Ar}), 136.5 (CH_{Ar}), 139.7 (C_{q}), 139.8 (C_{q}), 149.1 (CH_{Ar}), 152.8 (C_{q}), 156.3 (C_{q}), 158.9 (C_{q}). |
| | | | **MS (ESI+) : m/z calcd for C₂₁H₃₁N₇** : 382.27 [M+H]+, **found** : 382.45. |
| (165) | 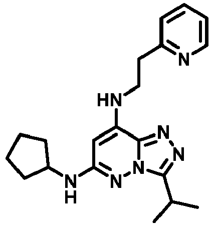 | > 95 %^{b} | Light brown solid. |
| | | | **¹H NMR (400 MHz, DMSO-*d*₆) δ :** 1.36 (dd, *J* = 7.0, 1.3 Hz, 6H, 2xCH_{3 iPr}), 1.42-1.59 (m, 4H, 2xCH₂), 1.67 (dd, *J* = 9.2, 5.3 Hz, 2H, CH₂), 1.94 (dt, *J* = 12.9, 6.4 Hz, 2H, CH₂), 3.08 (t, *J* = 7.4 Hz, 2H, CH₂), 3.28 - 3.38 (m, 1H, H_{iPr}), 3.54 (q, *J* = 6.8 Hz, 2H, CH₂), 3.99 (p, *J* = 6.3 Hz, 1H, H_{cPent}), 5.56 (s, 1H, H_{Ar}), 6.58 (d, *J* = 6.1 Hz, 1H, NH_{cPent}), 7.17 - 7.30 (m, 2H, NH_{-CH2} & H_{Ar}), 7.33 (d, *J* = 7.7 Hz, 1H, H_{Ar}), 7.72 (tt, *J* = 7.6, 1.6 Hz, 1H, H_{Ar}), 8.51 (dd, *J* = 4.7, 1.5 Hz, 1H, H_{Ar}). |
| | | | **¹³C NMR (101 MHz, DMSO-*d*₆) δ :** 19.6 (2xCH_{3 iPr}), 23.6 (2xCH₂), 24.8 (CH_{iPr}), 32.4 (2xCH₂), 35.8 (CH_{2-CH2-NH}), 41.7 (CH_{2-NH}), 52.3 (CH_{cPent}), 83.6 (CH_{Ar}), 121.6 (CH_{Ar}), 123.3 (CH_{Ar}), 136.5 (CH_{Ar}), 139.6 (C_{q}), 139.8 (C_{q}), 149.1 (CH_{Ar}), 152.8 (C_{q}), 155.7 (C_{q}), 158.9 (C_{q}). |
| | | | **MS (ESI+) : m/z calcd for C₂₀H₂₇N₇ :** 366.24 [M+H]+, **found :** 366.42. |
| (166) | 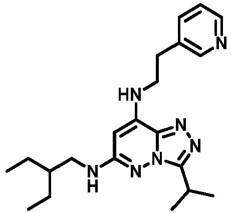 | > 96 %^{b} | Light brown solid. |
| | | | **Rf** (DCM/MeOH, 94/6) : 0.17 |
| | | | **¹H NMR (400 MHz, DMSO-*d*₆) δ :** 0.89 (t, *J* = 7.4 Hz, 6H, 2xCH_{3-CH2}), 1.34 (dd, *J* = 15.9, 6.9 Hz, 10H, 2xCH_{3 iPr} & 2xCH_{2-CH3}), 1.52 (hept, *J* = 6.3 Hz, 1H, H_{C-CH2-NH}), 2.94 (t, *J* = 7.3 Hz, 2H, CH₂), 3.14 (t, *J* = 5.9 Hz, 2H, CH₂), 3.27 - 3.39 (m, 1H, H_{iPr}), 3.43 (q, *J* = 6.8 Hz, 2H, CH₂), 5.61 (s, 1H, H_{Ar}), 6.52 (t, *J* = 5.8 Hz, 1H, NH), 7.24 - 7.39 (m, 2H, NH & H_{Ar}), 7.71 (dt, *J* = 7.9, 2.0 Hz, 1H, H_{Ar}), 8.43 (dd, *J* = 4.8, 1.6 Hz, 1H, H_{Ar}), 8.52 (d, *J* = 2.2 Hz, 1H, H_{Ar}). |
| | | | **¹³C NMR (101 MHz, DMSO-*d*₆) δ :** 10.9 (2xCH_{3-CH2}), 19.6 (2×CH_{3 iPr}), 23.5 (2xCH_{2-CH3}), 24.7 (CH_{iPr}), 30.8 (CH₂), 39.9 (CH_{-CH2-NH}), 43.1 (CH₂), 43.3 (CH₂), 83.7 (CH_{Ar}), 123.4 (CH_{Ar}), 134.7 (C_{q}), 136.3 (CH_{Ar}), 139.6 (C_{q}), 139.7 (C_{q}), 147.5 (CH_{Ar}), 149.9 (CH_{Ar}), 152.8 (C_{q}), 156.3 (C_{q}). |
| | | | **MS (ESI+) : m/z calcd for C₂₁H₃₁N₇ :** 382.27 [M+H]+, **found :** 380.40. |
| (167) | 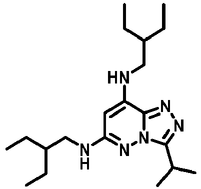 | 95 %^{b} | Light brown solid. |
| | | | **¹H NMR (400 MHz, DMSO-*d*₆) δ :** 0.88 (t, *J* = 7.5 Hz, 6H, 2xCH_{3-CH2}), 0.89 (t, *J* = 7.4 Hz, 6H, 2xCH_{3-CH2}), 1.35 (dd, *J* = 14.2, 7.0 Hz, 14H, 2xCH_{3 iPr} & 4xCH_{2-CH3}), 1.52 (dq, *J* = 11.4, 5.6, 5.1 Hz, 1H, H_{C-CH2-NH}), 1.68 (hept, *J* = 6.6 Hz, 1H, H_{C-CH2-NH}), 3.07 (t, *J* = 6.5 Hz, 2H, CH_{2-NH}), 3.13 (t, *J* = 6.0 Hz, 2H, CH_{2-NH}), 3.28 - 3.40 (m, 1H, H_{iPr}), 5.50 (s, 1H, H_{Ar}), 6.54 (t, *J* = 5.7 Hz, 1H, NH), 7.18 (t, *J* = 5.8 Hz, 1H, NH). |
| | | | **¹³C NMR (101 MHz, DMSO-*d*₆) δ :** 10.5 (2xCH_{3-CH2}), 10.9 (2xCH_{3CH2}), 19.6 (2xCH_{3iPr}), 23.1 (2×CH_{2-CH3}), 23.5 (2xCH_{2-CH3}), 24.7 (CH_{iPr}), 38.3 (CH_{-CH2}), 39.9 (CH_{-CH2}), 43.3 (CH_{2-NH}), 45.3 (CH_{2-NH}), 83.1 (CH_{Ar}), 139.7 (C_{q}), 140.1 (C_{q}), 152.8 (C_{q}), 156.3 (C_{q}). |
| | | | **MS (ESI+) : m/z calcd for C₂₀H₃₆N₆ :** 361.31 [M+H]+, **found :** 361.50. |
| (168) | 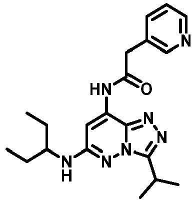 | > 96 %^{b} | White solid. |
| | | | **¹H NMR (400 MHz, DMSO-*d*₆) δ :** 0.86 (t, *J* = 7.3 Hz, 6H, 2xCH_{3-CH2}), 1.38 (d, *J* = 6.9 Hz, 6H, 2xCH_{3 iPr}), 1.52 (dtt, *J* = 38.8, 14.0, 6.6 Hz, 4H, 2xCH_{2-CH3}), 3.29 - 3.41 (m, 1H, H_{iPr}), 3.57 - 3.69 (m, 1H, H_{iPent}), 3.98 (s, 2H, CH_{2-CO}), 6.98 (d, *J* = 7.9 Hz, 1H, NH_{iPent}), 7.36 (dd, *J* = 7.8, 4.8 Hz, 1H, H_{Ar}), 7.58 (s, 1H, H_{Ar}), 7.76 (dt, *J* = 7.9, 2.0 Hz, 1H, H_{Ar}), 8.47 (dd, *J* = 4.8, 1.7 Hz, 1H, H_{Ar}), 8.55 (d, *J* = 2.2 Hz, 1H, H_{Ar}), 11.01 (s, 1H, NH_co). |
| | | | **¹³C NMR (101 MHz, DMSO-*d*₆) δ :** 10.3 (2xCH_{3-CH2}), 19.4 (2×CH_{3 iPr}), 24.8 (CH_{iPr}), 25.9 (2xCH_{2-CH3}), 39.8 (CH_{2-CO}), 53.3 (CH_{iPent}), 99.6 (CH_{Ar}), 123.4 (CH_{Ar}), 131.2 (C_{q}), 131.8 (C_{q}), 136.8 (CH_{Ar}), 138.5 (C_{q}), 147.9 (CH_{Ar}), 150.3 (CH_{Ar}), 153.2 (C_{q}), 155.2 (C_{q}), 171.4 (C_{q}). |
| | | | **MS (ESI+) : m/z calcd for C₂₀H₂₇N₇O :** 382.23 [M+H]+, **found :** 382.44. |
| (169) | 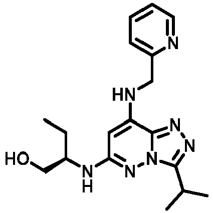 | > 96 %^{b} | White solid. |
| | | | **Rf** (DCM/MeOH, 92/8) : 0.29 |
| | | | **¹H NMR (400 MHz, DMSO-*d*₆) δ :** 0.85 (t, *J* = 7.4 Hz, 3H, CH_{3-CH2}), 1.36 (dd, *J* = 6.9, 1.2 Hz, 6H, 2xCH_{3 iPr}), 1.38 - 1.67 (m, 2H, CH_{2-CH3}), 3.24 - 3.37 (m, 1H, H_{iPr}), 3.41 (dq, *J* = 22.0, 5.2 Hz, 2H, CH_{2-OH}), 3.62 (h, *J* = 5.9 Hz, 1H, H_{c-NH}), 4.52 (d, *J* = 6.2 Hz, 2H, CH_{2Bn}), 4.55 (t, *J* = 5.4 Hz, 1H, OH), 5.46 (s, 1H, H_{Ar}), 6.33 (d, *J* = 7.7 Hz, 1H, NH_{-CH}), 7.28 (ddd, *J* = 7.5, 4.9, 1.2 Hz, 1H, H_{Ar}), 7.33 (dt, *J* = 7.9, 1.1 Hz, 1H, H_{Ar}), 7.75 (td, *J* = 7.8, 1.9 Hz, 1H, H_{Ar}), 7.80 (t, *J* = 6.5 Hz, 1H, NH_{Bn}), 8.55 (ddd, *J* = 4.8, 1.8, 0.9 Hz, 1H, H_{Ar}). |
| | | | **¹³C NMR (101 MHz, DMSO-*d*₆) δ :** 10.5 (CH_{3-CH2}), 19.6 (2xCH_{3 iPr}), 23.5 (CH_{2-CH3}), 24.7 (CH_{iPr}), 47.2 (CH_{2 Bn}), 53.8 (CH_{-NH}), 62.0 (CH_{2-OH}), 84.6 (CH_{Ar}), 120.7 (CH_{Ar}), 122.3 (CH_{Ar}), 137.0 (CH_{Ar}), 139.6 (C_{q}), 139.8 (C_{q}), 149.0 (CH_{Ar}), 152.8 (C_{q}), 155.8 (C_{q}), 157.7 (C_{q}). |
| | | | **MS (ESI+) : m/z calcd for C₁₈H₂₅N₇O :** 356.22 [M+H]+, **found :** 356.41. |
| (170) | 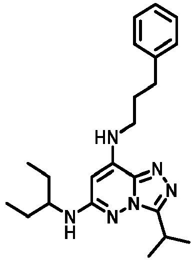 | 84 %^{b} | Brown oil. |
| | | | **¹H NMR (400 MHz, DMSO-*d*₆) δ :** 0.87 (t, *J* = 7.2 Hz, 6H, 2xCH_{3-CH2}), 1.36 (d, *J* = 6.9 Hz, 6H, 2xCH_{3 iPr}), 1.51 (ddq, *J* = 40.2, 13.9, 7.3 Hz, 4H, 2xCH_{2-CH3}), 1.93 (p, *J* = 7.3 Hz, 2H, CH_{2-CH2-NH}), 2.67 (t, *J* = 7.8 Hz, 2H, CH_{2-Ph}), 3.18 (q, *J* = 6.6 Hz, 2H, CH_{2-NH}), 3.30 (d, *J* = 7.6 Hz, 1H, H_{iPr}), 3.60 (h, *J* = 7.0 Hz, 1H, H_{iPent}), 5.50 (s, 1H, H_{Ar}), 6.28 (d, *J* = 8.0 Hz, 1H, NH_{iPent}), 7.23 (td, *J* = 18.1, 17.7, 7.2 Hz, 6H, NH_{Bn} & 5xH_{Ar}). |
| | | | **¹³C NMR (101 MHz, DMSO-*d*₆) δ :** N.D. |
| | | | **MS (ESI+) : m/z calcd for C₂₂H₂₁N₆ :** 381.28 [M+H]+, **found :** 381.47. |
| (171) | 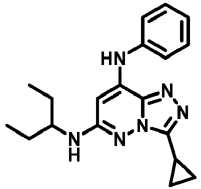 | > 96 %^{b} | White solid. |
| | | | **¹H NMR (400 MHz, DMSO-*d*₆) δ :** 0.86 (t, *J* = 7.3 Hz, 6H, 2xCH_{3-CH2}), 1.02 - 1.19 (m, 4H, 2xCH_{2 cPr}), 1.38 - 1.63 (m, 4H, 2xCH_{2-CH3}), 2.24 - 2.33 (m, 1H, H_{cPr}), 3.61 (h, *J* = 6.4 Hz, 1H, H_{iPent}), 6.20 (s, 1H, H_{Ar}), 6.49 (d, *J* = 7.9 Hz, 1H, NH_{iPent}), 7.13 (ddd, *J* = 8.6, 6.1, 2.2 Hz, 1H, H_{Ar}), 7.30 - 7.48 (m, 4H, 4xH_{Ar}), 9.19 (s, 1H, NH). |
| | | | **¹³C NMR (101 MHz, DMSO-*d*₆) δ :** 5.4 (CH_{cPr}), 6.4 (2xCH_{2 cPr}), 10.3 (2xCH_{3-CH2}), 26.1 (2xCH_{2-CH3}), 52.9 (CH_{iPent}), 86.8 (CH_{Ar}), 122.3 (2xCH_{Ar}), 123.7 (CH_{Ar}), 129.2 (2xCH_{Ar}), 137.0 (C_{q}), 139.5 (C_{q}), 139.6 (C_{q}), 150.2 (C_{q}), 156.1 (C_{q}). |
| | | | **MS (ESI+) : m/z calcd for C₁₉H₂₄N₆ :** 337.21 [M+H]+, **found :** 337.41. |
| (172) | 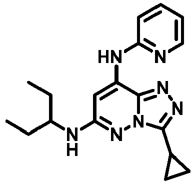 | > 96 %^{b} | White solid. |
| | | | **¹H NMR (400 MHz, DMSO-*d*₆) δ :** 0.91 (t, *J* = 7.4 Hz, 6H, 2xCH_{3-CH2}), 1.02 - 1.23 (m, 4H, 2xCH_{2 cPr}), 1.41 - 1.68 (m, 4H, 2xCH_{2-CH3}), 2.30 (ddd, *J* = 13.6, 8.4, 5.0 Hz, 1H, H_{cPr}), 3.60 - 3.75 (m, 1H, H_{iPent}), 6.82 (d, *J* = 8.1 Hz, 1H, NH_{iPent}), 7.00 (ddd, *J* = 7.2, 5.0, 1.0 Hz, 1H, H_{Ar}), 7.48 (dt, *J* = 8.5, 1.0 Hz, 1H, H_{Ar}), 7.72 (ddd, *J* = 9.0, 7.3, 2.0 Hz, 1H, H_{Ar}), 7.81 (s, 1H, H_{Ar}), 8.28 - 8.40 (m, 1H, H_{Ar}), 9.83 (s, 1H, NH). |
| | | | **¹³C NMR (101 MHz, DMSO-*d*₆) δ :** 5.4 (CH_{cPr}), 6.4 (2xCH_{2 cPr}), 10.4 (CH_{3-CH2}), 26.1 (2xCH_{2-CH3}), 53.1 (CH_{iPent}), 94.1 (CH_{Ar}), 113.7 (CH_{Ar}), 117.0 (CH_{Ar}), 133.5 (C_{q}), 137.7 (CH_{Ar}), 139.3 (C_{q}), 146.9 (CH_{Ar}), 150.2 (C_{q}), 154.9 (C_{q}), 156.2 (C_{q}). |
| | | | **MS (ESI+) : m/z calcd for C₁₈H₂₃N₇ :** 338.21 [M+H]+, **found :** 338.37. |
| (173) | 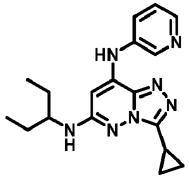 | 93 %^{b} | White solid. |
| | | | **¹H NMR (400 MHz, DMSO-*d*₆) δ :** 0.87 (t, *J* = 7.4 Hz, 6H, 2xCH_{3-CH2}), 1.03 - 1.19 (m, 4H, 2xCH_{2 cPr}), 1.39 - 1.62 (m, 4H, 2xCH_{2-CH3}), 2.29 (tt, *J* = 8.5, 5.1 Hz, 1H, H_{cPr}), 3.54 - 3.69 (m, 1H, H_{iPent}), 6.23 (s, 1H, H_{Ar}), 6.53 (d, *J* = 7.9 Hz, 1H, NH_{iPent}), 7.44 (dd, *J* = 8.2, 4.7 Hz, 1H, H_{Ar}), 7.80 (ddd, *J* = 8.3, 2.7, 1.5 Hz, 1H, H_{Ar}), 8.34 (dd, *J* = 4.7, 1.5 Hz, 1H, H_{Ar}), 8.66 (d, *J* = 2.6 Hz, 1H, H_{Ar}), 9.41 (s, 1H, NH). |
| | | | **¹³C NMR (101 MHz**, **DMSO-*d*₆) δ** : 5.4 (CH_{cPr}), 6.4 (2xCH_{2 cPr}), 10.2 (2xCH_{3-CH2}), 26.0 (2xCH_{2-CH3}), 52.8 (CH_{iPent}), 87.6 (CH_{Ar}), 123.9 (CH_{Ar}), 129.1 (CH_{Ar}), 136.4 (C_{q}), 136.6 (C_{q}), 139.4 (C_{q}), 143.8 (CH_{Ar}), 144.4 (CH_{Ar}), 150.3 (C_{q}), 156.0 (C_{q}). |
| | | | **MS (ESI+) : m/z calcd for C₁₈H₂₃N₇ :** 338.21 [M+H]+, **found :** 338.37. |
| (174) | 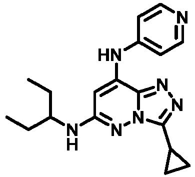 | > 97 %^{b} | White solid. |
| | | | **¹H NMR (400 MHz, DMSO-*d*₆) δ :** 0.90 (t, *J* = 7.4 Hz, 6H, 2xCH_{3-CH2}), 1.03 - 1.20 (m, 4H, 2xCH_{2 cPr}), 1.40 - 1.69 (m, 4H, 2xCH_{2-CH3}), 2.29 (tt, *J* = 8.5, 5.0 Hz, 1H, H_{cPr}), 3.65 (h, *J* = 6.1, 5.7 Hz, 1H, H_{iPent}), 6.60 (s, 1H, H_{Ar}), 6.68 (d, *J* = 8.0 Hz, 1H, NH_{iPent}), 7.29 - 7.40 (m, 2H, 2xH_{Ar}), 8.35 - 8.48 (m, 2H, 2xH_{Ar}), 9.71 (s, 1H, NH). |
| | | | **¹³C NMR (101 MHz, DMSO-*d*₆) δ :** 5.4 (CH_{cPr}), 6.5 (2xCH_{2 cPr}), 10.3 (2xCH_{3-CH2}), 26.0 (2xCH_{2-CH3}), 53.0 (CH_{iPent}), 91.7 (CH_{Ar}), 113.8 (2xCH_{Ar}), 134.6 (C_{q}), 139.3 (C_{q}), 147.2 (C_{q}), 150.4 (CH_{Ar}), 150.4 (C_{q}), 155.7 (C_{q}). |
| | | | **MS (ESI+) : m/z calcd for C₁₈H₂₃N₇ :** 338.21 [M+H]+, **found :** 338.37. |
| (175) | 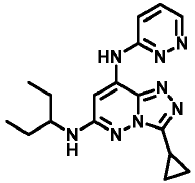 | > 98 %^{b} | White solid. |
| | | | **Rf** (DCM/MeOH, 95/5) : 0.38 |
| | | | **¹H NMR (400 MHz, DMSO-*d*₆) δ :** 0.90 (t, *J* = 7.4 Hz, 6H, 2xCH_{3-CH2}), 1.03 - 1.20 (m, 4H, 2xCH_{2 cPr}), 1.43 - 1.65 (m, 4H, 2xCH_{2-CH3}), 2.30 (td, *J* = 8.6, 4.3 Hz, 1H, H_{cPr}), 3.62 - 3.72 (m, 1H, H_{iPent}), 7.06 (d, *J* = 8.0 Hz, 1H, NH_{iPent}), 7.61 (dd, *J* = 9.0, 4.5 Hz, 1H, H_{Ar}), 7.78 (d, *J* = 9.1, 1.3 Hz, 1H, H_{Ar}), 7.95 (s, 1H, H_{Ar}), 8.89 (d, *J* = 4.5, 1.2 Hz, 1H, H_{Ar}), 10.10 (s, 1H, NH). |
| | | | **¹³C NMR (101 MHz, DMSO-*d*₆) δ :** 5.40 (CH_{cPr}), 6.47 (2xCH_{2 cPr}), 10.40 (2xCH_{3-CH2}), 25.99 (2xCH_{2-CH3}), 53.12 (CH_{iPent}), 96.09 (CH_{Ar}), 118.64 (CH_{Ar}), 128.08 (CH_{Ar}), 132.81 (C_{q}), 139.16 (C_{q}), 146.78 (CH_{Ar}), 150.34 (C_{q}), 156.11 (C_{q}), 157.60 (C_{q}). |
| | | | **MS (ESI+) : m/z calcd for C₁₇H₂₂N₈ :** 339.20 [M+H]+, **found :** 339.38. |
| (176) | 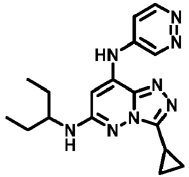 | > 97 %^{b} | White solid. |
| | | | **Rf** (DCM/MeOH, 94/6) : 0.11 |
| | | | **¹H NMR (400 MHz, DMSO-*d*₆) δ :** 0.90 (t, *J* = 7.4 Hz, 6H, 2xCH_{3-CH2}), 1.12 (ddt, *J* = 32.5, 7.3, 2.8 Hz, 4H, 2xCH_{2 cPr}), 1.55 (ddt, *J* = 47.4, 13.9, 6.8 Hz, 4H, 2xCH_{2-CH3}), 2.30 (dd, *J* = 9.7, 4.7 Hz, 1H, H_{cPr}), 3.66 (dq, *J* = 12.1, 6.2, 5.3 Hz, 1H, H_{iPent}), 6.68 (s, 1H, H_{Ar}), 6.72 (d, *J* = 7.9 Hz, 1H, NH_{iPent}), 7.54 (dd, *J* = 6.0, 3.0 Hz, 1H, H_{Ar}), 9.00 (d, *J* = 6.0 Hz, 1H, H_{Ar}), 9.33 (d, *J* = 2.9 Hz, 1H, H_{Ar}), 10.04 (s, 1H, NH). |
| | | | **¹³C NMR (101 MHz, DMSO-*d*₆) δ :** 5.3 (CH_{cPr}), 6.5 (2×CH_{2 cPr}), 10.2 (2xCH_{3-CH2}), 25.9 (2xCH_{2-CH3}), 53.0 (CH_{iPent}), 93.7 (CH_{Ar}), 112.8 (CH_{Ar}), 133.9 (C_{q}), 139.1 (C_{q}), 139.8 (C_{q}), 144.3 (CH_{Ar}), 150.5 (C_{q}), 150.8 (CH_{Ar}), 155.4 (C_{q}). |
| (177) | 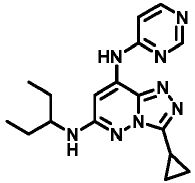 | > 97 %^{b} | Beige solid. |
| | | | Rf (DCM/MeOH, 95/5) : 0.25 |
| | | | **¹H NMR (400 MHz, DMSO-*d*₆) δ :** 0.90 (t, *J =* 7.4 Hz, 6H, 2xCH_{3-CH2}), 1.05 - 1.20 (m, 4H, 2xCH_{2 cPr}), 1.55 (ddt, *J* = 47.4, 13.9, 6.8 Hz, 4H, 2xCH_{2-CH3}), 2.26 - 2.33 (m, 1H, H_{cPr}), 3.66 (h, *J=* 8.1, 7.6 Hz, 1H, H_{iPent}), 6.68 (s, 1H, H_{Ar}), 6.72 (d, *J* = 8.0 Hz, 1H, NH_{iPent}), 7.54 (dd, *J* = 6.0, 3.0 Hz, 1H, H_{Ar}), 9.00 (d, *J=* 6.0 Hz, 1H, H_{Ar}), 9.33 (d, *J* = 2.9 Hz, 1H, H_{Ar}), 10.04 (s, 1H, NH_{Pyrimid}). |
| | | | **¹³C NMR (101 MHz, DMSO-*d*₆) δ :** 5.3 (CH_{cPr}), 6.5 (2xCH_{2 cPr}), 10.4 (2xCH_{3-CH2}), 26.0 (2xCH_{2-CH3}), 53.2 (CH_{iPent}), 97.7 (CH_{Ar}), 110.1 (CH_{Ar}), 132.5 (C_{q}), 139.0 (C_{q}), 150.4 (C_{q}), 155.7 (C_{q}), 156.2 (CH_{Ar}), 157.5 (CH_{Ar}), 160.2 (C_{q}). |
| | | | **MS (ESI+) : m/z calcd for C₁₇H₂₂N₈ :** 339.20 [M+H]+, **found :** 339.36. |
| (178) | 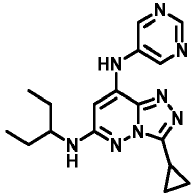 | > 97 %^{b} | Orange solid. |
| | | | **Rf** (DCM/MeOH, 98/2) : 0.37 |
| | | | **¹H NMR (400 MHz**, **DMSO-*d*₆) δ :** 0.88 (t, *J* = 7.3 Hz, 6H, 2xCH_{3-CH2}), 1.07 (dt, *J* = 8.5, 3.2 Hz, 2H, CH_{2 cPr}), 1.16 (dt, *J* = 5.6, 3.0 Hz, 2H, CH_{2 cPr}), 1.51 (ddq, *J* = 40.5, 14.0, 6.9 Hz, 4H, 2xCH_{2-CH3}), 2.24 - 2.32 (m, 1H, H_{cPr}), 3.63 (q, *J* = 6.5 Hz, 1H, H_{iPent}), 6.35 (s, 1H, H_{Ar}), 6.56 (d, *J* = 8.0 Hz, 1H, NH_{iPent}), 8.89 (s, 2H, 2×H_{Ar}), 8.93 (s, 1H, H_{Ar}), 9.59 (s, 1H, NH). |
| | | | **¹³C NMR (101 MHz, DMSO-*d*₆) δ :** 5.38 (CH_{cPr}), 6.50 (2xCH_{2 cPr}), 10.21 (2xCH_{3-CH2}), 25.97 (2xCH_{2-CH3}), 52.88 (CH_{iPent}), 88.84 (CH_{Ar}), 135.45 (C_{q}), 135.81 (C_{q}), 139.20 (C_{q}), 149.67 (2xCH_{Ar}), 150.35 (C_{q}), 152.85 (CH_{Ar}), 155.79 (C_{q}). |
| | | | **MS (ESI+) : m/z calcd for C₁₇H₂₂N₈ :** 339.20 [M+H]+, **found :** 339.36. |
| (179) | 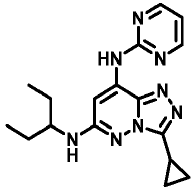 | > 97 %^{b} | White solid. |
| | | | **Rf** (DCM/MeOH, 95/5) : 0.4 |
| | | | **¹H NMR (400 MHz, DMSO-*d*₆) δ :** 0.90 (t, *J* = 7.4 Hz, 6H, 2xCH_{3-CH2}), 1.02 - 1.21 (m, 4H, 2xCH_{2 cPr}), 1.43 - 1.67 (m, 4H, 2xCH_{2-CH3}), 2.24 - 2.33 (m, 1H, H_{cPr}), 3.67 (q, *J* = 6.6 Hz, 1H, H_{iPent}), 6.99 (d, *J* = 8.0 Hz, 1H, NH_{iPent}), 7.15 (t, *J* = 4.8 Hz, 1H, H_{Ar}), 7.63 (s, 1H, H_{Ar}), 8.69 (d, *J* = 4.8 Hz, 2H, 2xH_{Ar}), 8.90 (s, 1H, NH). |
| | | | **¹³C NMR (101 MHz, DMSO-*d*₆) δ :** 5.36 (CH_{cPr}), 6.49 (2xCH_{2 cPr}), 10.40 (2xCH_{3-CH2}), 25.99 (2xCH_{2-CH3}), 53.15 (CH_{iPent}), 96.06 (CH_{Ar}), 115.24 (CH_{Ar}), 132.32 (C_{q}), 138.89 (C_{q}), 150.45 (C_{q}), 155.78 (C_{q}), 158.46 (2xCH_{Ar}), 158.91 (C_{q}). |
| | | | **MS (ESI+) : m/z calcd for C₁₇H₂₂N₈ :** 339.20 [M+H]+, **found :** 339.38. |
| (180) | 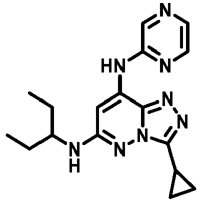 | > 98 % | White solid. |
| | | | **Rf** (DCM/MeOH, 95/5) : 0.36 |
| | | | **¹H NMR (400 MHz, DMSO-*d*₆) δ :** 0.89 (t, *J* = 7.4 Hz, 6H, 2xCH_{3-CH2}), 1.07 (dt, *J* = 8.7, 3.2 Hz, 2H, CH_{2 cPr}), 1.17 (dt, *J* = 5.6, 3.0 Hz, 2H, CH_{2 cPr}), 1.42 - 1.66 (m, 4H, 2xCH_{2-CH3}), 2.25 - 2.33 (m, 1H, H_{cPr}), 3.61 - 3.71 (m, 1H, H_{iPent}), 6.91 (d, *J* = 8.0 Hz, 1H, NH_{iPent}), 7.73 (s, 1H, H_{Ar}), 8.18 (d, *J* = 2.8 Hz, 1H, H_{Ar}), 8.32 (d, *J* = 2.3 Hz, 1H, H_{Ar}), 8.82 (s, 1H, H_{Ar}), 10.33 (s, 1H, NH). |
| | | | **¹³C NMR (101 MHz, DMSO-*d*₆) δ :** 5.37 (CH_{cPr}), 6.49(2xCH_{2 cPr}), 10.42 (2xCH_{3-CH2}), 26.03 (2xCH_{2-CH3}), 53.13 (CH_{iPent}), 95.10 (CH_{Ar}), 133.00 (C_{q}), 136.33 (CH_{Ar}), 137.23 (CH_{Ar}), 139.10 (C_{q}), 140.78 (CH_{Ar}), 150.35 (C_{q}), 151.54 (C_{q}), 155.90 (C_{q}). |
| | | | **MS (ESI+) : m/z calcd for C₁₇H₂₂N₈ :** 339.20 [M+H]+, **found :** 339.38. |
| (181) | 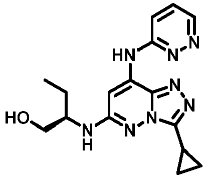 | > 99 % | White solid. |
| | | | **Rf** (DCM/MeOH, 91/9) : 0.42 |
| | | | **¹H NMR (400 MHz, DMSO-*d*₆) δ :** 0.92 (t, *J* = 7.4 Hz, 3H, CH_{3-CH2}), 1.03 - 1.12 (m, 2H, CH_{2 cPr}), 1.12 - 1.21 (m, 2H, CH_{2 cPr}), 1.42 - 1.54 (m, 1H, 0.5xCH_{2-CH3}), 1.63 - 1.78 (m, 1H, 0.5xCH_{2-CH3}), 2.26 - 2.38 (m, 1H, H_{cPr}), 3.44 (dt, *J* = 10.6, 5.9 Hz, 1H, 0.5×CH_{2-OH}), 3.52 (dt, *J* = 10.6, 5.3 Hz, 1H, 0.5×CH_{2-OH}), 3.76 (ddt, *J* = 13.1, 7.8, 5.5 Hz, 1H, H_{-butanol}), 4.62 (t, *J* = 5.6 Hz, 1H, OH), 7.04 (d, *J* = 7.9 Hz, 1H, NH_{-butanol}), 7.61 (dd, *J* = 9.0, 4.5 Hz, 1H, H_{Ar}), 7.78 (dd, *J* = 9.0, 1.4 Hz, 1H, H_{Ar}), 7.96 (s, 1H, H_{Ar}), 8.89 (dd, *J* = 4.5, 1.4 Hz, 1H, H_{Ar}), 10.11 (s, 1H, NH). |
| | | | **¹³C NMR (101 MHz, DMSO-*d*₆) δ :** 5.32 (CH_{cPr}), 6.64 (CH_{2 cPr}), 6.67 (CH_{2cPr}), 10.53 (CH_{3-CH2}), 23.52 (CH_{2-CH3}), 54.05 (CH-_{butanol}), 62.09 (CH_{2-OH}), 96.24 (CH_{Ar}), 118.62 (CH_{Ar}), 128.07 (CH_{Ar}), 132.82 (C_{q}), 139.17 (C_{q}), 146.78 (CH_{Ar}), 150.42 (C_{q}), 156.10 (C_{q}), 157.57 (C_{q}). |
| | | | **MS (ESI+) : m/z calcd for C₁₆H₂₀N₈O :** 341.18 [M+H]+, **found :** 341.36. |
| (182) | 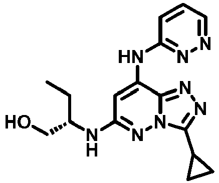 | > 99 % | White solid. |
| | | | **Rf** (DCM/MeOH, 90/10) : 0.41 |
| | | | **¹H NMR (400 MHz, DMSO-*d*₆) δ :** 0.92 (t, *J* = 7.4 Hz, 3H, CH_{3-CH2}), 1.04 - 1.21 (m, 4H, 2xCH_{2 cPr}), 1.40 - 1.56 (m, 1H, 0.5xCH_{2-CH3}), 1.63 - 1.76 (m, 1H, 0.5xCH_{2-CH3}), 2.32 (ddd, *J* = 13.7, 8.5, 5.1 Hz, 1H, H_{cPr}), 3.44 (dt, *J* = 11.0, 5.9 Hz, 1H, 0.5×CH_{2-OH}), 3.52 (dt, *J* = 10.5, 5.2 Hz, 1H, 0.5×CH_{2-OH})), 3.71 - 3.80 (m, 1H, H_{-butanol}), 4.62 (t, *J* = 5.6 Hz, 1H, OH), 7.04 (d, *J* = 7.9 Hz, 1H, NH_{-butanol}), 7.61 (dd, *J* = 9.0, 4.5 Hz, 1H, H_{Ar}), 7.78 (dd, *J* = 9.0, 1.5 Hz, 1H, H_{Ar}), 7.96 (s, 1H, H_{Ar}), 8.89 (dd, *J* = 4.6, 1.4 Hz, 1H, H_{Ar}), 10.12 (s, 1H, NH). |
| | | | **¹³C NMR (101 MHz, DMSO-*d*₆) δ :** 5.32 (CH_{cPr}), 6.64 (CH_{2cPr}), 6.67 (CH_{2cPr}), 10.53 (CH_{3-CH2}), 23.52 (CH_{2-CH3}), 54.05 (CH-butanol), 62.09 (CH_{2-OH}), 96.23 (CH_{Ar}), 118.62 (CH_{Ar}), 128.07 (CH_{Ar}), 132.82 (C_{q}), 139.17 (C_{q}), 146.78 (CH_{Ar}), 150.41 (C_{q}), 156.10 (C_{q}), 157.57 (C_{q}). |
| | | | **MS (ESI+) : m/z calcd for C₁₆H₂₀N₈O :** 341.18 [M+H]+, **found :** 341.37. |
| (183) | 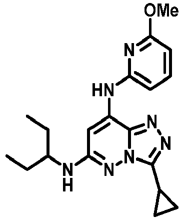 | > 95 % | White solid. |
| | | | **Rf** (DCM/MeOH, 96/4) : 0.28 |
| | | | **¹H NMR (400 MHz, DMSO-*d*₆) δ :** 0.90 (t, *J* = 7.4 Hz, 6H, 2xCH_{3-CH2}), 1.03 - 1.20 (m, 4H, 2xCH_{2 cPr}), 1.43 - 1.67 (m, 4H, 2xCH_{2-CH3}), 2.29 (tt, *J* = 8.5, 5.0 Hz, 1H, H_{cPr}), 3.59 - 3.72 (m, 1H, H_{iPent}), 3.94 (s, 3H, OCH₃), 6.40 (d, *J* = 7.9 Hz, 1H, H_{Ar}), 6.63 (d, *J* = 8.2 Hz, 1H, NH_{iPent}), 7.00 (d, *J* = 7.9 Hz, 1H, H_{Ar}), 7.44 (s, 1H, H_{Ar}), 7.63 (t, *J* = 7.9 Hz, 1H, H_{Ar}), 9.76 (s, 1H, NH_{Pyr}). |
| | | | **¹³C NMR (101 MHz, DMSO-*d*₆) δ :** 5.4 (CH_{cPr}), 6.4 (2×CH_{2 cPr}), 10.5 (2xCH_{3-CH2}), 26.2 (2xCH_{2-CH3}), 53.3 (CH_{iPent}), 53.9 (OCH₃), 93.6 (CH_{Ar}), 101.8 (CH_{Ar}), 105.3 (CH_{Ar}), 133.6 (C_{q}), 139.3 (C_{q}), 140.5 (CH_{Ar}), 150.2 (C_{q}), 152.9 (C_{q}), 156.1 (C_{q}), 162.6 (C_{q}). |
| | | | **MS (ESI+) : m/z calcd for C₁₉H₂₅N₇O :** 368.22 [M+H]+, **found :** 368.37. |
| (184) | 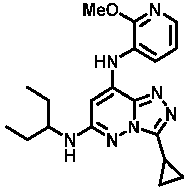 | 94 %^{b} | **Rf** (DCM/MeOH, 96/4) : 0.21 |
| | | | **¹H NMR (400 MHz, DMSO-*d*₆) δ :** 0.86 (t, *J* = 7.4 Hz, 6H, 2xCH_{3-CH2}), 1.02 - 1.19 (m, 4H, 2xCH_{2 cPr}), 1.37 - 1.60 (m, 4H, 2xCH_{2-CH3}), 2.28 (tt, *J* = 8.5, 5.1 Hz, 1H, H_{cPr}), 3.61 (dt, *J* = 13.4, 6.8 Hz, 1H, H_{iPent}), 3.92 (s, 3H, CH_{3-O}), 5.72 (s, 1H, H_{Ar}), 6.47 (d, *J* = 8.0 Hz, 1H, NH_{iPent}), 7.09 (dd, *J* = 7.6, 4.9 Hz, 1H, H_{Ar}), 7.71 (dd, *J* = 7.6, 1.7 Hz, 1H, H_{Ar}), 8.05 (dd, *J* = 4.9, 1.7 Hz, 1H, H_{Ar}), 8.48 (s, 1H, NH). |
| | | | **¹³C NMR (101 MHz, DMSO-*d*₆) δ :** 5.4 (CH_{cPr}), 6.4 (2×CH_{2 cPr}), 10.2 (2xCH_{3-CH2}), 26.0 (2xCH_{2-CH3}), 52.8 (CH_{iPent}), 53.4 (CH_{3-O}), 88.1 (CH_{Ar}), 117.3 (CH_{Ar}), 122.6 (C_{q}), 132.3 (CH_{Ar}), 136.7 (C_{q}), 139.4 (C_{q}), 142.5 (CH_{Ar}), 150.1 (C_{q}), 156.0 (C_{q}), 157.0 (C_{q}). |
| | | | **MS (ESI+) : m/z calcd for C₁₉H₂₅N₇O :** 368.22 [M+H]+, **found :** 368.37. |
| (185) | 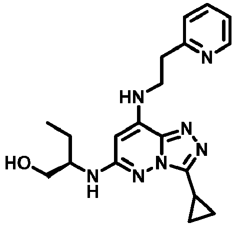 | - | Light yellow solid. |
| | | | **¹H NMR (400 MHz, DMSO-*d*₆) δ :** 0.90 (t, *J* = 7.4 Hz, 3H, CH_{3-CH2}), 1.07 (ddt, *J* = 29.3, 8.8, 3.5 Hz, 4H, 2xCH_{2 cPr}), 1.38 - 1.74 (m, 2H, CH_{2-CH3}), 2.24 (td, *J* = 8.4, 4.3 Hz, 1H, H_{cPr}), 3.08 (t, *J* = 7.4 Hz, 2H, CH_{2-CH2}), 3.45 (dq, *J* = 27.1, 5.3 Hz, 2H, CH_{2-OH}), 3.54 (q, *J* = 7.0 Hz, 2H, CH_{2-NH}), 3.68 (tt, *J* = 8.0, 3.9 Hz, 1H, H_{C-NH}), 4.62 (t, *J* = 5.4 Hz, 1H, OH), 5.64 (s, 1H, H_{Ar}), 6.31 (d, *J* = 8.0 Hz, 1H, NH_{-CH}), 7.17 - 7.27 (m, 2H, NH_{-CH2} & H_{Ar}), 7.33 (d, *J* = 7.7 Hz, 1H, H_{Ar}), 7.72 (td, *J* = 7.6, 1.8 Hz, 1H, H_{Ar}), 8.51 (dd, *J* = 5.1, 1.8 Hz, 1H, H_{Ar}). |
| | | | **¹³C NMR (101 MHz, DMSO-*d*₆) δ :** 5.3 (CH_{cPr}), 6.5 (CH_{2 cPr}), 6.6 (CH_{2 cPr}), 10.5 (CH_{3-CH2}), 23.7 (CH_{2-CH3}), 35.9 (CH_{2-CH2}), 41.8 (CH_{2-CH2}), 53.6 (CH_{-NH}), 62.2 (CH_{2-OH}), 83.8 (CH_{Ar}), 121.6 (CH_{Ar}), 123.3 (CH_{Ar}), 136.5 (CH_{Ar}), 139.5 (C_{q}), 139.8 (C_{q}), 149.1 (CH_{Ar}), 149.9 (C_{q}), 156.3 (C_{q}), 158.9 (C_{q}). |
| (186) | 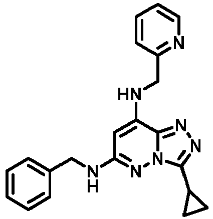 | - | Light yellow solid. |
| | | | **Rf** (DCM/MeOH, 94/6) : 0.36 |
| | | | **¹H NMR (400 MHz, DMSO-*d*₆) δ :** 0.96 - 1.14 (m, 4H, 2xCH_{2 cPr}), 2.25 (tt, *J* = 8.3, 5.1 Hz, 1H, H_{cPr}), 4.34 (d, *J* = 5.7 Hz, 2H, CH_{2-Ph}), 4.52 (d, *J* = 6.1 Hz, 2H, CH_{2-Pyr}), 5.45 (s, 1H, H_{Ar}), 7.12 (t, *J* = 5.8 Hz, 1H, NH_{-CH2-Ph}), 7.18 - 7.24 (m, 1H, H_{Ar}), 7.26 - 7.36 (m, 6H, 6xH_{Ar}), 7.75 (td, *J* = 7.7, 1.8 Hz, 1H, H_{Ar}), 7.85 (t, *J* = 6.2 Hz, 1H, NH_{-CH2-Pyr}), 8.54 (ddd, *J* = 4.8, 1.8, 0.9 Hz, 1H, H_{Ar}). |
| | | | **¹³C NMR (101 MHz, DMSO-*d*₆) δ :** 5.3 (CH_{cPr}), 6.5 (2xCH_{2 cPr}), 44.5 (CH_{2-Ph}), 47.2 (CH_{2-Pyr}), 84.3 (CH_{Ar}), 120.7 (CH_{Ar}), 122.3 (CH_{Ar}), 126.7 (CH_{Ar}), 127.7 (2xCH_{Ar}), 128.1 (2xCH_{Ar}), 136.9 (CH_{Ar}), 139.5 (C_{q}), 139.7 (C_{q}), 140.0 (C_{q}), 149.0 (CH_{Ar}), 150.1 (C_{q}), 155.9 (C_{q}), 157.5 (C_{q}). |
| (187) | 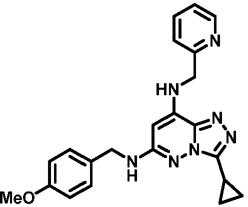 | - | White solid. |
| | | | **Rf** (DCM/MeOH, 95/5) : 0.46 |
| | | | **¹H NMR (400 MHz, DMSO-*d*₆) δ :** 1.01 - 1.12 (m, 4H, 2xCH_{2 cPr}), 2.27 (td, *J* = 8.3, 4.1 Hz, 1H, H_{cPr}), 3.71 (s, 3H, OCH₃), 4.26 (d, *J* = 5.6 Hz, 2H, CH_{2 PMB}), 4.51 (d, *J* = 6.0 Hz, 2H, CH_{2Bn}), 5.43 (s, 1H, H_{Ar}), 6.85 (d, *J* = 8.3 Hz, 2H, 2xH_{Ar}), 7.03 (t, *J* = 5.7 Hz, 1H, NH_{PMB}), 7.27 (t, *J* = 8.4 Hz, 3H, 3xH_{Ar}), 7.31 (d, *J* = 7.9 Hz, 1H, H_{Ar}), 7.75 (td, *J* = 7.6, 1.8 Hz, 1H, H_{Ar}), 7.84 (t, *J* = 6.2 Hz, 1H, NH_{Bn}), 8.54 (dd, *J* = 5.1, 1.6 Hz, 1H, H_{Ar}). |
| | | | **¹³C NMR (101 MHz, DMSO-*d*₆) δ :** 5.3 (CH_{cPr}), 6.6 (2xCH_{2 cPr}), 43.9 (CH_{2 PMB}), 47.2 (CH_{2 Bn}), 55.0 (OCH₃), 84.4 (CH_{Ar}), 113.5 (2xCH_{Ar}), 120.7 (CH_{Ar}), 122.3 (CH_{Ar}), 129.1 (2xCH_{Ar}), 131.6 (C_{q}), 136.9 (CH_{Ar}), 139.5 (C_{q}), 139.9 (C_{q}), 149.0 (CH_{Ar}), 150.1 (C_{q}), 155.8 (C_{q}), 157.5 (C_{q}), 158.2 (C_{q}). |
| (188) | 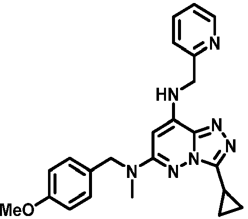 | > 97 %^{b} | White solid. |
| | | | **Rf** (DCM/MeOH, 95/5) : 0.36 |
| | | | **¹H NMR (400 MHz, DMSO-*d*₆) δ :** 1.00 - 1.15 (m, 4H, 2xCH_{2 cPr}), 2.29 (tt, *J* = 8.4, 5.1 Hz, 1H, H_{cPr}), 2.94 (s, 3H, CH_{3-N}), 3.71 (s, 3H, OCH₃), 4.52 (s, 2H, CH_{2 PMB}), 4.60 (d, *J* = 6.1 Hz, 2H, CH_{2Bn}), 5.75 (s, 1H, H_{Ar}), 6.76 - 6.86 (m, 2H, 2xH_{Ar}), 7.04 - 7.13 (m, 2H, 2xH_{Ar}), 7.26 (ddd, *J* = 7.5, 4.9, 1.2 Hz, 1H, H_{Ar}), 7.31 (dt, *J* = 7.9, 1.1 Hz, 1H, H_{Ar}), 7.73 (td, *J* = 7.7, 1.8 Hz, 1H, H_{Ar}), 7.98 (t, *J* = 6.1 Hz, 1H, NH_{Bn}), 8.49 (ddd, *J* = 4.8, 1.8, 0.9 Hz, 1H, H_{Ar}). |
| | | | **¹³C NMR (101 MHz, DMSO-*d*₆) δ :** 5.3 (CH_{cPr}), 6.6 (2xCH_{2 cPr}), 36.3 (CH_{3-N}), 47.2 (CH_{2 Bn}), 52.7 (CH_{2 PMB}), 55.0 (OCH₃), 82.5 (CH_{Ar}), 113.8 (2xCH_{Ar}), 121.3 (CH_{Ar}), 122.3 (CH_{Ar}), 128.6 (2xCH_{Ar}), 130.0 (C_{q}), 136.9 (CH_{Ar}), 138.9 (C_{q}), 140.5 (C_{q}), 148.9 (CH_{Ar}), 150.1 (C_{q}), 156.6 (C_{q}), 157.5 (C_{q}), 158.3 (C_{q}). |
| | | | **MS (ESI+) : m/z calcd for C₂₃H₂₅N₇O :** 416.22 [M+H]+, **found :** 416.51. |
| (189) | 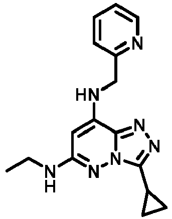 | > 96 %^{b} | White solid. |
| | | | **Rf** (DCM/MeOH, 94/6) : 0.15 |
| | | | **¹H NMR (400 MHz, DMSO-*d*₆) δ :** 1.00 - 1.16 (m, 7H, CH_{3-CH2} & 2xCH_{2 cPr}), 2.26 (tt, *J* = 8.4, 5.1 Hz, 1H, H_{cPr}), 3.11 - 3.21 (m, 2H, CH_{2-CH3}), 4.51 (d, *J* = 6.2 Hz, 2H, CH_{2Bn}), 5.34 (s, 1H, H_{Ar}), 6.55 (t, *J* = 5.2 Hz, 1H, NH_{Et}), 7.26 - 7.30 (m, 1H, H_{Ar}), 7.32 (d, *J* = 7.9 Hz, 1H, H_{Ar}), 7.76 (td, *J* = 7.7, 1.8 Hz, 1H, H_{Ar}), 7.81 (t, *J* = 6.2 Hz, 1H, NH_{Bn}), 8.50 - 8.61 (m, 1H, H_{Ar}). |
| | | | **¹³C NMR (101 MHz, DMSO-*d*₆) δ :** 5.3 (CH_{cPr}), 6.6 (2xCH_{2 cPr}), 14.2 (CH_{3-CH2}), 35.5 (CH_{2-CH3}), 47.2 (CH_{2 Bn}), 84.4 (CH_{Ar}), 120.7 (CH_{Ar}), 122.3 (CH_{Ar}), 137.0 (CH_{Ar}), 139.5 (C_{q}), 139.8 (C_{q}), 149.0 (CH_{Ar}), 150.1 (C_{q}), 156.0 (C_{q}), 157.6 (C_{q}). |
| | | | **MS (ESI+) : m/z calcd for C₁₆H₁₉N₇ :** 310.18 [M+H]+, **found :** 310.32. |
| (190) | 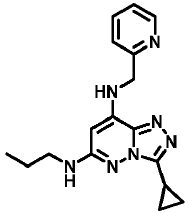 | > 97 %^{b} | Light yellow solid. |
| | | | **¹H NMR (400 MHz, DMSO-*d*₆) δ :** 0.88 (t, *J* = 7.4 Hz, 3H, CH_{3-CH2}), 1.01 - 1.14 (m, 4H, 2xCH_{2 cPr}), 1.51 (h, *J* = 7.3 Hz, 2H, CH_{2-CH3}), 2.26 (td, *J* = 8.5, 4.3 Hz, 1H, H_{cPr}), 3.09 (q, *J* = 6.5 Hz, 2H, CH_{2-NH}), 4.52 (d, *J* = 6.1 Hz, 2H, CH_{2 Bn}), 5.37 (s, 1H, H_{Ar}), 6.56 (t, *J* = 5.3 Hz, 1H, NH_{-CH2-CH2}), 7.28 (dd, *J* = 7.5, 4.9 Hz, 1H, H_{Ar}), 7.32 (d, *J* = 7.8 Hz, 1H, H_{Ar}), 7.76 (td, *J* = 7.7, 1.9 Hz, 1H, H_{Ar}), 7.80 (t, *J* = 6.1 Hz, 1H, NH_{Bn}), 8.55 (d, *J* = 4.8 Hz, 1H, H_{Ar}). |
| | | | **¹³C NMR (101 MHz, DMSO-*d*₆) δ :** 5.3 (CH_{cPr}), 6.6 (2xCH_{2 cPr}), 11.7 (CH_{3-CH2}), 21.7 (CH_{2-CH3}), 42.7 (CH_{2-NH}), 47.2 (CH_{2 Bn}), 84.4 (CH_{Ar}), 120.7 (CH_{Ar}), 122.3 (CH_{Ar}), 136.9 (CH_{Ar}), 139.5 (C_{q}), 139.8 (C_{q}), 149.0 (CH_{Ar}), 150.1 (C_{q}), 156.2 (C_{q}), 157.6 (C_{q}). |
| | | | **MS (ESI+) : m/z calcd for C₁₇H₂₁N₇ :** 324.19 [M+H]+, **found :** 324.39. |
| (191) | 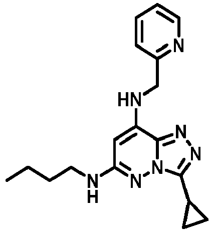 | > 97 %^{b} | White solid. |
| | | | **Rf** (DCM/MeOH, 95/5) : 0.46 |
| | | | **¹H NMR (400 MHz, DMSO-*d₆*) δ :** 0.83 - 0.92 (m, 3H, CH_{3-CH2}), 0.99 - 1.16 (m, 4H, 2xCH_{2 cPr}), 1.32 (h, *J* = 7.3 Hz, 2H, CH_{2-CH3}), 1.48 (p, *J* = 7.1 Hz, 2H, CH_{2-CH2-CH3}), 2.26 (tt, *J* = 9.0, 5.1 Hz, 1H, H_{ePr}), 3.06 - 3.22 (m, 2H, , CH_{2-NH nBut}), 4.51 (d, *J* = 6.1 Hz, 2H, CH_{2 Bn}), 5.37 (s, 1H, H_{Ar}), 6.53 (t, *J* = 5.3 Hz, 1H, NH_{nBu}), 7.26 - 7.30 (m, 1H, H_{Ar}), 7.31 (d, *J* = 7.8 Hz, 1H, H_{Ar}), 7.76 (tt, *J* = 7.7, 1.6 Hz, 1H, H_{Ar}), 7.80 (t, *J* = 6.2 Hz, 1H, NH_{Bn}), 8.54 (dd, *J* = 4.8, 1.4 Hz, 1H, H_{Ar}). |
| | | | **¹³C NMR (101 MHz, DMSO-*d₆*) δ :** 5.3 (CH_{cPr}), 6.6 (2xCH_{2 cPr}), 13.7 (CH_{3-CH2}), 19.8 (CH_{2-CH3}), 30.6 (CH_{2-CH2-CH3}), 40.4 (CH_{2-NH} n_{But}), 47.2 (CH_{2 Bn}), 84.4 (CH_{Ar}), 120.7 (CH_{Ar}), 122.3 (CH_{Ar}), 136.9 (CH_{Ar}), 139.5 (C_{q}), 139.7 (C_{q}), 149.0 (CH_{Ar}), 150.1 (C_{q}), 156.2 (C_{q}), 157.6 (C_{q}). |
| | | | **MS (ESI+) : m/z calcd for C₁₈H₂₃N₇** : 338.21 [M+H]+, **found** : 338.37. |
| (192) | 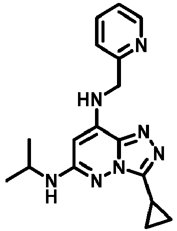 | 92 %^{b} | White solid. |
| | | | **Rf** (DCM/MeOH, 95/5) : 0.24 |
| | | | **¹H NMR (400 MHz, DMSO-*d₆*) δ :** 1.00 - 1.18 (m, 10H, 2×CH_{3 iPr} & 2xCH_{2 cPr}), 2.26 (tt, *J* = 8.8, 5.0 Hz, 1H, H_{cPr}), 3.83 (h, *J* = 6.5 Hz, 1H, H_{iPr}), 4.51 (d, *J* = 6.1 Hz, 2H, CH_{2Bn}), 5.34 (s, 1H, H_{Ar}), 6.43 (d, *J* = 7.2 Hz, 1H, NH_{iPr}), 7.28 (dd, *J* = 7.4, 4.9 Hz, 1H, H_{Ar}), 7.32 (d, *J* = 7.9 Hz, 1H, H_{Ar}), 7.73 - 7.78 (m, 1H, H_{Ar}), 7.79 (t, *J* = 5.3 Hz, 1H, NH_{Bn}), 8.55 (dd, *J* = 4.9, 1.6 Hz, 1H, H_{Ar}). |
| | | | **¹³C NMR (101 MHz, DMSO-*d₆*) δ :** 5.3 (CH_{cPr}), 6.4 (2xCH_{2 cPr}), 22.3 (2×CH_{3 iPr}), 41.9 (CH_{iPr}), 47.2 (CH_{2 Bn}), 84.6 (CH_{Ar}), 120.6 (CH_{Ar}), 122.3 (CH_{Ar}), 137.0 (CH_{Ar}), 139.5 (C_{q}), 139.8 (C_{q}), 149.0 (CH_{Ar}), 150.0 (C_{q}), 155.4 (C_{q}), 157.6 (C_{q}). |
| | | | **MS (ESI+) : m/z calcd for C₁₇H₂₁N₇ :** 324.19 [M+H]+, **found :** 324.37 |
| (193) | 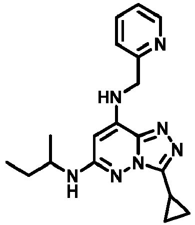 | > 97 %^{b} | White solid. |
| | | | **Rf** (DCM/MeOH, 94/6) : 0.33 |
| | | | **¹H NMR (400 MHz, DMSO-*d₆*) δ :** 0.85 (t, *J* = 7.5 Hz, 3H, CH_{3-CH2}), 0.99 - 1.06 (m, 2H, CH_{2 cPr}), 1.08 (d, *J* = 6.5 Hz, 3H, CH_{3-CH}), 1.13 (tt, *J* = 5.5, 3.0 Hz, 2H, CH_{2 ePr}), 1.36 - 1.57 (m, 2H, CH_{2-CH3}), 2.25 (tt, *J* = 8.4, 5.1 Hz, 1H, H_{ePr}), 3.66 (hept, *J* = 6.2 Hz, 1H, H_{C-CH3}), 4.51 (d, *J* = 6.2 Hz, 2H, H₂, CH_{2Bn}), 5.37 (s, 1H, H_{Ar}), 6.39 (d, *J* = 7.6 Hz, 1H, NH_{-CH2-CH}), 7.28 (ddd, *J* = 7.5, 4.8, 1.2 Hz, 1H, H_{Ar}), 7.32 (dt, *J* = 7.8, 1.1 Hz, 1H, H_{Ar}), 7.73 - 7.80 (m, 2H, NH_{Bn} & H_{Ar}), 8.55 (ddd, *J* = 4.9, 1.8, 0.9 Hz, 1H, H_{Ar}). |
| | | | **¹³C NMR (101 MHz, DMSO-*d₆*) δ :** 5.4 (CH_{cPr}), 6.4 (2xCH_{2 cPr}), 10.4 (CH_{3-CH2}), 19.7 (CH_{3-CH}), 28.6 (CH_{2-CH3}), 47.2 (CH_{2 Bn}), 47.3 (CH_{-CH3}), 84.6 (CH_{Ar}), 120.7 (CH_{Ar}), 122.3 (CH_{Ar}), 136.9 (CH_{Ar}), 139.5 (C_{q}), 139.8 (C_{q}), 149.0 (CH_{Ar}), 149.9 (C_{q}), 155.6 (C_{q}), 157.6 (C_{q}). |
| | | | **MS (ESI+) : m/z calcd for C₁₈H₂₃N₇** : 338.21 [M+H]+, **found** : 338.41 |
| (194) | 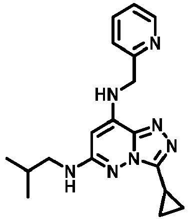 | > 96 %^{b} | White solid. |
| | | | **¹H NMR (400 MHz, DMSO-*d₆*) δ :** 0.88 (d, *J* = 6.7 Hz, 6H, 2×CH_{3 iBut}), 0.98 - 1.17 (m, 4H, 2xCH_{2 cPr}), 1.82 (hept, *J* = 6.7 Hz, 1H, H_{iBut}), 2.26 (tt, *J* = 8.4, 5.1 Hz, 1H, H_{ePr}), 2.96 (dd, *J* = 6.8, 5.5 Hz, 2H, CH_{2-CH}), 4.52 (d, *J* = 6.2 Hz, 2H, CH_{2 Bn}), 5.42 (s, 1H, H_{Ar}), 6.57 (t, *J* = 5.5 Hz, 1H, NH_{iBut}), 7.28 (ddd, *J* = 7.5, 4.8, 1.2 Hz, 1H, H_{Ar}), 7.32 (dt, *J* = 8.0, 1.1 Hz, 1H, H_{Ar}), 7.76 (td, *J* = 7.7, 1.8 Hz, 1H, H_{Ar}), 7.79 (t, *J* = 6.2 Hz, 1H, NH_{Bn}), 8.55 (ddd, *J* = 4.9, 1.8, 0.9 Hz, 1H, H_{Ar}). |
| | | | **¹³C NMR (101 MHz, DMSO-*d₆*) δ :** 5.3 (CH_{cPr}), 6.6 (2xCH_{2 cPr}), 20.4 (2×CH_{3 iBut}), 27.4 (CH_{iBut}), 47.2 (CH_{2 Bn}), 48.6 (CH_{2 iBut}), 84.4 (CH_{Ar}), 120.7 (CH_{Ar}), 122.3 (CH_{Ar}), 136.9 (CH_{Ar}), 139.5 (C_{q}), 139.7 (C_{q}), 149.0 (CH_{Ar}), 150.1 (C_{q}), 156.3 (C_{q}), 157.6 (C_{q}). |
| | | | **MS (ESI+) : m/z calcd for C₁₈H₂₃N₇** : 338.21 [M+H]+, **found** : 338.38 |
| (195) | 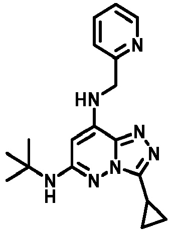 | 93 %^{b} | Light brown solid. |
| | | | **¹H NMR (400 MHz, DMSO-*d₆*) δ :** 0.98 - 1.16 (m, 4H, 2×CH_{2 cPr}), 1.35 (s, 9H, 3xCH₃ tBu), 2.27 (tt, *J* = 8.4, 5.1 Hz, 1H, H_{ePr}), 4.49 (d, *J* = 6.2 Hz, 2H, CH_{2Bn}), 5.41 (s, 1H, NH_{tBu}), 6.24 (s, 1H, H_{Ar}), 7.24 -7.35 (m, 2H, 2×H_{Ar}), 7.71 (t, *J* = 6.2 Hz, 1H, NH_{Bn}), 7.76 (td, *J* = 7.7, 1.8 Hz, 1H, H_{Ar}), 8.52 - 8.58 (m, 1H, H_{Ar}). |
| | | | **¹³C NMR (101 MHz, DMSO-*d₆*) δ :** 5.5 (CH_{cPr}), 6.2 (2xCH_{2 cPr}), 28.6 (3×CH_{3 tBu}), 47.1 (CH_{2 Bn}), 50.6 (C_{q}), 85.5 (CH_{Ar}), 120.5 (CH_{Ar}), 122.3 (CH_{Ar}), 137.0 (CH_{Ar}), 139.1 (C_{q}), 139.6 (C_{q}), 149.1 (CH_{Ar}), 149.9 (C_{q}), 155.4 (C_{q}), 157.7 (C_{q}). |
| | | | **MS (ESI+) : m/z calcd for C₁₈H₂₃N₇** : 338.21 [M+H]+, **found** : 338.37. |
| (196) | 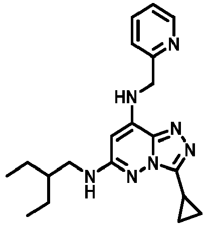 | > 99 %^{b} | White solid. |
| | | | **Rf** (DCM/MeOH, 94/6) : 0.20 |
| | | | **¹H NMR (400 MHz, DMSO-*d₆*) δ :** 0.85 (t, *J* = 7.4 Hz, 6H, 2×CH_{3-CH2}), 0.99 - 1.15 (m, 4H, 2xCH_{2 cPr}), 1.21 - 1.37 (m, 4H, 2xCH_{2-CH3}), 1.44 (hept, *J* = 6.3 Hz, 1H, H_{C-CH2-NH}), 2.25 (tt, *J* = 8.4, 5.1 Hz, 1H, H_{ePr}), 3.07 (t, *J* = 5.9 Hz, 2H, CH_{2-NH}), 4.52 (d, *J* = 6.2 Hz, 2H, CH_{2Bn}), 5.43 (s, 1H, H_{Ar}), 6.52 (t, *J* = 5.6 Hz, 1H, NH_{-CH2-CH}), 7.28 (ddd, *J* = 7.5, 4.9, 1.2 Hz, 1H, H_{Ar}), 7.32 (dt, *J* = 7.9, 1.1 Hz, 1H, H_{Ar}), 7.75 (dd, *J* = 7.7, 1.9 Hz, 1H, H_{Ar}), 7.76 - 7.81 (m, 1H, NH_{Bn}), 8.55 (ddd, *J* = 4.8, 1.8, 0.9 Hz, 1H, H_{Ar}). |
| | | | **¹³C NMR (101 MHz, DMSO-*d₆*) δ :** 5.3 (CH_{cPr}), 6.5 (2×CH_{2 cPr}), 10.9 (2×CH_{3-CH2}), 23.5 (2×CH_{2-cH3}), 39.9 (CH_{-CH2-NH}), 43.3 (CH_{2-NH}), 47.2 (CH_{2 Bn}), 84.5 (CH_{Ar}), 120.7 (CH_{Ar}), 122.3 (CH_{Ar}), 136.9 (CH_{Ar}), 139.5 (C_{q}), 139.7 (C_{q}), 149.0 (CH_{Ar}), 150.0 (C_{q}), 156.4 (C_{q}), 157.6 (C_{q}). |
| | | | **MS (ESI+) : m/z calcd for C₂₀H₂₇N₇** : 366.24 [M+H]+, **found** : 366.39. |
| (197) | 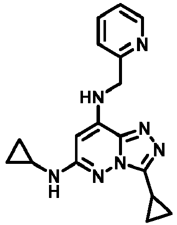 | 88 % ^{b} (unstable) | White solid. |
| | | | **¹H NMR (400 MHz, DMSO-*d₆*) δ :** 0.27 - 0.42 (m, 2H, CH_{2 cPr}), 0.61 (td, *J* = 6.8, 4.6 Hz, 2H, CH_{2 cPr}), 1.00 - 1.17 (m, 4H, 2×CH_{2 cPr}), 2.28 (tt, *J* = 8.4, 5.1 Hz, 1H, H_{cPr}), 2.52 - 2.52 (m, 1H, H_{cPr (DEPT135 observed})), 4.52 (d, *J* = 6.1 Hz, 2H, CH_{2Bn}), 5.38 (s, 1H, H_{Ar}), 6.83 (d, 7 = 2.9 Hz, 1H, NH_{ePr}), 7.28 (dd, *J* = 7.5, 5.0 Hz, 1H, H_{Ar}), 7.32 (d, *J* = 7.8 Hz, 1H, H_{Ar}), 7.76 (td, 7 = 7.7, 1.8 Hz, 1H, H_{Ar}), 7.90 (t, *J* = 6.2 Hz, 1H, NH_{Bn}), 8.49 - 8.61 (m, 1H, H_{Ar}). |
| | | | **¹³C NMR (101 MHz, DMSO-*d₆*) δ :** 5.3 (CH_{cPr}), 6.5 (2xCH_{2 cPr}), 6.7 (2xCH_{2 cPr}), 23.5 (CH_{cPr-N}), 47.3 (CH_{2 Bn}), 83.9 (CH_{Ar}), 120.8 (CH_{Ar}), 122.3 (CH_{Ar}), 136.9 (CH_{Ar}), 139.6 (C_{q}), 139.9 (C_{q}), 149.0 (CH_{Ar}), 150.1 (C_{q}), 157.2 (C_{q}), 157.6 (C_{q}). |
| | | | **MS (ESI+) : m/z calcd for C₁₇H₁₉N₇ :** 322.18 [M+H]+, **found** : 322.32. |
| (198) | 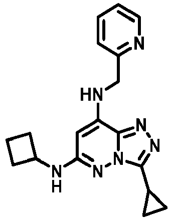 | > 97 %^{b} | White solid. |
| | | | **¹H NMR (400 MHz**, **DMSO-*d₆*) δ :** 1.00 - 1.16 (m, 4H, 2×CH_{2 cPr}), 1.65 (tdd, *J* = 11.1, 7.7, 5.8 Hz, 2H, CH_{2 cBut}), 1.82 (dddd, *J* = 12.0, 9.4, 7.2, 5.3 Hz, 2H, CH_{2 cBut}), 2.19 - 2.31 (m, 3H, CH_{2 cBut} &H_{cPr}), 4.11 (hept, *J* = 7.3 Hz, 1H, H_{cBut}), 4.51 (d, *J* = 6.2 Hz, 2H, CH_{2Bn}), 5.30 (s, 1H, H_{Ar}), 6.85 (d, *J* = 6.8 Hz, 1H, NH_{cBut}), 7.28 (ddd, *J* = 7.5, 4.9, 1.3 Hz, 1H, H_{Ar}), 7.30 - 7.33 (m, 1H, H_{Ar}), 7.76 (td, *J* = 7.7, 1.8 Hz, 1H, H_{Ar}), 7.84 (t, *J* = 6.2 Hz, 1H, NH_{Bn}), 8.55 (ddd, *J* = 4.8, 1.8, 0.9 Hz, 1H, H_{Ar}). |
| | | | **¹³C NMR (101 MHz, DMSO-*d₆*) δ :** 5.3 (CH_{cPr}), 6.5 (2xCH_{2 cPr}), 14.9 (CH_{2 cBut}), 30.3 (2×CH_{2 cBut}), 46.1 (CH_{cBut}), 47.2 (CH_{2 Bn}), 84.1 (CH_{Ar}), 120.7 (CH_{Ar}), 122.3 (CH_{Ar}), 137.0 (CH_{Ar}), 139.5 (C_{q}), 140.0 (C_{q}), 149.0 (CH_{Ar}), 150.0 (C_{q}), 155.1 (C_{q}), 157.6 (C_{q}). |
| | | | **MS (ESI+) : m/z calcd for C₁₈H₂₁N₇ :** 336.19 [M+H]+, **found** : 336.33 |
| (199) | 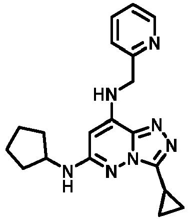 | > 96 %^{b} | White solid. |
| | | | **¹H NMR (400 MHz, DMSO-*d₆*) δ :** 1.09 (ddt, *J* = 41.4, 8.6, 3.6 Hz, 4H, 2×CH_{2 cPr}), 1.41 (tt, *J* = 13.6, 6.0 Hz, 2H, CH_{2 cPent}), 1.51 (dtd, *J* = 11.6, 7.2, 3.0 Hz, 2H, CH_{2 cPent}), 1.56 - 1.67 (m, 2H, CH_{2 cPent}), 1.90 (dq, *J* = 12.5, 6.4 Hz, 2H, CH_{2c Pent}), 2.27 (tt, *J* = 8.4, 5.1 Hz, 1H, H_{cPr}), 3.94 (h, *J* = 6.5 Hz, 1H, H_{cPent}), 4.51 (d, *J* = 6.1 Hz, 2H, CH_{2Bn}), 5.35 (s, 1H, H_{Ar}), 6.58 (d, *J* = 6.3 Hz, 1H, NH_{cPent}), 7.28 (dd, *J* = 7.5, 4.9 Hz, 1H, H_{Ar}), 7.31 (d, *J* = 7.9 Hz, 1H, H_{Ar}), 7.76 (td, *J* = 7.6, 1.8 Hz, 1H, H_{Ar}), 7.79 (t, *J* = 6.6 Hz, 1H, NH_{Bn}), 8.54 (d, *J* = 4.5 Hz, 1H, H_{Ar}). |
| | | | **¹³C NMR (101 MHz, DMSO-*d₆*) δ :** 5.4 (CH_{cPr}), 6.4 (2xCH_{2 cPr}), 23.5 (2×CH_{2 cPent}), 32.3 (2×CH_{2 cPent}), 47.2 (CH_{2 Bn}), 52.2 (CH_{cPent}), 84.5 (CH_{Ar}), 120.6 (CH_{Ar}), 122.3 (CH_{Ar}), 137.0 (CH_{Ar}), 139.5 (C_{q}), 139.7 (C_{q}), 149.0 (CH_{Ar}), 150.0 (C_{q}), 155.8 (C_{q}), 157.6 (C_{q}). |
| | | | **MS (ESI+) : m/z calcd for C₁₉H₂₃N₇ :** 350.21 [M+H]+, **found** : 350.37. |
| (200) | 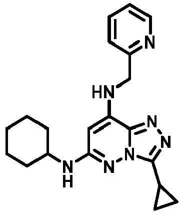 | > 98 %^{b} | White solid. |
| | | | **¹H NMR (400 MHz, DMSO-*d₆*) δ :** 0.98 - 1.06 (m, 2H, CH_{2 cPr}), 1.07 - 1.21 (m, 5H, CH_{2 cPr} &CH_{2 cHex} & H(_{CH2 cHex})), 1.21 - 1.35 (m, 2H, CH_{2 cHex}), 1.55 (dd, *J* = 12.3, 4.7 Hz, 1H, H(c_{H2 cHex)}), 1.68 (dt, *J* = 12.1, 4.3 Hz, 2H, CH_{2 cHex}), 1.90 (dd, *J* = 12.5, 3.7 Hz, 2H, CH_{2 cHex}), 2.25 (tt, *J* = 8.5, 5.1 Hz, 1H, H_{cPr}), 3.45 - 3.64 (m, 1H, H_{cHex}), 4.51 (d, *J* = 6.1 Hz, 2H, CH_{2Bn}), 5.37 (s, 1H, H_{Ar}), 6.43 (d, *J* = 7.3 Hz, 1H, NH_{cHex}), 7.28 (ddd, *J* = 7.5, 4.8, 1.2 Hz, 1H, H_{Ar}), 7.31 (dd, *J* = 7.9, 1.1 Hz, 1H, H_{Ar}), 7.67 - 7.86 (m, 2H, NH_{Bn} & H_{Ar}), 8.55 (ddd, *J* = 4.9, 1.8, 0.9 Hz, 1H, H_{Ar}). |
| | | | **¹³C NMR (101 MHz, DMSO-*d₆*) δ :** 5.3 (CH_{cPr}), 6.5 (2×CH_{2 cPr}), 24.5 (2xCH_{2 cHex}), 25.5 (CH_{2 cHex}), 32.2 (2×CH_{2 cHex}), 47.2 (CH_{2 Bn}), 49.1 (CH_{cHex}), 84.5 (CH_{Ar}), 120.6 (CH_{Ar}), 122.3 (CH_{Ar}), 137.0 (CH_{Ar}), 139.5 (C_{q}), 139.8 (C_{q}), 149.0 (CH_{Ar}), 149.9 (C_{q}), 155.3 (C_{q}), 157.6 (C_{q}). |
| | | | **MS (ESI+) : m/z calcd for C₂₀H₂₅N₇ :** 364.22 [M+H]+, **found** : 364.34 |
| (201) | 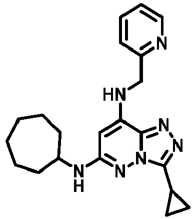 | > 97 %^{b} | White solid. |
| | | | **¹H NMR (400 MHz, DMSO-*d₆*) δ :** 0.99 - 1.20 (m, 4H, 2xCH_{2 cPr}), 1.38 - 1.63 (m, 10H, 5×CH_{2 CHept}), 1.86 (ddd, *J* = 9.9, 6.5, 4.3 Hz, 2H, CH_{2 cHept}), 2.25 (tt, *J* = 8.5, 5.1 Hz, 1H, H_{cPr}), 3.71 (q, *J* = 7.0, 5.5 Hz, 1H, H_{cHept}), 4.51 (d, *J* = 6.1 Hz, 2H, CH_{2Bn}), 5.37 (s, 1H, H_{Ar}), 6.48 (d, *J* = 7.3 Hz, 1H, NH_{cHept}), 7.28 (ddd, *J* = 7.5, 4.8, 1.1 Hz, 1H, H_{Ar}), 7.30 - 7.34 (m, 1H, H_{Ar}), 7.73 - 7.80 (m, 2H, NH_{Bn} & H_{Ar}), 8.55 (ddd, *J* = 4.8, 1.8, 0.9 Hz, 1H, H_{Ar}). |
| | | | **¹³C NMR (101 MHz, DMSO-*d₆*) δ :** 5.4 (CH_{cPr}), 6.3 (2xCH_{2 cPr}), 24.0 (2xCH_{2 cHept}), 28.0 (2×CH_{2 cHept}), 33.7 (2xCH_{2 cHept}), 47.2 (CH_{2 Bn}), 51.1 (CH_{cHept}), 84.6 (CH_{Ar}), 120.6 (CH_{Ar}), 122.3 (CH_{Ar}), 137.0 (CH_{Ar}), 139.5 (C_{q}), 139.7 (C_{q}), 149.0 (CH_{Ar}), 149.9 (C_{q}), 155.1 (C_{q}), 157.6 (C_{q}). |
| | | | **MS (ESI+) : m/z calcd for C₂₁H₂₇N₇ :** 378.24 [M+H]+, **found** : 378.38 |
| (202) | 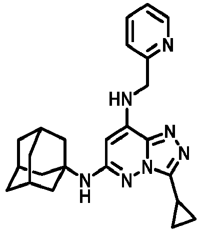 | 93 %^{b} | White solid. |
| | | | **¹H NMR (400 MHz, DMSO-*d₆*) δ :** 1.02 - 1.08 (m, 2H, CH_{2 cPr}), 1.17 (dt, *J* = 5.9, 2.9 Hz, 2H, CH_{2 cPr}), 1.63 (s, 6H, 3×CH_{2 Ada}), 2.03 (s, 9H, 3×H_{Ada} &3×CH_{2 Ada}), 2.26 (tt, *J* = 8.5, 5.2 Hz, 1H, H_{cPr}), 4.49 (d, *J* = 6.2 Hz, 2H, CH_{2Bn}), 5.42 (s, 1H, H_{Ar}), 6.14 (s, 1H, NH_{Ada}), 7.24 - 7.33 (m, 2H, 2×H_{Ar}), 7.71 (t, *J* = 6.2 Hz, 1H, NH_{Bn}), 7.76 (td, *J* = 7.7, 1.8 Hz, 1H, H_{Ar}), 8.55 (ddd, *J* = 4.8, 1.8, 0.9 Hz, 1H, H_{Ar}). |
| | | | **¹³C NMR (101 MHz, DMSO-*d₆*) δ :** 5.7 (CH_{cPr}), 6.0 (2xCH_{2 cPr}), 28.9 (3×CH_{Ada}), 36.2 (3×CH_{2 Ada}), 40.9 (3×CH_{2 Ada}), 47.1 (CH_{2 Bn}), 51.1 (C_{q Ada}), 85.5 (CH_{Ar}), 120.5 (CH_{Ar}), 122.3 (CH_{Ar}), 136.9 (CH_{Ar}), 139.1 (C_{q}), 139.6 (C_{q}), 149.0 (CH_{Ar}), 149.7 (C_{q}), 155.2 (C_{q}), 157.6 (C_{q}). |
| | | | **MS (ESI+) : m/z calcd for C₂₄H₂₉N₇ :** 416.26 [M+H]+, **found** : 416.44. |
| (203) | 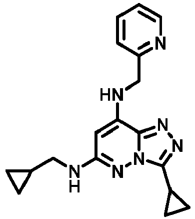 | > 96 %^{b} | White solid. |
| | | | **Rf** (DCM/MeOH, 94/6) : 0.16 |
| | | | **¹H NMR (400 MHz, DMSO-*d₆*) δ** : 0.18 (q, *J* = 5.0 Hz, 2H, CH_{2 cPr}), 0.37 - 0.50 (m, 2H, CH_{2 cPr}), 0.97 - 1.14 (m, 5H, H_{cPr} & 2×CH_{2 cPr}), 2.26 (ddd, *J* = 13.9, 8.5, 5.1 Hz, 1H, H_{cPr}), 3.00 (t, *J* = 6.1 Hz, 2H, CH_{2-NH}), 4.52 (d, *J* = 6.1 Hz, 2H, CH_{2Bn}), 5.41 (s, 1H, H_{Ar}), 6.69 (t, *J* = 5.3 Hz, 1H, NH_{-CH2}), 7.28 (dd, *J* = 7.4, 4.9 Hz, 1H, H_{Ar}), 7.32 (d, *J* = 7.9 Hz, 1H, H_{Ar}), 7.76 (td, *J* = 7.8, 1.8 Hz, 1H, H_{Ar}), 7.81 (t, *J* = 6.2 Hz, 1H, NH_{Bn}), 8.55 (d, *J* = 4.9 Hz, 1H, H_{Ar}). |
| | | | **¹³C NMR (101 MHz, DMSO-*d₆*) δ :** N.D. |
| | | | **MS (ESI+) : m/z calcd for C₁₈H₂₁N₇** : 336.19 [M+H]+, **found** : 336.38 |
| (204) | 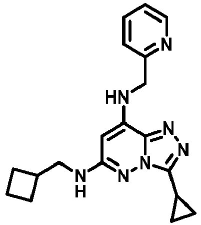 | > 97 %^{b} | White solid. |
| | | | **¹H NMR (400 MHz, DMSO-*d₆*) δ :** 1.01 - 1.15 (m, 4H, 2×CH_{2 cPr}), 1.61 - 1.73 (m, 2H, CH_{2 But}), 1.75 - 1.87 (m, 2H, CH_{2 cBut}), 1.94 - 2.06 (m, 2H, CH_{2 cBut}), 2.22 - 2.31 (m, 1H, H_{cPt}), 2.43 - 2.48 (m, 1H, H_{cBut}), 3.17 (dd, *J* = 7.2, 5.3 Hz, 2H, CH₂-_{CH cBut}), 4.51 (d, *J* = 6.2 Hz, 2H, CH_{2Bn}), 5.39 (s, 1H, H_{Ar}), 6.52 (t, *J* = 5.3 Hz, 1H, NH_{cBut}), 7.28 (ddd, *J* = 7.6, 4.9, 1.2 Hz, 1H, H_{Ar}), 7.31 (d, *J* = 8.0 Hz, 1H, H_{Ar}), 7.75 (td, *J* = 7.7, 1.8 Hz, 1H, H_{Ar}), 7.79 (t, *J* = 6.2 Hz, 1H, NH_{Bn}), 8.47 - 8.62 (m, 1H, H_{Ar}). |
| | | | **¹³C NMR (101 MHz, DMSO-*d₆*) δ :** 5.2 (CH_{cPr}), 6.7 (2×CH_{2 cPr}), 17.9 (CH_{2 cBut}), 25.6 (2×CH_{2 cBut}), 34.3 (CH_{cBut}), 46.3 (CH_{2-CHcBut}), 47.2 (CH_{2 Bn}), 84.4 (CH_{Ar}), 120.7 (CH_{Ar}), 122.3 (CH_{Ar}), 136.9 (CH_{Ar}), 139.5 (C_{q}), 139.7 (C_{q}), 149.0 (CH_{Ar}), 150.1 (C_{q}), 156.3 (C_{q}), 157.6 (C_{q}). |
| | | | **MS (ESI+) : m/z calcd for C₁₉H₂₃N₇** : 350.21 [M+H]+, **found** : 350.40 |
| (205) | 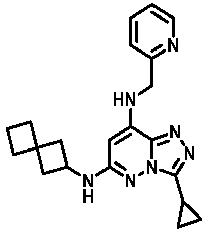 | - | White solid. |
| | | | **Rf** (DCM/MeOH, 94/6) : 0.19 |
| | | | **¹H NMR (400 MHz, DMSO-*d₆*) δ :** 1.00 - 1.16 (m, 4H, 2×CH_{2 cPr}), 1.74 - 1.83 (m, 4H, 2×CH₂), 1.87 (dd, *J* = 8.3, 5.6 Hz, 2H, CH₂), 1.96 - 2.03 (m, 2H, CH₂), 2.26 (tt, *J* = 8.4, 5.1 Hz, 1H, H_{cPr}), 2.35 (ddt, *J* = 12.0, 8.0, 2.2 Hz, 2H, CH₂), 3.93 (h, *J* = 7.8 Hz, 1H, H_{C-NH}), 4.50 (d, *J* = 6.1 Hz, 2H, CH_{2Bn}), 5.28 (s, 1H, H_{Ar}), 6.77 (d, *J* = 6.4 Hz, 1H, NH_{-CH}), 7.23 - 7.36 (m, 2H, 2×H_{Ar}), 7.75 (td, *J* = 7.7, 1.8 Hz, 1H, H_{Ar}), 7.83 (t, *J* = 6.2 Hz, 1H, NH_{Bn}), 8.54 (dt, *J* = 4.8, 1.5 Hz, 1H, H_{Ar}). |
| | | | **¹³C NMR (101 MHz, DMSO-*d₆*) δ :** 5.3 (CH_{cPr}), 6.6 (2xCH_{2 cPr}), 16.4 (CH₂), 34.0 (CH₂), 34.8 (CH₂), 37.1 (C_{q}), 41.4 (CH_{-NH}), 42.6 (2×CH₂), 47.2 (CH_{2 Bn}), 84.2 (CH_{Ar}), 120.7 (CH_{Ar}), 122.3 (CH_{Ar}), 137.0 (CH_{Ar}), 139.5 (C_{q}), 139.9 (C_{q}), 149.0 (CH_{Ar}), 150.0 (C_{q}), 155.2 (C_{q}), 157.6 (C_{q}). |
| (206) | 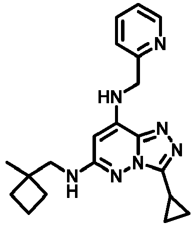 | > 98 % ^{b} | White solid. |
| | | | **Rf** (DCM/MeOH, 94/6) : 0.29 |
| | | | **¹H NMR (400 MHz, DMSO-*d₆*) δ :** 0.99 - 1.15 (m, 7H, 2×CH_{2 cPr} & CH_{3-Cq}), 1.61 (ddt, *J* = 12.0, 6.1, 3.2 Hz, 2H, CH_{2 cBut}), 1.75 - 1.92 (m, 4H, 2xCH_{2 cBut}), 2.27 (tt, *J* = 8.4, 5.1 Hz, 1H, H_{cPr}), 3.18 (d, *J* = 5.6 Hz, 2H, CH_{2-Cq}), 4_{.}52 (d, *J* = 6.2 Hz, 2H, CH_{2Bn}), 5.52 (s, 1H, H_{Ar}), 6.47 (t, *J* = 5.7 Hz, 1H, NH_{-CH2-Cq}), 7.28 (ddd, *J* = 7.5, 4.9, 1.3 Hz, 1H, H_{Ar}), 7.31 (d, *J* = 7.9 Hz, 1H, H_{Ar}), 7.73 - 7.81 (m, 2H, NH_{Bn} & H_{Ar}), 8.55 (ddd, *J* = 4.9, 1.8, 0.9 Hz, 1H, H_{Ar}). |
| | | | **¹³C NMR (101 MHz, DMSO-*d₆*) δ :** 5.3 (CH_{cPr}), 6.6 (2xCH_{2 cPr}), 14.6 (CH_{2 cBut}), 25.3 (CH_{3-Cq}), 30.8 (2×CH_{2cBut}), 38.8 (C_{q-CH3}), 47.1 (CH_{2 Bn}), 50.0 (CH_{2-Cq}), 84.5 (CH_{Ar}), 120.6 (CH_{Ar}), 122.3 (CH_{Ar}), 137.0 (CH_{Ar}), 139.6 (C_{q}), 139.7 (C_{q}), 149.0 (CH_{Ar}), 150.1 (C_{q}), 156.8 (C_{q}), 157.6 (C_{q}). |
| | | | **MS (ESI+) : m/z calcd for C₂₀H₂₅N₇ :** 364.22 [M+H]+, **found** : 364.41. |
| (207) | 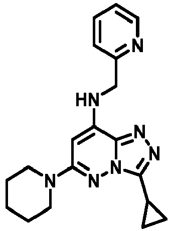 | > 99 %^{b} | Light grey solid. |
| | | | **Rf** (DCM/MeOH, 94/6) : 0.38 |
| | | | **¹H NMR (400 MHz, DMSO-*d₆*) δ :** 0.99 - 1.16 (m, 4H, 2xCH_{2 cPr}), 1.46 - 1.59 (m, 6H, 3×CH_{2 pip}), 2.28 (dq, *J* = 9.0, 5.2, 4.2 Hz, 1H, H_{cPr}), 3.36 - 3.39 (m, 4H, 2xCH_{2 pip}), 4.64 (d, *J* = 6.0 Hz, 2H, CH_{2 Bn}), 5.84 (s, 1H, H_{Ar}), 7.25 - 7.34 (m, 1H, H_{Ar}), 7.36 (d, *J* = 7.9 Hz, 1H, H_{Ar}), 7.77 (t, *J* = 7.7 Hz, 1H, H_{Ar}), 7.90 (t, *J* = 5.9 Hz, 1H, NH_{Bn}), 8.54 (d, *J* = 4.9 Hz, 1H, H_{Ar}). |
| | | | **¹³C NMR (101 MHz, DMSO-*d₆*) δ :** 5.3 (CH_{cPr}), 6.6 (2×CH_{2 cPr}), 24.1 (CH_{2 pip}), 24.9 (2xCH_{2 pip}), 46.7 (2xCH_{2 pip}), 47.1 (CH_{2 Bn}), 83.3 (CH_{Ar}), 121.4 (CH_{Ar}), 122.4 (CH_{Ar}), 137.0 (CH_{Ar}), 139.1 (C_{q}), 140.6 (C_{q}), 148.9 (CH_{Ar}), 150.3 (C_{q}), 157.4 (C_{q}), 157.6 (C_{q}). |
| | | | **MS (ESI+)** : **m/z calcd for C₁₉H₂₃N₇** : 350.21 [M+H]+, **found** : 350.40 |
| (208) | 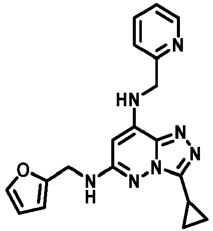 | > 96 % ^{b} | White solid. |
| | | | **¹H NMR (400 MHz, DMSO-*d₆*) δ :** 1.01 -1.15 (m, 4H, 2×CH_{2 cPr}), 2.26 - 2.33 (m, 1H, H_{cPr}), 4.33 (d, *J* = 5.5 Hz, 2H, CH_{2 Bn}), 4.52 (d, *J* = 6.2 Hz, 2H, CH_{2Bn}), 5.43 (s, 1H, H_{Ar}), 6.28 (dd, *J* = 3.2, 0.9 Hz, 1H, H_{Ar}), 6.37 (dd, *J* = 3.2, 1.9 Hz, 1H, H_{Ar}), 7.04 (t, *J* = 5.6 Hz, 1H, NH), 7.28 (ddd, *J* = 7.5, 4.8, 1.2 Hz, 1H, H_{Ar}), 7.31 (dt, *J* = 7.9, 1.1 Hz, 1H, H_{Ar}), 7.55 (dd, *J* = 1.9, 0.9 Hz, 1H, H_{Ar}), 7.75 (td, *J* = 7.7, 1.8 Hz, 1H, H_{Ar}), 7.88 (t, *J* = 6.2 Hz, 1H, NH), 8.54 (ddd, *J* = 4.8, 1.9, 1.0 Hz, 1H, H_{Ar}). |
| | | | **¹³C NMR (101 MHz, DMSO-*d₆*) δ :** 5.3 (CH_{cPr}), 6.6 (2×CH_{cPr}), 37.5 (CH_{2 Bn}), 47.2 (CH_{2 Bn}), 84.2 (CH_{Ar}), 107.1 (CH_{Ar}), 110.4 (CH_{Ar}), 120.7 (CH_{Ar}), 122.3 (CH_{Ar}), 136.9 (CH_{Ar}), 139.5 (C_{q}), 140.1 (C_{q}), 141.9 (CH_{Ar}), 149.0 (CH_{Ar}), 150.2 (C_{q}), 152.5 (C_{q}), 155.6 (C_{q}), 157.5 (C_{q}). |
| | | | **MS (ESI+) : m/z calcd for C₁₉H₁₉N₇O :** 362.42 [M+H]+, **found** : 362.33. |
| (209) | 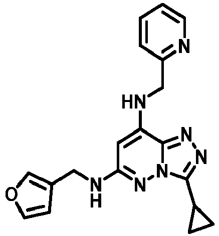 | > 95 %^{b} | White solid. |
| | | | **¹H NMR (400 MHz, DMSO-*d₆*) δ :** 0.95 - 1.20 (m, 4H, 2×CH_{2 cPr}), 2.30 (tt, *J* = 8.3, 5.2 Hz, 1H, H_{cPr}), 4.17 (d, *J* = 5.4 Hz, 2H, CH_{2 Fur}), 4.52 (d, *J* = 6.1 Hz, 2H, CH_{2 Bn}), 5.43 (s, 1H, H_{Ar}), 6.48 (dd, *J* = 1.9, 0.9 Hz, 1H, H_{Ar}), 6.86 (t, *J* = 5.5 Hz, 1H, NH_{-CH2 Fur}), 7.27 (ddd, *J* = 7.6, 4.8, 1.2 Hz, 1H, H_{Ar}), 7.31 (dd, *J* = 7.9, 1.1 Hz, 1H, H_{Ar}), 7.57 (t, *J* = 1.7 Hz, 1H, H_{Ar}), 7.59 (dd, *J* = 1.7, 0.9 Hz, 1H, H_{Ar}), 7.75 (td, *J* = 7.7, 1.8 Hz, 1H, H_{Ar}), 7.86 (t, *J* = 6.2 Hz, 1H, NH_{Bn}), 8.54 (ddd, *J* = 4.8, 1.8, 0.9 Hz, 1H, H_{Ar}). |
| | | | **¹³C NMR (101 MHz, DMSO-*d₆*) δ :** 5.2 (CH_{cPr}), 6.7 (2×CH_{2 cPr}), 35.5 (CH_{2 Fur}), 47.2 (CH_{2 Bn}), 84.4 (CH_{Ar}), 111.0 (CH_{Ar}), 120.7 (CH_{Ar}), 122.3 (CH_{Ar}), 122.9 (C_{q}), 136.9 (CH_{Ar}), 139.6 (C_{q}), 140.0 (C_{q}), 140.2 (CH_{Ar}), 143.1 (CH_{Ar}), 149.0 (CH_{Ar}), 150.2 (C_{q}), 155.9 (C_{q}), 157.5 (C_{q}). |
| | | | **MS (ESI+) : m/z calcd for C₁₉H₁₉N₇O** : 362.17 [M+H]+, **found** : 362.31. |
| (210) | 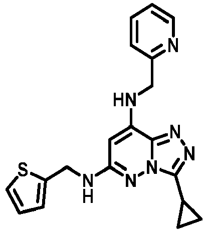 | > 98 %^{b} | White solid. |
| | | | **¹H NMR (400 MHz, DMSO-*d₆*) δ :** 1.00 - 1.17 (m, 4H, 2×CH_{2 cPr}), 2.29 - 2.37 (m, 1H, H_{cPr}), 4.52 (d, *J* = 5.3 Hz, 4H, 2×CH_{2-NH}), 5.40 (s, 1H, H_{Ar}), 6.93 (dd, *J* = 5.1, 3.4 Hz, 1H, H_{Ar}), 7.02 (dd, *J* = 3.4, 1.2 Hz, 1H, H_{Ar}), 7.17 (t, *J* = 5.9 Hz, 1H, NH_{Bn}), 7.27 (ddd, *J* = 7.6, 4.8, 1.2 Hz, 1H, H_{Ar}), 7.32 (d, *J* = 7.9 Hz, 1H, H_{Ar}), 7.34 (dd, *J* = 5.1, 1.3 Hz, 1H, H_{Ar}), 7.75 (td, *J* = 7.7, 1.8 Hz, 1H, H_{Ar}), 7.90 (t, *J* = 6.2 Hz, 1H, NH_{Bn}), 8.54 (dt, *J* = 4.7, 1.6 Hz, 1H, H_{Ar}). |
| | | | **¹³C NMR (101 MHz, DMSO-*d₆*) δ :** 5.3 (CH_{cPr}), 6.7 (2×CH_{2 cPr}), 39.2 (CH_{2-NH}), 47.2 (CH_{2-NH}), 84.2 (CH_{Ar}), 120.8 (CH_{Ar}), 122.3 (CH_{Ar}), 125.0 (CH_{Ar}), 125.8 (CH_{Ar}), 126.4 (CH_{Ar}), 136.9 (CH_{Ar}), 139.5 (C_{q}), 140.1 (C_{q}), 142.6 (C_{q}), 149.0 (CH_{Ar}), 150.3 (C_{q}), 155.5 (C_{q}), 157.5 (C_{q}). |
| | | | **MS (ESI+) : m/z calcd for C₁₉H₁₉N₇S :** 378.15 [M+H]+, **found** : 378.28. |
| (211) | 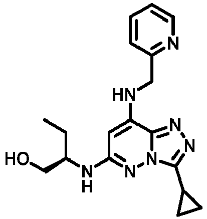 | > 99 %^{b} | Light yellow solid. |
| | | | **¹H NMR (400 MHz, DMSO-*d₆*) δ :** 0.85 (t, *J* = 7.4 Hz, 3H, CH_{3-CH2}), 1.01 - 1.15 (m, 4H, 2xCH_{2 cPr}), 1.32 - 1.69 (m, 2H, CH_{2-CH3}), 2.25 (tt, *J* = 8.5, 5.1 Hz, 1H, H_{ePr}), 3.33 - 3.47 (m, 2H, CH_{2-OH}), 3.63 (dtt, *J* = 10.6, 7.6, 3.8 Hz, 1H, H_{C-NH}), 4.51 (d, *J* = 6.2 Hz, 2H, CH_{2 Bn}), 4.56 (t, *J* = 5.5 Hz, 1H, OH), 5.45 (s, 1H, H_{Ar}), 6.33 (d, *J* = 7.9 Hz, 1H, NH_{-CH}), 7.28 (ddd, *J* = 7.5, 4.8, 1.2 Hz, 1H, H_{Ar}), 7.32 (dt, *J* = 7.9, 1.1 Hz, 1H, H_{Ar}), 7.74 - 7.80 (m, 2H, NH_{Bn} & H_{Ar}), 8.55 (ddd, *J* = 4.8, 1.9, 0.9 Hz, 1H, H_{Ar}). |
| | | | **¹³C NMR (101 MHz, DMSO-*d₆*) δ :** 5.3 (CH_{cPr}), 6.5 (CH_{2 cPr}), 6.5 (CH_{2 cPr}), 10.5 (CH_{3-CH2}), 23.6 (CH_{2-CH3}), 47.2 (CH_{2 Bn}), 53.7 (CH_{-NH}), 62.1 (CH_{2-OH}), 84.6 (CH_{Ar}), 120.7 (CH_{Ar}), 122.3 (CH_{Ar}), 136.9 (CH_{Ar}), 139.5 (C_{q}), 139.8 (C_{q}), 149.0 (CH_{Ar}), 150.0 (C_{q}), 156.1 (C_{q}), 157.6 (C_{q}). |
| | | | **MS (ESI+) : m/z calcd for C₁₈H₂₃N₇O :** 354.20 [M+H]+, **found** : 354.36. |
| (212) | 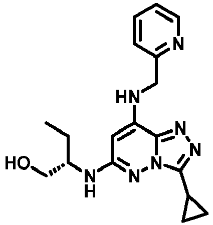 | > 98 %^{b} | White solid. |
| | | | **¹H NMR (400 MHz, DMSO-*d₆*) δ :** 0.85 (t, *J* = 7.4 Hz, 3H, CH_{3-CH2}), 1.00 - 1.15 (m, 4H, 2xCH_{2 cPr}), 1.33 - 1.69 (m, 2H, CH_{2-CH3}), 2.25 (tt, *J* = 8.4, 5.1 Hz, 1H, H_{ePr}), 3.33 - 3.48 (m, 2H, CH_{2-CH3}), 3.64 (tq, *J* = 7.5, 5.3 Hz, 1H, H_{C-NH}), 4.51 (d, *J* = 6.2 Hz, 2H, CH_{2Bn}), 4.56 (t, *J* = 5.4 Hz, 1H, OH), 5.45 (s, 1H, H_{Ar}), 6.32 (d, *J* = 7.9 Hz, 1H, NH_ _{CH}), 7.28 (ddd, *J* = 7.7, 4.8, 1.1 Hz, 1H, H_{Ar}), 7.32 (d, *J* = 7.9 Hz, 1H, H_{Ar}), 7.73 - 7.81 (m, 2H, NH_{Bn} & H_{Ar}), 8.55 (dt, *J* = 4.7, 1.4 Hz, 1H, H_{Ar}). |
| | | | **¹³C NMR (101 MHz, DMSO-*d₆*) δ :** 5.3 (CH_{cPr}), 6.5 (CH_{2 cPr}), 6.5 (CH_{2 cPr}), 10.5 (CH_{3-CH2}), 23.6 (CH_{2-CH3}), 47.2 (CH_{2 Bn}), 53.7 (CH_{-NH}), 62.1 (CH_{2-OH}), 84.6 (CH_{Ar}), 120.7 (CH_{Ar}), 122.3 (CH_{Ar}), 136.9 (CH_{Ar}), 139.5 (C_{q}), 139.8 (C_{q}), 149.0 (CH_{Ar}), 150.0 (C_{q}), 156.1 (C_{q}), 157.6 (C_{q}). |
| | | | **MS (ESI+) : m/z calcd for C₁₈H₂₃N₇O :** 354.20 [M+H]+, **found** : 354.36. |
| (213) | 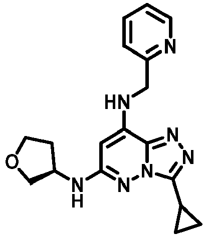 | > 98 % ^{b} | White solid. |
| | | | ¹**H NMR (400 MHz**, **DMSO**-***d₆*)** δ : 1.01 - 1.18 (m, 4H, 2xCH_{2 c}pᵣ), 1.71 - 1.82 (m, 1H, H_{(CH2)}), 2.13 (dq, *J* = 12.6, 7.6 Hz, 1H, H(_{CH2})), 2.28 (tt, *J* = 8.5, 5.1 Hz, 1H, H_{cPr}), 3.50 (dd, *J* = 8.9, 4.0 Hz, 1H, H(_{CH2})), 3.62 - 3.81 (m, 2H, CH₂), 3.86 (dd, *J* = 8.9, 5.9 Hz, 1H, H(_{CH2})), 4.17 (dtt, *J* = 8.1, 5.8, 4.2 Hz, 1H, H_{C-NH}), 4.51 (d, *J* = 6.2 Hz, 2H, CH_{2 Bn}), 5.36 (s, 1H, H_{Ar}), 6.85 (d, *J =* 5.6 Hz, 1H, NH_{THF}), 7.28 (ddd, *J* = 7.6, 4.8, 1.2 Hz, 1H, H_{Ar}), 7.31 (dd, *J* = 7.8, 1.1 Hz, 1H, H_{Ar}), 7.76 (td, *J* = 7.7, 1.8 Hz, 1H, H_{Ar}), 7.88 (t, *J=* 6.2 Hz, 1H, NH_{Bn}), 8.55 (ddd, *J* = 4.9, 1.8, 0.9 Hz, 1H, H_{Ar}). |
| | | | ¹³**C** NMR (101 MHz, **DMSO**-***d₆***) *δ* : 5.3 (CH_{cPr}), 6.4 (CH_{2 cPr}), 6.5 (CH_{2 cPr}), 32.1 (CH_{2THF}), 47.2 (CH_{2 Bn}), 51.4 (CH_{THF}), 66.3 (CH_{2 THF}), 72.7 (CH_{2 THF}), 84.2 (CH_{Ar}), 120.7 (CH_{Ar}), 122.3 (CH_{Ar}), 137.0 (CH_{Ar}), 139.5 (C_{q}), 140.0 (C_{q}), 149.0 (CH_{Ar}), 150.1 (C_{q}), 155.7 (C_{q}), 157.6 (C_{q}). |
| | | | MS (ESI+) : m/z calcd for **C₁₈H₂₁N₇O :** 352.19 [M+H]+, found : 352.82. |
| (214) | 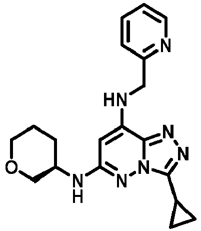 | > 97 % ^{b} | White solid. |
| | | | **¹H NMR (400 MHz, DMSO-*d₆*) δ :** 1.01 - 1.15 (m, 4H, 2xCH_{2 cPr}), 1.37 - 1.59 (m, 2H, 2×H_{(CH2) THP}), 1.68 (dd, *J* = 13.5, 4.8 Hz, 1H, H_{(CH2) THP}), 1.91 (dd, *J* = 10.5, 5.8 Hz, 1H, H_{(CH2) THP}), 2.25 (tt, *J* = 8.4, 5.1 Hz, 1H, H_{cPr}), 3.06 (dd, *J* = 10.8, 8.3 Hz, 1H, H_{(CH2) THP}), 3.29 (d, *J* = 2.9 Hz, 1H, H(_{CH2}) _{THP}), 3.68 (dt, *J* = 11.1, 4.1 Hz, 2H, H(_{CH2}) _{THP} & H _{THP}), 3.90 (ddd, *J* = 10.7, 4.0, 1.5 Hz, 1H, H_{(CH2) THP}), 4.51 (d, *J* = 6.2 Hz, 2H, CH_{2 Bn}), 5.41 (s, 1H, H_{Ar}), 6.53 (d, *J* = 7.3 Hz, 1H, NH_{THP}), 7.28 (ddd, *J* = 7.5, 4.8, 1.1 Hz, 1H, H_{Ar}), 7.31 (dt, *J* = 7.8, 1.1 Hz, 1H, H_{Ar}), 7.76 (td, *J* = 7.7, 1.8 Hz, 1H, H_{Ar}), 7.85 (t, *J* = 6.2 Hz, 1H, NH_{Bn}), 8.55 (ddd, *J* = 4.9, 1.8, 1.0 Hz, 1H, H_{Ar}). |
| | | | **¹³C NMR (101 MHz, DMSO**-***d₆*) δ :** 5.3 (CH_{cPr}), 6.5 (CH_{2 cPr}), 6.7 (CH_{2 cPr}), 24.3 (CH_{2 THP}), 28.8 (CH_{2 THP}), 46.5 (CH_{THP}), 47.2 (CH_{2 Bn}), 67.1 (CH_{2 THP}), 70.3 (CH_{2 THP}), 84.4 (CH_{Ar}), 120.6 (CH_{Ar}), 122.3 (CH_{Ar}), 137.0 (CH_{Ar}), 139.5 (C_{q}), 140.0 (C_{q}), 149.1 (CH_{Ar}), 150.1 (C_{q}), 155.4 (C_{q}), 157.6 (C_{q}). |
| | | | **MS (ESI+) : m/z calcd for C₁₉H₂₃N₇O :** 366.20 [M+H]+, **found :** 366.34. |
| (215) | 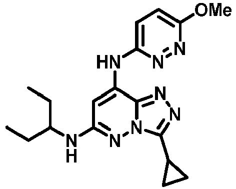 | - | Beige solid. |
| | | | **¹H NMR (400 MHz, DMSO**-***d*₆) δ :** 0.90 (t, *J* = 7.4 Hz, 6H, 2xCH_{3-CH2}), 1.03 - 1.11 (m, 2H, CH_{2 cPr}), 1.17 (dt, *J* = 5.7, 2.9 Hz, 2H, CH_{2 cPr}), 1.42 - 1.66 (m, 4H, 2×CH_{2-CH3}), 2.27 - 2.32 (m, 1H, H_{cPr}), 3.67 (dq, J = 13.2, 7.4, 6.8 Hz, 1H, H_{iPent}), 3.98 (s, 3H, CH₃₋₀), 7.02 (d, *J* = 8.0 Hz, 1H, NH_{iPent}), 7.24 (d, *J* = 9.4 Hz, 1H, H_{Ar}), 7.78 (d, *J* = 9.5 Hz, 1H, H_{Ar}), 7.90 (s, 1H, H_{Ar}), 10.00 (s, 1H, NH). |
| | | | **¹³C NMR (101 MHz, DMSO-*d*₆) δ :** 5.4 (CH_{cPr}), 6.5 (2xCH_{2 cPr}), 10.4 (2×CH_{3-CH2}), 26.0 (2×CH_{2-CH3}), 53.1 (CH_{iPent}), 54.1 (CH_{3-O}), 95.2 (CH_{Ar}), 119.6 (CH_{Ar}), 123.5 (CH_{Ar}), 133.0 (C_{q}), 139.2 (C_{q}), 150.3 (C_{q}), 154.2 (C_{q}), 156.2 (C_{q}), 160.9 (C_{q}). |
| (216) | 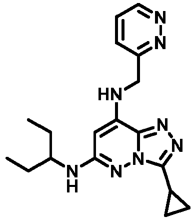 | > 97 % ^{b} | Light brown solid. |
| | | | **Rf** (DCM/MeOH, 96/4) : 0.19 |
| | | | **¹H NMR (400 MHz, DMSO-*d₆*) δ :** 0.82 (t, *J* = 7.4 Hz, 6H, 2xCH_{3-CH2}), 0.99 - 1.16 (m, 4H, 2xCH_{2 c}pᵣ), 1.33 - 1.58 (m, 4H, 2xCH_{2-CH3}), 2.24 (tt, *J* = 8.4, 5.1 Hz, 1H, H_{cPr}), 3.54 (hept, *J* = 5.8, 5.1 Hz, 1H, H_{iPent}), 4.73 (d, *J* = 6.3 Hz, 2H, CH_{2 Bn}), 5.46 (s, 1H, H_{Ar}), 6.39 (d, *J* = 8.0 Hz, 1H, NH_{iPent}), 7.63 (dd, *J* = 8.5, 1.8 Hz, 1H, H_{Ar}), 7.68 (dd, *J* = 8.5, 4.8 Hz, 1H, H_{Ar}), 7.90 (t, *J* = 6.4 Hz, 1H, NH_{Bn}), 9.16 (dd, *J* = 4.8, 1.8 Hz, 1H, H_{Ar}). |
| | | | **¹³C NMR (101 MHz, DMSO-de) δ :** 5.4 (CH_{cPr}), 6.4 (2xCH_{2 cPr}), 10.3 (2×CH_{3-CH2}), 26.0 (2×CH_{2-CH3}), 45.7 (CH_{2 Bn}), 52.8 (CH_{iPent}), 84.8 (CH_{Ar}), 125.0 (CH_{Ar}), 127.5 (CH_{Ar}), 139.4 (C_{q}), 139.6 (C_{q}), 149.9 (C_{q}), 150.9 (CH_{Ar}), 156.0 (C_{q}), 160.3 (C_{q}). |
| | | | **MS (ESI+) : m/z calcd for C₁₈H₂₄N₈ :** 353.22 [M+H]+, **found :** 353.32. |
| (217) | 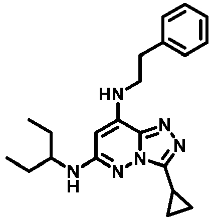 | - | White cloudy oil. |
| | | | Rf (DCM/MeOH, 96/4) : 0.47 |
| | | | **¹H NMR (400 MHz, DMSO**-***d₆*) δ :** 0.88 (t, *J* = 7.4 Hz, 6H, 2xCH_{3-CH2}), 0.99 - 1.15 (m, 4H, 2xCH_{2 cPr}), 1.40 - 1.62 (m, 4H, 2xCH_{2-CH3}), 2.24 (tt, *J* = 8.4, 5.1 Hz, 1H, H_{cPr}), 2.91 (dd, *J* = 8.8, 6.4 Hz, 2H, CH_{2-CH2-NH}), 3.40 (q, *J* = 6.8 Hz, 2H, CH_{2-NH}), 3.62 (h, *J* = 6.6 Hz, 1H, H_{iPent}), 5.61 (s, 1H, H_{Ar}), 6.30 (d, *J* = 8.1 Hz, 1H, NH_{iPent}), 7.15 - 7.25 (m, 2H, H_{Ar} & NH_{-CH2}), 7.27 - 7.37 (m, 4H, 4×H_{Ar}). |
| | | | **¹³C NMR (101 MHz, DMSO-*d₆*) δ :** 5.4 (CH_{cPr}), 6.4 (2xCH_{2 cPr}), 10.4 (2×CH_{3-CH2}), 26.2 (2×CH_{2-cH3}), 33.8 (CH_{2-CH2-NH}), 43.6 (CH_{2-NH}), 52.8 (CH_{iPent}), 83.6 (CH_{Ar}), 126.2 (CH_{Ar}), 128.4 (2×CH_{Ar}), 128.7 (2xCH_{Ar}), 139.2 (C_{q}), 139.5 (C_{q}), 139.7 (C_{q}), 149.8 (C_{q}), 156.3 (C_{q}). |
| (218) | 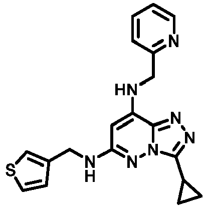 | > 97 %^{b} | White solid. |
| | | | **¹H NMR (400 MHz, DMSO**-***d₆*) δ :** 0.98 - 1.18 (m, 4H, 2xCH_{2 c}pᵣ), 2.30 (tt, *J* = 8.4, 5.2 Hz, 1H, H_{cPr}), 4.35 (d, *J* = 5.5 Hz, 2H, CH_{2-NH}), 4.53 (d, *J* = 6.2 Hz, 2H, CH_{2-NH}), 5.46 (s, 1H, H_{Ar}), 7.01 (t, *J* = 5.7 Hz, 1H), 7.10 (dd, *J* = 4.9, 1.3 Hz, 1H, NH_{-CH2}), 7.28 (ddd, *J* = 7.5, 4.8, 1.2 Hz, 1H, H_{Ar}), 7.32 (d, *J* = 7.8 Hz, 1H, H_{Ar}), 7.35 (dd, *J* = 2.9, 1.2 Hz, 1H, H_{Ar}), 7.46 (dd, *J* = 5.0, 2.9 Hz, 1H, H_{Ar}), 7.76 (td, *J* = 7.7, 1.8 Hz, 1H, H_{Ar}), 7.87 (t, *J* = 6.2 Hz, 1H, NH_{-CH2}), 8.55 (dt, *J* = 4.7, 1.5 Hz, 1H, H_{Ar}). |
| | | | **¹³C NMR (101 MHz, DMSO**-***d₆***) **δ :** 5.3 (CH_{cPr}), 6.6 (2xCH_{2 cPr}), 39.8 (CH_{2-NH}), 47.2 (CH_{2-NH}), 84.4 (CH_{Ar}), 120.7 (CH_{Ar}), 122.1 (CH_{Ar}), 122.3 (CH_{Ar}), 126.0 (CH_{Ar}), 128.0 (CH_{Ar}), 136.9 (CH_{Ar}), 139.6 (C_{q}), 140.0 (C_{q}), 140.3 (C_{q}), 149.0 (CH_{Ar}), 150.2 (C_{q}), 155.9 (C_{q}), 157.5 (C_{q}). |
| | | | MS (ESI+) : m/z calcd for C₁₉H₁₉N₇S : 378.15 [M+H]+, found : 378.29. |
| (219) | 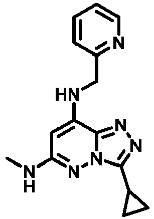 | > 98 % ^{b} | White solid |
| | | | **Rf** (DCM/MeOH, 94/6) : 0.15 |
| | | | **¹H NMR (400 MHz, DMSO**-***d₆***) **δ :** 1.02 - 1.13 (m, 4H, 2xCH_{2 cPr}), 2.28 (tt, *J* = 8.4, 5.2 Hz, 1H, H_{cPr}), 2.70 (d, *J* = 4.8 Hz, 3H, CH_{3-NH}), 4.52 (d, *J* = 6.2 Hz, 2H, CH_{2 Bn}), 5.34 (s, 1H, H_{Ar}), 6.56 (q, *J* = 4.7 Hz, 1H, NH_{-CH3}), 7.28 (ddd, *J* = 7.5, 4.9, 1.2 Hz, 1H, H_{Ar}), 7.32 (dd, *J* = 7.8, 1.1 Hz, 1H, H_{Ar}), 7.75 (td, *J* = 7.7, 1.8 Hz, 1H, H_{Ar}), 7.81 (t, *J* = 6.2 Hz, 1H, NH_{Bn}), 8.54 (ddd, *J* = 4.9, 1.8, 1.0 Hz, 1H, H_{Ar}). |
| | | | **¹³C NMR (101 MHz, DMSO-*d₆*) δ :** 5.2 (CH_{cPr}), 6.8 (2xCH_{2 cPr}), 27.6 (CH_{3-NH}), 47.2 (CH_{2 Bn}), 84.4 (CH_{Ar}), 120.8 (CH_{Ar}), 122.3 (CH_{Ar}), 136.9 (CH_{Ar}), 139.5 (C_{q}), 139.7 (C_{q}), 149.0 (CH_{Ar}), 150.2 (C_{q}), 156.7 (C_{q}), 157.6 (C_{q}). |
| | | | **MS (ESI+) : m/z calcd for C₁₅H₁₇N₇ :** 296.16 [M+H]+, **found :** 296.31. |
| (220) | 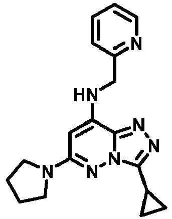 | > 98 % ^{b} | White solid |
| | | | **¹H NMR (400 MHz, DMSO**-***d₆*) δ :** 1.01 - 1.15 (m, 4H, 2×CH_{2 cPr}), 1.86 - 1.91 (m, 4H, 2×CH_{2-CH2-N}), 2.28 (tt, *J* = 8.5, 5.1 Hz, 1H, H_{cPr}), 3.29 (d, *J* = 6_{.}7 Hz, 4H, 2×CH_{2- N}), 4.62 (d, *J* = 6.1 Hz, 2H, CH_{2 Bn}), 5.51 (s, 1H, H_{Ar}), 7.23 - 7.33 (m, 1H, H_{Ar}), 7.38 (d, *J* = 7.8 Hz, 1H, H_{Ar}), 7.76 (td, *J* = 7.7, 1.8 Hz, 1H, H_{Ar}), 7.93 (t, *J* = 6.1 Hz, 1H, NH_{Bn}), 8.48 - 8.65 (m, 1H, H_{Ar}). |
| | | | **¹³C NMR (101 MHz, DMSO**-***d₆*) δ :** 5.3 (CH_{cPr}), 6.6 (2xCH_{2 cPr}), 24.8 (2×CH_{2-CH2-N}), 46.6 (2×CH_{2-N}), 47.3 (CH_{2 Bn}), 82.9 (CH_{Ar}), 121.4 (CH_{Ar}), 122.4 (CH_{Ar}), 136.9 (CH_{Ar}), 139.1 (C_{q}), 140.3 (C_{q}), 148.9 (CH_{Ar}), 149.9 (C_{q}), 155.0 (C_{q}), 157.6 (C_{q}). |
| | | | **MS (ESI+) : m/z calcd for C₁₈H₂₁N₇ :** 336.19 [M+H]+, **found :** 336.36 |

| | | | |
|---|---|---|---|
| ^{a}Acidic conditions, ^{b}Alkaline conditions | | | |

### PATHOLOGIES

Examples of diseases mediated A₃AR include, but are not limited to, ulcerative colitis, atopic dermatitis, glaucoma, asthma, liver fibrosis, kidney diseases in particular kidney fibrosis, nephropathy, acute kidney injury (AKI), chronic kidney disease (CKD) more particularly induced by ischemia and/or reperfusion injury, toxic nephropathy or myoglobinuria, diabetic kidney disease, kidney diseases induced by nephrotoxicity caused by therapeutic drugs, atherosclerosis, hypercholestemia, partial or a complete hearing loss induced by noise, acoustic trauma or ototoxic agents or conditions, and cancers developing under hypoxic conditions, in particular glioblastoma.

The compounds of formula (I) or any of its pharmaceutically acceptable salts, may in particular be useful in the treatment and/or in the prevention of hearing loss, in particular induced by ototoxic agents, as well as in the treatment and/or in the prevention of kidney diseases, in particular induced by nephrotoxicity.

The compounds of formula (I) or any of its pharmaceutically acceptable salts may in particular be useful in the treatment and/or in the prevention of partial or a complete hearing loss, in particular induced by noise, acoustic trauma or ototoxic agents or conditions, more particularly induced by ototoxic agents or conditions.

The compounds of formula (I) or any of its pharmaceutically acceptable salts may in particular be useful in the treatment and/or in the prevention of partial or a complete hearing loss induced by ototoxic agents or conditions as described hereinafter.

As ototoxic agents, i.e. causing hear loss, the following may be cited:
- solvents, such as polyethylene glycol, propylene glycol or benzalkonium chloride;
- antibiotics, in particular topical and/or systemic antibiotics, more particularly aminoglycosides, macrolides or phenicol antibiotics, such as gentamycin, neomycin, tobramycin, kanamycin, nystatin, polymyxin B, amphotericin B, bacitracin or chloramphenicol;
- anticancer drugs, in particular platinum-based anticancer drugs, such as cisplatin or carboplatin,
- antiseptics, in particular acetic acid, alcohols such as ethanol, chlorhexidine, cresylate, gentian violet or povidone iodine;
- nonsteroidal anti-inflammatory drugs (NSAIDs), in particular salicylates, cyclodextrins, indomethacin, ibuprofen, phenylbutazone or paracetamol;
- topical combinations, in particular polymyxin/neomycin/hydrocortisone or ticarcilline/clavulanate;
- drugs for COVID-19, in particular lopinavir or ritonavir;
- antimalarial drugs, in particular quinine or chloroquine;
- cardiovascular drugs, in particular loop diuretics; or
- erectile dysfunction drugs, in particular phosphodiesterase type 5 (PDE-5) inhibitors.

The compounds of formula (I) or any of its pharmaceutically acceptable salts may in particular be useful in the treatment and/or in the prevention of hearing loss being a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors.

The compounds of formula (I) or any of its pharmaceutically acceptable salts may in particular be useful in the treatment and/or in the prevention of kidney diseases, in particular selected from nephropathy, acute kidney injury (AKI), chronic kidney disease (CKD) more particularly induced by ischemia and/or reperfusion injury, toxic nephropathy or myoglobinuria, and diabetic kidney disease, or kidney diseases induced by nephrotoxicity caused by therapeutic drugs.

The compounds of formula (I) or any of its pharmaceutically acceptable salts may in particular be useful in the treatment and/or in the prevention of kidney diseases induced by nephrotoxicity caused by therapeutic drugs as described hereinafter.

As therapeutic drugs causing nephrotoxicity, the following may be cited: acyclovir, ambisome, amikacin, aminoglycosides, antibiotics, amphotericin B, captopril, carboplatin, cefotaxime, ceftazidime, cefuroxime, cephalosporins, cidofovir, ciprofloxacin, cisplatin, colistimethate, cyclosporine, dapsone, enalaprilat, enalapril, Foscarnet, gadopentetate dimeglumine, gadoxetate, ganciclovir gentamicin, ibuprofen, ifosfamide, iodixanol, iohexol, iopamidol, ioversol, ketorolac, lisinopril, lithium, mesalamine, methotrexate, nafcillin disodium, penicillins, piperacillin/tazobactam, piperacillin, rifampin, sirolimus, sulfasalazine, tacrolimus, ticarcillin/clavulanic acid, tobramycin, topiramate, valacyclovir, valganciclovir, vancomycin or zonisamide.

The following examples illustrate in detail the preparation of some compounds according to the invention. The structures of the products obtained have been confirmed by NMR analyses and mass spectroscopy. The following examples further illustrate some biological activities of some compounds according to the invention.

### Example 1: Synthesis of 3,6-dichloro-pyridazin-4-amine derivatives, as illustrated in step 1 of scheme 1

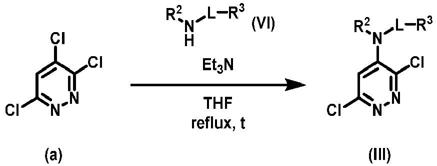

**Example 1.1: Synthesis of *N*-benzyl-3,6-dichloro-pyridazin-4-amine (1.1)** To a solution of 3,4,6-trichloropyridazine **(a)** (10.000 g, 52.88 mmol, 1.0 eq.) in THF (100.0 mL), triethylamine (8.93 mL, 63.46 mmol, 1.2 eq.) then benzylamine (6.48 mL, 58.17 mmol, 1.1 eq.) was added and the mixture was refluxed on 3 h. After cooling, the solid was filtered off, washed with THF then the filtrate was concentrated under vacuum. The resulting solid was triturated in Et₂O, filtered off, washed with a little amount of Et₂O and dried under vacuum to give **(1.1)** (13.000 g, 97 %) as a beige solid.

**¹H NMR (400 MHz, DMSO**-***d₆*) δ :** 4.52 (d, *J* = 6.3 Hz, 2H, CH₂), 6.82 (s, 1H, H_{Ar}), 7.26 (td, *J =* 5.9, 2.8 Hz, 1H, H_{Ar}), 7.34 (d, *J=* 5.4 Hz, 4H, 4×H_{Ar}), 7.97 (t, *J=* 6.3 Hz, 1H, NH). **¹³C NMR (101 MHz, DMSO-*d₆*) δ :** 44.7 (CH₂), 105.5 (CH_{Ar}), 127.0 (2xCH_{Ar}), 127.2 (CH_{Ar}), 128.6 (2xCH_{Ar}), 137.0 (C_{q}), 143.8 (C_{q}), 144.4 (C_{q}), 154.5 (C_{q}).

**MS (ESI+) : m/z calcd for C₁₁H₉Cl₂N₃ :** 254.0 [M+H]+, **found :** 254.0.

### Example 1.2: Synthesis of 3,6-dichloro-N-(2-pyridylmethyl)pyridazin-4-amine (1.2)

To a solution of 3,4,6-trichloropyridazine **(a)** (20.000 g, 105.77 mmol, 1.0 eq.) in THF (210.0 mL), triethylamine (19.36 mL, 137.50 mmol, 1.3 eq.) then corresponding amine (12.67 mL, 121.63 mmol, 1.15 eq.) was added and the mixture was refluxed on 4 h. After cooling, the solid was filtered off and washed with THF. The solid was washed with water then, in another vacuum flask, triturated in MeOH, washed with Et₂O and dried under vacuum to give first part of **(1.2)** (12.500 g). THF and MeOH/Et₂O filtrates were combined and concentrated under vacuum. The resulting solid was triturated in MeOH, filtered off, washed with Et₂O and dried under vacuum to give a second part of **(1.2)** (10.100 g). The filtrate was concentrated and the resulting solid triturated in a little amount of MeOH, filtered off, washed with Et₂O and dried under vacuum to give a third part of **(1.2)** (1.000 g). Total of **(1.2):** 23.600 g, 87 % as a white solid.

**¹H NMR (400 MHz, DMSO**-***d₆*) δ :** 4.60 (d, *J* = 6.1 Hz, 2H, CH_{2 Bn}), 6.90 (s, 1H, H_{Ar}), 7.31 (ddd, *J* = 7.5, 4.8, 1.1 Hz, 1H, H_{Ar}), 7.35 (dt, *J* = 7.8, 1.1 Hz, 1H, H_{Ar}), 7.79 (td, *J* = 7.7, 1.8 Hz, 1H, H_{Ar}), 7.87 (t, *J* = 6.0 Hz, 1H, NH_{Bn}), 8.54 (ddd, *J* = 4.9, 1.9, 0.9 Hz, 1H).

**¹³C NMR (101 MHz, DMSO-*d₆*) δ :** 46.6 (CH_{2 Bn}), 105.9 (CH_{Ar}), 121.6 (CH_{Ar}), 122.7 (CH_{Ar}), 137.1 (CH_{Ar}), 143.8 (C_{q}), 144.7 (C_{q}), 149.2 (CH_{Ar}), 154.6 (C_{q}), 156.3 (C_{q}).

**MS (ESI+) : m/z calcd for C₁₀H₈Cl₂N₄ :** 255.0 [M+H]+, **found :** 255.1.

### Example 1.3: Synthesis of 3,6-dichloro-N-(3-pyridylmethyl)pyridazin-4-amine (1.3)

To a solution of 3,4,6-trichloropyridazine **(a)** (3.100 g, 16.39 mmol, 1.0 eq.) in THF (33.0 mL), triethylamine (3.00 mL, 21.31 mmol, 1.3 eq.) then corresponding amine (2.04 mL, 19.67 mmol, 1.2 eq.) was added and the mixture was refluxed on 2 h. After cooling, the solid was filtered off and washed with THF. The filtrate was concentrated and the resulting solid was washed with water, then triturated with a little amount of MeOH and dried under vacuum to give **(1.3)** (2.320 g, 55 %) as a pale orange solid.
**Rf** (DCM/MeOH, 94/6) : 0.55

**¹H NMR (400 MHz, DMSO**-***d₆*) δ :** 4.56 (d, *J* = 6.3 Hz, 2H, CH_{2 Bn}), 6.96 (s, 1H, H_{Ar}), 7.37 (dd, *J* = 7.8, 4.7 Hz, 1H, H_{Ar}), 7.73 (dt, *J* = 7.8, 2.0 Hz, 1H, H_{Ar}), 7.96 (t, *J* = 6.3 Hz, 1H, NH_{Bn}), 8.48 (dd, *J* = 4.8, 1.7 Hz, 1H, H_{Ar}), 8.59 (d, *J* = 2.3 Hz, 1H, H_{Ar}).

**¹³C NMR (101 MHz, DMSO**-***d₆*) δ :** 42.4 (CH_{2 Bn}), 105.5 (CH_{Ar}), 123.6 (CH_{Ar}), 132.7 (C_{q}), 134.9 (CH_{Ar}), 143.9 (C_{q}), 144.3 (C_{q}), 148.5 (CH_{Ar}), 148.8 (CH_{Ar}), 154.7 (C_{q}).

**MS (ESI+) : m/z calcd for C₁₀H₈Cl₂N₄ :** 255.0 [M+H]+, **found :** 255.1.

### Example 1.4: Synthesis of 3,6-dichloro-N-(4-pyridylmethyl)pyridazin-4-amine (1.4)

To a solution of 3,4,6-trichloropyridazine **(a)** (11.500 g, 60.82 mmol, 1.0 eq.) in THF (120.0 mL), triethylamine (11.13 mL, 79.06 mmol, 1.3 eq.) then corresponding amine (7.640 g, 69.94 mmol, 1.15 eq.) was added and the mixture was refluxed on 2 h. After cooling, the solid was filtered off and washed with THF. The filtrate was concentrated and the resulting solid was washed with water, then triturated with a little amount of MeOH and dried under vacuum to give **(1.4)** (2.320 g, 55 %) as a pale orange solid.

¹**H NMR (400 MHz**, **DMSO**-***d₆*) δ :** 4.57 (d, *J* = 6.4 Hz, 2H, CH_{2 Bn}), 6.84 (s, 1H, HAr), 7.27 - 7.36 (m, 2H, 2×HAr), 7.98 (t, *J* = 6.4 Hz, 1H, NH_{Bn}), 8.45 - 8.58 (m, 2H, 2×HAr).

**¹³C NMR (101 MHz, DMSO**-***d₆*) δ :** 43.7 (CH_{2 Bn}), 105.6 (CH_{Ar}), 122.0 (2xCH_{Ar}), 143.9 (C_{q}), 144.5 (C_{q}), 146.3 (C_{q}), 149.8 (2×CH_{Ar}), 154.7 (C_{q}).

**MS (ESI+) : m/z calcd for C₁₀H₈Cl₂N₄ :** 255.0 [M+H]+, **found :** 255.1.

### Example 1.5: Synthesis of 4-[[(3,6-dichloropyridazin-4-yl)amino]methyl]phenol (1.5)

In a sealed vial 2-5 mL with a stir bar was charged 3,4,6-trichloropyridazine **(a)** (1.500 g, 7.93 mmol, 1.0 eq.), DCM (19.0 mL), triethylamine (1.68 mL, 11.90 mmol, 1.5 eq.) and corresponding amine (1.172 g, 9.52 mmol, 1.2 eq.). The vial was sealed and put in heating bloc 15 h at 50 °C. After cooling, the mixture was concentrated with SiO₂ to make solid deposit then directly purified by flash chromatography with DCM/MeOH (98/2 to 96/4) as eluent to give **(1.5)** (0.515 g, 24 %) as a white solid.

**¹H NMR (400 MHz, DMSO**-***d₆*) δ :** 4.38 (d, *J* = 6.2 Hz, 2H, CH_{2 Bn}), 6.68 - 6.76 (m, 2H, 2xHAr), 6.80 (s, 1H, H_{Ar}), 7.08 - 7.21 (m, 2H, 2xH_{Ar}), 7.87 (t, *J=* 6.2 Hz, 1H, NH_{Bn}), 9.36 (s, 1H, OH).

**¹³C NMR (101 MHz, DMSO**-***d₆*) δ :** 44.3 (CH_{2 Bn}), 105.4 (CH_{Ar}), 115.3 (2xCH_{Ar}), 126.9 (C_{q}), 128.3 (2xCH_{Ar}), 143.8 (C_{q}), 144.3 (C_{q}), 154.5 (C_{q}), 156.6 (C_{q}).

**MS (ESI+) : m/z calcd for C₁₁H₉Cl₂N₃O :** 270.0 [M+H]+, **found :** 270.1.

### Example 1.6: Synthesis of 3-[[(3,6-dichloropyridazin-4-yl)amino]methyl]phenol (1.6)

In a sealed vial 2-5 mL with a stir bar was charged 3,4,6-trichloropyridazine **(a)** (1.500 g, 7.93 mmol, 1.0 eq.), DCM (19.0 mL), triethylamine (1.68 mL, 11.90 mmol, 1.5 eq.) and corresponding amine (1.172 g, 9.52 mmol, 1.2 eq.). The vial was sealed and put in heating bloc 15 h at 50 °C. After cooling, the mixture was concentrated with SiO₂ to make solid deposit then directly purified by flash chromatography with DCM/MeOH (98/2 to 96/4) as eluent to give **(1.6)** (0.670 g, 31 %) as a white solid.

**¹H NMR (400 MHz, DMSO-*d*₆) δ :** 4.43 (d, *J* = 6.3 Hz, 2H, CH_{2 Bn}), 6.64 (dd, *J* = 8.0, 2.4 Hz, 1H, H_{Ar}), 6.69 (t, *J* = 2.0 Hz, 1H, H_{Ar}), 6.74 (d, *J* = 7.6 Hz, 1H, H_{Ar}), 6.77 (s, 1H, H_{Ar}), 7.13 (t, *J* = 7.8 Hz, 1H, H_{Ar}), 7.94 (t, *J* = 6.3 Hz, 1H, NH_{Bn}), 9.39 (s, 1H, OH).

**¹³C NMR (101 MHz, DMSO**-***d₆*) δ :** 44.6 (CH_{2 Bn}), 105.5 (CH_{Ar}), 113.4 (CH_{Ar}), 114.2 (CH_{Ar}), 117.4 (CH_{Ar}), 129.6 (CH_{Ar}), 138.4 (C_{q}), 143.7 (C_{q}), 144.4 (C_{q}), 154.5 (C_{q}), 157.6 (C_{q}).

**MS (ESI+) : m/z calcd for C₁₁H₉Cl₂N₃O :** 270.0 [M+H]+, **found :** 270.1.

### Example 1.7: Synthesis of 2-[[(3,6-dichloropyridazin-4-yl)amino]methyl]phenol (1.7)

To a solution of 3,4,6-trichloropyridazine **(a)** (3.700 g, 20.2 mmol, 1.0 eq.) in THF (50 mL), triethylamine (3.41 mL, 24.2 mmol, 1.2 eq.) then corresponding benzylamine (3.700 g, 24.2 mmol, 1.2 eq.) was added and the mixture was refluxed 3 h. After cooling, the mixture was filtered, washed with THF then the filtrate was concentrated, the crude residue was triturated in DCM/MeOH (94/4) to give a first part of **(1.7)** (1.450 g) after filtration. The filtrate was concentrated and the residue triturated in DCM/MeOH (98/2) to give a second part of **(1.7)** (0.950 g) after filtration. The filtrate was concentrated and purified by flash chromatography (96/4) to give a third part of **(1.7)** (0.900 g). Total of **(1.7):** 3.400g, 62 %, as a yellow solid.

**¹H NMR (400 MHz, DMSO-*d*₆) δ :** 4.39 (d, *J* = 6.2 Hz, 2H, CH_{2 Bn}), 6.76 (td, *J* = 7.4, 1.2 Hz, 1H, H_{Ar}), 6.81 (s, 1H, H_{Ar}), 6.85 (dd, *J* = 8.0, 1.2 Hz, 1H, H_{Ar}), 7.06 - 7.14 (m, 2H, 2xH_{Ar}), 7.75 (t, *J* = 6.2 Hz, 1H, NH_{Bn}), 9.84 (s, 1H, OH).

**¹³C NMR (101 MHz, DMSO**-***d₆*) δ :** 40.1 (CH_{2 Bn}), 105.3 (CH_{Ar}), 115.1 (CH_{Ar}), 119.2 (CH_{Ar}), 122.4 (C_{q}), 128.2 (CH_{Ar}), 128.4 (CH_{Ar}), 143.7 (C_{q}), 144.4 (C_{q}), 154.6 (C_{q}), 154.9 (C_{q}).

**MS (ESI+) : m/z calcd for C₁₁H₉Cl₂N₃O :** 270.0 [M+H]+, **found :** 270.1.

### Example 1.8: Synthesis of 3,6-dichloro-N-[(2-methoxyphenyl)methyl]pyridazin-4-amine (1.8)

To a solution of 3,4,6-trichloropyridazine **(a)** (2.600 g, 13.75 mmol, 1.0 eq.) in DCM (70.0 mL), triethylamine (3.87 mL, 27.50 mmol, 2.0 eq.) and corresponding amine (2.887 g, 20.63 mmol, 1.5 eq.) was added and the mixture was refluxed 19 h. After cooling, HCl 1M (50.0 mL) was added then the mixture was vigorously stirred 5 min and layers were separated. Aqueous layer was extracted twice with DCM (2x20.0 mL) then organics layers were combined, dried over MgSO₄, filtered, concentrated and purified by flash chromatography with DCM/MeOH (100/0 to 98/2) as eluent to give **(1.8)** (3.200 g, 82 %) as a white solid.

**¹H NMR (400 MHz, DMSO**-***d*₆) δ :** 3.85 (s, 3H, OCH₃), 4.44 (s, 2H, CH_{2 Bn}), 6.75 (s, 1H, H_{Ar}), 6.87 - 6.95 (m, 1H, H_{Ar}), 7.00 -7.06 (m, 1H, H_{Ar}), 7.13 (dd, *J* = 7.5, 1.8 Hz, 1H, H_{Ar}), 7.27 (td, *J* = 7.8, 1.8 Hz, 1H, H_{Ar}), 7.73 (s, 1H, NH_{Bn}).

**¹³C NMR (101 MHz, DMSO**-***d₆*) δ :** 40.2 (CH_{2 Bn}), 55.4 (CH₃), 105.4 (CH_{Ar}), 110.9 (CH_{Ar}), 120.5 (CH_{Ar}), 124.1 (C_{q}), 127.5 (CH_{Ar}), 128.7 (CH_{Ar}), 143.7 (C_{q}), 144.5 (C_{q}), 154.6 (C_{q}), 156.8 (C_{q}).

**MS (ESI+) : m/z calcd for C₁₂H₁₁Cl₂N₃O :** 284.0 [M+H]+, **found :** 284.1.

### Example 1.9: Synthesis of 3,6-dichloro-N-(1H-indol-3-ylmethyl)pyridazin-4-amine (1.9)

In a 10 - 20 mL vial was introduced a stir bar, 3,4,6-trichloropyridazine **(a)** (1.750 g, 9.25 mmol, 1.0 eq.), DCM (18 mL), 1H-indol-2-ylmethanamine hydrochloride (2.135 g, 11.11 mmol, 1.2 eq.) and triethylamine (2.87 mL, 20.36 mmol, 2.2 eq.). The vial was sealed and put in heating bloc 17 h at 50 °C. After cooling, the mixture was concentrated then triturated in HCl 0.5M (60 mL, pH < 5) and EtOAc was added (30 mL). Heterogenous mixture was vigorously stirred then the precipitate was filtered off, washed with water (up to pH 7) then EtOAc and dried under vacuum to give the first part of **(1.9)** (1.465 g) as a white solid. Aqueous layer was extracted EtOAc (30mL) and organics layers were combined, dried over MgSO₄, filtered, concentrated and purified by flash chromatography with DCM/MeOH (95/5) as eluent to give the second part of **(1.9)** (0.430 g) as a white solid. Total of **(1.9)** : 1.895 g, 70%.

**¹H NMR (400 MHz, DMSO**-***d₆*) δ :** 4.63 (d, *J* = 5.9 Hz, 2H, CH_{2 Bn}), 7.01 (d, *J* = 7.4 Hz, 2H, 2xH_{Ar}), 7.08 (t, *J* = 7.5 Hz, 1H, H_{Ar}), 7.35 (d, *J =* 8.1 Hz, 1H, H_{Ar}), 7.44 (d, *J =* 2.4 Hz, 1H, H_{Ar}), 7.73 (d, *J* = 7.9 Hz, 1H, H_{Ar}), 7.85 (t, *J=* 5.9 Hz, 1H, NH_{Bn}), 10.97 (s, 1H, NH_{Indole}).

**¹³C NMR (101 MHz, DMSO-*d₆*) δ :** 37.7 (CH_{2 Bn}), 105.4 (CH_{Ar}), 109.1 (C_{q}), 111.6 (CH_{Ar}), 118.7 (CH_{Ar}), 118.8 (CH_{Ar}), 121.3 (CH_{Ar}), 124.7 (CH_{Ar}), 126.2 (C_{q}), 136.5 (C_{q}), 143.8 (C_{q}), 144.2 (C_{q}), 154.3 (C_{q}).

**MS (ESI+) : m/z calcd for C₁₃H₁₀Cl₂N₄ :** 293.0 [M+H]+, **found :** 293.1.

### Example 1.10: Synthesis of 3,6-dichloro-N-(1H-indol-2-ylmethyl)pyridazin-4-amine (1.10)

In a 10 - 20 mL vial was introduced a stir bar, 3,4,6-trichloropyridazine **(a)** (1.750 g, 9.25 mmol, 1.0 eq.), DCM (18 mL), 1H-indol-2-ylmethanamine hydrochloride (2.135 g, 11.11 mmol, 1.2 eq.) and triethylamine (2.87 mL, 20.36 mmol, 2.2 eq.). The vial was sealed and put in heating bloc 17 h at 50 °C. After cooling, the mixture was concentrated then triturated in HCl 0.5M (60 mL, pH < 5) and EtOAc was added (30 mL). Heterogenous mixture was vigorously stirred then the precipitate was filtered off, washed with water (up to pH 7) then EtOAc and dried under vacuum to give the first part of **(1.10)** (2.040 g) as a white solid. Aqueous layer was extracted EtOAc (30mL) and organics layers were combined, dried over MgSO₄, filtered, concentrated and purified by flash chromatography with DCM/MeOH (95/5) as eluent to give the second part of **(1.10)** (0.300 g) as a white solid. Total of **(1.10):** 2.340 g, 86 %.

**¹H NMR (400 MHz, DMSO**-***d₆*) δ :** 4.66 (d, *J* = 6.1 Hz, 2H, CH_{2 Bn}), 6.33 (d, *J* = 2.0 Hz, 1H, H_{Ar}), 6.95 (t, *J* = 7.4 Hz, 1H, H_{Ar}), 6.98 (s, 1H, H_{Ar}), 7.04 (t, *J* = 7.4 Hz, 1H, H_{Ar}), 7.33 (d, *J* = 8.0 Hz, 1H, H_{Ar}), 7.45 (d, *J* = 8.0 Hz, 1H, H_{Ar}), 7.79 (t, *J* = 6.1 Hz, 1H, NH_{Bn}), 10.96 (s, 1H, H_{Indole}).

**¹³C NMR (101 MHz, DMSO**-***d₆*) δ :** 39.3 (CH_{2 Bn}), 99.7 (CH_{Ar}), 105.7 (CH_{Ar}), 111.2 (CH_{Ar}), 119.0 (CH_{Ar}), 119.7 (CH_{Ar}), 120.9 (CH_{Ar}), 127.8 (C_{q}), 134.6 (C_{q}), 136.2 (C_{q}), 143.9 (C_{q}), 144.5 (C_{q}), 154.6 (C_{q}).

**MS (ESI+) : m/z calcd for C₁₃H₁₀Cl₂N₄ :** 293.0 [M+H]+, **found :** 293.1.

### Example 1.11: Synthesis of N-[(4-aminophenyl)methyl]-3,6-dichloro-pyridazin-4-amine (1.11)

To a solution of 3,4,6-trichloropyridazine **(a)** (22.953 g, 125.14 mmol, 1.3 eq.) in THF (200.0 mL), triethylamine (20.33 mL, 144.69 mmol, 1.5 eq.) and 4-(aminomethyl)aniline (12.000 g, 96.26 mmol, 1.0 eq.) was added then the mixture was refluxed 18 *N*-[(4-aminophenyl)methyl]-6-chloro-3-isopropyl-[1,2,4]triazolo[4,3-*b*]pyridazin-8-amine h. After cooling, the mixture was filtered off, the solid was washed with THF then the filtrate was concentrated. The residue was triturated in DCM and the solid was filtered off, washed with DCM and dried under vacuum to give **(1.11)** (23.950 g, 92 %) as a yellow solid.

**¹H NMR (400 MHz, DMSO-*d₆*) δ :** 4.30 (d, *J* = 6.1 Hz, 2H, CH₂), 5.09 (s, 2H, NH₂), 6.41 - 6.69 (m, 2H, 2xH_{Ar}), 6.77 (s, 1H, H_{Ar}), 6.89 - 7.12 (m, 2H, 2xH_{Ar}), 7.80 (t, *J* = 6.1 Hz, 1H, NH).

**¹³C NMR (101 MHz, DMSO-*d₆*) δ :** 44.6 (CH₂), 105.4 (CH_{Ar}), 113.9 (2xCH_{Ar}), 123.4 (C_{q}), 128.0 (2xCH_{Ar}), 143.7 (C_{q}), 144.3 (C_{q}), 147.8 (C_{q}), 154.4 (C_{q}).

**MS (ESI+) : m/z calcd for C₁₁H₁₀CI₂N₄ :** 269.0 [M+H]+, **found :** 269.1.

### Example 1.12: Synthesis of N-[(2-aminophenyl)methyl]-3,6-dichloro-pyridazin-4-amine (1.12)

To a solution of 3,4,6-trichloropyridazine **(a)** (5.000 g, 27.3 mmol, 1.0 eq.) in THF (52 mL), triethylamine (4.22 mL, 30.0 mmol, 1.1 eq.) then 2-(aminomethyl)aniline (3.228 g, 25.9 mmol, 0.95 eq.) was added and the mixture was refluxed 3.5 h. After cooling, the mixture was filtered off, the solid was washed with THF then the filtrate was concentrated and directly purified by flash chromatography with DCM/MeOH (98/2 to 96/4) as eluent to give **(1.12)** (5.280 g, 76 %) as a white solid.

**¹H NMR (400 MHz, DMSO-*d*₆) δ :** 4.30 (d, *J* = 6.0 Hz, 2H, CH_{2 Bn}), 5.15 (s, 2H, NH₂), 6.52 (td, *J* = 7.4, 1.3 Hz, 1H, H_{Ar}), 6.65 (dd, *J* = 8.0, 1.3 Hz, 1H, H_{Ar}), 6.74 (s, 1H, H_{Ar}), 6.97 (td, *J* = 7.6, 1.6 Hz, 1H, H_{Ar}), 7.02 (dd, *J* = 7.5, 1.6 Hz, 1H, H_{Ar}), 7.74 (t, *J* = 6.0 Hz, 1H, NH_{Bn}).

**¹³C NMR (101 MHz, DMSO**-***d₆*) δ :** 42.0 (CH_{2 Bn}), 105.6 (CH_{Ar}), 115.2 (CH_{Ar}), 116.1 (CH_{Ar}), 118.8 (C_{q}), 127.9 (CH_{Ar}), 128.1 (CH_{Ar}), 143.8 (C_{q}), 144.4 (C_{q}), 146.3 (C_{q}), 154.5 (C_{q}).

**MS (ESI+) : m/z calcd for C₁₁H₁₀Cl₂N₄ :** 269.0 [M+H]+, **found :** 269.1.

### Example 1.13: Synthesis of N-[(4-bromophenyl)methyl]-3,6-dichloro-pyridazin-4-amine (1.13)

To a solution of 3,4,6-trichloropyridazine **(a)** (4.000 g, 24.81 mmol, 1.0 eq.) in THF (87.0 mL), triethylamine (3.68 mL, 26.17 mmol, 1.2 eq.) and corresponding amine (3.03 mL, 23.99 mmol, 1.1 eq.) was added and the mixture was refluxed 3.5 h. After cooling, the solid was filtered off, washed with THF then the filtrate was concentrated. The residue was taken up in DCM (100.0 mL), washed with HCl 1M (40.0 mL) then organic layer was dried over MgSO₄, filtered, concentrated and purified by flash chromatography with DCM/MeOH (100/0 to 98/2) as eluent to give **(1.13)** (6.500 g, 90 %) as a pale yellow solid.

**¹H NMR (400 MHz, DMSO-*d*₆) δ :** 4.49 (d, *J* = 6.3 Hz, 2H, CH_{2 Bn}), 6.84 (s, 1H, H_{Ar}), 7.25 - 7.35 (m, 2H, 2xH_{Ar}), 7.50 - 7.58 (m, 2H, 2xH_{Ar}), 7.97 (t, *J =* 6.4 Hz, 1H, NH_{Bn}).

**¹³C NMR (101 MHz, DMSO-*d₆*) δ :** 44.0 (CH_{2 Bn}), 105.5 (CH_{Ar}), 120.3 (C_{q}), 129.3 (2xCH_{Ar}), 131.4 (2xCH_{Ar}), 136.6 (C_{q}), 143.8 (C_{q}), 144.3 (C_{q}), 154.6 (C_{q}).

**MS (ESI+) : m/z calcd for C₁₁H₈BrCl₂N₃:** 331.9 [M+H]+, **found :** 331.9.

### Example 1.14: Synthesis of 3,6-dichloro-N-[[4-(2-pyridyl)phenyl]methyl]pyridazin-4-amine (1.14)

To a solution of 3,4,6-trichloropyridazine **(a)** (0.500 g, 2.73 mmol, 1.0 eq.) in THF (13.5 mL), triethylamine (1.15 mL, 8.18 mmol, 3.0 eq.) then [4-(2-pyridyl)phenyl]methanamine (0.603 g, 3.27 mmol, 1.2 eq.) was added and the mixture was refluxed 3 h. After cooling, the mixture was filtered off, the solid was washed with THF then the filtrate was concentrated and directly purified by flash chromatography with DCM/MeOH (99/1 to 95/5) as eluent to give **(1.14)** (0.360 g, 40 %) as a yellow solid.

¹H **NMR (400 MHz, DMSO-*d*₆) δ :** 4.59 (d, *J* = 6.3 Hz, 2H, CH_{2 Bn}), 6.86 (s, 1H, H_{Ar}), 7.33 (ddd, *J* = 7.5, 4.8, 1.2 Hz, 1H, H_{Ar}), 7.45 (d, *J* = 8.1 Hz, 2H, 2xH_{Ar}), 7.86 (td, *J* = 7.7, 1.9 Hz, 1H, H_{Ar}), 7.93 (d, *J* = 8.0 Hz, 1H, H_{Ar}), 8.02 (t, *J* = 6.0 Hz, 1H, NH_{Bn}), 8.04 - 8.10 (m, 2H, 2×H_{Ar}), 8.62 - 8.67 (m, 1H, H_{Ar}).

**¹³C NMR (101 MHz, DMSO**-***d₆*) δ :** 44.5 (CH_{2 Bn}), 105.6 (CH_{Ar}), 120.1 (CH_{Ar}), 122.6 (CH_{Ar}), 126.7 (2×CH_{Ar}), 127.4 (2xCH_{Ar}), 137.2 (CH_{Ar}), 137.7 (C_{q}), 138.0 (C_{q}), 143.9 (C_{q}), 144.4 (C_{q}), 149.5 (CH_{Ar}), 154.6 (C_{q}), 155.7 (C_{q}).

**MS (ESI+) : m/z calcd for C₁₆H₁₂Cl₂N₄:** 331.1 [M+H]+, **found :** 331.1.

### Example 1.15: Synthesis of 2-(3,6-dichloropyridazin-4-yl)-3,4-dihydro-1H-isoquinoline (1.15)

To a solution of 3,4,6-trichloropyridazine **(a)** (5.000 g, 27.3 mmol, 1.0 eq.) in THF (54.0 mL), triethylamine (4.41 mL, 31.3 mmol, 1.1 eq.) then 2-(aminomethyl)aniline (3.228 g, 25.9 mmol, 0.95 eq.) was added and the mixture was refluxed 3.5 h. After cooling, the mixture was filtered off, the solid was washed with THF then the filtrate was concentrated and directly purified by flash chromatography with DCM/EtOAc (100/0 to 92/8) as eluent to give **(1.15)** (6.800 g, 89 %) as a white crystalline solid.

**Rf** (DCM, 100%) : 0.42.

**¹H NMR (400 MHz, DMSO**-***d₆*) δ :** 2.99 (t, *J* = 5.8 Hz, 2H, CH_{2-CH2-N}), 3.69 (t, *J* = 5.8 Hz, 2H, _{N-}CH_{2-CH2}), 4.50 (s, 2H, CH_{2-N}), 7.13 - 7.29 (m, 4H, 4×H_{Ar}), 7.42 (s, 1H, H_{Ar}).

**¹³C NMR (101 MHz, DMSO**-***d₆*) δ :** 28.2 (CH_{2-CH2-N}), 47.5 (_{N-}CH_{2-CH2}), 50.4 (CH_{2-N}), 115.4 (CH_{Ar}), 126.1 (CH_{Ar}), 126.4 (CH_{Ar}), 126.7 (CH_{Ar}), 128.7 (CH_{Ar}), 132.8 (C_{q}), 133.9 (C_{q}), 147.9 (C_{q}), 148.9 (C_{q}), 154.9 (C_{q}).

**MS (ESI+) : m/z calcd for C₁₃H₁₁Cl₂N₃ :** 280.0 [M+H]+, **found :** 280.1.

### Example 1.16: Synthesis of 3,6-dichloro-N-(2-phenylethyl)pyridazin-4-amine (1.16)

To a solution of 3,4,6-trichloropyridazine **(a)** (4.000 g, 21.8 mmol, 1.0 eq.) in THF (42 mL), Et₃N (6.45 mL, 45.8.0 mmol, 2.1 eq.) then corresponding amine hydrochloride (3.438 g, 21.8 mmol, 1.0 eq.) was added and the mixture was refluxed 3 h. After cooling, the mixture was filtered off, the solid was washed with THF then the filtrate was concentrated. The residue was triturated in HCl 1M (25.0 mL) then the resulting solid was filtered, washed with water up to neutral pH and taken up in EtOAc. Organic filtrate was dried over MgSO₄, filtered, concentrated to give (**1.16**) (5.515 g, 88 %) as a beige solid.

**¹H NMR (400 MHz, DMSO**-***d₆***) δ : 2.86 (t, *J* = 7.4 Hz, 2H, CH_{2-CH2-NH}), 3.43 - 3.56 (m, 2H, CH_{2-NH}), 6.95 (s, 1H, H_{Ar}), 7.20 (ddd, *J* = 8.6, 5.6, 2.4 Hz, 1H, H_{Ar}), 7.27 (q, *J* = 4.2 Hz, 5H, NH & 5×H_{Ar}).

**¹³C NMR (101 MHz, DMSO-*d₆*) δ :** 33.8 (CH_{2-CH2-NH}), 43.1 (CH_{2-NH}), 105.2 (CH_{Ar}), 126.3 (CH_{Ar}), 128.3 (2xCH_{Ar}), 128.9 (2xCH_{Ar}), 138.8 (C_{q}), 143.5 (C_{q}), 144.3 (C_{q}), 154.7 (C_{q}).

**MS (ESI+) : m/z calcd for C₁₂H₁₁Cl₂N₃ :** 268.0 [M+H]+, **found :** 268.1.

### Example 2: Synthesis of 6,8-dichloro-[1,2,4]triazolo[4,3-b]pyridazine derivatives, as illustrated in step 1 of scheme 2

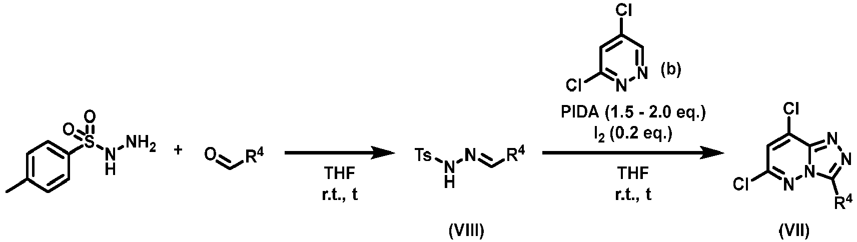

### Example 2.1: Synthesis of 6,8-dichloro-3-isopropyl-[1,2,4]triazolo[4,3-b]pyridazine (2.1)

A solution of 4-methylbenzenesulfonohydrazide (3.895 g, 20.91 mmol, 1.0 eq.) in THF (21.0 mL), corresponding aldehyde (1.97 mL, 21.33 mmol, 1.02 eq.) was added and the mixture was stirred 5 min at 50°C. The solvent was completely removed by evaporation then the crude residue was solubilised in THF (63.0 mL), 3,5-dichloropyridazine **(b)** (3.915 g, 24.97 mmol, 1.2 eq.) was added then successively I₂ (1.056 g, 4.16 mmol, 0.2 eq.) and portion wise PIDA (10.257g, 31.21 mmol, 1.5 eq.). The reaction was slightly exothermic and it was stirred 22 h at room temperature. Saturated Na₂S₂O₃ (30.0 mL) and water (15.0 mL) was added and vigorously stirred during 5 min. Layers were separated, aqueous layer was extracted twice with EtOAc (2x 20.0 mL) then organics layers were combined, washed with brine, dried over MgSO₄, filtered, concentrated and purified by flash chromatography with EtOAc/Cyclohexane as eluent to give **(2.1)** (2.100 g, 44 %) as a pale yellow solid.

**¹H NMR (400 MHz, DMSO**-***d₆*) δ :** 1.41 (d, *J* = 7.0 Hz, 6H, 2×CH_{3 iPr}), 3.50 (hept, *J* = 6.9 Hz, 1H, H_{iPr}), 7.93 (s, 1H, H_{Ar}).

**¹³C NMR (101 MHz, DMSO**-***d₆*) δ :** 19.7 (2×CH_{3 iPr}), 24.6 (CH_{iPr}), 121.2 (CH_{Ar}), 133.2 (C_{q}), 142.1 (C_{q}), 147.5 (C_{q}), 154.8 (C_{q}).

**MS (ESI+) : m/z calcd for C₈H₈Cl₂N₄ :** 231.0 [M+H]+, **found :** 230.9.

### Example 2.2: Synthesis of 6,8-dichloro-3-sec-butyl-[1,2,4]triazolo[4,3-b]pyridazine (2.2)

To a solution of 4-methylbenzenesulfonohydrazide (1.900 g, 9.90 mmol, 1.0 eq.) in THF (25.0 mL), 2-methylbutanal (1.12 mL, 9.90 mmol, 1.0 eq.) was added then the mixture was stirred 25 min at room temperature. After completion, 3,5-dichloropyridazine **(b)** (1.552 g, 9.90 mmol, 1.0 eq.) was added then, in one portion, PIDA (6.505 g, 19.79 mmol, 2.0 eq.) and I₂ (0.502 g, 1.98 mmol, 0.2 eq.). The mixture was stirred 17 h at room temperature then saturated Na₂S₂O₃ (10.0 mL) and saturated NaHCO₃ (10.0 mL) was added, the mixture was stirred vigorously 5 min. Layers were separated and aqueous layer was extracted twice with EtOAc (2 x 10.0 mL), organics layers were combined, dried over MgSO₄, filtered and purified by flash chromatography with EtOAc/Cyclohexane (30/70) as eluent to give **(2.2)** (0.670 g, 28 %) as a white solid.

**¹H NMR (400 MHz, DMSO**-***d₆*) δ :** 0.86 (t, *J* = 7.4 Hz, 3H, CH_{3-CH2}), 1.38 (d, *J=* 7.0 Hz, 3H, CH_{3-CH}), 1.69 - 2.00 (m, 2H, CH_{2-CH3}), 3.33 - 3.41 (m, 1H, H_{C-CH3}), 7.93 (s, 1H, H_{Ar}).

**¹³C NMR (101 MHz, DMSO**-***d₆*) δ :** 11.4 (CH_{3-CH2}), 17.4 (CH_{3-CH}), 26.7 (CH_{2-CH3}), 31.1 (CH._{CH3}), 121.3 (CH_{Ar}), 133.3 (C_{q}), 142.1 (C_{q}), 147.6 (C_{q}), 154.0 (C_{q}).

**MS (ESI+) : m/z calcd for C₉H₁₀Cl₂N₄ :** 245.0 [M+H]+, **found :** 245.1.

### Example 2.3: Synthesis of 6,8-dichloro-3-(1-ethylpropyl)-[1,2,4]triazolo[4,3-b]pyridazine (2.3)

To a solution of 4-methylbenzenesulfonohydrazide (1.600 g, 8.59 mmol, 1.0 eq.) in THF (20.0 mL), 2-ethylbutanal (1.14 mL, 8.59 mmol, 1.0 eq.) was added then the mixture was stirred 25 min at room temperature. After completion, 3,5-dichloropyridazine **(b)** (1.307 g, 8.59 mmol, 1.0 eq.) was added then PIDA (5.478 g, 16.67 mmol, 2.0 eq.) and I₂ (0.423 g, 1.67 mmol, 0.2 eq.) in one portion. The mixture was stirred 17 h at room temperature then saturated Na₂S₂O₃ (10.0 mL) and saturated NaHC0₃ (10.0 mL) was added, the mixture was stirred vigorously 5 min. Layers were separated and aqueous layer was extracted twice with EtOAc (2 x 10.0 mL), organics layers were combined, dried over MgSO₄, filtered and purified by flash chromatography with EtOAc/Cyclohexane (30/70) as eluent to give **(2.3)** (0.570 g, 26 %) as a white solid.

**¹H NMR (400 MHz, DMSO**-***d₆*) δ :** 0.80 (t, *J* = 7.4 Hz, 6H, 2xCH_{3-CH2}), 1.75 - 1.97 (m, 4H, 2xCH_{2-CH3}), 3.23 (tt, *J* = 8.3, 5.7 Hz, 1H, H_{iPent}), 7.94 (s, 1H, H_{Ar}).

**¹³C NMR (101 MHz, DMSO**-***d₆*) δ :** 11.4 (2×CH_{3-CH2}), 24.8 (2×CH_{2-CH3}), 38.3 (CH_{iPent}), 121.3 (CH_{Ar}), 133.3 (C_{q}), 142.1 (C_{q}), 147.7 (C_{q}),

**MS (ESI+) : m/z calcd for C₁₀H₁₂Cl₂N₄ :** 259.1 [M+H]+, **found :** 259.1.

### Example 2.4: Synthesis of 6,8-dichloro-3-isobutyl-[1,2,4]triazolo[4,3-b]pyridazine (2.4)

To a solution of 4-methylbenzenesulfonohydrazide (1.000 g, 5.37 mmol, 1.0 eq.) in THF (20.0 mL), 3-methylbutanal (0.58 mL, 5.37 mmol, 1.0 eq.) was added then the mixture was stirred 20 min at room temperature. After completion, 3,5-dichloropyridazine **(b)** (0.927 g, 5.91 mmol, 1.1 eq.) was added then, in one portion, PIDA (3.530 g, 10.74 mmol, 2.0 eq.) and I₂ (0.273 g, 1.07 mmol, 0.2 eq.). The mixture was stirred 2 h at room temperature then saturated Na₂S₂O₃ (20.0 mL) and H₂O (10.0 mL) was added, the mixture was stirred vigorously 5 min. Layers were separated and aqueous layer was extracted twice with EtOAc (2 x 20.0 mL), organics layers were combined, dried over MgSO₄, filtered, concentrated and purified by flash chromatography with EtOAc/Cyclohexane (85/15 to 50/50) as eluent to give **(2.4)** (0.452 g, 34 %) as a beige solid.

**¹H NMR (400 MHz, DMSO**-***d₆*) δ :** 0.96 (d, *J* = 6.7 Hz, 6H, 2×CH_{3 iPr}), 2.23 (dp, *J* = 13.6, 6.8 Hz, 1H, H_{iPr}), 2.97 (d, *J* = 7.1 Hz, 2H, CH_{2-CH}), 7.93 (s, 1H, H_{Ar}).

**¹³C NMR (101 MHz, DMSO**-***d₆***) **δ :** 22.2 (2×CH_{3 iPr}), 26.3 (CH_{-CH2}), 32.2 (CH_{2-CH}), 121.2 (CH_{Ar}), 133.2 (C_{q}), 141.9 (C_{q}), 147.7 (C_{q}), 150.1 (C_{q}).

**MS (ESI+) : m/z calcd for C₉H₁₂Cl₂N₄ :** 245.0 [M+H]+, **found :** 245.1.

### Example 2.5: Synthesis of 6,8-dichloro-3-cyclopropyl-[1,2,4]triazolo[4,3-b]pyridazine (2.5)

To a solution of 3,5-dichloropyridazine **(b)** (2.000 g, 12.7 mmol, 1.0 eq.) and *N-*(cyclopropylmethyleneamino)-4-methyl-benzenesulfonamide (4.559 g, 19.1 mmol, 1.5 eq.) in THF (95.0 mL), PIDA (6.288 g, 19.1mmol, 1.5 eq.) and I₂ (0.647 g, 2.5 mmol, 0.2 eq.) was added in one portion and the mixture was stirred 2 h at room temperature. After completion, a mixture of saturated Na₂S₂O₃ (20.0 mL) and water (10.0 mL) was added, the biphasique mixture was vigorously stirred 5 min then layers were separated. Aqueous layer was extracted twice with EtOAc (2 x 15.0 mL) and organics layers were combined, dried over MgSO₄, filtered and purified by flash chromatography with EtOAc/Cyclohexane (75/25 then 70/30) as eluent to give **(2.5)** (0.400 g, 14 %) as a white solid.

**¹H NMR (400 MHz, DMSO**-***d₆*) δ :** 1.07 - 1.27 (m, 4H, 2×CH_{2 cPr}), 2.40 (tt, *J* = 8.3, 5.0 Hz, 1H, H_{cPr}), 7.91 (s, 1H, H_{Ar}).

**¹³C NMR (101 MHz, DMSO**-***d₆***) δ : 4.9 (CH_{cPr}), 7.5 (2×CH_{2 cPr}), 121.1 (CH_{Ar}), 133.1 (C_{q}), 142.1 (C_{q}), 147.7 (C_{q}), 152.3 (C_{q}).

**MS (ESI+) : m/z calcd for C₈H₆Cl₂N₄:** 229.0 [M+H]+, **found :** 228.9.

### Example 2.6: Synthesis of 6,8-dichloro-3-cyclopentyl-[1,2,4]triazolo[4,3-b]pyridazine (2.6)

To a solution of 4-methylbenzenesulfonohydrazide (1.800 g, 9.38 mmol, 1.0 eq.) in THF (140.0 mL), cyclopentanecarbaldehyde (0.995 g, 9.84 mmol, 1.05 eq.) was added then the mixture was stirred 20 min at room temperature. After completion, 3,5-dichloropyridazine **(b)** (1.470 g, 9.38 mmol, 1.0 eq.) was added then, in one portion, PIDA (4.622 g, 14.06 mmol, 1.5 eq.) and I₂ (0.476 g, 1.88 mmol, 0.2 eq.). The mixture was stirred 1.3 h at room temperature then partially concentrated, the residue was diluted in EtOAc (50.0 mL). Organic layer was washed with a mixture of saturated Na₂S₂O₃ (40.0 mL) and water (80.0 mL). Aqueous layer was extracted twice with EtOAc (2 x 30.0 mL) and organics layers were combined, dried over MgSO₄, filtered and purified by flash chromatography with EtOAc/Cyclohexane (15/85 to 50/50) as eluent to give **(2.6)** (0.260 g, 11 %) as a white flakes.

**¹H NMR (400 MHz, DMSO**-***d*₆) δ :** 1.63 - 1.86 (m, 4H, 2×CH_{2 cPent}), 1.90 - 2.03 (m, 2H, CH_{2 cPent}), 2.11 (dq, *J* = 12.3, 7.0, 6.0 Hz, 2H, CH_{2 cPent}), 3.61 (p, *J* = 7.9 Hz, 1H, H_{cPent}), 7.92 (s, 1H, H_{Ar}).

**¹³C NMR (101 MHz, DMSO-*d₆*) δ :** 25.1 (2xCH_{2 cPent}), 30.1 (2xCH_{2 cPent}), 34.3 (CH_{cPent}), 121.2 (CH_{Ar}), 133.2 (C_{q}), 142.2 (C_{q}), 147.5 (C_{q}), 154.0 (C_{q}).

**MS (ESI+) : m/z calcd for C₁₀H₁₀Cl₂N₄ :** 257.0 [M+H]+, **found :** 257.1.

### Example 2.7: Synthesis of 6,8-dichloro-3-sec-butyl-[1,2,4]triazolo[4,3-b]pyridazine (2.7)

To a solution of 4-methylbenzenesulfonohydrazide (1.102 g, 5.74 mmol, 1.2 eq.) in THF (15.0 mL), butanal (0.52 mL, 5.74 mmol, 1.2 eq.) was added then the mixture was stirred 10 min at room temperature. After completion, 3,5-dichloropyridazine **(b)** (0.750 g, 4.78 mmol, 1.0 eq.) was added then, in one portion, PIDA (6.505 g, 19.79 mmol, 2.0 eq.) and I₂ (0.502 g, 1.98 mmol, 0.2 eq.). The mixture was stirred 17 h at room temperature then saturated Na₂S₂O₃ (10.0 mL) and saturated NaHCO₃ (10.0 mL) was added, the mixture was stirred vigorously 5 min. Layers were separated and aqueous layer was extracted twice with EtOAc (2 x 10.0 mL), organics layers were combined, dried over MgSO₄, filtered and purified by flash chromatography with EtOAc/Cyclohexane (30/70) as eluent to give (**2.7**) (0.957g with impurities (RMN purity 70%), calculated = 0.670 g, 28 %) as a white solid.

**¹H NMR** (**400 MHz, DMSO-*d*₆**) **δ :** 0.86 (t, *J* = 7.4 Hz, 3H, CH_{3-CH2}), 1.38 (d, *J* = 7.0 Hz, 3H, CH_{3-CH}), 1.69 - 2.00 (m, 2H, CH_{2-CH3}), 3.33 - 3.41 (m, 1H, H_{C-CH3}), 7.93 (s, 1H, H_{Ar}).

**¹³C NMR (**101 **MHz, DMSO-*d₆***) **δ** : 11.4 (CH_{3-CH2}), 17.4 (CH_{3-CH}), 26.7 (CH_{2-CH3}), 31.1 (CH_{-CH3}), 121.3 (CH_{Ar}), 133.3 (C_{q}), 142.1 (C_{q}), 147.6 (C_{q}), 154.0 (C_{q}).

**MS (ESI+) : m/z calcd for C₉H₁₀Cl₂N₄ :** 245.0 [M+H]+, **found** : 245.1.

### Example 3: Synthesis of 6-chloro-[1,2,4]triazolo[4,3-b]pyridazin-8-amine derivatives, as illustrated in step 2 of scheme 1 or in step 2 of scheme 2

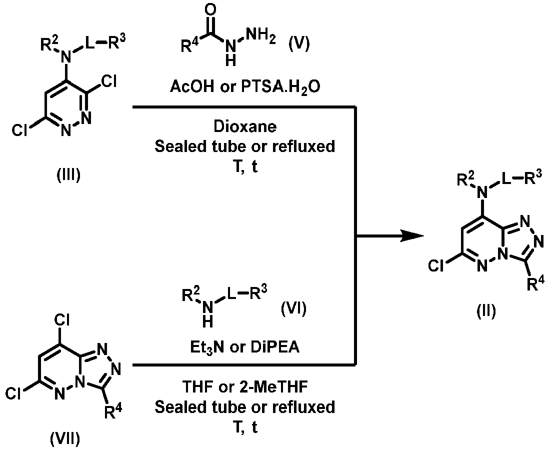

### Example 3.1: Synthesis of N-benzyl-6-chloro-3-isopropyl-[1,2,4]triazolo[4,3-b]pyridazine-8-amine (3.1)

In a sealed tube 10 - 20 mL with a stir bar was charged, (**1**.**1**) (0.250 g, 0.98 mmol, 1.0 eq.), dioxane (3.5 mL), isobutyric acid hydrazide (0.121 g, 1.18 mmol, 1.2 eq.) and AcOH (0.05 mL, 0.79 mmol, 0.8 eq.). The vial was sealed and then put on heating block, 4 h at 100 °C. The reaction mixture was cooled and NaHCO₃ sat. (8.0 mL) was added then diluted with water (8.0 mL). The precipitate was filtered off, washed with water up to neutral pH then triturated in Et₂O and dried under vacuum to give the title compound (**3.1**) (0.220 g, 74 %) as a white solid.

**Rf** (DCM/EtOAc, 75/25) : 0.30.

**¹H NMR (400 MHz, DMSO-*d₆***) **δ :** 1.38 (d, *J* = 6.9 Hz, 6H, 2xCH₃), 3.43 (hept, *J* = 7.1 Hz, 1H, H_{Alk}), 4.60 (d, *J* = 4.2 Hz, 2H, CH₂), 6.14 (s, 1H, H_{Ar}), 7.22 - 7.31 (m, 1H, H_{Ar}), 7.32 - 7.44 (m, 4H, 4xH_{Ar}), 9.13 (s, 1H, NH).

**¹³C NMR (101 MHz, DMSO-*d₆***) **δ :** 20.3 (CH₃), 24.9 (CH_{Alk}), 45.6 (CH₂), 92.0 (CH_{Ar}), 127.6 (CH_{Ar}), 127.7 (CH_{Ar}), 129.0 (CH_{Ar}), 137.9 (C_{q}), 139.9 (C_{q}), 142.8 (C_{q}), 149.9 (C_{q}), 154.4 (C_{q}).

**MS** (**ESI+**) **: m/z calcd for C₁₅H₁₆ClN₅** : 302.12 [M+H]+, **found** : 302.33.

### Example 3.2: Synthesis of N-benzyl-6-chloro-[1,2,4]triazolo[4,3-b]pyridazin-8-amine (3.2)

In a sealed tube 2 - 5 mL with a stir bar was charged, (**1.1**) (0.850 g, 3.34 mmol, 1.0 eq.), dioxane (17.0 mL), formic hydrazide (0.221 g, 3.68 mmol, 1.1 eq.) and PTSA.H₂O (0.318 g, 1.67 mmol, 0.5 eq.). The mixture was refluxed for 36 h and after cooling, MeOH (2mL) then DCM (2mL) was added. The mixture was concentrated and purified by flash chromatography with DCM/EtOAc (8/2) as eluent to give the title compound (**3.2**) (0.250 g, 29 %) as a white solid.

**Rf** (DCM/EtOAc, 75/25) : 0.21.

**¹H NMR (400 MHz, DMSO-*d₆***) **δ :** 4.60 (d, *J* = 6.4 Hz, 2H, CH_{2 Bn}), 6.17 (s, 1H, H_{Ar}), 7.26 (t, *J* = 7.4 Hz, 1H, H_{Ar}), 7.35 (t, *J* = 7.4 Hz, 2H, 2xH_{Ar}), 7.40 (d, *J* = 7.6 Hz, 2H, 2xH_{Ar}), 9.17 (s, 1H, NH), 9.41 (s, 1H, H_{Ar}).

**¹³C NMR (101 MHz, DMSO-*d₆***) **δ** : 45.2 (CH₂), 92.1 (CH_{Ar}), 127.2 (2xCH_{Ar}), 127.2 (CH_{Ar}), 128.5 (2xCH_{Ar}), 137.4 (C_{q}), 139.1 (C_{q}), 139.6 (CH_{Ar}), 142.1 (C_{q}), 150.0 (C_{q}).

**MS (ESI+) : m/z calcd for C₁₂H₁₀ClN₅ :** 260.07 [M+H]+, **found** : 260.1.

### Example 3.3: Synthesis of N-benzyl-6-chloro-3-cyclopropyl-[1,2,4]triazolo[4,3-b]pyridazin-8-amine (3.3)

In a sealed tube 2 - 5 mL with a stir bar was charged, (**1.1**) (0.300 g, 1.18 mmol, 1.0 eq.), dioxane (3.9 mL), cyclopropanecarboxylic acid hydrazide (0.130 g, 1.30 mmol, 1.1 eq.) and PTSA.H₂O (0.023 g, 0.12 mmol, 0.1 eq.). The vial was sealed and then put on heating block, 20 h at 100 °C. The reaction mixture was cooled and MeOH (2mL) then DCM (2mL) was added. The mixture was concentrated and purified by flash chromatography with DCM/EtOAc (7/3) as eluent to give the title compound (**3.3**) (0.184 g, 52 %) as a white solid.
**Rf** (DCM/EtOAc, 7/3) : 0.44

**¹H NMR (400 MHz, DMSO-*d₆***) **δ** : 1.06 - 1.15 (m, 4H, 2xCH_{2 Alk}), 2.31 (ddd, *J* = 13.3, 8.0, 5.4 Hz, 1H, H_{Alk}), 4.58 (d, *J* = 6.5 Hz, 2H, CH₂), 6.12 (s, 1H, H_{Ar}), 7.26 (t, *J* = 7.1 Hz, 1H, H_{Ar}), 7.32 - 7.41 (m, 4H, 4xH_{Ar}), 9.08 (s, 1H, NH).

**¹³C NMR (101 MHz, DMSO-*d₆***) **δ :** 4.8 (CH_{Alk}), 7.1 (2xCH_{2 Alk}), 45.1 (CH₂), 91.5 (CH), 127.2 (2xCH_{Ar}), 127.2 (CH_{Ar}), 128.5 (2xCH_{Ar}), 137.4 (C_{q}), 139.4 (C_{q}), 142.3 (C_{q}), 149.6 (C_{q}), 151.4 (C_{q}).

**MS (ESI+) : m/z calcd for C₁₅H₁₄ClN₅ :** 300.1 [M+H]+, **found** : 300.1.

### Example 3.4: Synthesis of N-benzyl-6-chloro-3-cyclopentyl-[1,2,4]triazolo[4,3-b]pyridazin-8-amine (3.4)

To a solution of (**2.6**) (0.200 g, 0.75 mmol, 1.0 eq.) in THF (15.0 mL), corresponding amine (0.17 mL, 1.51 mmol, 2.0 eq.) and Et₃N (0.21 mL, 1.51 mmol, 2.0 eq.) was added then the mixture was refluxed in 3 h. After cooling, the mixture was concentrated and the residue was triturated in water, solid was filtered off, washed wih water, then Et₂O and dried under vacuum to give (**3.4**) (0.220 g, 89 %) as a white solid.

**¹H NMR (400 MHz, DMSO-*d₆***) **δ :** 1.61-1.84 (m, 4H, 2xCH_{2 cPent}), 1.92 (dq, *J* = 12.0, 7.4 Hz, 2H, CH_{2 cPent}), 2.02 - 2.18 (m, 2H, CH_{2 cPent}), 3.52 (p, *J* = 8.0 Hz, 1H, H_{cPent}), 4.58 (s, 2H, CH_{2 Bn}), 6.13 (s, 1H, H_{Ar}), 7.26 (t, *J* = 7.1 Hz, 1H, H_{Ar}), 7.34 (t, *J* = 7.5 Hz, 2H, 2xH_{Ar}), 7.39 (d, *J* = 7.5 Hz, 2H, 2xH_{Ar}), 9.09 (s, 1H, NH_{Bn}).

**¹³C NMR (101 MHz, DMSO-*d₆***) **δ** : 25.0 (2xCH_{2 cPent}), 30.2 (2xCH_{2 cPent}), 34.3 (CH_{cPent}), 45.1 (CH_{2 Bn}), 91.5 (CH_{Ar}), 127.2 (2xCH_{Ar}), 127.2 (CH_{Ar}), 128.5 (2xCH_{Ar}), 137.4 (C_{q}), 139.5 (C_{q}), 142.28 (C_{q}), 149.4 (C_{q}), 153.1 (C_{q}).

**MS (ESI+) : m/z calcd for C₁₇H₁₈ClN₅** : 328.1 [M+H]+, **found** : 328.2.

### Example 3.5: Synthesis of N-benzyl-6-chloro-3-(trifluoromethyl)-[1,2,4]triazolo[4,3-b]pyridazin-8-amine (3.5)

In a sealed tube 2 - 5 mL with a stir bar was charged, (**1.1**) (0.300 g, 1.18 mmol, 1.0 eq.), dioxane (3.9 mL), trifluoroacetic acid hydrazide (0.166 g, 1.30 mmol, 1.1 eq.) and PTSA.H₂O (0.023 g, 0.12 mmol, 0.1 eq.). The vial was sealed and then put on heating block, 20 h at 100 °C. The reaction mixture was cooled and MeOH (2mL) then DCM (2mL) was added. The mixture was concentrated and purified by flash chromatography with DCM/EtOAc (9/1) as eluent to give the title compound (3.5) (0.070 g, 18 %) as a white solid.
**Rf** (DCM/EtOAc, 9/1) : 0.63

**¹H NMR (400 MHz, DMSO-*d₆***) **δ** : 4.65 (d, *J* = 6.2 Hz, 2H, CH₂), 6.43 (s, 1H, H_{Ar}), 7.28 (dq, *J* = 7.2, 4.9, 3.3 Hz, 1H, H_{Ar}), 7.35 (dd, *J* = 8.3, 6.6 Hz, 2H, 2xH_{Ar}), 7.41 (d, *J* = 7.2 Hz, 2H, 2xH_{Ar}), 9.52 (s, 1H, NH).

**¹³C NMR (101 MHz, DMSO-*d₆***) **δ** : 45.19 (CH₂), 93.80 (CH_{Ar}), 118.24 (q, *J* = 269.9 Hz, CF₃), 127.26 (2xCH_{Ar}), 127.32 (CH_{Ar}), 128.53 (2xCH_{Ar}), 136.98 (C_{q}), 138.45 (q, *J* = 41.0, 40.3 Hz, C_{q-CF3}), 141.85 (C_{q}), 142.29 (C_{q}), 151.64 (C_{q}).

**MS (ESI+) : m/z calcd for C₁₃H₉ClF₃N₅:** 328.1 [M+H]+, **found** : 328.1.

### Example 3.6: Synthesis of N-benzyl-6-chloro-3-methyl-[1,2,4]triazolo[4,3-b]pyridazin-8-amine (3.6)

To a solution of (**1.1**) (0.750 g, 2.95 mmol, 1.0 eq.) in dioxane (15.0 mL), acetohydrazide (0.228 g, 3.25 mmol, 1.1 eq.) and PTSA.H₂O (0.281 g, 1.48 mmol, 0.5 eq.) was added and refluxed during 24 h. The reaction mixture was cooled and MeOH (10.0 mL) was added. The mixture was concentrated with SiO₂ to make solid deposit and directly purified by flash chromatography with DCM/EtOAc/MeOH (60/40/0 to 60/30/10) as eluent to give the title compound (**3.6**) (0.350 g, 43 %) as a white solid.
**Rf** (DCM/EtOAc, 75/25) : 0.24

**¹H NMR (400 MHz, DMSO-*d₆***) **δ :** 2.58 (s, 3H, CH₃), 4.60 (s, 2H, CH₂), 6.14 (s, 1H, H_{Ar}), 7.22 - 7.42 (m, 5H, 5xH_{Ar}), 9.09 (s, 1H, NH).

**¹³C NMR (101 MHz, DMSO-*d₆***) **δ** : 9.4 (CH₃), 45.1 (CH₂), 91.4 (CH_{Ar}), 127.1 (2xCH_{Ar}), 127.2 (CH_{Ar}), 128.5 (2xCH_{Ar}), 137.4 (C_{q}), 139.2 (C_{q}), 142.3 (C_{q}), 146.8 (C_{q}), 149.6 (C_{q}). **MS (ESI+) : m/z calcd for C₁₃H₁₂ClN₅** : 274.1 [M+H]+, **found** : 274.1.

### Example 3.7: Synthesis of N-benzyl-6-chloro-3-phenyl-[1,2,4]triazolo[4,3-b]pyridazin-8-amine (3.7)

To a solution of **(1.1)** (0.750 g, 2.95 mmol, 1.0 eq.) in dioxane (15.0 mL), benzohydrazide (0.442 g, 3.25 mmol, 1.1 eq.) and PTSA.H₂O (0.281 g, 1.48 mmol, 0.5 eq.) was added and refluxed during 22.5 h. The reaction mixture was cooled and MeOH (5.0 mL) was added. The mixture was concentrated with SiO₂ to make solid deposit and directly purified by flash chromatography with DCM/MeOH (94/6) as eluent to give the title compound **(3.7)** (0.425 g, 46 %) as a white solid.
**Rf** (DCM/MeOH, 94/6) : 0.46

**¹H NMR (400 MHz, DMSO-*d₆***) **δ :** 4.64 (d, *J* = 6.4 Hz, 2H, CH_{2 Bn}), 6.27 (s, 1H, H_{Ar}), 7.27 (t, *J* = 7.2 Hz, 1H, H_{Ar}), 7.36 (t, *J* = 7.5 Hz, 2H, 2xH_{Ar}), 7.43 (d, *J* = 7.6 Hz, 2H, 2xH_{Ar}), 7.58 (dt, *J* = 13.3, 7.2 Hz, 3H, 3xH_{Ar}), 8.31 (d, *J* = 7.5 Hz, 2H, 2xH_{Ar}), 9.25 (s, 1H, NH_{Bn}).

**¹³C NMR (101 MHz, DMSO-*d₆***) **δ :** N.D.

**MS (ESI+) : m/z calcd for C₁₈H₁₄ClN₅** : 336.1 [M+H]+, **found** : 336.1.

### Example 3.8: Synthesis of 2-[[(6-chloro-3-isopropyl-[1,2,4]triazolo[4,3-b]pyridazin-8-yl)amino]methyl]phenol (3.8)

To a suspension of (**1.7**) (1.900 g, 7.03 mmol, 1.0 eq.) in dioxane (40.0 mL), isobutyric acid hydrazide (1.150 g, 11.25 mmol, 1.6 eq.) then AcOH (0.45 mL, 7.74 mmol, 1.1 eq.) was added. The mixture was refluxed 16 h and after cooling, MeOH (5.0 mL) was added then SiO₂ to make solid deposit directly. The mixture was concentrated and purified by flash chromatography with DCM/MeOH (97/3 to 93/7) as eluent to give **(3.8)** (1.000 g, 45 %) as a white solid.
**Rf** (DCM/MeOH, 92/8) : 0.15

**¹H NMR (400 MHz, DMSO-*d₆***) **δ :** 1.38 (d, *J* = 6.9 Hz, 6H, 2xCH_{3 iPr}), 3.42 (p, *J* = 6.9 Hz, 1H, CH_{iPr}), 4.37 - 4.61 (m, 2H, CH_{2 Bn}), 6.12 (s, 1H, CH_{Ar}), 6.76 (t, *J* = 7.4 Hz, 1H, CH_{Ar}), 6.85 (d, *J* = 8.0 Hz, 1H, CH_{Ar}), 7.10 (t, *J* = 7.7 Hz, 1H, CH_{Ar}), 7.18 (d, *J* = 7.6 Hz, 1H, CH_{Ar}), 8.90 (s, 1H, NH_{Bn}), 9.82 (s, 1H, OH).

**¹³C NMR (101 MHz, DMSO-*d₆***) **δ** : 19.8 (2xCH_{3 iPr}), 24.4 (CH_{iPr}), 40.2 (CH_{2 Bn}), 91.3 (CH_{Ar}), 115.1 (CH_{Ar}), 119.1 (CH_{Ar}), 122.9 (C_{q}), 128.4 (2xCH_{Ar}), 139.4 (C_{q}), 142.3 (C_{q}), 149.4 (C_{q}), 154.0 (C_{q}), 154.8 (C_{q}).

**MS (ESI+)** : **m/z calcd for C₁₅H₁₆ClN₅O** : 318.1 [M+H]+, **found** : 318.2.

### Example 3.9: Synthesis of 3-[[(6-chloro-3-isopropyl-[1,2,4]triazolo[4,3-b]pyridazin-8-yl)amino]methyl]phenol (3.9)

In a sealed vial 10 - 20 mL with a stir bar was charged (**1.6**) (0.460 g, 1.70 mmol, 1.0 eq.) in dioxane (10.0 mL), isobutyric acid hydrazide (0.211 g, 2.04 mmol, 1.2 eq.) and PTSA.H₂O (0.197 g, 1.02 mmol, 0.6 eq.) was added. The vial was sealed and put on heating bloc 17 h at 100°C. After cooling, MeOH (3.0 mL) was added then the mixture was concentrated with SiO₂ to make solid deposit and directly purified by flash chromatography with DCM/MeOH (97/3 to 94/6) as eluent to give **(3.9)** (0.250 g, 46 %) as a white solid.
**Rf** (DCM/MeOH, 92/8) : 0.23

**¹H NMR (400 MHz, DMSO-*d₆***) **δ :** 1.38 (d, *J* = 6.9 Hz, 6H, 2xCH_{3 iPr}), 3.42 (p, *J* = 7.0 Hz, 1H, H_{iPr}), 4.50 (d, *J* = 6.2 Hz, 2H, CH_{2 Bn}), 6.09 (s, 1H, H_{Ar}), 6.64 (dd, *J* = 8.0, 1.8 Hz, 1H, H_{Ar}), 6.75 (t, *J* = 2.0 Hz, 1H, H_{Ar}), 6.79 (d, *J* = 7.6 Hz, 1H, H_{Ar}), 7.12 (t, *J* = 7.8 Hz, 1H, H_{Ar}), 9.09 (s, 1H, NH_{Bn}), 9.37 (s, 1H, OH).

**¹³C NMR (101 MHz, DMSO-*d₆***) **δ :** 19.9 (2xCH_{3 iPr}), 24.4 (CH_{iPr}), 45.0 (CH_{2 Bn}), 91.5 (CH_{Ar}), 113.7 (CH_{Ar}), 114.2 (CH_{Ar}), 117.7 (CH_{Ar}), 129.5 (CH_{Ar}), 138.8 (Cq), 139.4 (Cq), 142.3 (Cq), 149.4 (Cq), 154.0 (Cq), 157.5 (Cq).

**MS (ESI+)** : **m/z calcd for C₁₅H₁₆ClN₅O :** 318.1 [M+H]+, **found** : 318.3.

### Example 3.10: Synthesis of 4-[[(6-chloro-3-isopropyl-[1,2,4]triazolo[4,3-b]pyridazin-8-yl)amino]methyl]phenol (3.10)

In a sealed vial 10 - 20 mL with a stir bar was charged **(1.5)** (0.520 g, 1.93 mmol, 1.0 eq.) in dioxane (10.0 mL), isobutyric acid hydrazide (0.238 g, 2.31 mmol, 1.2 eq.) and PTSA.H₂O (0.223 g, 1.16 mmol, 0.6 eq.) was added. The vial was sealed and put on heating bloc 17 h at 100°C. After cooling, MeOH (3.0 mL) was added then the mixture was concentrated with SiO₂ to make solid deposit and directly purified by flash chromatography with DCM/MeOH (97/3 to 94/6) as eluent to give **(3.10)** (0.115 g, 19 %) as a white solid.

**Rf** (DCM/MeOH, 92/8) : 0.25.

**¹H NMR (400 MHz, DMSO-*d₆***) **δ :** 1.37 (d, *J* = 6.9 Hz, 6H, 2xCH_{3 iPr}), 3.41 (hept, *J* = 7.0 Hz, 1H, H_{iPr}), 4.44 (d, *J* = 6.2 Hz, 2H, CH_{2 Bn}), 6.12 (s, 1H, H_{Ar}), 6.71 (d, *J* = 8.5 Hz, 2H, 2xH_{Ar}), 7.20 (d, *J* = 8.5 Hz, 2H, 2xH_{Ar}), 9.02 (s, 1H, NH_{Bn}), 9.34 (s, 1H, OH).

**¹³C NMR (101 MHz, DMSO-*d₆***) **δ** : 19.8 (2xCH_{3 iPr}), 24.4 (CH_{iPr}), 44.7 (CH_{2 Bn}), 91.4 (CH_{Ar}), 115.2 (2xCH_{Ar}), 127.5 (C_{q}), 128.6 (2xCH_{Ar}), 139.5 (C_{q}), 142.2 (C_{q}), 149.4 (C_{q}), 153.9 (C_{q}), 156.6 (C_{q}).

**MS (ESI+)** : **m/z calcd for C₁₅H₁₆ClN₅O** : 318.1 [M+H]+, **found** : 318.3.

### Example 3.11: Synthesis of 6-chloro-3-isopropyl-N-[(2-methoxyphenyl)methyl]-[1,2,4]triazolo[4,3-b]pyridazin-8-amine (3.11)

To a suspension of (**1.8**) (3.000 g, 10.56 mmol, 1.0 eq.) in dioxane (40.0 mL), isobutyric acid hydrazide (1.307 g, 12.67 mmol, 1.2 eq.) then AcOH (0.67 mL, 11.61 mmol, 1.1 eq.) was added and the mixture was refluxed 16 h. After cooling, MeOH (10.0 mL) was added, stirred vigorously 5 min then SiO₂ to make solid deposit directly. The mixture was concentrated and purified by flash chromatography with DCM/MeOH (99/1 to 97/3) as eluent to give **(3.11)** (1.490 g, 43 %) as a white solid.

**¹H NMR (400 MHz, DMSO-*d₆***) **δ :** 1.38 (d, *J* = 6.9 Hz, 6H, 2xCH_{3 iPr}), 3.43 (hept, *J* = 7.1 Hz, 1H, H_{iPr}), 3.87 (s, 3H, OCH₃), 4.51 (d, *J* = 6.3 Hz, 2H, CH_{2 Bn}), 6.06 (s, 1H, H_{Ar}), 6.91 (t, *J* = 7.4 Hz, 1H, H_{Ar}), 7.05 (d, *J*= 8.1 Hz, 1H, H_{Ar}), 7.22 (d, *J* = 7.5 Hz, 1H, H_{Ar}), 7.28 (td, *J* = 7.9, 1.8 Hz, 1H, H_{Ar}), 8.90 (s, 1H, NH_{Bn}).

**¹³C NMR (101 MHz, DMSO-*d₆***) **δ :** 19.8 (2xCH_{3 iPr}), 24.4 (CH_{iPr}), 40.2 (CH_{2 Bn}), 55.4 (OCH₃), 91.3 (CH_{Ar}), 110.8 (CH_{Ar}), 120.4 (CH_{Ar}), 124.4 (C_{q}), 127.7 (CH_{Ar}), 128.6 (CH_{Ar}), 139.4 (C_{q}), 142.4 (C_{q}), 149.4 (C_{q}), 154.0 (C_{q}), 156.7 (C_{q}).

**MS (ESI+) : m/z calcd for C₁₆H₁₈ClN₅O** : 332.1 [M+H]+, **found** : 332.3.

### Example 3.12: Synthesis of 6-chloro-3-isopropyl-N-phenyl-[1,2,4]triazolo[4,3-b]pyridazin-8-amine (3.12)

To a solution of **(2.1)** (0.150 g, 0.65 mmol, 1.0 eq.) in dry THF (5.0 mL), aniline (0.12 mL, 1.30 mmol, 2.0 eq.) then tBuOK 1M in THF (1.30 mL, 1.30 mmol, 2.0 eq.) was added and the mixture was stirred 1 h at room temperature. After completion, saturated NH₄Cl (5.0 mL) and water (1.5 mL) was added then biphasic mixture was vigorously stirred 5 min. Layers were separated, aqueous layer was extracted with EtOAc (2x5.0 mL) then organic layers were combined, dried over MgSO₄, filtered, concentrated and purified by flash chromatography with DCM/MeOH (99/1 to 98/2) as eluent to give **(3.12)** (0.175 g, 94 %) as a white solid.
**Rf** (DCM/MeOH, 96/4) : 0.40

**¹H NMR (400 MHz, DMSO-*d₆***) **δ :** 1.41 (d, *J* = 7.0 Hz, 6H, 2xCH_{3 iPr}), 3.48 (hept, *J* = 6.9 Hz, 1H, H_{iPr}), 6.35 (s, 1H, H_{Ar}), 7.27 (tt, *J* = 5.7, 2.4 Hz, 1H, H_{Ar}), 7.40 - 7.58 (m, 4H, 4xH_{Ar}), 10.46 (s, 1H, NH).

**¹³C NMR (101 MHz, DMSO-*d₆***) **δ** : 19.8 (2xCH_{3 iPr}), 24.5 (CH_{iPr}), 92.9 (CH_{Ar}), 123.6 (2xCH_{Ar}), 125.7 (CH_{Ar}), 129.5 (2xCH_{Ar}), 137.7 (C_{q}), 139.5 (C_{q}), 140.2 (C_{q}), 149.6 (C_{q}), 154.2 (C_{q}).

**MS (ESI+) : m/z calcd for C₁₄H₁₄ClN₅ :** 288.1 [M+H]+, **found** : 288.1.

### Example 3.13: Synthesis of 6-chloro-3-isopropyl-N-(2-pyridyl)-[1,2,4]triazolo[4,3-b]pyridazin-8-amine (3.13)

To a solution of **(2.1)** (0.100 g, 0.43 mmol, 1.0 eq.) in dry THF (5.0 mL), 2-aminopyridine (0.082, 0.87 mmol, 2.0 eq.) then tBuOK 1M in THF (0.87 mL, 0.87 mmol, 2.0 eq.) was added and the mixture was stirred 1 h at room temperature. After completion, saturated NH₄Cl (3.5 mL) and water (1.5 mL) was added then biphasic mixture was vigorously stirred 5 min. Layers were separated, aqueous layer was extracted with EtOAc (2x5.0 mL) then organic layers were combined, dried over MgSO₄, filtered, concentrated and purified by flash chromatography with DCM/MeOH (98/2) as eluent to give **(3.13)** (0.120 g, 96 %) as a white solid.
**Rf** (DCM/MeOH, 96/4) : 0.27

**¹H NMR (400 MHz, DMSO-*d₆***) **δ :** 1.42 (d, *J* = 6.9 Hz, 6H, 2xCH_{3 iPr}), 3.50 (hept, *J* = 7.1, 6.5 Hz, 1H, H_{iPr}), 7.13 (ddd, *J* = 7.4, 5.0, 1.0 Hz, 1H, H_{Ar}), 7.61 (d, *J* = 8.3 Hz, 1H, H_{Ar}), 7.82 (ddd, *J* = 9.0, 7.3, 2.0 Hz, 1H, H_{Ar}), 8.32 (s, 1H, H_{Ar}), 8.46 (d, *J* = 1.9 Hz, 1H, H_{Ar}), 10.90 (s, 1H, NH).

**¹³C NMR (101 MHz, DMSO-*d₆***) δ : 19.8 (2xCH_{3 iPr}), 24.5 (CH_{iPr}), 99.5 (CH_{Ar}), 114.7 (CH_{Ar}), 118.6 (CH_{Ar}), 136.6 (C_{q}), 138.3 (CH_{Ar}), 139.3 (C_{q}), 147.3 (CH_{Ar}), 149.7 (C_{q}), 153.9 (C_{q}), 154.3 (C_{q}).

**MS (ESI+) : m/z calcd for C₁₃H₁₃ClN₆ :** 289.1 [M+H]+, **found** : 289.2.

### Example 3.14: Synthesis of 6-chloro-3-isopropyl-N-(3-pyridyl)-[1,2,4]triazolo[4,3-b]pyridazin-8-amine (3.14)

To a solution of **(2.1)** (0.100 g, 0.43 mmol, 1.0 eq.) in dry THF (5.0 mL), 3-aminopyridine (0.082, 0.87 mmol, 2.0 eq.) then tBuOK 1M in THF (0.87 mL, 0.87 mmol, 2.0 eq.) was added and the mixture was stirred 1 h at room temperature. After completion, saturated NH₄Cl (3.5 mL) and water (1.5 mL) was added then biphasic mixture was vigorously stirred 5 min. Layers were separated, aqueous layer was extracted with EtOAc (2x5.0 mL) then organic layers were combined, dried over MgSO₄, filtered, concentrated and purified by flash chromatography with DCM/MeOH (98/2 to 96/4) as eluent to give **(3.14)** (0.115 g, 92 %) as a white solid.
**Rf** (DCM/MeOH, 96/4) : 0.17

**¹H NMR (400 MHz, DMSO-*d₆***) **δ :** 1.41 (d, *J* = 6.9 Hz, 6H, 2xCH_{3 iPr}), 3.48 (hept, *J* = 6.9 Hz, 1H, H_{iPr}), 6.43 (s, 1H, H_{Ar}), 7.50 (dd, *J* = 8.2, 4.7 Hz, 1H, H_{Ar}), 7.91 (dt, *J* = 8.3, 1.9 Hz, 1H, H_{Ar}), 8.46 (dd, *J* = 4.8, 1.5 Hz, 1H, H_{Ar}), 8.69 (d, *J* = 2.6 Hz, 1H, H_{Ar}), 10.55 (s, 1H, NH).

**¹³C NMR (101 MHz, DMSO-*d₆*) δ** : 19.8 (2xCH₃ i_{Pr}), 24.5 (CHi_{Pr}), 93.8 (CH_{Ar}), 124.1 (CH_{Ar}), 130.8 (CH_{Ar}), 134.6 (C_{q}), 139.4 (C_{q}), 140.0 (C_{q}), 145.1 (CH_{Ar}), 146.4 (CH_{Ar}), 149.6 (C_{q}), 154.2 (C_{q}).

**MS (ESI+) : m/z calcd for C₁₃H₁₃ClN₆** : 289.1 [M+H]+, **found** : 289.1.

### Example 3.15: Synthesis of 6-chloro-3-isopropyl-N-(4-pyridyl)-[1,2,4]triazolo[4,3-b]pyridazin-8-amine (3.15)

To a solution of **(2.1)** (0.150 g, 0.65 mmol, 1.0 eq.) in dry THF (5.0 mL), 4-aminopyridine (0.123 g, 1.30 mmol, 2.0 eq.) then tBuOK 1M in THF (1.30 mL, 1.30 mmol, 2.0 eq.) was added and the mixture was stirred 1 h at room temperature. After completion, saturated NH₄Cl (5.0 mL) and water (1.5 mL) was added then biphasic mixture was vigorously stirred 5 min. Layers were separated, aqueous layer was extracted with EtOAc (2x5.0 mL) then organic layers were combined, dried over MgSO₄, filtered, concentrated and purified by flash chromatography with DCM/MeOH (99/1 to 96/4) as eluent to give **(3.15)** (0.180 g, 96 %) as a white solid.
**Rf** (DCM/MeOH, 96/4) : 0.10

**¹H NMR (400 MHz, DMSO-*d₆***) **δ :** 1.42 (d, *J* = 6.9 Hz, 6H, 2xCH_{3 iPr}), 3.50 (hept, *J* = 6.9 Hz, 1H, H_{iPr}), 6.88 (s, 1H, H_{Ar}), 7.34 - 7.70 (m, 2H, 2xH_{Ar}), 8.39 - 8.68 (m, 2H, 2xH_{Ar}), 10.69 (s, 1H, NH).

**¹³C NMR (101 MHz, DMSO-*d₆***) **δ** : 19.8 (2xCH_{3 iPr}), 24.5 (CH_{iPr}), 96.7 (CH_{Ar}), 115.5 (CH_{Ar}), 138.1 (C_{q}), 139.5 (C_{q}), 145.7 (C_{q}), 149.6 (C_{q}), 150.8 (CH_{Ar}), 154.3 (C_{q}).

**MS (ESI+) : m/z calcd for C₁₃H₁₃ClN₆** : 289.1 [M+H]+, **found** : 289.1.

### Example 3.16: Synthesis of 6-chloro-3-isopropyl-N-(4-pyridylmethyl)-[1,2,4]triazolo[4,3-b]pyridazin-8-amine (3.16)

To a suspension of **(1.4)** (0.390 g, 1.53 mmol, 1.0 eq.) in dioxane (25.0 mL), isobutyric acid hydrazide (0.189 g, 1.89 mmol, 1.2 eq.) then APTS.H₂O (0.177 g, 0.92 mmol, 0.6 eq.) was added and the mixture was refluxed 20 h. After cooling, DCM (5.0 mL), MeOH (5.0 mL) and NaHCO₃ (0.100 g) was added and the mixture was stirred vigorously 5 min. SiO₂ was added to make solid deposit, the mixture was concentrated and purified by flash chromatography with DCM/MeOH (95/5 to 93/7) as eluent to give **(3.16)** (0.175 g, 38 %) as a beige solid.

**¹H NMR (400 MHz, DMSO-*d₆***) **δ :** 1.38 (d, *J* = 6.9 Hz, 6H, 2xCH_{3 iPr}), 3.43 (p, *J* = 7.0 Hz, 1H, H_{iPr}), 4.64 (s, 2H, CH_{2 Bn}), 6.15 (s, 1H, H_{Ar}), 7.31-7.43 (m, 2H, 2xH_{Ar}), 8.44 - 8.60 (m, 2H, 2xH_{Ar}), 9.12 (s, 1H, NH_{Bn}).

**¹³C NMR (101 MHz, DMSO-*d₆***) **δ** : 19.8 (2xCH_{3 iPr}), 24.4 (CH_{iPr}), 44.1 (CH_{2 Bn}), 91.7 (CH_{Ar}), 122.1 (2xCH_{Ar}), 139.4 (Cq), 142.4 (Cq), 146.5 (C_{q}), 149.5 (Cq), 149.7 (2xCH_{Ar}), 154.0 (Cq).

**MS (ESI+) : m/z calcd for C₁₄H₁₅ClN₆** : 303.1 [M+H]+, **found** : 303.2.

### Example 3.17: Synthesis of 6-chloro-3-isopropyl-N-(3-pyridylmethyl)-[1,2,4]triazolo[4,3-b]pyridazin-8-amine (3.17)

To a suspension of **(1.3)** (1.500 g, 5.88 mmol, 1.0 eq.) in dioxane (20.0 mL), isobutyric acid hydrazide (0.667 g, 6.47 mmol, 1.1 eq.) then AcOH (0.37 mL, 6.47 mmol, 1.1 eq.) was added and the mixture was refluxed 16 h. After cooling, MeOH (10.0 mL) and NaHCO₃ (1.000 g) was added, stirred vigorously 5 min then SiO₂ to make solid deposit directly. The mixture was concentrated and purified by flash chromatography with DCM/MeOH (98/2 to 93/7) as eluent to give **(3.17)** (0.700 g, 39 %) as a white solid.

**¹H NMR (400 MHz, DMSO-*d₆***) **δ :** 1.37 (d, *J* = 6.9 Hz, 6H, 2xCH_{3 iPr}), 3.42 (hept, *J* = 7.1 Hz, 1H, H_{iPr}), 4.63 (s, 2H, CH_{2 Bn}), 6.26 (s, 1H, H_{Ar}), 7.37 (dd, *J* = 7.9, 4.8 Hz, 1H, H_{Ar}), 7.79 (dt, *J* = 7.9, 2.0 Hz, 1H, H_{Ar}), 8.48 (dd, *J* = 4.9, 1.6 Hz, 1H, H_{Ar}), 8.64 (d, *J =* 2.3 Hz, 1H, H_{Ar}), 9.10 (s, 1H, NH_{Bn}).

**¹³C NMR (101 MHz, DMSO-*d₆***) **δ :** 19.8 (2xCH_{3 iPr}), 24.4 (CH_{iPr}), 42.8 (CH_{2 Bn}), 91.6 (CH_{Ar}), 123.6 (CH_{Ar}), 133.1 (C_{q}), 135.1 (CH_{Ar}), 139.4 (C_{q}), 142.2 (C_{q}), 148.5 (CH_{Ar}), 148.9 (CH_{Ar}), 149.5 (C_{q}), 154.0 (C_{q}).

**MS (ESI+) : m/z calcd for C₁₄H₁₅ClN₆** : 303.1 [M+H]+, **found** : 303.3.

### Example 3.18: Synthesis of 6-chloro-3-isopropyl-N-(2-pyridylmethyl)-[1,2,4]triazolo[4,3-b]pyridazin-8-amine (3.18)

To a solution of **(1.2)** (5.000 g, 19.60 mmol, 1.0 eq.) in dioxane (80.0 mL), isobutyric acid hydrazide (2.224 g, 21.560 mmol, 1.1 eq.) then AcOH (1.25 mL, 21.56 mmol, 1.1 eq.) was added and the mixture was refluxed 15 h. After cooling, the solvent was removed and the crude was triturated in water (150.0 ml). The resulting solid was filtered off, washed with water up to neutral pH, EtOH and Et₂O then dried under vacuum to give a first part of (**3.18**) (1.880 g). The aqueous layer was extracted twice with EtOAc (2x 50.0 mL) then organics layers were combined, washed with saturated NaHCO₃, dried over MgSO₄, filtrered and concentrated to give second part of **(3.18)** (0.500 g). Quantity of (**3.18**): 2.380 g, as a white solid.
**Rf** (DCM/MeOH, 94/6) : 0.30

**¹H NMR (400 MHz, DMSO-*d₆***) **δ :** 1.38 (d, *J* = 6.9 Hz, 6H, 2xCH_{3 iPr}), 3.43 (hept, *J* = 7.0 Hz, 1H, H_{iPr}), 4.46 - 4.78 (m, 2H, CH_{2 Bn}), 6.15 (s, 1H, H_{Ar}), 7.30 (dd, *J* = 7.6, 4.8 Hz, 1H, H_{Ar}), 7.38 (d, *J* = 7.8 Hz, 1H, H_{Ar}), 7.78 (td, *J* = 7.7, 1.9 Hz, 1H, H_{Ar}), 8.55 (d, *J* = 4.9 Hz, 1H, H_{Ar}), 8.98 (s, 1H, NH_{Bn}).

¹**³C NMR (101 MHz**, **DMSO-*d₆***) **δ :** N.D.

**MS (ESI+) : m/z calcd for C₁₄H₁₅ClN₆** : 303.1 [M+H]+, **found** : 303.2.

### Example 3.19: Synthesis of 6-chloro-N-(2-pyridylmethyl)-[1,2,4]triazolo[4,3-b]pyridazin-8-amine (3.19)

To a solution of **(1.2)** (4.000 g, 15.68 mmol, 1.0 eq.) in dioxane (52.0 mL), corresponding acid hydrazide (1.130 g, 18.82 mmol, 1.2 eq.) and APTS.H₂O (3.028 g, 15.68 mmol, 1.0 eq.) was added. The mixture was refluxed during 1 h then after cooling, MeOH (20.0 mL) and Na₂CO₃ (1.000 g) was added and stirred 5 min. SiO₂ was added to make solid deposit and after concentration it was directly purified by flash chromatography with DCM/MeOH (98/2 to 95/5) as eluent to give **(3.19)** (0.580 g, 14 %) as a light brown solid.

**¹H NMR (400 MHz, DMSO-*d₆***) **δ :** 4.69 (d, *J* = 5.9 Hz, 2H, CH_{2 Bn}), 6.20 (s, 1H, H_{Ar}), 7.31 (dd, *J* = 7.5, 5.0 Hz, 1H, H_{Ar}), 7.39 (d, *J* = 7.8 Hz, 1H, H_{Ar}), 7.78 (tt, *J* = 7.7, 1.6 Hz, 1H, H_{Ar}), 8.55 (d, *J* = 4.8 Hz, 1H, H_{Ar}), 9.02 (s, 1H, NH_{Bn}), 9.42 (d, *J* = 1.3 Hz, 1H, H_{Ar}).

**¹³C NMR (101 MHz, DMSO-*d₆***) **δ :** 47.2 (CH_{2 Bn}), 92.4 (CH_{Ar}), 121.5 (CH_{Ar}), 122.6 (CH_{Ar}), 137.1 (CH_{Ar}), 139.0 (C_{q}), 139.6 (CH_{Ar}), 142.2 (C_{q}), 149.1 (CH_{Ar}), 150.0 (C_{q}), 156.6 (C_{q}).

**MS (ESI+) : m/z calcd for C₁₁H₉ClN₆ :** 261.1 [M+H]+, **found** : 261.2.

### Example 3.20: Synthesis of 6-chloro-3-methyl-N-(2-pyridylmethyl)-[1,2,4]triazolo[4,3-b]pyridazin-8-amine (3.20)

To a solution of **(1.2)** (4.000 g, 15.68 mmol, 1.0 eq.) in dioxane (52.0 mL), corresponding acid hydrazide (1.467 g, 18.82 mmol, 1.2 eq.) and APTS.H₂O (3.028 g, 15.68 mmol, 1.0 eq.) was added. The mixture was refluxed during 1 h then after cooling, MeOH (10.0 mL) and NH₃ 7N in MeOH (6.0 mL) was added and stirred 10 min. SiO₂ was added to make solid deposit and after concentration it was directly purified by flash chromatography with DCM/MeOH (98/2 to 95/5) as eluent to give **(3.20)** (1.150 g, 27 %) as a grey solid.

**¹H NMR (400 MHz, DMSO-*d₆***) **δ :** 2.59 (s, 3H, CH₃), 4.56 - 4.86 (m, 2H, CH_{2 Bn}), 6.16 (s, 1H, H_{Ar}), 7.30 (dd, *J* = 7.5, 4.9 Hz, 1H, H_{Ar}), 7.38 (d, *J* = 7.9 Hz, 1H, H_{Ar}), 7.78 (td, *J* = 7.7, 1.8 Hz, 1H, H_{Ar}), 8.48 - 8.60 (m, 1H, H_{Ar}), 8.93 (s, 1H, NH_{Bn}).

**¹³C NMR (101 MHz, DMSO-*d₆***) **δ** : 9.4 (CH₃), 47.2 (CH_{2 Bn}), 91.7 (CH_{Ar}), 121.4 (CH_{Ar}), 122.6 (CH_{Ar}), 137.0 (CH_{Ar}), 139.2 (C_{q}), 142.4 (C_{q}), 146.8 (C_{q}), 149.1 (CH_{Ar}), 149.6 (C_{q}), 156.6 (C_{q}).

**MS (ESI+) : m/z calcd for C₁₂H₁₁ClN₆ :** 275.1 [M+H]+, **found** : 275.2.

### Example 3.21: Synthesis of 6-chloro-3-ethyl-N-(2-pyridylmethyl)-[1,2,4]triazolo[4,3-b]pyridazin-8-amine (3.21)

In a sealed tube 10 - 20 mL with a stir bar was charged **(1.2)** (2.000 g, 7.84 mmol, 1.0 eq.), dioxane sec (15.6 mL), corresponding hydrazide (0.768 g, 8.62 mmol, 1.1 eq.) and AcOH (0.50 mL, 8.62 mmol, 1.1 eq.). The vial was sealed and then put on heating block, 23h h at 115°C. After cooling, the mixture was poured onto saturated NaHCO₃ (20.0 mL) then water (40.0 mL) was added. Aqueous mixture was extracted three times with EtOAc (3x30 mL) then organics layers were combined, dried over MgSO₄, filtered, concentrated and purified by flash chromatography with DCM/MeOH (96/4 to 94/6) as eluent to give **(3.21)** (0.550 g, 24 %) as a white solid.

**¹H NMR (400 MHz, DMSO-*d₆***) **δ :** 1.34 (t, *J* = 7.5 Hz, 3H, CH_{3-CH2}), 3.00 (q, *J* = 7.6 Hz, 2H, CH_{2-CH3}), 4.68 (d, *J* = 6.2 Hz, 2H, CH_{2 Bn}), 6.16 (s, 1H, H_{Ar}), 7.30 (dd, *J* = 7.5, 4.8 Hz, 1H, H_{Ar}), 7.38 (d, *J* = 7.8 Hz, 1H, H_{Ar}), 7.78 (td, *J* = 7.7, 1.8 Hz, 1H, H_{Ar}), 8.55 (d, *J* = 5.3 Hz, 1H, H_{Ar}), 8.96 (s, 1H, NH_{Bn}).

**¹³C NMR (101 MHz, DMSO-*d₆***) **δ** : 10.8 (CH_{3-CH2}), 17.3 (CH_{2-CH3}), 47.2 (CH_{2 Bn}), 91.8 (CH_{Ar}), 121.4 (CH_{Ar}), 122.6 (CH_{Ar}), 137.1 (CH_{Ar}), 139.3 (C_{q}), 142.5 (C_{q}), 149.1 (CH_{Ar}), 149.6 (C_{q}), 150.9 (C_{q}), 156.6 (C_{q}).

**MS (ESI+) : m/z calcd for C₁₃H₁₃ClN₆** : 289.1 [M+H]+, **found** : 289.2.

### Example 3.22: Synthesis of 6-chloro-3-propyl-N-(2-pyridylmethyl)-[1,2,4]triazolo[4,3-b]pyridazin-8-amine (3.22)

To a solution of (2.7) (~70 % purity, 0.800 g, 2.42 mmol, 1.0 eq.) in THF (11.0 mL), Et₃N (0.48 mL, 3.39 mmol, 1.4 eq.) and corresponding amine (0.367 g, 3.39 mmol, 1.4 eq.) was added and the mixture was refluxed during 1.5 h. After cooling, the solid was filtered off, washed with THF then the filtrate was concentrated and purified by flash chromatography with DCM/EtOAc (98/2 to 96/4) as eluent to give **(3.22)** (0.480 g, 65 %) as a white solid.

**¹H NMR (400 MHz, DMSO-*d₆***) **δ** : 0.96 (t, *J* = 7.4 Hz, 3H, CH₃), 1.80 (h, *J* = 7.4 Hz, 2H, CH₂), 2.97 (t, *J* = 7.5 Hz, 2H, CH₂), 4.58 - 4.81 (m, 2H, CH_{2 Bn}), 6.16 (s, 1H, H_{Ar}), 7.31 (dd, *J* = 7.5, 4.9 Hz, 1H, H_{Ar}), 7.38 (d, *J* = 7.9 Hz, 1H, H_{Ar}), 7.78 (td, *J* = 7.7, 1.8 Hz, 1H, H_{Ar}), 8.55 (dd, *J* = 5.2, 1.8 Hz, 1H, H_{Ar}), 8.96 (s, 1H, NH_{Bn}).

**¹³C NMR (101 MHz, DMSO-*d₆***) **δ** : 13.6 (CH₃), 19.4 (CH₂), 25.4 (CH₂), 47.2 (CH_{2 Bn}), 91.8 (CH_{Ar}), 121.4 (CH_{Ar}), 122.6 (CH_{Ar}), 137.0 (CH_{Ar}), 139.2 (C_{q}), 142.5 (C_{q}), 149.1 (CH_{Ar}), 149.5 (C_{q}), 149.8 (C_{q}), 156.6 (C_{q}).

**MS (ESI+) : m/z calcd for C₁₄H₁₅ClN₆** : 303.1 [M+H]+, **found** : 303.3.

### Example 3.23: Synthesis of 3-tert-butyl-6-chloro-N-(2-pyridylmethyl)-[1,2,4]triazolo[4,3-b]pyridazin-8-amine (3.23)

To a solution of **(1.2)** (2.000 g, 7.84 mmol, 1.0 eq.) in dry dioxane (31.0 mL), corresponding hydrazide (1.033 g, 8.62 mmol, 1.1 eq.) and AcOH (0.50 mL, 8.62 mmol, 1.1 eq.) was added then the mixture was refluxed 17 h. After cooling, the reaction mixture was poured onto saturated NaHCO₃ (100.0 mL) and EtOAc (60.0 mL). The biphasique mixture was vigorously stirred 10 min then layers were separated. Aqueous layer was extracted twice with EtOAc (2x 50.0 mL) then organics layers were combined, dried over MgSO₄, filtered, concentrated and purified by flash chromatography with DCM/MeOH (97/3 to 93/7) as eluent to give **(3.23)** (0.880 g, 35 %) as a white solid.

**¹H NMR (400 MHz, DMSO-d6) δ :** 1.50 (s, 9H, 3xCH_{3 tBu}), 4.67 (d, *J* = 6.9 Hz, 2H, CH_{2 Bn}), 6.15 (s, 1H, H_{Ar}), 7.30 (dd, *J* = 7.5, 4.9 Hz, 1H, H_{Ar}), 7.38 (d, *J* = 7.8 Hz, 1H, H_{Ar}), 7.78 (td, *J* = 7.7, 1.8 Hz, 1H, H_{Ar}), 8.49 - 8.63 (m, 1H, H_{Ar}), 8.96 (s, 1H, NH_{Bn}).

**¹³C NMR (101 MHz, DMSO-*d₆***) **δ** : 27.0 (3xCH_{3 tBu}), 32.4 (C_{q tBu}), 47.2 (CH_{2 Bn}), 91.5 (CH_{Ar}), 121.4 (CH_{Ar}), 122.6 (CH_{Ar}), 137.1 (CH_{Ar}), 140.2 (CH_{Ar}), 142.5 (C_{q}), 148.7 (C_{q}), 149.1 (C_{q}), 155.2 (C_{q}), 156.7 (C_{q}).

M**S (ESI+) : m/z calcd for C₁₅H₁₇ClN₆** : 317.13 [M+H]+, **found** : 317.3.

### Example 3.24: Synthesis of 6-chloro-3-cyclopropyl-N-(2-pyridylmethyl)-[1,2,4]triazolo[4,3-b]pyridazin-8-amine (3.24)

To a solution of **(1.2)** (4.000 g, 15.68 mmol, 1.0 eq.) in dioxane (52.0 mL), corresponding hydrazide (1.884 g, 18.82 mmol, 1.2 eq.) and AcOH (1.00 mL, 17.25 mmol, 1.1 eq.) was added. The mixture was refluxed 15h and after cooling, MeOH (20 mL) and DCM (20 mL) was added to solubilize reaction mixture. SiO₂ was added to make solid deposit and after concentration it was directly purified by flash chromatography with DCM/MeOH (98/2 to 95/5) as eluent to give **(3.24)** (1.620 g, 34 %) as a rose pale solid.
**Rf** (DCM/MeOH, 94/6) : 0.28

**¹H NMR (400 MHz, DMSO-*d₆***) **δ** : 1.02 - 1.23 (m, 4H, 2xCH_{2cPr}), 2.32 (td, *J* = 8.2, 4.3 Hz, 1H, H_{cPr}), 4.67 (d, *J* = 6.0 Hz, 2H, CH_{2 Bn}), 6.15 (s, 1H, H_{Ar}), 7.30 (dd, *J* = 7.4, 4.9 Hz, 1H, H_{Ar}), 7.37 (d, *J* = 7.8 Hz, 1H, H_{Ar}), 7.78 (t, *J* = 7.7 Hz, 1H, H_{Ar}), 8.55 (d, *J* = 4.9 Hz, 1H, H_{Ar}), 8.94 (s, 1H, NH_{Bn}).

**¹³C NMR (101 MHz, DMSO-*d₆***) **δ :** 4.8 (CH_{cPr}), 7.1 (2xCH_{2 cPr}), 47.2 (CH_{2 Bn}), 91.8 (CH_{Ar}), 121.4 (CH_{Ar}), 122.6 C(H_{Ar}), 137.0 (CH_{Ar}), 139.3 (C_{q}), 142.4 (C_{q}), 149.1 (CH_{Ar}), 149.6 (C_{q}), 151.4 (C_{q}), 156.6 (C_{q}).

**MS (ESI+) : m/z calcd for C₁₄H₁₃ClN₆** : 301.1 [M+H]+, **found** : 301.2.

### Example 3.25: Synthesis of 6-chloro-3-cyclobutyl-N-(2-pyridylmethyl)-[1,2,4]triazolo[4,3-b]pyridazin-8-amine (3.25)

In a sealed tube 10 - 20 mL with a stir bar was charged **(1.2)** (1.900 g, 7.45 mmol, 1.0 eq.), dioxane sec (15.0 mL), corresponding hydrazide (0.984 g, 8.19 mmol, 1.1 eq.) and AcOH (0.47 mL, 8.19 mmol, 1.1 eq.). The vial was sealed and then put on heating block, 23h h at 115°C. After cooling, the mixture was poured onto saturated NaHCO₃ (20.0 mL) then water (40.0 mL) was added. Aqueous mixture was extracted three times with EtOAc (3x30 mL) then organics layers were combined, dried over MgSO₄, filtered, concentrated and purified by flash chromatography with EtOAc (100 %) as eluent to give **(3.25)** (0.550 g, 23 %) as a white solid.
**Rf** (EtOAc, 100 %) : 0.14

**¹H NMR (400 MHz, DMSO-*d₆*) δ** : 1.91 - 2.03 (m, 1H, H_{(CH2 cBut)}), 2.12 (dq, *J* = 10.8, 8.6 Hz, 1H, H(_{CH2 cBut})), 2.43 (ddd, *J* = 12.2, 6.2, 2.9 Hz, 4H, 2xCH_{2 cBut}), 3.95 (p, *J* = 8.4 Hz, 1H, H_{CBut}), 4.68 (s, 2H, CH_{2 Bn}), 6.14 (s, 1H, H_{Ar}), 7.30 (dd, *J* = 7.5, 4.9 Hz, 1H, H_{Ar}), 7.38 (d, *J* = 7.9 Hz, 1H, H_{Ar}), 7.78 (td, *J* = 7.7, 1.8 Hz, 1H, H_{Ar}), 8.55 (d, *J* = 5.4 Hz, 1H, H_{Ar}), 8.97 (s, 1H, NH_{Bn}).

**¹³C NMR (101 MHz, DMSO-*d₆***) **δ :** N.D.

**MS (ESI+) : m/z calcd for C₁₅H₁₅ClN₆** : 315.1 [M+H]+, **found** : 315.3.

### Example 3.26: Synthesis of 6-chloro-3-cyclopentyl-N-(2-pyridylmethyl)-[1,2,4]triazolo[4,3-b]pyridazin-8-amine (3.26)

To a solution of **(1.2)** (4.000 g, 15.68 mmol, 1.0 eq.) in dioxane (52 mL), corresponding hydrazide (1.884 g, 18.82 mmol, 1.2 eq.) and AcOH (1.00 mL, 17.25 mmol, 0.1 eq.) was added. The mixture was refluxed 15h and after cooling, MeOH (20 mL) and DCM (20 mL) was added to solubilize reaction mixture. SiO₂ was added to make solid deposit and after concentration it was directly purified by flash chromatography with DCM/MeOH (94/6) as eluent to give **(3.26)** (1.620 g, 34 %) as a rose pale solid.

**¹H NMR (400 MHz, DMSO-*d₆***) **δ :** 1.64 - 1.85 (m, 4H, 2xCH_{2c Pent}), 1.93 (dq, *J* = 11.9, 7.2 Hz, 2H, CH_{2 cPent}), 2.10 (qd, *J* = 11.8, 9.6, 6.5 Hz, 2H, CH_{2 cPent}), 3.53 (p, *J* = 8.0 Hz, 1H, CH_{cPent}), 4.60 - 4.76 (s, 2H, CH_{2 Bn}), 6.15 (s, 1H, H_{Ar}), 7.30 (dd, *J* = 7.5, 4.8 Hz, 1H, H_{Ar}), 7.38 (d, *J* = 7.8 Hz, 1H, H_{Ar}), 7.78 (td, *J* = 7.7, 1.8 Hz, 1H, H_{Ar}), 8.51 - 8.61 (m, 1H, H_{Ar}), 8.95 (s, 1H, NH_{Bn}).

**¹³C NMR (101 MHz, DMSO-*d₆***) **δ :** N.D.

**MS (ESI+) : m/z calcd for C₁₆H₁₇ClN₆ :** 329.1 [M+H]+, **found** : 329.3.

### Example 3.27: Synthesis of 6-chloro-3-cyclohexyl-N-(2-pyridylmethyl)-[1,2,4]triazolo[4,3-b]pyridazin-8-amine (3.27)

In a sealed tube 10 - 20 mL with a stir bar was charged **(1.2)** (1.500 g, 5.88 mmol, 1.0 eq.), dioxane (17.8 mL), corresponding acid hydrazide (0.939 g, 6.47 mmol, 1.1 eq.) and AcOH (0.37 mL, 6.47 mmol, 1.1 eq.). The vial was sealed and then put on heating block, 21 h at 110 °C. The reaction mixture was cooled and MeOH (3.0 mL) was added. The mixture was concentrated with SiO₂ to make solid deposit and purified by flash chromatography with DCM/MeOH (97/3 to 93/7) as eluent to give **(3.27)** (0.800 g, 40 %) as a white solid.

**¹H NMR (400 MHz, DMSO-*d₆***) **δ :** 1.39 (dt, *J* = 36.4, 12.5 Hz, 3H, H(_{CH2}) _{cHex} & CH_{2 chex}), 1.67 (dt, *J* = 24.1, 12.5 Hz, 3H, H(_{cH2}) _{cHex} & CH_{2 cHex}), 1.81 (d, *J* = 12.3 Hz, 2H, CH_{2 cHex}), 2.01 (d, *J* = 12.6 Hz, 2H, CH_{2 cHex}), 3.15 (t, *J* = 11.5 Hz, 1H, H_{cHex}), 4.68 (s, 2H, CH_{2 Bn}), 6.14 (s, 1H, H_{Ar}), 7.30 (dd, *J* = 7.5, 4.9 Hz, 1H, H_{Ar}), 7.38 (d, *J* = 7.7 Hz, 1H, H_{Ar}), 7.78 (td, *J* = 7.7, 3.7 Hz, 1H, H_{Ar}), 8.51 - 8.63 (m, 1H, H_{Ar}), 8.97 (s, 1H, NH_{Bn}).

**¹³C NMR (101 MHz, DMSO-*d₆***) **δ** : 25.3 (2xCH_{2 cHex}), 25.5 (CH_{2 cHex}), 29.8 (2xCH_{2 cHex}), 33.3 (CH_{cHex}), 47.2 (CH_{2 Bn}), 91.8 (CH_{Ar}), 121.4 (CH_{Ar}), 122.6 (CH_{Ar}), 129.5 (C_{q}), 137.1 (CH_{Ar}), 139.2 (C_{q}), 142.5 (C_{q}), 149.1 (CH_{Ar}), 149.4 (C_{q}), 153.2 (C_{q}).

**MS (ESI+) : m/z calcd for C₁₇H₁₉ClN₆ :** 343.1 [M+H]+, **found** : 343.3.

### Example 3.28: Synthesis of 6-chloro-3-phenyl-N-(2-pyridylmethyl)-[1,2,4]triazolo[4,3-b]pyridazin-8-amine (3.28)

In a sealed tube 10 - 20 mL with a stir bar was charged **(1.2)** (1.500 g, 5.88 mmol, 1.0 eq.), dioxane (17.8 mL), corresponding acid hydrazide (0.899 g, 6.47 mmol, 1.1 eq.) and AcOH (0.37 mL, 6.47 mmol, 1.1 eq.). The vial was sealed and then put on heating block, 21 h at 110 °C. The reaction mixture was cooled and MeOH (3.0 mL) was added. The mixture was concentrated with SiO₂ to make solid deposit and purified by flash chromatography with DCM/MeOH (97/3 to 93/7) as eluent to give **(3.28)** (0.750 g, 38 %) as a beige solid.

**¹H NMR (400 MHz, DMSO-*d₆*) δ** : 4.73 (d, *J* = 5.9 Hz, 2H, CH_{2 Bn}), 7.32 (dd, *J* = 7.5, 4.9 Hz, 1H, H_{Ar}), 7.42 (d, *J* = 7.8 Hz, 1H, H_{Ar}), 7.59 (dt, *J* = 13.6, 7.1 Hz, 3H, 3xH_{Ar}), 7.80 (t, *J* = 7.7 Hz, 1H, H_{Ar}), 8.32 (d, *J* = 7.6 Hz, 2H, H_{Ar}), 8.57 (d, *J* = 4.9 Hz, 1H, H_{Ar}), 9.11 (s, 1H, NH_{Bn}).

**¹³C NMR (101 MHz, DMSO-*d₆***) **δ :** N.D.

**MS (ESI+) : m/z calcd for C₁₇H₁₃ClN₆ :** 337.1 [M+H]+, **found** : 337.3.

### Example 3.29: Synthesis of 6-chloro-N-(2-pyridylmethyl)-3-sec-butyl-[1,2,4]triazolo[4,3-b]pyridazin-8-amine (3.29)

To a solution of **(2.2)** (0.565 g, 2.31 mmol, 1.0 eq.) in THF (23.0 mL), corresponding amine (0.44 mL, 3.00 mmol, 1.3 eq.) and DIPEA (0.61 mL, 3.46 mmol, 1.5 eq.) was added and the mixture was refluxed during 5 h. After cooling, the solvent was removed, the crude was triturated in H₂O (10.0 mL) and saturated NaHCO₃ (10.0 mL) and the resulting solid was filtered off, washed with water up to neutral pH then Et₂O and dried under vacuum to give **(3.29)** (0.600 g, 82 %) as a white solid.

**¹H NMR (400 MHz, DMSO-*d₆***) **δ** : 0.84 (t, *J* = 7.4 Hz, 3H, CH_{3-CH2}), 1.35 (d, *J* = 7.0 Hz, 3H, CH_{3-CH}), 1.82 (ddq, *J* = 68.8, 13.8, 7.1 Hz, 2H, CH_{2-CH3}), 3.24 - 3.31 (m, 1H, H_{C-CH3}), 4.67 (d, *J* = 5.7 Hz, 2H, CH_{2 Bn}), 6.15 (s, 1H, H_{Ar}), 7.31 (dd, *J* = 7.5, 4.9 Hz, 1H, H_{Ar}), 7.39 (d, *J* = 7.9 Hz, 1H, H_{Ar}), 7.78 (td, *J* = 7.7, 1.8 Hz, 1H, H_{Ar}), 8.55 (d, *J* = 4.9 Hz, 1H, H_{Ar}), 8.98 (s, 1H, NH_{Bn}).

**¹³C NMR (101 MHz, DMSO-*d₆***) **δ** : 11.4 (CH_{3-CH2}), 17.6 (CH_{3-CH}), 26.8 (CH_{2-CH3}), 30.9 (CH_{-CH3}), 47.2 (CH_{2 Bn}), 91.8 (CH_{Ar}), 121.5 (CH_{Ar}), 122.6 (CH_{Ar}), 137.1 (CH_{Ar}), 139.3 (C_{q}), 142.5 (C_{q}), 149.1 (CH_{Ar}), 149.4 (C_{q}), 153.2 (C_{q}), 156.6 (C_{q}).

**MS (ESI+)** : **m/z calcd for C₁₅H₁₇ClN₆:** 317.8 [M+H]+, **found** : 317.2.

### Example 3.30: Synthesis of 6-chloro-3-(1-ethylpropyl)-N-(2-pyridylmethyl)-[1,2,4]triazolo[4,3-b]pyridazin-8-amine (3.30)

To a solution of **(2.3)** (0.510 g, 1.97 mmol, 1.0 eq.) in THF (20.0 mL), corresponding amine (0.38 mL, 2.56 mmol, 1.3 eq.) and DIPEA (0.52 mL, 2.95 mmol, 1.5 eq.) was added and the mixture was refluxed during 5 h. After cooling, the solvent was removed, the crude was triturated in H₂O (10.0 mL) and saturated NaHCO₃ (10.0 mL) and the resulting solid was filtered off, washed with water up to neutral pH then Et₂O and dried under vacuum to give (3.30) (0.580 g, 89 %) as a white solid.

**¹H NMR (400 MHz, DMSO-*d₆***) **δ :** 0.77 (t, *J* = 7.4 Hz, 6H, 2xCH_{3-CH2}), 1.83 (ddt, *J* = 37.0, 13.5, 6.9 Hz, 4H, 2xCH_{2-CH3}), 3.10 - 3.24 (m, 1H, H_{iPent}), 4.67 (d, *J* = 5.8 Hz, 2H, CH_{2 Bn}), 6.15 (s, 1H, H_{Ar}), 7.31 (dd, *J* = 7.4, 4.9 Hz, 1H, H_{Ar}), 7.40 (d, *J* = 7.9 Hz, 1H, H_{Ar}), 7.79 (td, *J* = 7.7, 1.7 Hz, 1H, H_{Ar}), 8.55 (d, *J* = 4.9 Hz, 1H, H_{Ar}), 8.98 (s, 1H, NH_{Bn}).

**¹³C NMR (101 MHz, DMSO-*d₆***) **δ :** N.D.

**MS (ESI+): m/z calcd for C₁₆H₁₉ClN₆ :** 331.1 [M+H]+, **found** : 331.2.

### Example 3.31: Synthesis of 6-chloro-3-isobutyl-N-(2-pyridylmethyl)-[1,2,4]triazolo[4,3-b]pyridazin-8-amine (3.31)

To a solution of **(2.4)** (0.290 g, 1.18 mmol, 1.0 eq.), THF (11.0 mL), corresponding amine (0.23 mL, 1.54 mmol, 1.3 eq.) and DIPEA (0.31 mL, 1.78 mmol, 1.5 eq.) was added then the mixture was refluxed 1 h. After cooling, Et₂O (30.0 mL), the precipitate was filtered off, washed with water, triturated in Et₂O then dried under vacuum to give **(3.31)** (0.285 g, 76 %) as a white solid.

**¹H NMR (400 MHz, DMSO-*d₆***) **δ :** 0.94 (d, *J* = 6.6 Hz, 6H, 2xCH_{3 iPr}), 2.21 (hept, *J* = 6.8 Hz, 1H, H_{iPr}), 2.89 (d, *J* = 7.2 Hz, 2H, CH_{2-CH}), 4.68 (s, 2H, CH_{2 Bn}), 6.16 (s, 1H, H_{Ar}), 7.31 (dd, *J* = 7.5, 4.9 Hz, 1H, H_{Ar}), 7.39 (d, *J* = 7.8 Hz, 1H, H_{Ar}), 7.79 (td, *J* = 7.7, 1.8 Hz, 1H, H_{Ar}), 8.55 (d, *J* = 4.7 Hz, 1H, H_{Ar}), 8.95 (s, 1H, NH_{Bn}).

**¹³C NMR (101 MHz, DMSO-*d₆***) **δ :** N.D.

**MS (ESI+) : m/z calcd for C₁₅H₁₇ClN₆:** 317.8 [M+H]+, **found** : 317.2.

### Example 3.32: Synthesis of 6-chloro-3-(2-pyridyl)-N-(2-pyridylmethyl)-[1,2,4]triazolo[4,3-b]pyridazin-8-amine (3.32)

In a sealed tube 10 - 20 mL with a stir bar was charged **(1.2)** (1.250 g, 4.90 mmol, 1.0 eq.), dioxane (10.0 mL), corresponding acid hydrazide (0.778 g, 5.39 mmol, 1.1 eq.) and PTSA.H₂O (0.946 g, 4.90 mmol, 1.0 eq.). The vial was sealed and then put on heating block, 19 h at 110 °C. After cooling, MeOH (5.0 mL) and NH₃ 7M in MeOH (3.0 mL) was added then the mixture was concentrated with SiO₂ to make solid deposit and directly purified by flash chromatography with DCM/MeOH (98/2 to 90/10) as eluent to give **(3.32)** (0.300 g, 18 %) as a yellow solid.
**Rf** (DCM/MeOH, 94/6) : 0.10

**¹H NMR (400 MHz, DMSO-*d₆***) **δ** : 4.74 (s, 2H, CH_{2 Bn}), 6.32 (s, 1H, H_{Ar}), 7.32 (dd, *J* = 7.5, 5.0 Hz, 1H, H_{Ar}), 7.43 (d, *J* = 7.9 Hz, 1H, H_{Ar}), 7.53 - 7.65 (m, 1H, H_{Ar}), 7.80 (td, *J* = 7.7, 1.8 Hz, 1H, H_{Ar}), 8.05 (td, *J* = 7.8, 1.8 Hz, 1H, H_{Ar}), 8.23 (d, *J* = 7.9 Hz, 1H, H_{Ar}), 8.57 (d, *J* = 4.9 Hz, 1H, H_{Ar}), 8.81 (d, *J* = 4.9 Hz, 1H, H_{Ar}), 9.13 (s, 1H, NH_{Bn}).

**¹³C NMR (101 MHz, DMSO-*d₆***) **δ :** N.D.

**MS (ESI+) : m/z calcd for C₁₆H₁₂ClN₇** : 338.1 [M+H]+, **found** : 338.2.

### Example 3.33: Synthesis of 6-chloro-3-(3-pyridyl)-N-(2-pyridylmethyl)-[1,2,4]triazolo[4,3-b]pyridazin-8-amine (3.33)

In a sealed tube 10 - 20 mL with a stir bar was charged (1.2) (1.250 g, 4.90 mmol, 1.0 eq.), dioxane (10.0 mL), corresponding hydrazide (0.754 g, 5.39 mmol, 1.1 eq.) and PTSA.H₂O (0.946 g, 4.90 mmol, 1.0 eq.). The vial was sealed and then put on heating block, 19h h at 110 °C. After cooling, NH₃ 7N in MeOH (2.80 mL, 19.6 mmol, 4.0 eq.) was added and the mixture was concentrated with silica then directly purified by flash chromatography with DCM/MeOH (98/2 to 94/6) as eluent to give **(3.33)** (0.275 g, 17 %) as a white solid.
**Rf** (DCM/MeOH, 94/6) : 0.25

**¹H NMR (400 MHz, DMSO-*d₆*) δ :** 4.74 (s, 2H, CH_{2 Bn}), 6.34 (s, 1H, H_{Ar}), 7.24 - 7.39 (m, 1H, H_{Ar}), 7.42 (d, *J* = 7.9 Hz, 1H, H_{Ar}), 7.65 (dd, *J* = 8.1, 4.9 Hz, 1H, H_{Ar}), 7.75 - 7.90 (m, 1H, H_{Ar}), 8.57 (d, *J* = 4.8 Hz, 1H, H_{Ar}), 8.64 (dt, *J* = 8.1, 2.0 Hz, 1H, H_{Ar}), 8.74 (dd, *J* = 4.8, 1.7 Hz, 1H, H_{Ar}), 9.19 (s, 1H, NH_{Bn}), 9.44 (d, *J* = 2.2 Hz, 1H, H_{Ar}).

**¹³C NMR (101 MHz, DMSO-*d₆*) δ : N.D.**

**MS (ESI+) : m/z calcd for C₁₆H₁₂ClN₇ :** 338.1 [M+H]+, **found :** 338.2.

### Example 3.34: Synthesis of 6-chloro-3-(4-pyridyl)-N-(2-pyridylmethyl)-[1,2,4]triazolo[4,3-b]pyridazin-8-amine (3.34)

In a sealed tube 10 - 20 mL with a stir bar was charged **(1.2)** (1.250 g, 4.90 mmol, 1.0 eq.), dioxane (10.0 mL), corresponding hydrazide (0.778 g, 5.39 mmol, 1.1 eq.) and PTSA.H₂O (0.946 g, 4.90 mmol, 1.0 eq.). The vial was sealed and then put on heating block, 19h h at 110 °C. After cooling, NH₃ 7N in MeOH (2.80 mL, 19.6 mmol, 4.0 eq.) was added and the mixture was concentrated with silica then directly purified by flash chromatography with DCM/MeOH (98/2 to 94/6) as eluent to give **(3.34)** (0.310 g, 19 %) as a white solid.
**Rf** (DCM/MeOH, 94/6) : 0.29

**¹H NMR (400 MHz, DMSO-*d₆*) δ :** 4.74 (d, *J* = 6.1 Hz, 2H, CH_{2 Bn}), 6.37 (s, 1H, H_{Ar}), 7.32 (dd, *J* = 7.3, 5.1 Hz, 1H, H_{Ar}), 7.42 (d, *J* = 7.8 Hz, 1H, H_{Ar}), 7.80 (dd, *J* = 8.2, 6.5 Hz, 1H, H_{Ar}), 8.23 - 8.38 (m, 2H, 2xH_{Ar}), 8.56 (d, *J* = 4.9 Hz, 1H, H_{Ar}), 8.72 - 8.92 (m, 2H, 2xH_{Ar}), 9.21 (s, 1H, NH_{Bn}).

**¹³C NMR (101 MHz, DMSO-*d₆*) δ :** N.D.

**MS (ESI+)** : **m/z calcd for C₁₆H₁₂ClN₇** : 338.1 [M+H]+, **found** : 338.2.

### Example 3.35: Synthesis of N-[(4-aminophenyl)methyl]-6-chloro-3-isopropyl-[1,2,4]triazolo[4,3-b]pyridazin-8-amine (3.35)

To a solution of **(2.1)** (0.300 g, 1.30 mmol, 1.0 eq.) in THF (18.0 mL), 4-(aminomethyl)aniline dihydrochloride (0.512 g, 2.60 mmol, 2.0 eq.) and Et₃N (1.28 mL, 9.09 mmol, 7.0 eq.) were added then the mixture was refluxed 2 h. After cooling, the solid was filtered off, washed with THF then the filtrate was concentrated and directly purified by flash chromatography with DCM/MeOH (98/2 to 96/4) as eluent to give (3.35) (0.245 g, 60 %) as a pale yellow solid.
**Rf** (DCM/MeOH, 94/6) : 0.24

**¹H NMR (400 MHz, DMSO-*d₆*) δ :** 1.37 (d, *J* = 7.0 Hz, 6H, 2×CH_{3 iPr}), 3.42 (h, *J* = 6.9 Hz, 1H, H_{iPr}), 4.37 (d, *J* = 6.3 Hz, 2H, CH_{2 Bn}), 5.01 (s, 2H, NH₂), 6.10 (s, 1H, H_{Ar}), 6.51 (d, *J* = 8.4 Hz, 2H, 2xH_{Ar}), 7.05 (d, *J* = 8.3 Hz, 2H, 2xH_{Ar}), 8.95 (t, *J* = 6.2 Hz, 1H, NH_{Bn}).

**¹³C NMR (101 MHz, DMSO-*d₆*) δ** : 19.8 (2×CH_{3 iPr}), 24.4 (CH_{iPr}), 45.0 (CH_{2 Bn}), 91.3 (CH_{Ar}), 113.8 (2×CH_{Ar}), 124.0 (C_{q}), 128.2 (2×CH_{Ar}), 139.5 (C_{q}), 142.1 (C_{q}), 147.9 (C_{q}), 149.4 (C_{q}), 153.9 (C_{q}).

**MS (ESI+)** : **m/z calcd for C₁₅H₁₇ClN₆** : 317.1 [M+H]+, **found** : 317.2.

### Example 3.36: Synthesis of N-[(3-aminophenyl)methyl]-6-chloro-3-isopropyl-[1,2,4]triazolo[4,3-b]pyridazin-8-amine (3.36)

To a solution of **(2.1)** (0.300 g, 1.30 mmol, 1.0 eq.) in THF (18.0 mL), 3-(aminomethyl)aniline (0.320 g, 2.60 mmol, 2.0 eq.) and Et₃N (0.37 mL, 2.60 mmol, 2.0 eq.) were added then the mixture was refluxed 2 h. After cooling, the solid was filtered off, washed with THF then the filtrate was concentrated and directly purified by flash chromatography with DCM/MeOH (98/2 to 96/4) as eluent to give (3.36) (0.280 g, 68 %) as a yellow solid.
**Rf** (DCM/MeOH, 96/4) : 0.27

**¹H NMR (400 MHz, DMSO-*d₆*) δ** : 1.38 (d, *J* = 6.9 Hz, 6H, 2×CH_{3 iPr}), 3.42 (hept, *J* = 6.9 Hz, 1H, H_{iPr}), 4.43 (d, *J* = 6.4 Hz, 2H, CH_{2 Bn}), 5.06 (s, 2H, NH₂), 6.02 (s, 1H, H_{Ar}), 6.40 - 6.56 (m, 3H, 3×H_{Ar}), 6.96 (t, *J* = 7.7 Hz, 1H, H_{Ar}), 9.03 (s, 1H, NH_{Bn}).

**¹³C NMR (101 MHz, DMSO-*d₆*) δ :** N.D.

**MS (ESI+)** : **m/z calcd for C₁₅H₁₇ClN₆** : 317.13 [M+H]+, **found** : 317.3.

### Example 3.37: Synthesis of N-[(2-aminophenyl)methyl]-6-chloro-3-isopropyl-[1,2,4]triazolo[4,3-b]pyridazin-8-amine (3.37)

To a solution of **(1.12)** (4.000 g, 14.86 mmol, 1.0 eq.) in dry dioxane (60.0 mL), isobutyric acid hydrazide (1.840 g, 17.84 mmol, 1.2 eq.) and AcOH (0.86 mL, 14.86 mmol, 1.0 eq.) was added then the mixture was refluxed 18 h. After cooling, the reaction mixture was concentrated, the residue was triturated in water, filtered off, washed with water up to neutral pH. The solid was taken up with DCM and MeOH, the filtrate was concentrated and purified by flash chromatography with DCM/MeOH (97/3 to 95/5) as eluent to give (3.37) (0.600 g, 13 %) as a pale yellow solid.
**Rf** (DCM/MeOH, 94/6) : 0.41

**¹H NMR (400 MHz, DMSO-*d₆*) δ :** 1.37 (d, *J* = 6.9 Hz, 6H, 2×CH_{3 iPr}), 3.42 (p, *J* = 6.9 Hz, 1H, H_{iPr}), 4.39 (s, 2H, CH_{2 Bn}), 5.17 (s, 2H, NH₂), 6.07 (s, 1H, H_{Ar}), 6.51 (t, *J* = 7.4 Hz, 1H, H_{Ar}), 6.65 (d, *J* = 7.9 Hz, 1H, H_{Ar}), 6.97 (td, *J* = 7.6, 1.6 Hz, 1H, H_{Ar}), 7.09 (dd, *J* = 7.6, 1.6 Hz, 1H, H_{Ar}), 8.92 (s, 1H, NH_{Bn}).

**¹³C NMR (101 MHz, DMSO-*d₆*) δ** : 19.8 (2×CH_{3 iPr}), 24.4 (CH_{iPr}), 42.4 (CH_{2 Bn}), 91.6 (CH_{Ar}), 115.1 (CH_{Ar}), 115.9 (CH_{Ar}), 119.3 (C_{q}), 128.1 (CH_{Ar}), 128.2 (CH_{Ar}), 139.5 (C_{q}), 142.3 (C_{q}), 146.3 (C_{q}), 149.4 (C_{q}), 154.0 (C_{q}).

**MS (ESI+)** : **m/z calcd for C₁₅H₁₇ClN₆** : 317.1 [M+H]+, **found** : 317.3.

### Example 3.38: Synthesis of 2-(6-chloro-3-isopropyl-[1,2,4]triazolo[4,3-b]pyridazin-8-yl)-3,4-dihydro-1H-isoquinoline (3.38)

To a suspension of **(1.15)** (4.600 g, 16.42 mmol, 1.0 eq.) in dry dioxane (66 mL), isobutyric acid hydrazide (1.863 g, 18.06 mmol, 1.1 eq.) and AcOH (1.04 mL, 18.06 mmol, 1.1 eq.) was added then the mixture was refluxed 22 h. After cooling, MeOH (10.0 mL) was added, the mixture was concentrated with SiO₂ to make solid deposit and directly purified by flash chromatography with DCM/EtOAc (98/2 to 96/4) as eluent to give **(3.38)** (1.390 g, 26 %) as a white solid.
**Rf** (DCM/EtOAc, 98/2) : 0.23

**¹H NMR (400 MHz, DMSO-*d₆*) δ :** 1.38 (d, *J* = 7.0 Hz, 6H, 2×CH_{3 iPr}), 3.02 (t, *J* = 5.9 Hz, 2H, CH_{2-CH2-N}), 3.44 (hept, *J* = 6.9 Hz, 1H, H_{iPr}), 4.42 (s, 2H, _{N-}CH_{2-CH2}), 5.18 (s, 2H, CH_{2-N}), 6.40 (s, 1H, H_{Ar}), 7.24 (td, *J* = 4.9, 4.5, 2.6 Hz, 4H, 4×H_{Ar}).

**¹³C NMR (101 MHz, DMSO-*d₆*) δ :** 19.7 (2×CH_{3 iPr}), 24.4 (CH_{iPr}), 28.0 (CH_{2-CH2-N}), 45.9 (_{N-}CH_{2-CH2}), 49.3 (CH_{2-N}), 94.0 (CH_{Ar}), 126.3 (CH_{Ar}), 126.3 (CH_{Ar}), 126.8 (CH_{Ar}), 128.3 (CH_{Ar}), 134.5 (C_{q}), 140.4 (C_{q}), 142.7 (C_{q}), 149.4 (C_{q}), 153.6 (C_{q}).

**MS (ELMS) : m/z calcd for C₁₇H₁₈ClN₅ : 328.1 [M+H]+, found : 328.2.**

### Example 3.39: Synthesis of 6-chloro-8-isoindolin-2-yl-3-isopropyl-[1,2,4]triazolo[4,3-b]pyridazine (3.39)

To a solution of **(2.1)** (0.350 g, 1.52 mmol, 1.0 eq.) in THF (15.0 mL), corresponding amine hydrochloride (0.340 g, 2.12 mmol, 1.4 eq.) and Et₃N (0.64 mL, 4.54 mmol, 3.0 eq.) was added then the mixture was refluxed 1.5 h. After cooling, the precipitate was filtered, washed with THF then the filtrate was concentrated and purified by flash chromatography with DCM/MeOH (100/0 to 98/2) as eluent. The impur fraction was concentrated then the residu was triturated in Et₂O, filtered, washed with a little amount of Et₂O and dried under vacuum to give **(3.39)** (0.280 g, 59 %) as a white solid.

**¹H NMR (400 MHz, DMSO-*d₆*) δ :** 1.40 (d, *J* = 7.0 Hz, 6H, 2×CH_{3 iPr}), 3.46 (hept, *J* = 6.9 Hz, 1H, H_{iPr}), 4.87 (s, 2H, CH₂), 5.61 (s, 2H, CH₂), 6.10 (s, 1H, H_{Ar}), 7.37 (dd, *J* = 5.6, 3.2 Hz, 2H, 2xH_{Ar}), 7.44 (s, 1H, H_{Ar}), 7.50 (s, 1H, H_{Ar}).

**¹³C NMR (101 MHz, DMSO-*d₆*) δ :** 19.7 (2×CH_{3 iPr}), 24.4 (CH_{iPr}), 54.9 (CH₂), 57.4 (CH₂), 93.4 (CH_{Ar}), 122.5 (CH_{Ar}), 122.8 (CH_{Ar}), 127.6 (2×CH_{Ar}), 134.8 (C_{q}), 136.7 (C_{q}), 140.2 (C_{q}), 141.5 (C_{q}), 149.3 (C_{q}), 153.6 (C_{q}).

**MS (EI-MS)** : **m/z calcd for C₁₆H₁₆ClN₅** : 314.1 [M+H]+, **found** : 314.3.

### Example 3.40: Synthesis of 6-chloro-N-indan-1-yl-3-isopropyl-[1,2,4]triazolo[4,3-b]pyridazin-8-amine (3.40)

To a solution of **(2.1)** (0.250 g, 1.05 mmol, 1.0 eq.) in THF (13.0 mL), corresponding amine hydrochloride (0.273 g, 1.57 mmol, 1.5 eq.) and Et₃N (0.44 mL, 3.15 mmol, 3.0 eq.) was added then the mixture was refluxed 4 h. After cooling, the solvent was removed, the residue was triturated in water, filtered off, washed with water up to neutral pH and the solid was taken up in EtOAc. Organic filtrate was dried over MgSO₄, filtered, concentrated and purified by flash chromatography with DCM/MeOH (99/1 to 95/5) as eluent to give **(3.40)** (0.300 g, 87 %) as a beige solid.

**¹H NMR (400 MHz, DMSO-*d₆*) δ :** 1.39 (d, *J* = 6.9 Hz, 6H, 2×CH_{3 iPr}), 2.19 (dt, *J* = 17.4, 8.0 Hz, 1H, H_{(CH2)indane}), 2.55 (s, 1H, H_{(CH2)indane}), 2.85 (dt, *J* = 16.2, 8.3 Hz, 1H, H_{(CH2)indane}), 3.03 (ddd, *J* = 15.9, 8.9, 3.1 Hz, 1H, H_{(CH2)indane}), 3.45 (hept, *J* = 7.7, 7.2 Hz, 1H, H_{iPr}), 5.41 (s, 1H, Hi_{ndane}), 6.48 (s, 1H, H_{Ar}), 7.14 - 7.34 (m, 4H, 4×H_{Ar}), 8.75 (s, 1H, NH_{indane}).

**¹³C NMR (101 MHz, DMSO-*d₆*) δ :** 19.9 (2×CH_{3 iPr}), 24.4 (CH_{iPr}), 29.8 (CH_{2 indane}), 31.6 (CH_{2 indane}), 57.3 (CH_{indane}), 91.4 (CH_{Ar}), 123.8 (CH_{Ar}), 124.8 (CH_{Ar}), 126.4 (CH_{Ar}), 127.8 (CH_{Ar}), 139.4 (C_{q}), 142.3 (C_{q}), 142.7 (C_{q}), 143.1 (C_{q}), 149.8 (C_{q}), 154.0 (C_{q}).

**MS (ESI+) : m/z calcd for C₁₇H₁₈ClN₅ :** 328.1 [M+H]+, **found :** 328.3.

### Example 3.41: Synthesis of 6-chloro-N-(1H-indol-2-ylmethyl)-3-isopropyl-[1,2,4]triazolo[4,3-b]pyridazin-8-amine (3.41)

In a sealed tube 10 - 20 mL with a stir bar was charged **(1.10)** (1.100 g, 3.57 mmol, 1.0 eq.), dioxane (15.0 mL), isobutyric acid hydrazide (0.441 g, 4.28 mmol, 1.2 eq.) and AcOH (0.23 mL, 3.92 mmol, 1.1 eq.). The vial was sealed and then put on heating block, 17 h at 100 °C. After cooling, MeOH (10.0 mL) was added, stirred vigorously 5 min then the mixture was concentrated with SiO₂ to make solid deposit and directly purified by flash chromatography with DCM/MeOH (97/3 to 94/6) as eluent to give **(3.41)** (0.270 g, 22 %) as a beige solid.

**¹H NMR (400 MHz, DMSO-*d₆*) δ :** 1.38 (d, *J* = 6.9 Hz, 6H, 2×CH_{3 iPr}), 3.43 (hept, *J* = 7.1 Hz, 1H, H_{iPr}), 4.74 (s, 2H, CH_{2 Bn}), 6.29 (s, 1H, H_{Ar}), 6.36 (d, *J* = 2.0 Hz, 1H, H_{Ar}), 6.94 (t, *J* = 7.4 Hz, 1H, H_{Ar}), 7.00 - 7.08 (m, 1H, H_{Ar}), 7.33 (d, *J* = 8.0 Hz, 1H, H_{Ar}), 7.45 (d, *J* = 7.8 Hz, 1H, H_{Ar}), 8.89 (s, 1H, NH), 10.99 (s, 1H, NH_{indol}).

**¹³C NMR (101 MHz, DMSO-*d₆*) δ :** N.D.

**MS (ESI+)** : **m/z calcd for C₁₇H₁₇ClN₆** : 341.1 [M+H]+, **found** : 341.3.

### Example 3.42: Synthesis of N-[(4-bromophenyl)methyl]-6-chloro-3-isopropyl-[1,2,4]triazolo[4,3-b]pyridazin-8-amine (3.42)

To a solution of **(1.13)** (4.100 g, 12.32 mmol, 1.0 eq.) in dioxane (50.0 mL), isobutyric acid hydrazide (1.524 g, 14.77 mmol, 1.2 eq.) and AcOH (0.78 mL, 13.54 mmol, 1.1 eq.) was added then the miture was refluxed 18 h. After cooling, the mixture was poured onto water (200.0 mL) then the precipitate was triturated, filtered off, washed with water up to neutral pH. The solid was triturated in EtOAc, filtered and dried under vacuum to give **(3.42)** (2.400 g, 51 %) as a pale yellow solid.

**¹H NMR (400 MHz, DMSO-*d₆*) δ :** 1.37 (d, *J* = 6.9 Hz, 6H, 2×CH_{3 iPr}), 3.42 (hept, *J* = 7.0 Hz, 1H, H_{iPr}), 4.40 - 4.73 (m, 2H, CH_{2 Bn}), 6.15 (s, 1H, H_{Ar}), 7.35 (d, *J* = 8.2 Hz, 2H, 2xH_{Ar}), 7.54 (d, *J* = 8.4 Hz, 2H, 2xH_{Ar}), 9.11 (s, 1H, NH_{Bn}).

**¹³C NMR (101 MHz, DMSO-*d₆*) δ :** N.D.

**MS (ESI+)** : **m/z calcd for C₁₅H₁₅BrClN₅** : 380.0 [M+H]+, **found** : 380.2.

### Example 3.43: Synthesis of 6-chloro-3-isopropyl-N-(2-phenylethyl)-[1,2,4]triazolo[4,3-b]pyridazin-8-amine (3.43)

To a solution of **(1.16)** (4.000 g, 14.92 mmol, 1.0 eq.) in dry dioxane (60 mL), isobutyric acid hydrazide (1.847 g, 17.90 mmol, 1.2 eq.) then AcOH (0.95 mL, 16.41 mmol, 1.1 eq.) was added and the mixture was refluxed 4 h. After cooling, the mixture was concentrated then the residue was suspended and triturated into water (120 mL). The solid was filtered off, washed with water up to neutral pH then taken up with EtOH, concentrated en dried under vacuum to give the title compound **(3.43)** (2.350 g, 50 %) as a white solid.

**¹H NMR (400 MHz, DMSO-*d₆*) δ :** 1.37 (d, *J* = 7.0 Hz, 6H, 2×CH_{3 iPr}), 2.94 (t, *J* = 7.3 Hz, 2H, CH_{2-CH2-NH}), 3.42 (p, *J* = 6.9 Hz, 1H, H_{iPr}), 3.52 - 3.68 (m, 2H, CH_{2-NH}), 6.21 (s, 1H, H_{Ar}), 7.20 (tt, *J* = 5.6, 2.9 Hz, 1H, H_{Ar}), 7.25 - 7.38 (m, 4H, 4×H_{Ar}), 8.51 (s, 1H, NH).

**¹³C NMR (101 MHz, DMSO-*d₆*) δ :** 19.8 (2×CH_{3 iPr}), 24.4 (CH_{iPr}), 33.8 (CH_{2-CH2-NH}), 43.5 (CH_{2-NH}), 91.0 (CH_{Ar}), 126.2 (CH_{Ar}), 128.3 (2×CH_{Ar}), 128.9 (2×CH_{Ar}), 138.9 (C_{q}), 139.4 (C_{q}), 142.2 (C_{q}), 149.7 (C_{q}), 153.9 (C_{q}).

**MS (ESI+)** : **m/z calcd for C₁₆H₁₈ClN₅** : 316.1 [M+H]+, **found** : 316.2.

### Example 3.44: Synthesis of 6-chloro-3-isopropyl-N-[2-(2-pyridyl)ethyl]-[1,2,4]triazolo[4,3-b]pyridazin-8-amine (3.44)

To a solution of **(2.1)** (0.300 g, 1.30 mmol, 1.0 eq.) in THF (3.9 mL), corresponding amine (0.190 g, 1.56 mmol, 1.2 eq.) and DIPEA (0.27 mL, 1.56 mmol, 1.2 eq.) was added and the mixture was stirred 16 h at room temperature. The precitpitate was filtered off, washed with THF then the filtrate was concentrated and purified by flash chromatography with DCM/MeOH (98/2 to 95/5) as eluent to give **(3.44)** (0.270 g, 66 %) as a beige solid.

**¹H NMR (400 MHz, DMSO-*d₆*) δ :** 1.37 (d, *J* = 6.9 Hz, 6H, 2×CH_{3 iPr}), 3.09 (t, *J* = 7.1 Hz, 2H, CH_{2-CH2-NH}), 3.42 (h, *J* = 7.0 Hz, 1H, H_{iPr}), 3.71 (d, *J* = 6.9 Hz, 2H, CH_{2-NH}), 6.18 (s, 1H, H_{Ar}), 7.23 (dd, *J* = 7.5, 4.9 Hz, 1H, H_{Ar}), 7.34 (d, *J* = 7.8 Hz, 1H, H_{Ar}), 7.70 (t, *J* = 7.7 Hz, 1H, H_{Ar}), 8.51 (d, *J* = 4.9 Hz, 1H, H_{Ar}), 8.56 (s, 1H, NH_{-CH2}).

**¹³C NMR (101 MHz, DMSO-*d₆*) δ :** 19.8 (2×CH_{3 iPr}), 24.4 (CH_{iPr}), 35.9 (CH_{2-CH2-NH}), 41.8 (CH_{2-NH}), 91.0 (CH_{Ar}), 121.7 (CH_{Ar}), 123.5 (CH_{Ar}), 136.5 (CH_{Ar}), 139.4 (C_{q}), 142.2 (C_{q}), 149.1 (CH_{Ar}), 149.6 (C_{q}), 153.9 (C_{q}), 158.6 (C_{q}).

**MS (ESI+)** : **m/z calcd C₁₅H₁₇ClN₆**: 317.13 [M+H]+, **found** : 317.2.

### Example 3.45: Synthesis of 6-chloro-3-isopropyl-N-[2-(3-pyridyl)ethyl]-[1,2,4]triazolo[4,3-b]pyridazin-8-amine (3.45)

To a solution of **(2.1)** (0.250 g, 1.08 mmol, 1.0 eq.), THF (10.0 mL), corresponding amine (0.181 g, 1.41 mmol, 1.3 eq.) and DIPEA (0.28 mL, 1.62 mmol, 1.5 eq.) was added then the mixture was refluxed 3 h. After cooling, Et₂O (30.0 mL) was added, the precipitate was filtered off, washed with water, triturated in Et₂O then dried under vacuum to give **(3.45)** (0.260 g, 76 %) as a white solid.
**Rf** (DCM/MeOH, 94/6) : 0.24

**¹H NMR (400 MHz, DMSO-*d₆*) δ :** 1.37 (d, *J* = 6.9 Hz, 6H, 2×CH_{3 iPr}), 2.96 (t, *J* = 7.1 Hz, 2H, CH_{2-CH2-NH}), 3.41 (hept, *J* = 7.0 Hz, 1H, H_{iPr}), 3.62 (d, *J* = 7.1 Hz, 2H, CH_{2-NH}), 6.26 (s, 1H, H_{Ar}), 7.31 (dd, *J* = 7.9, 4.8 Hz, 1H, H_{Ar}), 7.72 (d, *J* = 7.7 Hz, 1H, H_{Ar}), 8.41 (d, *J* = 4.8 Hz, 1H, H_{Ar}), 8.49 (d, *J* = 2.2 Hz, 1H, H_{Ar}), 8.55 (s, 1H, NH_{-CH2}).

**¹³C NMR (101 MHz, DMSO-*d₆*) δ :** 19.8 (2×CH_{3 iPr}), 24.4 (CH_{iPr}), 30.9 (CH_{2-CH2-NH}), 42.9 (CH_{2-NH}), 91.1 (CH_{Ar}), 123.3 (CH_{Ar}), 134.4 (C_{q}), 136.4 (CH_{Ar}), 139.4 (C_{q}), 142.2 (C_{q}), 147.5 (CH_{Ar}), 149.7 (C_{q}), 150.0 (CH_{Ar}), 153.9 (C_{q}).

**MS (ESI+) : m/z calcd for C₁₅H₁₇ClN₆ :** 317.13 [M+H]+, **found : 317.2.**

### Example 3.46: Synthesis of 6-chloro-3-isopropyl-N-[2-(4-pyridyl)ethyl]-[1,2,4]triazolo[4,3-b]pyridazin-8-amine (3.46)

To a solution of **(2.1)** (0.250 g, 1.08 mmol, 1.0 eq.), THF (10.0 mL), corresponding amine (0.181 g, 1.41 mmol, 1.3 eq.) and DIPEA (0.28 mL, 1.62 mmol, 1.5 eq.) was added then the mixture was refluxed 3 h. After cooling, Et₂O (30.0 mL) was added, the precipitate was filtered off, washed with water, triturated in Et₂O then dried under vacuum to give (3.46) (0.285 g, 82 %) as a white solid.
**Rf** (DCM/MeOH, 94/6) : 0.27

**¹H NMR (400 MHz, DMSO-*d₆*) δ :** 1.37 (d, *J* = 6.9 Hz, 6H, 2×CH_{3 iPr}), 2.97 (t, *J* = 7.1 Hz, 2H, CH_{2-CH2-NH}), 3.42 (p, *J* = 7.0 Hz, 1H, H_{iPr}), 3.56 - 3.75 (m, 2H, CH_{2-NH}), 6.29 (s, 1H, H_{Ar}), 7.33 (d, *J* = 5.1 Hz, 2H, 2xH_{Ar}), 8.44 - 8.51 (m, 2H, 2xH_{Ar}), 8.54 (s, 1H, NH_{-CH2}).

**¹³C NMR (101 MHz, DMSO-*d₆*) δ :** 19.8 (2×CH_{3 iPr}), 24.4 (CH_{iPr}), 32.9 (CH_{2-CH2-NH}), 42.2 (CH_{2-NH}), 91.2 (CH_{Ar}), 124.4 (2×CH_{Ar}), 139.3 (C_{q}), 142.2 (C_{q}), 147.9 (C_{q}), 149.4 (2×CH_{Ar}), 149.7 (C_{q}), 153.9 (C_{q}).

**MS (ESI+)** : **m/z calcd for C₁₅H₁₇ClN₆** : 317.13 [M+H]+, found : 317.2.

### Example 3.47: Synthesis of 6-chloro-3-cyclopropyl-N-[2-(2-pyridyl)ethyl]-[1,2,4]triazolo[4,3-b]pyridazin-8-amine (3.47)

To a solution of **(2.5)** (0.125 g, 0.51 mmol, 1.0 eq.) in THF (2.5 mL), corresponding amine (0.080 g, 0.62 mmol, 1.2 eq.) and Et₃N (0.15 mL, 1.04 mmol, 2.0 eq.) was added and the mixture was refluxed 1.5 h. After cooling, THF (10.0 mL) wad added, the precipitate was filtered off, washed with THF then the filtrate was concentrated. The residue was triturated in water, filtered off, washed with water then triturated in Et₂O, filtered, washed with Et₂O and dried under vacuum to give **(3.47)** (0.130 g, 80 %) as a beige solid.
Rf (DCM/MeOH, 96/4) : 0.14

**¹H NMR (400 MHz, DMSO-*d₆*) δ :** 1.03 - 1.18 (m, 4H, 2xCH_{2 cPr}), 2.31 (tt, *J* = 8.2, 5.2 Hz, 1H, H_{cPr}), 3.09 (t, *J* = 7.1 Hz, 2H, CH₂), 3.64 - 3.81 (m, 2H, CH₂), 6.16 (s, 1H, H_{Ar}), 7.23 (dd, *J* = 7.6, 4.8 Hz, 1H, H_{Ar}), 7.33 (d, *J* = 7.8 Hz, 1H, H_{Ar}), 7.70 (td, *J* = 7.7, 1.9 Hz, 1H, H_{Ar}), 8.46 - 8.61 (m, 2H, NH & H_{Ar}).

**¹³C NMR (101 MHz, DMSO-*d₆*) δ** : 4.8 (CH_{cPr}), 7.2 (2×CH_{2 cPr}), 35.9 (CH₂), 41.9 (CH₂), 91.0 (CH_{Ar}), 121.7 (CH_{Ar}), 123.5 (CH_{Ar}), 136.5 (CH_{Ar}), 139.3 (C_{q}), 142.2 (C_{q}), 149.1 (CH_{Ar}), 149.8 (C_{q}), 151.3 (C_{q}), 158.6 (C_{q}).

**MS (ESI+)** : **m/z calcd for C₁₅H₁₅ClN₆** : 315.1 [M+H]+, **found** : 315.1.

### Example 3.48: Synthesis of 6-chloro-3-cyclopropyl-N-[2-(3-pyridyl)ethyl]-[1,2,4]triazolo[4,3-b]pyridazin-8-amine (3.48)

To a solution of **(2.5)** (0.125 g, 0.51 mmol, 1.0 eq.) in THF (2.5 mL), corresponding amine (0.080 g, 0.62 mmol, 1.2 eq.) and Et₃N (0.15 mL, 1.04 mmol, 2.0 eq.) was added and the mixture was refluxed 1.5 h. After cooling, THF (10.0 mL) wad added, the precitpitate was filtered off, washed with THF then the filtrate was concentrated. The residue was triturated in water, filtered off, washed with water then triturated in Et₂O, filtered, washed with Et₂O and dried under vacuum to give **(3.48)** (0.127 g, 80 %) as a beige solid.
**Rf** (DCM/MeOH, 94/6) : 0.20

**¹H NMR (400 MHz, DMSO-*d₆*) δ :** 1.00 - 1.20 (m, 4H, 2xCH_{2 cPr}), 2.31 (tt, *J* = 8.1, 5.3 Hz, 1H, H_{cPr}), 2.96 (t, *J* = 7.1 Hz, 2H, CH₂), 3.49 - 3.76 (m, 2H, CH₂), 6.24 (s, 1H, H_{Ar}), 7.31 (dd, *J* = 7.8, 4.8 Hz, 1H, H_{Ar}), 7.71 (dt, *J* = 7.8, 2.0 Hz, 1H, H_{Ar}), 8.41 (dd, *J* = 4.8, 1.7 Hz, 1H, H_{Ar}), 8.49 (d, *J* = 2.3 Hz, 1H, H_{Ar}), 8.52 (s, 1H, NH).

**¹³C NMR (101 MHz, DMSO-*d₆*) δ :** 4.8 (CH), 7.1 (2×CH₂), 30.9 (CH₂), 42.9 (CH₂), 91.1 (CH_{Ar}), 123.3 (CH_{Ar}), 134.4 (C_{q}), 136.4 (CH_{Ar}), 139.3 (C_{q}), 142.2 (C_{q}), 147.5 (CH_{Ar}), 149.9 (C_{q}), 150.0 (CH_{Ar}), 151.3 (C_{q}).

**MS (ESI+)** : **m/z calcd for C₁₅H₁₅ClN₆** : 315.1 [M+H]+, **found** : 315.2.

### Example 3.49: Synthesis of 6-chloro-3-cyclopropyl-N-[2-(4-pyridyl)ethyl]-[1,2,4]triazolo[4,3-b]pyridazin-8-amine (3.49)

To a solution of **(2.5)** (0.125 g, 0.51 mmol, 1.0 eq.) in THF (2.5 mL), corresponding amine (0.080 g, 0.62 mmol, 1.2 eq.) and Et₃N (0.15 mL, 1.04 mmol, 2.0 eq.) was added and the mixture was refluxed 1.5 h. After cooling, THF (10.0 mL) wad added, the precitpitate was filtered off, washed with THF then the filtrate was concentrated. The residue was triturated in water, filtered off, washed with water then triturated in Et₂O, filtered, washed with Et₂O and dried under vacuum to give **(3.49)** (0.134 g, 82 %) as an orange solid.

**¹H NMR (400 MHz, DMSO-*d₆*) δ :** 1.05 - 1.17 (m, 4H, 2xCH_{2 cPr}), 2.31 (ddt, *J* = 10.9, 8.1, 5.2 Hz, 1H, H_{cPr}), 2.96 (t, *J* = 7.1 Hz, 2H, CH₂), 3.63 (d, *J* = 7.9 Hz, 2H, CH₂), 6.28 (s, 1H, H_{Ar}), 7.27 - 7.39 (m, 2H, 2xH_{Ar}), 8.42 - 8.48 (m, 2H, 2xH_{Ar}), 8.51 (s, 1H, NH).

**¹³C NMR (101 MHz, DMSO-*d₆*) δ** : 4.8 (CH_{cPr}), 7.2 (2×CH_{2 cPr}), 32.9 (CH₂), 42.3 (CH₂), 91.2 (CH_{Ar}), 124.4 (CH_{Ar}), 139.3 (C_{q}), 142.2 (C_{q}), 147.9 (C_{q}), 149.4 (CH_{Ar}), 149.9 (C_{q}), 151.3 (C_{q}).

**MS (ESI+)** : **m/z calcd for C₁₅H₁₅ClN₆** : 315.1 [M+H]+, **found** : 315.2.

### Example 3.50: Synthesis of 6-chloro-3-isopropyl-N-[3-(2-pyridyl)propyl]-[1,2,4]triazolo[4,3-b]pyridazin-8-amine (3.50)

To a solution of **(2.1)** (0.200 g, 0.87 mmol, 1.0 eq.) in THF (8.7 mL), corresponding amine (0.161 g, 1.13 mmol, 1.3 eq.) and Et₃N (0.24 mL, 1.73 mmol, 2.0 eq.) was added and the mixture was refluxed 3 h. After cooling, the solvent was removed, the residue was triturated in water (20.0 mL) and saturated NaHCO₃ (10.0 mL), the solid was filtered off, washed with water up to neutral pH then taken up in EtOAc. Organic filtrate was dried over MgSO₄, filtered and concentrated to give **(3.50)** (0.250 g, 87 %) as a pale yellow solid.
**Rf** (DCM/MeOH, 94/6) : 0.42

**¹H NMR (400 MHz, DMSO-*d₆*) δ :** 1.38 (d, *J* = 6.9 Hz, 6H, 2×CH_{3 iPr}), 2.03 (p, *J* = 7.3 Hz, 2H, CH₂), 2.83 (t, *J* = 7.5 Hz, 2H, CH₂), 3.41 (td, *J* = 13.7, 6.8 Hz, 3H, H_{iPr} & CH₂), 6.20 (s, 1H, H_{Ar}), 7.20 (dd, *J* = 7.6, 4.9 Hz, 1H, H_{Ar}), 7.28 (d, *J* = 7.8 Hz, 1H, H_{Ar}), 7.69 (td, *J* = 7.6, 1.9 Hz, 1H, H_{Ar}), 8.45 - 8.54 (m, 1H, H_{Ar}), 8.70 (s, 1H, NH).

**¹³C NMR (101 MHz, DMSO-*d₆*) δ :** 19.8 (2×CH_{3 iPr}), 24.4 (CH_{iPr}), 27.2 (CH₂), 34.5 (CH₂), 41.8 (CH₂), 90.8 (CH_{Ar}), 121.3 (CH_{Ar}), 122.9 (CH_{Ar}), 136.5 (CH_{Ar}), 139.5 (C_{q}), 142.4 (C_{q}), 148.9 (CH_{Ar}), 153.9 (C_{q}), 160.8 (C_{q}).

**MS (ESI+)** : **m/z calcd for C₁₆H₁₉ClN₆** : 331.1 [M+H]+, **found** : 331.2.

### Example 3.51: Synthesis of 6-chloro-3-isopropyl-N-[3-(3-pyridyl)propyl]-[1,2,4]triazolo[4,3-b]pyridazin-8-amine (3.51)

To a solution of **(2.1)** (0.200 g, 0.87 mmol, 1.0 eq.) in THF (8.7 mL), corresponding amine dihydrochloride (0.248 g, 1.13 mmol, 1.3 eq.) and Et₃N (0.73 mL, 5.19 mmol, 6.0 eq.) was added and the mixture was refluxed 3 h. Water (0.43 mL) was added and the mixture was refluxed 3 h to complete the reaction. After cooling, EtOAc (15.0 mL) was added, the mixture was dried over MgSO₄, filtered and concentrated. The residue was triturated in water (20.0 mL), the solid was filtered off, taken up in EtOAc then organic filtrate was dried over MgSO₄, filtered and concentrated to give **(3.51)** (0.240 g, 84 %) as a beige solid.
**Rf** (DCM/MeOH, 94/6) : 0.35

**¹H NMR (400 MHz, DMSO-*d₆*) δ :** 1.39 (d, *J* = 7.0 Hz, 6H, 2×CH_{3 iPr}), 1.89 - 1.98 (m, 2H, CH_{2-CH2-NH}), 2.66 - 2.75 (m, 2H, CH_{2-CH2-CH2-NH}), 3.36 (s, 2H, CH_{2-NH}), 3.43 (p, *J* = 6.9 Hz, 1H, H_{iPr}), 6.18 (s, 1H, H_{Ar}), 7.31 (dd, *J* = 7.8, 4.8 Hz, 1H, H_{Ar}), 7.68 (dt, *J* = 7.9, 2.0 Hz, 1H, H_{Ar}), 8.40 (dd, *J* = 4.8, 1.7 Hz, 1H, H_{Ar}), 8.47 (d, *J* = 2.3 Hz, 1H, H_{Ar}), 8.61 (s, 1H, NH_{-CH2}).

**¹³C NMR (101 MHz, DMSO-*d₆*) δ :** N.D.

**MS (ESI+)** : **m/z calcd for C₁₆H₁₉ClN₆** : 331.1 [M+H]+, **found** : 331.2.

### Example 3.52: Synthesis of 6-chloro-3-isopropyl-N-[3-(4-pyridyl)propyl]-[1,2,4]triazolo[4,3-b]pyridazin-8-amine (3.52)

To a solution of **(2.1)** (0.200 g, 0.87 mmol, 1.0 eq.) in THF (8.7 mL), corresponding amine (0.161 g, 1.13 mmol, 1.3 eq.) and Et₃N (0.24 mL, 1.73 mmol, 2.0 eq.) was added and the mixture was refluxed 3 h. After cooling, the solvent was removed, the residue was triturated in water (20.0 mL) and saturated NaHCO₃ (10.0 mL), the solid was filtered off, washed with water up to neutral pH then taken up in EtOAc. Organic filtrate was dried over MgSO₄, filtered and concentrated to give **(3.52)** (0.260 g, 91 %) as an orange solid.
**Rf** (DCM/MeOH, 94/6) : 0.33

**¹H NMR (400 MHz, DMSO-*d₆*) δ :** 1.38 (d, *J* = 6.9 Hz, 6H, 2×CH_{3 iPr}), 1.94 (p, *J* = 7.2 Hz, 2H, CH2), 2.65 - 2.72 (m, 2H, CH2), 3.33 - 3.38 (m, 2H, CH2), 3.42 (p, *J* = 7.0 Hz, 1H, H_{iPr}), 6.18 (s, 1H, H_{Ar}), 7.19 - 7.37 (m, 2H, 2xH_{Ar}), 8.31 - 8.48 (m, 2H, 2xH_{Ar}), 8.60 (s, 1H, NH).

**¹³C NMR (101 MHz, DMSO-*d₆*) δ :** N.D.

**MS (ESI+)** : **m/z calcd for C₁₆H₁₉ClN₆** : 331.1 [M+H]+, **found** : 331.2.

### Example 3.53: Synthesis of 6-chloro-3-cyclopropyl-N-[3-(2-pyridyl)propyl]-[1,2,4]triazolo[4,3-b]pyridazin-8-amine (3.53)

To a solution of **(2.5)** (0.170 g, 0.74 mmol, 1.0 eq.) in THF/H₂O (15.0/0.5 mL), corresponding amine dihydrochloride (0.229 g, 1.04 mmol, 1.3 eq.) and Et₃N (0.63 mL, 4.45 mmol, 6.0 eq.) was added and the mixture was refluxed 16 h. After cooling, THF (10.0 mL) was added, the precipitate was filtered off, washed with THF then the filtrate was concentrated and purified by flash chromatography with DCM/MeOH (98/2 to 95/5) as eluent to give **(3.53)** (0.080 g, 33 %) as a beige solid.

**¹H NMR (400 MHz, DMSO-*d₆*) δ :** 1.11 (tt, *J* = 8.0, 2.7 Hz, 4H, 2×CH_{2 cPr}), 1.85 - 2.02 (m, 2H, CH₂), 2.32 (tt, *J* = 8.2, 5.2 Hz, 1H, H_{cPr}), 2.64 - 2.76 (m, 2H, CH₂), 3.32 - 3.42 (m, 2H, CH₂), 6.16 (s, 1H, H_{Ar}), 7.30 (dd, *J* = 7.8, 4.7 Hz, 1H, H_{Ar}), 7.66 (dt, *J* = 7.9, 2.0 Hz, 1H, H_{Ar}), 8.39 (dd, *J* = 4.8, 1.6 Hz, 1H, H_{Ar}), 8.46 (d, *J* = 2.3 Hz, 1H, H_{Ar}), 8.57 (t, *J* = 5.6 Hz, 1H, NH).

**¹³C NMR (101 MHz, DMSO-*d₆*) δ** : 4.8 (CH_{cPr}), 7.1 (2×CH_{2 cPr}), 29.1 (CH₂), 29.4 (CH₂), 41.7 (CH₂), 90.8 (CH_{Ar}), 123.4 (CH_{Ar}), 135.8 (CH_{Ar}), 136.9 (C_{q}), 139.4 (C_{q}), 142.3 (C_{q}), 147.2 (CH_{Ar}), 149.6 (CH_{Ar}), 149.8 (C_{q}), 151.3 (C_{q}).

**MS (ESI+)** : **m/z calcd for C₁₆H₁₇ClN₆** : 329.1 [M+H]+, **found** : 329.2.

### Example 3.54: Synthesis of 6-chloro-3-isopropyl-N-(pyrimidin-2-ylmethyl)-[1,2,4]triazolo[4,3-b]pyridazin-8-amine (3.54)

To a solution of **(2.1)** (0.190 g, 0.82 mmol, 1.0 eq.) in THF (9.0 mL), corresponding amine hydrochloride (0.164 g, 1.07 mmol, 1.3 eq.) and DIPEA (0.36 mL, 2.06 mmol, 2.5 eq.) was added then the mixture was refluxed 3 h. After cooling, the solvent was removed, the residue was triturated in saturated NaHCO₃ (10.0 mL), the solid was filtered off, washed with water then EtOAc to give a first part of **(3.54)** (0.072 g). The biphasique filtrate was separated, aqueous layer was extracted twice with EtOAc (2x10.0 mL). Organic layers were combined, dried over MgSO₄, filtered, concentrated and purified by flash chromatography with DCM/MeOH (98/2 to 94/6) as eluent to give the second part of **(3.54)** (0.020 g). Total of **(3.54):** 0.092 g, 37 % yield, as a white solid.
**Rf** (DCM/MeOH, 94/6) : 0.40

**¹H NMR (400 MHz, DMSO-*d₆*) δ :** 1.39 (d, *J* = 6.9 Hz, 6H, 2×CH_{3 iPr}), 3.44 (p, *J* = 7.0 Hz, 1H, H_{iPr}), 4.80 (s, 2H, CH_{2 Bn}), 6.22 (s, 1H, H_{Ar}), 7.45 (t, *J* = 4.9 Hz, 1H, NH_{Bn}), 8.74 - 8.89 (m, 3H, 3×H_{Ar}).

**¹³C NMR (101 MHz, DMSO-*d₆*) δ :** N.D.

**MS (ESI+)** : **m/z calcd for C₁₃H₁₄ClN₇** : 304.1 [M+H]+, **found** : 304.2.

### Example 3.55: Synthesis of 6-chloro-3-isopropyl-N-(pyrazin-2-ylmethyl)-[1,2,4]triazolo[4,3-b]pyridazin-8-amine (3.55)

To a solution of **(2.1)** (0.290 g, 1.26 mmol, 1.0 eq.) in THF (10.0 mL), corresponding amine (0.187 g, 1.63 mmol, 1.3 eq.) and DIPEA (0.33 mL, 1.88 mmol, 1.5 eq.) was added then the mixture was refluxed 1 h. After cooling, the precipitate was filtered off and washed with THF. The solid was washed with water then Et₂O and dried under vacuum to give a first part of **(3.55)** (0.103 g). THF filtrate was concentrated and purified by flash chromatography with DCM/MeOH (98/2 to 95/5) as eluent to give the second part of **(3.55)** (0.015 g). Total of **(3.55):** 0.118 g, 31 % yield, as a white solid.
**Rf** (EtOAc/Cyclohexane, 7/3) : 0.37

**¹H NMR (400 MHz, DMSO-*d₆*) δ :** 1.38 (d, *J* = 6.9 Hz, 6H, 2×CH_{3 iPr}), 3.43 (p, *J* = 7.0 Hz, 1H, H_{iPr}), 4.77 (s, 2H, CH_{2 Bn}), 6.29 (s, 1H, H_{Ar}), 8.57 (d, *J* = 2.6 Hz, 1H, H_{Ar}), 8.60 - 8.63 (m, 1H, H_{Ar}), 8.69 (d, *J* = 1.5 Hz, 1H, H_{Ar}), 8.98 (s, 1H, NH_{Bn}).

**¹³C NMR (101 MHz, DMSO-*d₆*) δ :** N.D.

**MS (ESI+)** : **m/z calcd for C₁₃H₁₄ClN₇** : 304.1 [M+H]+, **found** : 304.2.

### Example 3.56: Synthesis of 6-chloro-3-isopropyl-N-(pyrimidin-5-ylmethyl)-[1,2,4]triazolo[4,3-b]pyridazin-8-amine (3.56)

To a solution of **(2.1)** (0.190 g, 0.82 mmol, 1.0 eq.) in THF (9.0 mL), corresponding amine hydrochloride (0.164 g, 1.07 mmol, 1.3 eq.) and DIPEA (0.36 mL, 2.06 mmol, 2.5 eq.) was added then the mixture was refluxed 3 h. After cooling, the solvent was removed, the residue was triturated in saturated NaHCO₃ (10.0 mL), the solid was filtered off, washed with water then EtOAc to give a first part of **(3.56)** (0.055 g). The biphasique filtrate was separated, aqueous layer was extracted twice with EtOAc (2x10.0 mL). Orgnics layers were combined, dired over MgSO₄, filtered, concentrated and purified by flash chromatography with DCM/MeOH (98/2 to 94/6) as eluent to give the second part of **(3.56)** (0.055 g), total of **(3.56):** 0.110 g, 44 % yield, as a white solid.
**Rf** (DCM/MeOH, 94/6) : 0.30

**¹H NMR (400 MHz, DMSO-*d₆*) δ :** 1.37 (d, *J* = 6.9 Hz, 6H, 2×CH_{3 iPr}), 3.42 (hept, *J* = 6.6 Hz, 1H, H_{iPr}), 4.66 (s, 2H, CH_{2 Bn}), 6.37 (s, 1H, H_{Ar}), 8.85 (s, 2H, 2×H_{Ar}), 9.05 (s, 1H, NH_{Bn}), 9.10 (s, 1H, H_{Ar}).

**¹³C NMR (101 MHz, DMSO-*d₆*) δ :** N.D.

**MS (ESI+)** : **m/z calcd for C₁₃H₁₄ClN₇** : 304.1 [M+H]+, **found** : 304.2.

### Example 3.57: Synthesis of 6-chloro-3-isopropyl-N-(pyridazin-3-ylmethyl)-[1,2,4]triazolo[4,3-b]pyridazin-8-amine (3.57)

To a solution of **(2.1)** (0.300 g, 1.30 mmol, 1.0 eq.) in THF (9.0 mL), corresponding amine dihydrochloride (0.323 g, 1.69 mmol, 1.3 eq.) and DIPEA (0.75 mL, 4.28 mmol, 3.3 eq.) was added and the mixture was stirred 16 h at room temperature then refluxed 2 h to complete the reaction. After cooling, the solvent was removed, the crude was triturated in water and the resulting solid was filtered off, washed with water up to neutral pH, triturated in Et₂O, filtered and dried to give **(3.57)** (0.286 g, 73 %) as a white solid.
**Rf** (DCM/MeOH, 94/6) : 0.38

**¹H NMR (400 MHz, DMSO-*d₆*) δ :** 1.38 (d, *J* = 6.9 Hz, 6H, 2×CH_{3 iPr}), 3.43 (p, *J* = 6.9 Hz, 1H, H_{iPr}), 4.89 (s, 2H, CH_{2 Bn}), 6.27 (s, 1H, H_{Ar}), 7.69 (d, *J* = 3.3 Hz, 2H, 2xH_{Ar}), 9.08 (s, 1H, NH_{Bn}), 9.17 (t, *J* = 3.4 Hz, 1H, H_{Ar}).

**¹³C NMR (101 MHz, DMSO-*d₆*) δ :** 19.8 (CH_{3 iPr}), 24.4 (CH_{iPr}), 45.7 (CH_{2 Bn}), 92.0 (CH_{Ar}), 125.7 (CH_{Ar}), 127.5 (CH_{Ar}), 139.4 (C_{q}), 142.5 (C_{q}), 149.4 (C_{q}), 151.0 (CH_{Ar}), 154.0 (C_{q}), 159.3 (C_{q}).

**MS (ESI+)** : **m/z calcd for C₁₃H₁₄ClN₇** : 304.1 [M+H]+, **found** : 304.1.

### Example 3.58: Synthesis of 6-chloro-3-isopropyl-N-[[4-(2-pyridyl)phenyl]methyl]-[1,2,4]triazolo[4,3-b]pyridazin-8-amine (3.58)

A 10 - 20 mL vial with a stir bar was charged **(1.14)** (0.300 g, 0.91 mmol, 1.0 eq.), dioxane (4.9 mL), isobutyric acid hydrazide (0.112 g, 1.09 mmol, 1.2 eq.) and AcOH (0.06 mL, 1.00 mmol, 1.1 eq.). The vial was sealed and then put on heating block, 22 h at 110 °C. After cooling, reaction mixture was poured onto saturated NaHCO₃ (20.0 mL) and vigorously stirred 5 min. The resulting precipitate was filtered off, washed with water then solubilized in DCM (10.0 mL). Aqueous filtrate was extracted twice with EtOAc (2 x 10.0 mL) and combined with DCM solution, all was dried over MgSO₄, filtered, concentrated and purified by flash chromatography with DCM/MeOH (98/2 to 95/5) as eluent to give the title compound **(3.58)** (0.140 g, 41 %) as a white solid.
**Rf** (DCM/MeOH, 94/6) : 0.47

**¹H NMR (400 MHz, DMSO-*d₆*) δ :** 1.38 (d, *J* = 6.9 Hz, 6H, 2×CH_{3 iPr}), 3.42 (hept, *J* = 7.1 Hz, 1H, H_{iPr}), 4.65 (d, *J* = 3.9 Hz, 2H, CH_{2 Bn}), 6.18 (s, 1H, H_{Ar}), 7.33 (dd, *J* = 7.4, 4.9 Hz, 1H, H_{Ar}), 7.51 (d, *J* = 8.1 Hz, 2H, 2xH_{Ar}), 7.86 (td, *J* = 7.7, 1.9 Hz, 1H, H_{Ar}), 7.93 (d, *J* = 8.0 Hz, 1H, H_{Ar}), 8.06 (d, *J* = 8.1 Hz, 2H, 2xH_{Ar}), 8.65 (d, *J* = 3.8 Hz, 1H, H_{Ar}), 9.17 (s, 1H, NH_{Bn}).

**¹³C NMR (101 MHz, DMSO-*d₆*) δ** : 19.8 (2×CH_{3 iPr}), 24.4 (CH_{iPr}), 44.9 (CH_{2 Bn}), 91.6 (CH_{Ar}), 120.1 (CH_{Ar}), 122.5 (CH_{Ar}), 126.7 (2×CH_{Ar}), 127.5 (2×CH_{Ar}), 137.2 (CH_{Ar}), 137.7 (C_{q}), 138.3 (C_{q}), 139.4 (C_{q}), 142.3 (C_{q}), 149.4 (C_{q}), 149.5 (CH_{Ar}), 154.0 (C_{q}), 155.7 (C_{q}).

**MS (ESI+)** : **m/z calcd for C₂₀H₁₉ClN₆** : 379.1 [M+H]+, **found** : 379.3.

### Example 3.59: Synthesis of 6-chloro-3-isopropyl-N-[[4-(4-pyridyl)phenyl]methyl]-[1,2,4]triazolo[4,3-b]pyridazin-8-amine (3.59)

To a solution of 4-(4-pyridyl)benzonitrile (0.224 g, 1.24 mmol, 1.15 eq.) in dry THF (12.0 mL), LiAlH₄ (0.066 g, 1.73 mmol, 1.6 eq.) was added portionwise then the mixture was stirred 45 min. After completion, saturated NH₄Cl (10.0 mL), water (5.0 mL) and EtOAc (10.0 mL) was added and the mixture was vigorously stirred 5 min. Layers were separated, aqueous layer was extracted with EtOAc (2x10.0 mL) then organic layers were combined, dried over MgSO₄, filtered and concentrated. The residu was directly used in THF (18.0 mL) with **(2.1)** (0.250 g, 1.08 mmol, 1.0 eq.) and Et₃N (0.46 mL, 3.25 mmol, 3.0 eq.) and the mixture was refluxed 3 h. After cooling, the solid was filtered, washed with THF then the filtrate was concentrated and directly purified by flash chromatography with DCM/MeOH (98/2 to 93/7) as eluent to give **(3.59)** (0.110 g, 27 %) as a white solid.
**Rf** (DCM/MeOH, 94/6) : 0.25

**¹H NMR (400 MHz, DMSO-*d₆*) δ :** 1.38 (d, *J* = 6.9 Hz, 6H, 2×CH_{3 iPr}), 3.42 (p, *J* = 6.9 Hz, 1H, H_{iPr}), 4.66 (d, *J* = 6.2 Hz, 2H, CH_{2 Bn}), 6.19 (s, 1H, H_{Ar}), 7.54 (d, *J* = 8.1 Hz, 2H, 2xH_{Ar}), 7.66 - 7.75 (m, 2H, 2xH_{Ar}), 7.76 - 7.87 (m, 2H, 2xH_{Ar}), 8.56 - 8.70 (m, 2H, 2xH_{Ar}), 9.18 (s, 1H, NH).

**¹³C NMR (101 MHz, DMSO-*d₆*) δ :** 19.8 (2×CH_{3 iPr}), 24.4 (CH_{iPr}), 44.7 (CH₂), 91.6 (CH_{Ar}), 121.1 (2×CH_{Ar}), 127.0 (2×CH_{Ar}), 128.0 (2×CH_{Ar}), 136.0 (C_{q}), 138.8 (C_{q}), 139.4 (C_{q}), 142.3 (C_{q}), 146.6 (C_{q}), 149.4 (C_{q}), 150.2 (2×CH_{Ar}), 154.0 (C_{q}).

**MS (ESI+)** : **m/z calcd for C₂₀H₁₉ClN₆** : 379.1 [M+H]+, **found** : 379.2.

### Example 3.60: Synthesis of 6-chloro-3-isopropyl-N-(pyridazin-3-yl)-[1,2,4]triazolo[4,3-b]pyridazin-8-amine (3.60)

To a solution of **(2.1)** (0.100 g, 0.433 mmol, 1.0 eq.) in dry THF (3.33 mL), 3-aminopyridazine (0.084 g, 0.866 mmol, 2.0 eq.) then tBuOK 1.65M in THF (0.52 mL, 0.866 mmol, 2.0 eq.) were added and the mixture was stired 0.8h at room temperature. After completion, saturated NH₄Cl (3.33 mL) and water (1 mL) were added then biphasic mixture was vigorously stired 5 min. The precipitate formed was filtered off, washed with H₂O and Et₂O to give **(3.60)** (0.023 g, 18%) as a white solid. Layers of filtrate were separated, aqueous layer was extracted with EtOAc (2x10.0 mL) then organic layers were combined, dried over MgSO₄, filtered and concentrated. The residue was triturated in Et₂O, filtered off, washed with Et₂O and dried under vacuum to give **(3.60)** as a beige solid (0.063 g, 51%). **(3.60)** was obtained with a yield of 69% (0.086 g).
**Rf** (DCM/MeOH, 96/4) : 0.28

**¹H NMR (400 MHz, DMSO-*d₆*) δ :** 1.43 (d, *J* = 6.9 Hz, 6H, 2×CH_{3 iPr}), 3.53 (h, *J* = 6.9 Hz, 1H, H_{iPr}), 7.73 (dd, *J* = 9.0, 4.6 Hz, 1H, H_{Ar}), 7.89 (dd, *J* = 9.0, 1.4 Hz, 1H, H_{Ar}), 8.39 (s, 1H, H_{Ar}), 9.00 (dd, *J* = 4.6, 1.4 Hz, 1H, H_{Ar}), 11.13 (s, 1H, NH).

**¹³C NMR (101 MHz, DMSO-*d₆*) δ :** 19.83 (2×CH_{3 iPr}), 24.50 (CH_{iPr}), 101.29 (CH_{Ar}), 119.50 (CH_{Ar}), 128.69 (CH_{Ar}), 136.05 (C_{q}), 139.24 (C_{q}), 147.89 (CH_{Ar}), 149.82 (C_{q}), 154.39 (C_{q}), 157.23 (C_{q}).

**MS (ESI+)** : **m/z calcd for C₁₂H₁₂ClN₇** : 290.1 [M+H]+, **found** : 290.2.

### Example 3.61: Synthesis of 6-chloro-3-isopropyl-N-(pyridazin-4-yl)-[1,2,4]triazolo[4,3-b]pyridazin-8-amine (3.61)

To a solution of **(2.1)** (0.350 g, 1.515 mmol, 1.0 eq.) in dry THF (11.5 mL), 4-aminopyridazine (0.294 g, 3.029 mmol, 2.0 eq.) then tBuOK 1M in THF (1.84 mL, 3.029 mmol, 2.0 eq.) were added and the mixture was stired 1.25h at room temperature. After completion, saturated NH₄Cl (11.5 mL) and water (5.23 mL) were added then biphasic mixture was vigorously stired 5 min. Layers of mixture were separated, aqueous layer was extracted with EtOAc (2x10.0 mL) then organic layers were combined, dried over MgSO₄, filtered and concentrated. The residue was purified by flash chromatography with DCM/MeOH (99/1 to 94/6) as eluent to give **(3.61)** (0.256 g, 58%) as a yellow solid.
**Rf** (DCM/MeOH, 94/6) : 0.28

**¹H NMR (400 MHz, DMSO-*d₆*) δ :** 1.42 (d, *J* = 6.9 Hz, 6H, 2×CH_{3 iPr}), 3.50 (hept, *J* = 6.9 Hz, 1H, H_{iPr}), 7.06 (s, 1H, H_{Ar}), 7.77 (dd, *J* = 6.2, 2.9 Hz, 1H, H_{Ar}), 9.09 (d, *J* = 5.9 Hz, 1H, H_{Ar}), 9.38 (d, *J* = 2.7 Hz, 1H, H_{Ar}), 10.88 (s, 1H, NH).

**¹³C NMR (101 MHz, DMSO-*d₆*) δ :** 19.80 (2×CH_{3 iPr}), 24.50 (CH_{iPr}), 98.77 (CH_{Ar}), 115.13 (CH_{Ar}), 137.58 (C_{q}), 138.40 (C_{q}), 139.48 (C_{q}), 145.30 (CH_{Ar}), 149.54 (C_{q}), 151.27 (CH_{Ar}), 154.37 (C_{q}).

**MS (ESI+)** : **m/z calcd for C₁₂H₁₂ClN₇** : 290.1 [M+H]+, **found** : 290.1.

### Example 3.62: Synthesis of 6-chloro-3-isopropyl-N-(pyrimidin-4-yl)-[1,2,4]triazolo[4,3-b]pyridazin-8-amine (3.62)

To a solution of **(2.1)** (0.40 g, 1.731 mmol, 1.0 eq.) in dry THF (13.14 mL), 4-aminopyrimidine (0.281 g, 2.891 mmol, 2.0 eq.) then tBuOK 1.65M in THF (1.75 mL, 2.891 mmol, 2.0 eq.) were added and the mixture was stired 1 h at room temperature. After completion, saturated NH₄Cl (13.14 mL) and water (5.97 mL) were added then biphasic mixture was vigorously stired 5 min. Layers of mixture were separated, aqueous layer was extracted with EtOAc (2x10.0 mL) then organic layers were combined, dried over MgSO₄, filtered and concentrated. The residue was triturated in DCM, filtered off, washed with MeOH and Et₂O and dried under vacuum to give **(3.62)** as a pale yellow/beige solid (0.171 g, 34%). The filtrate was purified by flash chromatography with DCM/MeOH (99/1 to 94/6) as eluent to give the second part of **(3.62)** (0.150 g, 30%) as a beige solid. Total of **(3.62):** 0.321 g, 64%.
**Rf** (DCM/MeOH, 94/6) : 0.35

**¹H NMR (400 MHz, DMSO-*d₆*) δ :** 1.42 (d, *J* = 6.9 Hz, 6H, 2×CH_{3 iPr}), 3.50 (hept, *J* = 7.0 Hz, 1H, H_{iPr}), 7.60 (dd, *J* = 5.8, 1.3 Hz, 1H, H_{Ar}), 8.37 (s, 1H, H_{Ar}), 8.62 (d, *J* = 5.8 Hz, 1H, H_{Ar}), 8.99 (d, *J* = 1.2 Hz, 1H, H_{Ar}), 11.28 (s, 1H, NH).

**¹³C NMR (101 MHz, DMSO-*d₆*) δ :** 19.81 (2×CH_{3 iPr}), 24.48 (CH_{iPr}), 102.67 (CH_{Ar}), 110.88 (CH_{Ar}), 135.84 (C_{q}), 139.11 (C_{q}), 149.48 (C_{q}), 154.39 (C_{q}), 157.07 (CH_{Ar}), 157.72 (CH_{Ar}), 159.68 (C_{q}).

**MS (ESI+)** : **m/z calcd for C₁₂H₁₀ClN₇** : 290.1 [M+H]+, **found** : 290.1.

### Example 3.63: Synthesis of 6-chloro-3-isopropyl-N-(pyrimidin-5-yl)-[1,2,4]triazolo[4,3-b]pyridazin-8-amine (3.63)

To a solution of **(2.1)** (0.200 g, 0.866 mmol, 1.0 eq.) in dry THF (6.63 mL), 5-aminopyrimidine (0.166 g, 1.731 mmol, 2.0 eq.) then tBuOK 1.65M in THF (1.05 mL, 1.731 mmol, 2.0 eq.) were added and the mixture was stired 1.5h at room temperature. After completion, saturated NH₄Cl (6.63 mL) and water (1.77 mL) were added then biphasic mixture was vigorously stired 5 min. The precipitate formed was filtered off, washed with H₂O and Et₂O to give **(3.63)** (0.150 g, 60%) as a white solid. Layers of filtrate were separated, aqueous layer was extracted with EtOAc (2x10.0 mL) then organic layers were combined, dried over MgSO₄, filtered and concentrated. The residue was purified by flash chromatography with DCM/MeOH (99/1 to 94/6) as eluent to give the second part of **(3.63)** (0.048 g, 19%) as a white solid. Total of **(3.63)** was obtained with a yield of 79% (0.198 g).
**Rf** (DCM/MeOH, 94/6) : 0.25

**¹H NMR (400 MHz, DMSO-*d₆*) δ :** 1.42 (d, *J* = 7.0 Hz, 6H, 2×CH_{3 iPr}), 3.49 (p, *J* = 6.9 Hz, 1H, H_{iPr}), 6.65 (s, 1H, H_{Ar}), 8.95 (s, 2H, 2xH_{Ar}), 9.06 (s, 1H, H_{Ar}), 10.57 (s, 1H, NH).

**¹³C NMR (101 MHz, DMSO) δ** : 19.81 (2×CH_{3 iPr}), 24.49 (CH_{iPr}), 95.16 (CH_{Ar}), 133.98 (C_{q}), 139.40 (C_{q}), 139.60 (C_{q}), 149.66 (C_{q}), 151.64(2×CH_{Ar}), 154.23 (C_{q}), 154.71 (CH_{Ar}).

**MS (ESI+)** : **m/z calcd** for **C₁₂H₁₀ClN₇** : 290.1 [M+H]+, **found** : 290.2.

### Example 3.64: Synthesis of 6-chloro-3-isopropyl-N-(pyrimidin-2-yl)-[1,2,4]triazolo[4,3-b]pyridazin-8-amine (3.64)

To a solution of **(2.1)** (0.152 g, 0.664 mmol, 1.0 eq.) in dry THF (5.04 mL), 2-aminopyrimidine (0.126 g, 1.316 mmol, 2.0 eq.) then tBuOK 1.65M in THF (0.80 mL, 1.316 mmol, 2.0 eq.) were added and the mixture was stired 2.25h at room temperature. After completion, saturated NH₄Cl (5.04 mL) and water (2.3 mL) were added then biphasic mixture was vigorously stired 5 min. Layers were separated, aqueous layer was extracted with EtOAc (2x10.0 mL) then organic layers were combined, dried over MgSO₄, filtered and concentrated. The residue was triturated in Et₂O, filtered off, washed with Et₂O and dried under vacuum to give **(3.64)** (0.124 g, 65%) as a beige solid.
Rf (DCM/MeOH, 94/6) : 0.48

**¹H NMR (400 MHz, DMSO-*d₆*) δ :** 1.42 (d, *J* = 6.9, 2.0 Hz, 6H, 2×CH_{3 iPr}), 3.45 - 3.56 (m, 1H, H_{iPr}), 7.26 (dt, *J* = 4.9, 3.1 Hz, 1H, H_{Ar}), 8.16 (s, 1H, H_{Ar}), 8.80 (dd, *J* = 4.9, 1.9 Hz, 2H, 2xH_{Ar}), 10.31 (s, 1H, NH).

**¹³C NMR (101 MHz, DMSO-d6) δ :** 19.81 (2×CH_{3 iPr}), 24.49 (CH_{iPr}), 101.32 (CH_{Ar}), 116.40 (CH_{Ar}), 136.11 (C_{q}), 139.01 (C_{q}), 149.61 (C_{q}), 154.38 (C_{q}), 158.56 (C_{q}), 158.64 (2×CH_{Ar}).

**MS (ESI+)** : **m/z calcd for C₁₂H₁₂ClN₇** : 290.1 [M+H]+, **found** : 290.2.

### Example 3.65: Synthesis of 6-chloro-3-isopropyl-N-(pyrazin-2-yl)-[1,2,4]triazolo[4,3-b]pyridazin-8-amine (3.65)

To a solution of **(2.1)** (0.100 g, 0.433 mmol, 1.0 eq.) in dry THF (3.33 mL), 2-aminopyrazine (0.082 g, 0.866 mmol, 2.0 eq.) then tBuOK 1.65M in THF (0.52 mL, 0.866 mmol, 2.0 eq.) were added and the mixture was stired 0.8h at room temperature. After completion, saturated NH₄Cl (3.33 mL) and water (1 mL) were added then biphasic mixture was vigorously stired 5 min. Layers were separated, aqueous layer was extracted with EtOAc (2x10.0 mL) then organic layers were combined, dried over MgSO₄, filtered and concentrated. The residue was triturated in Et₂O, filtered off, washed with Et₂O and dried under vacuum to give **(3.65)** (0.093 g, 74%) as a beige solid.
**Rf** (DCM/MeOH, 96/4) : 0.31

**¹H NMR (400 MHz, DMSO-*d*₆) δ** : 1.43 (d, *J* = 7.0 Hz, 6H, 2xCH_{3 iPr}), 3.51 (hept, *J* = 6.8 Hz, 1H, H_{iPr}), 8.19 (s, 1H, H_{Ar}), 8.33 (d, *J* = 2.7 Hz, 1H, H_{Ar}), 8.47 (dd, *J* = 2.7, 1.4 Hz, 1H, H_{Ar}), 8.93 (d, *J* = 1.5 Hz, 1H, H_{Ar}), 11.32 (s, 1H, NH).

**¹³C NMR (101 MHz, DMSO-*d₆*) δ** : 19.83 (2×CH_{3 iPr}), 24.48 (CH_{iPr}), 100.32 (CH_{Ar}), 136.30 (C_{q}), 137.69 (CH_{Ar}), 138.02 (CH_{Ar}), 139.21 (C_{q}), 141.16 (CH_{Ar}), 149.60 (C_{q}), 150.64 (C_{q}), 154.37 (C_{q}).

**MS (ESI+) : m/z calcd for C₁₂H₁₂ClN₇ :** 290.1 [M+H]+, **found** : 290.2

### Example 3.66: Synthesis of 6-chloro-3-isopropyl-N-(6-methoxypyridin-2-yl)-[1,2,4]triazolo[4,3-b]pyridazin-8-amine (3.66)

Under inert gas, to a solution of **(2.1)** (0.125 g, 0.54 mmol, 1.0 eq.) and corresponding aminopyridine derivative (0.102 g, 0.81 mmol, 1.5 eq.) in dry THF (7.5 mL), tBuOK 1M in THF (0.81 mL, 0.81 mmol, 1.5 eq.) was added dropwise. The mixture was stired 3.5 h at room temperature then saturated NH₄Cl (3.0 mL), water (1.0 mL) and EtOAc (3.0 mL) were added. The mixture was vigorously stired 5 min then layers were separated and aqueous layer was extracted twice with EtOAc (2x5.0 mL). Organic layers were combined, dried over MgSO₄, filtered, concentrated and purified by flash chromatography with DCM/MeOH (99/1 to 98/2) as eluent to give **(3.66)** (0.128 g, 74 %) as a light orange solid.
**Rf** (DCM/MeOH, 98/2) : 0.30

**¹H NMR (400 MHz, DMSO-*d*₆) δ** : 1.42 (d, *J* = 6.9 Hz, 6H, 2xCH_{3 iPr}), 3.50 (hept, *J* = 6.9 Hz, 1H, H_{iPr}), 3.95 (s, 3H, CH_{3-O}), 6.55 (d, *J* = 8.0 Hz, 1H, H_{Ar}), 7.18 (d, *J* = 7.8 Hz, 1H, H_{Ar}), 7.73 (t, *J* = 8.0 Hz, 1H, H_{Ar}), 8.26 (s, 1H, H_{Ar}), 10.91 (s, 1H, NH).

**¹³C NMR (101 MHz, DMSO-*d₆*) δ :** 19.8 (2xCH_{3 iPr}), 24.5 (CH_{iPr}), 53.6 (CH_{3-O}), 99.7 (CH_{Ar}), 103.7 (CH_{Ar}), 106.3 (CH_{Ar}), 136.4 (C_{q}), 139.3 (C_{q}), 141.0 (CH_{Ar}), 149.6 (C_{q}), 152.0 (C_{q}), 154.3 (C_{q}), 162.4 (C_{q}).

### Example 3.67: Synthesis of 6-chloro-3-isopropyl-N-(5-methoxypyridin-2-yl)-[1,2,4]triazolo[4,3-b]pyridazin-8-amine (3.67)

Under inert gas, to a solution of **(2.1)** (0.125 g, 0.54 mmol, 1.0 eq.) and corresponding aminopyridine derivative (0.102 g, 0.81 mmol, 1.5 eq.) in dry THF (7.5 mL), tBuOK 1M in THF (0.81 mL, 0.81 mmol, 1.5 eq.) was added dropwise. The mixture was stired 3.5 h at room temperature then saturated NH₄Cl (3.0 mL), water (1.0 mL) and EtOAc (3.0 mL) were added. The mixture was vigorously stired 5 min then layers were separated and aqueous layer was extracted twice with EtOAc (2x5.0 mL). Organic layers were combined, dried over MgSO₄, filtered, concentrated and purified by flash chromatography with DCM/MeOH (99/1 to 98/2) as eluent to give **(3.67)** (0.112 g, 65 %) as an orange solid.
**Rf** (DCM/MeOH, 98/2) : 0.24

**¹H NMR (400 MHz, DMSO-*d₆*) δ** : 1.42 (d, *J* = 6.9 Hz, 6H, 2xCH_{3 iPr}), 3.48 (hept, *J* = 6.9 Hz, 1H, H_{iPr}), 3.83 (s, 3H, CH_{3-O}), 7.50 (dd, *J* = 9.0, 3.0 Hz, 1H, H_{Ar}), 7.56 (d, *J* = 9.0 Hz, 1H, H_{Ar}), 8.15 (s, 1H, H_{Ar}), 8.18 (d, *J* = 3.0 Hz, 1H, H_{Ar}), 10.82 (s, 1H, NH).

**¹³C NMR (101 MHz, DMSO-*d₆*) δ :** 19.8 (2xCH_{3 iPr}), 24.5 (CH_{iPr}), 55.9 (CH_{3-O}), 97.8 (CH_{Ar}), 115.6 (CH_{Ar}), 124.9 (CH_{Ar}), 132.7 (CH_{Ar}), 136.7 (C_{q}), 139.3 (C_{q}), 147.3 (C_{q}), 149.8 (C_{q}), 151.8 (C_{q}), 154.2 (C_{q}).

**MS (ESI+) : m/z calcd for C₁₄H₁₅ClN₆O :** 319.1 [M+H]+, **found** : 319.2.

### Example 3.68: Synthesis of 6-chloro-3-isopropyl-N-(4-methoxypyridin-2-yl)-[1,2,4]triazolo[4,3-b]pyridazin-8-amine (3.68)

Under inert gas, to a solution of **(2.1)** (0.125 g, 0.54 mmol, 1.0 eq.) and corresponding aminopyridine derivative (0.102 g, 0.81 mmol, 1.5 eq.) in dry THF (7.5 mL), tBuOK 1M in THF (0.81 mL, 0.81 mmol, 1.5 eq.) was added dropwise. The mixture was stirred 3.5 h at room temperature then saturated NH₄Cl (3.0 mL), water (1.0 mL) and EtOAc (3.0 mL) were added. The mixture was vigorously stirred 5 min then layers were separated and aqueous layer was extracted twice with EtOAc (2x5.0 mL). Organic layers were combined, dried over MgSO₄, filtered, concentrated and purified by flash chromatography with DCM/MeOH (99/1 to 98/2) as eluent to give **(3.68)** (0.125 g, 72 %) as a light orange solid.
**Rf** (DCM/MeOH, 98/2) : 0.25

**¹H NMR (400 MHz, DMSO-*d₆*) δ :** 1.42 (d, *J* = 7.0 Hz, 6H, 2xCH_{3 iPr}), 3.49 (hept, *J* = 6.9 Hz, 1H, H_{iPr}), 3.83 (s, 3H, CH_{3-O}), 6.76 (dd, *J* = 5.9, 2.3 Hz, 1H, H_{Ar}), 7.25 (d, *J* = 2.3 Hz, 1H, H_{Ar}), 8.26 (d, *J* = 5.9 Hz, 1H, H_{Ar}), 8.32 (s, 1H, H_{Ar}), 10.74 (s, 1H, NH).

**¹³C NMR (101 MHz, DMSO-*d₆*) δ :** 19.8 (2xCH_{3 iPr}), 24.5 (CH_{iPr}), 55.3 (CH_{3-O}), 99.1 (CH_{Ar}), 99.7 (CH_{Ar}), 106.6 (CH_{Ar}), 136.7 (C_{q}), 139.3 (C_{q}), 148.5 (CH_{Ar}), 149.7 (C_{q}), 154.3 (C_{q}), 155.6 (C_{q}), 166.5 (C_{q}).

### Example 3.69: Synthesis of 6-chloro-3-isopropyl-N-(3-methoxypyridin-2-yl)-[1,2,4]triazolo[4,3-b]pyridazin-8-amine (3.69)

Under inert gas, to a solution of **(2.1)** (0.125 g, 0.54 mmol, 1.0 eq.) and corresponding aminopyridine derivative (0.102 g, 0.81 mmol, 1.5 eq.) in dry THF (7.5 mL), tBuOK 1M in THF (0.81 mL, 0.81 mmol, 1.5 eq.) was added dropwise. The mixture was stired 3.5 h at room temperature then saturated NH₄Cl (3.0 mL), water (1.0 mL) and EtOAc (3.0 mL) were added. The mixture was vigorously stired 5 min then layers were separated and aqueous layer was extracted twice with EtOAc (2x5.0 mL). Organic layers were combined, dried over MgSO₄, filtered, concentrated and purified by flash chromatography with DCM/MeOH (99/1 to 98/2) as eluent to give **(3.69)** (0.120 g, 70 %) as a white solid.
**Rf** (DCM/MeOH, 98/2) : 0.38

**¹H NMR (400 MHz, DMSO-*d₆*) δ** : 1.42 (d, *J* = 6.9 Hz, 6H, 2xCH_{3 iPr}), 3.49 (hept, *J* = 7.0 Hz, 1H, H_{iPr}), 4.01 (s, 3H, CH_{3-O}), 7.10 - 7.29 (m, 1H, H_{Ar}), 7.55 (d, *J* = 8.1 Hz, 1H, H_{Ar}), 8.04 (d, *J* = 4.9 Hz, 1H, H_{Ar}), 8.14 (s, 1H, H_{Ar}), 8.78 (s, 1H, NH).

**¹³C NMR (101 MHz, DMSO-*d₆*) δ** : 19.8 (2xCH_{3 iPr}), 24.5 (CH_{iPr}), 56.4 (CH_{3-O}), 99.3 (CH_{Ar}), 118.1 (CH_{Ar}), 119.4 (CH_{Ar}), 135.2 (C_{q}), 138.3 (CH_{Ar}), 139.1 (C_{q}), 142.7 (C_{q}), 144.0 (C_{q}), 149.7 (C_{q}), 154.5 (C_{q}).

**MS (ESI+) : m/z calcd for C₁₄H₁₅ClN₆O :** 319.1 [M+H]+, **found** : 319.2.

### Example 3.70: Synthesis of 6-chloro-3-isopropyl-N-(3-phenylpropyl)-[1,2,4]triazolo[4,3-b]pyridazin-8-amine (3.70)

To a solution of **(2.1)** (0.130 g, 0.56 mmol, 1.0 eq.) in THF (6.0 mL), corresponding amine (0.16 mL, 1.12 mmol, 2.0 eq.) and Et₃N (0.16 mL, 1.12 mmol, 2.0 eq.) were added and the mixture was refluxed 2 h. After cooling, the precipitate was filtered off, washed with THF then the filtrate was concentrated and the residu was purified by flash chromatography with DCM/MeOH (98/2) as eluent to give **(3.70)** (0.177 g, 95 %) as a white solid.

**¹H NMR (400 MHz, DMSO-*d₆*) δ** : 1.39 (d, *J* = 6.9 Hz, 6H, 2xCH_{3 iPr}), 1.84 - 2.02 (m, 2H, CH_{2-CH2-NH}), 2.68 (dd, *J* = 8.8, 6.7 Hz, 2H, CH_{2-Ph}), 3.33 - 3.39 (m, 2H, CH_{2-NH}), 3.44 (h, *J* = 7.0 Hz, 1H, H_{iPr}), 6.13 (s, 1H, H_{Ar}), 7.14 - 7.23 (m, 1H, H_{Ar}), 7.21 - 7.33 (m, 4H, 4×H_{Ar}), 8.61 (s, 1H, NH).

**¹³C NMR (101 MHz, DMSO-*d₆*) δ** : 20.3 (2xCH_{3 iPr}), 24.9 (CH_{iPr}), 29.9 (CH_{2-CH2-NH}), 32.9 (CH_{2-Ph}), 42.2 (CH_{2-NH}), 91.2 (CH_{Ar}), 126.3 (CH_{Ar}), 128.8 (2xCH_{Ar}), 128.8 (CH_{Ar}), 139.9 (C_{q}), 142.0 (C_{q}), 142.9 (C_{q}), 150.1 (C_{q}), 154.4 (C_{q}).

### Example 3.71: Synthesis of 6-chloro-3-cyclopropyl-N-phenyl-[1,2,4]triazolo[4,3-b]pyridazin-8-amine (3.71)

Under inert gas, to a solution of **(2.5)** (0.150 g, 0.65 mmol, 1.0 eq.) in dry THF (5.0 mL), aniline (0.12 mL, 1.31 mmol, 2.0 eq.) was added then tBuOK 1M in THF (1.31 mL, 1.31 mmol, 2.0 eq.). The mixture was stired 1 h at room temperature then saturated NH₄Cl (5.0 mL) and water (1.5 mL) were added and stired vigorously 5 min. Layers were separated and aqueous layer was extracted twice with EtOAc (2x5.0 mL). Orgnics layers were combined, dried over MgSO₄, filtered, concentrated and the residu was purified by flash chromatography with DCM/MeOH (99/1 to 98/2) as eluent to give **(3.71)** (0.175 g, 94 %) as a white solid.

**¹H NMR (400 MHz, DMSO-*d₆*) δ** : 1.10 - 1.26 (m, 4H, 2×CH_{2 cPr}), 2.37 (tt, *J* = 8.1, 5.3 Hz, 1H, H_{cPr}), 6.34 (s, 1H, H_{Ar}), 7.27 (ddd, *J* = 8.5, 5.9, 2.2 Hz, 1H, H_{Ar}), 7.38 - 7.55 (m, 4H, 4×H_{Ar}), 10.43 (s, 1H, NH).

**¹³C NMR (101 MHz, DMSO-*d₆*) δ** : 4.9 (CH_{cPr}), 7.2 (2×CH_{2 cPr}), 92.9 (CH_{Ar}), 123.5 (2xCH_{Ar}), 125.7 (CH_{Ar}), 129.5 (2×CH_{Ar}), 137.7 (C_{q}), 139.5 (C_{q}), 140.2 (C_{q}), 149.8 (C_{q}), 151.6 (C_{q}).

**MS (ESI+) : m/z calcd for C₁₄H₁₂ClN₅ :** 286.1 [M+H]+, **found** : 286.2.

### Example 3.72: Synthesis of 6-chloro-3-cyclopropyl-N-(2-pyridyl)-[1,2,4]triazolo[4,3-b]pyridazin-8-amine (3.72)

Under inert gas, to a solution of **(2.5)** (0.150 g, 0.65 mmol, 1.0 eq.) in dry THF (5.0 mL), corresponding aminopyridine (0.125 g, 1.31 mmol, 2.0 eq.) was added then tBuOK 1M in THF (1.31 mL, 1.31 mmol, 2.0 eq.). The mixture was stired 1 h at room temperature then saturated NH₄Cl (5.0 mL) and water (1.5 mL) were added and stired vigorously 5 min. Layers were separated and aqueous layer was extracted twice with EtOAc (2x5.0 mL). Orgnics layers were combined, dried over MgSO₄, filtered, concentrated and the residu was purified by flash chromatography with DCM/MeOH (99/1 to 96/4) as eluent to give **(3.72)** (0.085 g, 45 %) as a white solid.

**¹H NMR (400 MHz, DMSO-*d₆*) δ :** 1.11 - 1.21 (m, 4H, 2×CH_{2 cPr}), 2.39 (tt, *J* = 8.0, 5.4 Hz, 1H, CH_{cPr}), 7.13 (ddd, *J* = 7.3, 5.0, 1.0 Hz, 1H, H_{Ar}), 7.48 - 7.66 (m, 1H, H_{Ar}), 7.82 (ddd, *J* = 8.4, 7.3, 2.0 Hz, 1H, H_{Ar}), 8.31 (s, 1H, H_{Ar}), 8.45 (dd, *J* = 5.3, 1.9 Hz, 1H, H_{Ar}), 10.87 (s, 1H, NH).

**¹³C NMR (101 MHz, DMSO-*d₆*) δ :** 4.9 (CH_{cPr}), 7.2 (2×CH_{2 cPr}), 99.5 (CH_{Ar}), 114.6 (CH_{Ar}), 118.6 (CH_{Ar}), 136.6 (C_{q}), 138.3 (CH_{Ar}), 139.2 (C_{q}), 147.3 (CH_{Ar}), 150.0 (C_{q}), 151.7 (C_{q}), 153.9 (C_{q}).

**MS (ESI+) : m/z calcd for C₁₃H₁₁ClN₆ :** 287.1 [M+H]+, **found** : 287.2.

### Example 3.73: Synthesis of 6-chloro-3-cyclopropyl-N-(3-pyridyl)-[1,2,4]triazolo[4,3-b]pyridazin-8-amine (3.73)

Under inert gas, to a solution of **(2.5)** (0.150 g, 0.65 mmol, 1.0 eq.) in dry THF (5.0 mL), corresponding aminopyridine (0.125 g, 1.31 mmol, 2.0 eq.) was added then tBuOK 1M in THF (1.31 mL, 1.31 mmol, 2.0 eq.). The mixture was stirred 1 h at room temperature then saturated NH₄Cl (5.0 mL) and water (1.5 mL) were added and stired vigorously 5 min. Layers were separated and aqueous layer was extracted twice with EtOAc (2x5.0 mL). Organic layers were combined, dried over MgSO₄, filtered, concentrated and the residue was purified by flash chromatography with DCM/MeOH (99/1 to 96/4) as eluent to give **(3.73)** (0.080 g, 43 %) as a white solid.

**¹H NMR (400 MHz, DMSO-*d₆*) δ :** 1.15 (tt, *J* = 7.7, 2.6 Hz, 4H, 2×CH_{2 cPr}), 2.38 (tt, *J* = 8.1, 5.3 Hz, 1H, H_{cPr}), 6.42 (s, 1H, H_{Ar}), 7.50 (dd, *J* = 8.2, 4.7 Hz, 1H, H_{Ar}), 7.91 (ddd, *J* = 8.3, 2.7, 1.5 Hz, 1H, H_{Ar}), 8.46 (dd, *J* = 4.8, 1.5 Hz, 1H, H_{Ar}), 8.69 (d, *J* = 2.6 Hz, 1H, H_{Ar}), 10.51 (s, 1H, NH).

**¹³C NMR (101 MHz, DMSO-*d₆*) δ :** N.D.

**MS (ESI+) : m/z calcd for C₁₃H₁₁ClN₆** : 287.1 [M+H]+, **found :** 287.2.

### Example 3.74: Synthesis of 6-chloro-3-cyclopropyl-N-(4-pyridyl)-[1,2,4]triazolo[4,3-b]pyridazin-8-amine (3.74)

Under inert gas, to a solution of **(2.5)** (0.150 g, 0.65 mmol, 1.0 eq.) in dry THF (5.0 mL), corresponding aminopyridine (0.125 g, 1.31 mmol, 2.0 eq.) was added then tBuOK 1M in THF (1.31 mL, 1.31 mmol, 2.0 eq.). The mixture was stired 1 h at room temperature then saturated NH₄Cl (5.0 mL) and water (1.5 mL) were added and stired vigorously 5 min. Layers were separated and aqueous layer was extracted twice with EtOAc (2x5.0 mL). Orgnics layers were combined, dried over MgSO₄, filtered, concentrated and the residu was purified by flash chromatography with DCM/MeOH (99/1 to 96/4) as eluent to give **(3.74)** (0.090 g, 48 %) as a white solid.

**¹H NMR (400 MHz, DMSO-*d₆*) δ :** 1.16 (tt, *J* = 8.0, 2.8 Hz, 4H, 2×CH_{2 cPr}), 2.38 (ddd, *J* = 10.5, 8.3, 5.2 Hz, 1H, H_{cPr}), 6.87 (s, 1H, H_{Ar}), 7.42 - 7.58 (m, 2H, 2xH_{Ar}), 8.54 (d, *J* = 5.4 Hz, 2H, 2xH_{Ar}), 10.66 (s, 1H, NH).

**¹³C NMR (101 MHz, DMSO-*d₆*) δ :** 4.9 (CH_{cPr}), 7.3 (2×CH_{2 cPr}), 96.8 (CH_{Ar}), 115.5 (CH_{Ar}), 138.0 (C_{q}), 139.4 (C_{q}), 145.7 (C_{q}), 149.8 (C_{q}), 150.7 (CH_{Ar}), 151.8 (C_{q}).

**MS (ESI+) : m/z calcd for C₁₃H₁₁ClN₆** : 287.1 [M+H]+, **found** : 287.2.

### Example 3.75: Synthesis of 6-chloro-3-cyclopropyl-N-(pyridazin-3-yl)-[1,2,4]triazolo[4,3-b]pyridazin-8-amine (3.75)

To a solution of **(2.5)** (0.250 g, 1.091 mmol, 1.0 eq.) in dry THF (8.38 mL), 3-aminopyridazine (0.212 g, 2.183 mmol, 2.0 eq.) then tBuOK 1M in THF (1.32 mL, 2.183 mmol, 2.0 eq.) were added and the mixture was stired 2h at room temperature. After completion, saturated NH₄Cl (8.38 mL) and water (2.53 mL) were added then biphasic mixture was vigorously stired 5 min. The precipitate formed was filtered off, washed with H₂O and Et₂O to give **(3.75)** (0.286 g, 91%) as a beige solid.
**Rf** (DCM/MeOH, 96/4) : 0.38

**¹H NMR (400 MHz, DMSO-*d₆*) δ :** 1.12 - 1.22 (m, 4H, 2xCH_{2 cPr}), 2.40 (td, *J* = 8.1, 4.0 Hz, 1H, H_{cPr}), 7.72 (dd, *J* = 9.0, 4.6 Hz, 1H, H_{Ar}), 7.87 (d, 1H, H_{Ar}), 8.37 (s, 1H, H_{Ar}), 9.00 (d, *J =* 4.6 Hz, 1H, H_{Ar}), 11.10 (s, 1H, NH).

**¹³C NMR (101 MHz, DMSO-*d₆*) δ :** 4.87 (CH_{cPr}),7.28 (2×CH_{2 cPr}), 101.25 (CH_{Ar}), 119.51 (CH_{Ar}), 128.67 (CH_{Ar}), 136.07 (C_{q}), 139.23 (C_{q}), 147.86 (CH_{Ar}), 150.02 (C_{q}), 151.82 (C_{q}), 157.27 (C_{q}).

**MS (ESI+) : m/z calcd for C₁₂H₁₀ClN₇** : 288.1 [M+H]+, **found :** 288.1.

### Example 3.76: Synthesis of 6-chloro-3-cyclopropyl-N-(pyridazin-4-yl)-[1,2,4]triazolo[4,3-b]pyridazin-8-amine (3.76)

To a solution of **(2.5)** (0.087 g, 0.380 mmol, 1.0 eq.) in dry THF (2.94 mL), 4-aminopyridazine (0.074 g, 0.760 mmol, 2.0 eq.) then tBuOK 1M in THF (0.76 mL, 0.760 mmol, 2.0 eq.) were added and the mixture was stired 1.2h at room temperature. After completion, saturated NH₄Cl (2.94 mL) and water (0.871 mL) were added then biphasic mixture was vigorously stired 5 min. Layers were separated, aqueous layer was extracted with EtOAc (2x10 mL) then organic layers were combined, dried over MgSO₄, filtered and concentrated. The residue was triturated in Et₂O, filtered off, washed with Et₂O and dried under vacuum. The residue was then triturated in DCM, filtered off, washed with DCM and dried under vacuum to give **(3.76)** (0.094 g, 86%) as a white solid.
**Rf** (DCM/MeOH, 96/4) : 0.35

**¹H NMR (400 MHz, DMSO-*d₆*) δ :** 1_{.}10 - 1.23 (m, 4H, 2xCH_{2 cPr}), 2.35 - 2.44 (m, 1H, H_{cPr}), 7.05 (s, 1H, H_{Ar}), 7.76 (dd, *J* = 6.2, 2.9 Hz, 1H, H_{Ar}), 9.08 (d, *J* = 5.9 Hz, 1H, H_{Ar}), 9.37 (s, 1H, H_{Ar}), 10.87 (s, 1H, NH).

**¹³C NMR (101 MHz, DMSO-*d₆*) δ :** 4.85 (CH_{cPr}), 7.30 (2×CH_{2 cPr}), 98.78 (CH_{Ar}), 115.07 (CH_{Ar}), 137.63 (C_{q}), 138.57 (C_{q}), 139.43 (C_{q}), 145.29 (CH_{Ar}), 149.72 (C_{q}), 151.17 (CH_{Ar}), 151.82 (C_{q}).

**MS (ESI+) : m/z calcd for C₁₂H₁₀ClN₇** : 288.1 [M+H]+, **found :** 288.2.

### Example 3.77: Synthesis of 6-chloro-3-cyclopropyl-N-pyrimidin-4-yl-[1,2,4]triazolo[4,3-b]pyridazin-8-amine (3.77)

To a solution of **(2.5)** (0.500 g, 2.183 mmol, 1.0 eq.) in dry THF (16.77 mL), 4-aminopyrimidine (0.416 g, 4.366 mmol, 2.0 eq.) then tBuOK 1.65M in THF (2.65 mL, 4.366 mmol, 2.0 eq.) were added and the mixture was stired 1.3h at room temperature. After completion, saturated NH₄Cl (16.77 mL) and water (5.06 mL) were added then biphasic mixture was vigorously stired 5 min. The precipitate formed was filtered off, washed with H₂O, Et₂O and pentane and dried under vacuum to give **(3.77)** (0.463 g, 74%) as a yellow solid.
**Rf** (DCM/MeOH, 96/4) : 0.31

**¹H NMR (400 MHz, DMSO-*d₆*) δ :** 1.11 - 1.23 (m, 4H, 2×CH_{2 cPr}), 2.35 - 2.44 (m, 1H, CH_{cPr}), 7.58 (d, *J* = 5.8 Hz, 1H, H_{Ar}), 8.36 (s, 1H, H_{Ar}), 8.62 (d, *J* = 5.8 Hz, 1H, H_{Ar}), 9.00 (s, 1H, H_{Ar}), 11.25 (s, 1H, NH).

**¹³C NMR (101 MHz, DMSO-*d₆*) δ :** 4.84 (CH_{cPr}), 7.30 (2×CH_{2 cPr}), 102.66 (CH_{Ar}), 110.86 (CH_{Ar}), 135.79 (C_{q}), 139.05 (C_{q}), 149.69 (C_{q}), 151.85 (C_{q}), 157.08 (CH_{Ar}), 157.73 (CH_{Ar}), 159.67 (C_{q}).

**MS (ESI+) : m/z calcd for C₁₂H₁₀ClN₇** : 288.1 [M+H]+, **found :** 288.2.

### Example 3.78: Synthesis of 6-chloro-3-cyclopropyl-N-(pyrimidin-5-yl)-[1,2,4]triazolo[4,3-b]pyridazin-8-amine (3.78)

To a solution of **(2.5)** (0.135 g, 0.589 mmol, 1.0 eq.) in dry THF (4.53 mL), 5-aminopyrimidine (0.113 g, 1.179 mmol, 2.0 eq.) then tBuOK 1M in THF (1.18 mL, 1.179 mmol, 2.0 eq.) were added and the mixture was stired 1h at room temperature. After completion, saturated NH₄Cl (4.53 mL) and water (1.35 mL) were added then biphasic mixture was vigorously stired 5 min. Layers were separated, aqueous layer was extracted with EtOAc (2x10.0 mL) then organic layers were combined, dried over MgSO₄, filtered and concentrated. The residue was triturated in Et₂O, filtered off, washed with Et₂O, solubilised in DCM and concentrated under vacuum to give **(3.78)** (0.153 g, 90%) as a beige solid.
**Rf** (DCM/MeOH, 92/8) : 0.54

**¹H NMR (400 MHz, DMSO-*d₆*) δ** : 1.09 - 1.20 (m, 4H, 2xCH_{2 cPr}), 2.34 - 2.42 (m, 1H, H_{cPr}), 6.63 (s, 1H, H_{Ar}), 8.94 (d, *J* = 1.4 Hz, 2H, 2xH_{Ar}), 9.05 (d, *J* = 1.4 Hz, 1H, H_{Ar}), 10.54 (s, 1H, NH).

**¹³C NMR (101 MHz, DMSO-*d₆*) δ** : 4.86 (CH_{cPr}), 7.26 (2×CH_{2 cPr}), 95.16 (CH_{Ar}), 133.99 (C_{q}), 139.34 (C_{q}), 139.54 (C_{q}), 149.85 (C_{q}), 151.58 (2×CH_{Ar}), 151.68 (C_{q}), 154.67 (CH_{Ar}).

**MS (ESI+) : m/z calcd for C₁₂H₁₀ClN₇** : 288.1 [M+H]+, **found :** 288.2.

### Example 3.79: Synthesis of 6-chloro-3-cyclopropyl-N-(pyrimidin-2-yl)-[1,2,4]triazolo[4,3-b]pyridazin-8-amine (3.79)

To a solution of **(2.5)** (0.100 g, 0.437 mmol, 1.0 eq.) in dry THF (3.36 mL), 2-aminopyrimidine (0.084 g, 0.873 mmol, 2.0 eq.) then tBuOK 1M in THF (0.87 mL, 0.873 mmol, 2.0 eq.) were added and the mixture was stired 1.4h at room temperature. After completion, saturated NH₄Cl (3.36 mL) and water (1.0 mL) were added then biphasic mixture was vigorously stired 5 min. Layers were separated, aqueous layer was extracted with EtOAc (2x5.0 mL) then organic layers were combined, dried over MgSO₄, filtered and concentrated. The residue was triturated in Et₂O, filtered off, washed with Et₂O and dried under vacuum to give **(3.79)** (0.100 g, 80%) as a white solid.
**Rf** (DCM/MeOH, 96/4) : 0.57

**¹H NMR (400 MHz, DMSO-*d₆*) δ :** 1.12 - 1.22 (m, 4H, 2xCH_{2 cPr}), 2.39 (td, *J* = 8.2, 4.1 Hz, 1H, H_{cPr}), 7.26 (t, *J* = 4.8 Hz, 1H, H_{Ar}), 8.15 (s, 1H, H_{Ar}), 8.79 (d, *J* = 4.8 Hz, 2H, 2xH_{Ar}), 10.28 (s, 1H, NH).

**¹³C NMR (101 MHz, DMSO-*d₆*) δ :** 4.84 (CH_{cPr}), 7.28 (2×CH_{2 cPr}), 99.52 (C_{q}), 101.30 (CH_{Ar}), 116.39 (CH_{Ar}), 136.08 (C_{q}), 138.96 (C_{q}), 149.82 (C_{q}), 151.82 (C_{q}), 158.64 (2xCH_{Ar}).

**MS (ESI+)** : **m/z calcd for C₁₂H₁₀ClN₇** : 288.1 [M+H]+, **found** : 288.2.

### Example 3.80: Synthesis of 6-chloro-3-cyclopropyl-N-(pyrazin-2-yl)-[1,2,4]triazolo[4,3-b]pyridazin-8-amine (3.80)

To a solution of **(2.5)** (0.080 g, 0.349 mmol, 1.0 eq.) in dry THF (2.68 mL), 2-aminopyrazine (0.066 g, 0.699 mmol, 2.0 eq.) then tBuOK 1M in THF (0.70 mL, 0.699 mmol, 2.0 eq.) were added and the mixture was stired 1.2h at room temperature. After completion, saturated NH₄Cl (2.68 mL) and water (0.81 mL) were added then biphasic mixture was vigorously stired 5 min. The precipitate formed was filtered off, washed with H₂O and Et₂O to give **(3.80)** (0.072 g, 72%) as a white solid. Layers of filtrate were separated, aqueous layer was extracted with EtOAc (2x5.0 mL) then organic layers were combined, dried over MgSO₄, filtered and concentrated. The residue was triturated in Et₂O, filtered off, washed with Et₂O and dried under vacuum to give the second part of **(3.80)** (0.012 g, 12%) as a white solid. Total of **(3.80)** : 0.084 g, 84%.
**Rf** (DCM/MeOH, 96/4) : 0.11

**¹H NMR (400 MHz, DMSO-*d₆*) δ** : 1.12 - 1.21 (m, 4H, 2xCH_{2 c}Pᵣ), 2.39 (tt, *J* = 8.0, 5.3 Hz, 1H, H_{cPr}), 8.18 (s, 1H, H_{Ar}), 8.32 (d, *J* = 2.8 Hz, 1H, H_{Ar}), 8.44 - 8.50 (m, 1H, H_{Ar}), 8.91 (s, 1H, H_{Ar}), 11.29 (s, 1H, NH).

**¹³C NMR (101 MHz, DMSO-*d₆*) δ** : 4_{.}84 (CH_{cPr}), 7.28 (2×CH_{2 cPr}), 100.30 (CH_{Ar}), 136.25 (C_{q}), 137.68 (CH_{Ar}), 138.02 (CH_{Ar}), 139.14 (C_{q}), 141.16 (CH_{Ar}), 149.80 (C_{q}), 150.63 (C_{q}), 151.81 (C_{q}).

**MS (ESI+)** : **m/z calcd for C₁₂H₁₀ClN₇** : 288.1 [M+H]+, **found :** 288.1.

### Example 3.81: Synthesis of 6-chloro-3-cyclopropyl-N-(6-methoxypyridin-2-yl)-[1,2,4]triazolo[4,3-b]pyridazin-8-amine (3.81)

Under inert gas, to a solution of **(2.5)** (0.125 g, 0.55 mmol, 1.0 eq.) in dry THF (7.5 mL), 6-methoxypyridin-2-amine (0.103 g, 0.82 mmol, 1.5 eq.) was added then tBuOK 1M in THF (0.82 mL, 0.82 mmol, 1.5 eq.). The mixture was stired 3 h at room temperature then saturated NH₄Cl (6.0 mL) and water (1.5 mL) were added and stired vigorously 5 min. Layers were separated and aqueous layer was extracted twice with EtOAc (2x5.0 mL). Orgnics layers were combined, dried over MgSO₄, filtered, concentrated and the residu was purified by flash chromatography with DCM/MeOH (99/1 to 96/4) as eluent to give **(3.81)** (0.115 g, 67 %) as a white solid.
**Rf** (DCM/MeOH, 98/2) : 0.28

**¹H NMR (400 MHz, DMSO-*d₆*) δ :** 1.12 - 1.20 (m, 4H, 2×CH_{2 cPr}), 2.38 (tt, *J* = 8.0, 5.4 Hz, 1H, H_{cPr}), 3.95 (s, 3H, OCH₃), 6.54 (d, *J* = 8.0 Hz, 1H, H_{Ar}), 7.16 (d, *J* = 7.8 Hz, 1H, H_{Ar}), 7.72 (t, *J* = 7.9 Hz, 1H, H_{Ar}), 8.25 (s, 1H, H_{Ar}), 10.87 (s, 1H, NH).

**¹³C NMR (101 MHz, DMSO-*d₆*) δ** : 4.9 (C_{q}), 7.3 (2×CH_{2 cPr}), 53.6 (OCH₃), 99.6 (CH_{Ar}), 103.7 (CH_{Ar}), 106.3 (CH_{Ar}), 136.4 (C_{q}), 139.2 (C_{q}), 140.9 (CH_{Ar}), 149.8 (C_{q}), 151.7 (C_{q}), 152.0 (C_{q}), 162.4 (C_{q}).

**MS (ESI+) : m/z calcd for C₁₄H₁₃ClN₆O :** 317.1 [M+H]+, **found :** 317.3.

### Example 3.82: Synthesis of 6-chloro-3-cyclopropyl-N-(2-methoxypyridin-3-yl)-[1,2,4]triazolo[4,3-b]pyridazin-8-amine (3.82)

Under inert gas, to a solution of **(2.5)** (0.125 g, 0.55 mmol, 1.0 eq.) in dry THF (7.5 mL), 2-methoxypyridin-3-amine (0.103 g, 0.82 mmol, 1.5 eq.) was added then tBuOK 1M in THF (0.82 mL, 0.82 mmol, 1.5 eq.). The mixture was stired 3 h at room temperature then saturated NH₄Cl (6.0 mL) and water (1.5 mL) were added and stired vigorously 5 min. Layers were separated and aqueous layer was extracted twice with EtOAc (2x5.0 mL). Orgnics layers were combined, dried over MgSO₄, filtered, concentrated and the residu was purified by flash chromatography with DCM/MeOH (99/1 to 96/4) as eluent to give **(3.82)** (0.113 g, 65 %) as a white solid.
**Rf** (DCM/MeOH, 98/2) : 0.28

**¹H NMR (400 MHz, DMSO-*d₆*) δ** : 1.08 - 1.21 (m, 4H, 2×CH_{2 cPr}), 2.36 (tt, *J* = 7.6, 5.1 Hz, 1H, H_{cPr}), 3.90 (s, 3H, OCH₃), 5.85 (s, 1H, H_{Ar}), 7.11 (dd, *J* = 7.5, 4.9 Hz, 1H, H_{Ar}), 7.78 (dd, *J* = 7.6, 1.7 Hz, 1H, H_{Ar}), 8.18 (dd, *J* = 4.9, 1.8 Hz, 1H, H_{Ar}), 9.99 (s, 1H, NH).

**¹³C NMR (101 MHz, DMSO-*d₆*) δ :** 4.9 (CH_{cPr}), 7.2 (2×CH_{2 cPr}), 53.5 (OCH₃), 94.2 (CH_{Ar}), 117.5 (CH_{Ar}), 120.7 (C_{q}), 135.7 (CH_{Ar}), 139.3 (C_{q}), 140.5 (C_{q}), 145.0 (CH_{Ar}), 149.5 (C_{q}), 151.5 (C_{q}), 157.9 (C_{q}).

**MS (ESI+) : m/z calcd for C₁₄H₁₃ClN₆O :** 317.1 [M+H]+, **found :** 317.3.

### Example 3.83: Synthesis of 6-chloro-3-cyclopropyl-N-(2-methoxypyrimidin-4-yl)-[1,2,4]triazolo[4,3-b]pyridazin-8-amine (3.83)

Under inert gas, to a solution of 2-methoxypyrimidin-4-amine (0.112 g, 0.85 mmol, 1.3 eq.) in dry THF (6.5 mL), KHMDS 0.5M in toluene (1.83 mL, 0.92 mmol, 1.4 eq.) was added. The mixture was stired 10 min at room temperature then **(2.5)** (0.150 g, 0.65 mmol, 1.0 eq.) was added and the mixture was stired 2.5 h at room temperature. After completion, NH₄Cl (0.5 mL) and water (0.5 mL) were added and stired vigorously 5 min then the mixture was concentrated with SiO₂ to make solid deposit and purified by flash chromatography with DCM/MeOH (99/1 to 95/5) as eluent to give **(3.83)** (0.097 g, 47 %) as a white solid.
**Rf** (DCM/MeOH, 96/4) : 0.22

**¹H NMR (400 MHz, DMSO-*d₆*) δ :** 1.11 - 1.23 (m, 4H, 2×CH_{2 cPr}), 2.39 (tt, *J* = 8.1, 5.3 Hz, 1H, H_{cPr}), 3.95 (s, 3H, CH_{3-O}), 7.20 (d, *J* = 5.6 Hz, 1H, H_{Ar}), 8.30 (s, 1H, H_{Ar}), 8.39 (d, *J* = 5.6 Hz, 1H, H_{Ar}), 11.21 (s, 1H, NH).

**¹³C NMR (101 MHz, DMSO-*d₆*) δ** : 4.8 (CH_{cPr}), 7.3 (2×CH_{cPr}), 54.5 (CH_{3-O}), 103.0 (CH_{Ar}), 104.4 (CH_{Ar}), 135.7 (C_{q}), 139.0 (C_{q}), 149.6 (C_{q}), 151.8 (C_{q}), 158.9 (CH_{Ar}), 161.7 (C_{q}), 164.4 (C_{q}).

### Example 3.84: Synthesis of 6-chloro-3-cyclopropyl-N-(6-methoxypyridazin-3-yl)-[1,2,4]triazolo[4,3-b]pyridazin-8-amine (3.84)

Under inert gas, to a solution of 6-methoxypyridazin-3-amine (0.112 g, 0.85 mmol, 1.3 eq.) in dry THF (6.5 mL), KHMDS 0.5M in toluene (1.83 mL, 0.92 mmol, 1.4 eq.) was added. The mixture was stired 10 min at room temperature then **(2.5)** (0.150 g, 0.65 mmol, 1.0 eq.) was added and the mixture was stired 2.5 h at room temperature. After completion, NH₄Cl (0.5 mL) and water (0.5 mL) were added and stired vigorously 5 min then the mixture was concentrated with SiO₂ to make solid deposit and purified by flash chromatography with DCM/MeOH (99/1 to 95/5) as eluent to give **(3.84)** (0.110 g, 53 %) as a white solid.
**Rf** (DCM/MeOH, 96/4) : 0.34

**¹H NMR (400 MHz, DMSO-*d₆*) δ :** 1.17 (dd, *J* = 7.8, 5.4 Hz, 4H, 2×CH_{2 cPr}), 2.39 (tt, *J* = 8.0, 5.3 Hz, 1H, H_{cPr}), 4.02 (s, 3H, CH_{3-O}), 7.32 (d, *J* = 9.4 Hz, 1H, H_{Ar}), 7.85 (d, *J* = 9.5 Hz, 1H, H_{Ar}), 8.26 (s, 1H, H_{Ar}), 10.98 (s, 1H, NH).

**¹³C NMR (101 MHz, DMSO-*d₆*) δ :** 4.9 (CH_{cPr}), 7.3 (2×CH_{cPr}), 54.4 (CH_{3-O}), 100.3 (CH_{Ar}), 120.1 (CH_{Ar}), 124.0 (CH_{Ar}), 136.0 (C_{q}), 139.1 (C_{q}), 150.1 (C_{q}), 151.8 (C_{q}), 153.7 (C_{q}), 161.6 (C_{q}).

### Example 3.85: Synthesis of 6-chloro-3-cyclopropyl-N-(pyridazin-3-ylmethyl)-[1,2,4]triazolo[4,3-b]pyridazin-8-amine (3.85)

To a solution of **(2.5)** (0.100 g, 0.44 mmol, 1.0 eq.) in THF/H₂O (6.0/0.1 mL), corresponding amine dihydrochloride (0.103 g, 0.57 mmol, 1.3 eq.) and DIPEA (0.33 mL, 1.88 mmol, 4.3 eq.) were added and the mixture was refluxed 2 h. After cooling, the precipitate was filtered off and washed with THF. In another vacuum flask, washed the solid with water then washed again with THF in the first flask. The solid was dried under vacuum to give the first part of **(3.85)** (0.063 g). Organics filtrates were combined, dried ver MgSO₄, concentrated and the residu was purified by flash chromatography with DCM/MeOH (98/2) as eluent to give the second part of **(3.85)** (0.018 g). Total of **(3.85):** 0.081 g, 61 %, as a white solid.
**Rf** (DCM/MeOH, 96/4) : 0.18

**¹H NMR (400 MHz, DMSO-*d₆*) δ** : 1.06 - 1.16 (m, 4H, 2×CH_{2 cPr}), 2.32 (tt, *J* = 8.1, 5.3 Hz, 1H, H_{cPr}), 4.89 (s, 2H, CH_{2 Bn}), 6.26 (s, 1H, H_{Ar}), 7.63 - 7.75 (m, 2H, 2xH_{Ar}), 9.04 (s, 1H, NH_{Bn}), 9.17 (dd, *J* = 4.0, 2.6 Hz, 1H, H_{Ar}).

**¹³C NMR (101 MHz, DMSO-*d₆*) δ :** 4.8 (CH_{cPr}), 7.1 (2×CH_{2 cPr}), 45.7 (CH_{2 Bn}), 92.0 (CH_{Ar}), 125.7 (CH_{Ar}), 127.5 (CH_{Ar}), 139.3 (C_{q}), 142.5 (C_{q}), 149.7 (C_{q}), 151.0 (CH_{Ar}), 151.4 (C_{q}), 159.3 (C_{q}).

### Example 3.86: Synthesis of 6-chloro-3-cyclopropyl-N-phenethyl-[1,2,4]triazolo[4,3-b]pyridazin-8-amine (3.86)

To a solution of **(2.5)** (0.100 g, 0.44 mmol, 1.0 eq.) in THF/H₂O (6.0/0.1 mL), corresponding amine hydrochloride (0.089 g, 0.57 mmol, 1.3 eq.) and DIPEA (0.23 mL, 1.31 mmol, 3.0 eq.) were added and the mixture was refluxed 1.5 h. After cooling, the solvent was removed then the residu was triturated in water, the solid was filtered off, washed with water then Et₂O and dried under vacuum to give **(3.86)** (0.115 g, 84 %) as a white solid.
**Rf** (DCM/MeOH, 96/4) : 0.66

**¹H NMR (400 MHz, DMSO-*d₆*) δ** : 1.10 (ddt, *J* = 8.7, 7.5, 2.6 Hz, 4H, 2×CH_{2 cPr}), 2.31 (tt, *J* = 8.1, 5.3 Hz, 1H, H_{cPr}), 2.94 (dd, *J* = 8.0, 6.6 Hz, 2H, CH_{2-CH2-NH}), 3.58 (d, *J* = 7.2 Hz, 2H, CH_{2-NH}), 6.19 (s, 1H, H_{Ar}), 7.12 - 7.35 (m, 5H, 5×H_{Ar}), 8.48 (s, 1H, NH).

**¹³C NMR (101 MHz, DMSO-*d₆*) δ** : 4.8 (CH_{cPr}), 7.2 (2×CH_{2 cPr}), 33.8 (CH_{2-CH2-NH}), 43.5 (CH_{2-NH}), 91.0 (CH_{Ar}), 126.2 (CH_{Ar}), 128.3 (2×CH_{Ar}), 128.9 (CH_{Ar}), 138.9 (C_{q}), 139.3 (C_{q}), 142.2 (C_{q}), 149.9 (C_{q}), 151.3 (C_{q}).

**MS (ESI+) : m/z calcd for C₁₆H₁₆ClN₅** : 314.1 [M+H]+, **found :** 314.2.

### Example 3.87: Synthesis of 6-chloro-3-isopropyl-N-(6-methoxypyridin-2-yl)-[1,2,4]triazolo[4,3-b]pyridazin-8-amine (3.87)

Reaction was carried out on **(2.1)** (0.180 g, 0.78 mmol, 1 eq), aminopyridine derivative (0.145 g, 1.17 mmol, 1.5 eq), and tBuOK 1M in THF (1.17 mL, 1.17 mmol, 1.5 eq) in dry THF (10.8 mL) and stirred in a round flask at r.t for 4h. After completion, saturated NH₄Cl, water and EtOAc were added. The reaction mixture was extracted twice with AcOEt, filtered over MgSO₄, concentrated and purified by flash chromatography with DCM/MeOH (99/1 to 98/2) has eluent to give **(3.87)** (0.175 g, 71%) as a light orange solid.

**¹H NMR (400 MHz, DMSO) δ :** 10.04 (s, 1H), 8.18 (dd, *J* = 4.9, 1.8 Hz, 1H), 7.78 (dd, *J* = 7.6, 1.8 Hz, 1H), 7.12 (dd, *J* = 7.6, 4.9 Hz, 1H), 5.84 (s, 1H), 3.91 (s, 3H), 3.48 (hept, *J* = 6.9 Hz, 1H), 1.41 (d, *J* = 7.0 Hz, 6H).

**¹³C NMR (101 MHz, DMSO) δ :** 158.0, 154.1, 149.3, 145.1, 140.5, 139.3, 135.7, 120.6, 117.5, 94.2, 53.5, 24.5, 19.8.

### Example 3.88: Synthesis of 6-chloro-3-isopropyl-N-(6-methoxypyridin-3-yl)-[1,2,4]triazolo[4,3-b]pyridazin-8-amine (3.88)

Reaction was carried out on **(2.1)** (0.180 g, 0.78 mmol, 1 eq), aminopyridine derivative (0.145 g, 1.17 mmol, 1.5 eq), and tBuOK 1M in THF (1.17 mL, 1.17 mmol, 1.5 eq) in dry THF (10.8 mL) and stirred in a round flask at r.t for 4h. After completion, saturated NH₄Cl, water and EtOAc were added. The reaction mixture was extracted twice with AcOEt, filtered over MgSO₄, concentrated and purified by flash chromatography with DCM/MeOH (99/1 to 98/2) has eluent to give **(3.88)** (0.106 g, 43%) as a light orange solid.

**¹H NMR (400 MHz, DMSO) δ :** 10.32 (s, 1H), 8.24 (d, *J=* 2.7 Hz, 1H), 7.80 (dd, *J* = 8.8, 2.7 Hz, 1H), 6.93 (d, *J* = 8.8 Hz, 1H), 6.15 (s, 1H), 3.88 (s, 3H), 3.47 (hept, *J* = 6.9 Hz, 1H), 1.41 (d, *J* = 6.9 Hz, 6H).

**¹³C NMR (101 MHz, DMSO) δ :** 161.6, 154.2, 149.6, 143.0, 141.2, 139.4, 136.1, 128.3, 111.1, 92.9, 53.5, 24.9, 19.8.

### Example 3.89: Synthesis of 6-chloro-3-isopropyl-N-(5-methoxypyridin-3-yl)-[1,2,4]triazolo[4,3-b]pyridazin-8-amine (3.89)

Reaction was carried out on **(2.1)** (0.180 g, 0.78 mmol, 1 eq), aminopyridine derivative (0.145 g, 1.17 mmol, 1.5 eq), and tBuOK 1M in THF (1.17 mL, 1.17 mmol, 1.5 eq) in dry THF (10.8 mL) and stirred in a round flask at r.t for 4h. After completion, saturated NH₄Cl, water and EtOAc were added. The reaction mixture was extracted twice with AcOEt, filtered over MgSO₄, concentrated and purified by flash chromatography with DCM/AcOEt (8/2 to 35/65) has eluent to give (3.89) (0.162 g, 65%) as a light orange solid.

**¹H NMR (400 MHz, DMSO) δ :** 10.50 (s, 1H), 8.31 (d, *J* = 2.0 Hz, 1H), 8.20 (d, *J* = 2.6 Hz, 1H), 7.49 (s, 1H), 6.49 (s, 1H), 3.86 (s, 3H), 3.49 (hept, *J* = 6.9 Hz, 1H), 1.41 (d, *J* = 6.9 Hz, 6H).

**¹³C NMR (101 MHz, DMSO) δ :** 155.7, 154.2, 149.7, 140.0, 139.5, 136.9, 135.3, 134.2, 115.7, 94.3, 55.8, 24.5, 19.8.

### Example 3.90: Synthesis of 6-chloro-3-isopropyl-N-(4-methoxypyridin-3-yl)-[1,2,4]triazolo[4,3-b]pyridazin-8-amine (3.90)

Reaction was carried out on **(2.1)** (0.400 g, 1.73 mmol, 1 eq), aminopyridine derivative (0.322 g, 2.60 mmol, 1.5 eq), and tBuOK 1M in THF (2.6 mL, 2.60 mmol, 1.5 eq) in dry THF (20.0 mL) and stirred in a round flask at r.t for 4h. After completion, saturated NH₄Cl , water and EtOAc were added. The reaction mixture was extracted twice with AcOEt, filtered over MgSO₄, concentrated and purified by flash chromatography with DCM/AcOEt (8/2 to 35/65) has eluent to give **(3.90)** (0.261 g, 47%) as a brown solid.

**¹H NMR (400 MHz, DMSO) δ :** 10.15 (s, 1H), 8.48 (d, *J* = 5.7 Hz, 1H), 8.40 (s, 1H), 7.26 (d, *J* = 5.7 Hz, 1H), 5.74 (s, 1H), 3.89 (s, 3H), 3.53 - 3.41 (m, 1H), 1.41 (d, *J* = 7.0 Hz, 6H). **¹³C NMR (101 MHz, DMSO) δ :** 160.6, 154.6, 150.7, 149.8, 148.8, 141.7, 139.8, 123.0, 108.8, 94.5, 56.4, 23.0, 20.3.

### Example 3.91: Synthesis of 6-chloro-3-isopropyl-N-(3-phenylpropyl)-[1,2,4]triazolo[4,3-b]pyridazin-8-amine

To a solution of **(2.1)** (0.130 g, 0.56 mmol, 1.0 eq.) in THF (6.0 mL), corresponding amine (0.152 g, 1.12 mmol, 2.0 eq.) and Et₃N (0.16 mL, 1.12 mmol, 2.0 eq.) was added and the mixture was refluxed 2 h. After cooling, the precitpitate was filtered off, washed with THF then the filtrate was concentrated and the residu was purified by flash chromatography with DCM/MeOH (98/2) as eluent to give **(3.91)** (0.177 g, 95 %) as a white solid.
**Rf** (DCM/MeOH, 94/6) : 0.20

**¹H NMR (400 MHz, DMSO-*d₆*) δ :** 1.00 - 1.20 (m, 4H, 2×CH_{2 cPr}), 2.31 (tt, *J* = 8.1, 5.3 Hz, 1H, H_{cPr}), 2.96 (t, *J* = 7.1 Hz, 2H, CH₂), 3.49 - 3.76 (m, 2H, CH₂), 6.24 (s, 1H, H_{Ar}), 7.31 (dd, *J* = 7.8, 4.8 Hz, 1H, H_{Ar}), 7.71 (dt, *J =* 7.8, 2.0 Hz, 1H, H_{Ar}), 8.41 (dd, *J* = 4.8, 1.7 Hz, 1H, H_{Ar}), 8.49 (d, *J* = 2.3 Hz, 1H, H_{Ar}), 8.52 (s, 1H, NH).

**¹³C NMR (101 MHz, DMSO-*d₆*) δ** : 4.8 (CH), 7.1 (2×CH₂), 30.9 (CH₂), 42.9 (CH₂), 91.1 (CH_{Ar}), 123.3 (CH_{Ar}), 134.4 (C_{q}), 136.4 (CH_{Ar}), 139.3 (C_{q}), 142.2 (C_{q}), 147.5 (CH_{Ar}), 149.9 (C_{q}), 150.0 (CH_{Ar}), 151.3 (C_{q}).

### Example 4: Synthesis of [1,2,4]triazolo[4,3-b]pyridazine-6,8-diamine derivatives, as illustrated in scheme 1 or in step 3 of scheme 2

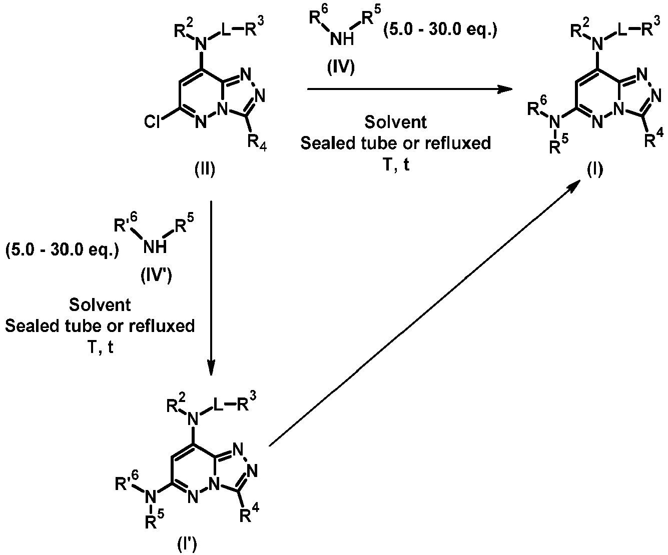

### Example 4.1: Synthesis of (2R)-2-[[8-(benzylamino)-3-isopropyl-[1,2,4]triazolo[4,3-b]pyridazin-6-yl]amino]butan-1-ol (1)

In a sealed tube 2 - 5 mL with a stir bar was charged **(3.1)** (0.100 g, 0.33 mmol, 1.0 eq.), NMP (1.0 mL) and corresponding amine (0.42 mL, 5.30 mmol, 16.0 eq.). The vial was sealed and then put on heating block, 20 h at 180 °C. After cooling, the mixture was poured in EtOAc (10 mL) then organic layer was washed twice with water and brine. The organic layer was dried over Na₂SO₄, filtered, concentrated and purified by flash chromatography with DCM/MeOH (94/6) as eluent to give **(1)** (0.070 g, 61 %) as a pale yellow solid.

### Example 4.2: Synthesis of (2S)-2-[[8-(benzylamino)-3-isopropyl-[1,2,4]triazolo[4,3-b]pyridazin-6-yl]amino]butan-1-ol (2)

In a sealed tube 2 - 5 mL with a stir bar was charged **(3.1)** (0.200 g, 0.66 mmol, 1.0 eq.), NMP (2.0 mL) and corresponding amine (0.32 mL, 3.98 mmol, 6.0 eq.). The vial was sealed and then put on heating block, 18 h at 180 °C. After cooling, the mixture was poured in water (10 mL), the resulting precipitate was filtered off then washed with water. A new precipitate was formed in the filtrate and it was filtered off then washed with water. The residue was taken up in EtOAc/MeOH (7/3), the organic filtrate was dried over Na₂SO₄, filtered, concentrated and purified by flash chromatography with DCM/MeOH (95/5 then 94/6) as eluent to give (2) (0.094 g, 40 %) as a white solid.

### Example 4.3: Synthesis of 2-[[8-(benzylamino)-3-isopropyl-[1,2,4]triazolo[4,3-b]pyridazin-6-yl]amino]propane-1,3-diol (3)

In a sealed tube 2 - 5 mL with a stir bar was charged **(3.1)** (0.200 g, 0.66 mmol, 1.0 eq.), NMP (2.0 mL) and serinol (0.362 g, 3.98 mmol, 6.0 eq.). The vial was sealed and then put on heating block, 18 h at 180 °C. After cooling, the mixture was poured in water (10 mL), the precipitate was filtered off, washed with water. A new precipitate was formed in filtrate, it was filtered off and washed with water. The residue was taken up in EtOAc/MeOH (7/3), the organic filtrate was dried over Na₂SO₄, filtered, concentrated and purified by flash chromatography with DCM/MeOH (95/5 then 94/6) as eluent to give **(3)** (0.092 g, 39 %) as a white solid.

### Example 4.4: Synthesis of (2R,3R)-2-[[8-(benzylamino)-3-isopropyl-[1,2,4]triazolo[4,3-b]230yridazine-6-yl]amino]butane-1,3-diol (4)

In a sealed tube 2 - 5 mL with a stir bar was charged **(3.1)** (0.175 g, 0.58 mmol, 1.0 eq.), NMP (1.75 mL) and D-threoninol (0.754 g, 6.96 mmol, 12.0 eq.). The vial was sealed and then put on heating block, 18 h at 180 °C. After cooling, the mixture was poured in a funnel with water (30 mL), aqueous layer was extracted twice with EtOAc (2x20 mL) and combined organics layers were washed with HCl 0.1M (20 mL) then brine and dried over Na₂SO₄, filtered, concentrated and purified by flash chromatography with DCM/MeOH (95/5 then 94/6) as eluent to give **(4)** (0.137 g, 64 %) as a white solid.

### Example 4.5: (2R,3R)-2-[[8-(benzylamino)-3-isopropyl-[1,2,4]triazolo[4,3-b]pyridazin-6-yl]amino]butane-1,3-diol (5)

In a sealed tube 2 - 5 mL with a stir bar was charged **(3.1)** (0.175 g, 0.58 mmol, 1.0 eq.), NMP (1.75 mL) and D-threoninol (0.754 g, 6.96 mmol, 12.0 eq.). The vial was sealed and then put on heating block, 18 h at 180 °C. After cooling, the mixture was poured in a funnel with water (30 mL), aqueous layer was extracted twice with EtOAc (2x20 mL) and combined organics layers were washed with HCl 0.1M (20 mL) then brine and dried over Na₂SO₄, filtered, concentrated and purified by flash chromatography with DCM/MeOH (95/5 then 94/6) as eluent to give **(5)** (0.137 g, 64 %) as a white solid.

### Example 4.6: Synthesis of N-benzyl-3-isopropyl-6-morpholino-[1,2,4]triazolo[4,3-b]pyridazin-8-amine (6)

In a sealed tube 2 - 5 mL with a stir bar was charged **(3.1)** (0.200 g, 0.66 mmol, 1.0 eq.), NMP (1.5 mL) and morpholine (0.82 mL, 9.28 mmol, 14.0 eq.). The vial was sealed and then put on heating block, 18 h at 180 °C. After cooling, the mixture was poured in a funnel with water (30 mL), aqueous layer was extracted twice with EtOAc (2x25 mL) and combined organics layers were washed with brine, dried over Na₂SO₄, filtered, concentrated and purified by flash chromatography with DCM/MeOH (97/3 then 96/4) as eluent to give (6) (0.116 g, 50 %) as a white solid.

### Example 4.7: Synthesis of (2S)-3-[[8-(benzylamino)-3-isopropyl-[1,2,4]triazolo[4,3-b]pyridazin-6-yl]amino]propane-1,2-diol (7)

In a sealed tube 2 - 5 mL with a stir bar was charged, **(3.1)** (0.175 g, 0.58 mmol, 1.0 eq.), NMP (1.25 mL) and (*S*)-(-)-3-amino-1,2-propanediol (0.755 g, 8.12 mmol, 14.0 eq.). The vial was sealed and then put on heating block, 18 h at 180 °C. After cooling, the mixture was poured in a funnel with NaHCO₃ sat. (15 mL), aqueous layer was extracted twice with EtOAc (2x15 mL) and combined organics layers were washed with brine, dried over Na₂SO₄, filtered, concentrated and purified by flash chromatography with DCM/MeOH (95/5) as eluent to give (7) (0.087 g, 42 %) as a white solid.

### Example 4.8: Synthesis of N6-[2-[2-(2-aminoethoxy)ethoxy]ethyl]-N8-benzyl-3-isopropyl-[1,2,4]triazolo[4,3-b]pyridazine-6,8-diamine (8)

In a vial 2 - 5 mL with a stir bar was charged **(3.1)** (0.250 g, 0.83 mmol, 1.0 eq.), NMP (1.25 mL) and 2-[2-(2-aminoethoxy)ethoxy]ethanamine (0.97 mL, 6.63 mmol, 8.0 eq.). The vial was closed and put under microwave irradiation 4h at 180 °C. After cooling, the mixture was diluted in EtOAc (20 mL), organic layer was washed with water (40 mL). Aqueous Layer was extracted with EtOAc (20 mL) and organics layer were combined, dried over Na₂SO₄, filtered, concentrated and purified by flash chromatography with DCM/MeOH (97/3 then 86/14) as eluent to give **(8)** (0.125 g, 36 %) as a white crystalline solid.

### Example 4.9: Synthesis of 3-[[8-(benzylamino)-3-isopropyl-[1,2,4]triazolo[4,3-b]pyridazin-6-yl]amino]propan-1-ol (9)

In a sealed tube 2 - 5 mL with a stir bar was charged **(3.1)** (0.200 g, 0.66 mmol, 1.0 eq.), NMP (0.3 mL) and corresponding amine (0.72 mL, 9.28 mmol, 14.0 eq.). The vial was sealed and then put on heating block, 17 h at 180 °C. After cooling, the mixture was diluted in EtOAc (20.0 mL) and organic layer was washed with a mixture brine/water (2/1, 3x20.0 mL). Organic layer was dried over Na₂SO₄, filtered, concentrated and purified by flash chromatography with DCM/MeOH (96/4) as eluent to give **(9)** (0.072 g, 32 %) as a white solid.

### Example 4.10: Synthesis of N8-benzyl-3-isopropyl-N6-(3-methoxypropyl)-[1,2,4]triazolo[4,3-b]pyridazine-6,8-diamine (10)

In a sealed tube 2 - 5 mL with a stir bar was charged, **(3.1)** (0.500 g, 1.66 mmol, 1.0 eq.), NMP (1.3 mL) and 3-methoxypropylamine (2.40 mL, 23.20 mmol, 14.0 eq.). The vial was sealed and then put on heating block, 4 h at 180 °C. After cooling, the mixture was diluted in EtOAc (40 mL) and organic layer was washed three times with a mixture brine/water (2/1, 3x50 mL). Organic layer was dried over Na₂SO₄, filtered, concentrated and purified by flash chromatography with DCM/MeOH (97/3) as eluent to give **(10)** (0.460 g, 78 %) as a white solid.

### Example 4.11: Synthesis of 2-[[8-(benzylamino)-3-isopropyl-[1,2,4]triazolo[4,3-b]pyridazin-6-yl]amino]ethanol (11)

In a sealed tube 2 - 5 mL with a stir bar was charged, **(3.1)** (0.200 g, 0.66 mmol, 1.0 eq.), NMP (0.6 mL) and ethanolamine (0.57 mL, 9.28 mmol, 14.0 eq.). The vial was sealed and then put on heating block, 16 h at 180 °C. After cooling, the mixture was diluted in EtOAc (20 mL) and organic layer was washed three times with a mixture brine/water (2/1, 3x20 mL). Organic layer was dried over Na₂SO₄, filtered, concentrated and purified by flash chromatography with DCM/MeOH (96/4) as eluent to give **(11)** (0.082 g, 38 %) as a white solid.

### Example 4.12: Synthesis of N8-benzyl-3-isopropyl-N6-(2-methoxyethyl)-[1,2,4]triazolo[4,3-b]pyridazine-6,8-diamine (12)

In a sealed tube 2 - 5 mL with a stir bar was charged, **(3.1)** (0.200 g, 0.66 mmol, 1.0 eq.), NMP (0.6 mL) and 2-methoxyethanamine (0.81 mL, 9.28 mmol, 14.0 eq.). The vial was sealed and then put on heating block, 16 h at 180 °C. After cooling, the mixture was diluted in EtOAc (20 mL) and organic layer was washed three times with a mixture brine/water (2/1, 3x20 mL). Organic layer was dried over Na₂SO₄, filtered, concentrated and purified by flash chromatography with DCM/MeOH (95/5) as eluent to give **(12)** (0.152 g, 67 %) as a white crystalline solid.

### Example 4.13: Synthesis of 3-isopropyl-N6,N8-bis(2-methoxyethyl)-[1,2,4]triazolo[4,3-b]pyridazine-6,8-diamine (112)

In a sealed tube 2 - 5 mL with a stir bar was charged, **(3.1)** (0.200 g, 0.66 mmol, 1.0 eq.), NMP (0.6 mL) and 2-methoxyethanamine (0.81 mL, 9.28 mmol, 14.0 eq.). The vial was sealed and then put on heating block, 16 h at 180 °C. After cooling, the mixture was diluted in EtOAc (20 mL) and organic layer was washed three times with a mixture brine/water (2/1, 3x20 mL). Organic layer was dried over Na₂SO₄, filtered, concentrated and purified by flash chromatography with DCM/MeOH (95/5) as eluent to give the secondary compound **(112)** (0.052 g, 25 %) as a beige solid.

### Example 4.14: Synthesis of 4-[[8-(benzylamino)-3-isopropyl-[1,2,4]triazolo[4,3-b]pyridazin-6-yl]amino]butan-1-ol (13)

In a sealed tube 2 - 5 mL with a stir bar was charged **(3.1)** (0.400 g, 1.33 mmol, 1.0 eq.), NMP (1.2 mL) and corresponding amine (1.76 mL, 18.56 mmol, 14.0 eq.). The vial was sealed and then put on heating block, 16 h at 180 °C. After cooling, the mixture was diluted in EtOAc (40.0 mL) and organic layer was washed with a mixture brine/water (2/1, 3x50 mL). Organic layer was dried over Na₂SO₄, filtered, concentrated and purified by flash chromatography with DCM/MeOH (96/4) as eluent to give **(13)** (0.220 g, 47 %) as a white solid.

### Example 4.15: Synthesis of N8-benzyl-3-isopropyl-N6-(4-methoxybutyl)-[1,2,4]triazolo[4,3-b]pyridazine-6,8-diamine (14)

In a sealed tube 2 - 5 mL with a stir bar was charged **(3.1)** (0.100 g, 0.33 mmol, 1.0 eq.), NMP (1.0 mL) and corresponding amine (0.249 g, 2.65 mmol, 8.0 eq.) then the vial was sealed and put on heating block 17 h at 170 °C. After cooling, the mixture was diluted in EtOAc (20.0 mL) and organic layer was washed three times with a mixture brine/water (3/1, 3x10 mL), dried over MgSO₄, filtered, concentrated and purified by flash chromatography with DCM/MeOH (99/1 to 95/5) as eluent to give **(14)** (0.096 g, 79 %) as a white crystalline solid.

### Example 4.16: Synthesis of 5-[[8-(benzylamino)-3-isopropyl-[1,2,4]triazolo[4,3-b]pyridazin-6-yl]amino]pentan-1-ol (15)

In a sealed tube 2 - 5 mL with a stir bar was charged **(3.1)** (0.400 g, 1.33 mmol, 1.0 eq.), NMP (1.0 mL) and corresponding amine (2.24 mL, 18.56 mmol, 14.0 eq.). The vial was sealed and then put on heating block, 16 h at 180 °C. After cooling, the mixture was diluted in EtOAc (40.0 mL) and organic layer was washed with a mixture brine/HCl 0.05M (2/1, 3x40 mL). Organic layer was dried over Na₂SO₄, filtered, concentrated and purified by flash chromatography with DCM/MeOH (95/5) as eluent to give **(15)** (0.250 g, 51 %) as a white solid.

### Example 4.17: Synthesis of N8-benzyl-3-isopropyl-N6-(5-methoxypentyl)-[1,2,4]triazolo[4,3-b]pyridazine-6,8-diamine (16)

In a sealed tube 2 - 5 mL with a stir bar was charged **(3.1)** (0.100 g, 0.33 mmol, 1.0 eq.), NMP (1.0 mL) and corresponding amine (0.327 g, 2.65 mmol, 8.0 eq.) then the vial was sealed and put on heating block 17 h at 170 °C. After cooling, the mixture was diluted in EtOAc (20.0 mL) and organic layer was washed three times with a mixture brine/water (3/1, 3x10 mL), dried over MgSO₄, filtered, concentrated and purified by flash chromatography with DCM/MeOH (99/1 to 95/5) as eluent to give **(16)** (0.100 g, 79 %) as a light yellow solid.

### Example 4.18: Synthesis of N8-benzyl-3-isopropyl-N6-(3-methylsulfanylpropyl)-[1,2,4]triazolo[4,3-b]pyridazine-6,8-diamine (17)

In a sealed tube 2 - 5 mL with a stir bar was charged, **(3.1)** (0.250 g, 1.30 mmol, 1.0 eq.), NMP (0.75 mL) and 3-methylsulfanylpropan-1-amine (1.46 mL, 13.02 mmol, 10.0 eq.).

The vial was sealed and then put on heating block, 2 h at 180 °C. After cooling, the mixture was diluted in EtOAc (30 mL) and organic layer was washed three times with a mixture brine/water (2/1, 3x30 mL). Organic layer was dried over Na₂SO₄, filtered, concentrated and purified by flash chromatography with DCM/MeOH (96/4) as eluent to give **(17)** (0.270 g, 88 %) as a beige solid.

### Example 4.19: Synthesis of N8-benzyl-N6-(1-ethylpropyl)-3-isopropyl-[1,2,4]triazolo[4,3-b]pyridazine-6,8-diamine (18)

In a 2 - 5 mL vial with a stir bar was charged, **(3.1)** (0.250 g, 0.83 mmol, 1.0 eq.), NMP (0.8 mL) and pentan-3-amine (0.88 mL, 7.46 mmol, 9.0 eq.). The vial was sealed and then put on heating block, 24 h at 180 °C. After cooling, the mixture was diluted in EtOAc (20 mL) and organic layer was washed three times with a mixture brine/water (2/1, 3x20 mL). Organic layer was dried over Na₂SO₄, filtered, concentrated and purified by flash chromatography with DCM/MeOH (97/3) as eluent. Fractions containing NMP traces were concentrated, diluted in EtOAc (20 mL), washed three times with water (3x25 mL) and organic layer was dried over Na₂SO₄, filtered, concentrated to give **(18)** (0.096 g, 33 %) as a white crystalline solid.

### Example 4.20: Synthesis of N8-benzyl-3-isopropyl-N6-[(3R)-tetrahydrofuran-3-yl]-[1,2,4]triazolo[4,3-b]pyridazine-6,8-diamine (19)

In a 2 - 5 mL vial with a stir bar was charged, **(3.1)** (0.200 g, 0.66 mmol, 1.0 eq.), NMP (0.6 mL) and corresponding amine (0.84 mL, 9.28 mmol, 14.0 eq.). The vial was sealed and then put on heating block, 24 h at 180 °C. After cooling, the mixture was diluted in EtOAc (30.0 mL) and organic layer was washed with a mixture brine/water (2/1, 3x20 mL). Organic layer was dried over Na₂SO₄, filtered, concentrated and purified by flash chromatography with DCM/MeOH (96/4) as eluent to give **(19)** (0.149 g, 64 %) as a white solid.

### Example 4.21: Synthesis of N8-benzyl-3-cyclopentyl-N6-[(3R)-tetrahydrofuran-3-yl]-[1,2,4]triazolo[4,3-b]pyridazine-6,8-diamine (20)

In a sealed tube 2 - 5 mL with a stir bar was charged, **(3.4)** (0.160 g, 0.49 mmol, 1.0 eq.), NMP (0.8 mL) and corresponding amine (0.358 g, 3.91 mmol, 8.0 eq.). The vial was sealed and then put on heating block 23 h at 170 °C. After cooling, water (7.0 mL) is added and stirred 5 min, the resulting dough ball was filtered, washed with water then solubilise with DCM. Organic filtrate was dried over MgSO₄, filtered, concentrated and purified by flash chromatography with DCM/MeOH (98/2 to 94/6) as eluent to give **(20)** (0.071 g, 38 %) as a white solid.

### Example 4.22: Synthesis of N8-benzyl-3-isopropyl-N6-[(3R)-tetrahydropyran-3-yl]-[1,2,4]triazolo[4,3-b]pyridazine-6,8-diamine (21)

In a sealed tube 2 - 5 mL with a stir bar was charged **(3.1)** (0.420 g, 1.24 mmol, 1.0 eq.), dioxane (1.2 mL) then benzylamine (0.83 mL, 7.42 mmol, 6.0 eq.). The vial was sealed and then put on heating block, 4h at 110 °C. After cooling, the mixture was diluted in DCM (20 mL) and the resulting organic layer was washed with HCl 0.5 M (20 mL). Aqueous layer was extracted twice with DCM (2x10 mL) and organic layers were combined, dried over MgSO₄, filtered, concentrated and purified by flash chromatography with DCM/MeOH (97/3 to 95/5) as eluent to give **(21)** (0.175 g, 39 %) as a white solid.

### Example 4.23: Synthesis of N8-benzyl-3-isopropyl-N6-[(3S)-tetrahydropyran-3-yl]-[1,2,4]triazolo[4,3-b]pyridazine-6,8-diamine (22)

In a sealed tube 2 - 5 mL with a stir bar was charged, **(3.1)** (0.250 g, 0.83 mmol, 1.0 eq.) and NMP (3.5 mL) then (*3S*)-tetrahydropyran-3-amine hydrochloride (1.080 g, 7.46 mmol, 9.0 eq.) and K₂CO₃ (0.555 g, 3.98 mmol, 4.8 eq.). The vial was sealed and then put on heating block, 15 h at 180 °C. After cooling, the mixture was poured onto water (50 mL), stirring 5min vigorously and the resulting precipitate was filtered off, washed with water (up to neutral pH) then EtOAc and the organic filtrate was dried over MgSO₄, filtered, concentrated and purified by flash chromatography with DCM/MeOH (97/3 to 95/5) as eluent to give **(22)** (0.040 g, 13 %) as a white crystalline solid.

### Example 4.24: Synthesis of N8-benzyl-3-isopropyl-N6-tetrahydropyran-4-yl-[1,2,4]triazolo[4,3-b]pyridazine-6,8-diamine (23)

In a sealed tube 2 - 5 mL with a stir bar was charged **(3.1)** (0.250 g, 0.83 mmol, 1.0 eq.) and corresponding amine (1.29 mL, 12.43 mmol, 15.0 eq.) then the vial was sealed and then put on heating block, 5.5 h at 180 °C. After cooling, the mixture was diluted in EtOAc (3.0 mL), water (3.0 mL) was added and the biphasique mixture was stirred 5 min. Layers were separated, aqueous layer was extracted twice with EtOAc (2x3.0 mL), organics layers were combined, dried over MgSO₄, filtered, concentrated and purified by flash chromatography with DCM/MeOH (94/6) as eluent to give **(23)** (0.085 g, 28 %) as a beige solid.

### Example 4.25: Synthesis of N-[3-[[8-(benzylamino)-3-isopropyl-[1,2,4]triazolo[4,3-b]pyridazin-6-yl]amino]propyl]acetamide (24)

In a sealed tube 2 - 5 mL with a stir bar was charged **(3.1)** (0.100 g, 0.33 mmol, 1.0 eq.), NMP (0.2 mL) then corresponding amine (0.405 g, 3.31 mmol, 10.0 eq.). The vial was sealed and then put on heating block, 15 h at 180 °C. After cooling, the mixture was diluted in EtOAc (20.0 mL) then organic layer was washed with brine/water (2/1, 3x20.0 mL), dried over Na₂SO₄, filtered, concentrated and purified by flash chromatography with DCM/MeOH (96/4) as eluent to give **(24)** (0.118g, 94 %) as a white solid.

### Example 4.26: Synthesis of 3-[[8-(benzylamino)-3-isopropyl-[1,2,4]triazolo[4,3-b]pyridazin-6-yl]amino]propanoic acid (4.26)

In a sealed tube 10 - 20 mL with a stir bar was charged, **(3.1)** (0.800 g, 2.65 mmol, 1.0 eq.) and DMSO (6.5 mL) then β-alanine (1.431 g, 15.91 mmol, 6.0 eq.) and K₃PO₄ (3.376 g, 15.91 mmol, 6.0 eq.). The vial was sealed and then put on heating block, 4 h at 180 °C. After cooling, the mixture was poured into HC11M (150 mL) in ice bath, and water (40 mL). After 20 min, the precipitate was filtered, washed with water (to pH 7) then the solid was taken off with EtOH and acetone, concentrated and purified by flash chromatography with DCM/MeOH (98/2 to 92/8 then 92/8) as eluent to give **(4.26)** (0.505 g, 54 %) as a beige solid.
**Rf** (DCM/MeOH, 92/8) : 0.30

**¹H NMR (400 MHz, DMSO-*d₆*) δ** : 1.36 (d, *J* = 7.0 Hz, 6H, 2xCH_{3 iPr}), 2.51 (m, 2H, CH₂₋co), 3.34 (d, *J* = 9.7 Hz, 3H, H_{iPr} & CH_{2-NH}), 4.42 (d, *J* = 6.2 Hz, 2H, CH_{2 Bn}), 5.39 (s, 1H, H_{Ar}), 6.64 (t, *J* = 5.4 Hz, 1H, NH), 7.17 - 7.43 (m, 5H, 5×H_{Ar}), 7.93 (t, *J* = 6.4 Hz, 1H, NH_{Bn}), 12.16 (s, 1H, COOH).

**¹³C NMR (101 MHz, DMSO-*d₆*) δ** : 19.6 (2xCH_{3 iPr}), 24.7 (CH_{iPr}), 33.3 (CH_{2-CO}), 36.9 (CH_{2-NH}), 45.2 (CH_{2 Bn}), 84.1 (CH_{Ar}), 126.8 (2xCH_{Ar}), 126.9 (CH_{Ar}), 128.4 (2xCH_{Ar}), 138.3 (C_{q}), 139.7 (C_{q}), 139.9 (C_{q}), 152.9 (C_{q}), 155.7 (C_{q}), 173.2 (C_{q}).

**MS (ESI+) : m/z calcd for C₁₈H₂₂N₆O₂ :** 355.19 [M+H]+, **found** : 355.27.

### Example 4.27: Synthesis of 3-[[8-(benzylamino)-3-isopropyl-[1,2,4]triazolo[4,3-b]pyridazin-6-yl]amino]propanamide (25)

To a solution of **(4.26)** (0.110 g, 0.31 mmol, 1.0 eq.) in THF (3.1 mL) was added HOBt.H₂O (0.062 g, 0.40 mmol, 1.3 eq.), EDCi (0.058 g, 0.37 mmol, 1.2 eq.) and ammonia 0.5M in THF (0.81 mL, 0.40 mmol, 1.3 eq.). The mixture was stirred 4 h at room temperature and after completion the mixture was directly concentrated and purified on solid deposit by flash chromatography with DCM/MeOH (94/6) as eluent to give **(25)** (0.090 g, 82 %) as a white solid.

### Example 4.28: Synthesis of 3-[[8-(benzylamino)-3-isopropyl-[1,2,4]triazolo[4,3-b]pyridazin-6-yl]amino]-N-methyl-propanamide (26)

To a solution of **(4.26)** (0.120 g, 0.34 mmol, 1.0 eq.) in THF (3 mL) was added HOBt.H₂O (0.069 g, 0.44 mmol, 1.3 eq.), methylamine 2M in THF (0.24 mL, 0.47 mmol, 1.4 eq.) and EDCi (0.065 g, 0.41 mmol, 1.2 eq.). The mixture was stirred 3 h at room temperature and after completion the mixture was directly concentrated and purified on solid deposit by flash chromatography with DCM/MeOH (95/5) as eluent to give **(26)** (0.121 g, 97 %) as a white solid.

### Example 4.29: Synthesis of 3-[[8-(benzylamino)-3-isopropyl-[1,2,4]triazolo[4,3-b]pyridazin-6-yl]amino]-N,N-dimethyl-propanamide (27)

To a solution of **(4.26)** (0.120 g, 0.34 mmol, 1.0 eq.) in THF (3 mL) was added HOBt.H₂O (0.069 g, 0.44 mmol, 1.3 eq.), dimethylamine 2M in THF (0.24 mL, 0.47 mmol, 1.4 eq.) and EDCi (0.065 g, 0.41 mmol, 1.2 eq.). The mixture was stirred 3 h at room temperature and after completion the mixture was directly concentrated and purified on solid deposit by flash chromatography with DCM/MeOH (95/5) as eluent to give **(27)** (0.124 g, 96 %) as a white solid.

### Example 4.30: Synthesis of methyl 3-[[8-(benzylamino)-3-isopropyl-[1,2,4]triazolo[4,3-b]pyridazin-6-yl]amino]propanoate (28)

A solution of **(4.26)** (0.110 g, 2.65 mmol, 1.0 eq.) in MeOH (10 mL) with two drops of H₂SO₄ was refluxed 30 min. After cooling, Na₂CO₃ (0.075 g) was added and stirred 5 min at room temperature. The mixture was directly concentrated and purified by flash chromatography with DCM/MeOH (95/5) as eluent to give **(28)** (0.100 g, 87 %) as a white solid.

### Example 4.31: Synthesis of ethyl 3-[[8-(benzylamino)-3-isopropyl-[1,2,4]triazolo[4,3-b]pyridazin-6-yl]amino]propanoate (29)

A solution of **(4.26)** (0.110 g, 2.65 mmol, 1.0 eq.) in EtOH (10 mL) with two drops of H₂SO₄ was refluxed 30 min. After cooling, Na₂CO₃ (0.075 g) was added and stirred 5 min at room temperature. The mixture was directly concentrated and purified by flash chromatography with DCM/MeOH (94/6) as eluent to give **(29)** (0.082 g, 69 %) as a white solid.

### Example 4.32: Synthesis of (2R)-2-[[8-(benzylamino)-3-cyclopropyl-[1,2,4]triazolo[4,3-b]pyridazin-6-yl]amino]butan-1-ol (30)

In a sealed tube 2 - 5 mL with a stir bar was charged, **(3.3)** (0.135 g, 0.45 mmol, 1.0 eq.), NMP (1.0 mL) and (*R*)-(-)-2-amino-1-butanol (0.57 mL, 7.21 mmol, 16.0 eq.). The vial was sealed and then put on heating block, 18 h at 180 °C. After cooling, the mixture was poured in EtOAc (20 mL) then organic layer was washed three times with brine (20 mL). The organic layer was dried over Na₂SO₄, filtered, concentrated and purified by flash chromatography with DCM/MeOH (96/4) as eluent to give **(30)** (0.112 g, 71 %) as a beige solid.

### Example 4.33: Synthesis of N8-benzyl-3-cyclopropyl-N6-(3-methoxypropyl)-[1,2,4]triazolo[4,3-b]pyridazine-6,8-diamine (31)

In a sealed tube 2 - 5 mL with a stir bar was charged, **(3.3)** (0.250 g, 0.83 mmol, 1.0 eq.), NMP (0.8 mL) and 3-methoxypropylamine (1.21 mL, 11.68 mmol, 14.0 eq.). The vial was sealed and then put on heating block, 18 h at 180 °C. After cooling, the mixture was diluted in EtOAc (30 mL) and organic layer was washed three times with a mixture brine/water (2/1, 3x20 mL). Organic layer was dried over Na₂SO₄, filtered, concentrated and purified by flash chromatography with DCM/MeOH (97/3) as eluent to give **(31)** (0.145 g, 49 %) as a white solid.

### Example 4.34: Synthesis of 3-cyclopropyl-N6,N8-bis(3-methoxypropyl)-[1,2,4]triazolo[4,3-b]pyridazine-6,8-diamine (110)

In a sealed tube 2 - 5 mL with a stir bar was charged, **(3.3)** (0.250 g, 0.83 mmol, 1.0 eq.), NMP (0.8 mL) and 3-methoxypropylamine (1.21 mL, 11.68 mmol, 14.0 eq.). The vial was sealed and then put on heating block, 18 h at 180 °C. After cooling, the mixture was diluted in EtOAc (30 mL) and organic layer was washed three times with a mixture brine/water (2/1, 3x20 mL). Organic layer was dried over Na₂SO₄, filtered, concentrated and purified by flash chromatography with DCM/MeOH (97/3) as eluent to give the compound **(110)** (0.120 g, 43 %) as a white solid.

### Example 4.35: Synthesis of N8-benzyl-N6-(3-methoxypropyl)-3-methyl-[1,2,4]triazolo[4,3-b]pyridazine-6,8-diamine (32)

In a sealed tube 2 - 5 mL with a stir bar was charged **(3.6)** (0.125 g, 0.46 mmol, 1.0 eq.), NMP (0.4 mL) and 3-methoxypropylamine (0.65 mL, 6.39 mmol, 14.0 eq.). The vial was sealed and then put on heating block, 16 h at 180 °C. After cooling, the mixture was diluted in EtOAc (20.0 mL), organic layer was washed with brine (3x10.0 mL), dired over Na₂SO₄, filtered, concentrated and purified by flash chromatography with DCM/MeOH (95/5) as eluent to give **(32)** (0.086 g, 58 %) as a white solid.

### Example 4.36: Synthesis of (2R)-2-[[8-(benzylamino)-3-methyl-[1,2,4]triazolo[4,3-b]pyridazin-6-yl]amino]butan-1-ol (33)

In a sealed tube 2-5mL with a stir bar was charged **(3.6)** (0.125 g, 0.46 mmol, 1.0 eq.), NMP (0.4 mL) and (*R*)-(-)-2-amino-1-butanol (0.51 mL, 5.48 mmol, 12.0 eq.). The vial was sealed and then put on heating block, 16 h at 180 °C. After cooling, the mixture was diluted in EtOAc (20.0 mL), organic layer was washed with brine (3x10.0 mL), dired over Na₂SO₄, filtered, concentrated and purified by flash chromatography with DCM/MeOH (95/5) as eluent to give **(33)** (0.086 g, 58 %) as a white solid.

### Example 4.37: Synthesis of N8-benzyl-N6-(3-methoxypropyl)-3-phenyl-[1,2,4]triazolo[4,3-b]pyridazine-6,8-diamine (34)

In a sealed tube 2 - 5 mL with a stir bar was charged **(3.7)** (0.125 g, 0.38 mmol, 1.0 eq.), NMP (0.35 mL) and 3-methoxypropylamine (0.55 mL, 5.34 mmol, 14.0 eq.). The vial was sealed and then put on heating block, 18 h at 180 °C. After cooling, the mixture was diluted in EtOAc (25.0 mL), organic layer was washed with brine/water (2/1, 3x20.0 mL), dired over Na₂SO₄, filtered, concentrated and purified by flash chromatography with DCM/MeOH (95/5) as eluent to give **(34)** (0.086 g, 58 %) as a white solid.

### Example 4.38: Synthesis of N6,N8-bis(3-methoxypropyl)-3-phenyl-[1,2,4]triazolo[4,3-b]pyridazine-6,8-diamine (109)

In a sealed tube 2 - 5 mL with a stir bar was charged **(3.7)** (0.125 g, 0.38 mmol, 1.0 eq.), NMP (0.35 mL) and 3-methoxypropylamine (0.55 mL, 5.34 mmol, 14.0 eq.). The vial was sealed and then put on heating block, 18 h at 180 °C. After cooling, the mixture was diluted in EtOAc (25.0 mL), organic layer was washed with brine/water (2/1, 3x20.0 mL), dired over Na₂SO₄, filtered, concentrated and purified by flash chromatography with DCM/MeOH (95/5) as eluent to give the secondary compound **(109)** (0.055 g, 39 %) as a white solid.

### Example 4.39: Synthesis of (2R)-2-[[8-(benzylamino)-3-phenyl-[1,2,4]triazolo[4,3-b]pyridazin-6-yl]amino]butan-1-ol (35)

In a sealed tube 2 - 5 mL with a stir bar was charged **(3.7)** (0.150 g, 0.46 mmol, 1.0 eq.), NMP (1.0 mL) and (*R*)-(-)-2-amino-1-butanol (0.59 mL, 6.41 mmol, 14.0 eq.). The vial was sealed and then put on heating block, 18 h at 180 °C. After cooling, the mixture was diluted in EtOAc (25.0 mL), organic layer was washed with brine/water (2/1, 3x20.0 mL), dired over Na₂SO₄, filtered, concentrated and purified by flash chromatography with DCM/MeOH (95/5) as eluent to give **(35)** (0.115 g, 65 %) as a white solid.

### Example 4.40: Synthesis of N8-benzyl-N6-(3-methoxypropyl)-3-(trifluoromethyl)-[1,2,4]triazolo[4,3-b]pyridazine-6,8-diamine (36)

In a sealed tube 2 - 5 mL with a stir bar was charged **(3.5)** (0.125 g, 0.38 mmol, 1.0 eq.), NMP (0.4 mL) and 3-methoxypropylamine (0.55 mL, 5.34 mmol, 14.0 eq.). The vial was sealed and then put on heating block, 18 h at 180 °C. After cooling, the mixture was diluted in EtOAc (20.0 mL) and organic layer was washed three times with a mixture brine/water (2/1, 3x20 mL). Organic layer was dried over Na₂SO₄, filtered, concentrated and purified by flash chromatography with DCM/MeOH (99/1) as eluent to give **(36)** (0.110 g, 76 %) as a white solid.

### Example 4.41: Synthesis of N6,N8-bis(3-methoxypropyl)-3-(trifluoromethyl)-[1,2,4]triazolo[4,3-b]pyridazine-6,8-diamine (111)

In a sealed tube 2 - 5 mL with a stir bar was charged **(3.5)** (0.125 g, 0.38 mmol, 1.0 eq.), NMP (0.4 mL) and 3-methoxypropylamine (0.55 mL, 5.34 mmol, 14.0 eq.). The vial was sealed and then put on heating block, 18 h at 180 °C. After cooling, the mixture was diluted in EtOAc (20.0 mL) and organic layer was washed three times with a mixture brine/water (2/1, 3x20 mL). Organic layer was dried over Na₂SO₄, filtered, concentrated and purified by flash chromatography with DCM/MeOH (99/1) as eluent to give the secondary compound **(111)** (0.010 g, 7 %) as a white solid.

### Example 4.42: Synthesis of (2R)-2-[[8-(benzylamino)-3-(trifluoromethyl)-[1,2,4]triazolo[4,3-b]pyridazin-6-yl]amino]butan-1-ol (37)

In a sealed tube 2 - 5 mL with a stir bar was charged **(3.5)** (0.110 g, 0.34 mmol, 1.0 eq.), NMP (0.3 mL) and (*R*)-(-)-2-amino-1-butanol (0.44 mL, 4.70 mmol, 14.0 eq.). The vial was sealed and then put on heating block, 18 h at 180 °C. After cooling, the mixture was diluted in EtOAc (20.0 mL) and organic layer was washed three times with a mixture brine/water (2/1, 3x20 mL). Organic layer was dried over Na₂SO₄, filtered, concentrated and purified by flash chromatography with DCM/MeOH (97/3) as eluent to give **(37)** (0.070 g, 55 %) as a white solid.

### Example 4.43: Synthesis of (2R)-2-[[8-(benzylamino)-3-isopropyl-7-methyl-[1,2,4]triazolo[4,3-b]pyridazin-6-yl]amino]butan-1-ol (115)

Under inert gas, in a solution of **(1)** (0.880 g, 2.48 mmol, 1.0 eq.) and dry THF (48.0 mL), NaH 60% (0.119 g, 2.98 mmol, 1.2 eq.) was added portionwise then the mixture was stirred 10 min at room temperature. The solution was cooled at 0 °C and MeI (0.17 mL, 2.73 mmol, 1.1 eq.) was added dropwise then stirred 2 h at room temperature. To complete the reaction, MeI (0.03 mL, 0.50 mmol, 0.2 eq.) was added and the mixture was stirred 1 h. After completion, MeOH (9.0 mL) was added slowly and the mixture was directly concentrated with SiO₂ to make solid deposit and purified by flash chromatography with DCM/MeOH (98/2 to 96/4) as eluent to give the secondary compound **(115)** (0.092 g, 10 %) as a beige solid.

### Example 4.44: Synthesis of 2-[[[6-[[(1R)-1-(hydroxymethyl)propyl]amino]-3-isopropyl-[1,2,4]triazolo[4,3-b]pyridazin-8-yl]amino]methyl]phenol (38)

In a sealed tube 2 - 5 mL with a stir bar was charged with **(3.8)** (0.150 g, 0.47 mmol, 1.0 eq.) and (*R*)-(-)-2-amino-1-butanol (0.91 mL, 9.44 mmol, 20.0 eq.) then the vial was sealed and put on heating block, 15 h at 160 °C. After cooling, the mixture was diluted in EtOAc (25.0 mL) and organic layer was washed twice with water/brine mixture (1/3, 2x30.0 mL) then dried over MgSO₄, filtered, concentrated and purified by flash chromatography with DCM/MeOH (96/4 to 92/8) as eluent to give the title compound **(38)** (0.010 g, 6 %) as a beige solid.

### Example 4.45: Synthesis of 2-[[[3-isopropyl-6-(3-methoxypropylamino)-[1,2,4]triazolo[4,3-b]pyridazin-8-yl]amino]methyl]phenol (39)

In a sealed tube 2 - 5 mL with a stir bar was charged with **(3.8)** (0.150 g, 0.47 mmol, 1.0 eq.) and 3-methoxypropylamine (1.07 mL, 9.44 mmol, 20.0 eq.) then the vial was sealed and put on heating block, 19 h at 150 °C. After cooling, the mixture was diluted in EtOAc (25.0 mL) and organic layer was washed twice with water/brine mixture (1/3, 2x30.0 mL) then dried over MgSO₄, filtered, concentrated and purified by flash chromatography with DCM/MeOH (93/7 to 92/8) as eluent to give the title compound **(39)** (0.070 g, 40 %) as a white solid.

### Example 4.46: Synthesis of 2-[[[6-(1-ethylpropylamino)-3-isopropyl-[1,2,4]triazolo[4,3-b]pyridazin-8-yl]amino]methyl]phenol (40)

In a sealed tube 2 - 5 mL with a stir bar was charged **(3.8)** (0.140 g, 0.44 mmol, 1.0 eq.), NMP (1.0 mL) and 3-aminopentane (0.52 mL, 4.41 mmol, 10.0 eq.). The vial was sealed and then put on heating block, 16 h at 170 °C. After cooling, the mixture was diluted in EtOAc (20.0 mL) and organic layer was washed three times with a mixture brine/water (2/1, 3x20 mL). Organic layer was dried over Na₂SO₄, filtered, concentrated and purified by flash chromatography with DCM/MeOH (98/2 to 95/5) as eluent to give **(40)** (0.021 g, 13 %) as a white crystalline solid.

### Example 4.47: Synthesis of 3-[[[6-(1-ethylpropylamino)-3-isopropyl-[1,2,4]triazolo[4,3-b]pyridazin-8-yl]amino]methyl]phenol (41)

In a sealed tube 2 - 5 mL with a stir bar was charged (**3.9**) (0.450 g, 1.42 mmol, 1.0 eq.), NMP (3.3 mL) and 3-aminopentane (3.33 mL, 28.32 mmol, 20.0 eq.). The vial was sealed and then put on heating block, 16 h at 170 °C. After cooling, the mixture was poured onto saturated NaHCO₃ (20.0 mL) then water (30.0 mL) was added and the mixture was stirred 5 min. The resulting solid was filtered off, washed wih water up to neutral pH then the solid was taken up in DCM, organic filtrate was dried over MgSO₄, filtered, concentrated and purified by flash chromatography with DCM/MeOH (98/2 to 94/6) as eluent to give **(41)** (0.080 g, 15 %) as a white solid.

### Example 4.48: Synthesis of 4-[[[6-(1-ethylpropylamino)-3-isopropyl-[1,2,4]triazolo[4,3-b]pyridazin-8-yl]amino]methyl]phenol (42)

In a sealed tube 2 - 5 mL with a stir bar was charged **(3.10)** (0.085 g, 0.27 mmol, 1.0 eq.), NMP (1.0 mL) and 3-aminopentane (0.31 mL, 2.68 mmol, 10.0 eq.). The vial was sealed and then put on heating block, 16 h at 170 °C. After cooling, the mixture was diluted in EtOAc (20.0 mL) and organic layer was washed three times with a mixture brine/water (2/1, 3x10.0 mL). Organic layer was dried over Na₂SO₄, filtered, concentrated and purified by flash chromatography with DCM/MeOH (98/2 to 95/5) as eluent to give **(42)** (0.015 g, 15 %) as a white crystalline solid.

### Example 4.49: Synthesis of 2-[[[3-isopropyl-6-[[(3R)-tetrahydropyran-3-yl]amino]-[1,2,4]triazolo[4,3-b]pyridazin-8-yl]amino]methyl]phenol (43)

In a sealed tube 2 - 5 mL with a stir bar was charged **(3.8)** (0.200 g, 0.63 mmol, 1.0 eq.), corresponding amine hydrochloride (0.912 g, 6.29 mmol, 10.0 eq.) and DIPEA (1.54 mL, 8.81 mmol, 14.0 eq.) then the vial was sealed and put on heating block 24 h at 150 °C. After cooling, water (5.0 mL) was added, the precipitate was triturated then the mixture was poured in water (15.0 mL), stirred 5 min and the precipitate was filtered off, washed with water up to neutral pH and taken up in EtOAc. Organic filtrate was dried over MgSO₄, filtered, concentrated and purified by flash chromatography with DCM/MeOH (96/4 to 94/6) as eluent to give **(43)** (0.018 g, 7 %) as a white solid.

### Example 4.50: Synthesis of 2-[[[3-isopropyl-6-(tetrahydropyran-4-ylamino)-[1,2,4]triazolo[4,3-b]pyridazin-8-yl]amino]methyl]phenol (44)

In a sealed tube 2 - 5 mL with a stir bar was charged **(3.8)** (0.250 g, 0.79 mmol, 1.0 eq.) and corresponding amine (0.98 mL, 9.44 mmol, 12.0 eq.) then the vial was sealed and put on heating block 15 h at 160 °C. After cooling, EtOAc (4.0 mL) and water (4.0 mL) was added, stirred 5 min then layers were separated. Aqueous layer was extracted twice with EtOAc (2x3.0 mL) then organics layers were combined, dried over MgSO₄, filtered, concentrated and purified by flash chromatography with DCM/MeOH (96/4 to 93/7) as eluent to give **(44)** (0.030 g, 10 %) as a white solid.

### Example 4.51: Synthesis of (2S)-2-[[8-[(4-bromophenyl)methylamino]-3-isopropyl-[1,2,4]triazolo[4,3-b]pyridazin-6-yl]amino]butan-1-o1 (101)

In a sealed tube 10 - 20 mL with a stir bar was charged **(3.42)** (1.800 g, 4.73 mmol, 1.0 eq.), NMP (5.0 mL) and (2*S*)-2-aminobutan-1-ol (8.12 mL, 85.11 mmol, 18.0 eq.) then the vial was sealed and put on heating block 24 h at 180 °C. After cooling, the reaction mixture was poured onto water (100.0 mL), vigorously stirred 5 min and the aqueous mixture was extracted with EtOAc (3x30.0 mL). Organic layers were combined, washed with brine (20.0 mL), dried over MgSO₄, filtered, concentrated and purified by flash chromatography with DCM/MeOH (98/2 to 93/7) as eluent to give **(101)** (1.290 g, 63 %) as a beige solid.

### Example 4.52: Synthesis of N8-[(4-bromophenyl)methyl]-3-isopropyl-N6-methyl-[1,2,4]triazolo[4,3-b]pyridazine-6,8-diamine (116)

In a sealed tube 10 - 20 mL with a stir bar was charged **(3.42)** (1.800 g, 4.73 mmol, 1.0 eq.), NMP (5.0 mL) and (2*S*)-2-aminobutan-1-ol (8.12 mL, 85.11 mmol, 18.0 eq.) then the vial was sealed and put on heating block 24 h at 180 °C. After cooling, the reaction mixture was poured onto water (100.0 mL), vigorously stirred 5 min and the aqueous mixture was extracted with EtOAc (3x30.0 mL). Organic layers were combined, washed with brine (20.0 mL), dried over MgSO₄, filtered, concentrated and purified by flash chromatography with DCM/MeOH (98/2 to 93/7) as eluent to give the secondary compound **(116)** (0.300 g, 17 %) as a white solid.

### Example 4.53: Synthesis of N6-(1-ethylpropyl)-3-isopropyl-N8-phenyl-[1,2,4]triazolo[4,3-b]pyridazine-6,8-diamine (45)

In a sealed tube 2 - 5 mL with a stir bar was charged **(3.12)** (0.090 g, 0.31 mmol, 1.0 eq.), cineol (0.7 mL) and 3-aminopentane (0.55 mL, 4.69 mmol, 15.0 eq.). The vial was sealed and put on heating block 40 h at 170 °C. After cooling, the mixture was concentrated with SiO₂ to make solid deposit then directly purified by flash chromatography with DCM/MeOH (99/1 to 97/3) as eluent to give **(45)** (0.010 g, 9 %) as a white solid.

### Example 4.54: Synthesis of N6-(1-ethylpropyl)-3-isopropyl-N8-(2-pyridyl)-[1,2,4]triazolo[4,3-b]pyridazine-6,8-diamine (46)

In a sealed tube 2 - 5 mL with a stir bar was charged **(3.13)** (0.090 g, 0.31 mmol, 1.0 eq.), cineol (0.7 mL) and 3-aminopentane (0.55 mL, 4.68 mmol, 15.0 eq.). The vial was sealed and put on heating block 40 h at 170 °C. After cooling, the mixture was concentrated with SiO₂ to make solid deposit then directly purified by flash chromatography with DCM/MeOH (99/1 to 97/3) as eluent to give **(46)** (0.021 g, 20 %) as a beige solid.

### Example 4.55: Synthesis of N6-(1-ethylpropyl)-3-isopropyl-N8-(3-pyridyl)-[1,2,4]triazolo[4,3-b]pyridazine-6,8-diamine (47)

In a sealed tube 2 - 5 mL with a stir bar was charged **(3.14)** (0.090 g, 0.31 mmol, 1.0 eq.), cineol (0.7 mL) and 3-aminopentane (0.55 mL, 4.68 mmol, 15.0 eq.). The vial was sealed and put on heating block 40 h at 170 °C. After cooling, the mixture was concentrated with SiO₂ to make solid deposit then directly purified by flash chromatography with DCM/MeOH (99/1 to 96/4) as eluent to give **(47)** (0.025 g, 24 %) as a beige solid.

### Example 4.56: Synthesis of N6-(1-ethylpropyl)-3-isopropyl-N8-(4-pyridyl)-[1,2,4]triazolo[4,3-b]pyridazine-6,8-diamine (48)

In a sealed tube 2 - 5 mL with a stir bar was charged **(3.15)** (0.090 g, 0.31 mmol, 1.0 eq.), cineol (0.7 mL) and 3-aminopentane (0.55 mL, 4.68 mmol, 15.0 eq.). The vial was sealed and put on heating block 40 h at 170 °C. After cooling, the mixture was concentrated with SiO₂ to make solid deposit then directly purified by flash chromatography with DCM/MeOH (99/1 to 95/5) as eluent to give **(48)** (0.060 g, 57 %) as a white solid.

### Example 4.57: Synthesis of N6-(1-ethylpropyl)-3-isopropyl-N8-(4-pyridylmethyl)-[1,2,4]triazolo[4,3-b]pyridazine-6,8-diamine (49)

In a sealed tube 2 - 5 mL with a stir bar was charged **(3.16)** (0.100 g, 0.33 mmol, 1.0 eq.), NMP (0.17 mL) and 3-aminopentane (0.58 mL, 4.95 mmol, 15.0 eq.) then the vial was sealed and put on heating block 15 h at 160 °C. To complete the reaction, NMP (0.34 mL) was added the the vial was sealed and put on heating block 23 h at 180 °C. After cooling, the mixture was diluted in EtOAc (10.0 mL) and organic layer was washed three times with a mixture brine/water (2/1, 3x10.0 mL). Organic layer was dried over MgSO₄, filtered, concentrated and purified by flash chromatography with DCM/MeOH (99/1 to 95/5) as eluent to give **(49)** (0.054 g, 46 %) as a white solid.

### Example 4.58: Synthesis of N6-(1-ethylpropyl)-3-isopropyl-N8-(3-pyridylmethyl)-[1,2,4]triazolo[4,3-b]pyridazine-6,8-diamine (50)

In a sealed tube 10 - 20 mL with a stir bar was charged **(3.17)** (0.580 g, 1.92 mmol, 1.0 eq.), NMP (4.1 mL) and 3-aminopentane (4.06 mL, 34.48 mmol, 18.0 eq.) then the vial was sealed and put on heating block 20 h at 180 °C. After cooling, the mixture was poured onto saturated NaHCO₃ (20.0 mL), rinse the vial and adjust with water up to 80.0 mL then the mixture was stirred 10 min. The resulting precipitate was filtered off, washed with water up to neutral pH, change the flask and washed the solid with EtOAc. Organic filtrate was dried over MgSO₄, filtered, concentrated and purified by flash chromatography with DCM/MeOH (98/2 to 94/6) as eluent to give **(50)** (0.160 g, 24 %) as a light pink solid.

### Example 4.59: Synthesis of N6-(1-ethylpropyl)-3-isopropyl-N8-(2-pyridylmethyl)-[1,2,4]triazolo[4,3-b]pyridazine-6,8-diamine (51)

In a sealed tube 10 - 20 mL with a stir bar was charged **(3.18)** (0.750 g, 2.48 mmol, 1.0 eq.), NMP (5.0 mL) and 3-aminopentane (4.96 mL, 42.11 mmol, 17.0 eq.) then the vial was sealed and put on heating block 69 h at 180 °C. After cooling, the mixture was poured onto saturated NaHCO₃ (20.0 mL), rinse the vial and adjust with water up to 80.0 mL then the mixture was stirred 10 min. The resulting precipitate was filtered off, washed with water up to neutral pH, change the flask and washed the solid with DCM. Organic filtrate was dried over MgSO₄, filtered, concentrated and purified by flash chromatography with DCM/MeOH (98/2 to 94/6) as eluent to give **(51)** (0.420 g, 48 %) as a white solid.

### Example 4.60: Synthesis of N6-tert-butyl-3-isopropyl-N8-(2-pyridylmethyl)-[1,2,4]triazolo[4,3-b]pyridazine-6,8-diamine (52)

In a sealed tube 2 - 5 mL with a stir bar was charged **(3.18)** (0.250 g, 0.83 mmol, 1.0 eq.), NMP (2.0 mL) and corresponding amine (0.45 mL, 4.13 mmol, 5.0 eq.). The vial was sealed and then put on heating block, 65 h at 170 °C. After cooling, the reaction mixture was poured onto brine/water (1/1, 30 mL) and stirred vigorously 5 min. The resulting precipitate was filtered, washed with water, taken up in DCM and organic filtrate was dried over MgSO₄, filtered, concentrated and purified by flash chromatography with DCM/MeOH (99/1 to 93/7) as eluent to give **(52)** (0.020 g, 7 %) as a white solid.

### Example 4.61: Synthesis of 3-isopropyl-N8-(2-pyridylmethyl)-N6-spiro[3.3]heptan-2-yl-[1,2,4]triazolo[4,3-b]pyridazine-6,8-diamine (53)

In a sealed tube 2 - 5 mL with a stir bar was charged, **(3.18)** (0.200 g, 0.66 mmol, 1.0 eq.), NMP (1.0 mL), corresponding amine hydrochloride (0.399 g, 2.64 mmol, 4.0 eq.) and DIPEA (0.69 mL, 3.96 mmol, 6.0 eq.). The vial was sealed and then put on heating block 23 h at 170 °C. After cooling, the mixture was diluted in water (10.0 mL), the resulting precipitate was triturated in water, filtered off and washed with a little amount of water. The solid was triturated with EtOAc, filtered and organic filtrate was dried over MgSO₄, filtered, concentrated and purified by flash chromatography with DCM/MeOH (98/2 to 93/7) as eluent to give **(53)** (0.035 g, 14 %) as a grey solid.

### Example 4.62: Synthesis of N6-(3,3-difluorocyclobutyl)-3-isopropyl-N8-(2-pyridylmethyl)-[1,2,4]triazolo[4,3-b]pyridazine-6,8-diamine (54)

In a sealed tube 2 - 5 mL with a stir bar was charged, **(3.18)** (0.200 g, 0.66 mmol, 1.0 eq.), NMP (1.0 mL), corresponding amine hydrochloride (0.399 g, 2.64 mmol, 4.0 eq.) and DIPEA (0.69 mL, 3.96 mmol, 6.0 eq.). The vial was sealed and then put on heating block 23 h at 170 °C. After cooling, the mixture was diluted in water (10.0 mL), the resulting precipitate was triturated in water, filtered off and washed with a little amount of water. The solid was triturated with EtOAc, filtered and organic filtrate was dried over MgSO₄, filtered, concentrated and purified by flash chromatography with DCM/MeOH (98/2 to 93/7) as eluent to give **(54)** (0.035 g, 14 %) as a beige solid.

### Example 4.63: Synthesis of N6-(4,4-difluorocyclohexyl)-3-isopropyl-N8-(2-pyridylmethyl)-[1,2,4]triazolo[4,3-b]pyridazine-6,8-diamine (55)

In a sealed tube 2 - 5 mL with a stir bar was charged, **(3.18)** (0.200 g, 0.66 mmol, 1.0 eq.), NMP (1.0 mL), corresponding amine hydrochloride (0.399 g, 2.64 mmol, 4.0 eq.) and DIPEA (0.69 mL, 3.96 mmol, 6.0 eq.). The vial was sealed and then put on heating block 23 h at 170 °C. After cooling, the mixture was diluted in water (10.0 mL), the resulting precipitate was triturated in water, filtered off and washed with a little amount of water. The solid was triturated with EtOAc, filtered and organic filtrate was dried over MgSO₄, filtered, concentrated and purified by flash chromatography with DCM/MeOH (98/2 to 93/7) as eluent to give **(55)** (0.010 g, 14 %) as a white solid.

### Example 4.64: Synthesis of N6-benzyl-3-isopropyl-N8-(2-pyridylmethyl)-[1,2,4]triazolo[4,3-b]pyridazine-6,8-diamine (56)

In a sealed tube 2 - 5 mL with a stir bar was charged **(3.18)** (0.150 g, 0.50 mmol, 1.0 eq.), NMP (1.0 mL) and benzylamine (0.33 mL, 2.97 mmol, 6.0 eq.). The vial was sealed and then put on heating block 17 h at 180 °C. After cooling, the mixture was diluted in brine (30.0 mL) and water (10.0 mL) then aqueous layer was extracted twice with EtOAc (2x20.0 mL). Organics layers were combined, washed with brine, dried over MgSO₄, filtered, concentrated and purified by flash chromatography with DCM/MeOH (99/1 to 84/6) as eluent to give **(56)** (0.070 g, 38 %) as a white solid.

### Example 4.65: Synthesis of N6-(1-ethylpropyl)-N8-(2-pyridylmethyl)-[1,2,4]triazolo[4,3-b]pyridazine-6,8-diamine (57)

In a sealed tube 10 - 20 mL with a stir bar was charged **(3.19)** (0.500 g, 1.92 mmol, 1.0 eq.), NMP (4.5 mL) and corresponding amine (4.51 mL, 38.36 mmol, 20.0 eq.). The vial was sealed and then put on heating block 18 h at 180 °C. After cooling, the mixture was poured onto saturated NaHCO₃ (50.0 mL), rinse the vial and adjust with water up to 100.0 mL then the mixture was stirred 10 min. The resulting precipitate was filtered off, washed with water up to neutral pH, change the flask and washed the solid with EtOAc. Organic filtrate was dried over MgSO₄, filtered, concentrated and purified by flash chromatography with DCM/MeOH (98/2 to 93/7) as eluent to give **(57)** (0.180 g, 30 %) as a white solid.

### Example 4.66: Synthesis of N6-(1-ethylpropyl)-3-methyl-N8-(2-pyridylmethyl)-[1,2,4]triazolo[4,3-b]pyridazine-6,8-diamine (58)

In a sealed tube 10 - 20 mL with a stir bar was charged **(3.20)** (0.550 g, 2.00 mmol, 1.0 eq.), NMP (4.7 mL) and corresponding amine (4.71 mL, 40.04 mmol, 20.0 eq.). The vial was sealed and then put on heating block 18 h at 180 °C. After cooling, the mixture was poured onto saturated NaHCO₃ (50.0 mL), rinse the vial and adjust with water up to 100.0 mL then the mixture was stirred 10 min. The resulting precipitate was filtered off, washed with water up to neutral pH, change the flask and washed the solid with EtOAc. Organic filtrate was dried over MgSO₄, filtered, concentrated and purified by flash chromatography with DCM/MeOH (98/2 to 93/7) as eluent to give **(58)** (0.100 g, 15 %) as a white solid.

### Example 4.67: Synthesis of 3-ethyl-N6-(1-ethylpropyl)-N8-(2-pyridylmethyl)-[1,2,4]triazolo[4,3-b]pyridazine-6,8-diamine (59)

In a sealed tube 10 - 20 mL with a stir bar was charged **(3.21)** (0.350 g, 1.21 mmol, 1.0 eq.), NMP (2.85 mL) and corresponding amine (2.85 mL, 24.24 mmol, 20.0 eq.). The vial was sealed and then put on heating block 21 h at 180 °C. After cooling, the mixture was poured onto saturated NaHCO₃ (20.0 mL), rinse the vial and adjust with water up to 40.0 mL then the mixture was stirred 10 min. Aqueous mixture was extracted three times with EtOAc (3x15.0 mL) then organics layers were combined, washed with brine (15.0 mL), dried over MgSO₄, filtered, concentrated and purified by flash chromatography with DCM/MeOH (98/2 to 94/6) as eluent to give **(59)** (0.074 g, 18 %) as a white solid.

### Example 4.68: Synthesis of N6-(1-ethylpropyl)-3-propyl-N8-(2-pyridylmethyl)-[1,2,4]triazolo[4,3-b]pyridazine-6,8-diamine (60)

In a sealed tube 2 - 5 mL with a stir bar was charged **(3.22)** (0.200 g, 0.66 mmol, 1.0 eq.), NMP (0.7 mL) and corresponding amine (0.78 mL, 6.61 mmol, 10.0 eq.) then the vial was sealed and put on heating block 22 h at 180 °C. After cooling, the mixture was diluted in brine (30.0 mL) and water (30.0 mL) and the mixture was stirred 10 min. The resulting precipitate was filtered off, washed with water up to neutral pH then taken up in EtOAc. Organic filtrate was dried over MgSO₄, filtered, concentrated and purified by flash chromatography with DCM/MeOH (98/2 to 95/5) as eluent to give **(60)** (0.065 g, 28 %) as a beige solid.

### Example 4.69: Synthesis of 3-tert-butyl-N6-(1-ethylpropyl)-N8-(2-pyridylmethyl)-[1,2,4]triazolo[4,3-b]pyridazine-6,8-diamine (61)

In a sealed tube 10 - 20 mL with a stir bar was charged **(3.23)** (0.500g, 1.58 mmol, 1.0 eq.), NMP (3.7mL) and corresponding amine (3.72 mL, 31.57 mmol, 20.0 eq.). The vial was sealed and then put on heating block 21 h at 180 °C. After cooling, the mixture was poured onto saturated NaHCO₃ (20.0 mL), rinse the vial and adjust with water up to 40.0 mL then the mixture was stirred 10 min. Aqueous mixture was extracted three times with EtOAc (3x15.0 mL) then organics layers were combined, washed with brine (15.0 mL), dried over MgSO₄, filtered, concentrated and purified by flash chromatography with DCM/MeOH (98/2 to 94/6) as eluent to give **(61)** (0.105 g, 18 %) as a white solid.

### Example 4.70: Synthesis of 3-cyclopropyl-N6-(1-ethylpropyl)-N8-(2-pyridylmethyl)-[1,2,4]triazolo[4,3-b]pyridazine-6,8-diamine (62)

In a sealed tube 10 - 20 mL with a stir bar was charged **(3.24)** (1.000 g, 3.32 mmol, 1.0 eq.), NMP (5.9 mL) and corresponding amine (5.87 mL, 49.88 mmol, 15.0 eq.). The vial was sealed and then put on heating block 18 h at 180 °C. After cooling, the mixture was poured onto saturated NaHCO₃ (50.0 mL), rinse the vial and adjust with water up to 100.0 mL then the mixture was stirred 10 min. The resulting precipitate was filtered off, washed with water up to neutral pH, change the flask and washed the solid with EtOAc. Organic filtrate was dried over MgSO₄, filtered, concentrated and purified by flash chromatography with DCM/MeOH (98/2 to 93/7) as eluent to give **(62)** (0.160 g, 14 %) as a grey solid.

### Example 4.71: Synthesis of 3-cyclobutyl-N6-(1-ethylpropyl)-N8-(2-pyridylmethyl)-[1,2,4]triazolo[4,3-b]pyridazine-6,8-diamine (63)

In a sealed tube 10 - 20 mL with a stir bar was charged **(3.25)** (0.320 g, 1.02 mmol, 1.0 eq.), NMP (2.4 mL) and corresponding amine (2.39 mL, 20.33 mmol, 20.0 eq.). The vial was sealed and then put on heating block 21 h at 180 °C. After cooling, the mixture was poured onto saturated NaHCO₃ (20.0 mL), rinse the vial and adjust with water up to 60.0 mL then the mixture was stirred 10 min. Aqueous mixture was extracted three times with EtOAc (3x15.0 mL) then organics layers were combined, washed with brine (15.0 mL), dried over MgSO₄, filtered, concentrated and purified by flash chromatography with DCM/MeOH (98/2 to 94/6) as eluent to give **(63)** (0.093 g, 25 %) as a white solid.

### Example 4.72: Synthesis of 3-cyclopentyl-N6-(1-ethylpropyl)-N8-(2-pyridylmethyl)-[1,2,4]triazolo[4,3-b]pyridazine-6,8-diamine (64)

In a sealed tube 10 - 20 mL with a stir bar was charged **(3.26)** (0.500 g, 1.52 mmol, 1.0 eq.), NMP (3.6 mL) and corresponding amine (3.58 mL, 30.41 mmol, 20.0 eq.). The vial was sealed and then put on heating block 21 h at 180 °C. After cooling, the mixture was poured onto saturated NaHCO₃ (20.0 mL), rinse the vial and adjust with water up to 60.0 mL then the mixture was stirred 10 min. The resulting precipitate was filtered off, washed with water up to neutral pH then the solid was taken up in DCM. Organic filtrate was dried over MgSO₄, filtered, concentrated and purified by flash chromatography with DCM/MeOH (98/2 to 94/6) as eluent to give **(64)** (0.124 g, 21 %) as a white solid.

### Example 4.73: Synthesis of 3-cyclohexyl-N6-(1-ethylpropyl)-N8-(2-pyridylmethyl)-[1,2,4]triazolo[4,3-b]pyridazine-6,8-diamine (65)

In a sealed tube 10 - 20 mL with a stir bar was charged **(3.27)** (0.600 g, 1.75 mmol, 1.0 eq.), NMP (3.9 mL), corresponding amine (3.91 mL, 33.25 mmol, 19.0 eq.). The vial was sealed and then put on heating block, 16 h at 180 °C. After cooling, the mixture was poured onto saturated NaHCO₃ (20.0 mL), rinse the vial and adjust with water up to 80.0 mL then the mixture was stirred 10 min. The resulting precipitate was filtered off, washed with water up to neutral pH, the vacuum flask was changed then the solid was triturated and washed with EtOAc. Organic filtrate was dried over MgSO₄, filtered, concentrated and purified by flash chromatography with DCM/MeOH (98/2 to 97/7) as eluent to give **(65)** (0.110 g, 16 %) as a white solid.

### Example 4.74: Synthesis of N6-(1-ethylpropyl)-3-phenyl-N8-(2-pyridylmethyl)-[1,2,4]triazolo[4,3-b]pyridazine-6,8-diamine (66)

In a sealed tube 10 - 20 mL with a stir bar was charged **(3.28)** (0.550 g, 1.63 mmol, 1.0 eq.), NMP (4.0 mL), corresponding amine (4.04 mL, 34.30 mmol, 21.0 eq.). The vial was sealed and then put on heating block, 20 h at 180 °C. After cooling, the mixture was poured onto saturated NaHCO₃ (20.0 mL), rinse the vial and adjust with water up to 80.0 mL then the mixture was stirred 10 min. The resulting precipitate was filtered off, washed with water up to neutral pH, the vacuum flask was changed then the solid was triturated and washed with a mixture of DCM/MeOH (98/2). Organic filtrate was dried over MgSO₄, filtered, concentrated and purified by flash chromatography with DCM/MeOH (98/2 to 94/6) as eluent to give **(66)** (0.265 g, 42 %) as a white solid.

### Example 4.75: Synthesis of N6-(1-ethylpropyl)-N8-(2-pyridylmethyl)-3-sec-butyl-[1,2,4]triazolo[4,3-b]pyridazine-6,8-diamine (67)

In a sealed tube 2 - 5 mL with a stir bar was charged **(3.29)** (0.100 g, 0.39 mmol, 1.0 eq.), mono ethylene glycol (1.0 mL) and 3-aminopentane (0.98 mL g, 8.29 mmol, 15.0 eq.) then the vial was sealed and put on heating block 16 h at 180 °C. After cooling, the reaction mixture was poured onto saturated NaHCO₃ (10.0 mL), the resulting precipitate was triturated, filtered, washed with water up to neutral pH and taken up in EtOAc. Organic filtrate was dried over MgSO₄, filtered, concentrated and purified by flash chromatography with DCM/MeOH (98/2 to 94/6) as eluent to **(67)** (0.047 g, 23 %) as a white solid.

### Example 4.76: Synthesis of N6,3-bis(1-ethylpropyl)-N8-(2-pyridylmethyl)-[1,2,4]triazolo[4,3-b]pyridazine-6,8-diamine (68)

In a sealed tube 2 - 5 mL with a stir bar was charged **(3.30)** (0.200 g, 0.61 mmol, 1.0 eq.), mono ethylene glycol (1.0 mL) and 3-aminopentane (1.07 mL, 9.07 mmol, 15.0 eq.) then the vial was sealed and put on heating block 19 h at 180 °C. After cooling, the reaction mixture was poured onto cold water (30.0 mL), stirred 5 min then the resulting precipitate was filtered off, washed with cold water up to neutral pH and taken up in DCM. Organic filtrate was dried over MgSO₄, filtered, concentrated and purified by flash chromatography with DCM/MeOH (98/2 to 94/6) as eluent to **(68)** (0.085 g, 37 %) as a white solid.

### Example 4.77: Synthesis of N6-(1-ethylpropyl)-3-isobutyl-N8-(2-pyridylmethyl)-[1,2,4]triazolo[4,3-b]pyridazine-6,8-diamine (69)

In a sealed tube 2 - 5 mL with a stir bar was charged **(3.31)** (0.250 g, 0.79 mmol, 1.0 eq.), NMP (1.9 mL) and 3-aminopentane (1.86 mL g, 15.78 mmol, 20.0 eq.) then the vial was sealed and put on heating block 15 h at 180 °C. After cooling, the reaction mixture was poured onto saturated NaHCO₃ (30.0 mL) then aqueous mixture was extracted with EtOAc (3x20.0 mL). Organics layers were combined, washed with brine (40.0 mL), dried over MgSO₄, filtered, concentrated and purified by flash chromatography with DCM/MeOH (99/1 to 94/6) as eluent to **(69)** (0.065 g, 22 %) as a white solid.

### Example 4.78: Synthesis of N6-(1-ethylpropyl)-3-(2-pyridyl)-N8-(2-pyridylmethyl)-[1,2,4]triazolo[4,3-b]pyridazine-6,8-diamine (70)

In a sealed tube 2 - 5 mL with a stir bar was charged **(3.32)** (0.200 g, 0.59 mmol, 1.0 eq.), mono ethylene glycol (1.0 mL) and 3-aminopentane (1.05 mL, 8.88 mmol, 15.0 eq.) then the vial was sealed and put on heating block 19 h at 180 °C. After cooling, the reaction mixture was poured onto cold water (30.0 mL) then aqueous mixture was extracted with 2-MeTHF (3x15.0 mL). Orgnics layers were combined, washed with brine (20.0 mL), dried over MgSO₄, filtered, concentrated and purified by flash chromatography with DCM/MeOH (97/3 to 90/10) as eluent to **(70)** (0.070 g, 30 %) as a white solid.

### Example 4.79: Synthesis of N6-(1-ethylpropyl)-3-(3-pyridyl)-N8-(2-pyridylmethyl)-[1,2,4]triazolo[4,3-b]pyridazine-6,8-diamine (71)

In a sealed tube 2 - 5 mL with a stir bar was charged **(3.33)** (0.200 g, 0.59 mmol, 1.0 eq.), mono ethylene glycol (1.0 mL) and 3-aminopentane (1.05 mL, 8.88 mmol, 15.0 eq.) then the vial was sealed and put on heating block 19 h at 180 °C. After cooling, the reaction mixture was poured onto cold water (30.0 mL), stirred 5 min then the resulting precipitate was filtered off, washed with cold water up to neutral pH and taken up in DCM. Organic filtrate was dried over MgSO₄, filtered, concentrated and purified by flash chromatography with DCM/MeOH (97/3 to 93/7) as eluent to **(71)** (0.086 g, 37 %) as a grey solid.

### Example 4.80: Synthesis of N6-(1-ethylpropyl)-3-(4-pyridyl)-N8-(2-pyridylmethyl)-[1,2,4]triazolo[4,3-b]pyridazine-6,8-diamine (72)

In a sealed tube 2 - 5 mL with a stir bar was charged **(3.34)** (0.200 g, 0.59 mmol, 1.0 eq.), mono ethylene glycol (1.0 mL) and 3-aminopentane (1.05 mL, 8.88 mmol, 15.0 eq.) then the vial was sealed and put on heating block 19 h at 180 °C. After cooling, the reaction mixture was poured onto cold water (30.0 mL), stirred 5 min then the resulting precipitate was filtered off, washed with cold water up to neutral pH and taken up in DCM. Organic filtrate was dried over MgSO₄, filtered, concentrated and purified by flash chromatography with DCM/MeOH (97/3 to 93/7) as eluent to **(72)** (0.160 g, 70 %) as a beige solid.

### Example 4.81: Synthesis of (2R)-2-[[8-[(4-aminophenyl)methylamino]-3-isopropyl-[1,2,4]triazolo[4,3-b]pyridazin-6-yl]amino]butan-1-ol (74)

In a sealed tube 10-20 mL with a stir bar was charged **(3.35)** (0.400 g, 1.26 mmol, 1.0 eq.), NMP (3.0 mL) and corresponding amine (3.04 mL, 31.57 mmol, 25.0 eq.). The vial was sealed and then put on heating block 17.5 h at 180 °C. After cooling, the mixture was poured onto water (100.0 mL), aqueous layer was extracted with EtOAc (3 x 30.0 mL) then organic layers were combined, dried over MgSO_{4,} filtered, concentrated and purified by flash chromatography with DCM/MeOH (97/3 to 90/10) as eluent to give **(74)** (0.100 g, 21 %) as a white solid.

### Example 4.82: Synthesis of N8-[(4-aminophenyl)methyl]-N6-(1-ethylpropyl)-3-isopropyl-[1,2,4]triazolo[4,3-b]pyridazine-6,8-diamine (75)

In a sealed tube 2 - 5 mL with a stir bar was charged **(3.35)** (0.100 g, 0.32 mmol, 1.0 eq.), NMP (0.3 mL) and corresponding amine (0.55 mL, 4.74 mmol, 15.0 eq.) then the vial was sealed and put on heating block 22 h at 180 °C. After cooling, the mixture was diluted in DCM (5.0 mL), Si₂O was added to make solid deposit then purfied by flash chromatography with DCM/MeOH (99/1 to 95/5) as eluent to give (75) (0.020 g, 17 %) as a white solid.

### Example 4.83: Synthesis of N8-[(3-aminophenyl)methyl]-N6-(1-ethylpropyl)-3-isopropyl-[1,2,4]triazolo[4,3-b]pyridazine-6,8-diamine (76)

In a sealed tube 2 - 5 mL with a stir bar was charged (3.36) (0.190 g, 0.60 mmol, 1.0 eq.), NMP (1.0 mL) and corresponding amine (0.71 mL, 6.00 mmol, 10.0 eq.) then the vial was sealed and put on heating block 22 h at 170 °C. After cooling, the mixture was diluted in EtOAc (20.0 mL) and organic layer was washed three times with a mixture brine/water (3/1, 3x10 mL), dried over MgSO₄, filtered, concentrated and purified by flash chromatography with DCM/MeOH (99/1 to 95/5) as eluent to give **(76)** (0.020 g, 9 %) as a light brown solid.

### Example 4.84: Synthesis of N8-[(2-aminophenyl)methyl]-N6-(1-ethylpropyl)-3-isopropyl-[1,2,4]triazolo[4,3-b]pyridazine-6,8-diamine (77)

In a sealed tube 2 - 5 mL with a stir bar was charged **(3.37)** (0.200 g, 0.63 mmol, 1.0 eq.), NMP (1.4 mL) and corresponding amine (0.74 mL, 6.31 mmol, 10.0 eq.) then the vial was sealed and put on heating block 19 h at 170 °C. After cooling, the mixture was diluted in EtOAc (25.0 mL) and organic layer was washed three times with a mixture brine/water (3/1, 3 x 20.0 mL), dried over MgSO₄, filtered, concentrated and purified by flash chromatography with DCM/MeOH (97/3 to 92/8) as eluent to give **(77)** (0.021 g, 14 %) as a light brown solid.

### Example 4.85: Synthesis of N6-(1-ethylpropyl)-N8-(1H-indol-2-ylmethyl)-3-isopropyl-[1,2,4]triazolo[4,3-b]pyridazine-6,8-diamine (78)

In a sealed tube 2 - 5 mL with a stir bar was charged **(3.41)** (0.150 g, 0.42 mmol, 1.0 eq.) and pentan-3-amine (1.48 mL, 12.54 mmol, 30.0 eq.). The vial was sealed and then put on heating block 21 h at 160 °C. After cooling, the mixture was poured into cold water (10 mL), the precipitate was filtered off, washed with water, dried under vacuum. The solid was taken up in EtOAc, the organic filtrate was dried over MgSO₄, filtered, concentrated and purified by flash chromatography with DCM/MeOH (97/3) as eluent to give **(78)** (0.015 g, 9 %) as a beige solid.

### Example 4.86: Synthesis of N-(1-ethylpropyl)-8-isoindolin-2-yl-3-isopropyl-[1,2,4]triazolo[4,3-b]pyridazin-6-amine (79)

In a sealed tube 2 - 5 mL with a stir bar was charged **(3.39)** (0.150 g, 0.48 mmol, 1.0 eq.), NMP (1.5 mL) and corresponding amine (0.45 mL, 3.82 mmol, 8.0 eq.) then the vial was sealed and put on heating block 65 h at 170 °C. After cooling, the mixture was diluted in EtOAc then organic layer was washed three times with a mixture of brine/water (3/1, 3x10.0 mL), dried over MgSO₄, filtered, concentrated and purified by flash chromatography with DCM/MeOH (99/1 to 96/4) as eluent to give the title compound **(79)** (0.065 g, 37 %) as a beige solid.

### Example 4.87: Synthesis of 8-(3,4-dihydro-1H-isoquinolin-2-yl)-N-(1-ethylpropyl)-3-isopropyl-[1,2,4]triazolo[4,3-b]pyridazin-6-amine (80)

In a sealed tube 2 - 5 mL with a stir bar was charged **(3.38)** (0.150 g, 0.46 mmol, 1.0 eq.), NMP (1.0 mL), corresponding amine (0.54 mL, 4.58 mmol, 10.0 eq.). The vial was sealed and then put on heating block 17 h at 170 °C. The reaction mixture was diluted in EtOAc (20.0 mL) then the organic layer was washed with a mixture of brine/saturated NaHCO₃ (3/1, 3 x 10.0 mL), dried over MgSO₄, filtered, concentrated and purified by flash chromatography with DCM/MeOH (99/1 to 96/4) as eluent to give **(80)** (0.033 g, 33 %) as light brown oil.

### Example 4.88: Synthesis of N6-(1-ethylpropyl)-N8-indan-1-yl-3-isopropyl-[1,2,4]triazolo[4,3-b]pyridazine-6,8-diamine (81)

In a sealed tube 2 - 5 mL with a stir bar was charged **(3.40)** (0.200 g, 0.61 mmol, 1.0 eq.), NMP (1.0 mL), corresponding amine (0.72 mL, 6.10 mmol, 10.0 eq.). The vial was sealed and then put on heating block, 16 h at 170 °C. The reaction mixture was poured onto cold water (30.0 mL), the mixture was stirred 5 min then the precipitate was filtered off, washed with water and taken up in EtOAc. Organic filtrate was dried over MgSO₄, filtered, concentrated and purified by flash chromatography with DCM/MeOH (98/2 to 92/8) as eluent to give **(81)** (0.025 g, 11 %) as a beige solid.

### Example 4.89: Synthesis of N6-(1-ethylpropyl)-3-isopropyl-N8-(2-phenylethyl)-[1,2,4]triazolo[4,3-b]pyridazine-6,8-diamine (82)

In a sealed tube 2 - 5 mL with a stir bar was charged **(3.43)** (0.240 g, 0.76 mmol, 1.0 eq.) in ethylene glycol (0.9 mL), corresponding amine (0.89 mL, 7.60 mmol, 10.0 eq.) was added then the tube was sealed and put on heating block 21 h at 180 °C. After cooling, the mixture was solublized in a mixture of DCM/MeOH (9/1) then concentrated with silica and directly purified by flash chromatography with DCM/MeOH (99/1 to 94/6) to give **(82)** (0.060 g, 22 %) as a white amorphous solid.

### Example 4.90: Synthesis of N6-(1-ethylpropyl)-3-isopropyl-N8-[2-(2-pyridyl)ethyl]-[1,2,4]triazolo[4,3-b]pyridazine-6,8-diamine (83)

In a sealed tube 2 - 5 mL with a stir bar was charged **(3.44)** (0.250 g, 0.79 mmol, 1.0 eq.), ethylene glycol (1.5 mL) and 3-aminopentane (1.39 mL, 11.84 mmol, 15.0 eq.). The vial was sealed and put on heating bloc 18 h at 170 °C. After cooling, the mixture was poured onto cold water (30.0 mL), stirred 5 min then the precipitate was filtered off and washed with cold water. On an other flask, the solid was washed with EtOAc, organic filtrate was dried over MgSO₄, filtered, concentrated and purified by flash chromatography with DCM/MeOH (98/2 to 93/7) as eluent to give **(83)** (0.045 g, 16 %) as a white solid.

### Example 4.91: Synthesis of N6-(1-ethylpropyl)-3-isopropyl-N8-[2-(3-pyridyl)ethyl]-[1,2,4]triazolo[4,3-b]pyridazine-6,8-diamine (84)

In a sealed tube 2 - 5 mL with a stir bar was charged **(3.45)** (0.235 g, 0.74 mmol, 1.0 eq.), ethylene glycol (1.5 mL) and 3-aminopentane (1.31 mL, 11.13 mmol, 15.0 eq.). The vial was sealed and put on heating bloc 16 h at 170 °C. After cooling, the mixture was poured onto cold water (15.0 mL), stirred 5 min then the precipitate was filtered off, washed with cold water and taken up in DCM. Aqueous filtrate was extracted with DCM (10.0 mL) then organics layers were combined, dried over MgSO₄, filtered, concentrated and purified by flash chromatography with DCM/MeOH (98/2 to 93/7) as eluent to give **(84)** (0.063 g, 23 %) as a white solid.

### Example 4.92: Synthesis of N6-(1-ethylpropyl)-3-isopropyl-N8-[2-(4-pyridyl)ethyl]-[1,2,4]triazolo[4,3-b]pyridazine-6,8-diamine (85)

In a sealed tube 2 - 5 mL with a stir bar was charged **(3.46)** (0.255 g, 0.81 mmol, 1.0 eq.), ethylene glycol (1.5 mL) and 3-aminopentane (1.42 mL, 12.07 mmol, 15.0 eq.). The vial was sealed and put on heating bloc 16 h at 170 °C. After cooling, the mixture was poured onto cold water (15.0 mL), stirred 5 min then the precipitate was filtered off, washed with cold water and taken up in DCM. Aqueous filtrate was extracted with DCM (10.0 mL) then organics layers were combined, dried over MgSO₄, filtered, concentrated and purified by flash chromatography with DCM/MeOH (98/2 to 93/7) as eluent to give **(85)** (0.048 g, 16 %) as a white solid.

### Example 4.93: Synthesis of 3-cyclopropyl-N6-(1-ethylpropyl)-N8-[2-(2-pyridyl)ethyl]-[1,2,4]triazolo[4,3-b]pyridazine-6,8-diamine (86)

In a sealed tube 2 - 5 mL with a stir bar was charged **(3.47)** (0.110 g, 0.35 mmol, 1.0 eq.), NMP (1.0 mL) and 3-aminopentane (0.41 mL, 3.50 mmol, 10.0 eq.) was added then the vial was sealed and put on heating bloc 17 h at 180 °C. After cooling, the mixture was poured onto cold water (20.0 mL) and brine (30.0 mL) then the resulting precipitate was filtered off, washed with water and taken up in EtOAc. Organic filtrate was dried over MgSO₄, filtered, concentrated and purified by flash chromatography wth DCM/MeOH (98/2 to 93/7) as eluent to give **(86)** (0.020 g, 16 %) as a white solid.

### Example 4.94: Synthesis of 3-cyclopropyl-N6-(1-ethylpropyl)-N8-[2-(3-pyridyl)ethyl]-[1,2,4]triazolo[4,3-b]pyridazine-6,8-diamine (87)

In a sealed tube 2 - 5 mL with a stir bar was charged **(3.48)** (0.110 g, 0.35 mmol, 1.0 eq.), NMP (1.0 mL) and 3-aminopentane (0.41 mL, 3.50 mmol, 10.0 eq.) was added then the vial was sealed and put on heating bloc 17 h at 180 °C. After cooling, the mixture was poured onto cold water (20.0 mL) and brine (30.0 mL) then the resulting precipitate was filtered off, washed with water and taken up in EtOAc. Organic filtrate was dried over MgSO₄, filtered, concentrated and purified by flash chromatography wth DCM/MeOH (97/3 to 92/8) as eluent to give **(87)** (0.030 g, 23 %) as a beige solid.

### Example 4.95: Synthesis of 3-cyclopropyl-N6-(1-ethylpropyl)-N8-[2-(4-pyridyl)ethyl]-[1,2,4]triazolo[4,3-b]pyridazine-6,8-diamine (88)

In a sealed tube 2 - 5 mL with a stir bar was charged **(3.49)** (0.110 g, 0.35 mmol, 1.0 eq.), NMP (1.0 mL) and 3-aminopentane (0.41 mL, 3.50 mmol, 10.0 eq.) was added then the vial was sealed and put on heating bloc 17 h at 180 °C. After cooling, the mixture was poured onto cold water (20.0 mL) and brine (30.0 mL) then the resulting precipitate was filtered off, washed with water and taken up in EtOAc. Organic filtrate was dried over MgSO₄, filtered, concentrated and purified by flash chromatography wth DCM/MeOH (97/3 to 92/8) as eluent to give **(88)** (0.039 g, 31 %) as a white solid.

### Example 4.96: Synthesis of N6-(1-ethylpropyl)-3-isopropyl-N8-[3-(2-pyridyl)propyl]-[1,2,4]triazolo[4,3-b]pyridazine-6,8-diamine (89)

In a sealed tube 2 - 5 mL with a stir bar was charged **(3.50)** (0.100 g, 0.30 mmol, 1.0 eq.), NMP (0.9 mL) and 3-aminopentane (0.36 mL, 3.02 mmol, 10.0 eq.) was added then the vial was sealed and put on heating bloc 64 h at 170 °C. After cooling, the mixture was poured onto cold water (30.0 mL) then aqueous layer was extracted with EtOAc (3x 15.0 mL). Oganic layers were combined, washed with brine (10.0 mL), dried over MgSO₄, filtered, concentrated and purified by flash chromatography wth DCM/MeOH (98/2 to 92/8) as eluent to give (**89**) (0.040 g, 35 %) as a beige solid.

### Example 4.97: Synthesis of N6-(1-ethylpropyl)-3-isopropyl-N8-[3-(3-pyridyl)propyl]-[1,2,4]triazolo[4,3-b]pyridazine-6,8-diamine (90)

In a sealed tube 2 - 5 mL with a stir bar was charged **(3.51)** (0.180 g, 0.54 mmol, 1.0 eq.), NMP (0.5 mL) and 3-aminopentane (0.64 mL, 5.44 mmol, 10.0 eq.) was added then the vial was sealed and put on heating bloc 17 h at 170 °C. After cooling, the mixture was diluted in EtOAc (20.0 mL), organic layer was washed with a mixture brine/water (3/1, 3x10.0 mL), dried over MgSO₄, filtered, concentrated and purified by flash chromatography wth DCM/MeOH (98/2 to 92/8) as eluent to give (**90**) (0.018 g, 9 %) as a white solid.

### Example 4.98: Synthesis of N6-(1-ethylpropyl)-3-isopropyl-N8-[3-(4-pyridyl)propyl]-[1,2,4]triazolo[4,3-b]pyridazine-6,8-diamine (91)

In a sealed tube 2 - 5 mL with a stir bar was charged **(3.52)** (0.200 g, 0.61 mmol, 1.0 eq.), NMP (0.6 mL) and 3-aminopentane (0.71 mL, 6.05 mmol, 10.0 eq.) was added then the vial was sealed and put on heating bloc 17 h at 170 °C. After cooling, the mixture was diluted in EtOAc (20.0 mL), organic layer was washed with a mixture brine/water (3/1, 3x10.0 mL), dried over MgSO₄, filtered, concentrated and purified by flash chromatography wth DCM/MeOH (98/2 to 92/8) as eluent to give **(91)** (0.030 g, 13 %) as a beige solid.

### Example 4.99: Synthesis of N6-(1-ethylpropyl)-3-isopropyl-N8-(pyrimidin-2-ylmethyl)-[1,2,4]triazolo[4,3-b]pyridazine-6,8-diamine (92)

In a sealed tube 2 - 5 mL with a stir bar was charged **(3.54)** (0.070 g, 0.23 mmol, 1.0 eq.), ethylene glycol (0.5 mL) and 3-aminopentane (0.41 mL, 3.46 mmol, 15.0 eq.) was added then the vial was sealed and put on heating bloc 17 h at 180 °C. After cooling, the mixture was diluted in DCM, concentrated then the crude was triturated in saturated NaHCO₃ (10.0 mL). The solid was filtered off, washed with water up to neutral pH, taken up in EtOAc then organic filtrate was dried over MgSO₄, filtered, concentrated and purified by flash chromatography wth DCM/MeOH (98/2 to 93/7) as eluent to give **(92)** (0.022 g, 27 %) as a white solid.

### Example 4.100: Synthesis of N6-(1-ethylpropyl)-3-isopropyl-N8-(pyrimidin-5-ylmethyl)-[1,2,4]triazolo[4,3-b]pyridazine-6,8-diamine (93)

In a sealed tube 2 - 5 mL with a stir bar was charged **(3.56)** (0.080 g, 0.26 mmol, 1.0 eq.), ethylene glycol (0.5 mL) and 3-aminopentane (0.46 mL, 3.95 mmol, 15.0 eq.) was added then the vial was sealed and put on heating bloc 17 h at 180 °C. After cooling, the mixture was diluted in DCM, concentrated then the crude was triturated in saturated NaHCO₃ (10.0 mL). The solid was filtered off, washed with water up to neutral pH, taken up in EtOAc then organic filtrate was dried over MgSO₄, filtered, concentrated and purified by flash chromatography wth DCM/MeOH (98/2 to 93/7) as eluent to give **(93)** (0.010 g, 11 %) as a white solid.

### Example 4.101: Synthesis of N6-(1-ethylpropyl)-3-isopropyl-N8-(pyrazin-2-ylmethyl)-[1,2,4]triazolo[4,3-b]pyridazine-6,8-diamine (94)

In a sealed tube 2 - 5 mL with a stir bar was charged **(3.55)** (0.080 g, 0.26 mmol, 1.0 eq.), ethylene glycol (0.8 mL) and 3-aminopentane (0.62 mL, 5.27 mmol, 20.0 eq.) was added then the vial was sealed and put on heating bloc 18 h at 180 °C. After cooling, saturated NaHCO₃ (5.0 mL) was added and the mixture was vigorously stirred 5 min, the precipitate was filtered off, washed with water up to neutral pH and taken up in DCM. Organic filtrate was dried over MgSO₄, filtered, concentrated and purified by flash chromatography wth DCM/MeOH (98/2 to 94/6) as eluent to give **(94)** (0.032 g, 34 %) as a white solid.

### Example 4.102: Synthesis of N6-(1-ethylpropyl)-3-isopropyl-N8-(pyridazin-3-ylmethyl)-[1,2,4]triazolo[4,3-b]pyridazine-6,8-diamine (95)

In a sealed tube 2 - 5 mL with a stir bar was charged **(3.57)** (0.270 g, 0.89 mmol, 1.0 eq.), ethylene glycol (2.0 mL) and 3-aminopentane (1.57 mL, 13.33 mmol, 15.0 eq.) was added then the vial was sealed and put on heating bloc 18 h at 170 °C. After cooling, the mixture was poured onto cold water (20.0 mL) and brine (30.0 mL) then the resulting precipitate was filtered off, washed with water and taken up in DCM. Organic filtrate was dried over MgSO₄, filtered, concentrated and purified by flash chromatography wth DCM/MeOH (98/2 to 93/7) as eluent to give **(95)** (0.045 g, 14 %) as a white solid.

### Example 4.103: Synthesis of N8-benzyl-N6-cyclobutyl-3-isopropyl-[1,2,4]triazolo[4,3-b]pyridazine-6,8-diamine (117)

Reaction carried out in NMP (0.2M) on a 232 µmol scale of **(3.1),** with 5.0 eq. cyclobutylamine at 180 °C for 6h. Purification by TLC Prep (DCM/MeOH gradient) gives **(117)** with 6 % yield as a beige solid.

### Example 4.104: Synthesis of N8-benzyl-N6-cyclopentyl-3-isopropyl-[1,2,4]triazolo[4,3-b]pyridazine-6,8-diamine (118)

Reaction carried out in NMP (0.2M) on a 232 µmol scale of **(3.1),** with 5.0 eq. of cyclopentylamine at 180 °C for 6h15. Purification by flash chromatography (DCM/MeOH gradient) gives **(118)** with 40 % yield as a beige solid.

### Example 4.105: Synthesis of N8-benzyl-N6-cyclohexyl-3-isopropyl-[1,2,4]triazolo[4,3-b]pyridazine-6,8-diamine (119)

Reaction carried out in NMP (0.2M) on a 232 µmol scale of **(3.1),** with 5.0 eq. of cyclohexylamine at 180 °C for 45h. Purification by flash chromatography (DCM/MeOH gradient) gives **(119)** with 45 % yield as a beige solid.

### Example 4.106: Synthesis of N8-benzyl-3-isopropyl-N6-(4-methoxycyclohexyl)-[1,2,4]triazolo[4,3-b]pyridazine-6,8-diamine (120)

Reaction carried out in NMP (0.2M) on a 232 µmol scale of **(3.1),** with 5.0 eq. of 4-methoxycyclohexanamine at 200 °C for 16h. Purification by flash chromatography (DCM/MeOH gradient) gives **(120)** with 37 % yield as a beige solid.

### Example 4.107: Synthesis of N8-benzyl-N6-cycloheptyl-3-isopropyl-[1,2,4]triazolo[4,3-b]pyridazine-6,8-diamine (121)

Reaction carried out in NMP (0.2M) on a 232 µmol scale of **(3.1),** with 5.0 eq. of cycloheptylamine at 200 °C for 45h. Purification by flash chromatography (DCM/MeOH gradient) gives **(121)** with 22 % yield as a white solid.

### Example 4.108: Synthesis of N8-benzyl-N6-(cyclopropylmethyl)-3-isopropyl-[1,2,4]triazolo[4,3-b]pyridazine-6,8-diamine (122)

Reaction carried out in NMP (0.2M) on a 232 µmol scale of **(3.1),** with 5.0 eq. aminomethylcyclopropane at 180 °C for 16h. Purification by TLC Prep (DCM/MeOH gradient) gives **(122)** with 22 % yield as a beige solid.

### Example 4.109: Synthesis of N8-benzyl-N6-(cyclopropylmethyl)-3-isopropyl-[1,2,4]triazolo[4,3-b]pyridazine-6,8-diamine (123)

Reaction carried out in NMP (0.2M) on a 232 µmol scale of **(3.1),** with 5.0 eq. of 1-cyclobutylmethylamine at 180 °C for 40h. Purification by flash chromatography (DCM/MeOH gradient) gives **(123)** with 40 % yield as a beige solid.

### Example 4.110: Synthesis of N8-benzyl-N6-(cyclohexylmethyl)-3-isopropyl-[1,2,4]triazolo[4,3-b]pyridazine-6,8-diamine (124)

Reaction carried out in NMP (0.2M) on a 232 µmol scale of **(3.1),** with 5.0 eq. of cyclohexanemethylamine at 180°C for 6h15. Purification by TLC Prep (DCM/MeOH gradient) gives **(124)** with 46 % yield as a white solid.

### Example 4.111: Synthesis of N8-benzyl-3-isopropyl-N6-(tetrahydropyran-4-ylmethyl)-[1,2,4]triazolo[4,3-b]pyridazine-6,8-diamine (125)

Reaction carried out in NMP (0.2M) on a 166 µmol scale of **(3.1),** with 5.0 eq. of aminomethyltetrahydropyran at 180°C for 16h. Purification by TLC Prep (DCM/MeOH gradient) gives **(125)** with 50 % yield as a white solid.

### Example 4.112: Synthesis of N8-benzyl-N6-(2-ethylbutyl)-3-isopropyl-[1,2,4]triazolo[4,3-b]pyridazine-6,8-diamine (126)

Reaction carried out in NMP (0.2M) on a 232 µmοl scale of **(3.1),** with 5.0 eq. of 2-ethyl-1-butylamine at 180 °C for 45h. Purification by flash chromatography (DCM/MeOH gradient) gives **(126)** with 25 % yield as a beige solid.

### Example 4.113: Synthesis of N8-benzyl-3-isopropyl-N6-(2-phenylethyl)-[1,2,4]triazolo[4,3-b]pyridazine-6,8-diamine (127)

Reaction carried out in NMP (0.2M) on a 166 µmol scale of **(3.1),** with 5.0 eq. of phenylethylamine at 180 °C for 20h. Purification by TLC Prep (DCM/MeOH gradient) gives **(127)** with 32 % yield as a white solid.

### Example 4.114: Synthesis of N8-benzyl-3-isopropyl-N6-(2-phenoxyethyl)-[1,2,4]triazolo[4,3-b]pyridazine-6,8-diamine (128)

Reaction carried out in NMP (0.2M) on a 166 µmol scale of **(3.1),** with 5.0 eq. of 2-phenoxyethylamine at 180 °C for 36h. Purification by TLC Prep (DCM/MeOH gradient) gives **(128)** with 14 % yield as a white solid.

### Example 4.115: Synthesis of N8-benzyl-3-isopropyl-N6-[(1-methylimidazol-2-yl)methyl]-[1,2,4]triazolo[4,3-b]pyridazine-6,8-diamine (129)

Reaction carried out in NMP (0.2M) on a 232 µmol scale of **(3.1),** with 5.0 eq. of (1-methyl-1H-imidazol-2-yl)methylamine at 180 °C for 16h. Purification by flash chromatography (DCM/MeOH gradient) gives **(129)** with 30 % yield as a white solid.

### Example 4.116: Synthesis of N6,N8-dibenzyl-3-isopropyl-[1,2,4]triazolo[4,3-b]pyridazine-6,8-diamine (130)

Reaction carried out in NMP (0.2M) on a 232 µmol scale of **(3.1),** with 5.0 eq. of 4-benzylamine at 180 °C for 16h. Purification by flash chromatography (DCM/MeOH gradient) gives **(130)** with 49 % yield as a white solid.

### Example 4.117: Synthesis of N8-benzyl-3-isopropyl-N6-(2-pyridylmethyl)-[1,2,4]triazolo[4,3-b]pyridazine-6,8-diamine (131)

Reaction carried out in NMP (0.2M) on a 232 µmol scale of **(3.1),** with 5.0 eq. of 2-(aminomethyl)pyridine at 180 °C for 16h. Purification by flash chromatography (DCM/MeOH gradient) gives **(131)** with 25 % yield as a brown solid.

### Example 4.118: Synthesis of N8-benzyl-3-isopropyl-N6-(3-pyridylmethyl)-[1,2,4]triazolo[4,3-b]pyridazine-6,8-diamine (132)

Reaction carried out in NMP (0.2M) on a 232 µmol scale of **(3.1),** with 5.0 eq. of 3-(aminomethyl)pyridine at 180 °C for 23h. Purification by flash chromatography (DCM/MeOH gradient) gives **(132)** with 33 % yield as a white solid.

### Example 4.119: Synthesis of N8-benzyl-3-isopropyl-N6-(4-pyridylmethyl)-[1,2,4]triazolo[4,3-b]pyridazine-6,8-diamine (133)

Reaction carried out in NMP (0.2M) on a 232 µmol scale of **(3.1),** with 5.0 eq. of 4-(aminomethyl)pyridine at 180 °C for 23h. Purification by flash chromatography (DCM/MeOH gradient) gives **(133)** with 10 % yield as a white solid.

### Example 4.120: Synthesis of racemic trans-2-[[8-(benzylamino)-3-isopropyl-[1,2,4]triazolo[4,3-b]pyridazin-6-yl]amino]cyclohexanol (134)

Reaction carried out in NMP (0.25M) on a 248 µmol scale of **(3.1),** with 5.0 eq. of (1S*,2S*)-2-aminocyclohexan-1-ol at 180 °C for 27h. Purification by flash chromatography (DCM/MeOH gradient) gives **(134)** with 58 % yield as a brown solid.

### Example 4.121: Synthesis of racemic trans-4-[[8-(benzylamino)-3-isopropyl-[1,2,4]triazolo[4,3-b]pyridazin-6-yl]amino]cyclohexanol (135)

Reaction carried out in NMP (0.25M) on a 248 µmol scale of **(3.1),** with 5.0 eq. of *trans-4-*aminocyclohexan-1-ol at 180 °C for 27h. Purification by flash chromatography (DCM/MeOH gradient) gives **(135)** with 59 % yield as a light brown solid.

### Example 4.122: Synthesis of racemic trans-4-[[8-(benzylamino)-3-isopropyl-[1,2,4]triazolo[4,3-b]pyridazin-6-yl]amino]cyclohexanol (136)

Reaction carried out in NMP (0.25M) on a 248 µmol scale of **(3.1),** with 5.0 eq. of (1-methylcyclohexyl)methanamine at 180 °C for 24h. Purification by flash chromatography and PTLC (DCM/MeOH gradient) gives **(136)** with 58 % yield as a beige solid.

### Example 4.123: Synthesis of N6-(1-adamantyl)-N8-benzyl-3-isopropyl-[1,2,4]triazolo[4,3-b]pyridazine-6,8-diamine (137)

Reaction carried out in NMP (0.2M) on a 215 µmol scale of **(3.1),** with 5.0 eq. of 1-adamantylamine at 180 °C for 7 days. Purification by flash chromatography (DCM/MeOH gradient) gives **(137)** with 10 % yield as a brown solid.

### Example 4.124: Synthesis of N8-benzyl-N6-indan-2-yl-3-isopropyl-[1,2,4]triazolo[4,3-b]pyridazine-6,8-diamine (138)

Reaction carried out in NMP (0.25M) on a 248 µmol scale of **(3.1),** with 5.0 eq. of 2-aminoindane at 180 °C for 96h. Purification by flash chromatography and PTLC (DCM/MeOH gradient) gives **(138)** with 12 % yield as a light brown solid.

### Example 4.125: Synthesis of N8-benzyl-3-isopropyl-N6-[(5-methylpyrazin-2-yl)methyl]-[1,2,4]triazolo[4,3-b]pyridazine-6,8-diamine (139)

Reaction carried out in NMP (0.25M) on a 248 µmol scale of **(3.1),** with 5.0 eq. of (5-methylpyrazin-2-yl)methan-amine at 180 °C for 40h. Purification by flash chromatography (DCM/MeOH gradient) and trituration in MTBE give **(139)** with 27 % yield as a beige solid.

### Example 4.126: Synthesis of N8-benzyl-3-isopropyl-N6-[(1-methylpyrazol-4-yl)methyl]-[1,2,4]triazolo[4,3-b]pyridazine-6,8-diamine (140)

Reaction carried out in NMP (0.2M) on a 215 µmol scale of **(3.1),** with 5.0 eq. of (1-methyl-1H-pyrazol-4-yl)methanamine at 180 °C for 16h. Purification by flash chromatography (DCM/MeOH gradient) gives **(140)** with 53 % yield as a pale yellow solid.

### Example 4.127: Synthesis of N8-benzyl-N6-[(3,5-dimethylphenyl)methyl]-3-isopropyl-[1,2,4]triazolo[4,3-b]pyridazine-6,8-diamine (141)

Reaction carried out in NMP (0.2M) on a 215 µmol scale of **(3.1),** with 5.0 eq. of 3,5-dimethylbenzylamine at 180 °C for 16h. Purification by flash chromatography (DCM/MeOH gradient) gives **(141)** with 15 % yield as a white solid.

### Example 4.128: Synthesis of N8-benzyl-3-isopropyl-N6-(tetrahydrofuran-2-ylmethyl)-[1,2,4]triazolo[4,3-b]pyridazine-6,8-diamine (142)

Reaction carried out in NMP (0.2M) on a 215 µmol scale of **(3.1),** with 5.0 eq. of tetrahydrofurfurylamine at 180 °C for 22h. Purification by flash chromatography (DCM/MeOH gradient) gives **(142)** with 24 % yield as a colorless oil.

### Example 4.129: Synthesis of N8-benzyl-3-isopropyl-N6-(2-methylsulfanylethyl)-[1,2,4]triazolo[4,3-b]pyridazine-6,8-diamine (143)

Reaction carried out in NMP (0.2M) on a 215 µmol scale of **(3.1),** with 5.0 eq. of 2-(methylthio)-ethylamine at 180 °C for 16h. Purification by flash chromatography (DCM/MeOH gradient) gives **(143)** with 36 % yield as a colourless oil.

### Example 4.130: Synthesis of N8-benzyl-3-isopropyl-N6-(1-methylimidazol-2-yl)-[1,2,4]triazolo[4,3-b]pyridazine-6,8-diamine (144)

Reaction carried out in NMP (0.2M) on a 215 µmol scale of **(3.1),** with 5.0 eq. of 1-Methyl-1H-imidazol-2-amine at 180 °C for 16h. Purification by flash chromatography (DCM/MeOH gradient) gives **(144)** with 11 % yield as a white oil.

### Example 4.131: Synthesis of N8-benzyl-N6-[(4,6-dimethylpyrimidin-2-yl)methyl]-3-isopropyl-[1,2,4]triazolo[4,3-b]pyridazine-6,8-diamine (145)

Reaction carried out in NMP (0.2M) on a 215 µmol scale of **(3.1),** with 5.0 eq. of (4,6-dimethylpyrimidin-2-yl)methanamine, hydrochloride and 3.0 eq. of K₂CO₃ at 180 °C for 16h. Purification by flash chromatography (DCM/MeOH gradient) gives **(145)** with 13 % yield as an off-white solid.

### Example 4.132: Synthesis of N8-benzyl-N6-indan-1-yl-3-isopropyl-[1,2,4]triazolo[4,3-b]pyridazine-6,8-diamine (146)

Reaction carried out in NMP (0.2M) on a 215 µmol scale of **(3.1),** with 5.0 eq. of 1-amino-indane at 180 °C for 16h. Purification by flash chromatography (DCM/MeOH gradient) gives **(146)** with 20 % yield as a colourless oil.

### Example 4.133: Synthesis of (2S)-2-[[3-isopropyl-8-[[4-(2-pyridyl)phenyl]methylamino]-[1,2,4]triazolo[4,3-b]pyridazin-6-yl]amino]butan-1-ol (105)

In a sealed tube 10 - 20 mL with a stir bar was charged **(3.58)** (0.120 g, 0.32 mmol, 1.0 eq.), NMP (0.5 mL) and (2*S*)-2-aminobutan-1-ol (0.54 mL, 5.70 mmol, 18.0 eq.) then the vial was sealed and put on heating block 16 h at 170 °C. After cooling, water (4.0 mL) and saturated NaHCO₃ (4.0 mL) was added then the mixture was vigorously stirred 5 min. The precipitate was filtered off, washed with water up to neutral pH, washed with EtOAc and DCM to give a first part of **(105)** (0.039 g). Organic filtrate was dried over MgSO₄, filtered, concentrated and purified by flash chromatography with DCM/MeOH (98/2 to 94/6) as eluent to give the second part of **(105)** (0.035 g). Total of **(105):** 0.074 g, 54 %, as a white solid.

### Example 4.134: Synthesis of (2R,3R)-2-[[8-[(4-bromophenyl)methylamino]-3-isopropyl-[1,2,4]triazolo[4,3-b]pyridazin-6-yl]amino]butane-1,3-diol (106)

In a sealed tube 2 - 5 mL with a stir bar was charged **(3.42)** (0.250 g, 0.66 mmol, 1.0 eq.), NMP (0.7 mL) and (2*R*,3*R*)-2-aminobutane-1,3-diol (0.712 g, 6.57 mmol, 10.0 eq.) then the vial was sealed and put on heating block 24 h at 180 °C. After cooling, the mixture was poured onto water (30.0 mL) and extracted with EtOAc (3x20.0 mL). Organic layers were combined, dried over MgSO₄, filtered, concentrated and purified by flash chromatography with DCM/MeOH (98/2 to 95/5) as eluent. Fractions contains the desired impur compound was concentrated, the residu was triturated in DCM and the solid was filtered off, washed with DCM and dried under vacuum to give **(106)** (0.135 g, 46 %) as a white solid.

### Example 4.135: Synthesis of (2R,3R)-2-[[3-isopropyl-8-[[4-(2-pyridyl)phenyl]methylamino]-[1,2,4]triazolo[4,3-b]pyridazin-6-yl]amino]butane-1,3-diol (107)

In a sealed tube 2 - 5 mL with a stir bar was charged **(3.58)** (0.200 g, 0.53 mmol, 1.0 eq.), NMP (0.5 mL) and (2*R*,3*R*)-2-aminobutane-1,3-diol (0.286 g, 2.64 mmol, 5.0 eq.) then the vial was sealed and put on heating block 17 h at 180 °C. After cooling, the reaction mixture was diluted in EtOAc (20.0 mL), the resulting solid was filtered, washed with EtOAc. Organic filtrate was washed with brine (2x20.0 mL), dried over MgSO₄, filtered, concentrated and purified by flash chromatography with DCM/MeOH (98/2 to 93/7) as eluent to give **(107)** (0.100 g, 42 %) as a beige solid.

### Example 4.136: Synthesis of (2R,3R)-2-[[3-isopropyl-8-[[4-(4-pyridyl)phenyl]methylamino]-[1,2,4]triazolo[4,3-b]pyridazin-6-yl]amino]butane-1,3-diol (108)

In a sealed tube 2 - 5 mL with a stir bar was charged **(3.58)** (0.200 g, 0.53 mmol, 1.0 eq.), NMP (0.5 mL) and (2*R*,3*R*)-2-aminobutane-1,3-diol (0.286 g, 2.64 mmol, 5.0 eq.) then the vial was sealed and put on heating block 17 h at 180 °C. After cooling, the reaction mixture was diluted in DCM (10.0 mL) with SiO₂ to make solid deposit and directly purified by flash chromatography with DCM/MeOH (97/3 to 90/10) as eluent to give **(108)** (0.015 g, 14 %) as a white solid.

### Example 4.137: Synthesis of (2S)-2-[[3-isopropyl-8-[(4-phenylphenyl)methylamino]-[1,2,4]triazolo[4,3-b]pyridazin-6-yl]amino]butan-1-ol (102)

In a sealed tube 10 - 20 mL with a stir bar was charged **(101)** (0.200 g, 0.46 mmol, 1.0 eq.), phenylboronic acid (0.116 g, 0.92 mmol, 2.0 eq.), K₂CO₃ (0.191 g, 1.39 mmol, 3.0 eq.) and a mixture of dioxane/water (9/1, 5.0 mL). The mixure was degassed 5 min with inert gas then Pd(PPh₃)₄ (0.053 g, 0.05 mmol, 0.1 eq.) was added, the vial was sealed and put on heating bloc 2.5 h at 110 °C. After cooling, the mixture was directly concentrated with SiO₂ to make solid deposit and purified by flash chromatography with DCM/MeOH (98/2 to 94/6) as eluent to give **(102)** (0.160 g, 81 %) as a white solid.

### Example 4.138: Synthesis of (2S)-2-[[3-isopropyl-8-[[4-(4-pyridyl)phenyl]methylamino]-[1,2,4]triazolo[4,3-b]pyridazin-6-yl]amino]butan-1-ol (103)

In a sealed tube 10 - 20 mL with a stir bar was charged **(101)** (0.200 g, 0.46 mmol, 1.0 eq.), 4-pyridylboronic acid (0.119 g, 0.92 mmol, 2.0 eq.), K₂CO₃ (0.191 g, 1.39 mmol, 3.0 eq.) and a mixture of dioxane/water (9/1, 5.0 mL). The mixure was degassed 5 min with inert gas then Pd(PPh₃)₄ (0.053 g, 0.05 mmol, 0.1 eq.) was added, the vial was sealed and put on heating bloc 2.5 h at 110 °C. After cooling, the mixture was directly concentrated with SiO₂ to make solid deposit and purified by flash chromatography with DCM/MeOH (97/3 to 92/8) as eluent to give **(103)** (0.190 g, 95 %) as a yellow solid.

### Example 4.139: Synthesis of (2S)-2-[[3-isopropyl-8-[[4-(3-pyridyl)phenyl]methylamino]-[1,2,4]triazolo[4,3-b]pyridazin-6-yl]amino]butan-1-ol (104)

In a sealed tube 10 - 20 mL with a stir bar was charged **(101)** (0.200 g, 0.46 mmol, 1.0 eq.), 3-pyridylboronic acid (0.119 g, 0.92 mmol, 2.0 eq.), K₂CO₃ (0.191 g, 1.39 mmol, 3.0 eq.) and a mixture of dioxane/water (9/1, 5.0 mL). The mixure was degassed 5 min with inert gas then Pd(PPh₃)₄ (0.053 g, 0.05 mmol, 0.1 eq.) was added, the vial was sealed and put on heating bloc 2.5 h at 110 °C. After cooling, the mixture was directly concentrated with SiO₂ to make solid deposit and purified by flash chromatography with DCM/MeOH (97/3 to 92/8) as eluent to give **(104)** (0.180 g, 90 %) as a yellow solid.

### Example 4.140: Synthesis of N6-(2-ethylbutyl)-3-isopropyl-N8-(pyridazin-3-yl)-[1,2,4]triazolo[4,3-b]pyridazine-6,8-diamine (147)

In a sealed tube 2 - 5 mL with a stir bar was charged **(3.60)** (0.080 g, 0.28 mmol, 1.0 eq.), NMP (1.0 mL) and 2-ethylbutan-1-amine (0.30 mL, 2.21 mmol, 8.0 eq.). The vial was sealed and put on heating block 21 h at 170 °C. After cooling, the mixture was diluted in AcOEt (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 96/4) as eluent. Impur solid product was triturated in HCl 0.5M, filtered off and washed with water up to neutral pH then dried under vacuum to give the title compound **(147)** (0.030 g, 31 %) as a beige solid.

### Example 4.141: Synthesis of 3-isopropyl-N6-(pentan-3-yl)-N8-(pyridazin-3-yl)-[1,2,4]triazolo[4,3-b]pyridazine-6,8-diamine (148)

In a sealed tube 2 - 5 mL with a stir bar was charged **(3.60)** (0.084 g, 4.349 mmol, 1.0 eq.), cineol (0.67 mL) and 3-aminopentane (0.53 mL, 4.349 mmol, 15.0 eq.). The vial was sealed and put on heating block 24h at 170 °C. After cooling, the mixture was diluted in THF and concentrated with SiO₂ to make solid deposit then directly purified by flash chromatography with DCM/MeOH (99/1 to 94/6) as eluent to give **(148)** (0.044 g, 45%) as a white solid.

### Example 4.142: Synthesis of 3-isopropyl-N6-(pentan-3-yl)-N8-(pyridazin-4-yl)-[1,2,4]triazolo[4,3-b]pyridazine-6,8-diamine (149)

In a sealed tube 2 - 5 mL with a stir bar was charged **(3.61)** (0.250 g, 0.863 mmol, 1.0 eq.), cineol (1.99 mL) and 3-aminopentane (1.57 mL, 12.943 mmol, 15.0 eq.). The vial was sealed and put on heating block 73h at 140 °C. After cooling, the mixture was diluted in THF and concentrated with SiO₂ to make solid deposit then directly purified by flash chromatography with DCM/MeOH (99/1 to 94/6) as eluent to give a moiety with (**13**) with an impurity. This moiety was triturated in MeOH, filtered off, washed with MeOH and dried under vacuum to give **(149)** (0.015 g, 5%) as a pale pink solid.

### Example 4.143: Synthesis of 3-isopropyl-N6-(pentan-3-yl)-N8-(pyrimidin-4-yl)-[1,2,4]triazolo[4,3-b]pyridazine-6,8-diamine (150)

In a sealed tube 2 - 5 mL with a stir bar was charged **(3.62)** (0.250 g, 0.863 mmol, 1.0 eq.), cineol (2 mL) and 3-aminopentane (1.57 mL, 12.943 mmol, 15.0 eq.). The vial was sealed and put on heating block 96h at 140°C. After cooling, the mixture was diluted in THF and concentrated with SiO₂ to make solid deposit then directly purified by flash chromatography with DCM/MeOH (99/1 to 94/6) as eluent to give **(150)** (0.060 g, 20%) as a yellow solid.

### Example 4.144: Synthesis of 3-isopropyl-N6-(pentan-3-yl)-N8-(pyrimidin-5-yl)-[1,2,4]triazolo[4,3-b]pyridazine-6,8-diamine (151)

In a sealed tube 2 - 5 mL with a stir bar was charged **(3.63)** (0.197 g, 0.680 mmol, 1.0 eq.), cineol (1.56 mL) and 3-aminopentane (1.24 mL, 10.199 mmol, 15.0 eq.). The vial was sealed and put on heating block 92.3h at 130 °C. After cooling, the mixture was diluted in THF and concentrated with SiO₂ to make solid deposit then directly purified by flash chromatography with EtOAc/MeOH (100/0 to 97/3) as eluent to give a moiety with **(151)** and an impurity. This one was concentrated and the residue was triturated with saturated NaHCO₃, filtered off, washed with NaHCO₃ and H₂O, solubilized with DCM and concentrated. The residue was purified by flash chromatography with DCM/MeOH (95/5) as eluent to give **(151)** (0.019 g, 8 %) as a white solid.

### Example 4.145: Synthesis of 3-isopropyl-N6-(pentan-3-yl)-N8-(pyrimidin-2-yl)-[1,2,4]triazolo[4,3-b]pyridazine-6,8-diamine (152)

In a sealed tube 2 - 5 mL with a stir bar was charged **(3.64)** (0.120 g, 0.414 mmol, 1.0 eq.), cineol (0.95 mL) and 3-aminopentane (0.75 mL, 6.213 mmol, 15.0 eq.). The vial was sealed and put on heating block 21h at 170 °C. After cooling, the mixture was diluted in THF and the precipitate in the mixture was filtered. The filtrate was concentrated with SiO₂ to make solid deposit then directly purified by flash chromatography with DCM/MeOH (99/1 to 94/6) as eluent to give a pure moiety. This one was concentrated and the residue was tritured in Et₂O, filtered off, washed with Et₂O and pentane and dried under vacuum to give **(152)** (0.012 g, 9 %) as a beige solid.

### Example 4.146: Synthesis of 3-isopropyl-N6-(pentan-3-yl)-N8-(pyrazin-2-yl)-[1,2,4]triazolo[4,3-b]pyridazine-6,8-diamine (153)

In a sealed tube 2 - 5 mL with a stir bar was charged **(3.65)** (0.086 g, 0.297 mmol, 1.0 eq.), cineol (0.68 mL) and 3-aminopentane (0.54 mL, 4.452 mmol, 15.0 eq.). The vial was sealed and put on heating block 21h at 170 °C. After cooling, the mixture was diluted in THF and concentrated with SiO₂ to make solid deposit then directly purified by flash chromatography with DCM/MeOH (99/1 to 94/6) as eluent to give **(153)** (0.023 g, 23 %) as a white solid.

### Example 4.147: Synthesis of 3-isopropyl-N8-(6-methoxypyridin-2-yl)-N6-(pentan-3-yl)-[1,2,4]triazolo[4,3-b]pyridazine-6,8-diamine (154)

In a sealed tube 2 - 5 mL with a stir bar was charged **(3.66)** (0.090 g, 0.28 mmol, 1.0 eq.), NMP (1.0 mL) and 3-aminopentane (0.33 mL, 2.82 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 95/5) as eluent to give the title compound **(154)** (0.049 g, 47 %) as a white solid.

### Example 4.148: Synthesis of 3-isopropyl-N8-(5-methoxypyridin-2-yl)-N6-(pentan-3-yl)-[1,2,4]triazolo[4,3-b]pyridazine-6,8-diamine (155)

In a sealed tube 2 - 5 mL with a stir bar was charged **(3.67)** (0.090 g, 0.28 mmol, 1.0 eq.), NMP (1.0 mL) and 3-aminopentane (0.33 mL, 2.82 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 95/5) as eluent to give the title compound **(155)** (0.047 g, 45 %) as a white solid.

### Example 4.149: Synthesis of 3-isopropyl-N8-(4-methoxypyridin-2-yl)-N6-(pentan-3-yl)-[1,2,4]triazolo[4,3-b]pyridazine-6,8-diamine (156)

In a sealed tube 2 - 5 mL with a stir bar was charged **(3.68)** (0.090 g, 0.28 mmol, 1.0 eq.), NMP (1.0 mL) and 3-aminopentane (0.33 mL, 2.82 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 95/5) as eluent to give the title compound **(156)** (0.047 g, 45 %) as a white solid.

### Example 4.150: Synthesis of 3-isopropyl-N8-(3-methoxypyridin-2-yl)-N6-(pentan-3-yl)-[1,2,4]triazolo[4,3-b]pyridazine-6,8-diamine (157)

In a sealed tube 2 - 5 mL with a stir bar was charged **(3.69)** (0.090 g, 0.28 mmol, 1.0 eq.), NMP (1.0 mL) and 3-aminopentane (0.33 mL, 2.82 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 95/5) as eluent to give the title compound **(157)** (0.039 g, 37 %) as a white solid.

### Example 4.151: Synthesis of 3-isopropyl-N8-(2-methoxypyridin-3-yl)-N6-(pentan-3-yl)-[1,2,4]triazolo[4,3-b]pyridazine-6,8-diamine (158)

In a sealed tube with a stir bar was charged **(3.87)** (80 mg, 250.97 µmol, 1.0 eq.), NMP (1.0 mL) and aminopentane (0.32 mL, 2.52 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 94/6) as eluent to give **(158)** (0.016 g, 17 %) as a pale grey solid.

### Example 4.152: Synthesis of 3-isopropyl-N8-(6-methoxypyridin-3-yl)-N6-(pentan-3-yl)-[1,2,4]triazolo[4,3-b]pyridazine-6,8-diamine (159)

In a sealed tube with a stir bar was charged **(3.88)** (80 mg, 250.97 µmol, 1.0 eq.), NMP (1.0 mL) and aminopentane (319 µL, 2.52 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 94/6) as eluent to give **(159)** (0.044 g, 47 %) as a brown viscous oil.

### Example 4.153: Synthesis of 3-isopropyl-N8-(5-methoxypyridin-3-yl)-N6-(pentan-3-yl)-[1,2,4]triazolo[4,3-b]pyridazine-6,8-diamine (160)

In a sealed tube with a stir bar was charged **(3.89)** (80 mg, 250.97 µmol, 1.0 eq.), NMP (1.0 mL) and aminopentane (319 µL, 2.52 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (99/1 to 94/6) as eluent to give **(160)** (0.045 g, 48 %) as a pale beige solid.

### Example 4.154: Synthesis of 3-isopropyl-N8-(4-methoxypyridin-3-yl)-N6-(pentan-3-yl)-[1,2,4]triazolo[4,3-b]pyridazine-6,8-diamine (161)

In a sealed tube with a stir bar was charged **(3.90)** (80 mg, 250.97 µmol, 1.0 eq.), NMP (1.0 mL) and aminopentane (319 µL, 2.52 mmol, 10.0 eq.). The vial was sealed and then put on heating block 17 h at 170 °C. After cooling, the mixture was diluted in EtOAc (20.0 mL) then organic layer was washed three times with sat. NaCl + H₂O (3/1, 3x5.0 mL), dried over MgSO₄, filtered, concentrated and the residue was purified by flash chromatography with DCM/MeOH (98/2) as eluent to give **(161)** (0.006 g, 6 %) as a brown solid.

### Example 4.155: Synthesis of (2R)-2-[[3-isopropyl-8-(2-pyridylamino)-[1,2,4]triazolo[4,3-b]pyridazin-6-yl]amino]butan-1-ol (163)

In a sealed tube 2 - 5 mL with a stir bar was charged **(3.13)** (0.070 g, 0.24 mmol, 1.0 eq.), NMP (1.0 mL) and (*R*)-2-aminobutan-1-ol (0.23 mL, 2.42 mmol, 10.0 eq.). The vial was sealed and put on heating block 64 h at 170 °C. After cooling, the mixture was diluted in AcOEt (25.0 mL) then organic layer was washed three times with sat. NaCl + H₂O (3/1, 3x10.0 mL), dried over MgSO₄, filtered, concentrated and the residue was triturated in DCM, filtered off, washed with DCM and dried under vacuum to give **(163)** (0.052 g, 63 %) as a white solid.

### Example 4.156: Synthesis of N6-(2-ethylbutyl)-3-isopropyl-N8-[2-(2-pyridyl)ethyl]-[1,2,4]triazolo[4,3-b]pyridazine-6,8-diamine (164)

In a sealed tube 2 - 5 mL with a stir bar was charged **(3.44)** (0.060 g, 0.19 mmol, 1.0 eq.), NMP (1.0 mL), corresponding amine hydrochloride (0.165 g, 1.14 mmol, 6.0 eq.) and DIPEA (0.23 mL, 1.33 mmol, 7.0 eq.). The vial was sealed and put on heating block 19 h at 180 °C. After cooling, the mixture was diluted in AcOEt (25.0 mL) then organic layer was washed two times with sat. NaCl + H₂O (2/1, 2x15.0 mL), dried over MgSO₄, filtered, concentrated and the residue was purified by flash chromatography with DCM/MeOH (99/1 to 92/8) as eluent to give **(164)** (0.018 g, 25 %) as a light brown solid.

### Example 4.157: Synthesis of N6-cyclopentyl-3-isopropyl-N8-[2-(2-pyridyl)ethyl]-[1,2,4]triazolo[4,3-b]pyridazine-6,8-diamine (165)

In a sealed tube 2 - 5 mL with a stir bar was charged **(3.44)** (0.060 g, 0.19 mmol, 1.0 eq.), NMP (0.8 mL) and corresponding amine (0.08 mL, 0.79 mmol, 5.0 eq.). The vial was sealed and put on heating block 19 h at 180 °C. After cooling, the mixture was diluted in AcOEt (25.0 mL) then organic layer was washed two times with sat. NaCl + H₂O (2/1, 2x15.0 mL), dried over MgSO₄, filtered, concentrated and the residue was purified by flash chromatography with DCM/MeOH (99/1 to 92/8) as eluent to give **(165)** (0.036 g, 62 %) as a light brown solid.

### Example 4.158: Synthesis of N6-(2-ethylbutyl)-3-isopropyl-N8-[2-(3-pyridyl)ethyl]-[1,2,4]triazolo[4,3-b]pyridazine-6,8-diamine (166)

In a sealed tube 2 - 5 mL with a stir bar was charged **(3.45)** (0.060 g, 0.19 mmol, 1.0 eq.), NMP (1.0 mL), corresponding amine hydrochloride (0.165 g, 1.14 mmol, 6.0 eq.) and DIPEA (0.23 mL, 1.33 mmol, 7.0 eq.). The vial was sealed and put on heating block 19 h at 180 °C. After cooling, the mixture was diluted in AcOEt (25.0 mL) then organic layer was washed two times with sat. NaCl + H₂O (2/1, 2x15.0 mL), dried over MgSO₄, filtered, concentrated and the residue was purified by flash chromatography with DCM/MeOH (99/1 to 92/8) as eluent to give **(166)** (0.029 g, 40 %) as a light brown solid.

### Example 4.159: Synthesis of N6,N8-bis(2-ethylbutyl)-3-isopropyl-[1,2,4]triazolo[4,3-b]pyridazine-6,8-diamine (167)

In a sealed tube 2 - 5 mL with a stir bar was charged **(3.45)** (0.060 g, 0.19 mmol, 1.0 eq.), NMP (1.0 mL), corresponding amine hydrochloride (0.165 g, 1.14 mmol, 6.0 eq.) and DIPEA (0.23 mL, 1.33 mmol, 7.0 eq.). The vial was sealed and put on heating block 19 h at 180 °C. After cooling, the mixture was diluted in AcOEt (25.0 mL) then organic layer was washed two times with sat. NaCl + H₂O (2/1, 2x15.0 mL), dried over MgSO₄, filtered, concentrated and the residue was purified by flash chromatography with DCM/MeOH (99/1 to 92/8) as eluent to give the secondary product **(167)** (0.006 g, 9 %) as a light brown solid.

### Example 4.160: Synthesis of (2R)-2-[[3-isopropyl-8-(2-pyridylmethylamino)imidazo[1,2-b]pyridazin-6-yl]amino]butan-1-ol (169)

In a sealed tube 2 - 5 mL with a stir bar was charged **(3.18)** (0.150 g, 0.47 mmol, 1.0 eq.), NMP (1.0 mL) and (*R*)-2-aminobutan-1-ol (0.45 mL, 4.71 mmol, 10.0 eq.). The vial was sealed and put on heating block 17 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (98/2 to 92/8) to give **(169)** (0.077 g, 46 %) as a white solid.

### Example 4.161: Synthesis of N6-(1-ethylpropyl)-3-isopropyl-N8-(3-phenylpropyl)-[1,2,4]triazolo[4,3-b]pyridazine-6,8-diamine (170)

In a sealed tube 2 - 5 mL with a stir bar was charged **(3.91)** (0.100 g, 0.30 mmol, 1.0 eq.), cineol (1.0 mL) and 3-aminopentane (0.54 mL, 4.55 mmol, 15.0 eq.). The vial was sealed and put on heating block 93 h at 190 °C. After cooling, the mixture was diluted in AcOEt (20.0 mL) then concentrated with SiO₂ to make solid deposit and purified by flash chromatography with DCM/MeOH (98/2 to 96/4) to give **(170)** (0.010 g, 7 %) as a brown oil.

### Example 4.162: Synthesis of 3-cyclopropyl-N6-(1-ethylpropyl)-N8-phenyl-[1,2,4]triazolo[4,3-b]pyridazine-6,8-diamine (171)

In a sealed tube 2 - 5 mL with a stir bar was charged **(3.71)** (0.100 g, 0.35 mmol, 1.0 eq.), cineol (1.0 mL) and 3-aminopentane (0.41 mL, 3.50 mmol, 10.0 eq.). The vial was sealed and put on heating block 70 h at 170 °C. After cooling, the mixture was diluted in THF (20.0 mL) then concentrated with SiO₂ to make solid deposit and purified by flash chromatography with DCM/MeOH (99/1 to 96/4) to give **(171)** (0.015 g, 13 %) as a white solid.

### Example 4.163: Synthesis of 3-cyclopropyl-N6-(1-ethylpropyl)-N8-(2-pyridyl)-[1,2,4]triazolo[4,3-b]pyridazine-6,8-diamine (172)

In a sealed tube 2 - 5 mL with a stir bar was charged **(3.72)** (0.070 g, 0.24 mmol, 1.0 eq.), cineol (1.0 mL) and 3-aminopentane (0.43 mL, 36.62 mmol, 15.0 eq.). The vial was sealed and put on heating block 18 h at 170 °C. After cooling, the mixture was diluted in THF (20.0 mL) then concentrated with SiO₂ to make solid deposit and purified by flash chromatography with DCM/MeOH (99/1 to 95/5) to give **(172)** (0.020 g, 24 %) as a white solid.

### Example 4.164: Synthesis of 3-cyclopropyl-N6-(1-ethylpropyl)-N8-(3-pyridyl)-[1,2,4]triazolo[4,3-b]pyridazine-6,8-diamine (173)

In a sealed tube 2 - 5 mL with a stir bar was charged **(3.73)** (0.070 g, 0.24 mmol, 1.0 eq.), cineol (1.0 mL) and 3-aminopentane (0.43 mL, 36.62 mmol, 15.0 eq.). The vial was sealed and put on heating block 18 h at 170 °C. After cooling, the mixture was diluted in THF (20.0 mL) then concentrated with SiO₂ to make solid deposit and purified by flash chromatography with DCM/MeOH (99/1 to 95/5) to give **(173)** (0.020 g, 24 %) as a white solid.

### Example 4.165: Synthesis of 3-cyclopropyl-N6-(1-ethylpropyl)-N8-(4-pyridyl)-[1,2,4]triazolo[4,3-b]pyridazine-6,8-diamine (174)

In a sealed tube 2 - 5 mL with a stir bar was charged **(3.74)** (0.070 g, 0.24 mmol, 1.0 eq.), cineol (1.0 mL) and 3-aminopentane (0.43 mL, 36.62 mmol, 15.0 eq.). The vial was sealed and put on heating block 18 h at 170 °C. After cooling, the mixture was diluted in THF (20.0 mL) then concentrated with SiO₂ to make solid deposit and purified by flash chromatography with DCM/MeOH (99/1 to 95/5) to give **(174)** (0.015 g, 18 %) as a white solid.

### Example 4.166: Synthesis of 3-cyclopropyl-N6-(pentan-3-yl)-N8-(pyridazin-3-yl)-[1,2,4]triazolo[4,3-b]pyridazine-6,8-diamine (175)

In a sealed tube 2 - 5 mL with a stir bar was charged **(3.75)** (0.075 g, 0.261 mmol, 1.0 eq.), cineol (0.6 mL) and 3-aminopentane (0.47 mL, 3.91 mmol, 15.0 eq.). The vial was sealed and put on heating block 24h at 170 °C. After cooling, the mixture was diluted in THF and the precipitate in the mixture was filtered. The filtrate was concentrated with SiO₂ to make solid deposit then directly purified by flash chromatography with DCM/MeOH (99/1 to 94/6) as eluent to give **(175)** (0.014 g, 16 %) as a white solid.

### Example 4.167: Synthesis of 3-cyclopropyl-N6-(pentan-3-yl)-N8-(pyridazin-4-yl)-[1,2,4]triazolo[4,3-b]pyridazine-6,8-diamine (176)

In a sealed tube 2 - 5 mL with a stir bar was charged **(3.76)** (0.140 g, 0.487 mmol, 1.0 eq.), cineol (1.1 mL) and 3-aminopentane (0.89 mL, 7.299 mmol, 15.0 eq.). The vial was sealed and put on heating block 25.3h at 150 °C. After cooling, the mixture was diluted in THF and the precipitate in the mixture was filtered. The precipitate was solubilised with DCM and MeOH and concentrated. The residue was purified by flash chromatography with DCM/MeOH (95/5 then 94/6) as eluent to give **(176)** (0.003 g, 2%) as a white solid.

### Example 4.168: Synthesis of 3-cyclopropyl-N6-(pentan-3-yl)-N8-(pyrimidin-4-yl)-[1,2,4]triazolo[4,3-b]pyridazine-6,8-diamine (177)

In four sealed tubes 2 - 5 mL with a stir bar was charged **(3.77)** (4x0.150 g, 2.085 mmol, 1.0 eq.), cineol (4x1.2 mL) and 3-aminopentane (4x0.95 mL, 31.281 mmol, 15.0 eq.). The vials were sealed and put on heating block 95h at 150 °C. After cooling, the mixture was diluted in THF and concentrated with SiO₂ to make solid deposit then directly purified by flash chromatography with DCM/MeOH (99/1 to 94/6) as eluent to give **(177)** (0.060 g, 9 %) as a beige solid.

### Example 4.169: Synthesis of 3-cyclopropyl-N6-(1-ethylpropyl)-N8-pyrimidin-5-yl-[1,2,4]triazolo[4,3-b]pyridazine-6,8-diamine (178)

In a sealed tube 2 - 5 mL with a stir bar was charged **(3.78)** (0.132 g, 0.459 mmol, 1.0 eq.), cineol (1.06 mL) and 3-aminopentane (0.84 mL, 6.882 mmol, 15.0 eq.). The vial was sealed and put on heating block 23h at 150 °C. After cooling, the mixture was diluted in THF and concentrated with SiO₂ to make solid deposit then directly purified by flash chromatography with EtOAc/MeOH (100/0 to 98/2) as eluent to give a moiety with **(178)** and an impurity. This moiety was purified by flash chromatography with DCM/MeOH (96/4 to 90/10) as eluent to give **(178)** (0.004 g, 3%) as an orange solid.

### Example 4.170: Synthesis of 3-cyclopropyl-N6-(pentan-3-yl)-N8-(pyrimidin-2-yl)-[1,2,4]triazolo[4,3-b]pyridazine-6,8-diamine (179)

In a sealed tube 2 - 5 mL with a stir bar was charged **(3.79)** (0.090 g, 0.313 mmol, 1.0 eq.), cineol (0.72 mL) and 3-aminopentane (0.57 mL, 4.692 mmol, 15.0 eq.). The vial was sealed and put on heating block 21h at 170 °C. After cooling, the mixture was diluted in THF and the precipitate in the mixture was filtered. The filtrate was concentrated with SiO₂ to make solid deposit then directly purified by flash chromatography with DCM/MeOH (99/1 to 94/6) as eluent to give **(179)** (0.015 g, 14 %) as a white solid.

### Example 4.171: Synthesis of 3-cyclopropyl-N6-(pentan-3-yl)-N8-(pyrazin-2-yl)-[1,2,4]triazolo[4,3-b]pyridazine-6,8-diamine (180)

In a sealed tube 2 - 5 mL with a stir bar was charged **(3.80)** (0.075 g, 0.261 mmol, 1.0 eq.), cineol (0.6 mL) and 3-aminopentane (0.47 mL, 3.91 mmol, 15.0 eq.). The vial was sealed and put on heating block 21h at 170 °C. After cooling, the mixture was diluted in THF and the precipitate in the mixture was filtered. The filtrate was concentrated with SiO₂ to make solid deposit then directly purified by flash chromatography with DCM/MeOH (99/1 to 94/6) as eluent to give **(180)** (0.009 g, 10 %) as a white solid.

### Example 4.172: Synthesis of (R)-2-((3-cyclopropyl-8-(pyridazin-3-ylamino)-[1,2,4]triazolo[4,3-b]pyridazin-6-yl)amino)butan-1-ol (181)

In a sealed tube 2 - 5 mL with a stir bar was charged **(3.75)** (0.100 g, 0.348 mmol, 1.0 eq.), cineol (0.785 mL) and (*R*)-(-)-2-amino-1-butanol (0.50 mL, 3.91 mmol, 15.0 eq.). The vial was sealed and put on heating block 25h at 170 °C. After cooling, the mixture was diluted in THF and the precipitate in the mixture was filtered, washed with H₂O and Et₂O to give (181) (0.011, 9%) as a white solid.

### Example 4.173: Synthesis of (S)-2-((3-cyclopropyl-8-(pyridazin-3-ylamino)-[1,2,4]triazolo[4,3-b]pyridazin-6-yl)amino)butan-1-ol (182)

In a sealed tube 2 - 5 mL with a stir bar was charged **(3.75)** (0.100 g, 0.348 mmol, 1.0 eq.), cineol (0.785 mL) and (*S*)-(+)-2-amino-1-butanol (0.50 mL, 5.214 mmol, 15.0 eq.). The vial was sealed and put on heating block 23h at 170 °C. After cooling, the mixture was diluted in THF and concentrated with SiO₂ to make solid deposit then directly purified by flash chromatography with DCM/MeOH (98/2 to 91/9) as eluent to give a moiety with **(182)** and an impurity which was concentrated. The residue was solubilised with DCM and the white solid in the mixture was filtrated, washed with H₂O and Et₂O to give **(182)** (0.019 g, 16 %) as a white solid.

### Example 4.174: Synthesis of 3-cyclopropyl-N8-(6-methoxypyridin-2-yl)-N6-(pentan-3-yl)-[1,2,4]triazolo[4,3-b]pyridazine-6,8-diamine (183)

In a sealed tube 2 - 5 mL with a stir bar was charged **(3.81)** (0.090 g, 0.28 mmol, 1.0 eq.), NMP (1.0 mL) and 3-aminopentane (0.33 mL, 2.84 mmol, 10.0 eq.). The vial was sealed and put on heating block 20 h at 160 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(183)** (0.045 g, 43 %) as a white solid.

### Example 4.175: Synthesis of 3-cyclopropyl-N8-(2-methoxypyridin-3-yl)-N6-(pentan-3-yl)-[1,2,4]triazolo[4,3-b]pyridazine-6,8-diamine (184)

In a sealed tube 2 - 5 mL with a stir bar was charged **(3.82)** (0.090 g, 0.28 mmol, 1.0 eq.), NMP (1.0 mL) and 3-aminopentane (0.33 mL, 2.84 mmol, 10.0 eq.). The vial was sealed and put on heating block 20 h at 160 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(184)** (0.015 g, 14 %) as a yellow crystalline solid.

### Example 4.176: Synthesis of (2R)-2-[[3-cyclopropyl-8-[2-(2-pyridyl)ethylamino]-[1,2,4]triazolo[4,3-b]pyridazin-6-yl]amino]butan-1-ol (185)

In a sealed tube 2 - 5 mL with a stir bar was charged **(3.47)** (0.090 g, 0.29 mmol, 1.0 eq.), NMP (1.0 mL) and (*R*)-(-)-2-amino-1-butanol (0.28 mL, 2.86 mmol, 10.0 eq.). The vial was sealed and put on heating block 19 h at 170 °C. After cooling, the mixture was diluted in AcOEt (25.0 mL) then organic layer was washed three times with sat. NaCl + H₂O (3/1, 3x10.0 mL), dried over MgSO₄, filtered, concentrated and the residue was purified by flash chromatography with DCM/MeOH (99/1 to 93/7) as eluent to give **(185)** (0.035 g, 33 %) as a light yellow solid.

### Example 4.177: Synthesis of N6-benzyl-3-cyclopropyl-N8-(pyridin-2-ylmethyl)-[1,2,4]triazolo[4,3-b]pyridazine-6,8-diamine (186)

In a sealed tube 2 - 5 mL with a stir bar was charged **(3.24)** (0.100 g, 0.33 mmol, 1.0 eq.), NMP (1.0 mL) and benzylamine (0.22 mL, 2.00 mmol, 6.0 eq.). The vial was sealed and put on heating block 20 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 2x10.0 mL), dried over MgSO₄, filtered, concentrated and the residue was purified by flash chromatography with DCM/MeOH (99/1 to 94/6) as eluent to give **(186)** (0.035 g, 33 %) as a light yellow solid.

### Example 4.178: Synthesis of 3-cyclopropyl-N6-(4-methoxybenzyl)-N8-(pyridin-2-ylmethyl)-[1,2,4]triazolo[4,3-b]pyridazine-6,8-diamine (187)

In a sealed tube 2 - 5 mL with a stir bar was charged **(3.24)** (0.100 g, 0.33 mmol, 1.0 eq.), NMP (1.0 mL) and 4-methoxybenzylamine (0.32 mL, 2.00 mmol, 6.0 eq.). The vial was sealed and put on heating block 17 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 3x10.0 mL), dried over MgSO₄, filtered, concentrated and the residue was purified by flash chromatography with DCM/MeOH (99/1 to 94/6) as eluent to give **(187)** (0.080 g, 60 %) as a white solid.

### Example 4.179: Synthesis of 3-cyclopropyl-N6-(4-methoxybenzyl)-N6-methyl-N8-(pyridin-2-ylmethyl)-[1,2,4]triazolo[4,3-b]pyridazine-6,8-diamine (188)

In a sealed tube 2 - 5 mL with a stir bar was charged **(3.24)** (0.100 g, 0.33 mmol, 1.0 eq.), NMP (1.0 mL) and 1-(4-methoxyphenyl)-N-methylmethanamine (0.308 g, 2.00 mmol, 6.0 eq.). The vial was sealed and put on heating block 17 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 3x10.0 mL), dried over MgSO₄, filtered, concentrated and the residue was purified by flash chromatography with DCM/MeOH (99/1 to 94/6) as eluent to give **(188)** (0.100 g, 72 %) as a white solid.

### Example 4.180: Synthesis of 3-cyclopropyl-N6-ethyl-N6-(4-methoxybenzyl)-N8-(pyridin-2-ylmethyl)-[1,2,4]triazolo[4,3-b]pyridazine-6,8-diamine (4.180)

In a sealed tube 2 - 5 mL with a stir bar was charged **(3.24)** (0.100 g, 0.33 mmol, 1.0 eq.), NMP (1.0 mL) and N-(4-methoxybenzyl)ethanamine (0.340 g, 2.00 mmol, 6.0 eq.). The vial was sealed and put on heating block 17 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 3x10.0 mL), dried over MgSO₄, filtered, concentrated and the residue was purified by flash chromatography with DCM/MeOH (99/1 to 94/6) as eluent to give **(4.180)** (0.030 g, 21 %) as a white solid.

**¹H NMR (400 MHz, DMSO-*d₆*) δ** : 0.98 (d, *J* = 6.9 Hz, 3H, CH_{3-CH2}), 1.07 (ddt, *J* = 26.3, 6.0, 2.9 Hz, 4H, 2xCH_{2cPr}), 2.23 - 2.31 (m, 1H, H_{cPr}), 3.39 (q, *J* = 6.9 Hz, 2H, CH_{2-CH3}), 3.71 (s, 3H, OCH₃), 4.50 (s, 2H, CH_{2PMB}), 4.57 (d, *J* = 6.0 Hz, 2H, CH_{2Bn}), 5.62 (s, 1H, H_{Ar}), 6.77 - 6.86 (m, 2H, 2xH_{Ar}), 7.10 - 7.18 (m, 2H, 2xH_{Ar}), 7.22 - 7.28 (m, 1H, H_{Ar}), 7.30 (d, *J* = 7.8 Hz, 1H, H_{Ar}), 7.72 (td, *J* = 7.7, 1.8 Hz, 1H, H_{Ar}), 7.99 (t, *J* = 6.1 Hz, 1H, NH), 8.47 (d, *J* = 4.8 Hz, 1H, H_{Ar}).

**¹³C NMR (101 MHz, DMSO-*d₆*) δ :** 5.3 (CH_{cPr}), 6.4 (2xCH_{2cPr}), 12.4 (CH_{3-CH2}), 42.9 (CH_{2-CH3}), 47.3 (CH_{2Bn}), 50.3 (CH_{2PMB}), 55.0 (OCH₃), 82.4 (CH_{Ar}), 113.7 (2xCH_{Ar}), 121.3 (CH_{Ar}), 122.3 (CH_{Ar}), 128.5 (2xCH_{Ar}), 130.6 (C_{q}), 136.9 (CH_{Ar}), 138.9 (C_{q}), 140.5 (C_{q}), 148.9 (CH_{Ar}), 149.9 (C_{q}), 155.6 (C_{q}), 157.6 (C_{q}), 158.2 (C_{q}).

**MS (ESI+) : m/z calcd for C₂₄H₂₇N₇O** : 430.23 [M+H]+, **found** : 430.42.

### Example 4.181: Synthesis of 3-cyclopropyl-N6-methyl-N8-(pyridin-2-ylmethyl)-[1,2,4]triazolo[4,3-b]pyridazine-6,8-diamine (219)

A solution of **(188)** (0.090 g, 0.22 mmol, 1.0 eq.) in DCM/TFA (9.0/3.0 mL) was stired 1 h at room temperature. After completion, solvent was removed, the residu was taken up in DCM, washed with sat. Na₂CO₃ (10.0 mL) then aqueous layer was extracted twice wih DCM (2x5.0 mL). Organics layers were combined, dired over MgSO₄, filtered, concentrated and the residu was purified by flash chromatography with DCM/MeOH (99/1 to 94/6) as eluent to give **(219)** (0.058 g, 91 %) as a white solid.

### Example 4.182: Synthesis of 3-cyclopropyl-N6-ethyl-N8-(2-pyridylmethyl)-[1,2,4]triazolo[4,3-b]pyridazine-6,8-diamine (189)

A solution of **(4.180)** (0.020 g, 0.05 mmol, 1.0 eq.) in DCM/TFA (5.0/1.5 mL) was stired 1 h at room temperature. After completion, solvent was removed, the residu was taken up in DCM, washed with sat. Na₂CO₃ (10.0 mL) then aqueous layer was extracted twice wih DCM (2x5.0 mL). Organics layers were combined, dired over MgSO₄, filtered, concentrated and the residu was purified by flash chromatography with DCM/MeOH (99/1 to 94/6) as eluent to give **(189)** (0.011 g, 76 %) as a white solid.

### Example 4.183: Synthesis of 3-cyclopropyl-N6-propyl-N8-(pyridin-2-ylmethyl)-[1,2,4]triazolo[4,3-b]pyridazine-6,8-diamine (190)

In a sealed tube 2 - 5 mL with a stir bar was charged **(3.24)** (0.130 g, 0.43 mmol, 1.0 eq.), NMP (1.0 mL) and corresponding amine (0.36 mL, 4.32 mmol, 10.0 eq.). The vial was sealed and put on heating block 22 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 2x10.0 mL), dried over MgSO₄, filtered, concentrated and the residue was purified by flash chromatography with DCM/MeOH (99/1 to 94/6) as eluent to give **(190)** (0.035 g, 33 %) as a light yellow solid.

### Example 4.184: Synthesis of N6-butyl-3-cyclopropyl-N8-(pyridin-2-ylmethyl)-[1,2,4]triazolo[4,3-b]pyridazine-6,8-diamine (191)

In a sealed tube 2 - 5 mL with a stir bar was charged **(3.24)** (0.130 g, 0.43 mmol, 1.0 eq.), NMP (1.0 mL) and corresponding amine (0.43 mL, 4.32 mmol, 10.0 eq.). The vial was sealed and put on heating block 22 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 2x10.0 mL), dried over MgSO₄, filtered, concentrated and the residue was purified by flash chromatography with DCM/MeOH (99/1 to 94/6) as eluent to give **(191)** (0.055 g, 38 %) as a white solid.

### Example 4.185: Synthesis of 3-cyclopropyl-N6-isopropyl-N8-(pyridin-2-ylmethyl)-[1,2,4]triazolo[4,3-b]pyridazine-6,8-diamine (192)

In a sealed tube 2 - 5 mL with a stir bar was charged **(3.24)** (0.130 g, 0.43 mmol, 1.0 eq.), NMP (1.0 mL) and corresponding amine (0.37 mL, 4.32 mmol, 10.0 eq.). The vial was sealed and put on heating block 48 h at 160 °C. After cooling, the mixture was diluted in AcOEt (20.0 mL) then organic layer was washed with water (20.0 mL). Aqueous layer was extracted twice with EtOAc (2x10.0 mL). Organics layers were combined, washed two times with sat. NaCl + H₂O (3/1, 2x10.0 mL), dried over MgSO₄, filtered, concentrated and the residue was purified by flash chromatography with DCM/MeOH (99/1 to 94/6) as eluent to give **(192)** (0.075 g, 54 %) as a white solid.

### Example 4.186: Synthesis of N6-(sec-butyl)-3-cyclopropyl-N8-(pyridin-2-ylmethyl)-[1,2,4]triazolo[4,3-b]pyridazine-6,8-diamine (193)

In a sealed tube 2 - 5 mL with a stir bar was charged **(3.24)** (0.130 g, 0.43 mmol, 1.0 eq.), NMP (1.0 mL) and corresponding amine (0.46 mL, 3.66 mmol, 10.0 eq.). The vial was sealed and then put on heating block, 24 h at 180 °C. After cooling, the mixture was diluted in EtOAc (20.0 mL) and organic layer was washed three times with a mixture brine/water (3/1, 3x8 mL). Organic layer was dried over MgSO₄, filtered, concentrated and purified by flash chromatography with DCM/MeOH (99/1 to 94/6) as eluent to give **(193)** (0.060 g, 41 %) as a white solid.

### Example 4.187: Synthesis of 3-cyclopropyl-N6-isobutyl-N8-(pyridin-2-ylmethyl)-[1,2,4]triazolo[4,3-b]pyridazine-6,8-diamine (194)

In a sealed tube 2 - 5 mL with a stir bar was charged **(3.24)** (0.110 g, 0.37 mmol, 1.0 eq.), NMP (1.0 mL) and corresponding amine (0.268 g, 3.66 mmol, 10.0 eq.). The vial was sealed and put on heating block 20 h 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(194)** (0.055 g, 45 %) as a white solid.

### Example 4.188: Synthesis of N6-(tert-butyl)-3-cyclopropyl-N8-(pyridin-2-ylmethyl)-[1,2,4]triazolo[4,3-b]pyridazine-6,8-diamine (195)

In a sealed tube 2 - 5 mL with a stir bar was charged **(3.24)** (0.110 g, 0.37 mmol, 1.0 eq.), NMP (1.0 mL) and corresponding amine (0.39 mL, 3.66 mmol, 10.0 eq.). The vial was sealed and put on heating block 65 h at 160 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give (195) (0.006 g, 5 %) as light brown solid.

### Example 4.189: Synthesis of 3-cyclopropyl-N6-(2-ethylbutyl)-N8-(pyridin-2-ylmethyl)-[1,2,4]triazolo[4,3-b]pyridazine-6,8-diamine (196)

In a sealed tube 2 - 5 mL with a stir bar was charged **(3.24)** (0.110 g, 0.37 mmol, 1.0 eq.), NMP (1.0 mL), corresponding amine hydrochloride (0.268 g, 1.83 mmol, 5.0 eq.) and DIPEA (0.38 mL, 2.19 mmol, 6.0 eq.). The vial was sealed and put on heating block 20 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(196)** (0.036 g, 27 %) as a white solid.

### Example 4.190: Synthesis of N6,3-dicyclopropyl-N8-(pyridin-2-ylmethyl)-[1,2,4]triazolo[4,3-b]pyridazine-6,8-diamine (197)

In a sealed tube 2 - 5 mL with a stir bar was charged **(3.24)** (0.130 g, 0.43 mmol, 1.0 eq.), NMP (1.0 mL) and corresponding amine (0.30 mL, 4.32 mmol, 10.0 eq.). The vial was sealed and put on heating block 48 h at 160 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(197)** (0.020 g, 14 %) as a white solid.

### Example 4.191: Synthesis of N6-cyclobutyl-3-cyclopropyl-N8-(pyridin-2-ylmethyl)-[1,2,4]triazolo[4,3-b]pyridazine-6,8-diamine (198)

In a sealed tube 2 - 5 mL with a stir bar was charged **(3.24)** (0.130 g, 0.43 mmol, 1.0 eq.), NMP (1.0 mL) and corresponding amine (0.37 mL, 4.32 mmol, 10.0 eq.). The vial was sealed and put on heating block 20 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(198)** (0.045 g, 32 %) as a white solid.

### Example 4.192: Synthesis of N6-cyclopentyl-3-cyclopropyl-N8-(pyridin-2-ylmethyl)-[1,2,4]triazolo[4,3-b]pyridazine-6,8-diamine (199)

In a sealed tube 2 - 5 mL with a stir bar was charged **(3.24)** (0.110 g, 0.37 mmol, 1.0 eq.), NMP (1.0 mL) and corresponding amine (0.36 mL, 3.66 mmol, 10.0 eq.). The vial was sealed and put on heating block 18 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **199** (0.057 g, 45 %) as a white solid.

### Example 4.193: Synthesis of N6-cyclohexyl-3-cyclopropyl-N8-(pyridin-2-ylmethyl)-[1,2,4]triazolo[4,3-b]pyridazine-6,8-diamine (200)

In a sealed tube 2 - 5 mL with a stir bar was charged **(3.24)** (0.110 g, 0.37 mmol, 1.0 eq.), NMP (1.0 mL) and corresponding amine (0.42 mL, 3.66 mmol, 10.0 eq.). The vial was sealed and put on heating block 18 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(200)** (0.075 g, 56 %) as a white solid.

### Example 4.194: Synthesis of N6-cycloheptyl-3-cyclopropyl-N8-(pyridin-2-ylmethyl)-[1,2,4]triazolo[4,3-b]pyridazine-6,8-diamine (201)

In a sealed tube 2 - 5 mL with a stir bar was charged **(3.24)** (0.110 g, 0.37 mmol, 1.0 eq.), NMP (1.0 mL) and corresponding amine (0.47 mL, 3.66 mmol, 10.0 eq.). The vial was sealed and put on heating block 18 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(201)** (0.049 g, 35 %) as a white solid.

### Example 4.195: Synthesis of N6-((3s,5s,7s)-adamantan-1-yl)-3-cyclopropyl-N8-(pyridin-2-ylmethyl)-[1,2,4]triazolo[4,3-b]pyridazine-6,8-diamine (202)

In a sealed tube 2 - 5 mL with a stir bar was charged **(3.24)** (0.110 g, 0.37 mmol, 1.0 eq.), NMP (1.0 mL) and corresponding amine (0.570 g, 3.66 mmol, 10.0 eq.). The vial was sealed and then put on heating block, 16 h at 170 °C. After cooling, the mixture was diluted in EtOAc (20.0 mL) and organic layer was washed three times with a mixture brine/water (3/1, 3x8 mL). Organic layer was dried over MgSO₄, filtered, concentrated and purified by flash chromatography with DCM/MeOH (99/1 to 94/6) as eluent to give **(202)** (0.012 g, 8 %) as a white solid.

### Example 4.196: Synthesis of 3-cyclopropyl-N6-(cyclopropylmethyl)-N8-(pyridin-2-ylmethyl)-[1,2,4]triazolo[4,3-b]pyridazine-6,8-diamine (203)

In a sealed tube 2 - 5 mL with a stir bar was charged **(3.24)** (0.100 g, 0.33 mmol, 1.0 eq.), NMP (1.0 mL) and corresponding amine (0.29 mL, 3.32 mmol, 10.0 eq.). The vial was sealed and put on heating block 20 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(203)** (0.020 g, 14 %) as a white solid.

### Example 4.197: Synthesis of N6-(cyclobutylmethyl)-3-cyclopropyl-N8-(pyridin-2-ylmethyl)-[1,2,4]triazolo[4,3-b]pyridazine-6,8-diamine (204)

In a sealed tube 2 - 5 mL with a stir bar was charged **(3.24)** (0.110 g, 0.37 mmol, 1.0 eq.), NMP (1.0 mL) and corresponding amine (0.321 g, 3.32 mmol, 10.0 eq.). The vial was sealed and put on heating block 18 h at 180 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(204)** (0.030 g, 23 %) as a white solid.

### Example 4.198: Synthesis of 3-cyclopropyl-N8-(pyridin-2-ylmethyl)-N6-(spiro[3.3]heptan-2-yl)-[1,2,4]triazolo[4,3-b]pyridazine-6,8-diamine (205)

In a sealed tube 2 - 5 mL with a stir bar was charged **(3.24)** (0.110 g, 0.37 mmol, 1.0 eq.), NMP (1.0 mL), corresponding amine hydrochloride (0.284 g, 1.83 mmol, 5.0 eq.) and DIPEA (0.38 mL, 2.19 mmol, 6.0 eq.). The vial was sealed and put on heating block 65 h at 160 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(205)** (0.013 g, 9 %) as a white solid.

### Example 4.199: Synthesis of 3-cyclopropyl-N6-((1-methylcyclobutyl)methyl)-N8-(pyridin-2-ylmethyl)-[1,2,4]triazolo[4,3-b]pyridazine-6,8-diamine (206)

In a sealed tube 2 - 5 mL with a stir bar was charged **(3.24)** (0.130 g, 0.43 mmol, 1.0 eq.), NMP (1.0 mL), corresponding amine hydrochloride (0.359 g, 2.59 mmol, 6.0 eq.) and DIPEA (0.53 mL, 3.03 mmol, 7.0 eq.). The vial was sealed and put on heating block 24 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(206)** (0.035 g, 22 %) as a white solid.

### Example 4.200: Synthesis of 3-cyclopropyl-N-(pyridin-2-ylmethyl)-6-(pyrrolidin-1-yl)-[1,2,4]triazolo[4,3-b]pyridazin-8-amine (220)

In a sealed tube 2 - 5 mL with a stir bar was charged **(3.24)** (0.080 g, 0.27 mmol, 1.0 eq.), NMP (0.8 mL) and corresponding amine (0.23 mL, 2.66 mmol, 10.0 eq.). The vial was sealed and then put on heating block, 3 h at 180 °C. After cooling, the mixture was poured on sat. NaHCO₃ (5.0 mL) and water (15.0 mL) and stired 5 min. The precipitate was filtered off, washed with water then MeOH and Et₂O. The solid was dried under vacuum to give **(220)** (0.070 g, 78 %) as a white solid.

### Example 4.201: Synthesis of 3-cyclopropyl-6-(1-piperidyl)-N-(2-pyridylmethyl)-[1,2,4]triazolo[4,3-b]pyridazin-8-amine (207)

In a sealed tube 2 - 5 mL with a stir bar was charged **(3.24)** (0.130 g, 0.33 mmol, 1.0 eq.), NMP (1.0 mL) and corresponding amine (0.0.33 mL, 3.33 mmol, 10.0 eq.). The vial was sealed and then put on heating block, 3 h at 180 °C. The reaction mixture was cooled and water (6.0 mL) was added. The precipitate was filtered, washed with water up to neutral pH then the solid was taken up in DCM. Organic filtrate was dried over MgSO4, filtered and concentrated to give **(207)** (0.090 g, 77 %) as a light grey solid.

### Example 4.202: Synthesis of 3-cyclopropyl-N6-(furan-2-ylmethyl)-N8-(pyridin-2-ylmethyl)-[1,2,4]triazolo[4,3-b]pyridazine-6,8-diamine (208)

In a sealed tube 2 - 5 mL with a stir bar was charged **(3.24)** (0.110 g, 0.37 mmol, 1.0 eq.), NMP (1.0 mL) and corresponding amine (0.299 g, 2.93 mmol, 8.0 eq.). The vial was sealed and put on heating block 21 h at 170 °C. After cooling, the mixture was diluted in AcOEt (20.0 mL) then organic layer was washed with water (20.0 mL). Aqueous layer was extracted twice with EtOAc (2x10.0 mL). Organics layers were combined, washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give (208) (0.045 g, 34 %) as a white solid.

### Example 4.203: Synthesis of 3-cyclopropyl-N6-(furan-3-ylmethyl)-N8-(pyridin-2-ylmethyl)-[1,2,4]triazolo[4,3-b]pyridazine-6,8-diamine (209)

In a sealed tube 2 - 5 mL with a stir bar was charged **(3.24)** (0.110 g, 0.37 mmol, 1.0 eq.), NMP (1.0 mL) and corresponding amine (0.299 g, 2.93 mmol, 8.0 eq.). The vial was sealed and put on heating block 21 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(209)** (0.042 g, 32 %) as a white solid.

### Example 4.204: Synthesis of 3-cyclopropyl-N8-(pyridin-2-ylmethyl)-N6-(thiophen-2-ylmethyl)-[1,2,4]triazolo[4,3-b]pyridazine-6,8-diamine (210)

In a sealed tube 2 - 5 mL with a stir bar was charged **(3.24)** (0.110 g, 0.37 mmol, 1.0 eq.), NMP (1.0 mL) and corresponding amine (0.349 g, 2.93 mmol, 8.0 eq.). The vial was sealed and put on heating block 21 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(210)** (0.050 g, 36 %) as a white solid.

### Example 4.205: Synthesis of (R)-2-((3-cyclopropyl-8-((pyridin-2-ylmethyl)amino)-[1,2,4]triazolo[4,3-b]pyridazin-6-yl)amino)butan-1-ol (211)

In a sealed tube 2 - 5 mL with a stir bar was charged **(3.24)** (0.110 g, 0.37 mmol, 1.0 eq.), NMP (1.0 mL) and corresponding amine (0.35 mL, 3.66 mmol, 10.0 eq.). The vial was sealed and put on heating block 18 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(211)** (0.035 g, 27 %) as a light yellow solid.

### Example 4.206: Synthesis of (S)-2-((3-cyclopropyl-8-((pyridin-2-ylmethyl)amino)-[1,2,4]triazolo[4,3-b]pyridazin-6-yl)amino)butan-1-ol (212)

In a sealed tube 2 - 5 mL with a stir bar was charged **(3.24)** (0.110 g, 0.37 mmol, 1.0 eq.), NMP (1.0 mL) and corresponding amine (0.35 mL, 3.66 mmol, 10.0 eq.). The vial was sealed and put on heating block 18 h at 180 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(212)** (0.055 g, 43 %) as a white solid.

### Example 4.207: Synthesis of (R)-3-cyclopropyl-N8-(pyridin-2-ylmethyl)-N6-(tetrahydrofuran -3-yl)-[1,2,4]triazolo[4,3-b]pyridazine-6,8-diamine (213)

In a sealed tube 2 - 5 mL with a stir bar was charged **(3.24)** (0.110 g, 0.37 mmol, 1.0 eq.), NMP (1.0 mL) and corresponding amine (0.31 mL, 3.66 mmol, 10.0 eq.). The vial was sealed and put on heating block 16 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(213)** (0.055 g, 43 %) as a white solid.

### Example 4.208: Synthesis of (R)-3-cyclopropyl-N8-(pyridin-2-ylmethyl)-N6-(tetrahydro-2H-pyran-3-yl)-[1,2,4]triazolo[4,3-b]pyridazine-6,8-diamine (214)

In a sealed tube 2 - 5 mL with a stir bar was charged **(3.24)** (0.110 g, 0.37 mmol, 1.0 eq.), NMP (1.0 mL), corresponding amine hydrochloride (0.403 g, 2.93 mmol, 8.0 eq.) and DIPEA (0.58 mL, 3.29 mmol, 9.0 eq.). The vial was sealed and put on heating block 16 h at 170 °C. After cooling, the mixture was poured onto cold water (25.0 mL), stired 10 min then the precipitate was filtered off, washed with cold water and taken up in DCM/MeOH (99/1). Organic filtrate was dried over MgSO₄, filtered, concentrated and the residue was purified by flash chromatography with DCM/MeOH (99/1 to 94/6) as eluent to give **(214)** (0.008 g, 6 %) as a white solid.

### Example 4.209: Synthesis of 3-cyclopropyl-N8-(6-methoxypyridazin-3-yl)-N6-(pentan-3-yl)-[1,2,4]triazolo[4,3-b]pyridazine-6,8-diamine (215)

In a sealed tube 2 - 5 mL with a stir bar was charged **(3.84)** (0.080 g, 0.25 mmol, 1.0 eq.), NMP (1.0 mL) and corresponding amine (0.30 mL, 2.52 mmol, 10.0 eq.). The vial was sealed and put on heating block 18 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(215)** (0.026 g, 28 %) as a beige solid.

### Example 4.210: Synthesis of 3-cyclopropyl-N6-(pentan-3-yl)-N8-(pyridazin-3-ylmethyl)-[1,2,4]triazolo[4,3-b]pyridazine-6,8-diamine (216)

In a sealed tube 2 - 5 mL with a stir bar was charged **(3.85)** (0.065 g, 0.21 mmol, 1.0 eq.), NMP (1.0 mL) and corresponding amine (0.25 mL, 2.15 mmol, 10.0 eq.). The vial was sealed and put on heating block 15 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(216)** (0.013 g, 17 %) as a light brown solid.

### Example 4.211: Synthesis of 3-cyclopropyl-N6-(pentan-3-yl)-N8-phenethyl-[1,2,4]triazolo[4,3-b]pyridazine-6,8-diamine (217)

In a sealed tube 2 - 5 mL with a stir bar was charged **(3.86)** (0.100 g, 0.32 mmol, 1.0 eq.), NMP (1.0 mL) and corresponding amine (0.38 mL, 3.19 mmol, 10.0 eq.). The vial was sealed and put on heating block 15 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 97/3) as eluent to give **(217)** (0.013 g, 17 %) as a white cloudy oil.

### Example 4.212: Synthesis of 3-cyclopropyl-N8-(pyridin-2-ylmethyl)-N6-(thiophen-3-ylmethyl)-[1,2,4]triazolo[4,3-b]pyridazine-6,8-diamine (218)

In a sealed tube 2 - 5 mL with a stir bar was charged **(3.24)** (0.110 g, 0.37 mmol, 1.0 eq.), NMP (1.0 mL) and corresponding amine (0.349 g, 2.93 mmol, 8.0 eq.). The vial was sealed and put on heating block 21 h at 170 °C. After cooling, the mixture was diluted in AcOEt (20.0 mL) then organic layer was washed three times with sat. NaCl + H₂O (3/1, 3x8.0 mL), dried over MgSO₄, filtered, concentrated and the residue was purified by flash chromatography with DCM/MeOH (99/1 to 94/6) as eluent to give **(218)** (0.055 g, 40 %) as a white solid.

### Example 5: 8-amino-[1,2,4]triazolo[4,3-b]pyridazine-6-amine derivatives as illustrated in steps 1 and 2 of scheme 4

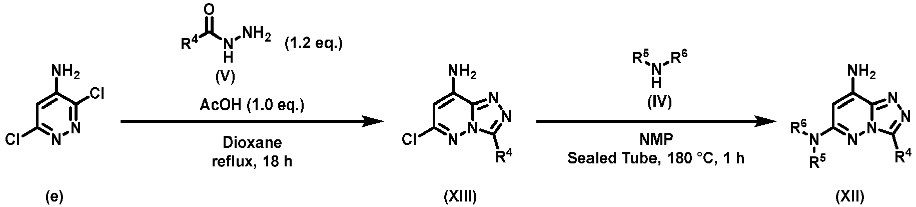

### Example 5.1: Synthesis of 6-chloro-3-isopropyl-[1,2,4]triazolo[4,3-b]pyridazin-8-amine (5.1)

To a solution of 3,6-dichloropyridazin-4-amine (3.600 g, 20.85 mmol, 1.0 eq.) in dioxane (84.0 mL), isobutyric acid hydrazide (2.582 g, 25.03 mmol, 1.2 eq.) and AcOH (1.20 mL, 20.85 mmol, 1.0 eq.) was added and refluxed during 18 h. The reaction mixture was concentrated and the resulting crude was triturated in water (100 mL), the solid was filtered off, washed with water until pH = 7 then Et₂O and dried under vacuum to give **(5.1)** (2.100 g, 48 %) as a white solid.
**Rf** (DCM/MeOH, 92/8) : 0.33

**¹H NMR (400 MHz, DMSO-*d₆*) δ :** 1.38 (d, *J* = 6.9 Hz, 6H, 2xCH_{3iPr}), 3.42 (hept, *J* = 7.0 Hz, 1H, HiPr), 6.11 (s, 1H, H_{Ar}), 7.89 (s, 2H, NH₂).

¹³C **NMR (101 MHz, DMSO-*d₆*) δ :** 19.8 (2xCH_{3iPr}), 24.5 (CH_{iPr}), 93.7 (CH_{Ar}), 139.6 (C_{q}), 143.9 (C_{q}), 149.2 (C_{q}), 153.9 (C_{q}).

**MS (ESI+) : m/z calcd for C₈H₁₀ClN₅ :** 212.1 [M+H]+, **found** : 212.1.

### Example 5.2: Synthesis of 6-chloro-3-cyclopropyl-[1,2,4]triazolo[4,3-b]pyridazin-8-amine (5.2)

To a solution of 3,6-dichloropyridazin-4-amine (3.800 g, 22.01 mmol, 1.0 eq.) in dioxane (110.0 mL), cyclopropanecarbohydrazide (2.784 g, 26.42 mmol, 1.2 eq.) and AcOH (1.27 mL, 22.01 mmol, 1.0 eq.) was added and refluxed during 24 h. The reaction mixture was concentrated and the resulting crude was triturated in water (150 mL), the solid was filtered off, washed with water until pH = 7 then the solid was taken up in MeOH and concentrated to remove water. The residue was purified by flash chromatography with DCM/MeOH (97/3 to 96/4) as eluent to give **(5.2)** with cyclopropanoic acid impurity. The resulting solid was triturated saturated NaHCO₃ to remove a maximum of acid, washed with water then Et₂O and dried under vacuum to give pure **(5.2)** (2.398 g, 52 %) as a white solid.
**Rf** (DCM/MeOH, 96/4) : 0.27

**¹H NMR (400 MHz, DMSO-*d₆*) δ :** 1.04 - 1.18 (m, 4H, 2xCH_{2cPr}), 2.31 (tt, *J* = 8.1, 5.3 Hz, 1H, H_{cPr}), 6.10 (s, 1H, H_{Ar}), 7.86 (s, 2H, NH₂).

**¹³C NMR (101 MHz, DMSO-*d₆*) δ :** 4.9 (CH_{cPr}), 7.2 (2xCH_{2cPr}), 93.7 (CH_{Ar}), 139.5 (C_{q}), 143.9 (C_{q}), 149.4 (C_{q}), 151.3 (C_{q}).

### Example 5.3: Synthesis of (2R)-2-[(8-amino-3-isopropyl-[1,2,4]triazolo[4,3-b]pyridazin-6-yl)amino]butan-1-ol (5.3) (illustrative intermediate compound (5.3), not reused in the following examples for preparing compounds of formula (I))

In a sealed tube 2 - 5 mL with a stir bar was charged **(5.1)** (0.100 g, 0.47 mmol, 1.0 eq.) and (R)-(-)-2-amino-1-butanol (1.34 mL, 14.17 mmol, 30.0 eq.). The vial was sealed and then put on heating block 22 h at 150 °C. After cooling, the mixture was diluted in EtOAc (15 mL) and organic layer was washed with water (2 x 30 mL). Aqueous layers were extracted twice with EtOAc (2 x 15 mL) then organic layers were combined, dried over Na₂SO₄, filtered, concentrated and purified by flash chromatography with DCM/MeOH (96/4 to 92/8) as eluent to give **(5.3)** (0.027 g, 22 %) as a pale yellow solid.

**¹H NMR (400 MHz, DMSO-*d₆*) δ :** 0.88 (t, *J* = 7.4 Hz, 3H, CH_{3-CH2}), 1.35 (d, *J* = 6.7 Hz, 6H, 2xCH_{3 i}pᵣ), 1.54 (ddt, *J* = 85.7, 14.1, 7.2 Hz, 2H, CH_{2-CH3}), 3.25 - 3.33 (m, 1H, H_{iPr}), 3.44 (dh, *J* = 22.0, 5.4 Hz, 2H, CH_{2-OH}), 3.66 (dq, *J* = 12.4, 6.7, 6.0 Hz, 1H, H_{Alk}), 4.58 (t, *J* = 5.4 Hz, 1H, OH), 5.64 (s, 1H, H_{Ar}), 6.29 (d, *J* = 7.8 Hz, 1H, NH), 6.61 (s, 2H, NH₂).

**¹³C NMR (101 MHz, DMSO-*d₆*) δ** : 10.6 (CH_{3-CH2}), 19.6 (2xCH_{3 iPr}), 23.6 (CH_{2-CH3}), 24.8 (CH _{iPr}), 53.8 (CH_{Alk}), 62.2 (CH_{2-OH}), 86.6 (CH_{Ar}), 140.0 (C_{q}), 140.8 (C_{q}), 152.6 (C_{q}), 156.1 (C_{q}).

**MS (ESI+)** : **m/z calcd for C₁₂H₂₀N₆O** : 265.18 [M+H]+, **found** : 265.24.

### Example 5.4: Synthesis of (2R)-2-[[6-[[(1R)-1-(hydroxymethyl)propyl]amino]-3-isopropyl-[1,2,4]triazolo[4,3-b]pyridazin-8-yl]amino]butan-1-ol (113)

In a sealed tube 2-5 mL with a stir bar was charged **(5.1)** (0.150 g, 0.71 mmol, 1.0 eq.), NMP (1.0 mL) and (*R*)-(-)-2-amino-1-butanol (1.03 mL, 10.63 mmol, 15.0 eq.). The vial was sealed and then put on heating block 22 h at 170 °C. After cooling, the mixture was diluted in EtOAc (15.0 mL), organic layer was washed with a mixture of brine/water (3/1, 3x20.0 mL), dried over Na₂SO₄, filtered, concentrated and purified by flash chromatography with DCM/MeOH (96/4 to 92/8) as eluent to give the secondary compound **(113)** (0.018 g, 8 %) as a white solid.

### Example 5.5: Synthesis of 3-isopropyl-N6-(3-methoxypropyl)-[1,2,4]triazolo[4,3-b]pyridazine-6,8-diamine (5.5) (illustrative intermediate compound (5.5), not reused in the following examples for preparing compounds of formula (I))

In a sealed tube 2-5 mL with a stir bar was charged **(5.1)** (0.150 g, 0.71 mmol, 1.0 eq.), NMP (1.0 mL) and 3-methoxypropylamine (0.98 mL, 10.63 mmol, 15.0 eq.). The vial was sealed and then put on heating block 5 h at 170 °C. After cooling, the mixture was diluted in EtOAc (15 mL) and organic layer was washed three times with a mixture brine/water (3/1, 3x20 mL). Organic layer was dried over Na₂SO₄, filtered, concentrated and purified by flash chromatography with DCM/MeOH (96/4 to 92/8) as eluent to give **(5.5)** (0.087 g, 46 %) as a white solid.
**Rf** (DCM/MeOH, 92/8) : 0.18

**¹H NMR (400 MHz, DMSO-*d₆*) δ :** 1.36 (d, *J* = 7.0 Hz, 6H, 2xCH_{3 iPr}), 1.78 (p, *J* = 6.6 Hz, 2H, CH_{2-CH2-NH}), 3.19 (dd, *J* = 6.9, 5.4 Hz, 2H, CH_{2-NH}), 3.23 (s, 3H, OCH₃), 3.32 (p, *J* = 6.9 Hz, 1H, H_{iPr}), 3.39 (t, *J* = 6.3 Hz, 2H, CH_{2-OCH3}), 5.58 (s, 1H, H_{Ar}), 6.57 (t, *J* = 5.4 Hz, 1H, NH), 6.65 (s, 2H, NH₂).

**¹³C NMR (101 MHz, DMSO-*d₆*) δ :** 19.6 (2xCH_{3 iPr}), 24.7 (CH_{iPr}), 28.6(CH_{2-CH2-NH}), 37.9 (CH_{2-NH}), 57.8 (OCH₃), 69.8 (CH_{2-OCH3}), 86.2 (CH_{Ar}), 140.0 (C_{q}), 140.9 (C_{q}), 152.7 (C_{q}), 156.1 (C_{q}).

**MS (ESI+)** : **m/z calcd for C₁₂H₂₀N₆O** : 265.18 [M+H]+, **found** : 265.19.

### Example 5.6: Synthesis of N6-(1-ethylpropyl)-3-isopropyl-[1,2,4]triazolo[4,3-b]pyridazine-6,8-diamine (5.6)

In a microwave tube 2-5 mL with a stir bar was charged **(5.1)** (1.200 g, 4.72 mmol, 1.0 eq.), NMP (9.0 mL) and pentan-3-amine (9.9 mL, 85.0 mmol, 15.0 eq.). The vial was closed and then put on microwave cavity 3 h at 220 °C. After cooling, the mixture was concentrated, the oil residue was diluted in EtOAc (100 mL) then organic layer was washed four times with 50 mL of brine/water (3/1), dried over MgSO₄, filtered, concentrated and purified by flash chromatography with DCM/MeOH (97/3 to 92/8) as eluent to give **(5.6)** (0.364 g, 24 %) as a beige solid.
**Rf** (DCM/MeOH, 98/2) : 0.34

**¹H NMR (400 MHz, DMSO-*d₆*) δ :** 0.86 (t, *J* = 7.3 Hz, 6H, 2xCH_{3-CH2}), 1.35 (d, *J* = 6.9 Hz, 6H, 2xCH_{3 iPr}), 1.39 - 1.62 (m, 4H, 2xCH_{2-CH3}), 3.27 - 3.35 (m, 1H, H_{iPr}), 3.59 (h, *J* = 6.9 Hz, 1H, H_{iPent}), 5.62 (s, 1H, H_{Ar}), 6.30 (d, *J* = 8.0 Hz, 1H, NH_{iPent}), 6.60 (s, 2H, NH₂).

**¹³C NMR (101 MHz, DMSO-*d₆*) δ** : 10.4 (2xCH_{3-CH2}), 19.5 (2xCH_{3 iPr}), 24.8 (CH_{iPr}), 26.3 (2xCH_{2-CH3}), 52.9 (CH_{iPent}), 86.5 (CH_{Ar}), 140.0 (C_{q}), 140.7 (C_{q}), 152.5 (C_{q}), 156.1 (C_{q}).

**MS (ESI+)** : **m/z calcd for C₁₃H₂₂N₆** : 263.20 [M+H]+, **found** : 263.26.

### Example 5.7: Synthesis of N6,N8-bis(1-ethylpropyl)-3-isopropyl-[1,2,4]triazolo[4,3-b]pyridazine-6,8-diamine (114)

In a sealed tube 2-5 mL with a stir bar was charged **(5.1)** (0.200 g, 0.95 mmol, 1.0 eq.), NMP (2.20 mL) and 3-aminopentane (1.10 mL, 9.45 mmol, 10.0 eq.). The vial was sealed and then put on microwave 1 h at 240 °C After cooling, the reaction mixture was diluted in EtOAc (30 mL) and the resulting organic layer was washed three times with a mixture of brine/water (3/1, 3x20 mL) then dried over MgSO₄, filtered, concentrated and purified by flash chromatography with DCM/MeOH (97/3 to 94/6) as eluent to give the secondary compound **(114)** (0.095 g, 30 %) as a white crystalline solid.

### Example 5.8: Synthesis of 3-cyclopropyl-N6-(pentan-3-yl)-[1,2,4]triazolo[4,3-b]pyridazine-6,8-diamine (5.8)

In a sealed tube 10 - 20 mL with a stir bar was charged **(5.2)** (1.200 g, 5.72 mmol, 1.0 eq.), NMP (5.7 mL) and 3-aminopentane (3.33 mL, 28.62 mmol, 5.0 eq.) then the vial was sealed and put on heating bloc : 200 °C, 17 h. After cooling, the mixture was poured onto cold water (100.0 mL) and stired 5 min. The resulting precipitate was filtered off and washed with water. The solid was taken up in THF to make solid deposit with Si₂O and purified by flash chromatography with DCM/MeOH (98/2 to 91/9) as eluent to give **(5.8)** (0.225 g, 15 %) as a yellow solid.
**Rf** (DCM/MeOH, 94/6) : 0.30

**¹H NMR (400 MHz, DMSO-*d₆*) δ** : 0.87 (t, *J* = 7.3 Hz, 6H, 2xCH_{3-CH2}), 0.97 - 1.17 (m, 4H, 2xCH_{2 c}pᵣ), 1.36 - 1.63 (m, 4H, 2xCH_{2-CH3}), 2.23 (tt, *J* = 8.8, 5.1 Hz, 1H, H_{cPr}), 3.59 (h, *J* = 6.5 Hz, 1H, H_{iPent}), 5.61 (s, 1H, H_{Ar}), 6.30 (d, *J* = 8.1 Hz, 1H, NH_{iPent}), 6.58 (s, 2H, NH₂).

**¹³C NMR (101 MHz, DMSO-*d₆*) δ** : 5.4 (CH_{cPr}), 6.3 (2xCH_{2 cPr}), 10.4 (2xCH_{3-CH2}), 26.3 (2xCH_{2-CH3}), 52.8 (CH_{iPent}), 86.5 (CH_{Ar}), 139.9 (C_{q}), 140.7 (C_{q}), 149.7 (C_{q}), 156.4 (C_{q}).

**MS (ESI+)** : **m/z calcd for C₁₃H₂₀N₆** : 261.18 [M+H]+, **found** : 261.28.

### Example 6: [1,2,4]triazolo[4,3-b]pyridazine-6,8-diamine derivatives, as illustrated in step 3 of scheme 4

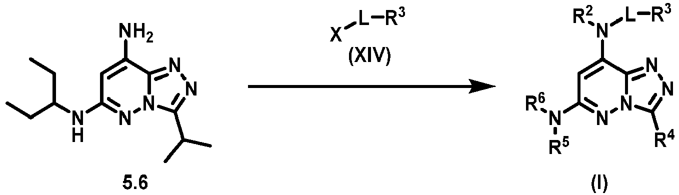

### Example 6.1: Synthesis of N-[6-(1-ethylpropylamino)-3-isopropyl-[1,2,4]triazolo[4,3-b]pyridazin-8-yl]benzamide (96)

To a solution of **(5.6)** (0.075 g, 0.29 mmol, 1.0 eq.) in dry ACN (1 mL), DMAP (0.035 g, 0.29 mmol, 1.0 eq.), DIPEA (0.07 mL, 0.40 mmol, 1.4 eq.) then corresponding acyle chloride (0.04 mL, 0.34 mmol, 1.2 eq.) was added and the mixture was refluxed on 2 h. After cooling, the mixture was concentrated, taken up in a minimum amount of DCM/MeOH (98/2) and purified directly by flash chromatography with DCM/MeOH (98/2) as eluent to give **(96)** (0.073 g, 70 %) as a white solid.

### Example 6.1: Synthesis of N-[6-(1-ethylpropylamino)-3-isopropyl-[1,2,4]triazolo[4,3-b]pyridazin-8-yl]pyridine-2-carboxamide (97)

To a suspension of **(5.6)** (0.090 g, 0.34 mmol, 1.0 eq.) in dry THF (6.0 mL), NaH 60% suspended in mineral oil (0.033 g, 0.82 mmol, 2.4 eq.) was added and the mixture was stirred 10 min at room temperature. Pyridine-2-carbonyl chloride hydrochloride (0.076 g, 0.41 mmol, 1.2 eq.) was added and the mixture was stirred 1.2 h at room temperature. After completion, saturated NH₄Cl (2.0 mL), water (2.0 mL) and EtOAc (6.0 mL) was added and the reaction mixture was vigorously stirred 5 min. Aqueous layer was extracted twice with EtOAC (2 x 10 mL) then organics layers were combined, dried over MgSO₄, filtered, concentrated and purified by flash chromatography with DCM/MeOH (99/1 to 97/3) as eluent to give **(97)** (0.019 g, 15 %) as a white solid.

### Example 6.3: Synthesis of N-[6-(1-ethylpropylamino)-3-isopropyl-[1,2,4] triazolo[4,3-b]pyridazin-8-yl]-2-phenyl-acetamide (98)

Under inert gas, to a solution of **(5.6)** (0.085 g, 0.32 mmol, 1.0 eq.) in dry THF (3.2 mL), was added NaH 60% suspended in mineral oil (0.026 g, 0.65 mmol, 2.0 eq.). The mixture was stirred 5 min at room temperature then corresponding acetyl chloride (0.05 mL, 0.39 mmol, 1.2 eq.) was added and stirred 1.6 h at room temperature. After completion, a mixture of 2 mL of saturated NH₄Cl and 0.5 mL of water was added then EtOAc (3.0 mL). The mixture was vigorously stirred during 5 min then layers were separated. Aqueous layer was extracted twice with EtOAc (2 x 2.0 mL), organics layers were combined, dried over MgSO₄, filtered, concentrated and purified by flash chromatography with DCM/MeOH (99/1 to 97/3) as eluent to give (98) (0.050 g, 41 %) as a pale yellow solid.

### Example 6.4: Synthesis of 2-(2-pyridyl)acetyl chloride hydrochloride (6.4)

To a solution of 2-(pyridin-3-yl)acetic acid hydrochloride (0.500 g, 2.74 mmol, 1.0 eq.) in dry DCM (27.0 mL), DMF (0.01 mL, 0.14 mmol, 0.05 eq.) then oxalyle chloride (0.30 mL, 3.56 mmol, 1.3 eq.) were added and the mixture was stired 19 h at room temperature. After completion, THF was added (50.0 mL) then the resulting precipitate was filtered off, washed with THF and dried under vacuum to give the title compound **(6.4)** (0.445 g, 85 %) as a black solid. The compound would be used without other purification.

**¹H NMR (400 MHz, DMSO-*d₆*) δ :** 4.02 (d, *J* = 16.7 Hz, 2H, CH₂₋co), 8.03 - 8.16 (m, 1H, H_{Ar}), 8.63 (d, *J* = 8.1 Hz, 1H, H_{Ar}), 8.80 (t, *J* = 5.7 Hz, 1H, H_{Ar}), 8.88 (s, 1H, H_{Ar}).

**¹³C NMR (101 MHz, DMSO-*d₆*) δ :** N.D.

### Example 6.5: Synthesis of N-[6-(1-ethylpropylamino)-3-isopropyl-[1_{.}2_{.}4]triazolo[4,3-b]pyridazin-8-yl]-2-(3-pyridyl)acetamide (168)

Under inert gaz, to a solution of **(5.6)** (0.100 g, 0.39 mmol, 1.0 eq.) in dry THF (11.5 mL), NaH 60% in mineral oil (0.061 g, 1.52 mmol, 4.0 eq.) was added and the mixture was stired 15 min at room temperature. The compound **(6.4)** (0.110 g, 0.57 mmol, 1.5 eq.) was added and the mixture was stired 3 h at room temperature. After completion, sat. NH₄Cl (10.0 mL), H₂O (5.0 mL) and EtOAc (10.0 mL) was added and the mixture was vigorously stired 5 min. Layers were separated and aqueous layer was extracted twice with EtOAc (2x10.0 mL), organivs layers were combined, dried over MgSO₄, filtered, concentrated and the residu was purified by flash chromatography with EtOAc/MeOH (100/0 to 95/5) as eluent to give **(168)** (0.052 g, 36 %) as a white solid.

### Example 6.6: Synthesis of ethyl N-[6-(1-ethylpropylamino)-3-isopropyl-[1_{.}2_{.}4] triazolo[4,3-b]pyridazin-8-yl]carbamate (99)

Under inert gas, to a solution of **(5.6)** (0.070 g, 0.25 mmol, 1.0 eq.) in dry THF (3.0 mL) was added NaH 60% suspended in mineral oil (0.012 g, 0.30 mmol, 1.2 eq.). The mixture was stirred 5 min at room temperature then corresponding chloroformate (0.034 g, 0.30 mmol, 1.1 eq.) was added and stirred 1.5 h at room temperature. After completion, 2 mL of saturated NH₄Cl and 0.5 mL of water were added then EtOAc (4.0 mL). The mixture was vigorously stirred during 5 min then layers were separated. Aqueous layer was extracted twice with EtOAc (2 x 2.0 mL), organics layers were combined, dried over MgSO₄, filtered, concentrated and purified by flash chromatography with DCM/MeOH (98/2 to 95/5) as eluent to give 99 (0.045 g, 53 %) as a white solid.

### Example 6.7: Synthesis of phenyl N-[6-(1-ethylpropylamino)-3-isopropyl-[1,2,4]triazolo[4,3-b]pyridazin-8-yl]carbamate (100)

Under inert gas, to a solution of **(5.6)** (0.100 g, 0.38 mmol, 1.0 eq.) in dry THF (3.0 mL) was added NaH 60% suspended in mineral oil (0.023 g, 0.57 mmol, 1.5 eq.). The mixture was stirred 10 min at room temperature then corresponding chloroformate (0.069 g, 0.42 mmol, 1.1 eq.) was added and stirred 5.5 h at room temperature. After completion, 2.5 mL of saturated NH₄Cl and 1.0 mL of water were added then EtOAc (5.0 mL). The mixture was vigorously stirred during 5 min then layers were separated. Aqueous layer was extracted twice with EtOAc (2 x 5.0 mL), organics layers were combined, dried over MgSO₄, filtered, concentrated and purified by flash chromatography with DCM/MeOH (99/1 to 96/4) as eluent to give **(100)** (0.075 g, 51 %) as a white solid.

### Example 6.8: Synthesis of 3-chloro-5-methoxypyridazine (6.8)

To a solution of 3,5-dichloropyridazine (1.000 g, 6.38 mmol, 1.0 eq.) in MeOH (33.0 mL), MeONa (0.399 g, 7.01 mmol, 1.1 eq.) was added and the mixture was stired 18 h at room temperature. The mixture was concentrated then the residue was taken up in DCM, solid was filtered off, washed with DCM and the filtrate was concentrated and directly purified by flash chromatography with DCM/EtOAc (100/0 to 80/20) as eluent to give **(6.8)** (0.520 g, 56 %) as a light yellow solid.
**Rf** (DCM, 100%) : 0.22

**¹H NMR (400 MHz, DMSO-*d₆*) δ :** 3.94 (s, 3H, CH₃₋₀, 7.54 (d, *J* = 2.6 Hz, 1H, H_{Ar}), 9.00 (d, *J* = 2.6 Hz, 1H, H_{Ar}).

**¹³C NMR (101 MHz, DMSO-*d₆*) δ :** 56.6 (CH₃₋₀), 110.4 (CH_{Ar}), 144.0 (CH_{Ar}), 156.1 (C_{q}), 159.2 (C_{q}).

**MS (ESI+)** : **m/z calcd for C₅H₅ClN₂O** : 145.0 [M+H]+, **found** : 144.9

### Example 6.9: Synthesis of 3-isopropyl-N8-(5-methoxypyridazin-3-yl)-N6-(pentan-3-yl)-[1,2,4]triazolo[4,3-b]pyridazine-6,8-diamine (162)

Under inert gas, in a sealed tube 2-5 mL with a stir bar was charged **(5.6)** (0.050 g, 0.19 mmol, 1.0 eq.), 3-chloro-5-methoxypyridazine **(6.8)** (0.030 g, 0.21 mmol, 1.1 eq.), C_{S2}CO₃ (0.094 g, 0.29 mmol, 1.5 eq.), Pd(OAc)₂ (0.003 g, 0.015 mmol, 0.08 eq.) and Xantphos (0.018 g, 0.030 mmol, 0.16 eq.). Dioxane (2.0 mL) degased 5min with argon was added then the tube was sealed and put on heating block 16 h at 110 °C. After cooling, the mixture was diluted with a little amount of DCM/MeOH (95/5) and SiO₂ was added to make solid deposit then directly purified by flash chromatography with DCM/MeOH (98/2 to 94/6) as eluent to give **(162)** (0.015 g, 34 %) as a light yellow solid.

### Example 7: 8-methylsulfanyl-[1,2,4]triazolo[4,3-b]pyridazine derivatives, as illustrated in steps 1 to 3 of scheme 3

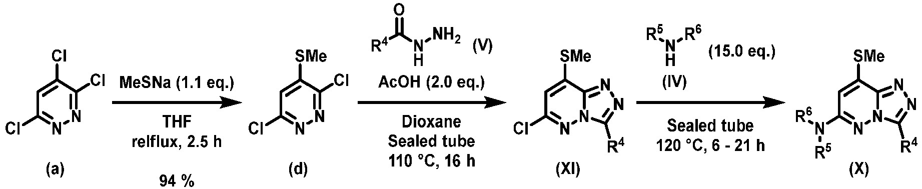

### Example 7.1: Synthesis of 3,6-dichloro-4-methylsulfanyl-pyridazine (d)

To a solution of 3,4,6-trichloropyridazine (4.000 g, 21.8 mmol, 1.0 eq.) in THF (43 mL), MeSNa (1.775 mL, 24.0 mmol, 1.1 eq.) was added and the mixture was refluxed 2.5 h. After cooling, MgSO₄ was added and the mixture was filtered, the solid was washed with THF then the filtrate was concentrated to give **(d)** (4.000 g, 94 %) as a white solid.
**Rf** (DCM, 100 %) : 0.46

**¹H NMR (400 MHz, DMSO-*d₆*) δ :** 2.63 (s, 3H, SCH₃), 7.76 (s, 1H, H_{Ar}).

**¹³C NMR (101 MHz, DMSO-*d₆*) δ :** 13.6 (CH₃), 124.1 (CH_{Ar}), 147.5 (C_{q}), 152.1 (C_{q}), 154.8 (C_{q}).

**MS (ESI+)** : **m/z calcd for C₅H₄Cl₂N₂S** : 195.0 [M+H]+, **found** : 195.0.

### Example 7.2: Synthesis of 6-chloro-8-methylsulfanyl-[1,2,4]triazolo[4,3-b]pyridazine (7.2)

In a 10 - 20 mL vial with a stir bar was charged **(d)** (1.250 g, 6.41 mmol, 1.0 eq.), dioxane (6.4 mL), formohydrazide (0.462 g, 7.69 mmol, 1.2 eq.) and AcOH (0.74 mL, 12.82 mmol, 2.0 eq.). The vial was sealed than put on heating bloc, 16 h at 110°C. After cooling, MeOH (5 mL) was added to solubilise the reaction mixture then it was concentrated with silica and directly purified by flash chromatography with DCM/MeOH (99/1 to 96/4) as eluent to give **7.2** (0.210 g, 16 %) as a beige solid.
**Rf** (DCM, 100%) : 0.23.

**¹H NMR (400 MHz, DMSO-*d₆*) δ :** 2.65 (s, 3H, CH₃₋s), 8.09 (s, 1H, H_{Ar}), 9.57 (s, 1H, H_{Ar}).

**¹³C NMR (101 MHz, DMSO-*d₆*) δ :** N.D.

**MS (ESI+)** : **m/z calcd for C₆H₅N₄ClOS** : 201.0 [M+H]+, **found** : N.D.

### Example 7.3: Synthesis of 6-chloro-3-isopropyl-8-methylsulfanyl-[1,2,4]triazolo[4,3-b]pyridazine (7.3)

In a 10 - 20 mL vial with a stir bar was charged **(d)** (1.250 g, 7.69 mmol, 1.0 eq.), dioxane (6.4 mL), corresponding hydrazide (0.952 g, 9.23 mmol, 1.2 eq.) and AcOH (0.89 mL, 15.38 mmol, 2.0 eq.). The vial was sealed than put on heating bloc, 16 h at 110°C. After cooling, MeOH (5 mL) and Na₂CO₃ (1.223 g, 11.53 mmol, 1.5 eq.) was added to solubilise and neutralise the reaction mixture then it was concentrated with silica and directly purified by flash chromatography with DCM/MeOH (99/1 to 96/4) as eluent to give **7.3** (0.810 g, 43 %) as a beige solid.

**¹H NMR (400 MHz, DMSO-*d₆*) δ :** 1.40 (d, *J* = 6.9 Hz, 6H, 2xCH_{3 iPr}), 2.64 (s, 3H, SCH₃), 3.47 (hept, *J* = 7.0 Hz, 1H, HiPr), 8.03 (s, 1H, HAr).

**¹³C NMR (101 MHz, DMSO-*d₆*) δ** : 14.5 (SCH₃), 19.8 (2xCH_{3 iPr}), 24.3 (CH_{iPr}), 117.2 (CH_{Ar}), 134.7 (C_{q}), 143.8 (C_{q}), 146.3 (C_{q}), 152.3 (C_{q}).

**MS (ESI+)** : **m/z calcd for C₉H₁₁ClN₄S** : 243.0 [M+H]+, **found** : 243.1.

### Example 7.4: Synthesis of N-(1-ethylpropyl)-8-methylsulfanyl-[1,2,4]triazolo[4,3-b]pyridazin-6-amine (7.4)

In a 2 - 5 mL vial with a stir bar was charged **(7.2)** (0.150 g, 0.75 mmol, 1.0 eq.), 3-aminopentane (1.32 mL, 11.21 mmol, 15.0 eq.). The vial was sealed and then put on heating block, 6 h at 120 °C. After cooling, the mixture was diluted in THF (15.0 mL), the solid was filtered, washed with THF and the filtrate was concentrated. The residu was purified by flash chromatography with DCM/MeOH (99/1 to 96/4) as eluent to give **7.4** (0.130 g, 69 %) as a white solid.
**Rf** (DCM/MeOH, 96/4) : 0.57

**¹H NMR (400 MHz, DMSO-*d₆*) δ** : 0.86 (t, *J* = 7.4 Hz, 6H (2xCH_{3-CH2}), 1.52 - 1.70 (m, 4H, 2xCH_{2-CH3}), 2.63 (s, 3H, SCH₃), 3.81 (h, *J* = 6.8 Hz, 1H, H_{iPent}), 5.95 (d, *J* = 8.4 Hz, 1H, NH_{iPent}), 7.76 (s, 1H, H_{Ar}), 9.05 (s, 1H, H_{Ar}).

**¹³C NMR (101 MHz, DMSO-*d₆*) δ :** 10.5 (2xCH_{3-CH2}), 14.5 (SCH₃), 25.8 (2xCH_{2-CH3}), 54.2 (CH_{iPent}), 115.8 (CH_{Ar}), 129.3 (C_{q}), 137.4 (CH_{Ar}), 142.1 (C_{q}), 151.1 (C_{q}).

**MS (ESI+)** : **m/z calcd for C₁₁H₁₇N₅S** : 252.13 [M+H]+, **found** : 252.14.

### Example 7.5: Synthesis of N-(1-ethylpropyl)-3-isopropyl-8-methylsulfanyl-[1,2,4]triazolo[4,3-b]pyridazin-6-amine (7.5)

A vial 2 - 5 mL with a stir bar was charged **(7.3)** (0.500 g, 2.06 mmol, 1.0 eq.), 3-aminopentane (3.64 mL, 30.90 mmol, 15.0 eq.). The vial was sealed and then put on heating block, 21 h at 120 °C. After cooling, the mixture was diluted in THF (15.0 mL), the solid was filtered, washed with THF and the filtrate was concentrated. The residu was purified by flash chromatography with DCM/MeOH (99/1 to 96/4) as eluent to give **(7.5)** (0.590 g, 98 %) as a white solid.
**Rf** (DCM/MeOH, 96/4) : 0.47

**¹H NMR (400 MHz, DMSO-*d₆*) δ :** 0.88 (t, *J* = 7.4 Hz, 6H, 2xCH_{3-CH2}), 1.38 (d, *J* = 6.9 Hz, 6H, 2xCH_{3 iPr}), 1.53 - 1.73 (m, 4H, 2xCH_{2-CH3}), 2.62 (s, 3H, SCH₃), 3.33 - 3.40 (m, 1H, H_{iPr}), 3.78 (dtd, *J* = 13.2, 7.7, 5.4 Hz, 1H, H_{iPent}), 5.96 (d, *J* = 8.1 Hz, 1H, NH_{Alk}), 7.71 (s, 1H, H_{Ar}).

**¹³C NMR (101 MHz, DMSO-*d₆*) δ** : 10.7 (2xCH_{3-CH2}), 14.6 (SCH₃), 19.4 (2xCH_{3 iPr}), 24.7 (CH_{iPr}), 25.9 (2xCH_{2-CH3}), 54.7 (CH_{iPent}), 116.7 (CH_{Ar}), 127.6 (C_{q}), 142.9 (C_{q}), 150.3 (C_{q}), 151.3 (C_{q}).

**MS (ESI+) : m/z calcd for C₁₄H₂₃N₅S :** 294.17 [M+H]+, **found :** 294.27.

### Example 7.6: Synthesis of 3-isopropyl-8-methylsulfanyl-N-[(3R)-tetrahydropyran-3-yl]-[1,2,4]triazolo[4,3-b]pyridazin-6-amine (7.6)

In a 10 - 20 mL vial with a stir bar was charged **(7.3)** (0.300 g, 0.93 mmol, 1.0 eq.), corresponding amine hydrochloride (0.772 g, 5.61 mmol, 4.5 eq.) and DIPEA (1.18 mL, 6.54 mmo, 5.5 eq.). The vial was sealed and then put on heating block, 18 h at 120 °C. After cooling, EtOAc (10.0 mL) and water (10.0 mL) was added and the mixture was vigorously stirred 5 min. Aqueous layer was extracted twice with EtOAc (2 x 10.0 mL) then organics layers were combined, washed with brine (20.0 mL), dried over MgSO₄, filtered, concentrated and purified by flash chromatography with DCM/MeOH (100/0 to 97/3) as eluent to give **(7.6)** (0.240 g, 64 %) as a beige solid.
**Rf** (DCM/MeOH, 96.4) : 0.09

**¹H NMR (400 MHz, DMSO-*d₆*) δ :** 1.38 (dd, *J* = 7.0, 2.7 Hz, 6H, 2xCH_{3 iPr}), 1.61 (dt, *J* = 9.3, 4.1 Hz, 1H, _{CH2-}H(_{CH2})_{-CH2}), 1.69 (dt, *J* = 13.5, 4.1 Hz, 1H, _{CH2-}H(_{CH2})_{-CH2}), 1.75 - 1.87 (m, 1H, _{CH-}H(_{CH2})_{-CH2}), 1.95 (d, *J* = 6.8 Hz, 1H, _{CH-}H(_{CH2})_{-CH2}), 2.62 (s, 3H, SCH₃), 3.37 (m, 3H, H_{iPr} & _{O-}H(_{CH2})_{-CH THP} & o-H(_{CH2})_{-CH2}), 3.73 (dt, *J* = 11.5, 4.1 Hz, 1H, _{O-}H(_{CH2})_{-CH2}), 3.92 (m, 2H, H_{THP} & _{O-}H(_{CH2})_{-CH THP}), 6.04 (d, *J* = 7.3 Hz, 1H, NH_{Alk}), 7.76 (s, 1H, H_{Ar}).

**¹³C NMR (101 MHz, DMSO-*d₆*) δ** : 14.6 (SCH₃), 19.5 (CH_{3 iPr}), 19.5 (CH_{3 iPr}), 24.4 (CH_{2 THP}), 24.7 (CH_{iPr}), 27.7 (CH_{2 THP}), 47.7 (CH_{THP}), 67.2 (CH_{2 THP}), 69.3 (CH_{2 THP}), 117.1 (CH_{Ar}), 127.6 (C_{q}), 142.9 (C_{q}), 149.5 (C_{q}), 151.5 (C_{q}).

**MS (ESI+)** : **m/z calcd for C₁₄H₂₁N₅OS** : 308.15 [M+H]+, **found** : 308.24.

### Example 7.7: Synthesis of (2R)-2-[(3-isopropyl-8-methylsulfanyl-[1,2,4]triazolo[4,3-b]pyridazin-6-yl)amino]butan-1-ol (7.7)

A solution of **(7.3)** (0.250 g, 1.02 mmol, 1.0 eq.) and (2R)-2-aminobutan-1-ol (0.98 mL, 10.20 mmol, 10.0 eq.) was heated 45 min at 120 °C. After cooling, the mixture was poured onto water (20 mL) then HCl 1M (20 mL) was added. Aqueous layer was extracted three times with DCM (3x15 mL), organics layers were combined, dried over MgSO₄, filtered, concentrated then purified by flash chromatography with DCM/MeOH (99/1 to 96/5) as eluent to give **(7.7)** (0.100 g, 83 %) as a pale pink solid.

**¹H NMR (400 MHz, DMSO-*d₆*) δ :** 0.90 (t, *J* = 7.4 Hz, 3H, CH_{3-CH2}), 1.38 (dd, *J* = 7.0, 4.7 Hz, 6H, 2xCH_{3 iPr}), 1.59 - 1.73 (m, 2H, CH_{2-CH3}), 2.62 (s, 3H, SCH₃), 3.37 (p, *J* = 7.0 Hz, 1H, H_{iPr}), 3.57 (dq, *J* = 12.9, 5.5 Hz, 2H, CH_{2-OH}), 3.83 (dtd, *J* = 11.7, 6.7, 4.9 Hz, 1H, H_{C-NH}), 4.84 (t, *J* = 5.4 Hz, 1H, OH), 5.84 (d, *J* = 7.6 Hz, 1H, NH), 7.76 (s, 1H, H_{Ar}).

**¹³C NMR (101 MHz, DMSO-*d₆*) δ :** 10.6 (CH_{3-CH2}), 14.7 (SCH₃), 19.4 (CH_{3 iPr}), 19.5 (CH_{3 iPr}), 23.0 (CH_{2-CH3}), 24.7 (CH_{iPr}), 54.8 (CH_{-NH}), 61.4 (CH_{2-OH}), 117.3 (CH_{Ar}), 127.4 (C_{q}), 142.9 (C_{q}), 150.0 (C_{q}), 151.4 (C_{q}).

**MS (ESI+)** : **m/z calcd for C₁₃H₂₁N₅OS** : 296.15 [M+H]+, **found** : 296.22.

### Example 8: 8-methylsulfonyl-[1,2,4]triazolo[4,3-b]pyridazin-6-amine derivatives

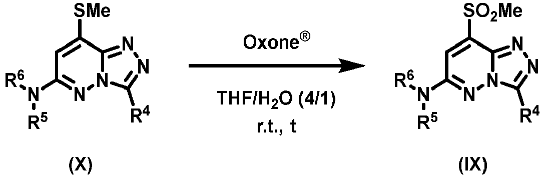

### Example 8.1: Synthesis of N-(1-ethylpropyl)-3-isopropyl-8-methylsulfonyl-[1,2,4]triazolo[4,3-b]pyridazin-6-amine (8.1)

To a solution of **(7.5)** (0.500 g, 1.70 mmol, 1.0 eq.) in THF/H₂O (4/1, 30 mL) was added Oxone (1.728 g, 5.62 mmol, 3.3 eq.). The reaction mixture was stirred 16 h at room temperature then brine (5 mL) was added. Aqueous layer was extracted twice with EtOAc (2 x 10 mL), orgnics layers were combined, dried over MgSO₄, filtered, concentrated and purified by flash chromatography with DCM/MeOH (99/1 to 97/3) as eluent to give (8.1) (0.500 g, 90 %) as a pale yellow solid.

**Rf** (DCM/MeOH, 94/6) : 0.62.

**¹H NMR (400 MHz, DMSO-*d₆*) δ :** 0.92 (t, *J* = 7.4 Hz, 6H, 2xCH_{3-CH2}), 1.41 (d, *J* = 7.0 Hz, 6H, 2xCH_{3 iPr}), 1.59 - 1.72 (m, 4H, 2xCH_{2-CH3}), 3.41 - 3.49 (m, 4H, H_{iPr} & CH_{3-SO2}), 3.82 (h, *J* = 6.3 Hz, 1H, H_{iPent}), 6.41 (d, *J* = 7.5 Hz, 1H, NH_{Alk}), 8.56 (s, 1H, H_{Ar}).

**¹³C NMR (101 MHz, DMSO-*d₆*) δ** : 10.2 (2xCH_{3-CH2}), 19.3 (2xCH_{3 iPr}), 24.8 (CH_{iPr}), 25.5 (2xCH_{2-CH3}), 42.8 (CH_{3-SO2}), 54.1 (CH_{iPent}), 124.7 (C_{q}), 129.0 (CH_{Ar}), 141.5 (C_{q}), 148.2 (C_{q}), 153.4 (C_{q}).

**MS (ESI+)** : **m/z calcd for C₁₄H₂₃N₅O₂S** : 326.16 [M+H]+, **found** : 326.25.

### Example 8.2: Synthesis of 3-isopropyl-8-methylsulfonyl-N-[(3R)-tetrahydropyran-3-yl]-[1,2,4]triazolo[4,3-b]pyridazin-6-amine (8.2)

To a solution of **(7.6)** (0.715 g, 2.33 mmol, 1.0 eq.) in THF (37 mL) was added Oxone (2.361 g, 7.68 mmol, 3.3 eq.) and water (9 mL). The mixture was stirred 18 h at room temperature then NaCl was added to give two phases. Aqueous layer was extracted twice with EtOAc (2x10 mL) and organic layers were combined, dried over MgSO₄, filtered, concentrated and purified by flash chromatography with DCM/MeOH (97/3 to 95/5) as eluent to give (8.2) (0.520 g, 66 %) as a pale yellow solid.

**¹H NMR (400 MHz, DMSO-*d₆*) δ :** 1.41 (d, *J* = 6.9 Hz, 6H, 2xCH_{3 iPr}), 1.51- 1.78 (m, 2H, _{CH2-}CH_{2-CH2}), 1.82 - 2.00 (m, 2H, _{CH-}CH_{2-CH2}), 3.40 - 3.51 (m, 4H, SO₂CH₃ & H_{iPr}), 3.54 - 3.59 (m, 1H, _{O-}H(_{CH2})_{-CH}), 3.61 (t, *J* = 5.3 Hz, 2H, _{O-}CH_{2-CH2}), 3.86 (dd, *J* = 11.1, 3.0 Hz, 1H, _{O-}H(_{CH2})_{-CH}), 3.96 (tt, *J* = 6.5, 3.1 Hz, 1H, H_{THP}), 6.75 (d, *J* = 7.4 Hz, 1H, NH_{THP}), 8.59 (s, 1H, H_{Ar}).

**¹³C NMR (101 MHz, DMSO-*d₆*) δ :** 19.4 (2xCH_{3 iPr}), 23.0 (CH_{2 THP}), 24.7 (CH_{iPr}), 26.7 (CH_{2 THP}), 42.9 (SO₂CH₃), 46.9 (CH_{THP}), 67.3 (CH_{2 THP}), 69.4 (CH_{2 THP}), 124.6 (C_{q}), 129.3 (CH_{Ar}), 141.5 (C_{q}), 147.6 (C_{q}), 153.5 (C_{q}).

**MS (ESI+)** : **m/z calcd for C₁₄H₂₁N₅O₃S** : 340.14 [M+H]+, **found** : 340.19.

### Example 8.3: Synthesis of (2R)-2-[(3-isopropyl-8-methylsulfonyl-[1,2,4]triazolo[4,3-b]pyridazin-6-yl)amino]butan-1-ol (8.3)

To a solution of **(7.7)** (0.720 g, 2.44 mmol, 1.0 eq.) in THF/H₂O (4/1, 45.0 mL) was added Oxone (2.472 g, 8.04 mmol, 3.3 eq.). The mixture was stirred 19 h at room temperature then brine (10.0 mL) and EtOAc (20.0 mL) was added to give two phases. Aqueous layer was extracted twice with EtOAc (2x10 mL) and organic layers were combined, dried over MgSO₄, filtered, concentrated and purified by flash chromatography with DCM/MeOH (99/1 to 94/6) as eluent to give **(8.3)** (0.310 g, 39 %) as a yellow solid.
**Rf** (DCM/MeOH, 96/4) : 0.22

**¹H NMR (400 MHz, DMSO-*d₆*) δ :** 0.94 (t, *J* = 7.5 Hz, 3H, CH_{3-CH2}), 1.41 (dd, *J* = 7.0, 3.1 Hz, 6H, 2xCH_{3 iPr}), 1.67 (pd, *J* = 7.1, 3.2 Hz, 2H, CH_{2-CH3}), 3.38 - 3.49 (m, 4H, H_{iPr} & CH_{3-SO2}), 3.54 - 3.67 (m, 2H, CH_{2-OH}), 3.85 (dtd, *J* = 11.1, 6.6, 4.4 Hz, 1H, H_{Alk}), 4.94 (t, *J* = 6.3 Hz, 1H, OH), 6.66 (d, *J* = 7.3 Hz, 1H, NH_{Alk}), 8.55 (s, 1H, H_{Ar}).

**¹³C NMR (101 MHz, DMSO-*d₆*) δ :** 10.4 (CH_{3-CH2}), 19.3 (CH_{3 iPr}), 19.3 (CH_{3 iPr}), 22.9 (CH_{2-CH3}), 24.7 (CH_{iPr}), 42.7 (CH_{3-SO2}), 54.4 (CH_{Alk}), 60.8 (CH_{2-OH}), 124.8 (C_{q}), 129.0 (CH_{Ar}), 141.5 (C_{q}), 148.2 (C_{q}), 153.4 (C_{q}).

**MS (ESI+)** : **m/z calcd for C₁₃H₂₁N₅O₃S** : 328.14 [M+H]+, **found** : 328.17.

### Example 9: Synthesis of [1,2,4]triazolo[4,3-b]pyridazine-6,8-diamine derivatives, as illustrated in step 5 of scheme 3

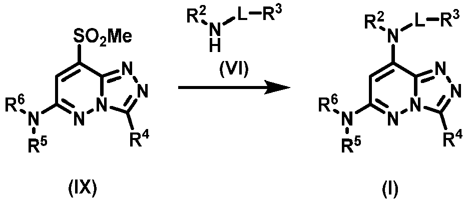

### Example 9.1: Synthesis of N6-(1-ethylpropyl)-3-isopropyl-N8-[(2-methoxyphenyl)methyl]-[1,2,4]triazolo[4,3-b]pyridazine-6,8-diamine (73)

A 2 - 5 mL vial with a stir bar was charged with **(8.1)** (0.100 g, 0.31 mmol, 1.0 eq.) and dioxane (0.3 mL) and corresponding amine (0.25 mL, 1.84 mmol, 6.0 eq.) then the vial was sealed and put on heating block 3 h at 110 °C. After cooling, the mixture was diluted in EtOAc (2.0 mL) then HCl 0.5M (3.8 mL) was added, stirred 5 min and layers were separated. Aqueous layer was extracted with EtOAc (2x3.0 mL) then organics layers were combined, dried over MgSO₄, filtered, concentrated and purified by flash chromatography with DCM/MeOH (98/2to 95/5) as eluent to give (73) (0.047 g, 40 %) as a white solid.

### Example 10: Biological Activity

### Example 10.1: ADORA3 inhibition potency

### MATERIAL AND METHODS

### Adenosine receptors antagonism assay (Eurofins)

The compounds of formula (I) were tested as potential antagonists on human recombinant A₃AR (expressed in HEK-293 cells) in an assay based on the use of an agonist radioligand, [¹²⁵I]-AB-MECA (0.15 nM) and 1 µM IB-MECA. Assays were carried out at r.t. for 120 min. Compounds were initially tested at three concentrations (0.01, 0.1, 1 µM). Active compounds were tested in duplicates in 8-point dose-response curves. IC₅₀ values, which is the concentration causing a half-maximal inhibition of control specific binding, and Hill coefficients (nH) were determined by non-linear regression analysis of the competition curves generated with mean replicate values using Hill equation curve fitting. The inhibition constants (Ki) were calculated using the Cheng Prusoff equation. IB-MECA was used as a positive control (IC₅₀: 0.215 nM; Ki: 0.130 nM).

### RESULTS

The A₃AR inhibition potency classification was done according to the following ranges of IC₅₀ values:
Some compounds have an IC₅₀ being inferior or equal to 1000 nM and superior or equal to 100 nM. These compounds correspond to the above-reported class **A.** Some compounds have an IC₅₀ inferior to 100 nM and superior or equal to 10 nM. These compounds correspond to the above-reported class **B.** Some compounds have an IC₅₀ inferior to 10 nM and superior or equal to 1 nM. These compounds correspond to the above-reported class **C.** Some compounds have an IC₅₀ inferior to 1 nM. These compounds correspond to the above-reported class **D.** Said letters **A** to **D** were used to quote the activity/efficacy of the compounds of the invention in the following **Table 3.**

**Table 3: A₃AR inhibitory activities of the compounds of formula (I). IC₅₀ values were calculated from dose-response curves. Compounds were further classified in terms of efficacy on A₃AR (increasing potency range: A to D).**

| **Cpd N°** | **Potency Class A₃AR** |
|---|---|
| **1** | **B** |
| **2** | **B** |
| **3** | **A** |
| **4** | **A** |
| **5** | **A** |
| **6** | **A** |
| **8** | **A** |
| **9** | **A** |
| **10** | **B** |
| **11** | **A** |
| **12** | **A** |
| **13** | **B** |
| **14** | **B** |
| **15** | **A** |
| **16** | **A** |
| **17** | **B** |
| **18** | **C** |
| **19** | **B** |
| **21** | **B** |
| **22** | **B** |
| **23** | **B** |
| **24** | **A** |
| **25** | **A** |
| **26** | **B** |
| **27** | **A** |
| **28** | **B** |
| **29** | **B** |
| **30** | **A** |
| **31** | **B** |
| **32** | **A** |
| **34** | **A** |
| **35** | **B** |
| **36** | **B** |
| **37** | **B** |
| **38** | **C** |
| **39** | **B** |
| **40** | **C** |
| **41** | **C** |
| **42** | **C** |
| **43** | **B** |
| **44** | **B** |
| **45** | **C** |
| **46** | **D** |
| **47** | **D** |
| **48** | **C** |
| **49** | **C** |
| **50** | **C** |
| **51** | **C** |
| **52** | **C** |
| **53** | **C** |
| **54** | **B** |
| **55** | **B** |
| **56** | **C** |
| **57** | **A** |
| **58** | **B** |
| **59** | **C** |
| **60** | **B** |
| **61** | **B** |
| **62** | **C** |
| **63** | **C** |
| **64** | **B** |
| **65** | **A** |
| **66** | **B** |
| **67** | **C** |
| **68** | **C** |
| **69** | **B** |
| **70** | **A** |
| **71** | **B** |
| **72** | **B** |
| **73** | **C** |
| **74** | **B** |
| **75** | **C** |
| **76** | **C** |
| **77** | **B** |
| **78** | **B** |
| **79** | **A** |
| **80** | **C** |
| **81** | **B** |
| **82** | **C** |
| **83** | **D** |
| **84** | **C** |
| **85** | **C** |
| **86** | **D** |
| **87** | **D** |
| **88** | **C** |
| **89** | **B** |
| **90** | **C** |
| **91** | **C** |
| **92** | **B** |
| **93** | **C** |
| **94** | **C** |
| **95** | **C** |
| **96** | **B** |
| **97** | **B** |
| **98** | **D** |
| **99** | **C** |
| **100** | **C** |
| **101** | **B** |
| **102** | **B** |
| **103** | **B** |
| **104** | **B** |
| **105** | **B** |
| **106** | **A** |
| **107** | **B** |
| **108** | **A** |
| **109** | **A** |
| **110** | **B** |
| **111** | **B** |
| **112** | **A** |
| **113** | **A** |
| **114** | **C** |
| **115** | **A** |
| **116** | **A** |
| **117** | **B** |
| **118** | **C** |
| **119** | **B** |
| **120** | **A** |
| **121** | **B** |
| **122** | **B** |
| **123** | **B** |
| **124** | **B** |
| **125** | **A** |
| **126** | **C** |
| **127** | **B** |
| **128** | **A** |
| **129** | **A** |
| **130** | **C** |
| **131** | **B** |
| **132** | **B** |
| **133** | **A** |
| **134** | **B** |
| **136** | **B** |
| **137** | **A** |
| **138** | **B** |
| **139** | **A** |
| **140** | **B** |
| **141** | **A** |
| **142** | **A** |
| **143** | **B** |
| **144** | **A** |
| **145** | **A** |
| **146** | **B** |
| **147** | **D** |
| **148** | **D** |
| **149** | **C** |
| **150** | **D** |
| **151** | **D** |
| **152** | **B** |
| **153** | **D** |
| **154** | **D** |
| **155** | **D** |
| **156** | **C** |
| **157** | **B** |
| **158** | **D** |
| **159** | **D** |
| **160** | **C** |
| **162** | **D** |
| **163** | **B** |
| **164** | **D** |
| **165** | **C** |
| **166** | **C** |
| **167** | **C** |
| **168** | **D** |
| **169** | **B** |
| **170** | **C** |
| **171** | **D** |
| **172** | **D** |
| **173** | **D** |
| **174** | **D** |
| **175** | **D** |
| **176** | **C** |
| **177** | **D** |
| **178** | **D** |
| **179** | **B** |
| **180** | **D** |
| **181** | **D** |
| **182** | **D** |
| **183** | **D** |
| **184** | **B** |
| **185** | **B** |
| **186** | **C** |
| **187** | **C** |
| **188** | **A** |
| **189** | **B** |
| **190** | **B** |
| **191** | **C** |
| **192** | **C** |
| **193** | **C** |
| **194** | **B** |
| **195** | **C** |
| **196** | **C** |
| **197** | **B** |
| **198** | **C** |
| **199** | **C** |
| **200** | **C** |
| **201** | **C** |
| **202** | **C** |
| **203** | **B** |
| **204** | **C** |
| **205** | **C** |
| **206** | **C** |
| **207** | **A** |
| **208** | **C** |
| **209** | **C** |
| **210** | **C** |
| **211** | **B** |
| **212** | **B** |
| **213** | **A** |
| **214** | **A** |
| **215** | **D** |
| **216** | **C** |
| **217** | **C** |
| **218** | **C** |

### CONCLUSION

Based on the previous results, it can be concluded that the compounds of formula (I) exert an inhibitory activity on A₃AR and thus are useful in the treatment of pathologies mediated by A₃AR. The most potent inhibitory activity on A₃AR is displayed by compounds (1), (2), (10), (13), (14), (17) to (19), (21) to (23), (26), (28), (29), (31), (35) to (56), (58) to (64), (66) to (69), (71) to (78), (80) to (105), (107), (110), (111), (114), (117) to (119), (121) to (124), (126), (127), (130) to (132), (134), (136), (138), (140), (143), (146) to (160), (162) to (187), (189) to (206), (208) to (212) and (215) to (218), in particular compounds (18), (38), (40) to (42), (45) to (53), (56), (59), (62), (63), (67), (68), (73), (75), (76), (80), (82) to (88), (90), (91), (93) to (95), (98) to (100), (114), (118), (126), (130), (147) to (151), (153) to (156), (158) to (160), (162), (164) to (168), (170) to (178), (180) to (183), (186), (187), (191) to (193), (195), (196), (198) to (202), (204) to (206), (208) to (210) and (215) to (218), and more particularly compounds (46), (47), (83), (86), (87), (98), (147), (148), (150), (151), (153) to (155), (158), (159), (162), (164), (168), (171) to (175), (177), (178), (180) to (183) and (215).

### Example 10.2: A₃R selectivity study on a panel of 168 GPCRs

### MATERIAL AND METHODS

Compound (175) was tested at 1 µM as a potential agonist or antagonist of a panel of 168 GPCRs utilizing the PathHunter β-arrestin enzyme fragment complementation (EFC) technology (Eurofins - DiscoveRx - LeadHunter Discovery Services). In brief, when the GPCR is activated and β-Arrestin is recruited to the receptor, the two fragments of β-galactosidase complementation occurs, restoring β- galactosidase activity which is measured using chemiluminescent PathHunter^{®} Detection Reagents. The tested compound can be evaluated as a potential agonist or, in the presnece of a specifi agonits of each GPCR, as a potential antagonist. Results are provided in reference to the assay performed in the absence of tested compound.

### RESULTS

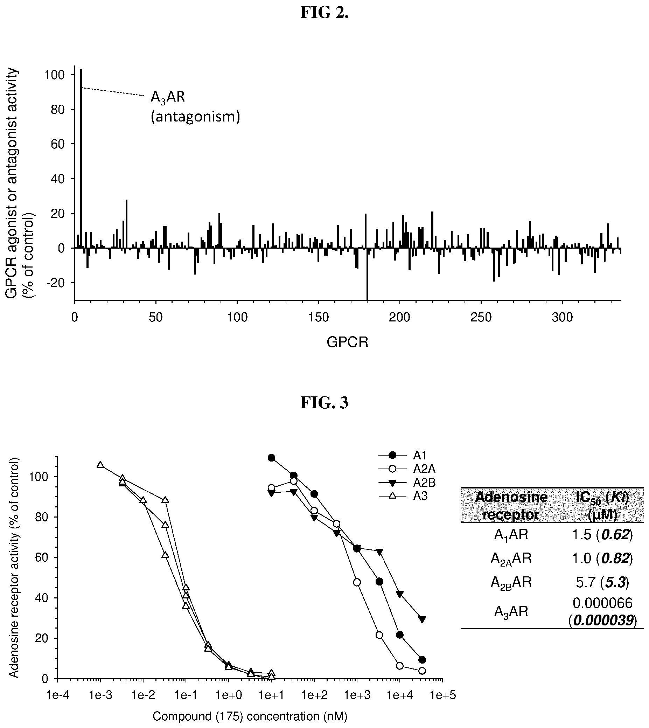

The results of an evaluation of the potential agonist or antagonist activity of compound (175) on 168 GPCRs are depicted in figure 2. As shown in figure 2, in the agonist mode (uneven numbers on X-axis), each GPCR was exposed to 0.1 µM of compound (175) only, while in the antagonist mode (even numbers on X-axis), each GPCR was exposed to its specific ligand and to 0.1 µM of compound (175). As demonstrated in figure 2, only A₃AR among 168 GPCRs was found to be fully inhibited by compound (175). All other GPCRs were not or marginally impacted by the presence of compound (175).

### CONCLUSION

Based on the previous results, it can be concluded that compound (175) is only selective to A₃AR among 168 GPCRs.

### Example 10.3: A₃R selectivity study among ARs and A₃R functional assay

### MATERIAL AND METHODS

### Adenosine receptors antagonism assay (Eurofins) for A₃R selectivity study

Compound (175) was tested as potential antagonist on human recombinant A₁AR (expressed in CHO cells) in an assay based on the use of an agonist radioligand, [³H]-CCPA (1 nM) and 10 µM CPA. The assay was run at r.t. for 60 min in duplicates in 8-point dose-response curves.

Compound (175) was tested as potential antagonist on human recombinant A_{2A}AR (expressed in HEK-293 cells) in an assay based on the use of an agonist radioligand, [³H]-CGS (6 nM) and 10 µM NECA. The assay was run at r.t. for 120 min in duplicates in 8-point dose-response curves.

Compound (175) was tested as potential antagonist on human recombinant A_{2B}AR (expressed in HEK-293 cells) in an assay based on the use of an agonist radioligand, [³H]-CPX (5 nM) and 100 µM NECA. The assay was run at r.t. for 120 min in duplicates in 8-point dose-response curves.

IC₅₀ values were calculated as described above in example 10.1 and the inhibition constants (Ki) were calculated using the Cheng Prusoff equation.

### A₃AR functional assay

Human recombinant A₃AR expressed in CHO cells was used to evaluate compound (175) as a potential A₃AR agonist (no stimulus, 100 nM IB-MECA as positive control) or as a potential A₃AR antagonist (stimulation: 10 nM IB-MECA). Both assays were run for 20 min at 37°C, and cAMP levels measured by HTRF (homogeneous time resolved fluorescence) was used as an endpoint. The EC₅₀ values (concentration producing a half-maximal response) and IC₅₀ values (concentration causing a half-maximal inhibition of the control agonist response) were determined by non-linear regression analysis of the concentration-response curves generated with mean replicate values using Hill equation curve fitting.

### RESULTS

The results of a study evaluating the selectivity of compound (175) towards various ARs are depicted in figure 3. Compound (175) was tested as a potential antagonist of all four adenosine receptors, A₁AR, A_{2A}AR, A_{2B}AR and A₃AR. IC₅₀ values (expressed in µM) were calculated from the dose-response curves. In figure 3, Ki values (expressed in µM) are shown in parentheses, in bold and italics. The A₃AR experiment was repeated three times independently.

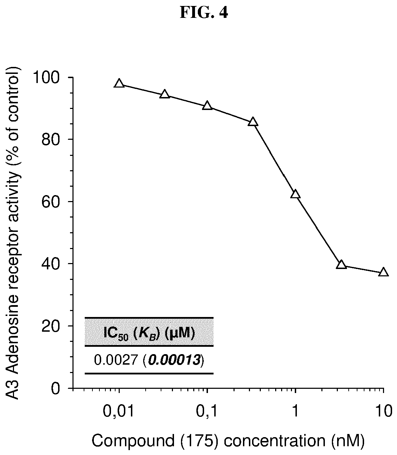

The results of the A₃AR functional assay is depicted in figure 4. It clearly demonstrates that compound (175) is an inhibitor of A₃AR, at least up to 65% under these conditions.

### CONCLUSION

As demonstrated in figure 3, compound (175) was found to be selective of A₃AR compared to other ARs notably A₁AR, A_{2A}AR and A_{2B}AR.

As demonstrates in figure 4, compound (175) acts as a partial antagonist inhibitor of A₃A.

### COMPLEMENTARY RESULTS

An antagonistic activity in a radioligand assay can be due to competition to the same site by either an agonist or an antagonist (inhibitor). Compound (175) was tested in a cellular functional assay using recombinant human A₃AR expressed in CHO cells in the absence of ligand (allowing the detection of an A₃AR agonist activity) or in the presence of an agonist (IB-MECA) (allowing the detection of an A₃AR antagonist activity). Assays were run for 20 min at 37°C and cAMP level measured by HTRF was used as an endpoint measure of the A₃AR activity. These experiments demonstrated that compound (175) behaves as an antagonist of A₃AR, displaying an IC₅₀ belonging to class D as defined in example 10.1. In contrast no agonist effect was detected (concentrations of compound (175) tested: 1 nM to 3.3 µM) (data not shown).

As a conclusion, compounds of formula (I) according to the present invention act as antagonists of A₃AR.

### Example 10.4: Otoprotective effects in ex vivo model of organs of Corti

### MATERIAL AND METHODS

### Biological reagents and organotypic culture

Organs of Corti were obtained from mice at the P0-P3 stage. Organs of Corti were cultured ex vivo for 24 h before addition of cisplatin (20 µM), gentamycin (100 µM) or neomycin (3 mM) with or without compound (175) (1 or 0.1 µM) added 2 hrs before the ototoxins. After 24 hrs incubation, outer and inner ciliated hair cells were stained with an antibody directed against myosine 7a.

### RESULTS

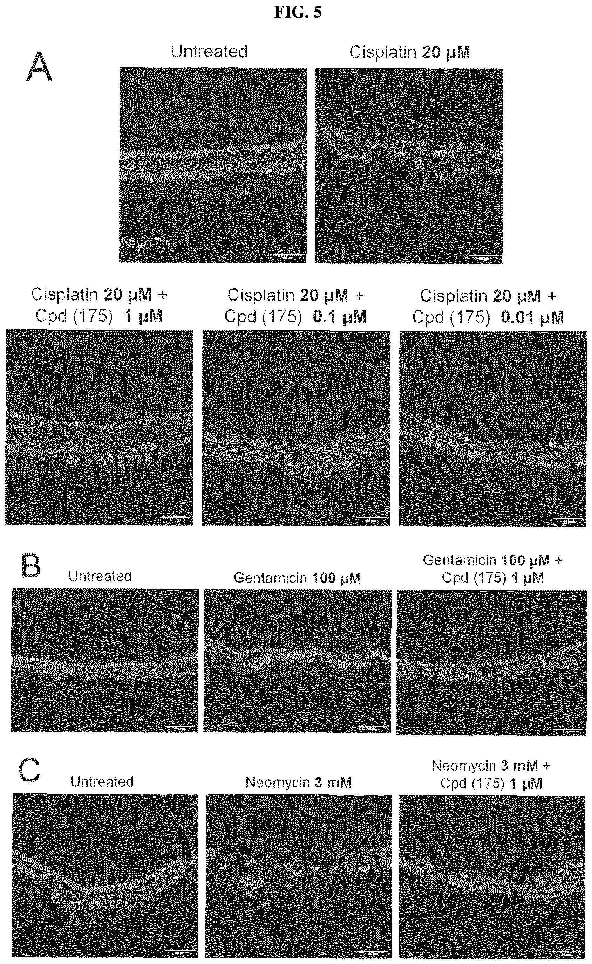

As expected, in figure 5, the anticancer drug cisplatin (figure 5A), or the aminoglycosides antibiotics gentamycin (figure 5B) and neomycin (figure 5C) induced the death of hair cells in isolated organs of Corti cultured *ex vivo.* Co-incubation with compound (175) (0.1-1 µM) prevented cell death induced by these three ototoxic agents. The levels of cell deaths between ototoxin- and ototoxin+ compound (175) were statistically different (data not shown).

### CONCLUSION

Based on the previous results, it can be concluded that compound (175) displays potent otoprotective effects towards death hair cells of organs of Corti induced by ototoxic agents, in particlar platinum-based anticancer drugs such as cisplatin, or by antibiotics, in particular aminoglycosides such as gentamycin and neomycin.

The present invention further relates to a pharmaceutical composition comprising at least one compound of formula (I) as defined above or any of its pharmaceutically acceptable salts, or at least any of compounds (1) to (220) as defined above or any of their pharmaceutically acceptable salts, and also at least one pharmaceutically acceptable excipient.

Pharmaceutical compositions of the invention can contain one or more compound(s) of the invention in any form described herein.

Still a further object of the present invention consists of the use of at least one compound of formula (I) as defined above, and compounds (1) to (220) as defined above, or one of their pharmaceutically acceptable salts according to the present invention for preparing a drug for preventing and/or treating diseases associated with A₃AR. According to a particular embodiment, the treatment is continuous or non-continuous.

A "continuous treatment" means a long-term treatment which can be implemented with various administration frequencies, such as once every day, every three days, once a week, or once every two weeks or once every month or by transdermal patch delivery.

According to one embodiment, the compound of formula (I), or any of its pharmaceutically acceptable salts, is administered at a dose varying from 0.1 to 1000 mg. in particular varying from 1 to 500 mg, or for example varying from 5 to 100 mg.

Another object of the invention relates to a therapeutic method for the treatment and/or for the prevention of diseases associated with A₃AR, and in particular for the treatment and/or for the prevention of ulcerative colitis, atopic dermatitis, glaucoma, asthma, liver fibrosis, kidney diseases in particular kidney fibrosis, nephropathy, acute kidney injury (AKI), chronic kidney disease (CKD) more particularly induced by ischemia and/or reperfusion injury, toxic nephropathy or myoglobinuria, diabetic kidney disease, kidney diseases induced by nephrotoxicity caused by therapeutic drugs, atherosclerosis, hypercholestemia, partial or a complete hearing loss induced by noise, acoustic trauma or ototoxic agents or conditions, and cancers developing under hypoxic conditions, in particular glioblastoma, consisting in administering to a patient in need thereof of a compound of formula (I) or any of its pharmaceutically acceptable salt or of at least any of compounds (1) to (220) as defined above, of any of their pharamecutically acceptable salt.

According to one embodiment, the invention relates to a therapeutic method for the treatment and/or for the prevention of hearing loss, in particular induced by ototoxic agents, as well as in the treatment and/or in the prevention of kidney diseases, in particular induced by nephrotoxicity, consisting in administering to a patient in need thereof of a compound of formula (I) or any of its pharmaceutically acceptable salt or of at least any of compounds (1) to (220) as defined above, of any of their pharamecutically acceptable salt.

According to one embodiment, the invention relates to a therapeutic method for the treatment and/or for the prevention of partial or a complete hearing loss, in particular induced by noise, acoustic trauma or ototoxic agents or conditions, more particularly induced by ototoxic agents or conditions, consisting in administering to a patient in need thereof of a compound of formula (I) or any of its pharmaceutically acceptable salt or of at least any of compounds (1) to (220) as defined above, of any of their pharamecutically acceptable salt.

According to one embodiment, the invention relates to a therapeutic method for the treatment and/or for the prevention of partial or a complete hearing loss induced by ototoxic agents or conditions as described hereinafter, consisting in administering to a patient in need thereof of a compound of formula (I) or any of its pharmaceutically acceptable salt or of at least any of compounds (1) to (220) as defined above, of any of their pharamecutically acceptable salt.

As ototoxic agents, i.e. causing hear loss, the following may be cited:
- solvents, such as polyethylene glycol, propylene glycol or benzalkonium chloride;
- antibiotics, in particular topical and/or systemic antibiotics, more particularly aminoglycosides, macrolides or phenicol antibiotics, such as gentamycin, neomycin, tobramycin, kanamycin, nystatin, polymyxin B, amphotericin B, bacitracin or chloramphenicol;
- anticancer drugs, in particular platinum-based anticancer drugs, such as cisplatin or carboplatin,
- antiseptics, in particular acetic acid, alcohols such as ethanol, chlorhexidine, cresylate, gentian violet or povidone iodine;
- nonsteroidal anti-inflammatory drugs (NSAIDs), in particular salicylates, cyclodextrins, indomethacin, ibuprofen, phenylbutazone or paracetamol;
- topical combinations, in particular polymyxin/neomycin/hydrocortisone or ticarcilline/clavulanate;
- drugs for COVID-19, in particular lopinavir or ritonavir;
- antimalarial drugs, in particular quinine or chloroquine;
- cardiovascular drugs, in particular loop diuretics; or
- erectile dysfunction drugs, in particular phosphodiesterase type 5 (PDE-5) inhibitors.

According to one embodiment, the invention relates to a therapeutic method for the treatment and/or for the prevention of hearing loss being a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors, consisting in administering to a patient in need thereof of a compound of formula (I) or any of its pharmaceutically acceptable salt or of at least any of compounds (1) to (220) as defined above, of any of their pharamecutically acceptable salt.

According to one embodiment, the invention relates to a therapeutic method for the treatment and/or for the prevention of kidney diseases, in particular selected from nephropathy, acute kidney injury (AKI), chronic kidney disease (CKD) more particularly induced by ischemia and/or reperfusion injury, toxic nephropathy or myoglobinuria, and diabetic kidney disease, or kidney diseases induced by nephrotoxicity caused by therapeutic drugs, consisting in administering to a patient in need thereof of a compound of formula (I) or any of its pharmaceutically acceptable salt or of at least any of compounds (1) to (220) as defined above, of any of their pharamecutically acceptable salt.

According to one embodiment, the invention relates to a therapeutic method for the treatment and/or for the prevention of kidney diseases induced by nephrotoxicity caused by therapeutic drugs as described hereinafter, consisting in administering to a patient in need thereof of a compound of formula (I) or any of its pharmaceutically acceptable salt or of at least any of compounds (1) to (220) as defined above, of any of their pharamecutically acceptable salt.

As therapeutic drugs causing nephrotoxicity, the following may be cited: acyclovir, ambisome, amikacin, aminoglycosides, antibiotics, amphotericin B, captopril, carboplatin, cefotaxime, ceftazidime, cefuroxime, cephalosporins, cidofovir, ciprofloxacin, cisplatin, colistimethate, cyclosporine, dapsone, enalaprilat, enalapril, Foscarnet, gadopentetate dimeglumine, gadoxetate, ganciclovir gentamicin, ibuprofen, ifosfamide, iodixanol, iohexol, iopamidol, ioversol, ketorolac, lisinopril, lithium, mesalamine, methotrexate, nafcillin disodium, penicillins, piperacillin/tazobactam, piperacillin, rifampin, sirolimus, sulfasalazine, tacrolimus, ticarcillin/clavulanic acid, tobramycin, topiramate, valacyclovir, valganciclovir, vancomycin or zonisamide.

All combinations of doses, frequencies and treatment period are encompassed within the scope of the present invention.

Various sequences or cycles of administration respectively of the compound of formula (I) as defined herein above, a pharmaceutically acceptable salt thereof, prodrug thereof may be implemented within the framework of the present invention.

The compounds of formula (I) as defined above can be administered through any mode of administration such as, for example, intramuscular, intravenous. intranasal or oral route, transtympanic, intra-cochlear, transdermal patch, etc.

The inventive composition can further include one or more additives such as diluents, excipients, stabilizers and preservatives. Such additives are well known to those skilled in the art and are described notably in *"*Ullmann's Encyclopedia of Industrial Chemistry, 6th Ed." (various editors, 1989-1998, Marcel Dekker) and in *"*Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) according to the present invention can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.

Oral route of administration is in particular preferred in the prophylaxis or treatment aspect of the invention.

## Claims

1. A compound of formula (I) or any of its pharmaceutically acceptable salt 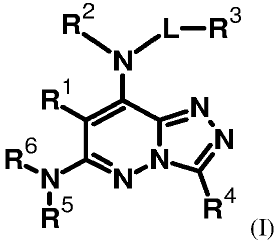 wherein
**R¹** represents a hydrogen atom or a (C₁-C₆)alkyl group;
**R²** represents a hydrogen atom;
**R³** represents
- a linear or branched (C₁-C₆)alkyl group, optionally substituted by
• one phenyl group, optionally substituted by at least one substituent selected from a -OR⁷ group, a -NH₂ group, a halogen atom, a phenyl group and a (C₅-C₆)heteroaryl group,
• one (C₆-C₁₀)heteroaryl group, or
• one -OR⁷ group,
- a phenyl group or a phenyl group fused with a (C₄-C₆)cycloalkyl group; or
- a (C₅-C₆)heteroaryl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom, in particular a fluorine atom or a chlorine atom;
**L** represents a bond, a -CO- group or a -C(O)O- group;
or alternately **L** is a bond and **R²** and **R³** form together with the nitrogen atom bearing them a (C₅-C₆)heterocycloalkyl group containing at least a nitrogen atom fused with a phenyl group;
**R⁴** represents a hydrogen atom, a linear or branched (C₁-C₆)alkyl group, a (C₃-C₆)cycloalkyl group, a (C₁-C₄)alkoxy group, a (C₅-C₆)heterocyclocalkyl group, a phenyl group, a (C₅-C₆)heteroaryl group or a -CF₃ group;
**R⁵** represents a hydrogen atom or a (C₁-C₄)alkyl group;
**R⁶** represents
- a linear or branched (C₁-C₆)alkyl group, optionally interrupted by one or two oxygen atoms and optionally substituted by
• one phenyl group, optionally substituted by one or two substituents selected from a (C₁-C₄)alkyl group and a (C₁-C₄)alkoxy group,
• one (C₃-C₈)cycloalkyl group, optionally substituted by one or two substituents selected from a halogen atom, a (C₁-C₄)alkyl group and a -OR⁷ group,
• one (C₅-C₆)heterocycloalkyl,
• one (C₅-C₆)heteroaryl group, optionally substituted by one or two (C₁-C₆)alkyl group,
• one or two -OR⁷ groups,
• one -SR⁷ group,
• one -C(O)NR⁸R⁹ group,
• one -C(O)OR¹⁰ group, or
• one -NHR¹¹ group,
- a (C₃-C₇)cycloalkyl group, optionally substituted by one or two substituents selected from a halogen atom, a (C₁-C₄)alkyl group and a -OR⁷ group,
- one bridged (C₆-C₁₀)cycloalkyl group,
- one spiro(C₅-C₁₁)bicyclic ring,
- a (C₅-C₆)heteroaryl group,
- a (C₅-C₆)heterocycloalkyl group, or
- a phenyl group fused with a (C₄-C₆)cycloalkyl group;
or **R⁵** and **R⁶** form together with the nitrogen atom bearing them a (C₅-C₆)heterocycloalkyl group;
**R⁷** represents a hydrogen atom, a (C₁-C₄)alkyl group or a deuterated (C₁-C₄)alkyl group;
**R⁸** and **R⁹** independently represent a hydrogen atom or a (C₁-C₆)alkyl group;
**R¹⁰** represents a (C₁-C₄)alkyl group; and
**R¹¹** represents a hydrogen atom or a -CO-(C₁-C₆)alkyl group;
provided that when NR²LR³ represents a benzylamino group, then R⁴ does not represent a hydrogen atom.

2. The compound of formula (I) or any of its pharmaceutically acceptable salt as defined in claim 1, wherein **R¹** represents a hydrogen atom or a methyl group.

3. The compound of formula (I) or any of its pharmaceutically acceptable salt, as defined in claim 1 or 2, wherein
**R²** represents a hydrogen atom;
**R³** represents
- a linear or branched (C₁-C₆)alkyl group, optionally substituted by
• one phenyl group, optionally substituted by one substituent selected from a hydroxy group, a methoxy group, an amino group, a halogen atom, a phenyl group and a pyridyl group,
• one pyridyl, one pyrazinyl, one pyrimidinyl or one pyridazinyl group, in particular one pyridyl, pyridazinyl or pyrimidinyl group, or one indolyl or isoindolyl group, in particular an indolyl group,
• one hydroxy group,
• one methoxy group, one deuterated methoxy group, or
• a halogen atom, in particular a fluorine atom or a chlorine atom,
- a phenyl group or an indanyl group, or
- a pyridyl, a pyrazinyl, a pyrimidinyl or a pyridazinyl group, optionally substituted by one methoxy group or deuterated methoxy group, in particular a pyridyl group;
**L** represents a bond, a -CO- group or a -C(O)O- group;
or alternately **L** is a bond and **R²** and **R³** form together with the nitrogen atom bearing them an indolinyl, an isoindolinyl, a tetrahydroquinolinyl or a tetrahydroisoquinolinyl group, in particular an isoindolinyl or a tetrahydroisoquinolinyl group.

4. The compound of formula (I) or any of its pharmaceutically acceptable salt, as defined in any claim 1 to 3, wherein **R⁴** represents a hydrogen atom, a linear or branched (C₁-C₅)alkyl group, a (C₃-C₆)cycloalkyl group, a phenyl group, a pyridyl group or a -CF₃ group.

5. The compound of formula (I) or any of its pharmaceutically acceptable salt, as defined in any claim 1 to 4, wherein
**R⁵ represents** a hydrogen atom or a (C₁-C₄) alkyl group, in particular a methyl group;
**R⁶** represents
- a linear or branched (C₁-C₆)alkyl group, optionally interrupted by one or two oxygen atoms and optionally substituted by
• one phenyl group, optionally substituted by one or two substituents selected from a methyl group and a methoxy group,
• one (C₃-C₆)cycloalkyl group, optionally substituted by a methyl group,
• one tetrahydropyranyl or one tetrahydrofuranyl group,
• one pyrazolyl, imidazolyl, pyrimidinyl, pyrazinyl, pyridyl, furanyl or thienyl group, optionally substituted by one or two methyl group,
• one or two hydroxy group or methoxy group,
• one methylthio group,
• one -CONH₂ group, one -CONHCH₃ group or one -CON(CH₃)₂ group,
• one -COOCH₃ or one -COOCH₂CH₃ group, or
• one -NH₂ group or one -NCOCH₃ group,
- a (C₃-C₇)cycloalkyl group, optionally substituted by one or two substituents selected from a halogen atom, a hydroxy group or a methoxy group,
- an adamantyl group,
- a spiro[3.3]heptanyl group,
- an imidazolyl group, optionally substituted by one or two methyl group,
- a morpholinyl group, a tetrahydropyranyl or a tetrahydrofuranyl group, or
- an indanyl group;
or **R⁵** and **R⁶** form together with the nitrogen atom bearing them a morpholinyl group or a piperidinyl group.

6. A compound of formula (I) as defined in anyone of claims 1 to 5, or any of its pharmaceutically acceptable salt, selected from
**(1)** (2*R*)-2-[[8-(benzylamino)-3-isopropyl-[1,2,4]triazolo[4,3-*b*]pyridazin-6-yl]amino]butan-1-ol,
**(2)** (2*S*)-2-[[8-(benzylamino)-3-isopropyl-[1,2,4]triazolo[4,3-*b*]pyridazin-6-yl]amino]butan-1-ol,
**(3)** 2-[[8-(benzylamino)-3-isopropyl-[1,2,4]triazolo[4,3-*b*]pyridazin-6-yl]amino]propane-1,3-diol,
**(4)** (2*R*,3*R*)-2-[[8-(benzylamino)-3-isopropyl-[1,2,4]triazolo[4,3-*b*]pyridazin-6-yl]amino]butane-1,3-diol,
**(5)** (2*S*,3*S*)-2-[[8-(benzylamino)-3-isopropyl-[1,2,4]triazolo[4,3-*b*]pyridazin-6-yl]amino]butane-1,3-diol,
**(6)** *N*-benzyl-3-isopropyl-6-morpholino-[1,2,4]triazolo[4,3-*b*]pyridazin-8-amine
**(7)** (2*S*)-3-[[8-(benzylamino)-3-isopropyl-[1,2,4]triazolo[4,3-*b*]pyridazin-6-yl]amino]propane-1,2-diol,
**(8)** *N*6-[2-[2-(2-aminoethoxy)ethoxy]ethyl]-*N*8-benzyl-3-isopropyl-[1,2,4] triazolo [4,3-*b*]pyridazine-6,8-diamine,
**(9)** 3-[[8-(benzylamino)-3-isopropyl-[1,2,4]triazolo[4,3-*b*]pyridazin-6-yl]amino]propan-1-ol,
**(10)** *N*8-benzyl-3-isopropyl-*N*6-(3-methoxypropyl)-[1,2,4]triazolo[4,3-*b*]pyridazine-6,8-diamine,
**(11)** 2-[[8-(benzylamino)-3-isopropyl-[1,2,4]triazolo[4,3-*b*]pyridazin-6-yl] amino] ethanol,
**(12)** *N8*-benzyl-3-isopropyl-*N6*-(2-methoxyethyl)-[1,2,4]triazolo[4,3-*b*]pyridazine-6,8-diamine,
**(13)** 4-[[8-(benzylamino)-3-isopropyl-[1,2,4]triazolo[4,3-*b*]pyridazin-6-yl]amino]butan-1-ol,
**(14)** *N8*-benzyl-3-isopropyl-*N6*-(4-methoxybutyl)-[1,2,4]triazolo[4,3-*b*]pyridazine-6,8-diamine,
**(15)** 5-[[8-(benzylamino)-3-isopropyl-[1,2,4]triazolo[4,3-*b*]pyridazin-6-yl]amino]pentan-1-ol,
**(16)** *N8*-benzyl-3-isopropyl-*N6*-(5-methoxypentyl)-[1,2,4]triazolo[4,3-*b*]pyridazine-6,8-diamine,
**(17)** *N8*-benzyl-3-isopropyl-*N6*-(3-methylsulfanylpropyl)-[1,2,4]triazolo[4,3-*b*]pyridazine-6,8-diamine,
**(18)** *N8*-benzyl-*N6*-(1-ethylpropyl)-3-isopropyl-[1,2,4]triazolo[4,3-*b*]pyridazine-6,8-diamine,
**(19)** *N8*-benzyl-3-isopropyl-*N6*-[(3*R*)-tetrahydrofuran-3-yl]-[1,2,4]triazolo[4,3-*b*]pyridazine-6,8-diamine,
**(20)** *N8*-benzyl-3-cyclopentyl-*N6*-[(3*R*)-tetrahydrofuran-3-yl]-[1,2,4]triazolo[4,3-*b*]pyridazine-6,8-diamine,
**(21)** *N8*-benzyl-3-isopropyl-*N6*-[(3*R*)-tetrahydropyran-3-yl]-[1,2,4]triazolo[4,3-*b*]pyridazine-6,8-diamine,
**(22)** *N8*-benzyl-3-isopropyl-*N6*-[(3*S*)-tetrahydropyran-3-yl]-[1,2,4]triazolo[4,3-*b*]pyridazine-6,8-diamine,
**(23)** *N8*-benzyl-3-isopropyl-*N6*-tetrahydropyran-4-yl-[1,2,4]triazolo[4,3-*b*]pyridazine-6,8-diamine,
**(24)** *N*-[3-[[8-(benzylamino)-3-isopropyl-[1,2,4]triazolo[4,3-*b*]pyridazin-6-yl] amino]propyl] acetamide,
**(25)** 3-[[8-(benzylamino)-3-isopropyl-[1,2,4]triazolo[4,3-*b*]pyridazin-6-yl]amino]propanamide,
**(26)** 3-[[8-(benzylamino)-3-isopropyl-[1,2,4]triazolo[4,3-*b*]pyridazin-6-yl]amino]-*N-*methyl-propanamide,
**(27)** 3-[[8-(benzylamino)-3-isopropyl-[1,2,4]triazolo[4,3-*b*]pyridazin-6-yl]amino]-*N*,*N*-dimethyl-propanamide,
**(28)** Methyl 3-[[8-(benzylamino)-3-isopropyl-[1,2,4]triazolo[4,3-*b*]pyridazin-6-yl]amino]propanoate,
**(29)** ethyl 3-[[8-(benzylamino)-3-isopropyl-[1,2,4]triazolo[4,3-*b*]pyridazin-6-yl]amino]propanoate,
**(30)** (2*R*)-2-[[8-(benzylamino)-3-cyclopropyl-[1,2,4]triazolo[4,3-*b*]pyridazin-6-yl]amino]butan-1-ol,
**(31)** *N8*-benzyl-3-cyclopropyl-*N6*-(3-methoxypropyl)-[1,2,4]triazolo[4,3-*b*]pyridazine-6,8-diamine,
**(32)** *N8*-benzyl-*N6*-(3-methoxypropyl)-3-methyl-[1,2,4]triazolo[4,3-*b*]pyridazine-6,8-diamine,
**(33)** (2*R*)-2-[[8-(benzylamino)-3-methyl-[1,2,4]triazolo[4,3-*b*]pyridazin-6-yl]amino]butan-1-ol,
**(34)** *N8*-benzyl-*N6*-(3-methoxypropyl)-3-phenyl-[1,2,4]triazolo[4,3-*b*]pyridazine-6,8-diamine,
**(35)** (2*R*)-2-[[8-(benzylamino)-3-phenyl-[1,2,4]triazolo[4,3-*b*]pyridazin-6-yl]amino]butan-1-ol,
**(36)** *N8*-benzyl-*N6*-(3-methoxypropyl)-3-(trifluoromethyl)-[1,2,4]triazolo[4,3-*b*]pyridazine-6,8-diamine,
**(37)** (*2R*)-2-[[8-(benzylamino)-3-(trifluoromethyl)-[1,2,4]triazolo[4,3-*b*]pyridazin-6-yl]amino]butan-1-ol,
**(38)** 2-[[[6-[[(1*R*)-1-(hydroxymethyl)propyl]amino]-3-isopropyl-[1,2,4]triazolo[4,3-b]pyridazin-8-yl] amino] methyl]phenol,
**(39)** 2-[[[3-isopropyl-6-(3-methoxypropylamino)-[1,2,4]triazolo[4,3-*b*]pyridazin-8-yl]amino] methyl]phenol,
**(40)** 2-[[[6-(1-ethylpropylamino)-3-isopropyl-[1,2,4]triazolo[4,3-*b*]pyridazin-8-yl]amino] methyl]phenol,
**(41)** 3-[[[6-(1-ethylpropylamino)-3-isopropyl-[1,2,4]triazolo[4,3-*b*]pyridazin-8-yl]amino] methyl]phenol,
**(42)** 4-[[[6-(1-ethylpropylamino)-3-isopropyl-[1,2,4]triazolo[4,3-*b*]pyridazin-8-yl]amino] methyl]phenol,
**(43)** 2-[[[3-isopropyl-6-[[(3*R*)-tetrahydropyran-3-yl]amino]-[1,2,4]triazolo[4,3-*b*]pyridazin-8-yl]amino]methyl]phenol,
**(44)** 2-[[[3-isopropyl-6-(tetrahydropyran-4-ylamino)-[1,2,4]triazolo[4,3-*b*]pyridazin-8-yl]amino]methyl]phenol,
**(45)** *N6*-(1-ethylpropyl)-3-isopropyl-*N8*-phenyl-[1,2,4]triazolo[4,3-*b*]pyridazine-6,8-diamine,
**(46)** *N6*-(1-ethylpropyl)-3-isopropyl-*N8*-(2-pyridyl)-[1,2,4]triazolo[4,3-*b*]pyridazine-6,8-diamine,
**(47)** *N6*-(1-ethylpropyl)-3-isopropyl-*N8*-(3-pyridyl)-[1,2,4]triazolo[4,3-*b*]pyridazine-6,8-diamine,
**(48)** *N6*-(1-ethylpropyl)-3-isopropyl-*N8*-(4-pyri_{d}yl)-[1,2,4]triazolo[4,3-*b*]pyridazine-6,8-diamine,
**(49)** *N6*-(1-ethylpropyl)-3-isopropyl-*N8*-(4-pyridylmethyl)-[1,2,4]triazolo[4,3-*b*]pyridazine-6,8-diamine,
**(50)** *N6*-(1-ethylpropyl)-3-isopropyl-*N8*-(3-pyridylmethyl)-[1,2,4]triazolo[4,3-*b*]pyridazine-6,8-diamine,
**(51)** *N6*-(1-ethylpropyl)-3-isopropyl-*N8*-(2-pyridylmethyl)-[1,2,4]triazolo[4,3-*b*]pyridazine-6,8-diamine,
**(52)** *N6*-tert-butyl-3-isopropyl-*N8*-(2-pyridylmethyl)-[1,2,4]triazolo[4,3-*b*]pyridazine-6,8-diamine,
**(53)** 3-isopropyl-*N8*-(2-pyridylmethyl)-*N6*-spiro[3.3]heptan-2-yl-[1,2,4]triazolo[4,3-*b*]pyridazine-6,8-diamine,
**(54)** *N6*-(3,3-difluorocyclobutyl)-3-isopropyl-*N8*-(2-pyridylmethyl)-[1,2,4]triazolo[4,3-*b*]pyridazine-6,8-diamine,
**(55)** *N6*-(4,4-difluorocyclohexyl)-3-isopropyl-*N8*-(2-pyridylmethyl)-[1,2,4]triazolo[4,3-*b*]pyridazine-6,8-diamine,
**(56)** *N6*-benzyl-3-isopropyl-*N8*-(2-pyridylmethyl)-[1,2,4]triazolo[4,3-*b*]pyridazine-6,8-diamine,
**(57)** *N6*-(1-ethylpropyl)-*N8*-(2-pyridylmethyl)-[1,2,4]triazolo[4,3-*b*]pyridazine-6,8-diamine,
**(58)** *N6*-(1-ethylpropyl)-3-methyl-*N8*-(2-pyridylmethyl)-[1,2,4]triazolo[4,3-*b*]pyridazine-6,8-diamine,
**(59)** 3-ethyl-*N6*-(1-ethylpropyl)-*N8-*(2-pyridylmethyl)-[1,2,4]triazolo[4,3-*b*]pyridazine-6,8-diamine,
**(60)** *N6*-(1-ethylpropyl)-3-propyl-*N8*-(2-pyridylmethyl)-[1,2,4]triazolo[4,3-*b*]pyridazine-6,8-diamine,
**(61)** 3-tert-butyl-*N6*-(1-ethylpropyl)-*N8*-(2-pyridylmethyl)-[1,2,4]triazolo[4,3-*b*]pyridazine-6,8-diamine,
**(62)** 3-cyclopropyl-*N6*-(1-ethylpropyl)-*N8*-(2-pyridylmethyl)-[1,2,4]triazolo[4,3-*b*]pyridazine-6,8-diamine,
**(63)** 3-cyclobutyl-*N6*-(1-ethylpropyl)-*N8*-(2-pyridylmethyl)-[1,2,4]triazolo[4,3-*b*]pyridazine-6,8-diamine,
**(64)** 3-cyclopentyl-*N6*-(1-ethylpropyl)-*N8*-(2-pyridylmethyl)-[1,2,4]triazolo[4,3-*b*]pyridazine-6,8-diamine,
**(65)** 3-cyclohexyl-*N6*-(1-ethylpropyl)-*N8*-(2-pyridylmethyl)-[1,2,4]triazolo[4,3-*b*]pyridazine-6,8-diamine,
**(66)** *N6*-(1-ethylpropyl)-3-phenyl-*N8*-(2-pyridylmethyl)-[1,2,4]triazolo[4,3-*b*]pyridazine-6,8-diamine,
**(67)** *N6*-(1-ethylpropyl)-*N8*-(2-pyridylmethyl)-3-sec-butyl-[1,2,4]triazolo[4,3-*b*]pyridazine-6,8-diamine,
**(68)** *N6*,3-bis(1-ethylpropyl)-*N8*-(2-pyridylmethyl)-[1,2,4]triazolo[4,3-*b*]pyridazine-6,8-diamine,
**(69)** *N6*-(1-ethylpropyl)-3-isobutyl-*N8*-(2-pyridylmethyl)-[1,2,4]triazolo[4,3-*b*]pyridazine-6,8-diamine,
**(70)** *N6*-(1-ethylpropyl)-3-(2-pyridyl)-*N8*-(2-pyridylmethyl)-[1,2,4]triazolo[4,3-*b*]pyridazine-6,8-diamine,
**(71)** *N6*-(1-ethylpropyl)-3-(3-pyridyl)-*N8*-(2-pyridylmethyl)-[1,2,4]triazolo[4,3-*b*]pyridazine-6,8-diamine,
**(72)** *N6*-(1-ethylpropyl)-3-(4-pyridyl)-*N8*-(2-pyridylmethyl)-[1,2,4]triazolo[4,3-*b*]pyridazine-6,8-diamine,
**(73)** *N6*-(1-ethylpropyl)-3-isopropyl-*N8*-[(2-methoxyphenyl)methyl]-[1,2,4]triazolo[4,3-*b*]pyridazine-6,8-diamine,
**(74)** (*2R*)-2-[[8-[(4-aminophenyl)methylamino]-3-isopropyl-[1,2,4]triazolo[4,3-*b*]pyridazin-6-yl]amino]butan-1-ol,
**(75)** *N8*-[(4-aminophenyl)methyl]-*N6*-(1-ethylpropyl)-3-isopropyl-[1,2,4]triazolo[4,3-*b*]pyridazine-6,8-diamine,
**(76)** *N8*-[(3-aminophenyl)methyl]-*N6*-(1-ethylpropyl)-3-isopropyl-[1,2,4]triazolo[4,3-*b*]pyridazine-6,8-diamine,
**(77)** *N8*-[(2-aminophenyl)methyl]-*N6*-(1-ethylpropyl)-3-isopropyl-[1,2,4]triazolo[4,3-*b*]pyridazine-6,8-diamine,
**(78)** *N6*-(1-ethylpropyl)-*N8*-(*1H*-indol-2-ylmethyl)-3-isopropyl-[1,2,4]triazolo[4,3-*b*]pyridazine-6,8-diamine,
**(79)** *N*-(1-ethylpropyl)-8-isoindolin-2-yl-3-isopropyl-[1,2,4]triazolo[4,3-*b*]pyridazin-6-amine,
**(80)** 8-(3,4-dihydro-*1H*-isoquinolin-2-yl)-*N*-(1-ethylpropyl)-3-isopropyl-[1,2,4]triazolo[4,3-*b*]pyridazin-6-amine,
**(81)** *N6*-(1-ethylpropyl)-*N8*-indan-1-yl-3-isopropyl-[1,2,4]triazolo[4,3-*b*]pyridazine-6,8-diamine,
**(82)** *N6*-(1-ethylpropyl)-3-isopropyl-*N8*-(2-phenylethyl)-[1,2,4]triazolo[4,3-*b*]pyridazine-6,8-diamine,
**(83)** *N6*-(1-ethylpropyl)-3-isopropyl-*N8*-[2-(2-pyridyl)ethyl]-[1,2,4]triazolo[4,3-*b*]pyridazine-6,8-diamine,
**(84)** *N6*-(1-ethylpropyl)-3-isopropyl-*N8*-[2-(3-pyridyl)ethyl]-[1,2,4]triazolo[4,3-*b*]pyridazine-6,8-diamine,
**(85)** *N6*-(1-ethylpropyl)-3-isopropyl-*N8*-[2-(4-pyridyl)ethyl]-[1,2,4]triazolo[4,3-*b*]pyridazine-6,8-diamine,
**(86)** 3-cyclopropyl-*N6*-(1-ethylpropyl)-*N8*-[2-(2-pyridyl)ethyl]-[1,2,4]triazolo[4,3-*b*]pyridazine-6,8-diamine,
**(87)** 3-cyclopropyl-*N6*-(1-ethylpropyl)-*N8*-[2-(3-pyridyl)ethyl]-[1,2,4]triazolo[4,3-*b*]pyridazine-6,8-diamine,
**(88)** 3-cyclopropyl-*N6*-(1-ethylpropyl)-*N8*-[2-(4-pyridyl)ethyl]-[1,2,4]triazolo[4,3-*b*]pyridazine-6,8-diamine,
**(89)** *N6*-(1-ethylpropyl)-3-isopropyl-*N8*-[3-(2-pyridyl)propyl]-[1,2,4]triazolo[4,3-*b*]pyridazine-6,8-diamine,
**(90)** *N6*-(1-ethylpropyl)-3-isopropyl-*N8*-[3-(3-pyridyl)propyl]-[1,2,4]triazolo[4,3-*b*]pyridazine-6,8-diamine,
**(91)** *N6*-(1-ethylpropyl)-3-isopropyl-*N8*-[3-(4-pyridyl)propyl]-[1,2,4]triazolo[4,3-*b*]pyridazine-6,8-diamine
**(92)** *N6*-(1-ethylpropyl)-3-isopropyl-*N8*-(pyrimidin-2-ylmethyl)-[1,2,4]triazolo[4,3-*b*]pyridazine-6,8-diamine,
**(93)** *N6*-(1-ethylpropyl)-3-isopropyl-*N8*-(pyrimidin-5-ylmethyl)-[1,2,4]triazolo[4,3-*b*]pyridazine-6,8-diamine,
**(94)** *N6*-(1-ethylpropyl)-3-isopropyl-*N8*-(pyrazin-2-ylmethyl)-[1,2,4]triazolo[4,3-*b*]pyridazine-6,8-diamine,
**(95)** *N6*-(1-ethylpropyl)-3-isopropyl-*N8*-(pyridazin-3-ylmethyl)-[1,2,4]triazolo[4,3-*b*]pyridazine-6,8-diamine,
**(96)** *N*-[6-(1-ethylpropylamino)-3-isopropyl-[1,2,4]triazolo[4,3-*b*]pyridazin-8-yl]benzamide,
**(97)** *N*-[6-(1-ethylpropylamino)-3-isopropyl-[1,2,4]triazolo[4,3-*b*]pyridazin-8-yl]pyridine-2-carboxamide,
**(98)** *N*-[6-(1-ethylpropylamino)-3-isopropyl-[1,2,4]triazolo[4,3-*b*]pyridazin-8-yl]-2-phenyl-acetamide,
**(99)** ethyl *N*-[6-(1-ethylpropylamino)-3-isopropyl-[1,2,4]triazolo[4,3-*b*]pyridazin-8-yl]carbamate,
**(100)** phenyl *N*-[6-(1-ethylpropylamino)-3-isopropyl-[1,2,4]triazolo[4,3-*b*]pyridazin-8-yl]carbamate,
**(101)** (2*S*)-2-[[8-[(4-bromophenyl)methylamino]-3-isopropyl-[1,2,4]triazolo[4,3-*b*]pyridazin-6-yl]amino]butan-1-ol,
**(102)** (2*S*)-2-[[3-isopropyl-8-[(4-phenylphenyl)methylamino]-[1,2,4]triazolo[4,3-*b*]pyridazin-6-yl] amino]butan-1-ol,
**(103)** (2*S*)-2-[[3-isopropyl-8-[[4-(4-pyridyl)phenyl]methylamino]-[1,2,4]triazolo[4,3-*b*]pyridazin-6-yl] amino]butan-1-ol,
**(104)** (2*S*)-2-[[3-isopropyl-8-[[4-(3-pyridyl)phenyl]methylamino]-[1,2,4]triazolo[4,3-*b*]pyridazin-6-yl] amino]butan-1-ol,
**(105)** (2*S*)-2-[[3-isopropyl-8-[[4-(2-pyridyl)phenyl]methylamino]-[1,2,4]triazolo[4,3-*b*]pyridazin-6-yl] amino]butan-1-ol,
**(106)** (2*R*,3*R*)-2-[[8-[(4-bromophenyl)methylamino]-3-isopropyl-[1,2,4]triazolo[4,3-*b*]pyridazin-6-yl] amino]butane-1,3-diol,
**(107)** (2*R*,3*R*)-2-[[3-isopropyl-8-[[4-(2-pyridyl)phenyl]methylamino]-[1,2,4]triazolo[4,3-*b*]pyridazin-6-yl] amino]butane-1,3-diol,
**(108)** (2*R*,3*R*)-2-[[3-isopropyl-8-[[4-(4-pyridyl)phenyl]methylamino]-[1,2,4] triazolo[4,3-*b*]pyridazin-6-yl] amino]butane-1,3-diol,
**(109)** *N6*,*N8*-bis(3-methoxypropyl)-3-phenyl-[1,2,4]triazolo[4,3-*b*]pyridazine-6,8-diamine,
**(110)** 3-cyclopropyl-*N6*,*N8*-bis(3-methoxypropyl)-[1,2,4]triazolo[4,3-*b*]pyridazine-6,8-diamine,
**(111)** *N6*,*N8*-bis(3-methoxypropyl)-3-(trifluoromethyl)-[1,2,4]triazolo[4,3-*b*]pyridazine-6,8-diamine,
**(112)** 3-isopropyl-*N6*,*N8*-bis(2-methoxyethyl)-[1,2,4]triazolo[4,3-*b*]pyridazine-6,8-diamine,
**(113)** (2*R*)-2-[[6-[[(1*R*)-1-(hydroxymethyl)propyl]amino]-3-isopropyl-[1,2,4] triazolo[4,3-*b*]pyridazin-8-yl] amino]butan-1-ol,
**(114)** *N6*,*N8*-bis(1-ethylpropyl)-3-isopropyl-[1,2,4]triazolo[4,3-*b*]pyridazine-6,8-diamine,
**(115)** (2*R*)-2-[[8-(benzylamino)-3-isopropyl-7-methyl-[1,2,4]triazolo[4,3-*b*]pyridazin-6-yl] amino]butan-1-ol,
**(116)** *N8*-[(4-bromophenyl)methyl]-3-isopropyl-*N6*-methyl-[1,2,4]triazolo[4,3-*b*]pyridazine-6,8-diamine,
**(117)** *N8*-benzyl-*N6*-cyclobutyl-3-isopropyl-[1,2,4]triazolo[4,3-*b*]pyridazine-6,8-diamine,
**(118)** *N8*-benzyl-*N6*-cyclopentyl-3-isopropyl-[1,2,4]triazolo[4,3-*b*]pyridazine-6,8-diamine,
**(119)** *N8*-benzyl-*N6*-cyclohexyl-3-isopropyl-[1,2,4]triazolo[4,3-*b*]pyridazine-6,8-diamine,
**(120)** *N8*-benzyl-3-isopropyl-*N6*-(4-methoxycyclohexyl)-[1,2,4]triazolo[4,3-*b*]pyridazine-6,8-diamine,
**(121)** *N8*-benzyl-*N6*-cycloheptyl-3-isopropyl-[1,2,4]triazolo[4,3-*b*]pyridazine-6,8-diamine,
**(122)** *N8*-benzyl-*N6*-(cyclopropylmethyl)-3-isopropyl-[1,2,4]triazolo[4,3-*b*]pyridazine-6,8-diamine,
**(123)** *N8*-benzyl-*N6*-(cyclobutylmethyl)-3-isopropyl-[1,2,4]triazolo[4,3-*b*]pyridazine-6,8-diamine,
**(124)** *N8*-benzyl-*N6*-(cyclohexylmethyl)-3-isopropyl-[1,2,4]triazolo[4,3-*b*]pyridazine-6,8-diamine,
**(125)** *N8*-benzyl-3-isopropyl-*N6*-(tetrahydropyran-4-ylmethyl)-[1,2,4]triazolo[4,3-*b*]pyridazine-6,8-diamine,
**(126)** *N8*-benzyl-*N6*-(2-ethylbutyl)-3-isopropyl-[1,2,4]triazolo[4,3-*b*]pyridazine-6,8-diamine,
**(127)** *N8*-benzyl-3-isopropyl-*N6*-(2-phenylethyl)-[1,2,4]triazolo[4,3-*b*]pyridazine-6,8-diamine,
**(128)** *N8*-benzyl-3-isopropyl-*N6*-(2-phenoxyethyl)-[1,2,4]triazolo[4,3-*b*]pyridazine-6,8-diamine,
**(129)** *N8*-benzyl-3-isopropyl-*N6*-[(1-methylimidazol-2-yl)methyl]-[1,2,4]triazolo[4,3-*b*]pyridazine-6,8-diamine,
**(130)** *N6*,*N8*-dibenzyl-3-isopropyl-[1,2,4]triazolo[4,3-*b*]pyridazine-6,8-diamine,
**(131)** *N8*-benzyl-3-isopropyl-*N6*-(2-pyridylmethyl)-[1,2,4]triazolo[4,3-*b*]pyridazine-6,8-diamine,
**(132)** *N8*-benzyl-3-isopropyl-*N6*-(3-pyridylmethyl)-[1,2,4]triazolo[4,3-*b*]pyridazine-6,8-diamine,
**(133)** *N8*-benzyl-3-isopropyl-*N6*-(4-pyridylmethyl)-[1,2,4]triazolo[4,3-*b*]pyridazine-6,8-diamine,
**(134)** racemic trans-2-[[8-(benzylamino)-3-isopropyl-[1,2,4]triazolo[4,3-*b*]pyridazin-6-yl] amino] cyclohexanol,
**(135)** racemic trans-4-[[8-(benzylamino)-3-isopropyl-[1,2,4]triazolo[4,3-*b*]pyridazin-6-yl] amino] cyclohexanol,
**(136)** *N8*-benzyl-3-isopropyl-*N6*-[(1-methylcyclohexyl)methyl]-[1,2,4]triazolo[4,3-*b*]pyridazine-6,8-diamine,
**(137)** *N6*-(1-adamantyl)-*N8*-benzyl-3-isopropyl-[1,2,4]triazolo[4,3-*b*]pyridazine-6,8-diamine,
**(138)** *N8*-benzyl-*N6*-indan-2-yl-3-isopropyl-[1,2,4]triazolo[4,3-*b*]pyridazine-6,8-diamine,
**(139)** *N8*-benzyl-3-isopropyl-*N6*-[(5-methylpyrazin-2-yl)methyl]-[1,2,4]triazolo[4,3-*b*]pyridazine-6,8-diamine,
**(140)** *N8*-benzyl-3-isopropyl-*N6*-[(1-methylpyrazol-4-yl)methyl]-[1,2,4]triazolo[4,3-*b*]pyridazine-6,8-diamine,
**(141)** *N8*-benzyl-*N6*-[(3,5-dimethylphenyl)methyl]-3-isopropyl-[1,2,4]triazolo[4,3-*b*]pyridazine-6,8-diamine,
**(142)** *N8*-benzyl-3-isopropyl-*N6*-(tetrahydrofuran-2-ylmethyl)-[1,2,4]triazolo[4,3-*b*]pyridazine-6,8-diamine,
**(143)** *N8*-benzyl-3-isopropyl-*N6*-(2-methylsulfanylethyl)-[1,2,4]triazolo[4,3-*b*]pyridazine-6,8-diamine,
**(144)** *N8*-benzyl-3-isopropyl-*N6*-(1-methylimidazol-2-yl)-[1,2,4]triazolo[4,3-*b*]pyridazine-6,8-diamine,
**(145)** *N8*-benzyl-*N6*-[(4,6-dimethylpyrimidin-2-yl)methyl]-3-isopropyl-[1,2,4] triazolo[4,3-*b*]pyridazine-6,8-diamine,
**(146)** *N8*-benzyl-*N6*-indan-1-yl-3-isopropyl-[1,2,4]triazolo[4,3-*b*]pyridazine-6,8-diamine,
**(147)** *N6*-(2-ethylbutyl)-3-isopropyl-*N8*-(pyridazin-3-yl)-[1,2,4]triazolo[4,3-*b*]pyridazine-6,8-diamine,
**(148)** 3-isopropyl-*N6*-(pentan-3-yl)-*N8*-(pyridazin-3-yl)-[1,2,4]triazolo[4,3-*b*]pyridazine-6,8-diamine,
**(149)** 3-isopropyl-*N6*-(pentan-3-yl)-*N8*-(pyridazin-4-yl)-[1,2,4]triazolo[4,3-*b*]pyridazine-6,8-diamine,
**(150)** 3-isopropyl-*N6*-(pentan-3-yl)-*N8*-(pyrimidin-4-yl)-[1,2,4]triazolo[4,3-*b*]pyridazine-6,8-diamine,
**(151)** 3-isopropyl-*N6*-(pentan-3-yl)-*N8*-(pyrimidin-5-yl)-[1,2,4]triazolo[4,3-*b*]pyridazine-6,8-diamine,
**(152)** 3-isopropyl-*N6*-(pentan-3-yl)-*N8*-(pyrimidin-2-yl)-[1,2,4]triazolo[4,3-*b*]pyridazine-6,8-diamine,
**(153)** 3-isopropyl-*N6*-(pentan-3-yl)-*N8*-(pyrazin-2-yl)-[1,2,4]triazolo[4,3-*b*]pyridazine-6,8-diamine,
**(154)** 3-isopropyl-*N8*-(6-methoxypyridin-2-yl)-*N6*-(pentan-3-yl)-[1,2,4]triazolo[4,3-*b*]pyridazine-6,8-diamine,
**(155)** 3-isopropyl-*N8*-(5-methoxypyridin-2-yl)-*N6*-(pentan-3-yl)-[1,2,4]triazolo[4,3-*b*]pyridazine-6,8-diamine,
**(156)** 3-isopropyl-*N8*-(4-methoxypyridin-2-yl)-*N6*-(pentan-3-yl)-[1,2,4]triazolo[4,3-*b*]pyridazine-6,8-diamine,
**(157)** 3-isopropyl-*N8*-(3-methxypyridin-2-yl)-*N6*-(pentan-3-yl)-[1,2,4]triazolo[4,3-*b*]pyridazine-6,8-diamine,
**(158)** 3-isopropyl-*N8*-(2-methoxypyridin-3-yl)-*N6*-(pentan-3-yl)-[1,2,4]triazolo[4,3-*b*]pyridazine-6,8-diamine,
**(159)** 3-isopropyl-*N8*-(6-methoxypyridin-3-yl)-*N6*-(pentan-3-yl)-[1,2,4]triazolo[4,3-*b*]pyridazine-6,8-diamine,
**(160)** isopropyl-*N8*-(5-methoxypyridin-3-yl)-*N6*-(pentan-3-yl)-[1,2,4]triazolo[4,3-*b*]pyridazine-6,8-diamine,
**(161)** 3-isopropyl-*N8*-(4-methoxypyridin-3-yl)-*N6*-(pentan-3-yl)-[1,2,4]triazolo[4,3-*b*]pyridazine-6,8-diamine,
**(162)** 3-isopropyl-*N8*-(5-methoxypyridazin-3-yl)-*N6*-(pentan-3-yl)-[1,2,4]triazolo[4,3-*b*] pyridazine- 6,8-diamine,
**(163)** (2*R*)-2-[[3-isopropyl-8-(2-pyridylamino)-[1,2,4]triazolo[4,3-*b*]pyridazin-6-yl]amino]butan-1-ol,
**(164)** *N6*-(2-ethylbutyl)-3-isopropyl-*N8*-[2-(2-pyridyl)ethyl]-[1,2,4]triazolo[4,3-*b*]pyridazine-6,8-diamine,
**(165)** *N6*-cyclopentyl-3-isopropyl-*N8*-[2-(2-pyridyl)ethyl]-[1,2,4]triazolo[4,3-*b*]pyridazine-6,8-diamine,
**(166)** *N6*-(2-ethylbutyl)-3-isopropyl-*N8*-[2-(3-pyridyl)ethyl]-[1,2,4]triazolo[4,3-*b*]pyridazine-6,8-diamine,
**(167)** *N6*,*N8*-bis(2-ethylbutyl)-3-isopropyl-[1,2,4]triazolo[4,3-*b*]pyridazine-6,8-diamine
**(168)** *N*-[6-(1-ethylpropylamino)-3-isopropyl-[1,2,4]triazolo[4,3-*b*]pyridazin-8-yl]-2-(3-pyridyl)acetamide,
**(169)** (*2R*)-2-[[3-isopropyl-8-(2-pyridylmethylamino)imidazo[1,2-*b*]pyridazin-6-yl]amino]butan-1-ol,
**(170)** *N6*-(1-ethylpropyl)-3-isopropyl-*N8*-(3-phenylpropyl)-[1,2,4]triazolo[4,3-*b*]pyridazine-6,8-diamine,
**(171)** 3-cyclopropyl-*N6*-(1-ethylpropyl)-*N8*-phenyl-[1,2,4]triazolo[4,3-*b*]pyridazine-6,8-diamine,
**(172)** 3-cyclopropyl-*N6*-(1-ethylpropyl)-*N8*-(2-pyridyl)-[1,2,4]triazolo[4,3-*b*]pyridazine-6,8-diamine,
**(173)** 3-cyclopropyl-*N6*-(1-ethylpropyl)-*N8*-(3-pyridyl)-[1,2,4]triazolo[4,3-*b*]pyridazine-6,8-diamine,
**(174)** 3-cyclopropyl-*N6-*(1-ethylpropyl)-*N8*-(4-pyridyl)-[1,2,4]triazolo[4,3-*b*]pyridazine-6,8-diamine,
**(175)** 3-cyclopropyl-*N6*-(pentan-3-yl)-*N8*-(pyridazin-3-yl)-[1,2,4]triazolo[4,3-*b*]pyridazine-6,8-diamine,
**(176)** 3-cyclopropyl-*N6*-(pentan-3-yl)-*N8*-(pyridazin-4-yl)-[1,2,4]triazolo[4,3-*b*]pyridazine-6,8-diamine,
**(177)** 3-cyclopropyl-*N6*-(pentan-3-yl)-*N8*-(pyrimidin-4-yl)-[1,2,4]triazolo[4,3-*b*]pyridazine-6,8-diamine,
**(178)** 3-cyclopropyl-*N6*-(1-ethylpropyl)-*N8*-pyrimidin-5-yl-[1,2,4]triazolo[4,3-*b*]pyridazine-6,8-diamine,
**(179)** 3-cyclopropyl-*N6*-(pentan-3-yl)-*N8*-(pyrimidin-2-yl)-[1,2,4]triazolo[4,3-*b*]pyridazine-6,8-diamine,
**(180)** 3-cyclopropyl-*N6*-(pentan-3-yl)-*N8*-(pyrazin-2-yl)-[1,2,4]triazolo[4,3-*b*]pyridazine-6,8-diamine,
**(181)** (*R*)-2-((3-cyclopropyl-8-(pyridazin-3-ylamino)-[1,2,4]triazolo[4,3-*b*]pyridazin-6-yl)amino)butan-1-ol,
**(182)** (*S*)-2-((3-cyclopropyl-8-(pyridazin-3-ylamino)-[1,2,4]triazolo[4,3-*b*]pyridazin-6-yl)amino)butan-1-ol,
**(183)** 3-cyclopropyl-*N8*-(6-methoxypyridin-2-yl)-*N6*-(pentan-3-yl)-[1,2,4]triazolo[4,3-*b*]pyridazine-6,8-diamine,
**(184)** 3-cyclopropyl-*N8*-(2-methoxypyridin-3-yl)-*N6*-(pentan-3-yl)-[1,2,4]triazolo[4,3-*b*]pyridazine-6,8-diamine,
**(185)** (2*R*)-2-[[3-cyclopropyl-8-[2-(2-pyridyl)ethylamino]-[1,2,4]triazolo[4,3-*b*]pyridazin-6-yl] amino]butan-1-ol,
**(186)** *N6*-benzyl-3-cyclopropyl-*N8*-(pyridin-2-ylmethyl)-[1,2,4]triazolo[4,3-*b*]pyridazine-6,8-diamine,
**(187)** 3-cyclopropyl-*N6*-(4-methoxybenzyl)-*N8*-(pyridin-2-ylmethyl)-[1,2,4] triazolo[4,3-*b*]pyridazine-6,8-diamine,
**(188)** 3-cyclopropyl-*N6*-(4-methoxybenzyl)-*N6*-methyl-*N8*-(pyridin-2-ylmethyl)-[1,2,4] triazolo [4,3 -b]pyridazine-6,8-diamine,
**(189)** 3-cyclopropyl-*N6*-ethyl-*N8*-(2-pyridylmethyl)-[1,2,4]triazolo[4,3-*b*]pyridazine-6,8-diamine,
**(190)** 3-cyclopropyl-*N6*-propyl-*N8*-(pyridin-2-ylmethyl)-[1,2,4]triazolo[4,3-*b*]pyridazine-6,8-diamine,
**(191)** *N6*-butyl-3-cyclopropyl-*N8*-(pyridin-2-ylmethyl)-[1,2,4]triazolo[4,3-*b*]pyridazine-6,8-diamine,
**(192)** 3-cyclopropyl-*N6*-isopropyl-*N8*-(pyridin-2-ylmethyl)-[1,2,4]triazolo[4,3-*b*]pyridazine-6,8-diamine,
**(193)** *N6*-(sec-butyl)-3-cyclopropyl-*N8*-(pyridin-2-ylmethyl)-[1,2,4]triazolo[4,3-*b*]pyridazine-6,8-diamine,
**(194)** 3-cyclopropyl-*N6*-isobutyl-*N8*-(pyridin-2-ylmethyl)-[1,2,4]triazolo[4,3-*b*]pyridazine-6,8-diamine,
**(195)** *N6*-(tert-butyl)-3-cyclopropyl-*N8*-(pyridin-2-ylmethyl)-[1,2,4]triazolo[4,3-*b*]pyridazine-6,8-diamine,
(196) 3-cyclopropyl-*N*6-(2-ethylbutyl)-*N*8-(pyridin-2-ylmethyl)-[1,2,4]triazolo[4,3-*b*]pyridazine-6,8-diamine,
(197) *N6*,3-dicyclopropyl-*N8*-(pyridin-2-ylmethyl)-[1,2,4]triazolo[4,3-*b*]pyridazine-6,8-diamine,
(198) *N6*-cyclobutyl-3-cyclopropyl-*N8*-(pyridin-2-ylmethyl)-[1,2,4]triazolo[4,3-*b*]pyridazine-6,8-diamine,
(199) *N6*-cyclopentyl-3-cyclopropyl-*N8*-(pyridin-2-ylmethyl)-[1,2,4]triazolo[4,3-*b*]pyridazine-6,8-diamine,
(200) *N6*-cyclohexyl-3-cyclopropyl-*N8*-(pyridin-2-ylmethyl)-[1,2,4]triazolo[4,3-*b*]pyridazine-6,8-diamine,
(201) *N6*-cycloheptyl-3-cyclopropyl-*N8*-(pyridin-2-ylmethyl)-[1,2,4]triazolo[4,3-*b*]pyridazine-6,8-diamine,
(202) *N6*-((3s,5s,7s)-adamantan-1-yl)-3-cyclopropyl-*N8*-(pyridin-2-ylmethyl)-[1,2,4]triazolo[4,3-*b*]pyridazine-6,8-diamine,
(203) 3-cyclopropyl-*N*6-(cyclopropylmethyl)-*N*8-(pyridin-2-ylmethyl)-[1,2,4]triazolo[4,3-*b*]pyridazine-6,8-diamine,
(204) *N6*-(cyclobutylmethyl)-3-cyclopropyl-*N8*-(pyridin-2-ylmethyl)-[1,2,4]triazolo[4,3-*b*]pyridazine-6,8-diamine,
(205) 3-cyclopropyl-*N*8-(pyridin-2-ylmethyl)-*N*6-(spiro[3.3]heptan-2-yl)-[1,2,4]triazolo[4,3-*b*]pyridazine-6,8-diamine,
(206) 3-cyclopropyl-*N6*-((1-methylcyclobutyl)methyl)-*N8*-(pyridin-2-ylmethyl)-[1,2,4]triazolo[4,3-*b*]pyridazine-6,8-diamine,
(207) 3-cyclopropyl-6-(1-piperidyl)-*N*-(2-pyridylmethyl)-[1,2,4]triazolo[4,3-*b*]pyridazin-8-amine,
(208) 3-cyclopropyl-*N6*-(furan-2-ylmethyl)-*N8*-(pyridin-2-ylmethyl)-[1,2,4]triazolo[4,3-*b*]pyridazine-6,8-diamine,
(209) 3-cyclopropyl-*N*6-(furan-3-ylmethyl)-*N*8-(pyridin-2-ylmethyl)-[1,2,4]triazolo[4,3-*b*]pyridazine-6,8-diamine,
(210) 3-cyclopropyl-*N8*-(pyridin-2-ylmethyl)-*N6*-(thiophen-2-ylmethyl)-[1,2,4]triazolo[4,3-*b*]pyridazine-6,8-diamine,
**(211)** (*R*)-2-((3-cyclopropyl-8-((pyridin-2-ylmethyl)amino)-[1,2,4]triazolo[4,3-*b*]pyridazin-6-yl)amino)butan-1-ol,
(212) (*S*)-2-((3-cyclopropyl-8-((pyridin-2-ylmethyl)amino)-[1,2,4]triazolo[4,3-*b*]pyridazin-6-yl)amino)butan-1-ol,
(213) (*R*)-3-cyclopropyl-*N8*-(pyridin-2-ylmethyl)-*N6*-(tetrahydrofuran-3-yl)-[1,2,4]triazolo[4,3-*b*]pyridazine-6,8-diamine,
(214) (*R*)-3-cyclopropyl-*N8*-(pyridin-2-ylmethyl)-*N6*-(tetrahydro-*2H*-pyran-3-yl)-[1,2,4]triazolo[4,3-*b*]pyridazine-6,8-diamine,
(215) 3-cyclopropyl-*N8*-(6-methoxypyridazin-3-yl)-*N6*-(pentan-3-yl)-[1,2,4]triazolo[4,3-*b*]pyridazine-6,8-diamine,
(216) 3-cyclopropyl-*N6*-(pentan-3-yl)-*N8*-(pyridazin-3-ylmethyl)-[1,2,4]triazolo[4,3-*b*]pyridazine-6,8-diamine,
(217) 3-cyclopropyl-*N6*-(pentan-3-yl)-*N8*-phenethyl-[1,2,4]triazolo[4,3-*b*]pyridazine-6,8-diamine,
(218) 3-cyclopropyl-*N8*-(pyridin-2-ylmethyl)-*N6*-(thiophen-3-ylmethyl)-[1,2,4]triazolo[4,3-*b*]pyridazine-6,8-diamine,
(219) 3-cyclopropyl-*N*6-methyl-*N*8-(pyridin-2-ylmethyl)-[1,2,4]triazolo[4,3-*b*]pyridazine-6,8-diamine,
(220) 3-cyclopropyl-*N*-(pyridin-2-ylmethyl)-6-(pyrrolidin-1-yl)-[1,2,4]triazolo[4,3-*b*]pyridazin-8-amine,
in particular selected from compounds (1), (2), (10), (13), (14), (17) to (19), (21) to (23), (26), (28), (29), (31), (35) to (56), (58) to (64), (66) to (69), (71) to (78), (80) to (105), (107), (110), (111), (114), (117) to (119), (121) to (124), (126), (127), (130) to (132), (134), (136), (138), (140), (143), (146) to (160), (162) to (187), (189) to (206), (208) to (212) and (215) to (218), and their pharmaceutically acceptable salts, more particularly from compounds (18), (38), (40) to (42), (45) to (53), (56), (59), (62), (63), (67), (68), (73), (75), (76), (80), (82) to (88), (90), (91), (93) to (95), (98) to (100), (114), (118), (126), (130), (147) to (151), (153) to (156), (158) to (160), (162), (164) to (168), (170) to (178), (180) to (183), (186), (187), (191) to (193), (195), (196), (198) to (202), (204) to (206), (208) to (210) and (215) to (218), and their pharmaceutically acceptable salts, and even more particularly from compounds (46), (47), (83), (86), (87), (98), (147), (148), (150), (151), (153) to (155), (158), (159), (162), (164), (168), (171) to (175), (177), (178), (180) to (183) and (215), and their pharmaceutically acceptable salts.

7. Synthesis process for manufacturing a compound of formula (I) as defined in anyone of claim 1 to 5 or any of its pharmaceutically acceptable salts, or at least any of compounds (1) to (220) as defined in claim 6 or any of their pharmaceutically acceptable salts, comprising at least a step of reacting a compound of formula (II) 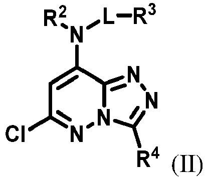 wherein L, R², R³ and R⁴ are as defined in anyone of claim 1, 3 or 4, with an amine of formula (IV)
NHR⁵R⁶ (IV)
wherein R⁵ and R⁶ are as defined in claim 1 or 5, for example in a molar ratio ranging from 1 to 20 eq, with respect to the compound of formula (II), in an aprotic solvent or without solvent or else in a protic solvent.

8. Synthesis process for manufacturing a compound of formula (I) as defined in anyone of claim 1 to 5 or any of its pharmaceutically acceptable salts, or at least any of compounds (1) to (220) as defined in claim 6 or any of their pharmaceutically acceptable salts, comprising at least a step of reacting a compound of formula (IX) 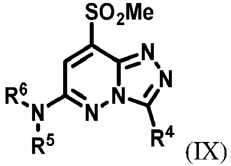 wherein R⁴, R⁵ and R⁶ are as defined in claim 1, 4 and 5, with a compound of formula (VI) 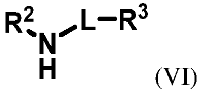 wherein R² and R³ are as defined in claim 1 or 3, in an aprotic solvent, for example in a molar ratio ranging from 1 to 10 eq, with respect to the compound of formula (IX).

9. Synthesis process for manufacturing a compound of formula (I) as defined in anyone of claim 1 to 5 or any of its pharmaceutically acceptable salts, or at least any of compounds (1) to (220) as defined in claim 6 or any of their pharmaceutically acceptable salts, comprising at least a step of reacting a compound of formula (XII) 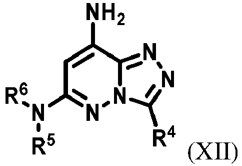 wherein R⁵ and R⁶ are as defined in claim 1 or 5, with a compound of formula (XIV) 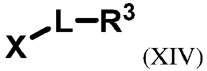 wherein L and R³ are as defined in claim 1 or 3 and X represents a hydroxy group, a chlorine atom, a bromine atom, an (C₁-C₃)alkyl sulfonate group or a phenyl sulfonate group, in an aprotic solvent for example in a molar ratio ranging from 1 to 10 eq, with respect to the compound of formula (XII).

10. A compound of formula (II), (X), (XI) or (VII), in particular as an intermediate compound 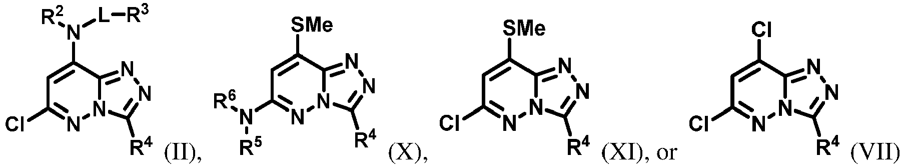 wherein L, R², R³, R⁴, R⁵ and R⁶ are as defined in claims 1, 3, 4 or 5, provided that R⁴ is not a methyl group in formula (XI).

11. A compound of formula (I) as defined in anyone of claim 1 to 5 or any of its pharmaceutically acceptable salts, or at least any of compounds (1) to (220) as defined in claim 6 or any of their pharmaceutically acceptable salts, for use as a medicament.

12. A compound of formula (I) as defined in anyone of claim 1 to 5 or any of its pharmaceutically acceptable salts, or at least any of compounds (1) to (220) as defined in claim 6 or any of their pharmaceutically acceptable salts, for use in the prevention and/or the treatment of ulcerative colitis, atopic dermatitis, glaucoma, asthma, liver fibrosis, kidney diseases, atherosclerosis, hypercholestemia, hearing loss and cancers developing under hypoxic conditions, in particular glioblastoma.

13. A compound for use as defined in claim 12, wherein the hearing loss is a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors.

14. A compound for use as defined in claim 12, wherein the kidney disease is selected from kidney fibrosis, nephropathy, acute kidney injury, chronic kidney disease in particular induced by ischemia and/or reperfusion injury, toxic nephropathy or myoglobinuria, and diabetic kidney disease, or the kidney disease is induced by nephrotoxicity caused by therapeutic drugs, more particularly cytotoxic agents, such as platin-based anticancer drugs for example cisplatin or carboplatin.

15. A pharmaceutical composition comprising at least one compound of formula (I) as defined in anyone of claim 1 to 5 or any of its pharmaceutically acceptable salt, or at least any of compounds (1) to (220) as defined in claim 6 or any of their pharmaceutically acceptable salts.
